(19) Europäisches Patentamt
European Patent Office
Office européen des brevets

(11) EP 2 112 229 A2

(12) **EUROPEAN PATENT APPLICATION**

(43) Date of publication:
**28.10.2009 Bulletin 2009/44**

(51) Int Cl.:
***C12Q 1/68*** *(2006.01)*

(21) Application number: **09005352.1**

(22) Date of filing: **25.11.2003**

(84) Designated Contracting States:
**AT BE BG CH CY CZ DE DK EE ES FI FR GB GR HU IE IT LI LU MC NL PT RO SE SI SK TR**

(30) Priority: **25.11.2002 US 429136 P**
**24.07.2003 US 490234 P**

(62) Document number(s) of the earlier application(s) in accordance with Art. 76 EPC:
**09003828.2**
**03787201.7 / 1 565 579**

(71) Applicant: **SEQUENOM, INC.**
**San Diego,**
**California 92121 (US)**

(72) Inventors:
- **Roth, Richard B.**
**La Jolla, CA 92037 (US)**
- **Nelson, Matthew Roberts**
**San Marcos, CA 92069 (US)**
- **Braun, Andreas**
**San Diego, CA 92130 (US)**
- **Kammerer, Stefan M.**
**San Diego, CA 92130 (US)**
- **Reneland, Rikard**
**San Diego, CA 92122 (US)**

(74) Representative: **Walker, Ross Thomson et al**
**Potts, Kerr & Co.**
**15 Hamilton Square**
**Birkenhead,**
**Merseyside CH41 6BR (GB)**

Remarks:
This application was filed on 15-04-2009 as a divisional application to the application mentioned under INID code 62.

# (54) Methods for identifying risk of breast cancer and treatments thereof

(57) Provided herein are methods for identifying risk of breast cancer in a subject and/or a subject at risk of breast cancer, reagents and kits for carrying out the methods, methods for identifying candidate therapeutics for treating breast cancer, and therapeutic methods for treating breast cancer in a subject. These embodiments are based upon an analysis of polymorphic variations in nucleotide sequences within the human genome.

## FIGURE 15

**MAPK10**

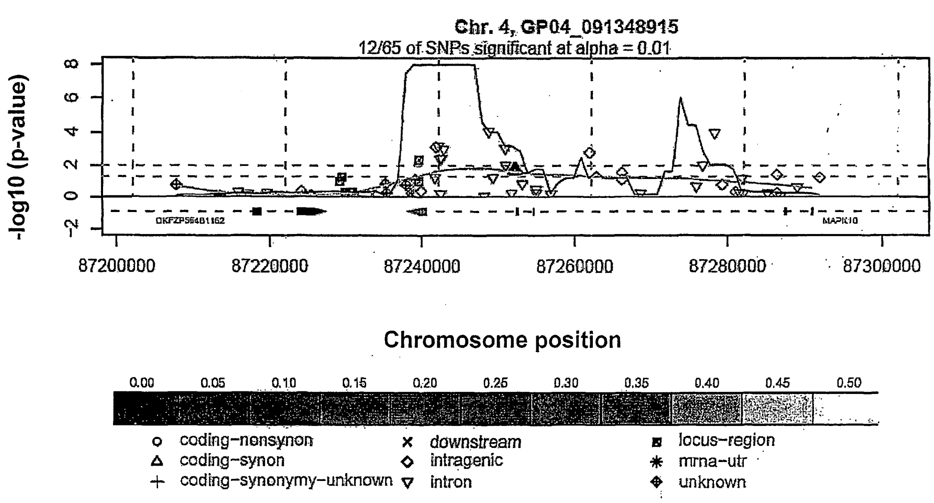


EP 2 112 229 A2

## Description

### Field of the Invention

The invention relates to genetic methods for identifying risk of breast cancer and treatments that specifically target the disease.

### Background

Breast cancer is the third most common cancer, and the most common cancer in women, as well as a cause of disability, psychological trauma, and economic loss. Breast cancer is the second most common cause of cancer death in women in the United States, in particular for women between the ages of 15 and 54, and the leading cause of cancer-related death (Forbes, Seminars in Oncology, vol.24(1), Suppl 1, 1997: pp.S1-20-S1-35). Indirect effects of the disease also contribute to the mortality from breast cancer including consequences of advanced disease, such as metastases to the bone or brain. Complications arising from bone marrow suppression, radiation fibrosis and neutropenic sepsis, collateral effects from therapeutic interventions, such as surgery, radiation, chemotherapy, or bone marrow transplantation-also contribute to the morbidity and mortality from this disease.

While the pathogenesis of breast cancer is unclear, transformation of normal breast epithelium to a malignant phenotype may be the result of genetic factors, especially in women under thirty (Miki, et al., Science, 266: 66-71 (1994)). However, it is likely that other, non-genetic factors also have a significant effect on the etiology of the disease. Regardless of its origin, breast cancer morbidity increases significantly if it is not detected early in its progression. Thus, considerable efforts have focused on the elucidation of early cellular events surrounding transformation in breast tissue. Such efforts have led to the identification of several potential breast cancer markers. For example, alleles of the *BRCA1* and *BRCA2* genes have been linked to hereditary and early-onset breast cancer (Wooster, et al., Science, 265: 2088-2090 (1994)). However, *BRCA1* is limited as a cancer marker because *BRCA1* mutations fail to account for the majority of breast cancers (Ford, et al., British J. Cancer, 72: 805-812 (1995)). Similarly, the *BRCA2* gene, which has been linked to forms of hereditary breast cancer, accounts for only a small portion of total breast cancer cases.

### Summary

It has been discovered that certain polymorphic variations in human genomic DNA are associated with the occurrence of breast cancer. In particular, polymorphic variants in loci containing *ICAM, MAPK10, KIAA0861, NUMA1*/*FLJ20625*/*LOC220074* (hereafter referred to as "*NUMA1*"), and *HT014*/*LOC148902*/*LYPLA2*/*GALE* (hereafter refered to as "*GALE*") regions in human genomic DNA have been associated with risk of breast cancer.

Thus, featured herein are methods for identifying a subject at risk of breast cancer and/or a risk of breast cancer in a subject, which comprises detecting the presence or absence of one or more polymorphic variations accociated with breast cancer in genomic regions described herein in a human nucleic acid sample. In an embodiment, two or more polymorphic variations are detected in two or more regions selected from the group consisting of *ICAM, MAPK10, KIAA0861, NUMA1* and *GALE*. In certain embodiments, 3 or more, or 4, 5, 6, 7, 8, 9, 10, 11, 12, 13, 14, 15, 16, 17, 18, 19 or 20 or more polymorphic variants are detected. In specific embodiments, the group of polymorphic variants detected comprise or consist of polymorphic variants in *ICAM, MAPK10, KIAA0861, NUMA1* and *GALE*, such as position 44247 in SEQ ID NO: 1 (*ICAM*), position 36424 in SEQ ID NO: 2 (*MAPK10*), position 48563 in SEQ ID NO: 3 (*KIAA0861*), position 49002 in SEQ ID NO: 4 (*NUMA1*) and position 174 in SEQ ID NO: *5* (*GALE*), for example.

Also featured are nucleic acids that include one or more polymorphic variations associated with the occurrence of breast cancer, as well as polypeptides encoded by these nucleic acids. Further, provided is a method for identifying a subject at risk of breast cancer and then prescribing to the subject a breast cancer detection procedure, prevention procedure and/or a treatment procedure. In addition, provided are methods for identifying candidate therapeutic molecules for treating breast cancer and related disorders, as well as methods for treating breast cancer in a subject by diagnosing breast cancer in the subject and treating the subject with a suitable treatment, such as administering a therapeutic molecule.

Also provided are compositions comprising a breast cancer cell and/or *ICAM, MAPK10, KIAA0861, NUMA1* or *GALE* nucleic acid with a RNAi, siRNA, antisense DNA or RNA, or ribozyme nucleic acid designed from a *ICAM, MAPK10, KIAA0861, NUMA1* or *GALE* nucleotide sequence. In an embodiment, the nucleic acid is designed from a *ICAM, MAPK10, KIAA0861, NUMA1* or *GALE* nucleotide sequence that includes one or more breast cancer associated polymorphic variations, and in some instances, specifically interacts with such a nucleotide sequence. Further, provided are arrays of nucleic acids bound to a solid surface, in which one or more nucleic acid molecules of the array have a *ICAM, MAPK10, KIAA0861, NUMA1* or *GALE* nucleotide sequence, or a fragment or substantially identical nucleic acid thereof, or a complementary nucleic acid of the foregoing. Featured also are compositions comprising a breast cancer cell and/or a *ICAM, MAPK10, KIAA0861, NUMA1* or *GALE* polypeptide, with an antibody that specifically binds to the polypeptide. In an embodiment, the antibody specifically binds to an epitope in the polypeptide that includes a non-synonymous amino acid modification associated with breast cancer (e.g., results in an amino acid substitution in the encoded polypeptide associated with breast cancer). In certain embodiments, the antibody specifically binds to an epitope that comprises a proline at amino acid position 352 or an alanine at amino acid position 348 in an *ICAM5* polypeptide.

Brief Description of the Figures

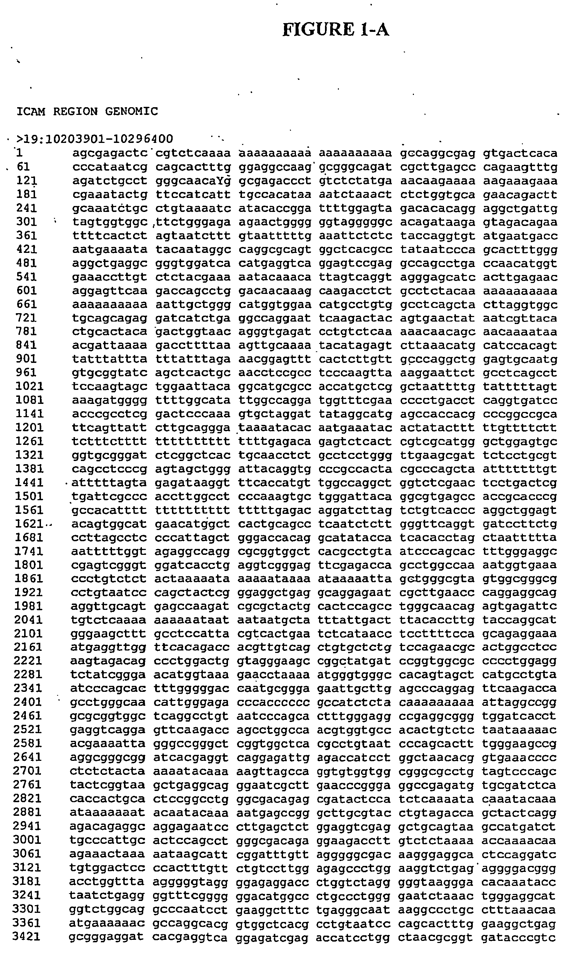

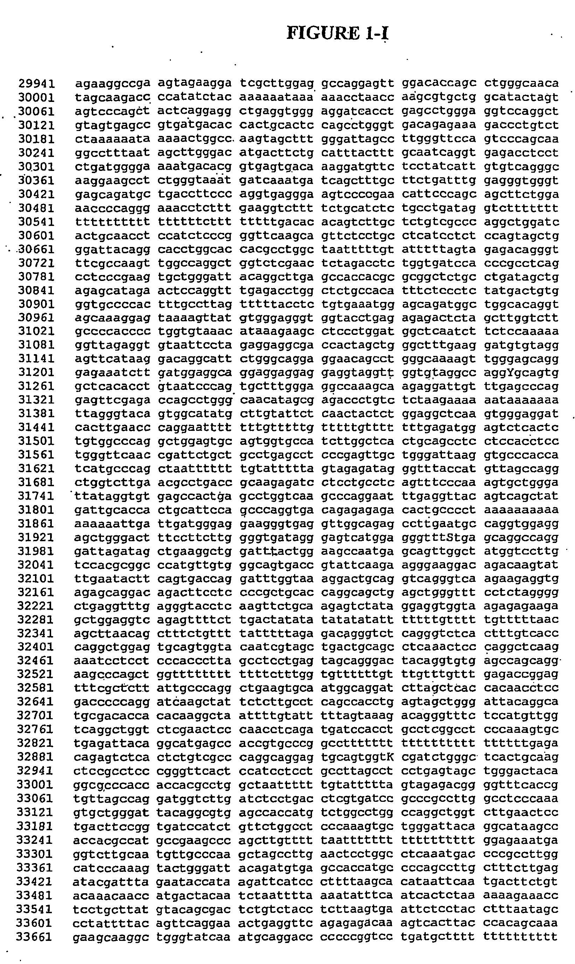

Figures 1A-1Y show a genomic nucleotide sequence for an ICAM region encoding ICAM 1, 4 and 5. The genomic nucleotide sequence is set forth in SEQ ID NO: 1. The following nucleotide representations are used throughout: "A" or "a" is adenosine, adenine, or adenylic acid; "C" or "c" is cytidine, cytosine, or cytidylic acid; "G" or "g" is guanosine, guanine, or guanylic acid; "T" or "t" is thymidine, thymine, or thymidylic acid; and "I" or "i" is inosine, hypoxanthine, or inosinic acid. Exons are indicated in italicized lower case type, introns are depicted in normal text lower case type, and polymorphic sites are depicted in bold upper case type. SNPs are designated by the following convention: "R" represents A or G, "M" represents A or C; "W" represents A or T; "Y" represents C or T; "S" represents C or G; "K" represents G or T; "V" represents A, C or G; "H" represents A, C, or T; "D" represents A, G, or T; "B" represents C, G, or T; and "N" represents A, G, C, or T.

Figures 2A-2U show a genomic nucleotide sequence of a MAPK10 region. The genomic nucleotide sequence is set forth in SEQ ID NO: 2.

Figures 3A-3NN show a genomic nucleotide sequence of a KIAA0861 region. The genomic nucleotide sequence is set forth in SEQ ID NO: 3.

Figures 4A-4JJ show a genomic nucleotide sequence of a NUMA1/FLJ20625/LOC220074 region, referred to herein as the NUMA1 region. The genomic nucleotide sequence is set forth in SEQ ID NO: 4.

Figure 5 shows a portion of a genomic nucleotide sequence of a HT014/LOC148902/LYPLA2/GALE region, referred to herein as the GALE region. The genomic nucleotide sequence is set forth in SEQ ID NO: 5.

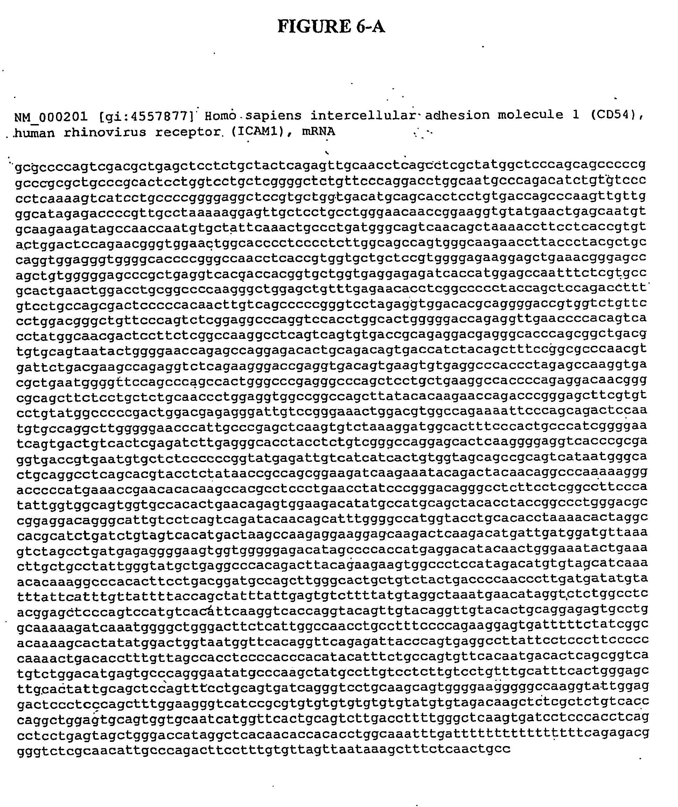

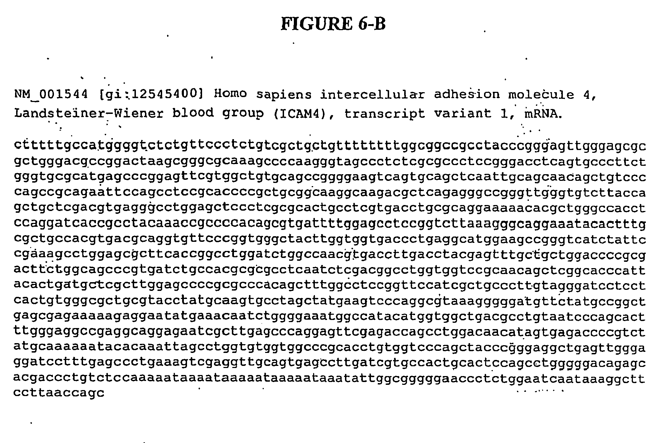

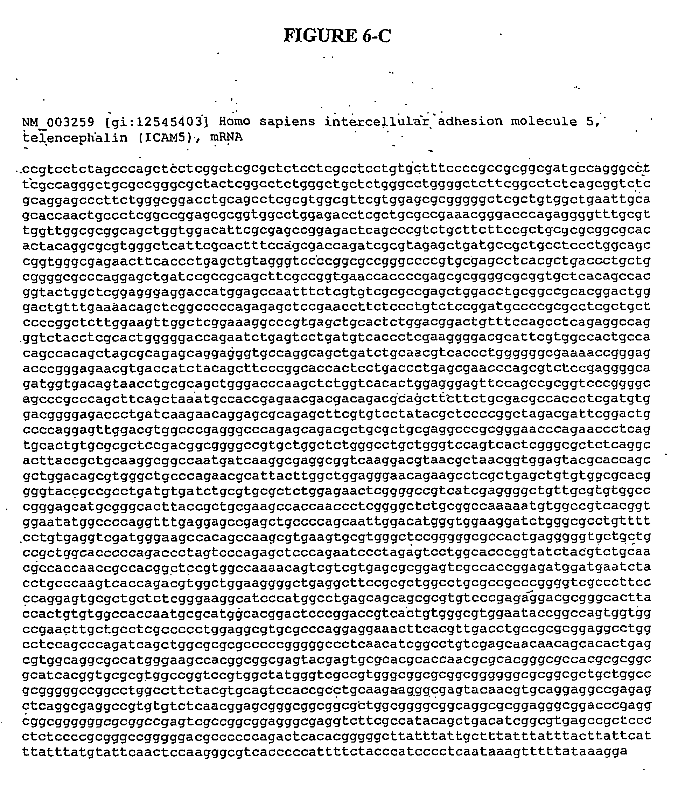

Figures 6A-6C show coding nucleotide sequences (cDNA) for ICAM1, ICAM4 and ICAM5, respectively. The nucleotide sequences are set forth in SEQ ID NOs: 6, 7 and 8, respectively.

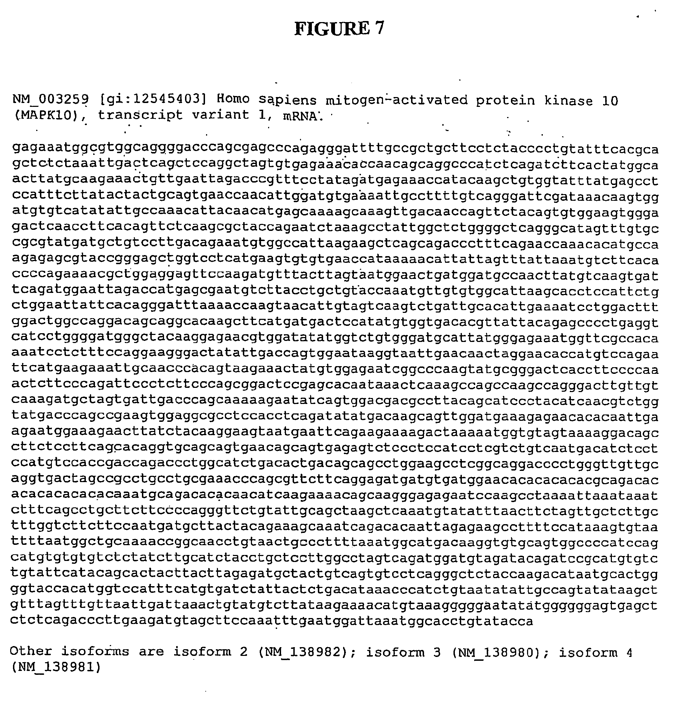

Figure 7 shows a coding nucleotide sequence (cDNA) for MAPK10. The nucleotide sequence is set forth in SEQ ID NO: 9.

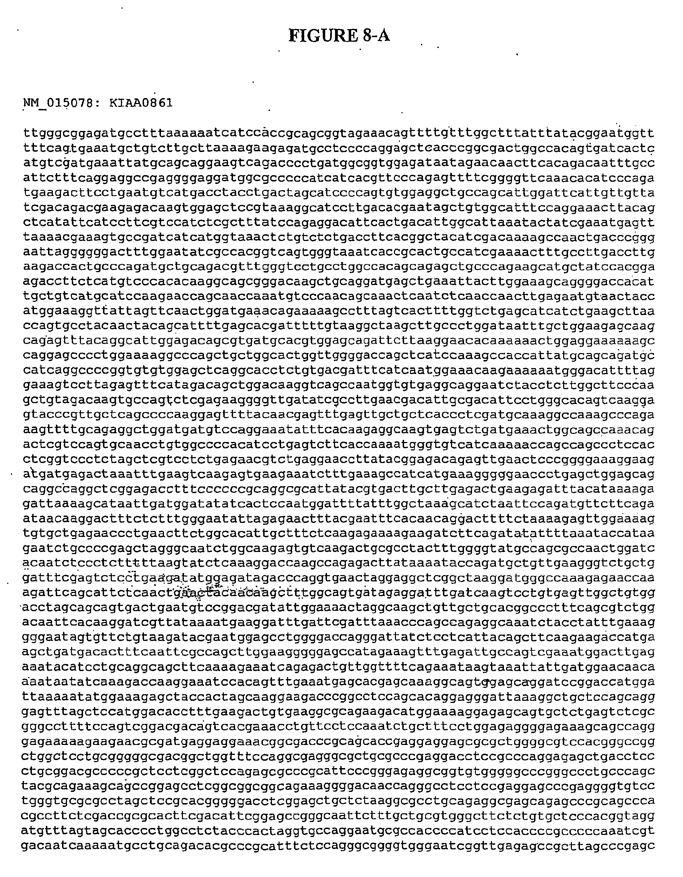

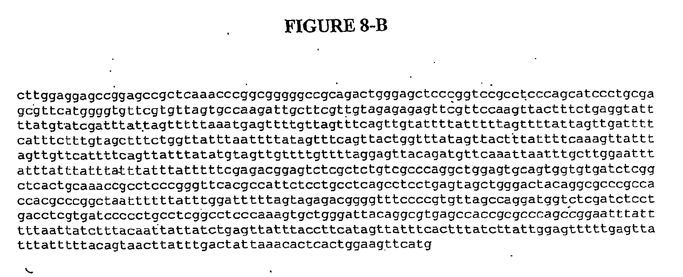

Figures 8A-8B show coding nucleotide sequences (cDNA) for KIAA0861. The nucleotide sequences are set forth in SEQ ID NO: 10 and 11, respectively.

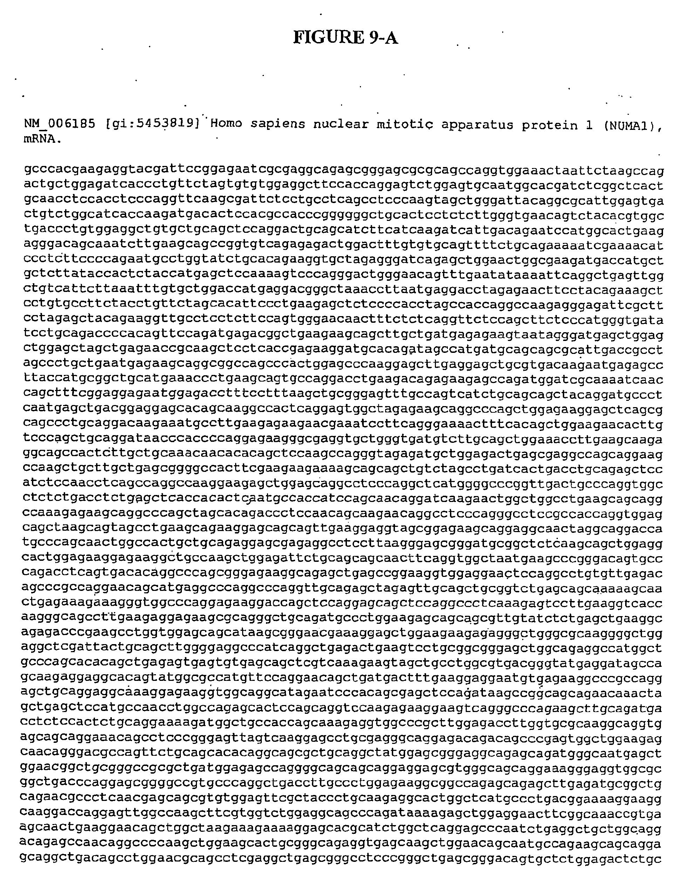

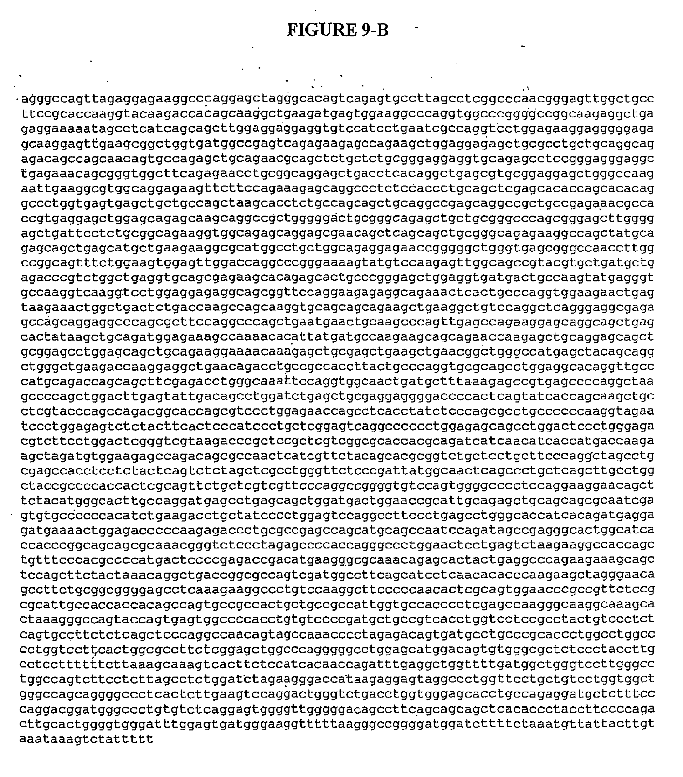

Figures 9A-9B show a coding nucleotide sequence (cDNA) for NUMA1. The nucleotide sequence is set forth in SEQ ID NO: 12.

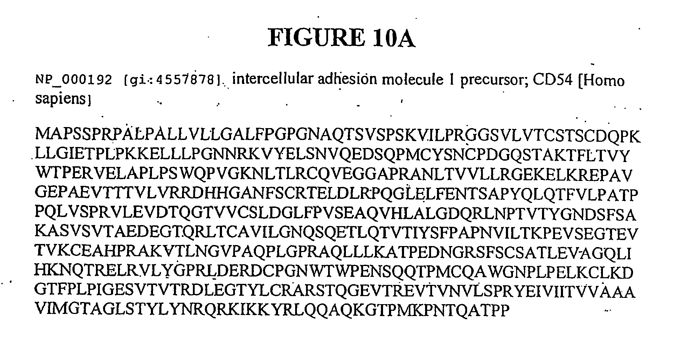

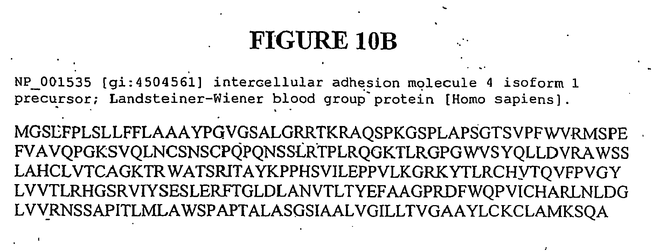

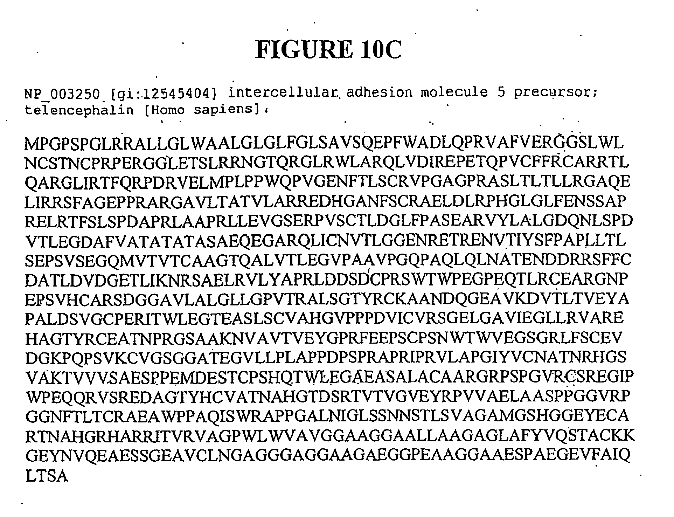

Figures 10A-10C show amino acid sequences for ICAM1, ICAM4 and ICAM5 polypeptides. The amino acid sequences are set forth in SEQ ID NOs: 13, 14 and 15, respectively.

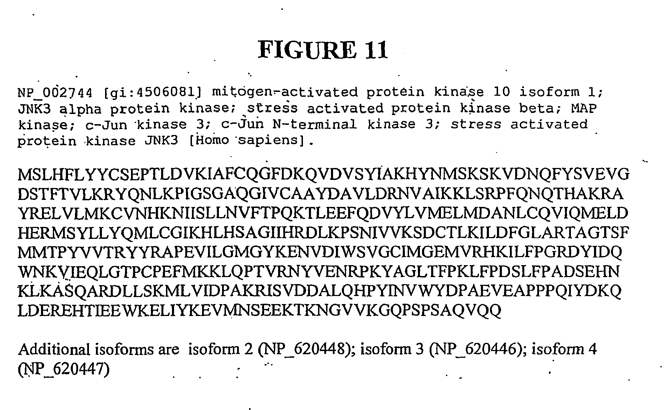

Figure 11 shows an amino acid sequence for a MAPK10 polypeptide, which is set forth in SEQ ID NO: 16.

Figure 12 shows an amino acid sequence for a KIAA0861 polypeptide, which is set forth in SEQ ID NO: 17.

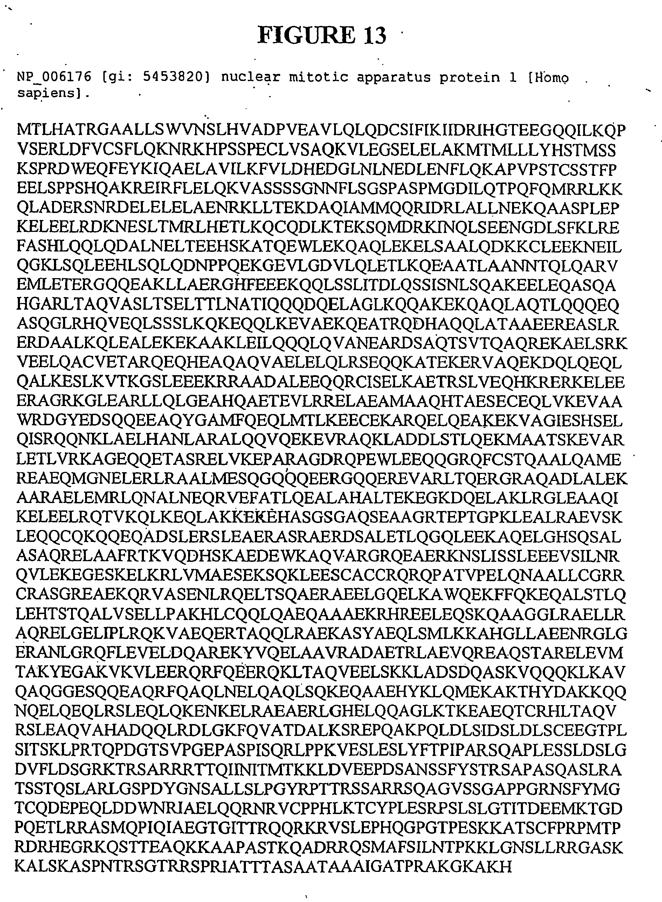

Figure 13 shows an amino acid sequence for a NUMA1 polypeptide, which is set forth in SEQ ID NO: 18.

Figure 14 shows proximal SNPs in the ICAM region in genomic DNA. The position of each SNP on the chromosome is shown on the x-axis and the y-axis provides the negative logarithm of the p-value comparing the estimated allele to that of the control group. Also shown in the figure are exons and introns of the genes in the approximate chromosomal positions. The figure indicates that polymorphic variants associated with breast cancer are in linkage disequilibrium in a region spanning positions 11851-24282, 36340-37868, 41213-41613, 70875-74228, 42407-45536, or 42407-51102 in SEQ ID NO: 1.

Figure 15 shows proximal SNPs in the MAP10 region in genomic DNA. The position of each SNP on the chromosome is shown on the x-axis and the y-axis provides the negative logarithm of the p-value comparing the estimated allele to that of the control group. Also shown in the figure are exons and introns of the genes in the approximate chromosomal positions. The figure indicates that polymorphic variants associated with breast cancer are in linkage disequilibrium in a region spanning positions 23826-36424, 46176-62572, 4512-8467 or 13787-14355 in SEQ ID NO: 2.

Figure 16 shows proximal SNPs in the KIAA0861 region in genomic DNA. The position of each SNP on the chromosome is shown on the x-axis and the y-axis provides the negative logarithm of the p-value comparing the estimated allele to that of the control group. Also shown in the figure are exons and introns of the genes in the approximate chromosomal positions. The figure indicates that polymorphic variants associated with breast cancer are in linkage disequilibrium in a region spanning positions 42164-48563 in SEQ ID NO: 3.

Figure 17 shows proximal SNPs in the KIAA0861 region in genomic DNA. The position of each SNP on the chromosome is shown on the x-axis and the y-axis provides the negative logarithm of the p-value comparing the estimated allele to that of the control group. Also shown in the figure are exons and introns of the genes in the approximate chromosomal positions. The figure indicates that polymorphic variants associated with breast cancer are in linkage disequilibrium in a region spanning positions 174-32954, 38115-43785, 45386-52058, 52257-54411, 55303-73803 or 96470-98184 in SEQ ID NO: 4.

Figure 18 shows results of an odds-ratio meta analysis for the ICAM region.

Figure 19 shows results of an odds-ratio meta analysis for the MAPK10 region.

Figure 20 shows results of an odds-ratio meta analysis for the KIAA0861 region.

Figure 21 shows results of an odds-ratio meta analysis for the NUMA1 region.

Figure 22 shows effects of ICAM-directed siRNA on cancer cell proliferation.

## Detailed Description

It has been discovered that polymorphic variations in the *ICAM, MAPK10, KIAA0861, NUMA1* and *GALE* regions described herein are associated with an increased risk of breast cancer.

All *ICAM* proteins are type I transmembrane glycoproteins, contain 2-9 immunoglobulin-like C2-type domains, and bind to the leukocyte adhesion LFA-1 protein. The proteins are members of the intercellular adhesion molecule (*ICAM*) family. The gene *ICAM1* (intercellular adhesion molecule-1) is also known as human rhinovirus receptor, BB2, CD54. and cell surface glycoprotein P3.58. ICAM1 has been mapped to chromosomal position 19p13.3-p13.2. ICAM1 (CD54) typically is expressed on endothelial cells and cells of the immune system. *ICAM1* binds to integrins of type CD11a / CD18, or CD11b / CD18. ICAM1 is also exploited by Rhinovirus as a receptor.

The gene *ICAM4* (intercellular adhesion molecule 4) is also known as the Landsteiner-Wiener blood group or LW. *ICAM4* has been mapped to 19p13.2-cen. The protein encoded by this gene is a member of the intercellular adhesion molecule (ICAM) family. A glutamine to arginine polymorphism in this protein is responsible for the Landsteiner-Wiener blood group system (GLN=WB(A); ARG=WB(B). This gene consists of 3 exons and alternative splicing generates 2 transcript variants.

The gene *ICAM5* (intercellular adhesion molecule 5) is also known as telencephalin. *ICAM5* has been mapped to 19p13.2. The protein encoded by the gene is expressed on the surface of telencephalic neurons and displays two types of adhesion activity, homophilic binding between neurons and heterophilic binding between neurons and leukocytes. It may be a critical component in neuron-microglial cell interactions in the course of normal development or as part of neurodegenerative diseases.

The gene *MAPK10* also is known as JNK3, JNK3A, PRKM10, p493F12, FLJ12099, p54bSAPK MAP kinase, c-Jun kinase 3, JNK3 alpha protein kinase, c-Jun N-terminal kinase 3, stress activated protein kinase JNK3, stress activated protein kinase beta. *MAPK10* has been mapped to chromosomal position 4q22.1-q23. The protein encoded by this gene is a member of the MAP kinase family. MAP kinases act as an integration point for multiple biochemical signals, and are involved in a wide variety of cellular processes such as proliferation, differentiation, transcription regulation and development. This protein is a neuronal-specific form of c-Jun N-terminal kinases (JNKs). Through its phosphorylation and nuclear localization, this kinase plays regulatory roles in the signaling pathways during neuronal apoptosis. Beta-arrestin 2, a receptor-regulated MAP kinase scaffold protein, is found to interact with, and stimulate the phosphorylation of this kinase by MAP kinase kinase 4 (MKK4). Cyclin-dependent kinase 5 can phosphorylate, and inhibit the activity of this kinase, which may be important in preventing neuronal apoptosis. Four alternatively spliced transcript variants encoding distinct isoforms have been reported.

The gene *KIAA0861* is a Rho family guanine-nucleotide exchange factor. *KIAA0861* has been mapped to chromosomal position 3q27.3. *KIAA0861* is a Rho family nucleotide exchange factor homolog that modulates the activity of Rho family GTPases, which control numerous cell functions, including cell growth, adhesion, movement and shape. RhoC GTPase is overexpressed in invasive (inflammatory) breast cancers.

The gene *FLJ20625* has been mapped to chromosomal position 11q13.3. The gene encoding LOC220074 also is known as Hypothetical 55.1 kDa protein F09G8.5 in chromosome III and has been mapped to chromosomal position 11q13.3.

The gene *HT014* has been mapped to chromosomal position 1p36.11. The gene *LYPLA2* (lysophospholipase II) also is known as APT-2, DJ886K2.4 and acyl-protein thioesterase and has been mapped to chromosomal position 1p36.12-p35.1. Lysophospholipases are enzymes that act on biological membranes to regulate the multifunctional lysophospholipids. There are alternatively spliced transcript variants described for this gene but the full length nature is not known yet.

The gene *GALE* (galactose-4-epimerase, UDP-) also is known as galactowaldenase UDP galactose-4-epimerase and has been mapped to chromosomal position 1p36-p35. This gene encodes UDP-galactose-4-epimerase which catalyzes 2 distinct but analogous reactions: the epimerization of UDP-glucose to UDP-galactose, and the epimerization of UDP-N-acetylglucosamine to UDP-N-acetylgalactosamine. The bifunctional nature of the enzyme has the important metabolic consequence that mutant cells (or individuals) are dependent not only on exogenous galactose, but also on exogenous N-acetylgalactosamine for necessary precursor for the synthesis of glycoproteins and glycolipids. The missense mutations in the *GALE* gene result in the epimerase-deficiency galactosemia.

Breast Cancer and Sample Selection

Breast cancer is typically described as the uncontrolled growth of malignant breast tissue. Breast cancers arise most commonly in the lining of the milk ducts of the breast (ductal carcinoma), or in the lobules where breast milk is produced (lobular carcinoma). Other forms of breast cancer include Inflammatory Breast Cancer and Recurrent Breast Cancer. Inflammatory breast cancer is a rare, but very serious, aggressive type of breast cancer. The breast may look red and feel warm with ridges, welts, or hives on the breast; or the skin may look wrinkled. It is sometimes misdiagnosed as a simple infection. Recurrent disease means that the cancer has come back after it has been treated. It may come back in the breast, in the soft tissues of the chest (the chest wall), or in another part of the body.

As used herein, the term "breast cancer" refers to a condition characterized by anomalous rapid proliferation of abnormal cells in one or both breasts of a subject. The abnormal cells often are referred to as "neoplastic cells," which are transformed cells that can form a solid tumor. The term "tumor" refers to an abnormal mass or population of cells (*i.e*. two or more cells) that result from excessive or abnormal cell division, whether malignant or benign, and pre-cancerous and cancerous cells. Malignant tumors are distinguished from benign growths or tumors in that, in addition to uncontrolled cellular proliferation, they can invade surrounding tissues and can metastasize. In breast cancer, neoplastic cells may be identified in one or both breasts only and not in another tissue or organ, in one or both breasts and one or more adjacent tissues or organs (*e.g*. lymph node), or in a breast and one or more non-adjacent tissues or organs to which the breast cancer cells have metastasized.

The term "invasion" as used herein refers to the spread of cancerous cells to adjacent surrounding tissues. The term "invasion" often is used synonymously with the term "metastasis," which as used herein refers to a process in which cancer cells travel from one organ or tissue to another non-adjacent organ or tissue. Cancer cells in the breast (s) can spread to tissues and organs of a subject, and conversely, cancer cells from other organs or tissue can invade or metastasize to a breast. Cancerous cells from the breast(s) may invade or metastasize to any other organ or tissue of the body. Breast cancer cells often invade lymph node cells and/or metastasize to the liver, brain and/or bone and spread cancer in these tissues and organs. Breast cancers can spread to other organs and tissues and cause lung cancer, prostate cancer, colon cancer, ovarian cancer, cervical cancer, gastrointestinal cancer, pancreatic cancer, glioblastoma, bladder cancer, hepatoma, colorectal cancer, uterine cervical cancer, endometrial carcinoma, salivary gland carcinoma, kidney cancer, vulval cancer, thyroid cancer, hepatic carcinoma, skin cancer, melanoma, ovarian cancer, neuroblastoma, myeloma, various types of head and neck cancer, acute lymphoblastic leukemia, acute myeloid leukemia, Ewing sarcoma and peripheral neuroepithelioma, and other carcinomas, lymphomas, blastomas, sarcomas, and leukemias.

Breast cancers arise most commonly in the lining of the milk ducts of the breast (ductal carcinoma), or in the lobules where breast milk is produced (lobular carcinoma). Other forms of breast cancer include Inflammatory Breast Cancer and Recurrent Breast Cancer. Inflammatory Breast Cancer is a rare, but very serious, aggressive type of breast cancer. The breast may look red and feel warm with ridges, welts, or hives on the breast; or the skin may look wrinkled. It is sometimes misdiagnosed as a simple infection. Recurrent disease means that the cancer has come back after it has been treated. It may come back in the breast, in the soft tissues of the chest (the chest wall), or in another part of the body. As used herein, the term "breast cancer" may include both Inflammatory Breast Cancer and Recurrent Breast Cancer.

In an effort to detect breast cancer as early as possible, regular physical exams and screening mammograms often are prescribed and conducted. A diagnostic mammogram often is performed to evaluate a breast complaint or abnormality detected by physical exam or routine screening mammography. If an abnormality seen with diagnostic mammography is suspicious, additional breast imaging (with exams such as ultrasound) or a biopsy may be ordered. A biopsy followed by pathological (microscopic) analysis is a definitive way to determine whether a subject has breast cancer. Excised breast cancer samples often are subjected to the following analyses: diagnosis of the breast tumor and confirmation of its malignancy; maximum tumor thickness; assessment of completeness of excision of invasive and *in situ* components and microscopic measurements of the shortest extent of clearance; level of invasion; presence and extent of regression; presence and extent of ulceration; histological type and special variants; pre-existing lesion; mitotic rate; vascular invasion; neurotropism; cell type; tumor lymphocyte infiltration; and growth phase.

The stage of a breast cancer can be classified as a range of stages from Stage 0 to Stage IV based on its size and the extent to which it has spread. The following table summarizes the stages:

**Table A**

| Stage | Tumor Size | Lymph Node Involvement | Metastasis (Spread) |
|---|---|---|---|
| I | Less than 2 cm | No | No |
| II | Between 2-5 cm | No or in same side of breast | No |

(continued)

| Stage | Tumor Size | Lymph Node Involvement | Metastasis (Spread) |
|---|---|---|---|
| III | More than 5 cm | Yes, on same side of breast | No |
| IV | Not applicable | Not applicable | Yes |

Stage 0 cancer is a contained cancer that has not spread beyond the breast ductal system. Fifteen to twenty percent of breast cancers detected by clinical examinations or testing are in Stage 0 (the earliest form of breast cancer). Two types of Stage 0 cancer are lobular carcinoma in situ (LCIS) and ductal carcinoma in situ (DCIS). LCIS indicates high risk for breast cancer. Many physicians do not classify LCIS as a malignancy and often encounter LCIS by chance on breast biopsy while investigating another area of concern. While the microscopic features of LCIS are abnormal and are similar to malignancy, LCIS does not behave as a cancer (and therefore is not treated as a cancer). LCIS is merely a marker for a significantly increased risk of cancer anywhere in the breast. However, bilateral simple mastectomy may be occasionally performed if LCIS patients have a strong family history of breast cancer. In DCIS the cancer cells are confined to milk ducts in the breast and have not spread into the fatty breast tissue or to any other part of the body (such as the lymph nodes). DCIS may be detected on mammogram as tiny specks of calcium (known as microcalcifications) 80% of the time. Less commonly DCIS can present itself as a mass with calcifications (15% of the time); and even less likely as a mass without calcifications (<5% of the time). A breast biopsy is used to confirm DCIS. A standard DCIS treatment is breast-conserving therapy (BCT), which is lumpectomy followed by radiation treatment or mastectomy. To date, DCIS patients have chosen equally among lumpectomy and mastectomy as their treatment option, though specific cases may sometimes favor lumpectomy over mastectomy or vice versa.

In Stage I, the primary (original) cancer is 2 cm or less in diameter and has not spread to the lymph nodes. In Stage IIA, the primary tumor is between 2 and 5 cm in diameter and has not spread to the lymph nodes. In Stage IIB, the primary tumor is between 2 and 5 cm in diameter and has spread to the axillary (underarm) lymph nodes; or the primary tumor is over 5 cm and has not spread to the lymph nodes. In Stage IIIA, the primary breast cancer of any kind that has spread to the axillary (underarm) lymph nodes and to axillary tissues. In Stage IIIB, the primary breast cancer is any size, has attached itself to the chest wall, and has spread to the pectoral (chest) lymph nodes. In Stage IV, the primary cancer has spread out of the breast to other parts of the body (such as bone, lung, liver, brain). The treatment of Stage IV breast cancer focuses on extending survival time and relieving symptoms.

Based in part upon selection criteria set forth above, individuals having breast cancer can be selected for genetic studies. Also, individuals having no history of cancer or breast cancer often are selected for genetic studies. Other selection criteria can include: a tissue or fluid sample is derived from an individual characterized as Caucasian; the sample was derived from an individual of German paternal and maternal descent; the database included relevant phenotype information for the individual; case samples were derived from individuals diagnosed with breast cancer; control samples were derived from individuals free of cancer and no family history of breast cancer, and sufficient genomic DNA was extracted from each blood sample for all allelotyping and genotyping reactions performed during the study. Phenotype information included pre- or post-menopausal, familial predisposition, country or origin of mother and father, diagnosis with breast cancer (date of primary diagnosis, age of individual as of primary diagnosis, grade or stage of development, occurrence of metastases, *e.g.*, lymph node metastases, organ metastases), condition of body tissue (skin tissue, breast tissue, ovary tissue, peritoneum tissue and myometrium), method of treatment (surgery, chemotherapy, hormone therapy, radiation therapy).

Provided herein is a set of blood samples and a set of corresponding nucleic acid samples isolated from the blood samples, where the blood samples are donated from individuals diagnosed with breast cancer. The sample set often includes blood samples or nucleic acid samples from 100 or more, 150 or more, or 200 or more individuals having breast cancer, and sometimes from 250 or more, 300 or more, 400 or more, or 500 or more individuals. The individuals can have parents from any place of origin, and in an embodiment, the set of samples are extracted from individuals of German paternal and German maternal ancestry. The samples in each set may be selected based upon five or more criteria and/or phenotypes set forth above.

## Polymorphic Variants Associated with Breast Cancer

A genetic analysis provided herein linked breast cancer with polymorphic variants in the *ICAM, MAPK10, KIAA0861, NUMA1* and *GALE* regions of the human genome disclosed herein. As used herein, the term "polymorphic site" refers to a region in a nucleic acid at which two or more alternative nucleotide sequences are observed in a significant number of nucleic acid samples from a population of individuals. A polymorphic site may be a nucleotide sequence of two or more nucleotides, an inserted nucleotide or nucleotide sequence, a deleted nucleotide or nucleotide sequence, or a microsatellite, for example. A polymorphic site that is two or more nucleotides in length may be 3, 4, 5, 6, 7, 8, 9, 10, 11, 12, 13, 14, 15 or more, 20 or more, 30 or more, 50 or more, 75 or more, 100 or more, 500 or more, or about 1000 nucleotides in length, where all or some of the nucleotide sequences differ within the region. A polymorphic site is often one nucleotide in length, which is referred to herein as a "single nucleotide polymorphism" or a "SNP."

Where there are two, three, or four alternative nucleotide sequences at a polymorphic site, each nucleotide sequence is referred to as a "polymorphic variant" or "nucleic acid variant." Where two polymorphic variants exist, for example, the polymorphic variant represented in a minority of samples from a population is sometimes referred to as a "minor allele" and the polymorphic variant that is more prevalently represented is sometimes referred to as a "major allele." Many organisms possess a copy of each chromosome (*e.g.*, humans), and those individuals who possess two major alleles or two minor alleles are often referred to as being "homozygous" with respect to the polymorphism, and those individuals who possess one major allele and one minor allele are normally referred to as being "heterozygous" with respect to the polymorphism. Individuals who are homozygous with respect to one allele are sometimes predisposed to a different phenotype as compared to individuals who are heterozygous or homozygous with respect to another allele.

Furthermore, a genotype or polymorphic variant may be expressed in terms of a "haplotype," which as used herein refers to two or more polymorphic variants occurring within genomic DNA in a group of individuals within a population. For example, two SNPs may exist within a gene where each SNP position includes a cytosine variation and an adenine variation. Certain individuals in a population may carry one allele (heterozygous) or two alleles (homozygous) having the gene with a cytosine at each SNP position. As the two cytosines corresponding to each SNP in the gene travel together on one or both alleles in these individuals, the individuals can be characterized as having a cytosine/cytosine haplotype with respect to the two SNPs in the gene.

As used herein, the term "phenotype" refers to a trait which can be compared between individuals, such as presence or absence of a condition, a visually observable difference in appearance between individuals, metabolic variations, physiological variations, variations in the function of biological molecules, and the like. An example of a phenotype is occurrence of breast cancer.

Researchers sometimes report a polymorphic variant in a database without determining whether the variant is represented in a significant fraction of a population. Because a subset of these reported polymorphic variants are not represented in a statistically significant portion of the population, some of them are sequencing errors and/or not biologically relevant. Thus, it is often not known whether a reported polymorphic variant is statistically significant or biologically relevant until the presence of the variant is detected in a population of individuals and the frequency of the variant is determined. Methods for detecting a polymorphic variant in a population are described herein, specifically in Example 2. A polymorphic variant is statistically significant and often biologically relevant if it is represented in 5% or more of a population, sometimes 10% or more, 15% or more, or 20% or more of a population, and often 25% or more, 30% or more, 35% or more, 40% or more, 45% or more, or 50% or more of a population.

A polymorphic variant may be detected on either or both strands of a double-stranded nucleic acid. For example, a thymine at a particular position in SEQ ID NO: 1 can be reported as an adenine from the complementary strand. Also, a polymorphic variant may be located within an intron or exon of a gene or within a portion of a regulatory region such as a promoter, a 5' untranslated region (UTR), a 3' UTR, and in DNA (*e.g.*, genomic DNA (gDNA) and complementary DNA (cDNA)), RNA (*e.g.*, mRNA, tRNA, and rRNA), or a polypeptide. Polymorphic variations may or may not result in detectable differences in gene expression, polypeptide structure, or polypeptide function.

In the genetic analysis that associated breast cancer with the polymorphic variants described hereafter, samples from individuals having breast cancer and individuals not having cancer were allelotyped and genotyped. The term "genotyped" as used herein refers to a process for determining a genotype of one or more individuals, where a "genotype" is a representation of one or more polymorphic variants in a population. Genotypes may be expressed in terms of a "haplotype," which as used herein refers to two or more polymorphic variants occurring within genomic DNA in a group of individuals within a population. For example, two SNPs may exist within a gene where each SNP position includes a cytosine variation and an adenine variation. Certain individuals in a population may carry one allele (heterozygous) or two alleles (homozygous) having the gene with a cytosine at each SNP position. As the two cytosines corresponding to each SNP in the gene travel together on one or both alleles in these individuals, the individuals can be characterized as having a cytosine/cytosine haplotype with respect to the two SNPs in the gene.

It was determined that polymorphic variations associated with an increased risk of breast cancer existed in *ICAM, MAPK10, KIAA0861, NUMA1* or *GALE* nucleotide sequences. Polymorphic variants in and around the *ICAM, MAPK10, KIAA0861, NUMA1* and *GALE* loci were tested for association with breast cancer. In the *ICAM* locus, these included polymorphic variants at positions in SEQ ID NO: 1 selected from the group consisting of 139, 11799, 11851, 11851, 11963, 24282, 26849, 29633, 31254, 31967, 32920, 33929, 35599, 36101, 36101, 36340, 36405, 36517, 36777, 36992, 37645, 37868, 38440, 38440, 38532, 38532, 38547, 38547, 38712, 40684, 40860, 41213, 41419, 41613, 42407, 43440, 43440, 44247, 44247, 44247, 44247, 44677, 44677, 45256, 45256, 45536, 45536, 46153, 47546, 47697, 47944, 47944, 48530, 51102, 57090, 60093, 60439, 62694, 66260, 67295, 67295, 67304, 67731, 67731, 68555, 68555, 70429, 70875, 72360, 74228, 76802, 77664, 78803, 79263, 80810, 81020, 82426, 82783, 85912, 85912, 86135, 86135, 87877, 87877, 88043, 88043, 88206, 88343, 90701, 90701, 90974, 91060, 91087, 91594, 91594, 92302, 92384, 36517, and 44677. Polymorphic variants in a region spanning positions 11851-24282, 36340-37868, 41213-41613, 70875-74228, 42407-45536, and 42407-51102 in SEQ ID NO: 1 in particular were associated with an increased risk of breast cancer, including polymorphic variants at positions 11963, 36340, 36992, 37868, 41213, 41419, 41613, 42407, 44247, 44677, 45256, 45536, 51102, 72360, 36517, and 44677 in SEQ ID NO: 1. At these positions in SEQ ID NO: 1, an adenine at position 11963, a guanine at position 36340, an adenine at position 36992, a guanine at position 37868, a cytosine at position 41213, a guanine at position 41419, a guanine at position 41613, a cytosine at position 42407, a cytosine at position 44247, an adenine or cytosine at position 44677, a thymine at position 45256, a guanine at position 45536, a cytosine at position 51102, a guanine at position 72360, a cytosine at position 36517, and guanine at position 44677, in particular were associated with risk of breast cancer. Also, a proline at amino acid position 352 or an alanine at amino acid position 348 in SEQ ID NO: 15 were in particular associated with an increased risk of breast cancer.

In the *MAPK10* locus, these included polymorphic variants at positions in SEQ ID NO: 2 selected from the group consisting of 191, 1490, 3781, 3935, 4512, 7573, 8467, 9001, 9732, 13477, 13787, 13903, 14355, 15053, 15459, 17762, 19482, 19631, 22170, 22688, 22748, 23376, 23826, 23868, 24154, 25972, 26057, 26361, 26599, 26712, 26812, 27069, 32421, 33557, 35127, 35222, 35999, 36424, 37403, 39203, 39226, 41147, 46176, 50452, 52919, 60214, 61093, 62572, 63601, 65362, 65863, 66207, 66339, 69512, 70759, 71217, 73382, and 76307. Polymorphic variants in a region spanning positions 23826-36424, 46176-62572, 4512-8467 or 13787-14355 in SEQ ID NO: 2 in particular were associated with an increased risk of breast cancer, including polymorphic variants at positions 7573, 13903, 23826, 26057, 26361, 26599, 26812, 27069, 35127, 35222, 36424, 46176, 50452, 61093, 62572, and 70759 in SEQ ID NO: 2. At these positions in SEQ ID NO: 2, a guanine at position 7573, a cytosine at position 13903, an adenine at position 23826, an adenine at position 26057, a thymine at position 26361, an adenine at position 26599, an adenine at position 26812, a cytosine at position 27069, an adenine at position 35127, a thymine at position 35222, a cytosine at position 36424, a cytosine at position 46176, a cytosine at position 50452, a guanine at position 61093, an adenine at position 62572, and a guanine at position 70759, in particular were associated with risk of breast cancer.

In the *KIAA0861* locus, these included polymorphic variants at positions in SEQ ID NO: 3 selected from the group consisting of 107, 2157, 7300, 8233, 9647, 9868, 9889, 10621, 11003, 11507, 11527, 11718, 11808, 12024, 13963, 14300, 14361, 16287, 18635, 19365, 24953, 25435, 26847, 27492, 27620, 27678, 27714, 29719, 30234, 31909, 32153, 33572, 42164, 43925, 45031, 45655, 48350, 48418, 48563, 53189,56468, 59358, 63761, 65931, 67040, 69491, 83308,126545, 137592, and 147169. Polymorphic variants in a region spanning positions 42164-48563 in SEQ ID NO: 3 in particular were associated with an increased risk of breast cancer, including polymorphic variants at positions 107, 42164, 45031, 45655, 48563, 19365 and 14361 in SEQ ID NO: 3. At these positions in SEQ ID NO: 3, an adenine at position 107, a thymine at position 14361, a guanine at position 19365, a thymine at position 42164, a cytosine at position 45031, a thymine at position 45655 and a cytosine at position 48563, in particular were associated with risk of breast cancer. Also, leucine at amino acid position 359 in SEQ ID NO: 17, a leucine at amino acid position 378 in SEQ ID NO: 17, or an alanine at amino acid position 857 in SEQ ID NO: 17 were in particular associated with an increased risk of breast cancer.

In the *NUMA1* locus, these included polymorphic variants at positions in SEQ ID NO: 4 selected from the group consisting of 174, 815, 3480, 9715, 14755, 15912, 19834, 19850, 20171, 20500, 20536, 23187, 25289, 25470, 28720, 29566, 30155, 30752, 32710, 32954, 33725, 33842, 36345, 38115, 39150, 40840, 41969, 42045, 43785, 44444, 44579, 45386, 46827, 47320, 47625, 47837, 47866, 49002, 49566, 52058, 52249, 52257, 52850, 53860, 54052, 54411, 55098, 55303, 59398, 59533, 60542, 61541, 62309, 72299, 73031, 73803, 80950, 82137, 96077, 96470, 98116, 98184, and 132952. Polymorphic variants in a region spanning positions 174-32954, 38115-43785, 45386-52058, 52257-54411, 55303-73803 or 96470-98184 in SEQ ID NO: 4 in particular were associated with an increased risk of breast cancer, including polymorphic variants at positions 174, 815, 3480, 19834, 19850, 20171, 20500, 20536, 23187, 25470, 30155, 30752, 32710, 32954, 38115, 39150, 40840, 41969, 42045, 43785, 45386, 46827, 47320, 47625, 47837, 47866, 49002, 49566, 52058, 52257, 52850, 53860, 54052, 54411, 55303, 59398, 60542, 62309, 72299, 73031, 73803, and 98116 in SEQ ID NO: 4. At these positions in SEQ ID NO: 4, a thymine at position 174, an adenine at position 815, a cytosine at position 3480, a guanine at position 19834, an adenine at position 19850, a thymine at position 20171, a thymine at position 20500, a cytosine at position 20536, a cytosine at position 23187, a thymine at position 25470, a thymine at position 30155, a guanine at position 30752, a thymine at position 32710, a guanine at position 32954, an adenine at position 38115, a cytosine at position 39150, a thymine at position 40840, an adenine at position 41969, a thymine at position 42045, a guanine at position 43785, a cytosine at position 45386, an adenine at position 46827, an adenine at position 47320, a cytosine at position 47625, a cytosine at position 47837, an adenine at position 47866, a cytosine at position 49002, a thymine at position 49566, a cytosine at position 52058, a thymine at position 52257, a thymine at position 52850, a cytosine at position 53860, a cytosine at position 54052, a thymine at position 54411, a cytosine at position 55303, an adenine at position 59398, an adenine at position 60542, an adenine at position 62309, a cytosine at position 72299, a thymine at position 73031, a guanine at position 73803, and a thymine at position 98116, in particular were associated with risk of breast cancer. In the *GALE* locus, a polymorphic variant at position 174 in SEQ ID NO: 5 was in particular associated with increased risk of breast cancer, and an adenine this position was the cancer-associated allele.

Additional Polymorphic Variants Associated with Breast Cancer

Also provided is a method for identifying polymorphic variants proximal to an incident, founder polymorphic variant associated with breast cancer. Thus, featured herein are methods for identifying a polymorphic variation associated with breast cancer that is proximal to an incident polymorphic variation associated with breast cancer, which comprises identifying a polymorphic variant proximal to the incident polymorphic variant associated with breast cancer, where the incident polymorphic variant is in a nucleotide sequence set forth in SEQ ID NO: 1-5. The nucleotide sequence often comprises a polynucleotide sequence selected from the group consisting of (a) a nucleotide sequence set forth in SEQ ID NO: 1-5; (b) a nucleotide sequence which encodes a polypeptide having an amino acid sequence encoded by a nucleotide sequence in SEQ ID NO: 1-5; (c) a nucleotide sequence which encodes a polypeptide that is 90% or more identical to an amino acid sequence encoded by a nucleotide sequence in SEQ ID NO: 1-5 or a nucleotide sequence about 90% or more identical to the nucleotide sequence set forth in SEQ ID NO: 1-5; and (d) a fragment of a nucleotide sequence of (a), (b), or (c), often a fragment that includes a polymorphic site associated with breast cancer. The presence or absence of an association of the proximal polymorphic variant with breast cancer then is determined using a known association method, such as a method described in the Examples hereafter. In an embodiment, the incident polymorphic variant is described in SEQ ID NO: 1-5. In another embodiment, the proximal polymorphic variant identified sometimes is a publicly disclosed polymorphic variant, which for example, sometimes is published in a publicly available database. In other embodiments, the polymorphic variant identified is not publicly disclosed and is discovered using a known method, including; but not limited to, sequencing a region surrounding the incident polymorphic variant in a group of nucleic acid samples. Thus, multiple polymorphic variants proximal to an incident polymorphic variant are associated with breast cancer using this method.

The proximal polymorphic variant often is identified in a region surrounding the incident polymorphic variant. In certain embodiments, this surrounding region is about 50 kb flanking the first polymorphic variant (*e.g*. about 50 kb 5' of the first polymorphic variant and about 50 kb 3' of the first polymorphic variant), and the region sometimes is composed of shorter flanking sequences, such as flanking sequences of about 40 kb, about 30 kb, about 25 kb, about 20 kb, about 15 kb, about 10 kb, about 7 kb, about 5 kb, or about 2 kb 5' and 3' of the incident polymorphic variant. In other embodiments, the region is composed of longer flanking sequences, such as flanking sequences of about 55 kb, about 60 kb, about 65 kb, about 70 kb, about 75 kb, about 80 kb, about 85 kb, about 90 kb, about 95 kb, or about 100 kb 5' and 3' of the incident polymorphic variant.

In certain embodiments, polymorphic variants associated with breast cancer are identified iteratively. For example, a first proximal polymorphic variant is associated with breast cancer using the methods described above and then another polymorphic variant proximal to the first proximal polymorphic variant is identified (*e.g*., publicly disclosed or discovered) and the presence or absence of an association of one or more other polymorphic variants proximal to the first proximal polymorphic variant with breast cancer is determined.

The methods described herein are useful for identifying or discovering additional polymorphic variants that may be used to further characterize a gene, region or loci associated with a condition, a disease (*e.g*., breast cancer), or a disorder. For example, allelotyping or genotyping data from the additional polymorphic variants may be used to identify a functional mutation or a region of linkage disequilibrium.

In certain embodiments, polymorphic variants identified or discovered within a region comprising the first polymorphic variant associated with breast cancer are genotyped using the genetic methods and sample selection techniques described herein, and it can be determined whether those polymorphic variants are in linkage disequilibrium with the first polymorphic variant. The size of the region in linkage disequilibrium with the first polymorphic variant also can be assessed using these genotyping methods. Thus, provided herein are methods for determining whether a polymorphic variant is in linkage disequilibrium with a first polymorphic variant associated with breast cancer, and such information can be used in prognosis methods described herein.

Isolated *ICAM, MAPK10, KIAA0861, NUMA1 or GALE* Nucleic Acids

Featured herein are isolated *ICAM, MAPK10, KIAA0861, NUMA1* or *GALE* nucleic acids, which include the nucleic acid having the nucleotide sequence of SEQ ID NO: 1, 2, 3, 4, 5, 6, 7, 8, 9, 10 or 11, nucleic acid variants, and substantially identical nucleic acids of the foregoing. Nucleotide sequences of the *ICAM, MAPK10, KIAA0861, NUMA1* or *GALE* nucleic acids sometimes are referred to herein as "*ICAM, MAPK10, KIAA0861, NUMA1* or *GALE* nucleotide sequences." A "*ICAM, MAPK10, KIAA0861, NUMA1* or *GALE* nucleic acid variant" refers to one allele that may have one or more different polymorphic variations as compared to another allele in another subject or the same subject. A polymorphic variation in the *ICAM, MAPK10, KIAA0861, NUMA1* or *GALE* nucleic acid variant may be represented on one or both strands in a double-stranded nucleic acid or on one chromosomal complement (heterozygous) or both chromosomal complements (homozygous).

As used herein, the term "nucleic acid" includes DNA molecules (e.g., a complementary DNA (cDNA) and genomic DNA (gDNA)) and RNA molecules (e.g., mRNA, rRNA, and tRNA) and analogs of DNA or RNA, for example, by use of nucleotide analogs. The nucleic acid molecule can be single-stranded and it is often double-stranded. The term "isolated or purified nucleic acid" refers to nucleic acids that are separated from other nucleic acids present in the natural source of the nucleic acid. For example, with regard to genomic DNA, the term "isolated" includes nucleic acids which are separated from the chromosome with which the genomic DNA is naturally associated. An "isolated" nucleic acid is often free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. As used herein, the term "*ICAM, MAPK10, KIAA0861, NUMA1* or *GALE* gene" refers to a nucleotide sequence that encodes a *ICAM, MAPK10, KIAA0861, NUMA1* or *GALE* polypeptide.

Also included herein are nucleic acid fragments. These fragments typically are a nucleotide sequence identical to a nucleotide sequence in SEQ ID NO: 1-12, a nucleotide sequence substantially identical to a nucleotide sequence in SEQ ID NO: 1-12, or a nucleotide sequence that is complementary to the foregoing. The nucleic acid fragment may be identical, substantially identical or homologous to a nucleotide sequence in an exon or an intron in SEQ ID NO: 1-5, and may encode a domain or part of a domain or motif of a *ICAM, MAPK10, KIAA0861, NUMA1* or *GALE* polypeptide. Sometimes, the fragment will comprises the polymorphic variation described herein as being associated with breast cancer. The nucleic acid fragment sometimes is 50, 100, or 200 or fewer base pairs in length, and is sometimes about 300, 400, 500, 600, 700, 800, 900, 1000, 1100, 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3800, 4000, 5000, 6000, 7000, 8000, 9000, 10000, 15000, 20000, 30000, 40000, 50000, 60000, 70000, 80000, 90000, 100000, 110000, 120000, 130000, 140000, 150000 or 160000 base pairs in length. A nucleic acid fragment complementary to a nucleotide sequence identical or substantially identical to the nucleotide sequence of SEQ ID NO: 1-12 and hybridizes to such a nucleotide sequence under stringent conditions often is referred to as a "probe." Nucleic acid fragments often include one or more polymorphic sites, or sometimes have an end that is adjacent to a polymorphic site as described hereafter.

An example of a nucleic acid fragment is an oligonucleotide. As used herein, the term "oligonucleotide" refers to a nucleic acid comprising about 8 to about 50 covalently linked nucleotides, often comprising from about 8 to about 35 nucleotides, and more often from about 10 to about 25 nucleotides. The backbone and nucleotides within an oligonucleotide may be the same as those of naturally occurring nucleic acids, or analogs or derivatives of naturally occurring nucleic acids, provided that oligonucleotides having such analogs or derivatives retain the ability to hybridize specifically to a nucleic acid comprising a targeted polymorphism. Oligonucleotides described herein may be used as hybridization probes or as components of prognostic or diagnostic assays, for example, as described herein.

Oligonucleotides are typically synthesized using standard methods and equipment, such as the ABI 3900 High Throughput DNA Synthesizer and the EXPEDITE™ 8909 Nucleic Acid Synthesizer, both of which are available from Applied Biosystems (Foster City, CA). Analogs and derivatives are exemplified in U.S. Pat. Nos. 4,469,863; 5,536,821; 5,541,306; 5,637,683; 5,637,684; 5,700,922; 5,717,083; 5,719,262; 5,739,308; 5,773,601; 5,886,165; 5,929,226; 5,977,296; 6,140,482; WO 00/56746; WO 01/14398, and related publications. Methods for synthesizing oligonucleotides comprising such analogs or derivatives are disclosed, for example, in the patent publications cited above and in U.S. Pat. Nos. 5,614,622; 5,739,314; 5,955,599; 5,962,674; 6,117,992; in WO 00/75372; and in related publications.

Oligonucleotides also may be linked to a second moiety. The second moiety may be an additional nucleotide sequence such as a tail sequence (e.g., a polyadenosine tail), an adapter sequence (e.g., phage M13 universal tail sequence), and others. Alternatively, the second moiety may be a non-nucleotide moiety such as a moiety which facilitates linkage to a solid support or a label to facilitate detection of the oligonucleotide. Such labels include, without limitation, a radioactive label, a fluorescent label, a chemiluminescent label, a paramagnetic label, and the like. The second moiety may be attached to any position of the oligonucleotide, provided the oligonucleotide can hybridize to the nucleic acid comprising the polymorphism.

## Uses for Nucleic Acid Sequences

Nucleic acid coding sequences depicted in SEQ ID NO: 1-12 may be used for diagnostic purposes for detection and control of polypeptide expression. Also, included herein are oligonucleotide sequences such as antisense RNA, small-interfering RNA (siRNA) and DNA molecules and ribozymes that function to inhibit translation of a polypeptide. Antisense techniques and RNA interference techniques are known in the art and are described herein.

Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Ribozymes may be engineered hammerhead motif ribozyme molecules that specifically and efficiently catalyze endonucleolytic cleavage of RNA sequences corresponding to or complementary to the nucleotide sequences set forth in SEQ ID NO: 1-12. Specific ribozyme cleavage sites within any potential RNA target are initially identified by scanning the target molecule for ribozyme cleavage sites which include the following sequences, GUA, GUU and GUC. Once identified, short RNA sequences of between fifteen (15) and twenty (20) ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridization with complementary oligonucleotides, using ribonuclease protection assays.

Antisense RNA and DNA molecules, siRNA and ribozymes may be prepared by any method known in the art for the synthesis of RNA molecules. These include techniques for chemically synthesizing oligodeoxyribonucleotides well known in the art such as solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

DNA encoding a polypeptide also may have a number of uses for the diagnosis of diseases, including breast cancer, resulting from aberrant expression of a target gene described herein. For example, the nucleic acid sequence may be used in hybridization assays of biopsies or autopsies to diagnose abnormalities of expression or function (e.g., Southern or Northern blot analysis, in situ hybridization assays).

In addition, the expression of a polypeptide during embryonic development may also be determined using nucleic acid encoding the polypeptide. As addressed, *infra*, production of functionally impaired polypeptide can be the cause of various disease states, such as breast cancer. *In situ* hybridizations using polynucleotide probes may be employed to predict problems related to breast cancer. Further, as indicated, *infra*, administration of human active polypeptide, recombinantly produced as described herein, may be used to treat disease states related to functionally impaired polypeptide. Alternatively, gene therapy approaches may be employed to remedy deficiencies of functional polypeptide or to replace or compete with dysfunctional polypeptide.

<u>Expression Vectors, Host Cells, and Genetically Engineered Cells</u>

Provided herein are nucleic acid vectors, often expression vectors, which contain a *ICAM, MAPK10, KIAA0861, NUMA1* or *GALE* nucleic acid. As used herein, the term "vector" refers to a nucleic acid molecule capable of transporting another nucleic acid to which it has been linked and can include a plasmid, cosmid, or viral vector. The vector can be capable of autonomous replication or it can integrate into a host DNA. Viral vectors may include replication defective retroviruses, adenoviruses and adeno-associated viruses for example.

A vector can include a *ICAM, MAPK10, KIAA0861, NUMA1* or *GALE* nucleic acid in a form suitable for expression of the nucleic acid in a host cell. The recombinant expression vector typically includes one or more regulatory sequences operatively linked to the nucleic acid sequence to be expressed. The term "regulatory sequence" includes promoters, enhancers and other expression control elements (e.g., polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of polypeptide desired, and the like. Expression vectors can be introduced into host cells to produce *ICAM, MAPK10, KIAA0861, NUMA1* or *GALE* polypeptides, including fusion polypeptides, encoded by *ICAM, MAPK10, KIAA0861, NUMA1* or *GALE* nucleic acids.

Recombinant expression vectors can be designed for expression of *ICAM, MAPK10, KIAA0861, NUMA1* or *GALE* polypeptides in prokaryotic or eukaryotic cells. For example, *ICAM, MAPK10, KIAA0861, NUMA1* or *GALE* polypeptides can be expressed in E. coli, insect cells (e.g., using baculovirus expression vectors), yeast cells, or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated in vitro, for example using T7 promoter regulatory sequences and T7 polymerase.

Expression of polypeptides in prokaryotes is most often carried out in E. coli with vectors containing constitutive or inducible promoters directing the expression of either fusion or non-fusion polypeptides. Fusion vectors add a number of amino acids to a polypeptide encoded therein, usually to the amino terminus of the recombinant polypeptide. Such fusion vectors typically serve three purposes: 1) to increase expression of recombinant polypeptide; 2) to increase the solubility of the recombinant polypeptide; and 3) to aid in the purification of the recombinant polypeptide by acting as a ligand in affinity purification. Often, a proteolytic cleavage site is introduced at the junction of the fusion moiety and the recombinant polypeptide to enable separation of the recombinant polypeptide from the fusion moiety subsequent to purification of the fusion polypeptide. Such enzymes, and their cognate recognition sequences, include Factor Xa, thrombin and enterokinase. Typical fusion expression vectors include pGEX (Pharmacia Biotech Inc; Smith & Johnson, Gene 67: 31-40 (1988)), pMAL (New England Biolabs, Beverly, MA) and pRIT5 (Pharmacia, Piscataway, NJ) which fuse glutathione S-transferase (GST), maltose E binding polypeptide, or polypeptide A, respectively, to the target recombinant polypeptide.

Purified fusion polypeptides can be used in screening assays and to generate antibodies specific for *ICAM, MAPK10, KIAA0861, NUMA1* or *GALE* polypeptides. In a therapeutic embodiment, fusion polypeptide expressed in a retroviral expression vector is used to infect bone marrow cells that are subsequently transplanted into irradiated recipients. The pathology of the subject recipient is then examined after sufficient time has passed (e.g., six (6) weeks).

Expressing the polypeptide in host bacteria with an impaired capacity to proteolytically cleave the recombinant polypeptide is often used to maximize recombinant polypeptide expression (Gottesman, S., Gene Expression Technology: Methods in Enzymology, Academic Press, San Diego, California 185: 119-128 (1990)). Another strategy is to alter the nucleotide sequence of the nucleic acid to be inserted into an expression vector so that the individual codons for each amino acid are those preferentially utilized in E. coli (Wada et al., Nucleic Acids Res. 20: 2111-2118 (1992)). Such alteration of nucleotide sequences can be carried out by standard DNA synthesis techniques.

When used in mammalian cells, the expression vector's control functions are often provided by viral regulatory elements. For example, commonly used promoters are derived from polyoma, Adenovirus 2, cytomegalovirus and Simian Virus 40. Recombinant mammalian expression vectors are often capable of directing expression of the nucleic acid in a particular cell type (e.g., tissue-specific regulatory elements are used to express the nucleic acid). Non-limiting examples of suitable tissue-specific promoters include an albumin promoter (liver-specific; Pinkert et al., Genes Dev. 1: 268-277 (1987)), lymphoid-specific promoters (Calame & Eaton, Adv. Immunol. 43: 235-275 (1988)), promoters of T cell receptors (Winoto & Baltimore, EMBO J. 8: 729-733 (1989)) promoters of immunoglobulins (Banerji et al., Cell 33: 729-740 (1983); Queen & Baltimore, Cell 33: 741-748 (1983)), neuron-specific promoters (e.g., the neurofilament promoter; Byme & Ruddle, Proc. Natl. Acad. Sci. USA 86: 5473-5477 (1989)), pancreas-specific promoters (Edlund et al., Science 230: 912-916 (1985)), and mammary gland-specific promoters (e.g., milk whey promoter; U.S. Patent No. 4,873,316 and European Application Publication No. 264,166). Developmentally-regulated promoters are sometimes utilized, for example, the murine hox promoters (Kessel & Gruss, Science 249: 374-379 (1990)) and the a-fetopolypeptide promoter (Campes & Tilghman, Genes Dev. 3: 537-546 (1989)).

A *ICAM, MAPK10, KIAA0861, NUMA1* or *GALE* nucleic acid may also be cloned into an expression vector in an antisense orientation. Regulatory sequences (e.g., viral promoters and/or enhancers) operatively linked to a *ICAM, MAPK10, KIAA0861, NUMA1* or *GALE* nucleic acid cloned in the antisense orientation can be chosen for directing constitutive, tissue specific or cell type specific expression of antisense RNA in a variety of cell types. Antisense expression vectors can be in the form of a recombinant plasmid, phagemid or attenuated virus. For a discussion of the regulation of gene expression using antisense genes see Weintraub et al., Antisense RNA as a molecular tool for genetic analysis, Reviews - Trends in Genetics, Vol. 1(1) (1986).

Also provided herein are host cells that include a *ICAM, MAPK10, KIAA0861, NUMA1* or *GALE* nucleic acid within a recombinant expression vector or *ICAM, MAPK10, KIAA0861, NUMA1* or *GALE* nucleic acid sequence fragments which allow it to homologously recombine into a specific site of the host cell genome. The terms "host cell" and "recombinant host cell" are used interchangeably herein. Such terms refer not only to the particular subject cell but rather also to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic or eukaryotic cell. For example, a *ICAM, MAPK10, KIAA0861, NUMA1* or *GALE* polypeptide can be expressed in bacterial cells such as E. coli, insect cells, yeast or mammalian cells (such as Chinese hamster ovary cells (CHO) or COS cells). Other suitable host cells are known to those skilled in the art.

Vectors can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, transduction/infection, DEAE-dextran-mediated transfection, lipofection, or electroporation.

A host cell provided herein can be used to produce (i.e., express) a *ICAM, MAPK10, KIAA0861, NUMA1* or *GALE* polypeptide. Accordingly, further provided are methods for producing a *ICAM, MAPK10, KIAA0861, NUMA1* or *GALE* polypeptide using the host cells described herein. In one embodiment, the method includes culturing host cells into which a recombinant expression vector encoding a *ICAM, MAPK10, KIAA0861, NUMA1* or *GALE* polypeptide has been introduced in a suitable medium such that a *ICAM, MAPK10, KIAA0861, NUMA1* or *GALE* polypeptide is produced. In another embodiment, the method further includes isolating a *ICAM, MAPK10, KIAA0861, NUMA1* or *GALE* polypeptide from the medium or the host cell.

Also provided are cells or purified preparations of cells which include a *ICAM, MAPK10, KIAA0861, NUMA1* or *GALE* transgene, or which otherwise misexpress *ICAM, MAPK10, KIAA0861, NUMA1* or *GALE* polypeptide. Cell preparations can consist of human or non-human cells, e.g., rodent cells, e.g., mouse or rat cells, rabbit cells, or pig cells. In certain embodiments, the cell or cells include a *ICAM, MAPK10, KIAA0861, NUMA1* or *GALE* trans gene (e.g., a heterologous form of a *ICAM, MAPK10, KIAA0861, NUMA1* or *GALE* such as a human gene expressed in non-human cells). The *ICAM, MAPK10, KIAA0861, NUMA1* or *GALE* trans gene can be misexpressed, e.g., overexpressed or underexpressed. In other embodiments, the cell or cells include a gene which misexpress an endogenous *ICAM, MAPK10, KIAA0861, NUMA1* or *GALE* polypeptide (e.g., expression of a gene is disrupted, also known as a knockout). Such cells can serve as a model for studying disorders which are related to mutated or mis-expressed *ICAM, MAPK10, KIAA0861, NUMA1* or *GALE* alleles or for use in drug screening. Also provided are human cells (e.g., a hematopoietic stem cells) transformed with a *ICAM, MAPK10, KIAA0861, NUMA1* or *GALE* nucleic acid.

Also provided are cells or a purified preparation thereof (e.g., human cells) in which an endogenous *ICAM, MAPK10, KIAA0861, NUMA1* or *GALE* nucleic acid is under the control of a regulatory sequence that does not normally control the expression of the endogenous *ICAM, MAPK10, KIAA0861, NUMA1* or *GALE* gene. The expression characteristics of an endogenous gene within a cell (e.g., a cell line or microorganism) can be modified by inserting a heterologous DNA regulatory element into the genome of the cell such that the inserted regulatory element is operably linked to the endogenous *ICAM, MAPK10, KIAA0861, NUMA1* or *GALE* gene. For example, an endogenous *ICAM, MAPK10, KIAA0861, NUMA1* or *GALE* gene (e.g., a gene which is "transcriptionally silent," not normally expressed, or expressed only at very low levels) may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published on May 16,1991.

Transgenic Animals

Non-human transgenic animals that express a heterologous *ICAM, MAPK10, KIAA0861, NUMA1* or *GALE* polypeptide (e.g., expressed from a *ICAM, MAPK10, KIAA0861, NUMA1* or *GALE* nucleic acid isolated from another organism) can be generated. Such animals are useful for studying the function and/or activity of a *ICAM, MAPK10, KIAA0861, NUMA1* or *GALE* polypeptide and for identifying and/or evaluating modulators of *ICAM, MAPK10, KIAA0861, NUMA1* or *GALE* nucleic acid and *ICAM, MAPK10, KIAA0861, NUMA1* or *GALE* polypeptide activity. As used herein, a "transgenic animal" is a non-human animal such as a mammal (e.g., a non-human primate such as chimpanzee, baboon, or macaque; an ungulate such as an equine, bovine, or caprine; or a rodent such as a rat, a mouse, or an Israeli sand rat), a bird (e.g., a chicken or a turkey), an amphibian (e.g., a frog, salamander, or newt), or an insect (e.g., Drosophila melanogaster), in which one or more of the cells of the animal includes a *ICAM, MAPK10, KIAA0861, NUMA1* or *GALE* transgene. A transgene is exogenous DNA or a rearrangement (e.g., a deletion of endogenous chromosomal DNA) that is often integrated into or occurs in the genome of cells in a transgenic animal. A transgene can direct expression of an encoded gene product in one or more cell types or tissues of the transgenic animal, and other transgenes can reduce expression (e.g., a knockout). Thus, a transgenic animal can be one in which an endogenous *ICAM, MAPK10, KIAA0861, NUMA1* or *GALE* gene has been altered by homologous recombination between the endogenous gene and an exogenous DNA molecule introduced into a cell of the animal (e.g., an embryonic cell of the animal) prior to development of the animal.

Intronic sequences and polyadenylation signals can also be included in the transgene to increase expression efficiency of the transgene. One or more tissue-specific regulatory sequences can be operably linked to a *ICAM, MAPK10, KIAA0861, NUMA1* or *GALE* trans gene to direct expression of a *ICAM, MAPK10, KIAA0861, NUMA1* or *GALE* polypeptide to particular cells. A transgenic founder animal can be identified based upon the presence of a *ICAM, MAPK10, KIAA0861, NUMA1* or *GALE* transgene in its genome and/or expression of *ICAM, MAPK10, KIAA0861, NUMA1* or *GALE* mRNA in tissues or cells of the animals. A transgenic founder animal can then be used to breed additional animals carrying the transgene. Moreover, transgenic animals carrying a transgene encoding a *ICAM, MAPK10, KIAA0861, NUMA1* or *GALE* polypeptide can further be bred to other transgenic animals carrying other transgenes.

*ICAM, MAPK10, KIAA0861, NUMA1* or *GALE* polypeptides can be expressed in transgenic animals or plants by introducing, for example, a nucleic acid encoding the polypeptide into the genome of an animal. In certain embodiments the nucleic acid is placed under the control of a tissue specific promoter, e.g., a milk or egg specific promoter, and recovered from the milk or eggs produced by the animal. Also included is a population of cells from a transgenic animal.

*ICAM, MAPK10, KIAA0861, NUMA1* and *GALE* Polypeptides

Featured herein are isolated *ICAM, MAPK10, KIAA0861, NUMA1* or *GALE* polypeptides, which include polypeptides having amino acid sequences set forth in SEQ ID NO: 13-18, and substantially identical polypeptides thereof. Such polypeptides sometimes are proteins or peptides. A *ICAM, MAPK10, KIAA0861, NUMA1* or *GALE* polypeptide is a polypeptide encoded by a *ICAM, MAPK10, KIAA0861, NUMA1* or *GALE* nucleic acid, where one nucleic acid can encode one or more different polypeptides. An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of a *ICAM, MAPK10, KIAA0861, NUMA1* or *GALE* polypeptide or *ICAM, MAPK10, KIAA0861, NUMA1* or *GALE* polypeptide variant having less than about 30%, 20%, 10% and sometimes 5% (by dry weight), of non-*ICAM, MAPK10, KIAA0861, NUMA1* or *GALE* polypeptide (also referred to herein as a "contaminating protein"), or of chemical precursors or non-*ICAM, MAPK10, KIAA0861, NUMA1* or *GALE* chemicals. When the *ICAM, MAPK10, KIAA0861, NUAM1* or *GALE* polypeptide or a biologically active portion thereof is recombinantly produced, it is also often substantially free of culture medium, specifically, where culture medium represents less than about 20%, sometimes less than about 10%, and often less than about 5% of the volume of the polypeptide preparation. Isolated or purified *ICAM, MAPK10, KIAA0861, NUMA1* or *GALE* polypeptide preparations are sometimes 0.01 milligrams or more or 0.1 milligrams or more, and often 1.0 milligrams or more and 10 milligrams or more in dry weight. In specific embodiments, a polypeptide comprises a leucine at amino acid position 359 in SEQ ID NO: 17, a leucine at amino acid position 378 in SEQ ID NO: 17, or an alanine at amino acid position 857 in SEQ ID NO: 17, or a ICAM5 polypeptide comprises a proline at amino acid position 352 or an alanine at amino acid position 348 in SEQ ID NO: 15.

In another aspect, featured herein are *ICAM, MAPK10, KIAA0861, NUMA1* or *GALE* polypeptides and biologically active or antigenic fragments thereof that are useful as reagents or targets in assays applicable to prevention, treatment or diagnosis of breast cancer. In another embodiment, provided herein are *ICAM, MAPK10, KIAA0861, NUMA1* or *GALE* polypeptides having a *ICAM, MAPK10, KIAA0861, NUMA1* or *GALE* activity or activities.

Further included herein are *ICAM, MAPK10, KIAA0861, NUMA1* or *GALE* polypeptide fragments. The polypeptide fragment may be a domain or part of a domain of a *ICAM, MAPK10, KIAA0861, NUMA1* or *GALE* polypeptide. The polypeptide fragment is often 50 or fewer, 100 or fewer, or 200 or fewer amino acids in length, and is sometimes 300, 400, 500, 600, 700, or 900 or fewer amino acids in length. In certain embodiments, the polypeptide fragment comprises, consists essentially of, or consists of, at least 6 consecutive amino acids and not more than 1211 consecutive amino acids of SEQ ID NO: 13-18, or the polypeptide fragment comprises, consists essentially of, or consists of, at least 6 consecutive amino acids and not more than 543 consecutive amino acids of SEQ ID NO: 13-18.

*ICAM, MAPK10, KUA0861, NUMA1* or *GALE* polypeptides described herein can be used as immunogens to produce anti-*ICAM, MAPK10, KIAA0861, NUMA1* or *GALE* antibodies in a subject, to purify *ICAM, MAPK10, KIAA0861, NUMA1* or *GALE* ligands or binding partners, and in screening assays to identify molecules which inhibit or enhance the interaction of *ICAM, MAPK10, KIAA0861, NUMA1* or *GALE* with a *ICAM, MAPK10, KIAA0861, NUMA1* or *GALE* substrate. Full-length *ICAM*, *MAPK10, KIAA0861, NUMA1* or *GALE* polypeptides and polynucleotides encoding the same may be specifically substituted for a *ICAM, MAPK10, KIAA0861, NUMA1* or *GALE* polypeptide fragment or polynucleotide encoding the same in any embodiment described herein.

Substantially identical polypeptides may depart from the amino acid sequences set forth in SEQ ID NO: 13-18 in different manners. For example, conservative amino acid modifications may be introduced at one or more positions in the amino acid sequences of SEQ ID NO: 13-18. A "conservative amino acid substitution" is one in which the amino acid is replaced by another amino acid having a similar structure and/or chemical function. Families of amino acid residues having similar structures and functions are well known. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Also, essential and non-essential amino acids may be replaced. A "non-essential" amino acid is one that can be altered without abolishing or substantially altering the biological function of a *ICAM, MAPK10, KIAA0861, NUMA1* or *GALE* polypeptide, whereas altering an "essential" amino acid abolishes or substantially alters the biological function of a *ICAM, MAPK10, KIAA0861, NUMA1* or *GALE* polypeptide. Amino acids that are conserved among *ICAM, MAPK10, KIAA0861, NUMA1* or *GALE* polypeptides are typically essential amino acids.

Also, *ICAM, MAPK10, KIAAA0861, NUMA1* or *GALE* polypeptides and polypeptide variants may exist as chimeric or fusion polypeptides. As used herein, a *ICAM, MAPK10, KIAA0861, NUMA1* or *GALE* "chimeric polypeptide" or "fusion polypeptide" includes a *ICAM, MAPK10, KIAA0861, NUMA1* or *GALE* polypeptide linked to a non-*ICAM, MAPK10, KIAA0861, NUMA1* or *GALE* polypeptide. A "non-*ICAM, MAPK10, KIAA0861, NUMA1* or *GALE* polypeptide" refers to a polypeptide having an amino acid sequence corresponding to a polypeptide which is not substantially identical to the *ICAM, MAPK10, KIAA0861, NUMA1* or *GALE* polypeptide, which includes, for example, a polypeptide that is different from the *ICAM, MAPK10, KIAA0861, NUMA1* or *GALE* polypeptide and derived from the same or a different organism. The *ICAM, MAPK10, KLAA0861, NUMA1* or *GALE* polypeptide in the fusion polypeptide can correspond to an entire or nearly entire *ICAM, MAPK10, KIAA0861, NUMA1* or *GALE* polypeptide or a fragment thereof. The non-*ICAM, MAPK10, KIAA0861, NUMA1* or *GALE* polypeptide can be fused to the N-terminus or C-terminus of the *ICAM,*

*MAPK10, KIAA0861, NUMA1* or *GALE* polypeptide.

Fusion polypeptides can include a moiety having high affinity for a ligand. For example, the fusion polypeptide can be a GST-*ICAM, MAPK10, KIAA0861, NUMA1* or *GALE* fusion polypeptide in which the *ICAM, MAPK10, KIAA0861, NUMA1* or *GALE* sequences are fused to the C-terminus of the GST sequences, or a polyhistidine-*ICAM, MAPK10, KIAA0861, NUMA1* or *GALE* fusion polypeptide in which the *ICAM, MAPK10, KIAA0861, NUMA1* or *GALE* polypeptide is fused at the N- or C-terminus to a string of histidine residues. Such fusion polypeptides can facilitate purification of recombinant *ICAM*, *MAPK10, KIAA0861, NUMA1* or *GALE*. Expression vectors are commercially available that already encode a fusion moiety (e.g., a GST polypeptide), and a *ICAM, MAPK10, KIAA0861, NUMA1* or *GALE* nucleic acid can be cloned into an expression vector such that the fusion moiety is linked in-frame to the *ICAM, MAPK10, KIAA0861, NUMA1* or *GALE* polypeptide. Further, the fusion polypeptide can be a *ICAM, MAPK10, KIAA0861, NUMA1* or *GALE* polypeptide containing a heterologous signal sequence at its N-terminus. In certain host cells (e.g., mammalian host cells), expression, secretion, cellular internalization, and cellular localization of a *ICAM, MAPK10, KIAA0861, NUMA1* or *GALE* polypeptide can be increased through use of a heterologous signal sequence. Fusion polypeptides can also include all or a part of a serum polypeptide (e.g., an IgG constant region or human serum albumin).

*ICAM, MAPK10, KIAA0861, NUMA1* or *GALE* polypeptides or fragments thereof can be incorporated into pharmaceutical compositions and administered to a subject in vivo. Administration of these *ICAM, MAPK10, KIAA0861, NUMA1* or *GALE* polypeptides can be used to affect the bioavailability of a *ICAM, MAPK10, KIAA0861, NUAM1* or *GALE* substrate and may effectively increase or decrease *ICAM, MAPK10, KIAA0861, NUMA1* or *GALE* biological activity in a cell or effectively supplement dysfunctional or hyperactive *ICAM, MAPK10, KIAA0861, NUMA1* or *GALE* polypeptide. *ICAM, MAPK10, KIAA0861, NUMA1* or *GALE* fusion polypeptides may be useful therapeutically for the treatment of disorders caused by, for example, (i) aberrant modification or mutation of a gene encoding a *ICAM, MAPK10, KIAA0861, NUMA1* or *GALE* polypeptide; (ii) mis-regulation of the *ICAM, MAPK10, KIAA0861, NUMA1* or *GALE* gene; and (iii) aberrant post-translational modification of a *ICAM, MAPK10, KIAA0861, NUMA1* or *GALE* polypeptide. Also, *ICAM, MAPK10, KIAA0861, NUMA1* or *GALE* polypeptides can be used as immunogens to produce anti-*ICAM, MAPK10, KIAA0861, NUMA1* or *GALE* antibodies in a subject, to purify *ICAM, MAPK10, KIAA0861, NUMA1* or *GALE* ligands or binding partners, and in screening assays to identify molecules which inhibit or enhance the interaction of *ICAM, MAPK10, KIAA0861, NUMA1* or *GALE* with a *ICAM, MAPK10, KIAA0861, NUMA1* or *GALE* substrate.

In addition, polypeptides can be chemically synthesized using techniques known in the art (See, *e.g.*, Creighton, 1983 Proteins. New York, N.Y.: W. H. Freeman and Company; and Hunkapiller et al., (1984) Nature July 12 -18;310 (5973):105-11). For example, a relative short polypeptide fragment can be synthesized by use of a peptide synthesizer. Furthermore, if desired, non-classical amino acids or chemical amino acid analogs can be introduced as a substitution or addition into the fragment sequence. Non-classical amino acids include, but are not limited to, to the D-isomers of the common amino acids, 2,4-diaminobutyric acid, α-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, b-alanine, fluoroamino acids, designer amino acids such as b-methyl amino acids, Ca-methyl amino acids, Na-methyl amino acids, and amino acid analogs in general. Furthermore, the amino acid can be D (dextrorotary) or L (levorotary).

Also included are polypeptide fragments which are differentially modified during or after translation, *e.g.*, by glycosylation, acetylation, phosphorylation, amidation, derivatization by known protecting/blocking groups, proteolytic cleavage, linkage to an antibody molecule or other cellular ligand, and the like. Any of numerous chemical modifications may be carried out by known techniques, including but not limited, to specific chemical cleavage by cyanogen bromide, trypsin, chymotrypsin, papain, V8 protease, $NaBH_4$; acetylation, formylation, oxidation, reduction; metabolic synthesis in the presence of tunicamycin; and the like.

Additional post-translational modifications include, for example, N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of prokaryotic host cell expression. The polypeptide fragments may also be modified with a detectable label, such as an enzymatic, fluorescent, isotopic or affinity label to allow for detection and isolation of the polypeptide.

Also provided are chemically modified polypeptide derivatives that may provide additional advantages such as increased solubility, stability and circulating time of the polypeptide, or decreased immunogenicity. See U.S. Pat. No: 4,179,337. The chemical moieties for derivitization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (*e.g.*, the duration of sustained release desired, the effects, if any on biological activity, the ease in handling, the degree or lack of antigenicity and other known effects of the polyethylene glycol to a therapeutic protein or analog).

The polyethylene glycol molecules (or other chemical moieties) should be attached to the polypeptide with consideration of effects on functional or antigenic domains of the polypeptide. There are a number of attachment methods available to those skilled in the art, *e.g.*, EP 0 401 384, herein incorporated by reference (coupling PEG to G-CSF), see also Malik et al. (1992) Exp Hematol. September;20(8):1028-35, reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues, glutamic acid residues and the C-terminal amino acid residue. Sulfhydryl groups may also be used as a reactive group for attaching the polyethylene glycol molecules. A polymer sometimes is attached at an amino group, such as attachment at the N-terminus or lysine group.

One may specifically desire proteins chemically modified at the N-terminus. Using polyethylene glycol as an illustration of the present composition, one may select from a variety of polyethylene glycol molecules (by molecular weight, branching, and the like), the proportion of polyethylene glycol molecules to protein (polypeptide) molecules in the reaction mix, the type of pegylation reaction to be performed, and the method of obtaining the selected N-terminally pegylated protein. The method of obtaining the N-terminally pegylated preparation (i.e., separating this moiety from other monopegylated moieties if necessary) may be by purification of the N-terminally pegylated material from a population of pegylated protein molecules. Selective proteins chemically modified at the N-terminus may be accomplished by reductive alkylation, which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

Substantially Identical Nucleic Acids and Polypeptides

Nucleotide sequences and polypeptide sequences that are substantially identical to a *ICAM, MAPK10, KIAA0861, NUMA1* or *GALE* nucleotide sequence and the *ICAM, MAPK10, KIAA0861, NUMA1* or *GALE* polypeptide sequences encoded by those nucleotide sequences are included herein. The term "substantially identical" as used herein refers to two or more nucleic acids or polypeptides sharing one or more identical nucleotide sequences or polypeptide sequences, respectively. Included are nucleotide sequences or polypeptide sequences that are 55% or more, 60% or more, 65% or more, 70% or more, 75% or more, 80% or more, 85% or more, 90% or more, 95% or more (each often within a 1%, 2%, 3% or 4% variability) or more identical to the nucleotide sequences in SEQ ID NO: 1-12 or the encoded *ICAM, MAPK10, KIAA0861, NUMA1* or *GALE* polypeptide amino acid sequences. One test for determining whether two nucleic acids are substantially identical is to determine the percent of identical nucleotide sequences or polypeptide sequences shared between the nucleic acids or polypeptides.

Calculations of sequence identity are often performed as follows. Sequences are aligned for optimal comparison purposes (*e.g.*, gaps can be introduced in one or both of a first and a second amino acid or nucleic acid sequence for optimal alignment and non-homologous sequences can be disregarded for comparison purposes). The length of a reference sequence aligned for comparison purposes is sometimes 30% or more, 40% or more, 50% or more, often 60% or more, and more often 70% or more, 80% or more, 90% or more, 90% or more, or 100% of the length of the reference sequence. The nucleotides or amino acids at corresponding nucleotide or polypeptide positions, respectively, are then compared among the two sequences. When a position in the first sequence is occupied by the same nucleotide or amino acid as the corresponding position in the second sequence, the nucleotides or amino acids are deemed to be identical at that position. The percent identity between the two sequences is a function of the number of identical positions shared by the sequences, taking into account the number of gaps, and the length of each gap, introduced for optimal alignment of the two sequences.

Comparison of sequences and determination of percent identity between two sequences can be accomplished using a mathematical algorithm. Percent identity between two amino acid or nucleotide sequences can be determined using the algorithm of Meyers & Miller, CABIOS 4: 11-17 (1989), which has been incorporated into the ALIGN program (version 2.0), using a PAM120 weight residue table, a gap length penalty of 12 and a gap penalty of 4. Also, percent identity between two amino acid sequences can be determined using the Needleman & Wunsch, J. Mol. Biol. 48: 444-453 (1970) algorithm which has been incorporated into the GAP program in the GCG software package (available at the http address www.gcg.com), using either a Blossum 62 matrix or a PAM250 matrix, and a gap weight of 16, 14, 12, 10, 8, 6, or 4 and a length weight of 1, 2, 3, 4, 5, or 6. Percent identity between two nucleotide sequences can be determined using the GAP program in the GCG software package (available at http address www.gcg.com), using a NWSgapdna.CMP matrix and a gap weight of 40, 50, 60, 70, or 80 and a length weight of 1, 2, 3, 4, 5, or 6. A set of parameters often used is a Blossum 62 scoring matrix with a gap open penalty of 12, a gap extend penalty of 4, and a frameshift gap penalty of 5.

Another manner for determining if two nucleic acids are substantially identical is to assess whether a polynucleotide homologous to one nucleic acid will hybridize to the other nucleic acid under stringent conditions. As use herein, the term "stringent conditions" refers to conditions for hybridization and washing. Stringent conditions are known to those skilled in the art and can be found in Current Protocols in Molecular Biology, John Wiley & Sons, N.Y. , 6.3.1-6.3.6 (1989). Aqueous and nonaqueous methods are described in that reference and either can be used. An example of stringent hybridization conditions is hybridization in 6X sodium chloride/sodium citrate (SSC) at about 45˚C, followed by one or more washes in 0.2X SSC, 0.1% SDS at 50˚C. Another example of stringent hybridization conditions are hybridization in 6X sodium chloride/sodium citrate (SSC) at about 45˚C, followed by one or more washes in 0.2X SSC, 0.1% SDS at 55˚C. A further example of stringent hybridization conditions is hybridization in 6X sodium chloride/sodium citrate (SSC) at about 45˚C, followed by one or more washes in 0.2X SSC, 0.1 % SDS at 60˚C. Often, stringent hybridization conditions are hybridization in 6X sodium chloride/sodium citrate (SSC) at about 45˚C, followed by one or more washes in 0.2X SSC, 0.1 % SDS at 65˚C. More often, stringency conditions are 0.5M sodium phosphate, 7% SDS at 65˚C, followed by one or more washes at 0.2X SSC, 1% SDS at 65˚C.

An example of a substantially identical nucleotide sequence to a *ICAM, MAPK10, KIAA0861, NUMA1* or *GALE* nucleotide sequence is one that has a different nucleotide sequence but still encodes the same polypeptide sequence encoded by the *ICAM, MAPK10, KIAA0861, NUMA1* or *GALE* nucleotide sequence. Another example is a nucleotide sequence that encodes a polypeptide having a polypeptide sequence that is more than 70% or more identical to, sometimes 75% or more, 80% or more, or 85% or more identical to, and often 90% or more and 95% or more identical to a polypeptide sequence encoded by a *ICAM, MAPK10, KIAA0861, NUMA1* or *GALE* nucleotide sequence.

*ICAM, MAPK10, KIAA0861, NUMA1* or *GALE* nucleotide sequences and *ICAM, MAPK10, KIAA0861, NUMA1* or *GALE* amino acid sequences can be used as "query sequences" to perform a search against public databases to identify other family members or related sequences, for example. Such searches can be performed using the NBLAST and XBLAST programs (version 2.0) of Altschul et al., J. Mol. Biol. 215: 403-10 (1990). BLAST nucleotide searches can be performed with the NBLAST program, score =100, wordlength =12 to obtain nucleotide sequences homologous to nucleotide sequences from SEQ ID NO: 1-12. BLAST polypeptide searches can be performed with the XBLAST program, score = 50, wordlength = 3 to obtain amino acid sequences homologous to polypeptides encoded by a *ICAM, MAPK10, KIAA0861, NUMA1* or *GALE* nucleotide sequence. To obtain gapped alignments for comparison purposes, Gapped BLAST can be utilized as described in Altschul et al., Nucleic Acids Res. 25(17): 3389-3402 (1997). When utilizing BLAST and Gapped BLAST programs, default parameters of the respective programs (*e.g.*, XBLAST and NBLAST) can be used (*see* the http address www.ncbi.nlm.nih.gov).

A nucleic acid that is substantially identical to a *ICAM, MAPK10, KIAA0861, NUMA1* or *GALE* nucleotide sequence may include polymorphic sites at positions equivalent to those described herein when the sequences are aligned. For example, using the alignment procedures described herein, SNPs in a sequence substantially identical to a sequence in SEQ ID NO: 1-12 can be identified at nucleotide positions that match (*i.e.*, align) with nucleotides at SNP positions in the nucleotide sequence of SEQ ID NO: 1-12. Also, where a polymorphic variation results in an insertion or deletion, insertion or deletion of a nucleotide sequence from a reference sequence can change the relative positions of other polymorphic sites in the nucleotide sequence.

Substantially identical nucleotide and polypeptide sequences include those that are naturally occurring, such as allelic variants (same locus), splice variants, homologs (different locus), and orthologs (different organism) or can be non-naturally occurring. Non-naturally occurring variants can be generated by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product). Orthologs, homologs, allelic variants, and splice variants can be identified using methods known in the art. These variants normally comprise a nucleotide sequence encoding a polypeptide that is 50% or more, about 55% or more, often about 70-75% or more, more often about 80-85% or more, and typically about 90-95% or more identical to the amino acid sequences of target polypeptides or a fragment thereof. Such nucleic acid molecules readily can be identified as being able to hybridize under stringent conditions to a nucleotide sequence in SEQ ID NO: 1-12 or a fragment thereof. Nucleic acid molecules corresponding to orthologs, homologs, and allelic variants of a nucleotide sequence in SEQ ID NO: 1-12 can be identified by mapping the sequence to the same chromosome or locus as the nucleotide sequence in SEQ ID NO: 1-12.

Also, substantially identical nucleotide sequences may include codons that are altered with respect to the naturally occurring sequence for enhancing expression of a target polypeptide in a particular expression system. For example, the nucleic acid can be one in which one or more codons are altered, and often 10% or more or 20% or more of the codons are altered for optimized expression in bacteria (*e.g.*, *E. coli*.), yeast (*e.g.*, *S. cervesiae*), human (*e.g.*, 293 cells), insect, or rodent (*e.g.*, hamster) cells.

Methods for Identifying Subjects at Risk of Breast Cancer and Breast Cancer Risk in a Subject

Methods for prognosing and diagnosing breast cancer in subjects are provided herein. These methods include detecting the presence or absence of one or more polymorphic variations associated with breast cancer in a nucleotide sequence set forth in SEQ ID NO: 1-5, or substantially identical sequence thereof, in a sample from a subject, where the presence of a polymorphic variant is indicative of a risk of breast cancer.

Thus, featured herein is a method for detecting a subject at risk of breast cancer or the risk of breast cancer in a subj ect, which comprises detecting the presence or absence of a polymorphic variation associated with breast cancer at a polymorphic site in a nucleic acid sample from a subject, where the nucleotide sequence comprises a polynucleotide sequence selected from the group consisting of: (a) a nucleotide sequence set forth in SEQ ID NO: 1-5; (b) a nucleotide sequence which encodes a polypeptide having an amino acid sequence encoded by a nucleotide sequence in SEQ ID NO: 1-5; (c) a nucleotide sequence which encodes a polypeptide that is 90% or more identical to an amino acid sequence encoded by a nucleotide sequence in SEQ ID NO: 1-5 or a nucleotide sequence about 90% or more identical to the nucleotide sequence set forth in SEQ ID NO: 1-5; and (d) a fragment of a nucleotide sequence of (a), (b), or (c), often a fragment that includes a polymorphic site associated with breast cancer; whereby the presence of the polymorphic variation is indicative of a risk of breast cancer in the subject.

In certain embodiments, determining the presence of a combination of two or more polymorphic variants associated with breast cancer in one or more genetic loci (e.g., one or more genes) of the sample is determined to identify, quantify and/or estimate, risk of breast cancer. The risk often is the probability of having or developing breast cancer. The risk sometimes is expressed as a relative risk with respect to a population average risk of breast cancer, and sometimes is expressed as a relative risk with resepect to the lowest risk group. Such relative risk assessments often are based upon penetrance values determined by statistical methods (see e.g., statistical analysis Example 9), and are particularly useful to clinicians and insurance companies for assessing risk of breast cancer (e.g., a clinician can target appropriate detection, prevention and therapeutic regimens to a partient after determining the patient's risk of breast cancer, and an insurance company can fine tune actuarial tables based upon population genotype assessments of breast cancer risk). Risk of breast cancer sometimes is expressed as an odds ratio, which is the odds of a particular person having a genotype has or will develop breast cancer with respect to another genotype group (e.g., the most disease protective genotype or population average). In related embodiments, the determination is utilized to identify a subject at risk of breast cancer. In an embodiment, two or more polymorphic variations are detected in two or more regions in human genomic DNA associated with increased risk of breast cancer, such as regions selected from the group of loci consisting of *ICAM, MAPK10, KIAA0861, NUMA1* and *GALE*, for example. In certain embodiments, 3 or more, or 4, 5, 6, 7, 8, 9, 10, 11, 12, 13, 14, 15, 16, 17, 18, 19 or 20 or more polymorphic variants are detected in the sample. In specific embodiments, polymorphic variants are detected in *ICAM, MAPK10, KIAA0861, NUMA1* and *GALE* loci, such as at positions 44247 in SEQ ID NO: 1 (*ICAM*), position 36424 in SEQ ID NO: 2 (*MAPK10*), position 48563 in SEQ ID NO: 3 (*KIAA0861*), position 49002 in SEQ ID NO: 4 (*NUMA1*) and position 174 in SEQ ID NO: 5 (*GALE*), for example. In certain embodiments, polymorphic variants are detected at other genetic loci (e.g., the polymorphic variants can be detected in *ICAM, MAPK10, KIAA0861, NUMA1* and/or *GALE* in addition to other loci or only in other loci), where the other loci include but are not limited to *RAD21, KLF12, SPUVE, GRIN3A, PFTK1, SERPINA5, LOC115209, HRMT1L3, DLG1, KIAA0783, DPF3, CENPC1, GP6, LAMA4, CHCB/C20ORF154, LOC338749,* and *TTN/LOC351327*, which are described in concurrently-filed patent applications having attorney docket numbers 524592006700, 524592006800, 524592007000, 524592007100 and 524592007200, and any others disclosed in patent application nos. 60/429,136 (filed November 25, 2002) 60/490,234 (filed July 24, 2003).

A risk of developing aggressive forms of breast cancer likely to metastasize or invade surrounding tissues (e.g., Stage IIIA, IIIB, and IV breast cancers), and subjects at risk of developing aggressive forms of breast cancer also may be identified by the methods described herein. These methods include collecting phenotype information from subjects having breast cancer, which includes the stage of progression of the breast cancer, and performing a secondary phenotype analysis to detect the presence or absence of one or more polymorphic variations associated with a particular stage form of breast cancer. Thus, detecting the presence or absence of one or more polymorphic variations in a *ICAM, MAPK10, KIAA0861, NUMA1* or *GALE* nucleotide sequence associated with a late stage form of breast cancer often is prognostic and/or diagnostic of an aggressive form of the cancer.

Results from prognostic tests may be combined with other test results to diagnose breast cancer. For example, prognostic results may be gathered, a patient sample may be ordered based on a determined predisposition to breast cancer, the patient sample is analyzed, and the results of the analysis may be utilized to diagnose breast cancer. Also breast cancer diagnostic methods can be developed from studies used to generate prognostic/diagnostic methods in which populations are stratified into subpopulations having different progressions of breast cancer. In another embodiment, prognostic results may be gathered; a patient's risk factors for developing breast cancer analyzed (*e.g.*, age, race, family history, age of first menstrual cycle, age at birth of first child); and a patient sample may be ordered based on a determined predisposition to breast cancer. In an alternative embodiment, the results from predisposition analyses described herein may be combined with other test results indicative of breast cancer, which were previously, concurrently, or subsequently gathered with respect to the predisposition testing. In these embodiments, the combination of the prognostic test results with other test results can be probative of breast cancer, and the combination can be utilized as a breast cancer diagnostic. The results of any test indicative of breast cancer known in the art may be combined with the methods described herein. Examples of such tests are mammography (*e.g.*, a more frequent and/or earlier mammography regimen may be prescribed); breast biopsy and optionally a biopsy from another tissue; breast ultrasound and optionally an ultrasound analysis of another tissue; breast magnetic resonance imaging (MRI) and optionally an MRI analysis of another tissue; electrical impedance (T-scan) analysis of breast and optionally of another tissue; ductal lavage; nuclear medicine analysis (*e.g.*, scintimammography); *BRCA1* and/or *BRCA2* sequence analysis results; and thermal imaging of the breast and optionally of another tissue. Testing may be performed on tissue other than breast to diagnose the occurrence of metastasis (*e.g.*, testing of the lymph node).

Risk of breast cancer sometimes is expressed as a probability, such as an odds ratio, percentage, or risk factor. The risk is based upon the presence or absence of one or more polymorphic variants described herein, and also may be based in part upon phenotypic traits of the individual being tested. Methods for calculating predispositions based upon patient data are well known (*see, e.g.*, Agresti, Categorical Data Analysis, 2nd Ed. 2002. Wiley). Allelotyping and genotyping analyses may be carried out in populations other than those exemplified herein to enhance the predictive power of the prognostic method. These further analyses are executed in view of the exemplified procedures described herein, and may be based upon the same polymorphic variations or additional polymorphic variations. Risk determinations for breast cancer are useful in a variety of applications. In one embodiment, breast cancer risk determinations are used by clinicians to direct appropriate detection, preventative and treatment procedures to subjects who most require these. In another embodiment, breast cancer risk determinations are used by health insurers for preparing actuarial tables and for calculating insurance premiums.

The nucleic acid sample typically is isolated from a biological sample obtained from a subject. For example, nucleic acid can be isolated from blood, saliva, sputum, urine, cell scrapings, and biopsy tissue. The nucleic acid sample can be isolated from a biological sample using standard techniques, such as the technique described in Example 2. As used herein, the term "subject" refers primarily to humans but also refers to other mammals such as dogs, cats, and ungulates (*e.g.*, cattle, sheep, and swine). Subjects also include avians (*e.g.*, chickens and turkeys), reptiles, and fish *(e.g.,* salmon), as embodiments described herein can be adapted to nucleic acid samples isolated from any of these organisms. The nucleic acid sample may be isolated from the subject and then directly utilized in a method for determining the presence of a polymorphic variant, or alternatively, the sample may be isolated and then stored (*e.g.*, frozen) for a period of time before being subjected to analysis.

The presence or absence of a polymorphic variant is determined using one or both chromosomal complements represented in the nucleic acid sample. Determining the presence or absence of a polymorphic variant in both chromosomal complements represented in a nucleic acid sample from a subject having a copy of each chromosome is useful for determining the zygosity of an individual for the polymorphic variant (*i.e.*, whether the individual is homozygous or heterozygous for the polymorphic variant). Any oligonucleotide-based diagnostic may be utilized to determine whether a sample includes the presence or absence of a polymorphic variant in a sample. For example, primer extension methods, ligase sequence determination methods (*e.g.*, U.S. Pat. Nos. 5,679,524 and 5,952,174, and WO 01/27326), mismatch sequence determination methods (*e.g.*, U.S. Pat. Nos. 5,851,770; 5,958,692; 6,110,684; and 6,183,958), microarray sequence determination methods, restriction fragment length polymorphism (RFLP), single strand conformation polymorphism detection (SSCP) (*e.g.*, U.S. Pat. Nos. 5,891,625 and 6,013,499), PCR-based assays (*e.g.*, TAQMAN® PCR System (Applied Biosystems)), and nucleotide sequencing methods may be used.

Oligonucleotide extension methods typically involve providing a pair of oligonucleotide primers in a polymerase chain reaction (PCR) or in other nucleic acid amplification methods for the purpose of amplifying a region from the nucleic acid sample that comprises the polymorphic variation. One oligonucleotide primer is complementary to a region 3' of the polymorphism and the other is complementary to a region 5' of the polymorphism. A PCR primer pair may be used in methods disclosed in U.S. Pat. Nos. 4,683,195; 4,683,202, 4,965,188; 5,656,493; 5,998,143; 6,140,054; WO 01/27327; and WO 01/27329 for example. PCR primer pairs may also be used in any commercially available machines that perform PCR, such as any of the GENEAMP® Systems available from Applied Biosystems. Also, those of ordinary skill in the art will be able to design oligonucleotide primers based upon a nucleotide sequence set forth in SEQ ID NO: 1-5 without undue experimentation using knowledge readily available in the art.

Also provided is an extension oligonucleotide that hybridizes to the amplified fragment adjacent to the polymorphic variation. As used herein, the term "adjacent" refers to the 3' end of the extension oligonucleotide being often 1 nucleotide from the 5' end of the polymorphic site, and sometimes 2, 3, 4, 5, 6, 7, 8, 9, or 10 nucleotides from the 5' end of the polymorphic site, in the nucleic acid when the extension oligonucleotide is hybridized to the nucleic acid. The extension oligonucleotide then is extended by one or more nucleotides, and the number and/or type of nucleotides that are added to the extension oligonucleotide determine whether the polymorphic variant is present. Oligonucleotide extension methods are disclosed, for example, in U.S. Pat. Nos. 4,656,127; 4,851,331; 5,679,524; 5,834,189; 5,876,934;

5,908,755; 5,912,118; 5,976,802; 5,981,186; 6,004,744; 6,013,431; 6,017,702; 6,046,005; 6,087,095; 6,210,891; and WO 01/20039. Oligonucleotide extension methods using mass spectrometry are described, for example, in U.S. Pat. Nos. 5,547,835; 5,605,798; 5,691,141; 5,849,542; 5,869,242; 5,928,906; 6,043,031; and 6,194,144, and a method often utilized is described herein in Example 2. Multiple extension oligonucleotides may be utilized in one reaction, which is referred to herein as "multiplexing."

A microarray can be utilized for determining whether a polymorphic variant is present or absent in a nucleic acid sample. A microarray may include any oligonucleotides described herein, and methods for making and using oligonucleotide microarrays suitable for diagnostic use are disclosed in U.S. Pat. Nos. 5,492,806; 5,525,464; 5,589,330; 5,695,940; 5,849,483; 6,018,041; 6,045,996; 6,136,541; 6,142,681; 6,156,501; 6,197,506; 6,223,127; 6,225,625; 6,229,911; 6,239,273; WO 00/52625; WO 01/25485; and WO 01/29259. The microarray typically comprises a solid support and the oligonucleotides may be linked to this solid support by covalent bonds or by non-covalent interactions. The oligonucleotides may also be linked to the solid support directly or by a spacer molecule. A microarray may comprise one or more oligonucleotides complementary to a polymorphic site set forth in SEQ ID NO: 1-5 or below.

A kit also may be utilized for determining whether a polymorphic variant is present or absent in a nucleic acid sample. A kit often comprises one or more pairs of oligonucleotide primers useful for amplifying a fragment of a *ICAM, MAPK10, KIAA0861, NUMA1* or *GALE* nucleotide sequence or a substantially identical sequence thereof, where the fragment includes a polymorphic site. The kit sometimes comprises a polymerizing agent, for example, a thermostable nucleic acid polymerase such as one disclosed in U.S. Pat. Nos. 4,889,818 or 6,077,664. Also, the kit often comprises an elongation oligonucleotide that hybridizes to a *ICAM, MAPK10, KIAA0861, NUMA1* or *GALE* nucleotide sequence in a nucleic acid sample adjacent to the polymorphic site. Where the kit includes an elongation oligonucleotide, it also often comprises chain elongating nucleotides, such as dATP, dTTP, dGTP, dCTP, and dTTP, including analogs of dATP, dTTP, dGTP, dCTP and dITP, provided that such analogs are substrates for a thermostable nucleic acid polymerase and can be incorporated into a nucleic acid chain elongated from the extension oligonucleotide. Along with chain elongating nucleotides would be one or more chain terminating nucleotides such as ddATP, ddTTP, ddGTP, ddCTP, and the like. In an embodiment, the kit comprises one or more oligonucleotide primer pairs, a polymerizing agent, chain elongating nucleotides, at least one elongation oligonucleotide, and one or more chain terminating nucleotides. Kits optionally include buffers, vials, microtiter plates, and instructions for use.

An individual identified as being at risk of breast cancer may be heterozygous or homozygous with respect to the allele associated with a higher risk of breast cancer. A subject homozygous for an allele associated with an increased risk of breast cancer is at a comparatively high risk of breast cancer, a subject heterozygous for an allele associated with an increased risk of breast cancer is at a comparatively intermediate risk of breast cancer, and a subject homozygous for an allele associated with a decreased risk of breast cancer is at a comparatively low risk of breast cancer. A genotype may be assessed for a complementary strand, such that the complementary nucleotide at a particular position is detected.

Also featured are methods for determining risk of breast cancer and/or identifying a subject at risk of breast cancer by contacting a polypeptide or protein encoded by a *ICAM, MAPK10, KIAA0861, NUMA1* or *GALE* nucleotide sequence from a subject with an antibody that specifically binds to an epitope associated with increased risk of breast cancer in the polypeptide. In certain embodiments, the antibody specifically binds to an epitope that comprises a leucine at amino acid position 359 in SEQ ID NO: 17, a leucine at amino acid position 378 in SEQ ID NO: 17, or an alanine at amino acid position 857 in SEQ ID NO: 17, a proline at amino acid position 352 in SEQ ID NO: 15 or an alanine at amino acid position 348 in SEQ ID NO: 15.

Applications of Prognostic and Diagnostic Results to Pharmacogenomic Methods

Pharmacogenomics is a discipline that involves tailoring a treatment for a subject according to the subject's genotype. For example, based upon the outcome of a prognostic test described herein, a clinician or physician may target pertinent information and preventative or therapeutic treatments to a subject who would be benefited by the information or treatment and avoid directing such information and treatments to a subject who would not be benefited (*e.g.*, the treatment has no therapeutic effect and/or the subject experiences adverse side effects). As therapeutic approaches for breast cancer continue to evolve and improve, the goal of treatments for breast cancer related disorders is to intervene even before clinical signs (*e.g.*, identification of lump in the breast) first manifest. Thus, genetic markers associated with susceptibility to breast cancer prove useful for early diagnosis, prevention and treatment of breast cancer.

The following is an example of a pharmacogenomic embodiment. A particular treatment regimen can exert a differential effect depending upon the subject's genotype. Where a candidate therapeutic exhibits a significant interaction with a major allele and a comparatively weak interaction with a minor allele (*e.g.*, an order of magnitude or greater difference in the interaction), such a therapeutic typically would not be administered to a subject genotyped as being homozygous for the minor allele, and sometimes not administered to a subject genotyped as being heterozygous for the minor allele. In another example, where a candidate therapeutic is not significantly toxic when administered to subjects who are homozygous for a major allele but is comparatively toxic when administered to subjects heterozygous or homozygous for a minor allele, the candidate therapeutic is not typically administered to subjects who are genotyped as being heterozygous or homozygous with respect to the minor allele.

The methods described herein are applicable to pharmacogenomic methods for detecting, preventing, alleviating and/or treating breast cancer. For example, a nucleic acid sample from an individual may be subjected to a genetic test described herein. Where one or more polymorphic variations associated with increased risk of breast cancer are identified in a subject, information for detecting, preventing or treating breast cancer and/or one or more breast cancer detection, prevention and/or treatment regimens then may be directed to and/or prescribed to that subject.

In certain embodiments, a detection, prevenative and/or treatment regimen is specifically prescribed and/or administered to individuals who will most benefit from it based upon their risk of developing breast cancer assessed by the methods described herein. Thus, provided are methods for identifying a subject at risk of breast cancer and then prescribing a detection, therapeutic or preventative regimen to individuals identified as being at risk of breast cancer. Thus, certain embodiments are directed to methods for treating breast cancer in a subject, reducing risk of breast cancer in a subject, or early detection of breast cancer in a subject, which comprise: detecting the presence or absence of a polymorphic variant associated with breast cancer in a nucleotide sequence in a nucleic acid sample from a subject, where the nucleotide sequence comprises a polynucleotide sequence selected from the group consisting of: (a) a nucleotide sequence set forth in SEQ ID NO: 1-5; (b) a nucleotide sequence which encodes a polypeptide having an amino acid sequence encoded by a nucleotide sequence in SEQ ID NO: 1-5; (c) a nucleotide sequence which encodes a polypeptide that is 90% or more identical to an amino acid sequence encoded by a nucleotide sequence in SEQ ID NO: 1-5 or a nucleotide sequence about 90% or more identical to the nucleotide sequence set forth in SEQ ID NO: 1-5; and (d) a fragment of a nucleotide sequence of (a), (b), or (c), sometimes comprising a polymorphic site associated with breast cancer; and prescribing or administering a breast cancer treatment regimen, preventative regimen and/or detection regimen to a subject from whom the sample originated where the presence of one or more polymorphic variations associated with breast cancer are detected in the nucleotide sequence. In these methods, genetic results may be utilized in combination with other test results to diagnose breast cancer as described above. Other test results include but are not limited to mammography results, imaging results, biopsy results and results from *BRCA1* or *BRAC2* test results, as described above.

Detection regimens include one or more mammography procedures, a regular mammography regimen (*e.g.*, once a year, or once every six, four, three or two months); an early mammography regimen (*e.g.*, mammography tests are performed beginning at age 25, 30, or 35); one or more biopsy procedures (*e.g.*, a regular biopsy regimen beginning at age 40); breast biopsy and biopsy from other tissue; breast ultrasound and optionally ultrasound analysis of another tissue; breast magnetic resonance imaging (MRI) and optionally MRI analysis of another tissue; electrical impedance (T-scan) analysis of breast and optionally another tissue; ductal lavage; nuclear medicine analysis (*e.g.*, scintimammography); *BRCA1* and/or *BRCA2* sequence analysis results; and/or thermal imaging of the breast and optionally another tissue.

Treatments sometimes are preventative (*e.g.*, is prescribed or administered to reduce the probability that a breast cancer associated condition arises or progresses), sometimes are therapeutic, and sometimes delay, alleviate or halt the progression of breast cancer. Any known preventative or therapeutic treatment for alleviating or preventing the occurrence of breast cancer is prescribed and/or administered. For example, certain preventative treatments often are prescribed to subjects having a predisposition to breast cancer and where the subject is not diagnosed with breast cancer or is diagnosed as having symptoms indicative of early stage breast cancer (*e.g.*, stage I). For subjects not diagnosed as having breast cancer, any preventative treatments known in the art can be prescribed and administered, which include selective hormone receptor modulators (*e.g.*, selective estrogen receptor modulators (SERMs) such as tamoxifen, reloxifene, and toremifene); compositions that prevent production of hormones (*e.g.*, aramotase inhibitors that prevent the production of estrogen in the adrenal gland, such as exemestane, letrozole, anastrozol, groserelin, and megestrol); other hormonal treatments (*e.g.*, goserelin acetate and fulvestrant); biologic response modifiers such as antibodies (*e.g.*, trastuzumab (herceptin/HER2)); surgery (*e.g.*, lumpectomy and mastectomy); drugs that delay or halt metastasis (*e.g.*, pamidronate disodium); and alternative/complementary medicine (*e.g.*, acupuncture, acupressure, moxibustion, qi gong, reiki, ayurveda, vitamins, minerals, and herbs (*e.g.*, astragalus root, burdock root, garlic, green tea, and licorice root)).

The use of breast cancer treatments are well known in the art, and include surgery, chemotherapy and/or radiation therapy. Any of the treatments may be used in combination to treat or prevent breast cancer (*e.g.*, surgery followed by radiation therapy or chemotherapy). Examples of chemotherapy combinations used to treat breast cancer include: cyclophosphamide (Cytoxan), methotrexate (Amethopterin, Mexate, Folex), and fluorouracil (Fluorouracil, 5-Fu, Adrucil), which is referred to as CMF; cyclophosphamide, doxorubicin (Adriamycin), and fluorouracil, which is referred to as CAF; and doxorubicin (Adriamycin) and cyclophosphamide, which is referred to as AC.

As breast cancer preventative and treatment information can be specifically targeted to subjects in need thereof (*e.g.*, those at risk of developing breast cancer or those that have early signs of breast cancer), provided herein is a method for preventing or reducing the risk of developing breast cancer in a subject, which comprises: (a) detecting the presence or absence of a polymorphic variation associated with breast cancer at a polymorphic site in a nucleotide sequence in a nucleic acid sample from a subject; (b) identifying a subject with a predisposition to breast cancer, whereby the presence of the polymorphic variation is indicative of a predisposition to breast cancer in the subject; and (c) if such a predisposition is identified, providing the subject with information about methods or products to prevent or reduce breast cancer or to delay the onset of breast cancer. Also provided is a method of targeting information or advertising to a subpopulation of a human population based on the subpopulation being genetically predisposed to a disease or condition, which comprises: (a) detecting the presence or absence of a polymorphic variation associated with breast cancer at a polymorphic site in a nucleotide sequence in a nucleic acid sample from a subject; (b) identifying the subpopulation of subjects in which the polymorphic variation is associated with breast cancer; and (c) providing information only to the subpopulation of subjects about a particular product which may be obtained and consumed or applied by the subject to help prevent or delay onset of the disease or condition.

Pharmacogenomics methods also may be used to analyze and predict a response to a breast cancer treatment or a drug. For example, ifpharmacogenomics analysis indicates a likelihood that an individual will respond positively to a breast cancer treatment with a particular drug, the drug may be administered to the individual. Conversely, if the analysis indicates that an individual is likely to respond negatively to treatment with a particular drug, an alternative course of treatment may be prescribed. A negative response may be defined as either the absence of an efficacious response or the presence of toxic side effects. The response to a therapeutic treatment can be predicted in a background study in which subjects in any of the following populations are genotyped: a population that responds favorably to a treatment regimen, a population that does not respond significantly to a treatment regimen, and a population that responds adversely to a treatment regiment (*e.g.*, exhibits one or more side effects). These populations are provided as examples and other populations and subpopulations may be analyzed. Based upon the results of these analyses, a subject is genotyped to predict whether he or she will respond favorably to a treatment regimen, not respond significantly to a treatment regimen, or respond adversely to a treatment regimen.

The methods describe herein also are applicable to clinical drug trials. One or more polymorphic variants indicative of response to an agent for treating breast cancer or to side effects to an agent for treating breast cancer may be identified using the methods described herein. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems. In certain embodiments, the agent for treating breast cancer described herein targets *ICAM, MAPK10, KIAA0861, NUMA1* or *GALE* or a target in the *ICAM, MAPK10, KIAA0861, NUMA1* or *GALE* pathway.

Thus, another embodiment is a method of selecting an individual for inclusion in a clinical trial of a treatment or drug comprising the steps of: (a) obtaining a nucleic acid sample from an individual; (b) determining the identity of a polymorphic variation which is associated with a positive response to the treatment or the drug, or at least one polymorphic variation which is associated with a negative response to the treatment or the drug in the nucleic acid sample, and (c) including the individual in the clinical trial if the nucleic acid sample contains said polymorphic variation associated with a positive response to the treatment or the drug or if the nucleic acid sample lacks said polymorphic variation associated with a negative response to the treatment or the drug. In addition, the methods for selecting an individual for inclusion in a clinical trial of a treatment or drug encompass methods with any further limitation described in this disclosure, or those following, specified alone or in any combination. The polymorphic variation may be in a sequence selected individually or in any combination from the group consisting of (i) a polynucleotide sequence set forth in SEQ ID NO: 1-5; (ii) a polynucleotide sequence that is 90% or more identical to a nucleotide sequence set forth in SEQ ID NO: 1-5; (iii) a polynucleotide sequence that encodes a polypeptide having an amino acid sequence identical to or 90% or more identical to an amino acid sequence encoded by a nucleotide sequence set forth in SEQ ID NO: 1-5; and (iv) a fragment of a polynucleotide sequence of (i), (ii), or (iii) comprising the polymorphic site. The including step (c) optionally comprises administering the drug or the treatment to the individual if the nucleic acid sample contains the polymorphic variation associated with a positive response to the treatment or the drug and the nucleic acid sample lacks said biallelic marker associated with a negative response to the treatment or the drug.

Also provided herein is a method of partnering between a diagnostic/prognostic testing provider and a provider of a consumable product, which comprises: (a) the diagnostic/prognostic testing provider detects the presence or absence of a polymorphic variation associated with breast cancer at a polymorphic site in a nucleotide sequence in a nucleic acid sample from a subject; (b) the diagnostic/prognostic testing provider identifies the subpopulation of subjects in which the polymorphic variation is associated with breast cancer; (c) the diagnostic/prognostic testing provider forwards information to the subpopulation of subjects about a particular product which may be obtained and consumed or applied by the subject to help prevent or delay onset of the disease or condition; and (d) the provider of a consumable product forwards to the diagnostic test provider a fee every time the diagnostic/prognostic test provider forwards information to the subject as set forth in step (c) above.

Compositions Comprising Breast Cancer-Directed Molecules

Featured herein is a composition comprising a breast cancer cell and one or more molecules specifically directed and targeted to a nucleic acid comprising a *ICAM, MAPK10, KIAA0861, NUMA1* or *GALE* nucleotide sequence or a *ICAM, MAPK10, KIAA0861, NUMA1* or *GALE* polypeptide. Such directed molecules include, but are not limited to, a compound that binds to a *ICAM, MAPK10, KIAA0861, NUMA1* or *GALE* nucleic acid or a *ICAM, MAPK10, KIAA0861, NUMA1* or *GALE* polypeptide; a RNAi or siRNA molecule having a strand complementary to a *ICAM, MAPK10, KIAA0861, NUMA1* or *GALE* nucleotide sequence; an antisense nucleic acid complementary to an RNA encoded by a *ICAM, MAPK10, KIAA0861, NUMA1* or *GALE* DNA sequence; a ribozyme that hybridizes to a *ICAM, MAPK10, KIAA0861, NUMA1* or *GALE* nucleotide sequence; a nucleic acid aptamer that specifically binds a *ICAM, MAPK10, KIAA0861, NUMA1* or *GALE* polypeptide; and an antibody that specifically binds to a *ICAM, MAPK10, KIAA0861, NUMA1* or *GALE* polypeptide or binds to a *ICAM, MAPK10, KIAA0861, NUMA1* or *GALE* nucleic acid. In certain embodiments, the antibody specifically binds to an epitope that comprises a leucine at amino acid position 359 in SEQ ID NO: 17, a leucine at amino acid position 378 in SEQ ID NO: 17, or an alanine at amino acid position 857 in SEQ ID NO: 17, a proline at amino acid position 352 in SEQ ID NO: 15 or an alanine at amino acid position 348 in SEQ ID NO: 15. In specific embodiments, the breast cancer directed molecule interacts with a *ICAM, MAPK10, KIAA0861, NUMA1* or *GALE* nucleic acid or polypeptide variant associated with breast cancer. In other embodiments, the breast cancer directed molecule interacts with a polypeptide involved in the *ICAM, MAPK10, KIAA0861, NUMA1* or *GALE* signal pathway, or a nucleic acid encoding such a polypeptide. Polypeptides involved in the *ICAM, MAPK10, KIAA0861, NUMA1* or *GALE* signal pathway are discussed herein.

Compositions sometimes include an adjuvant known to stimulate an immune response, and in certain embodiments, an adjuvant that stimulates a T-cell lymphocyte response. Adjuvants are known, including but not limited to an aluminum adjuvant (*e.g.*, aluminum hydroxide); a cytokine adjuvant or adjuvant that stimulates a cytokine response (*e.g.*, interleukin (IL)-12 and/or ?-interferon cytokines); a Freund-type mineral oil adjuvant emulsion (*e.g.*, Freund's complete or incomplete adjuvant); a synthetic lipoid compound; a copolymer adjuvant (*e.g.*, TitreMax); a saponin; Quil A; a liposome; an oil-in-water emulsion (*e.g.*, an emulsion stabilized by Tween 80 and pluronic polyoxyethlene/polyoxypropylene block copolymer (Syntex Adjuvant Formulation); TitreMax; detoxified endotoxin (MPL) and mycobacterial cell wall components (TDW, CWS) in 2% squalene (Ribi Adjuvant System)); a muramyl dipeptide; an immune-stimulating complex (ISCOM, *e.g.*, an Ag-modified saponin/cholesterol micelle that forms stable cage-like structure); an aqueous phase adjuvant that does not have a depot effect (*e.g.*, Gerbu adjuvant); a carbohydrate polymer (*e.g.*, AdjuPrime); L-tyrosine; a manide-oleate compound (*e.g.*, Montanide); an ethylene-vinyl acetate copolymer (*e.g.*, Elvax 40W1,2); or lipid A, for example. Such compositions are useful for generating an immune response against a breast cancer directed molecule (*e.g.*, an HLA-binding subsequence within a polypeptide encoded by a nucleotide sequence in SEQ ID NO: 1). In such methods, a peptide having an amino acid subsequence of a polypeptide encoded by a nucleotide sequence in SEQ ID NO: 1-5 is delivered to a subject, where the subsequence binds to an HLA molecule and induces a CTL lymphocyte response. The peptide sometimes is delivered to the subject as an isolated peptide or as a minigene in a plasmid that encodes the peptide. Methods for identifying HLA-binding subsequences in such polypeptides are known (*see e.g.*, publication WO02/20616 and PCT application US98/01373 for methods of identifying such sequences).

The breast cancer cell may be in a group of breast cancer cells and/or other types of cells cultured *in vitro* or in a tissue having breast cancer cells (*e.g.*, a melanocytic lesion) maintained *in vitro* or present in an animal *in vivo* (*e.g.*, a rat, mouse, ape or human). In certain embodiments, a composition comprises a component from a breast cancer cell or from a subject having a breast cancer cell instead of the breast cancer cell or in addition to the breast cancer cell, where the component sometimes is a nucleic acid molecule (*e.g.*, genomic DNA), a protein mixture or isolated protein, for example. The aforementioned compositions have utility in diagnostic, prognostic and pharmacogenomic methods described previously and in breast cancer therapeutics described hereafter. Certain breast cancer molecules are described in greater detail below.

Compounds

Compounds can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive (see, *e.g.*, Zuckermann et al., J. Med. Chem.37: 2678-85 (1994)); spatially addressable parallel solid phase or solution phase libraries; synthetic library methods requiring deconvolution; "one-bead one-compound" library methods; and synthetic library methods using affinity chromatography selection. Biological library and peptoid library approaches are typically limited to peptide libraries, while the other approaches are applicable to peptide, non-peptide oligomer or small molecule libraries of compounds (Lam, Anticancer Drug Des. 12: 145, (1997)). Examples of methods for synthesizing molecular libraries are described, for example, in DeWitt et al., Proc. Natl. Acad. Sci. U.S.A. 90: 6909 (1993); Erb et al., Proc. Natl. Acad. Sci. USA 91: 11422 (1994); Zuckermann et al., J. Med. Chem. 37: 2678 (1994); Cho et al., Science 261: 1303 (1993); Carrell et al., Angew. Chem. Int. Ed. Engl. 33: 2059 (1994); Carell et al., Angew. Chem. Int. Ed. Engl. 33: 2061 (1994); and in Gallop et al., J. Med. Chem. 37: 1233 (1994).

Libraries of compounds may be presented in solution (*e.g.*, Houghten, Biotechniques 13: 412-421 (1992)), or on beads (Lam, Nature 354: 82-84 (1991)), chips (Fodor, Nature 364: 555-556 (1993)), bacteria or spores (Ladner, United States Patent No. 5,223,409), plasmids (Cull et al., Proc. Natl. Acad. Sci. USA 89: 1865-1869 (1992)) or on phage (Scott and Smith, Science 249: 386-390 (1990); Devlin, Science 249: 404-406 (1990); Cwirla et al., Proc. Natl. Acad. Sci. 87: 6378-6382 (1990); Felici, J. Mol. Biol. 222: 301-310 (1991); Ladner supra.).

A compound sometimes alters expression and sometimes alters activity of a *ICAM*, *MAPK10, KIAA0861, NUMA1* or *GALE* polypeptide and may be a small molecule. Small molecules include, but are not limited to, peptides, peptidomimetics (*e.g.*, peptoids), amino acids, amino acid analogs, polynucleotides, polynucleotide analogs, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Antisense Nucleic Acid Molecules, Ribozymes, RNAi, siRNA and Modified Nucleic Acid Molecules

An "antisense" nucleic acid refers to a nucleotide sequence complementary to a "sense" nucleic acid encoding a polypeptide, *e.g.*, complementary to the coding strand of a double-stranded cDNA molecule or complementary to an mRNA sequence. The antisense nucleic acid can be complementary to an entire coding strand in SEQ ID NO: 1-12, or to a portion thereof or a substantially identical sequence thereof. In another embodiment, the antisense nucleic acid molecule is antisense to a "noncoding region" of the coding strand of a nucleotide sequence in SEQ ID NO: 1-12 (*e.g.*, 5' and 3' untranslated regions).

An antisense nucleic acid can be designed such that it is complementary to the entire coding region of an mRNA encoded by a nucleotide sequence in SEQ ID NO: 1-4 (e.g., SEQ ID NO: 6-12), and often the antisense nucleic acid is an oligonucleotide antisense to only a portion of a coding or noncoding region of the mRNA. For example, the antisense oligonucleotide can be complementary to the region surrounding the translation start site of the mRNA, *e.g.*, between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length. The antisense nucleic acids, which include the ribozymes described hereafter, can be designed to target a nucleotide sequence in SEQ ID NO: 1-12, often a variant associated with breast cancer, or a substantially identical sequence thereof. Among the variants, minor alleles and major alleles can be targeted, and those associated with a higher risk of breast cancer are often designed, tested, and administered to subjects.

An antisense nucleic acid can be constructed using chemical synthesis and enzymatic ligation reactions using standard procedures. For example, an antisense nucleic acid (*e.g.*, an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used. Antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

When utilized as therapeutics, antisense nucleic acids typically are administered to a subject (*e.g.*, by direct injection at a tissue site) or generated in situ such that they hybridize with or bind to cellular mRNA and/or genomic DNA encoding a polypeptide and thereby inhibit expression of the polypeptide, for example, by inhibiting transcription and/or translation. Alternatively, antisense nucleic acid molecules can be modified to target selected cells and then are administered systemically. For systemic administration, antisense molecules can be modified such that they specifically bind to receptors or antigens expressed on a selected cell surface, for example, by linking antisense nucleic acid molecules to peptides or antibodies which bind to cell surface receptors or antigens. Antisense nucleic acid molecules can also be delivered to cells using the vectors described herein. Sufficient intracellular concentrations of antisense molecules are achieved by incorporating a strong promoter, such as a pol II or pol III promoter, in the vector construct.

Antisense nucleic acid molecules sometimes are *-anomeric nucleic acid molecules. An *-anomeric nucleic acid molecule forms specific double-stranded hybrids with complementary RNA in which, contrary to the usual *-units, the strands run parallel to each other (Gaultier et al., Nucleic Acids. Res. 15: 6625-6641 (1987)). Antisense nucleic acid molecules can also comprise a 2'-o-methylribonucleotide (Inoue et al., Nucleic Acids Res. 15: 6131-6148 (1987)) or a chimeric RNA-DNA analogue (Inoue et al., FEBS Lett. 215: 327-330 (1987)). Antisense nucleic acids sometimes are composed of DNA or PNA or any other nucleic acid derivatives described previously.

In another embodiment, an antisense nucleic acid is a ribozyme. A ribozyme having specificity for a *ICAM, MAPK10, KIAA0861, NUMA1* or *GALE* nucleotide sequence can include one or more sequences complementary to such a nucleotide sequence, and a sequence having a known catalytic region responsible for mRNA cleavage (see *e.g.*, U.S. Pat. No. 5,093,246 or Haselhoff and Gerlach, Nature 334: 585-591 (1988)). For example, a derivative of a Tetrahymena L-19 IVS RNA is sometimes utilized in which the nucleotide sequence of the active site is complementary to the nucleotide sequence to be cleaved in a mRNA (see *e.g.*, Cech et al. U.S. Patent No. 4,987,071; and Cech et al. U.S. Patent No. 5,116,742). Also, target mRNA sequences can be used to select a catalytic RNA having a specific ribonuclease activity from a pool of RNA molecules (see *e.g.*, Bartel & Szostak, Science 261: 1411-1418 (1993)).

Breast cancer directed molecules include in certain embodiments nucleic acids that can form triple helix structures with a *ICAM, MAPK10, KIAA0861, NUMA1* or *GALE* nucleotide sequence or a substantially identical sequence thereof, especially one that includes a regulatory region that controls expression of a polypeptide. Gene expression can be inhibited by targeting nucleotide sequences complementary to the regulatory region of a *ICAM, MAPK10, KIAA0861, NUMA1* or *GALE* nucleotide sequence or a substantially identical sequence (*e.g.*, promoter and/or enhancers) to form triple helical structures that prevent transcription of a gene in target cells (see *e.g.*, Helene, Anticancer Drug Des. 6(6): 569-84 (1991); Helene et al., Ann. N.Y. Acad. Sci. 660: 27-36 (1992); and Maher, Bioassays 14(12): 807-15 (1992). Potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Breast cancer directed molecules include RNAi and siRNA nucleic acids. Gene expression may be inhibited by the introduction of double-stranded RNA (dsRNA), which induces potent and specific gene silencing, a phenomenon called RNA interference or RNAi. See, *e.g.*, Fire et al., US Patent Number 6,506,559; Tuschl et al. PCT International Publication No. WO 01/75164; Kay et al. PCT International Publication No. WO 03/010180A1; or Bosher JM, Labouesse, Nat Cell Biol 2000 Feb;2(2):E31-6. This process has been improved by decreasing the size of the double-stranded RNA to 20-24 base pairs (to create small-interfering RNAs or siRNAs) that "switched off" genes in mammalian cells without initiating an acute phase response, i.e., a host defense mechanism that often results in cell death (see, *e.g.*, Caplen et al. Proc Natl Acad Sci U S A. 2001 Aug 14;98(17):9742-7 and Elbashir et al. Methods 2002 Feb;26(2):199-213). There is increasing evidence of post-transcriptional gene silencing by RNA interference (RNAi) for inhibiting targeted expression in mammalian cells at the mRNA level, in human cells. There is additional evidence of effective methods for inhibiting the proliferation and migration of tumor cells in human patients, and for inhibiting metastatic cancer development (see, *e.g.*, U.S. Patent Application No. US2001000993183; Caplen et al. Proc Natl Acad Sci U S A; and Abderrahmani et al. Mol Cell Biol 2001 Nov21(21):7256-67).

An "siRNA" or "RNAi" refers to a nucleic acid that forms a double stranded RNA and has the ability to reduce or inhibit expression of a gene or target gene when the siRNA is delivered to or expressed in the same cell as the gene or target gene. "siRNA" refers to short double-stranded RNA formed by the complementary strands. Complementary portions of the siRNA that hybridize to form the double stranded molecule often have substantial or complete identity to the target molecule sequence. In one embodiment, an siRNA refers to a nucleic acid that has substantial or complete identity to a target gene and forms a double stranded siRNA.

When designing the siRNA molecules, the targeted region often is selected from a given DNA sequence beginning 50 to 100 nucleotides downstream of the start codon. See, *e.g.*, Elbashir et al,. Methods 26:199-213 (2002). Initially, 5' or 3' UTRs and regions nearby the start codon were avoided assuming that UTR-binding proteins and/or translation initiation complexes may interfere with binding of the siRNP or RISC endonuclease complex. Sometimes regions of the target 23 nucleotides in length conforming to the sequence motif AA(N19)TT (N, an nucleotide), and regions with approximately 30% to 70% G/C-content (often about 50% G/C-content) often are selected. If no suitable sequences are found, the search often is extended using the motif NA(N21). The sequence of the sense siRNA sometimes corresponds to (N19) TT or N21 (position 3 to 23 of the 23-nt motif), respectively. In the latter case, the 3' end of the sense siRNA often is converted to TT. The rationale for this sequence conversion is to generate a symmetric duplex with respect to the sequence composition of the sense and antisense 3' overhangs. The antisense siRNA is synthesized as the complement to position 1 to 21 of the 23-nt motif. Because position 1 of the 23-nt motif is not recognized sequence-specifically by the antisense siRNA, the 3'-most nucleotide residue of the antisense siRNA can be chosen deliberately. However, the penultimate nucleotide of the antisense siRNA (complementary to position 2 of the 23-nt motif) often is complementary to the targeted sequence. For simplifying chemical synthesis, TT often is utilized. siRNAs corresponding to the target motif NAR(N17)YNN, where R is purine (A,G) and Y is pyrimidine (C,U), often are selected. Respective 21 nucleotide sense and antisense siRNAs often begin with a purine nucleotide and can also be expressed from pol III expression vectors without a change in targeting site. Expression of RNAs from pol III promoters often is efficient when the first transcribed nucleotide is a purine.

The sequence of the siRNA can correspond to the full length target gene, or a subsequence thereof. Often, the siRNA is about 15 to about 50 nucleotides in length (*e.g.*, each complementary sequence of the double stranded siRNA is 15-50 nucleotides in length, and the double stranded siRNA is about 15-50 base pairs in length, sometimes about 20-30 nucleotides in length or about 20-25 nucleotides in length, *e.g.*, 20, 21, 22, 23, 24, 25, 26, 27, 28, 29, or 30 nucleotides in length. The siRNA sometimes is about 21 nucleotides in length. Methods of using siRNA are well known in the art, and specific siRNA molecules may be purchased from a number of companies including Dharmacon Research, Inc.

Antisense, ribozyme, RNAi and siRNA nucleic acids can be altered to form modified nucleic acid molecules. The nucleic acids can be altered at base moieties, sugar moieties or phosphate backbone moieties to improve stability, hybridization, or solubility of the molecule. For example, the deoxyribose phosphate backbone of nucleic acid molecules can be modified to generate peptide nucleic acids (see Hyrup et al., Bioorganic & Medicinal Chemistry 4 (1): 5-23 (1996)). As used herein, the terms "peptide nucleic acid" or "PNA" refers to a nucleic acid mimic such as a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. Synthesis of PNA oligomers can be performed using standard solid phase peptide synthesis protocols as described, for example, in Hyrup *et al*., (1996) supra and Perry-O'Keefe et al., Proc. Natl. Acad. Sci. 93: 14670-675 (1996).

PNA nucleic acids can be used in prognostic, diagnostic, and therapeutic applications. For example, PNAs can be used as antisense or antigene agents for sequence-specific modulation of gene expression by, for example, inducing transcription or translation arrest or inhibiting replication. PNA nucleic acid molecules can also be used in the analysis of single base pair mutations in a gene, (*e.g.*, by PNA-directed PCR clamping); as "artificial restriction enzymes" when used in combination with other enzymes, (*e.g.,* S1 nucleases (Hyrup (1996) supra)); or as probes or primers for DNA sequencing or hybridization (Hyrup *et al*., (1996) supra; Perry-O'Keefe supra).

In other embodiments, oligonucleotides may include other appended groups such as peptides (*e.g.,* for targeting host cell receptors *in vivo*), or agents facilitating transport across cell membranes (see *e.g*., Letsinger et al., Proc. Natl. Acad. Sci. USA 86: 6553-6556 (1989); Lemaitre et al., Proc. Natl. Acad. Sci. USA 84: 648-652 (1987); PCT Publication No. W088/09810) or the blood-brain barrier (see, *e.g.*, PCT Publication No. W089/10134). In addition, oligonucleotides can be modified with hybridization-triggered cleavage agents (See, *e.g.,* Krol et al., Bio-Techniques 6: 958-976 (1988)) or intercalating agents. (See, *e.g.*, Zon, Pharm. Res. 5: 539-549 (1988)). To this end, the oligonucleotide may be conjugated to another molecule, (*e.g.*, a peptide, hybridization triggered cross-linking agent, transport agent, or hybridization-triggered cleavage agent).

Also included herein are molecular beacon oligonucleotide primer and probe molecules having one or more regions complementary to a nucleotide sequence of SEQ ID NO: 1-12 or a substantially identical sequence thereof, two complementary regions one having a fluorophore and one a quencher such that the molecular beacon is useful for quantifying the presence of the nucleic acid in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

Antibodies

The term "antibody" as used herein refers to an immunoglobulin molecule or immunologically active portion thereof, i.e., an antigen-binding portion. Examples of immunologically active portions of immunoglobulin molecules include F(ab) and $F(ab')_2$ fragments which can be generated by treating the antibody with an enzyme such as pepsin. An antibody sometimes is a polyclonal, monoclonal, recombinant (*e.g.*, a chimeric or humanized), fully human, non-human (*e.g.*, murine), or a single chain antibody. An antibody may have effector function and can fix complement, and is sometimes coupled to a toxin or imaging agent.

A full-length polypeptide or antigenic peptide fragment encoded by a *ICAM, MAPK10, KIAA0861, NUMA1* or *GALE* nucleotide sequence can be used as an immunogen or can be used to identify antibodies made with other immunogens, *e.g*., cells, membrane preparations, and the like. An antigenic peptide often includes at least 8 amino acid residues of the amino acid sequences encoded by a nucleotide sequence of SEQ ID NO: 1-12, or substantially identical sequence thereof, and encompasses an epitope. Antigenic peptides sometimes include 10 or more amino acids, 15 or more amino acids, 20 or more amino acids, or 30 or more amino acids. Hydrophilic and hydrophobic fragments of polypeptides sometimes are used as immunogens.

Epitopes encompassed by the antigenic peptide are regions located on the surface of the polypeptide (*e.g.*, hydrophilic regions) as well as regions with high antigenicity. For example, an Emini surface probability analysis of the human polypeptide sequence can be used to indicate the regions that have a particularly high probability of being localized to the surface of the polypeptide and are thus likely to constitute surface residues useful for targeting antibody production. The antibody may bind an epitope on any domain or region on polypeptides described herein.

Also, chimeric, humanized, and completely human antibodies are useful for applications which include repeated administration to subjects. Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson et al International Application No. PCT/US86/02269; Akira, et al European Patent Application 184,187; Taniguchi, M., European Patent Application 171,496; Morrison et al European Patent Application 173,494; Neuberger et al PCT International Publication No. WO 86/01533; Cabilly et al U.S. Patent No. 4,816,567; Cabilly et al European Patent Application 125,023; Better et al., Science 240: 1041-1043 (1988); Liu et al., Proc. Natl. Acad. Sci. USA 84: 3439-3443 (1987); Liu et al., J. Immunol. 139: 3521-3526 (1987); Sun et al., Proc. Natl. Acad. Sci. USA 84: 214-218 (1987); Nishimura et al., Canc. Res. 47: 999-1005 (1987); Wood et al., Nature 314: 446-449 (1985); and Shaw et al., J. Natl. Cancer Inst. 80: 1553-1559 (1988); Morrison, S. L., Science 229: 1202-1207 (1985); Oi et al., BioTechniques 4: 214 (1986); Winter U.S. Patent 5,225,539; Jones et al., Nature 321: 552-525 (1986); Verhoeyan et al., Science 239: 1534; and Beidler et al., J. Immunol. 141: 4053-4060 (1988).

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. See, for example, Lonberg and Huszar, Int. Rev. Immunol. 13: 65-93 (1995); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above. Completely human antibodies that recognize a selected epitope also can be generated using a technique referred to as "guided selection." In this approach a selected non-human monoclonal antibody (*e.g.*, a murine antibody) is used to guide the selection of a completely human antibody recognizing the same epitope. This technology is described for example by Jespers et al., Bio/Technology 12: 899-903 (1994).

Antibody can be a single chain antibody. A single chain antibody (scFV) can be engineered (see, *e.g.*, Colcher et al., Ann. N Y Acad. Sci. 880: 263-80 (1999); and Reiter, Clin. Cancer Res. 2: 245-52 (1996)). Single chain antibodies can be dimerized or multimerized to generate multivalent antibodies having specificities for different epitopes of the same target polypeptide.

Antibodies also may be selected or modified so that they exhibit reduced or no ability to bind an Fc receptor. For example, an antibody may be an isotype or subtype, fragment or other mutant, which does not support binding to an Fc receptor (*e.g.*, it has a mutagenized or deleted Fc receptor binding region).

Also, an antibody (or fragment thereof) may be conjugated to a therapeutic moiety such as a cytotoxin, a therapeutic agent or a radioactive metal ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1 dehydrotestosterone, glucocorticoids, procaine, tetracaine, lidocaine, propranolol, and puromycin and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thiotepa chlorambucil, melphalan, carmustine (BCNU) and lomustine (CCNU), cyclophosphamide, busulfan, dibromomannitol, streptozotocin, mitomycin C, and cis-dichlorodiamine platinum (II) (DDP) cisplatin), anthracyclines (*e.g.*, daunorubicin (formerly daunomycin) and doxorubicin), antibiotics (*e.g.*, dactinomycin (formerly actinomycin), bleomycin, mithramycin, and anthramycin (AMC)), and anti-mitotic agents (*e.g*, vincristine and vinblastine).

Antibody conjugates can be used for modifying a given biological response. For example, the drug moiety may be a protein or polypeptide possessing a desired biological activity. Such proteins may include, for example, a toxin such as abrin, ricin A, pseudomonas exotoxin, or diphtheria toxin; a polypeptide such as tumor necrosis factor, ?-interferon, a-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Also, an antibody can be conjugated to a second antibody to form an antibody heteroconjugate as described by Segal in U.S. Patent No. 4,676,980, for example.

An antibody (*e.g.*, monoclonal antibody) can be used to isolate target polypeptides by standard techniques, such as affinity chromatography or immunoprecipitation. Moreover, an antibody can be used to detect a target polypeptide (*e.g.*, in a cellular lysate or cell supernatant) in order to evaluate the abundance and pattern of expression of the polypeptide. Antibodies can be used diagnostically to monitor polypeptide levels in tissue as part of a clinical testing procedure, *e.g.*, to determine the efficacy of a given treatment regimen. Detection can be facilitated by coupling (i.e., physically linking) the antibody to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$. Also, an antibody can be utilized as a test molecule for determining whether it can treat breast cancer, and as a therapeutic for administration to a subject for treating breast cancer.

An antibody can be made by immunizing with a purified antigen, or a fragment thereof, *e.g.*, a fragment described herein, a membrane associated antigen, tissues, *e.g.*, crude tissue preparations, whole cells, preferably living cells, lysed cells, or cell fractions.

Included herein are antibodies which bind only a native polypeptide, only denatured or otherwise non-native polypeptide, or which bind both, as well as those having linear or conformational epitopes. Conformational epitopes sometimes can be identified by selecting antibodies that bind to native but not denatured polypeptide. Also featured are antibodies that specifically bind to a polypeptide variant associated with breast cancer.

Screening Assays

Featured herein are methods for identifying a candidate therapeutic for treating breast cancer. The methods comprise contacting a test molecule with a target molecule in a system. A "target molecule" as used herein refers to a nucleic acid of SEQ ID NO: 1-12, a substantially identical nucleic acid thereof, or a fragment thereof, and an encoded polypeptide of the foregoing. The method also comprises determining the presence or absence of an interaction between the test molecule and the target molecule, where the presence of an interaction between the test molecule and the nucleic acid or polypeptide identifies the test molecule as a candidate breast cancer therapeutic. The interaction between the test molecule and the target molecule may be quantified.

Test molecules and candidate therapeutics include, but are not limited to, compounds, antisense nucleic acids, siRNA molecules, ribozymes, polypeptides or proteins encoded by a *ICAM, MAPK10, KIAA0861, NUMA1* or *GALE* nucleic acids, or a substantially identical sequence or fragment thereof, and immunotherapeutics (*e.g.*, antibodies and HLA-presented polypeptide fragments). A test molecule or candidate therapeutic may act as a modulator of target molecule concentration or target molecule function in a system. A "modulator" may agonize (i.e., up-regulates) or antagonize (i.e., downregulates) a target molecule concentration partially or completely in a system by affecting such cellular functions as DNA replication and/or DNA processing (*e.g.*, DNA methylation or DNA repair), RNA transcription and/or RNA processing (*e.g.*, removal of intronic sequences and/or translocation of spliced mRNA from the nucleus), polypeptide production (*e.g.*, translation of the polypeptide from mRNA), and/or polypeptide post-translational modification (*e.g.*, glycosylation, phosphorylation, and proteolysis of pro-polypeptides). A modulator may also agonize or antagonize a biological function of a target molecule partially or completely, where the function may include adopting a certain structural conformation, interacting with one or more binding partners, ligand binding, catalysis (*e.g.*, phosphorylation, dephosphorylation, hydrolysis, methylation, and isomerization), and an effect upon a cellular event (*e.g.*, effecting progression of breast cancer).

As used herein, the term "system" refers to a cell free *in vitro* environment and a cell-based environment such as a collection of cells, a tissue, an organ, or an organism. A system is "contacted" with a test molecule in a variety of manners, including adding molecules in solution and allowing them to interact with one another by diffusion, cell injection, and any administration routes in an animal. As used herein, the term "interaction" refers to an effect of a test molecule on test molecule, where the effect sometimes is binding between the test molecule and the target molecule, and sometimes is an observable change in cells, tissue, or organism.

There are many standard methods for detecting the presence or absence of an interaction between a test molecule and a target molecule. For example, titrametric, acidimetric, radiometric, NMR, monolayer, polarographic, spectrophotometric, fluorescent, and ESR assays probative of a target molecule interaction may be utilized.

In general, an interaction can be determined by labeling the test molecule and/or the *ICAM, MAPK10, KIAA0861, NUMA1* or *GALE* molecule, where the label is covalently or non-covalently attached to the test molecule or *ICAM, MAPK10, KIAA0861, NUMA1* or *GALE* molecule. The label is sometimes a radioactive molecule such as $^{125}I$, $^{131}I$, $^{35}S$ or $^{3}H$, which can be detected by direct counting of radioemission or by scintillation counting. Also, enzymatic labels such as horseradish peroxidase, alkaline phosphatase, or luciferase may be utilized where the enzymatic label can be detected by determining conversion of an appropriate substrate to product. Also, presence or absence of an interaction can be determined without labeling. For example, a microphysiometer (e.g., Cytosensor) is an analytical instrument that measures the rate at which a cell acidifies its environment using a light-addressable potentiometric sensor (LAPS). Changes in this acidification rate can be used as an indication of an interaction between a test molecule and *ICAM, MAPK10, KIAA0861, NUMA1* or *GALE* (McConnell, H. M. et al., Science 257: 1906-1912 (1992)).

In cell-based systems, cells typically include a *ICAM, MAPK10, KIAA0861, NUMA1* or *GALE* nucleic acid or polypeptide or variants thereof and are often of mammalian origin, although the cell can be of any origin. Whole cells, cell homogenates, and cell fractions (e.g., cell membrane fractions) can be subjected to analysis. Where interactions between a test molecule with a *ICAM, MAPK10, KL4A0861, NUMA1* or *GALE* polypeptide or variant thereof are monitored, soluble and/or membrane bound forms of the polypeptide or variant may be utilized. Where membrane-bound forms of the polypeptide are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl-N,N-dimethyl-3-ammonio-1-propane sulfonate.

An interaction between two molecules also can be detected by monitoring fluorescence energy transfer (FET) (see, for example, Lakowicz et al., U.S. Patent No. 5,631,169; Stavrianopoulos et al. U.S. Patent No. 4,868,103). A fluorophore label on a first, "donor" molecule is selected such that its emitted fluorescent energy will be absorbed by a fluorescent label on a second, "acceptor" molecule, which in turn is able to fluoresce due to the absorbed energy. Alternately, the "donor" polypeptide molecule may simply utilize the natural fluorescent energy of tryptophan residues. Labels are chosen that emit different wavelengths of light, such that the "acceptor" molecule label may be differentiated from that of the "donor". Since the efficiency of energy transfer between the labels is related to the distance separating the molecules, the spatial relationship between the molecules can be assessed. In a situation in which binding occurs between the molecules, the fluorescent emission of the "acceptor" molecule label in the assay should be maximal. An FET binding event can be conveniently measured through standard fluorometric detection means well known in the art (e.g., using a fluorimeter).

In another embodiment, determining the presence or absence of an interaction between a test molecule and a *ICAM, MAPK10, KIAA0861, NUMA1* or *GALE* molecule can be effected by using real-time Biomolecular Interaction Analysis (BIA) (see, e.g., Sjolander & Urbaniczk, Anal. Chem. 63: 2338-2345 (1991) and Szabo et al., Curr. Opin. Struct. Biol. 5: 699-705 (1995)). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In another embodiment, the *ICAM, MAPK10, KIAA0861, NUMA1* or *GALE* molecule or test molecules are anchored to a solid phase. The *ICAM, MAPK10, KIAA0861, NUMA1* or *GALE* molecule/test molecule complexes anchored to the solid phase can be detected at the end of the reaction. The target *ICAM, MAPK10, KIAA0861, NUMA1* or *GALE* molecule is often anchored to a solid surface, and the test molecule, which is not anchored, can be labeled, either directly or indirectly, with detectable labels discussed herein.

It may be desirable to immobilize a *ICAM, MAPK10, KIAA0861, NUMA1* or *GALE* molecule, an anti-*ICAM, MAPK10, KIAA0861, NUMA1* or *GALE* antibody, or test molecules to facilitate separation of complexed from uncomplexed forms of *ICAM, MAPK10, KIAA0861, NUMA1* or *GALE* molecules and test molecules, as well as to accommodate automation of the assay. Binding of a test molecule to a *ICAM, MAPK10, KIAA0861, NUMA1* or *GALE* molecule can be accomplished in any vessel suitable for containing the reactants. Examples of such vessels include microtiter plates, test tubes, and micro-centrifuge tubes. In one embodiment, a fusion polypeptide can be provided which adds a domain that allows a *ICAM, MAPK10, KIAA0861, NUMA1* or *GALE* molecule to be bound to a matrix. For example, glutathione-S-transferase/*ICAM, MAPK10, KIAA0861, NUMA1* or *GALE* fusion polypeptides or glutathione-S-transferase/target fusion polypeptides can be adsorbed onto glutathione sepharose beads (Sigma Chemical, St. Louis, MO) or glutathione derivitized microtiter plates, which are then combined with the test compound or the test compound and either the non-adsorbed target polypeptide or *ICAM, MAPK10, KIAA0861, NUMA1* or *GALE* polypeptide, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of *ICAM, MAPK10, KIAA0861, NUMA1* or *GALE* binding or activity determined using standard techniques.

Other techniques for immobilizing a *ICAM, MAPK10, KIAA0861, NUMA1* or *GALE* molecule on matrices include using biotin and streptavidin. For example, biotinylated *ICAM, MAPK10, KIAA0861, NUMA1* or *GALE* polypeptide or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with *ICAM, MAPK10, KIAA0861, NUMA1* or *GALE* polypeptide or test molecules but which do not interfere with binding of the *ICAM, MAPK10, KIAA0861, NUMA1* or *GALE* polypeptide to its test molecule. Such antibodies can be derivitized to the wells of the plate, and unbound target or *ICAM, MAPK10, KIAA0861, NUMA1* or *GALE* polypeptide trapped in the wells by antibody conjugation. Methods for detecting such complexes, in addition to those described above for the GST-immobilized complexes, include immunodetection of complexes using antibodies reactive with the *ICAM, MAPK10, KIAA0861, NUMA1* or *GALE* polypeptide or target molecule, as well as enzyme-linked assays which rely on detecting an enzymatic activity associated with the *ICAM, MAPK10, KIAA0861*, *NUMA1* or *GALE* polypeptide or test molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A. P., Trends Biochem Sci Aug;18(8): 284-7 (1993)); chromatography (gel filtration chromatography, ionexchange chromatography); electrophoresis (see, e.g., Ausubel et al., eds. Current Protocols in Molecular Biology, J. Wiley: New York (1999)); and immunoprecipitation (see, for example, Ausubel, F. et al., eds. Current Protocols in Molecular Biology, J. Wiley: New York (1999)). Such resins and chromatographic techniques are known to one skilled in the art (see, e.g., Heegaard, J Mol. Recognit. Winter; 11(1-6): 141-8 (1998); Hage & Tweed, J. Chromatogr. B Biomed. Sci. Appl. Oct 10; 699 (1-2): 499-525 (1997)). Further, fluorescence energy transfer may also be conveniently utilized, as described herein, to detect binding without further purification of the complex from solution.

In another embodiment, modulators of *ICAM, MAPK10, KIAA0861, NUMA1* or *GALE* expression are identified. For example, a cell or cell free mixture is contacted with a candidate compound and the expression of *ICAM, MAPK10*, *KIAA0861, NUMA1* or *GALE* mRNA or polypeptide evaluated relative to the level of expression of *ICAM, MAPK10, KIAA0861, NUMA1* or *GALE* mRNA or polypeptide in the absence of the candidate compound. When expression of *ICAM, MAPK10, KIAA0861, NUAM1* or *GALE* mRNA or polypeptide is greater in the presence of the candidate compound than in its absence, the candidate compound is identified as a stimulator of *ICAM, MAPK10, KIAA0861, NUMA1* or *GALE* mRNA or polypeptide expression. Alternatively, when expression of *ICAM, MAPK10, KIAA0861, NUMA1* or *GALE* mRNA or polypeptide is less (statistically significantly less) in the presence of the candidate compound than in its absence, the candidate compound is identified as an inhibitor of *ICAM, MAPK10, KIAA0861, NUMA1* or *GALE* mRNA or polypeptide expression. The level of *ICAM, MAPK10, KIAA0861, MUMA1* or *GALE* mRNA or polypeptide expression can be determined by methods described herein for detecting *ICAM, MAPK10, KIAA0861, NUMA1* or *GALE* mRNA or polypeptide.

In another embodiment, binding partners that interact with a *ICAM, MAPK10, KIAA0861, NUMA1* or *GALE* molecule are detected. The *ICAM, MAPK10, KIAA0861, NUMA1* or *GALE* molecules can interact with one or more cellular or extracellular macromolecules, such as polypeptides, in vivo, and these molecules that interact with *ICAM, MAPK10, KIAA0861, NUMA1* or *GALE* molecules are referred to herein as "binding partners." Molecules that disrupt such interactions can be useful in regulating the activity of the target gene product. Such molecules can include, but are not limited to molecules such as antibodies, peptides, and small molecules. Target genes/products for use in this embodiment often are the *ICAM, MAPK10, KIAA0861, NUMA1* or *GALE* genes herein identified. In an alternative embodiment, provided is a method for determining the ability of the test compound to modulate the activity of a *ICAM, MAPK10, KIAA0861, NUMA1* or *GALE* polypeptide through modulation of the activity of a downstream effector of a *ICAM, MAPK10, KIAA0861, NUMA1* or *GALE* target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), e.g., a substrate, a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases where it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

Also, binding partners of *ICAM, MAPK10, KIAA0861, NUMA1* or *GALE* molecules can be identified in a two-hybrid assay or three-hybrid assay (see, e.g., U.S. Patent No. 5,283,317; Zervos et al., Cell 72:223-232 (1993); Madura et al., J. Biol. Chem. 268: 12046-12054 (1993); Bartel et al., Biotechniques 14: 920-924 (1993); Iwabuchi et al., Oncogene 8: 1693-1696 (1993); and Brent WO94/10300), to identify other polypeptides, which bind to or interact with *ICAM, MAPK10, KIAA0861, NUMA1* or *GALE* ("*ICAM, MAPK10, KIAA0861, NUMA1* or *GALE*-binding polypeptides" or "*ICAM, MAPK10, KIAA0861, NUMA1* or *GALE*-bp") and are involved in *ICAM, MAPK10, KIAA0861, NUMA1* or *GALE* activity. Such *ICAM, MAPK10, KIAA0861, NUMA1* or *GALE*-bps can be activators or inhibitors of signals by the *ICAM, MAPK10, KIAA0861, NUMA1* or *GALE* polypeptides or *ICAM, MAPK10, KIAA0861, NUMA1* or *GALE* targets as, for example, downstream elements of a *ICAM, MAPK10, KIAA0861, NUMA1* or *GALE*-mediated signaling pathway.

A two-hybrid system is based on the modular nature of most transcription factors, which consist of separable DNA-binding and activation domains. Briefly, the assay utilizes two different DNA constructs. In one construct, the gene that codes for a *ICAM, MAPK10, KIAA0861, NUMA1* or *GALE* polypeptide is fused to a gene encoding the DNA binding domain of a known transcription factor (e.g., GAL-4). In the other construct, a DNA sequence, from a library of DNA sequences, that encodes an unidentified polypeptide ("prey" or "sample") is fused to a gene that codes for the activation domain of the known transcription factor. (Alternatively the: *ICAM, MAPK10, KIAA0861, NUMA1* or *GALE* polypeptide can be the fused to the activator domain.) If the "bait" and the "prey" polypeptides are able to interact, in vivo, forming a *ICAM, MAPK10, KIAA0861, NUMA1* or GALE-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the polypeptide which interacts with the *ICAM, MAPK10, KIAA0861, NUMA1* or *GALE* polypeptide.

Candidate therapeutics for treating breast cancer are identified from a group of test molecules that interact with a *ICAM, MAPK10, KIAA0861, NUMA1* or *GALE* nucleic acid or polypeptide. Test molecules are normally ranked according to the degree with which they interact or modulate (e.g., agonize or antagonize) DNA replication and/or processing, RNA transcription and/or processing, polypeptide production and/or processing, and/or function of *ICAM, MAPK10, KIAA0861, NUMA1* or *GALE* molecules, for example, and then top ranking modulators are selected. In a preferred embodiment, the candidate therapeutic (i.e., test molecule) acts as a *ICAM, MAPK10, KIAA0861, NUAM1* or *GALE* antagonist. Also, pharmacogenomic information described herein can determine the rank of a modulator. Candidate therapeutics typically are formulated for administration to a subject.

Therapeutic Treatments

Formulations or pharmaceutical compositions typically include in combination with a pharmaceutically acceptable carrier, a compound, an antisense nucleic acid, a ribozyme, an antibody, a binding partner that interacts with a *ICAM, MAPK10, KIAA0861, NUMA1* or *GALE* polypeptide, a *ICAM, MAPK10, KIAA0861, NUMA1* or *GALE* nucleic acid, or a fragment thereof. The formulated molecule may be one that is identified by a screening method described above. As used herein, the term "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, isotonic agents, for example, sugars, polyalcohols such as mannitol, sorbitol, sodium chloride sometimes are included in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, methods of preparation often utilized are vacuum drying and freeze-drying which yields a powder of the active ingredient plus any additional desired ingredient from a previously sterile-filtered solution thereof.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. Molecules can also be prepared in the form of suppositories (e.g., with conventional suppository bases such as cocoa butter and other glycerides) or retention enemas for rectal delivery.

In one embodiment, active molecules are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. Materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Molecules which exhibit high therapeutic indices often are utilized. While molecules that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such molecules often lies within a range of circulating concentrations that include the $ED_{50}$ with little or no toxicity. The dosage may vary within this range depending upon the dosage form employed and the route of administration utilized. For any molecules used in the methods described herein, the therapeutically effective dose can be estimated initially from cell culture assays. A dose may be formulated in animal models to achieve a circulating plasma concentration range that includes the $IC_{50}$ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, sometimes about 0.01 to 25 mg/kg body weight, often about 0.1 to 20 mg/kg body weight, and more often about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, sometimes between 2 to 8 weeks, often between about 3 to 7 weeks, and more often for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment, or sometimes can include a series of treatments.

With regard to polypeptide formulations, featured herein is a method for treating breast cancer in a subject, which comprises contacting one or more cells in the subject with a first *ICAM, MAPK10, KIAA0861, NUMA1* or *GALE* polypeptide, where the subject comprises a second *ICAM, MAPK10, KIAA0861, NUMA1* or *GALE* polypeptide having one or more polymorphic variations associated with cancer, and where the first polypeptide comprises fewer polymorphic variations associated with cancer than the second polypeptide. The first and second polypeptides are encoded by a nucleic acid which comprises a nucleotide sequence selected from the group consisting of the nucleotide sequence of SEQ ID NO: 1-12; a nucleotide sequence which encodes a polypeptide consisting of an amino acid sequence encoded by a nucleotide sequence of SEQ ID NO: 1-12; a nucleotide sequence which encodes a polypeptide that is 90% or more identical to an amino acid sequence encoded by a nucleotide sequence of SEQ ID NO: 1-12 and a nucleotide sequence 90% or more identical to a nucleotide sequence of SEQ ID NO: 1-12. The subject is often a human.

For antibodies, a dosage of 0.1 mg/kg of body weight (generally 10 mg/kg to 20 mg/kg) is often utilized. If the antibody is to act in the brain, a dosage of 50 mg/kg to 100 mg/kg is often appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al., J. Acquired Immune Deficiency Syndromes and Human Retrovirology 14:193 (1997).

Antibody conjugates can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a polypeptide such as tumor necrosis factor, .alpha.-interferon, beta.-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors. Alternatively, an antibody can be conjugated to a second antibody to form an antibody heteroconjugate as described by Segal in U.S. Patent No. 4,676,980.

For compounds, exemplary doses include milligram or microgram amounts of the compound per kilogram of subject or sample weight, for example, about 1 microgram per kilogram to about 500 milligrams per kilogram, about 100 micrograms per kilogram to about 5 milligrams per kilogram, or about 1 microgram per kilogram to about 50 micrograms per kilogram. It is understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid described herein, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

*ICAM, MAPK10, KIAA0861, NUMA1* or *GALE* nucleic acid molecules can be inserted into vectors and used in gene therapy methods for treating breast cancer. Featured herein is a method for treating breast cancer in a subject, which comprises contacting one or more cells in the subject with a first *ICAM, MAPK10, KIAA0861, NUMA1* or *GALE* nucleic acid, where genomic DNA in the subject comprises a second *ICAM, MAPK10, KIAA0861, NUMA1* or *GALE* nucleic acid comprising one or more polymorphic variations associated with breast cancer, and where the first nucleic acid comprises fewer polymorphic variations associated with breast cancer. The first and second nucleic acids typically comprise a nucleotide sequence selected from the group consisting of the nucleotide sequence of SEQ ID NO: 1-5; a nucleotide sequence which encodes a polypeptide consisting of an amino acid sequence encoded by a nucleotide sequence in SEQ ID NO: 1-5; a nucleotide sequence that is 90% or more identical to the nucleotide sequence of SEQ ID NO: 1-5, and a nucleotide sequence which encodes a polypeptide that is 90% or more identical to an amino acid sequence encoded by a nucleotide sequence in SEQ ID NO: 1-5. The subject often is a human.

Gene therapy vectors can be delivered to a subject by, for example, intravenous injection, local administration (see U.S. Patent 5,328,470) or by stereotactic injection (see e.g., Chen et al., (1994) Proc. Natl. Acad. Sci. USA 91: 3054-3057). Pharmaceutical preparations of gene therapy vectors can include a gene therapy vector in an acceptable diluent, or can comprise a slow release matrix in which the gene delivery vehicle is imbedded. Alternatively, where the complete gene delivery vector can be produced intact from recombinant cells (e.g., retroviral vectors) the pharmaceutical preparation can include one or more cells which produce the gene delivery system. Examples of gene delivery vectors are described herein.

Pharmaceutical compositions can be included in a container, pack, or dispenser together with instructions for administration.

Pharmaceutical compositions of active ingredients can be administered by any of the paths described herein for therapeutic and prophylactic methods for treating breast cancer. With regard to both prophylactic and therapeutic methods of treatment, such treatments may be specifically tailored or modified, based on knowledge obtained from pharmacogenomic analyses described herein. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

Administration of a prophylactic agent can occur prior to the manifestation of symptoms characteristic of the *ICAM, MAPK10, KIAA0861, NUMA1* or *GALE* aberrance, such that a disease or disorder is prevented or, alternatively, delayed in its progression. Depending on the type of *ICAM, MAPK10, KIAA0861, NUMA1* or *GALE* aberrance, for example, a *ICAM, MAPK10, KIAA0861, NUMA1* or *GALE* molecule, *ICAM, MAPK10, KIAA0861, NUMA1* or *GALE* agonist, or *ICAM, MAPK10, KIAA0861, NUMA1* or *GALE* antagonist agent can be used for treating the subject. The appropriate agent can be determined based on screening assays described herein.

As discussed, successful treatment of *ICAM, MAPK10, KIAA0861, NUMA1* or *GALE* disorders can be brought about by techniques that serve to inhibit the expression or activity of target gene products. For example, compounds (e.g., an agent identified using an assays described above) that exhibit negative modulatory activity can be used to prevent and/or treat breast cancer. Such molecules can include, but are not limited to peptides, phosphopeptides, small organic or inorganic molecules, or antibodies (including, for example, polyclonal, monoclonal, humanized, anti-idiotypic, chimeric or single chain antibodies, and FAb, F(ab')2 and FAb expression library fragments, scFV molecules, and epitope-binding fragments thereof).

Further, antisense and ribozyme molecules that inhibit expression of the target gene can also be used to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances where the target gene encodes an extracellular polypeptide, normal target gene polypeptide often is co-administered into the cell or tissue to maintain the requisite level of cellular or tissue target gene activity.

Another method by which nucleic acid molecules may be utilized in treating or preventing a disease characterized by *ICAM, MAPK10, KIAA0861, NUMA1* or *GALE* expression is through the use of aptamer molecules specific for *ICAM, MAPK10, KIAA0861, NUMA1* or *GALE* polypeptide. Aptamers are nucleic acid molecules having a tertiary structure which permits them to specifically bind to polypeptide ligands (see, e.g., Osborne, et al., Curr. Opin. Chem. Biol.1(1): 5-9 (1997); and Patel, D. J., Curr. Opin. Chem. Biol. Jun;1(1): 32-46 (1997)). Since nucleic acid molecules may in many cases be more conveniently introduced into target cells than therapeutic polypeptide molecules may be, aptamers offer a method by which *ICAM, MAPK10, KIAA0861, NUMA1* or *GALE* polypeptide activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of *ICAM, MAPK10, KIAA0861, NUMA1* or *GALE* disorders. For a description of antibodies, see the Antibody section above.

In circumstances where injection of an animal or a human subject with a *ICAM, MAPK10, KIAA0861, NUMA1* or *GALE* polypeptide or epitope for stimulating antibody production is harmful to the subject, it is possible to generate an immune response against *ICAM, MAPK10, KIAA0861, NUMA1* or *GALE* through the use of anti-idiotypic antibodies (see, for example, Herlyn, D., Ann. Med.;31(1): 66-78 (1999); and Bhattacharya-Chatterjee & Foon, Cancer Treat. Res.; 94: 51-68 (1998)). If an anti-idiotypic antibody is introduced into a mammal or human subj ect, it should stimulate the production of anti-anti-idiotypic antibodies, which should be specific to the *ICAM, MAPK10, KIAA0861, NUMA1* or *GALE* polypeptide. Vaccines directed to a disease characterized by *ICAM, MAPK10, KIAA0861, NUMA1* or *GALE* expression may also be generated in this fashion.

In instances where the target antigen is intracellular and whole antibodies are used, internalizing antibodies may be utilized. Lipofectin or liposomes can be used to deliver the antibody or a fragment of the Fab region that binds to the target antigen into cells. Where fragments of the antibody are used, the smallest inhibitory fragment that binds to the target antigen often is utilized. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco et al., Proc. Natl. Acad. Sci. USA 90: 7889-7893 (1993)).

*ICAM, MAPK10, KIAA0861, NUMA1* or *GALE* molecules and compounds that inhibit target gene expression, synthesis and/or activity can be administered to a patient at therapeutically effective doses to prevent, treat or ameliorate *ICAM, MAPK10, KIAA0861, NUMA1* or *GALE* disorders. A therapeutically effective dose refers to that amount of the compound sufficient to result in amelioration of symptoms of the disorders.

Toxicity and therapeutic efficacy of such compounds can be determined by standard pharmaceutical procedures in cell cultures or experimental animals, e.g., for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Compounds that exhibit large therapeutic indices often are utilized. While compounds that exhibit toxic side effects can be used, care should be taken to design a delivery system that targets such compounds to the site of affected tissue in order to minimize potential damage to uninfected cells and, thereby, reduce side effects.

Data obtained from cell culture assays and animal studies can be used in formulating a range of dosage for use in humans. The dosage of such compounds often lies within a range of circulating concentrations that include the $ED_{50}$ with little or no toxicity. The dosage can vary within this range depending upon the dosage form employed and the route of administration utilized. For any compound used in a method described herein, the therapeutically effective dose can be estimated initially from cell culture assays. A dose can be formulated in animal models to achieve a circulating plasma concentration range that includes the $IC_{50}$ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of effective dose determination for an individual is the ability to directly assay levels of "free" and "bound" compound in the serum of the test subject. Such assays may utilize antibody mimics and/or "biosensors" that have been created through molecular imprinting techniques. The compound which is able to modulate *ICAM, MAPK10, KIAA0861, NUMA1* or *GALE* activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell et al., Current Opinion in Biotechnology 7: 89-94 (1996) and in Shea, Trends in Polymer Science 2: 166-173 (1994). Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, et al., Nature 361: 645-647 (1993). Through the use of isotope-labeling, the "free" concentration of compound which modulates the expression or activity of *ICAM, MAPK10, KIAA0861, NUMA1* or *GALE* can be readily monitored and used in calculations of $IC_{50}$. Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual $IC_{50}$. A rudimentary example of such a "biosensor" is discussed in Kriz et al., Analytical Chemistry 67: 2142-2144 (1995).

Provided herein are methods of modulating *ICAM, MAPK10, KIAA0861, NUMA1* or *GALE* expression or activity for therapeutic purposes. Accordingly, in an exemplary embodiment, the modulatory method involves contacting a cell with a *ICAM, MAPK10, KIAA0861, NUMA1* or *GALE* or agent that modulates one or more of the activities of *ICAM, MAPK10, KIAA0861, NUMA1* or *GALE* polypeptide activity associated with the cell. An agent that modulates *ICAM, MAPK10, KIAA0861, NUMA1* or *GALE* polypeptide activity can be an agent as described herein, such as a nucleic acid or a polypeptide, a naturally-occurring target molecule of a *ICAM, MAPK10, KIAA0861, MUMA1* or *GALE* polypeptide (e.g., a *ICAM, MAPK10, KIAA0861, NUMA1* or *GALE* substrate or receptor), a *ICAM, MAPK10, KIAA0861, NUMA1* or *GALE* antibody, a *ICAM, MAPK10, KIAA0861, NUMA1* or *GALE* agonist or antagonist, a peptidomimetic of a *ICAM, MAPK10, KIAA0861, NUMA1* or *GALE* agonist or antagonist, or other small molecule.

In one embodiment, the agent stimulates one or more *ICAM, MAPK10, KIAA0861, NUMA1* or *GALE* activities. Examples of such stimulatory agents include active *ICAM*, *MAPK10, KIAA0861, NUMA1* or *GALE* polypeptide and a nucleic acid molecule encoding *ICAM, MAPK10, KIAA0861, NUMA1* or *GALE.* In another embodiment, the agent inhibits one or more *ICAM, MAPK10, KIAA0861, NUMA1* or *GALE* activities. Examples of such inhibitory agents include antisense *ICAM, MAPK10, KIAA0861, NUMA1* or *GALE* nucleic acid molecules, anti-*ICAM, MAPK10, KIAA0861, NUMA1* or *GALE* antibodies, and *ICAM, MAPK10, KIAA0861, NUMA1* or *GALE* inhibitors, and competitive inhibitors that target *ICAM, MAPK10, KIAA0861, NUMA1* or *GALE.* These modulatory methods can be performed in vitro (e.g., by culturing the cell with the agent) or, alternatively, in vivo (e.g., by administering the agent to a subject). As such, provided are methods of treating an individual afflicted with a disease or disorder characterized by aberrant or unwanted expression or activity of a *ICAM, MAPK10, KIAA0861, NUMA1* or *GALE* polypeptide or nucleic acid molecule. In one embodiment, the method involves administering an agent (e.g., an agent identified by a screening assay described herein), or combination of agents that modulates (e.g., upregulates or downregulates) *ICAM, MAPK10, KIAA0861, NUMA1* or *GALE* expression or activity. In another embodiment, the method involves administering a *ICAM, MAPK10, KL4A0861, NUMA1* or *GALE* polypeptide or nucleic acid molecule as therapy to compensate for reduced, aberrant, or unwanted *ICAM, MAPK10, KIAA0861, NUMA1* or *GALE* expression or activity.

Stimulation of *ICAM, MAPK10, KIAA0861, NUMA1* or *GALE* activity is desirable in situations in which *ICAM, MAPK10, KIAA0861, NUMA1* or *GALE* is abnormally downregulated and/or in which increased *ICAM, MAPK10, KIAA0861, NUMA1* or *GALE* activity is likely to have a beneficial effect. For example, stimulation of *ICAM, MAPK10, KIAA0861, NUMA1* or *GALE* activity is desirable in situations in which a *ICAM, MAPK10, KIAA0861, NUMA1* or *GALE* is downregulated and/or in which increased *ICAM, MAPK10, KIAA0861, NUMA1* or *GALE* activity is likely to have a beneficial effect. Likewise, inhibition of *ICAM, MAPK10, KIAA0861, NUMA1* or *GALE* activity is desirable in situations in which *ICAM, MAPK10, KIAA0861, NUMA1* or *GALE* is abnormally upregulated and/or in which decreased *ICAM, MAPK10, KIAA0861, NUMA1* or *GALE* activity is likely to have a beneficial effect.

Methods of Treatment

In another aspect, provided are methods for identifying a risk of cancer in an individual as described herein and, if a genetic predisposition is identified, treating that individual to delay or reduce or prevent the development of cancer. Such a procedure can be used to treat breast cancer. Optionally, treating an individual for cancer may include inhibiting cellular proliferation, inhibiting metastasis, inhibiting invasion, or preventing tumor formation or growth as defined herein. Suitable treatments to prevent or reduce or delay breast cancer focus on inhibiting additional cellular proliferation, inhibiting metastasis, inhibiting invasion, and preventing further tumor formation or growth. Treatment usually includes surgery followed by radiation therapy. Surgery may be a lumpectomy or a mastectomy (e.g., total, simple or radical). Even if the doctor removes all of the cancer that can be seen at the time of surgery, the patient may be given radiation therapy, chemotherapy, or hormone therapy after surgery to try to kill any cancer cells that may be left. Radiation therapy is the use of x-rays or other types of radiation to kill cancer cells and shrink tumors. Radiation therapy may use external radiation (using a machine outside the body) or internal radiation. Chemotherapy is the use of drugs to kill cancer cells. Chemotherapy may be taken by mouth, or it may be put into the body by inserting a needle into a vein or muscle. Hormone therapy often focuses on estrogen and progesterone, which are hormones that affect the way some cancers grow. If tests show that the cancer cells have estrogen and progesterone receptors (molecules found in some cancer cells to which estrogen and progesterone will attach), hormone therapy is used to block the way these hormones help the cancer grow. Hormone therapy with tamoxifen is often given to patients with early stages of breast cancer and those with metastatic breast cancer. Other types of treatment being tested in clinical trials include sentinel lymph node biopsy followed by surgery and high-dose chemotherapy with bone marrow transplantation and peripheral blood stem cell transplantation. Any preventative/therapeutic treatment known in the art may be prescribed and/or administered, including, for example, surgery, chemotherapy and/or radiation treatment, and any of the treatments may be used in combination with one another to treat or prevent breast cancer (e.g., surgery followed by radiation therapy).

Also provided are methods of preventing or treating cancer comprising providing an individual in need of such treatment with a *ICAM, MAPK10, KIAA0861, NUMA1* or *GALE* inhibitor that reduces or inhibits the overexpression of mutant *ICAM, MAPK10, KIAA0861, NUMA1* or *GALE* (e.g., a *ICAM, MAPK10, KIAA0861, NUMA1* or *GALE* polynucleotide with an allele that is associated with cancer). Included herein are methods of reducing or blocking the expression of *ICAM, MAPK10, KIAA0861, NUMA1* or *GALE* comprising providing or administering to individuals in need of reducing or blocking the expression of *ICAM, MAPK10, KIAA0861, NUMA1* or *GALE* a pharmaceutical or physiologically acceptable composition comprising a molecule capable of inhibiting expression of *ICAM, MAPK19, KIAA0861, NUMA1* or *GALE,* e.g., a siRNA molecule. Also included herein are methods of reducing or blocking the expression of secondary regulatory genes regulated by *ICAM, MAPK10, KIAA0861, NUMA1* or *GALE* that play a role in oncogenesis which comprises introducing competitive inhibitors that target *ICAM, MAPK10, KIAA0861, NUMA1* or *GALE*'s effect on these regulatory genes or that block the binding of positive factors necessary for the expression of these regulatory genes.

The examples set forth below are intended to illustrate but not limit the invention.

## Examples

In the following studies a group of subjects were selected according to specific parameters relating to breast cancer. Nucleic acid samples obtained from individuals in the study group were subjected to genetic analysis, which identified associations between breast cancer and certain polymorphic variants in *ICAM* region, *MAPK10, KIAA0861, NUMA1*/*FLJ20625*/*LOC220074* region, and *HT014*/*LOC148902*/*LYPLA2*/*GALE* region loci (herein referred to as "target genes", "target nucleotides", "target polypeptides" or simply "targets"). In addition, methods are described for combining information from multiple SNPs from the target genes found to be independently associated with breast cancer status in a case-control study. The resulting model permits a powerful, more informative quantitation of the combined value of the SNPs for predicting breast cancer susceptibility.

### Example 1

### Samples and Pooling Strategies

### Sample Selection

Blood samples were collected from individuals diagnosed with breast cancer, which were referred to as case samples. Also, blood samples were collected from individuals not diagnosed with breast cancer as gender and age-matched controls. All of the samples were of German/German descent. A database was created that listed all phenotypic trait information gathered from individuals for each case and control sample. Genomic DNA was extracted from each of the blood samples for genetic analyses.

### DNA Extraction from Blood Samples

Six to ten milliliters of whole blood was transferred to a 50 ml tube containing 27 ml of red cell lysis solution (RCL). The tube was inverted until the contents were mixed. Each tube was incubated for 10 minutes at room temperature and inverted once during the incubation. The tubes were then centrifuged for 20 minutes at 3000 x g and the supernatant was carefully poured off. 100-200 μl of residual liquid was left in the tube and was pipetted repeatedly to resuspend the pellet in the residual supernatant. White cell lysis solution (WCL) was added to the tube and pipetted repeatedly until completely mixed. While no incubation was normally required, the solution was incubated at 37°C or room temperature if cell clumps were visible after mixing until the solution was homogeneous. 2 ml of protein precipitation was added to the cell lysate. The mixtures were vortexed vigorously at high speed for 20 sec to mix the protein precipitation solution uniformly with the cell lysate, and then centrifuged for 10 minutes at 3000 x g. The supernatant containing the DNA was then poured into a clean 15 ml tube, which contained 7 ml of 100% isopropanol. The samples were mixed by inverting the tubes gently until white threads of DNA were visible. Samples were centrifuged for 3 minutes at 2000 x g and the DNA was visible as a small white pellet. The supernatant was decanted and 5 ml of 70% ethanol was added to each tube. Each tube was inverted several times to wash the DNA pellet, and then centrifuged for 1 minute at 2000 x g. The ethanol was decanted and each tube was drained on clean absorbent paper. The DNA was dried in the tube by inversion for 10 minutes, and then 1000 μl of 1X TE was added. The size of each sample was estimated, and less TE buffer was added during the following DNA hydration step if the sample was smaller. The DNA was allowed to rehydrate overnight at room temperature, and DNA samples were stored at 2-8°C.

DNA was quantified by placing samples on a hematology mixer for at least 1 hour. DNA was serially diluted (typically 1:80, 1:160, 1:320, and 1:640 dilutions) so that it would be within the measurable range of standards. 125 μl of diluted DNA was transferred to a clear U-bottom microtitre plate, and 125 μl of 1X TE buffer was transferred into each well using a multichannel pipette. The DNA and 1X TE were mixed by repeated pipetting at least 15 times, and then the plates were sealed. 50 μl of diluted DNA was added to wells A5-H12 of a black flat bottom microtitre plate. Standards were inverted six times to mix them, and then 50 μl of 1X TE buffer was pipetted into well A1, 1000 ng/ml of standard was pipetted into well A2, 500 ng/ml of standard was pipetted into well A3, and 250 ng/ml of standard was pipetted into well A4. PicoGreen (Molecular Probes, Eugene, Oregon) was thawed and freshly diluted 1:200 according to the number of plates that were being measured. PicoGreen was vortexed and then 50μl was pipetted into all wells of the black plate with the diluted DNA. DNA and PicoGreen were mixed by pipetting repeatedly at least 10 times with the multichannel pipette. The plate was placed into a Fluoroskan Ascent Machine (microplate fluorometer produced by Labsystems) and the samples were allowed to incubate for 3 minutes before the machine was run using filter pairs 485 nm excitation and 538 nm emission wavelengths. Samples having measured DNA concentrations of greater than 450 ng/μl were re-measured for conformation. Samples having measured DNA concentrations of 20 ng/μl or less were re-measured for confirmation.

Pooling Strategies

Samples were placed into one of two groups based on disease status. The two groups were female case groups and female control groups. A select set of samples from each group were utilized to generate pools, and one pool was created for each group. Each individual sample in a pool was represented by an equal amount of genomic DNA. For example, where 25 ng of genomic DNA was utilized in each PCR reaction and there were 200 individuals in each pool, each individual would provide 125 pg of genomic DNA. Inclusion or exclusion of samples for a pool was based upon the following criteria: the sample was derived from an individual characterized as Caucasian; the sample was derived from an individual of German paternal and maternal descent; the database included relevant phenotype information for the individual; case samples were derived from individuals diagnosed with breast cancer; control samples were derived from individuals free of cancer and no family history of breast cancer; and sufficient genomic DNA was extracted from each blood sample for all allelotyping and genotyping reactions performed during the study. Phenotype information included pre- or post-menopausal, familial predisposition, country or origin of mother and father, diagnosis with breast cancer (date of primary diagnosis, age of individual as of primary diagnosis, grade or stage of development, occurrence of metastases, e.g., lymph node metastases, organ metastases), condition of body tissue (skin tissue, breast tissue, ovary tissue, peritoneum tissue and myometrium), method of treatment (surgery, chemotherapy, hormone therapy, radiation therapy). Samples that met these criteria were added to appropriate pools based on gender and disease status.

The selection process yielded the pools set forth in Table 1, which were used in the studies that follow:

**Table 1**

| | **Female CASE** | **Female CONTROL** |
|---|---|---|
| **Pool size** (Number) | 272 | 276 |
| **Pool Criteria** (ex: case/control) | case | control |
| **Mean Age** (ex: years) | 59.6 | 55.4 |

Example 2

Association of Polymorphic Variants with Breast cancer

A whole-genome screen was performed to identify particular SNPs associated with occurrence of breast cancer. As described in Example 1, two sets of samples were utilized, which included samples from female individuals having breast cancer (breast cancer cases) and samples from female individuals not having cancer (female controls). The initial screen of each pool was performed in an allelotyping study, in which certain samples in each group were pooled. By pooling DNA from each group, an allele frequency for each SNP in each group was calculated. These allele frequencies were then compared to one another. Particular SNPs were considered as being associated with breast cancer when allele frequency differences calculated between case and control pools were statistically significant. SNP disease association results obtained from the allelotyping study were then validated by genotyping each associated SNP across all samples from each pool. The results of the genotyping were then analyzed, allele frequencies for each group were calculated from the individual genotyping results, and a p-value was calculated to determine whether the case and control groups had statistically significantly differences in allele frequencies for a particular SNP. When the genotyping results agreed with the original allelotyping results, the SNP disease association was considered validated at the genetic level.

SNP Panel Used for Genetic Analyses

A whole-genome SNP screen began with an initial screen of approximately 25,000 SNPs over each set of disease and control samples using a pooling approach. The pools studied in the screen are described in Example 1. The SNPs analyzed in this study were part of a set of 25,488 SNPs confirmed as being statistically polymorphic as each is characterized as having a minor allele frequency of greater than 10%. The SNPs in the set reside in genes or in close proximity to genes, and many reside in gene exons. Specifically, SNPs in the set are located in exons, introns, and within 5,000 base-pairs upstream of a transcription start site of a gene. In addition, SNPs were selected according to the following criteria: they are located in ESTs; they are located in Locuslink or Ensemble genes; and they are located in Genomatix promoter predictions. SNPs in the set also were selected on the basis of even spacing across the genome, as depicted in Table 2.

A case-control study design using a whole genome association strategy involving approximately 28,000 single nucleotide polymorphisms (SNPs) was employed. Approximately 25,000 SNPs were evenly spaced in gene-based regions of the human genome with a median inter-marker distance of about 40,000 base pairs. Additionally, approximately 3,000 SNPs causing amino acid substitutions in genes described in the literature as candidates for various diseases were used. The case-control study samples were of female German origin (German paternal and maternal descent) 548 individuals were equally distributed in two groups (female controls and female cases). The whole genome association approach was first conducted on 2 DNA pools representing the 2 groups. Significant markers were confirmed by individual genotyping.

**Table 2**

| General Statistics | | Spacing Statistics | |
|---|---|---|---|
| Total # of SNPs | 25,488 | Median | 37,058 bp |
| # of Exonic SNPs | >4,335 (17%) | Minimum* | 1,000 bp |
| # SNPs with refSNP ID | 20,776 (81 %) | Maximum* | 3,000,000 bp |
| Gene Coverage | >10,000 | Mean | 122,412 bp |
| Chromosome Coverage | All | Std Deviation | 373,325 bp |
| | | **Excludes outliers* | |

Allelotyping and Genotyping Results

The genetic studies summarized above and described in more detail below identified allelic variants associated with breast cancer. The allelic variants identified from the SNP panel described in Table 2 are summarized below in Table 3.

**Table 3**

| SNP Reference | Chromosome Position | Position in Figure | Contig Identification | Contig Position | Sequence Identification | Locus | Sequence Position | Allelic Variability |
|---|---|---|---|---|---|---|---|---|
| 1056538 | 10248147 | 44247 | NT_011295 | | NM_000201 | ICAM region | | C/T |
| 1541998 | 87342924 | 36424 | NT_016354 | 11444849 | NM_002753 | MAPK10 | intragenic | C/T |
| 2001449 | 184330963 | 48563 | NT_005962 | 18141399 | NM_015078 | KIAA0861 | intragenic | G/C |
| 673478 | 72021802 | 49002 | NT_033927 | 1998133 | NM_006185 | NUMA1 | | T/C |
| | | | | | NM_017907 | FLJ20625 | downstream | |
| | | | | | NM_145309 | LOC220074 | | |
| 4237 | 10291777 | 87877 | NT_004391 | 454476 | NM_000403 | GALE | downstream | A/G |
| | | | NT_004610 | | NM_020362 | HT014 | | |
| | | | NO. INFO. | | NO INFO. | LOC148902 | | |
| | | | NT_004610 | | NM_007260 | LYPLA2 | | |

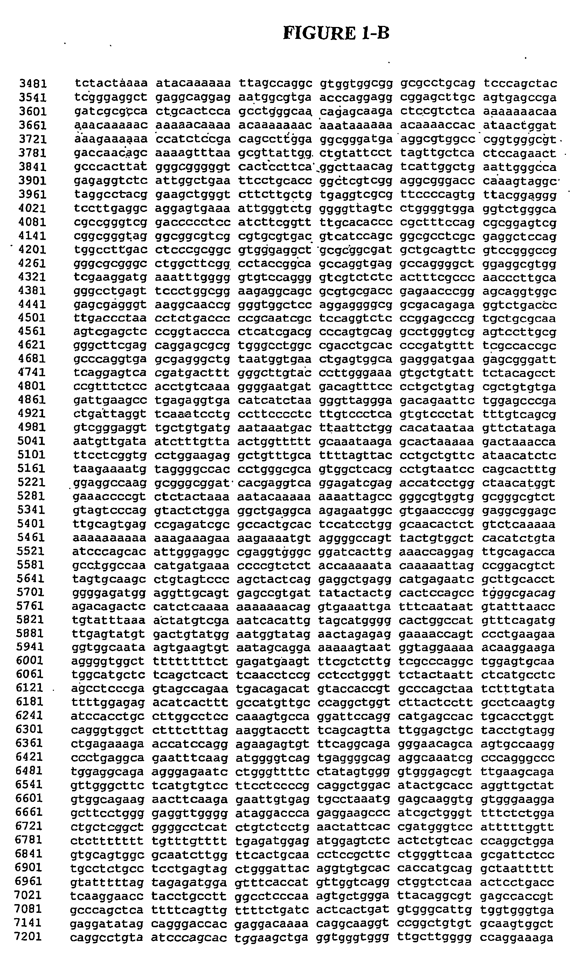

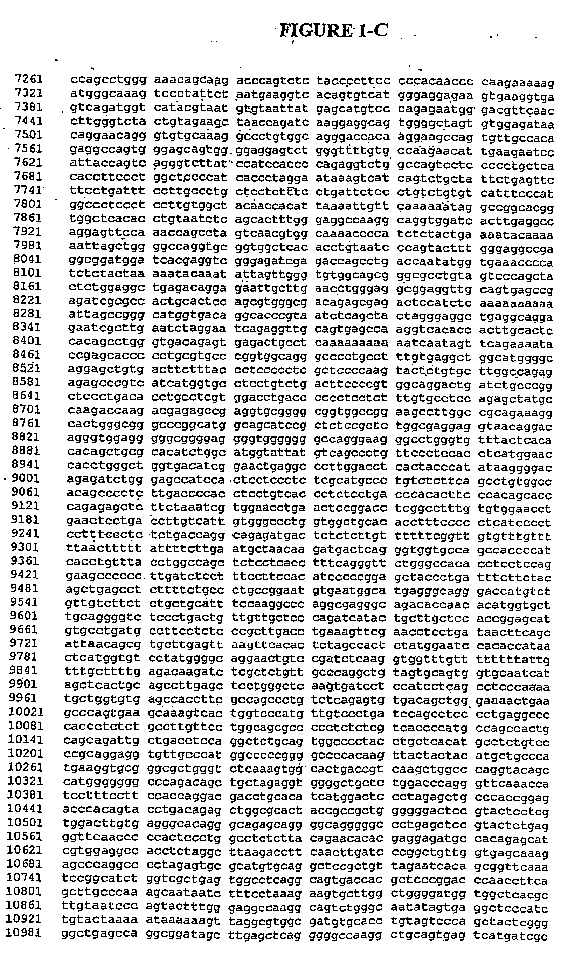

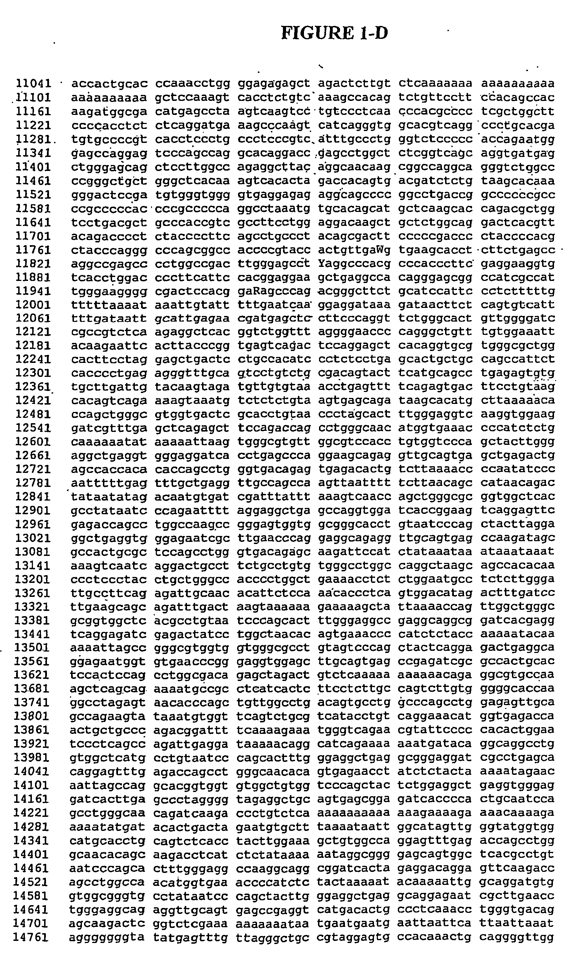

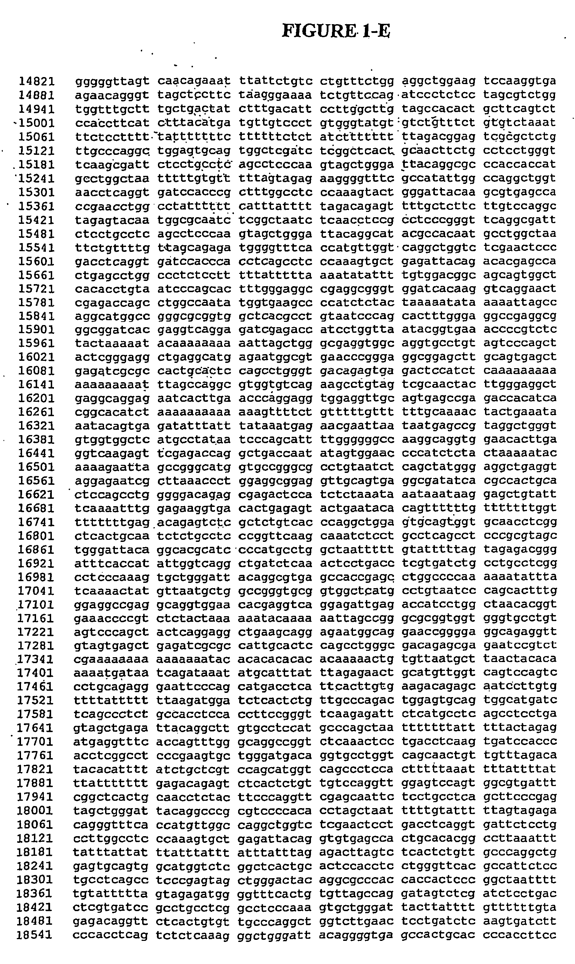

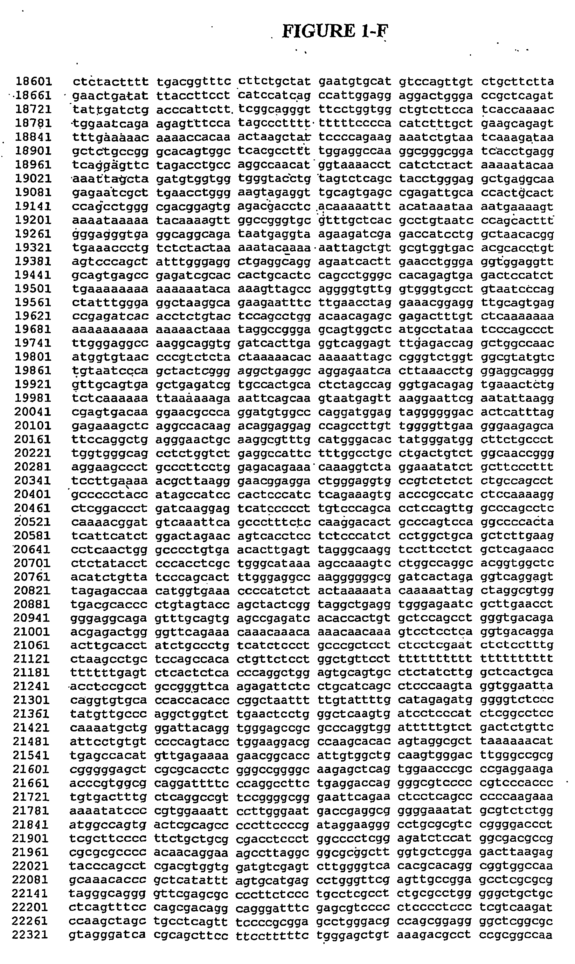

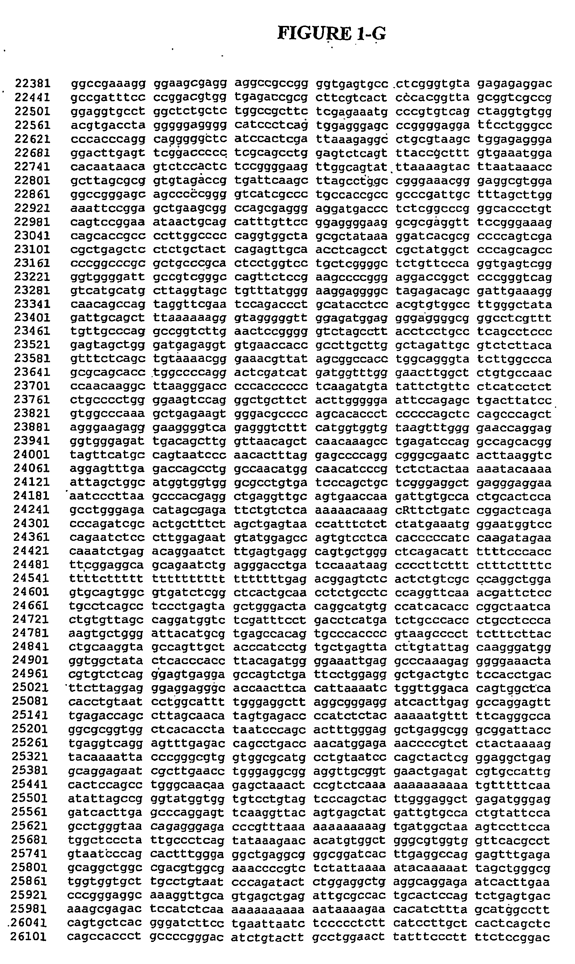

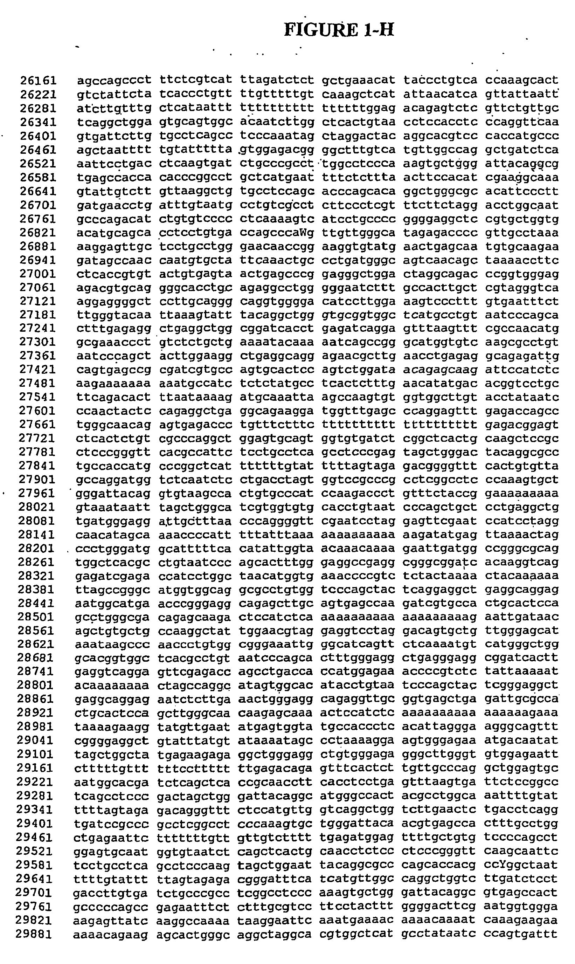

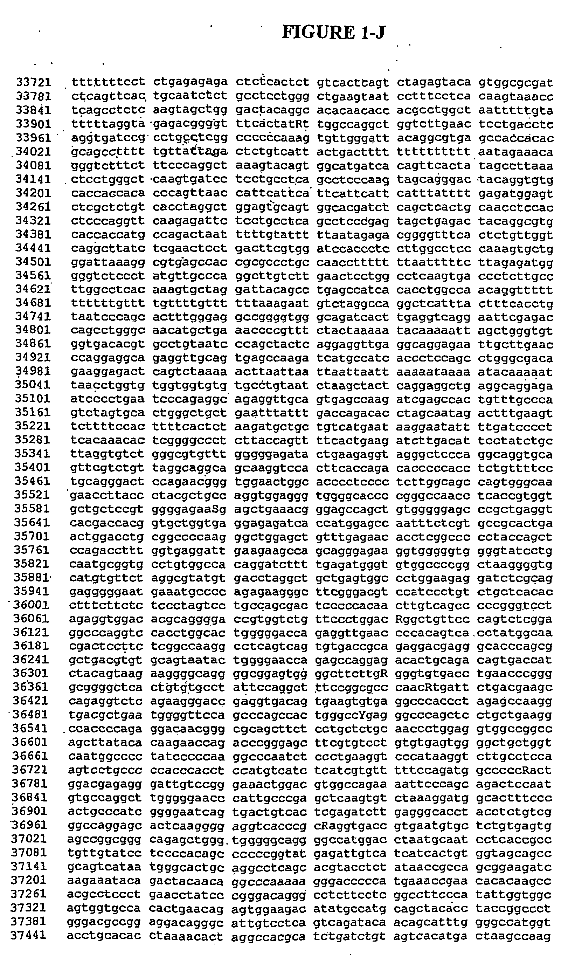

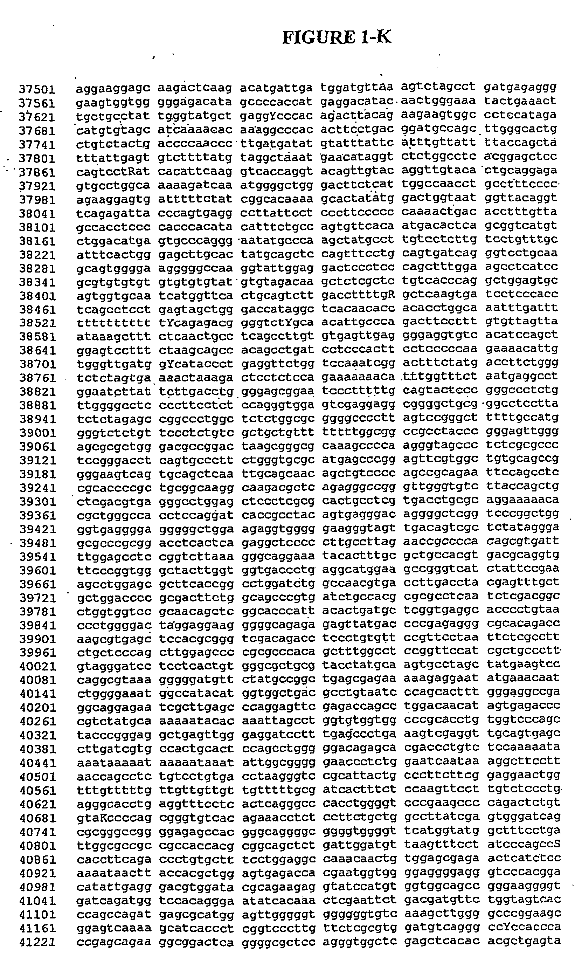

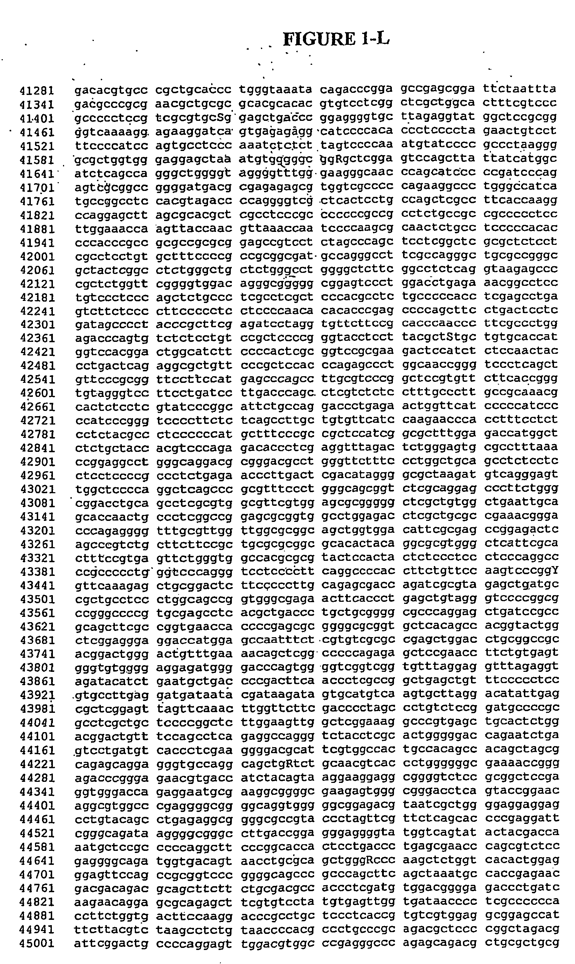

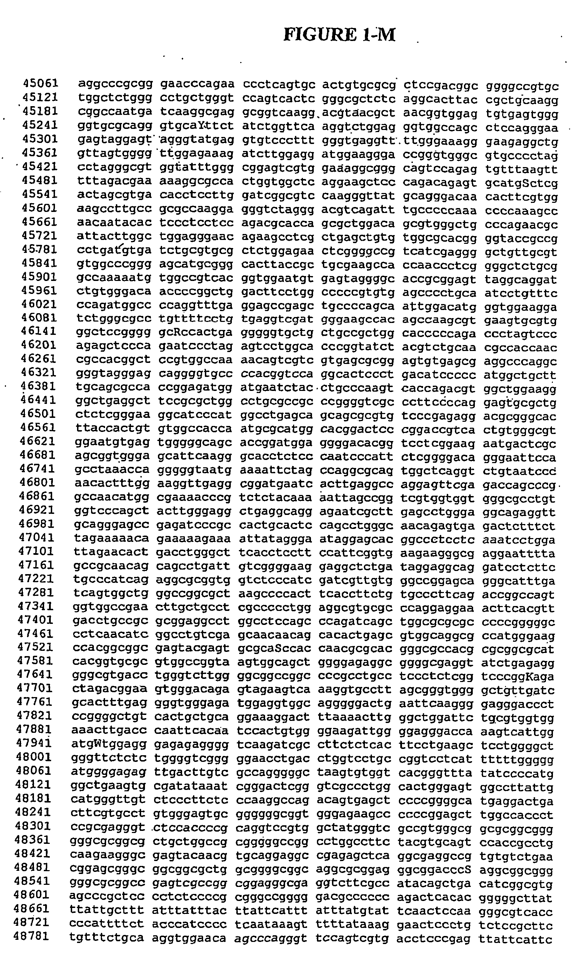

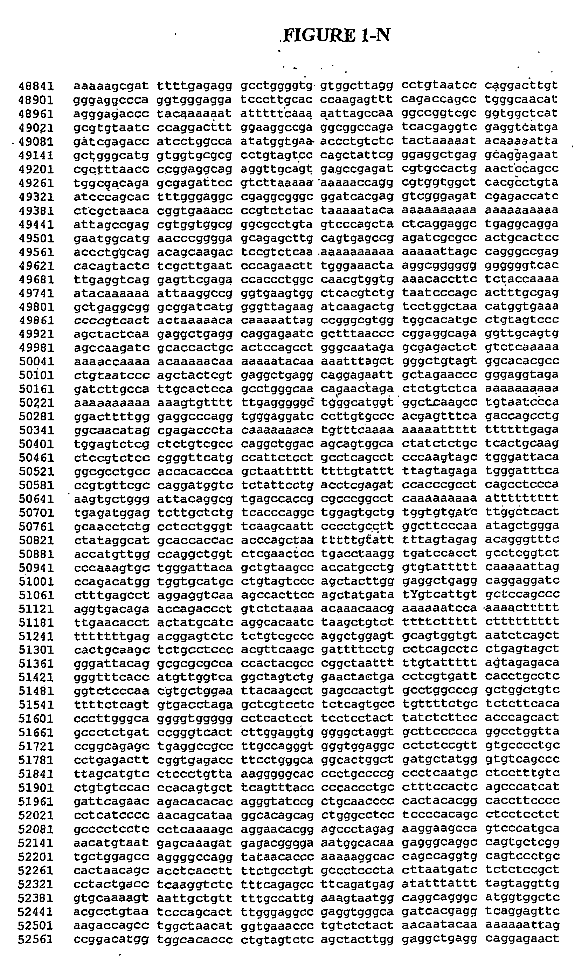

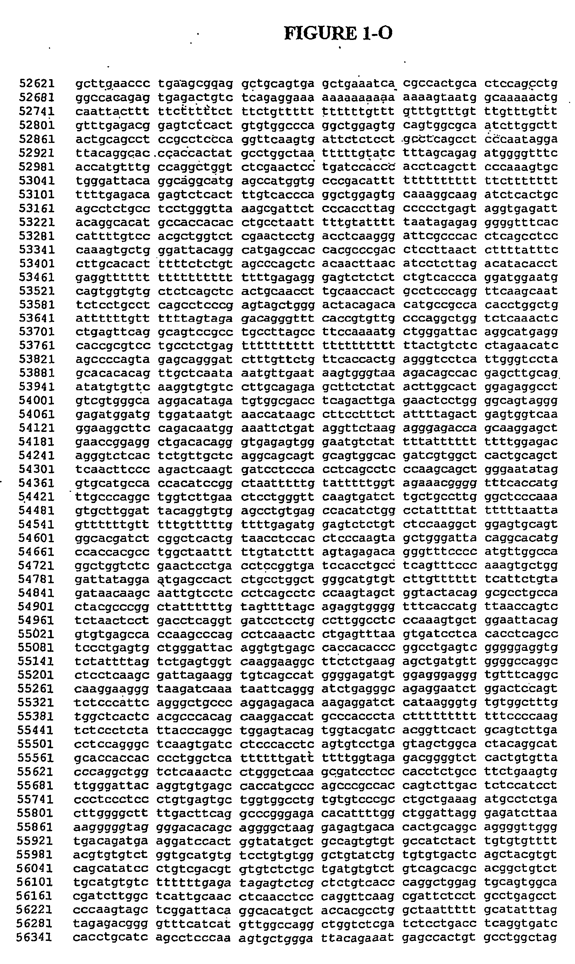

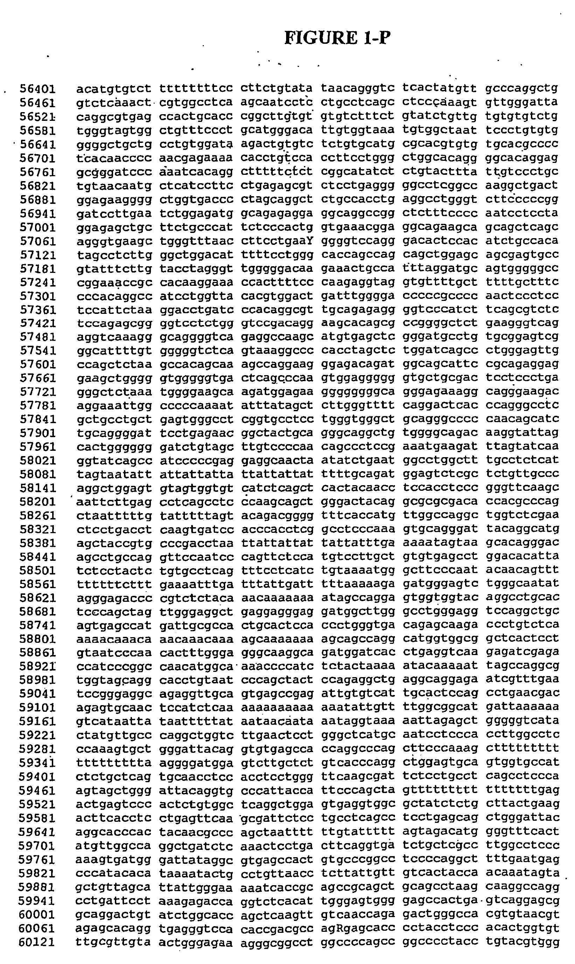

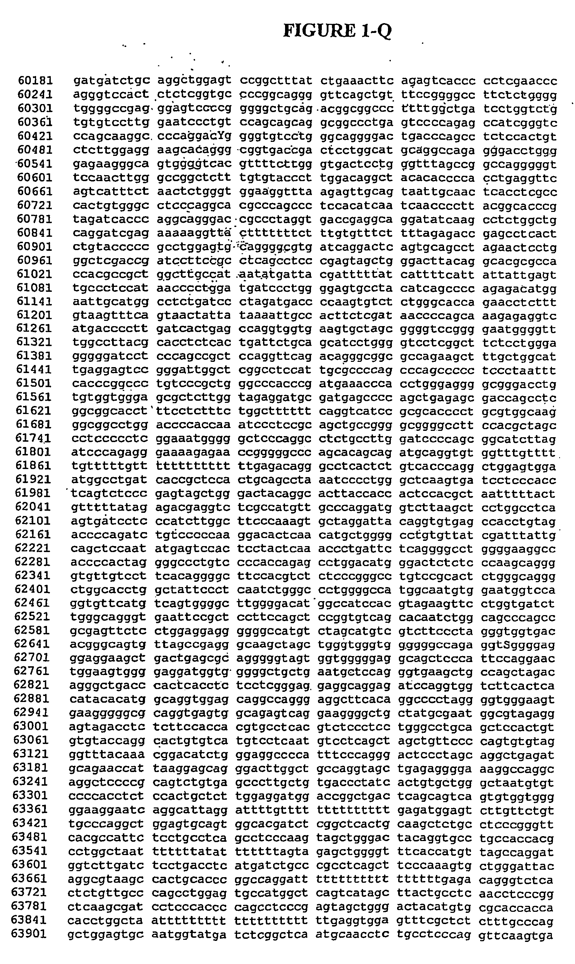

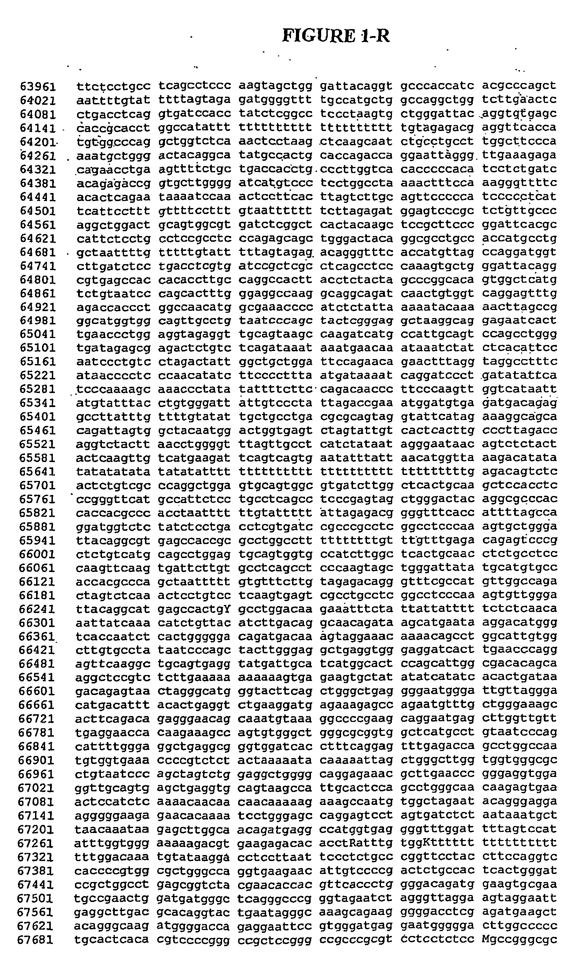

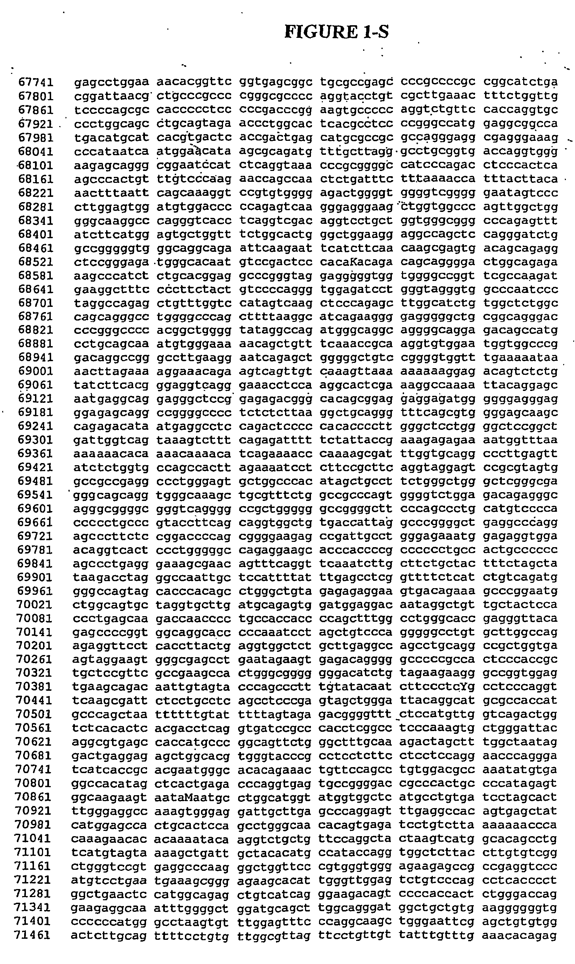

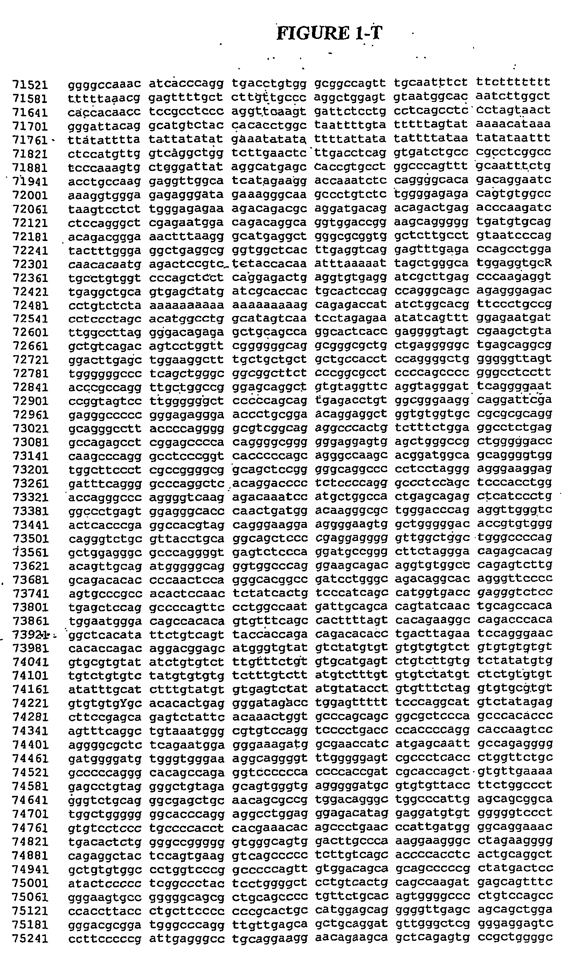

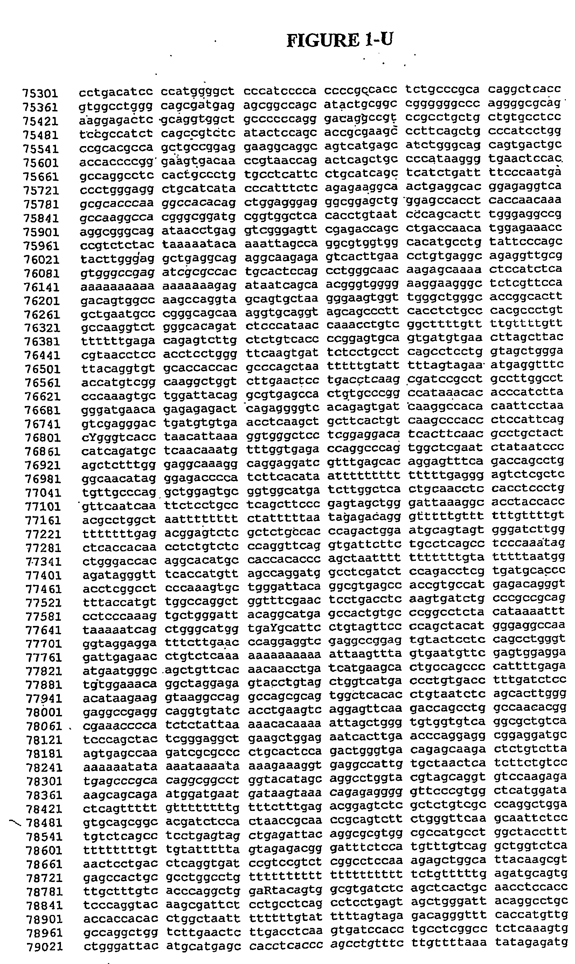

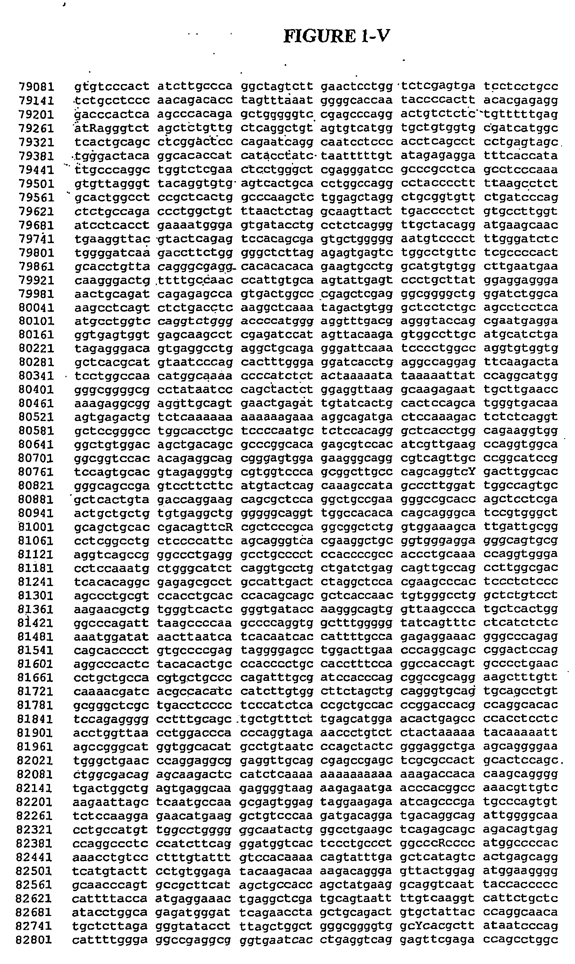

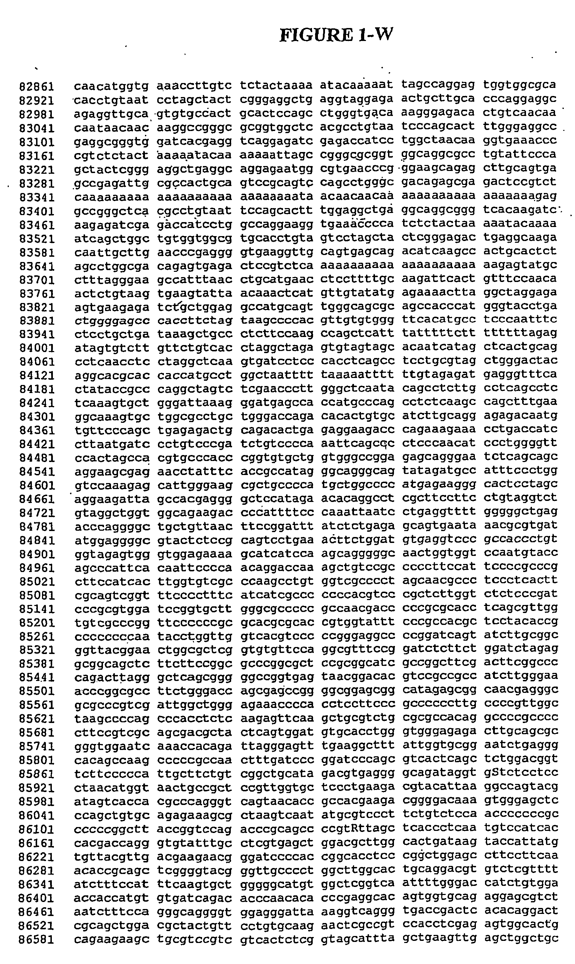

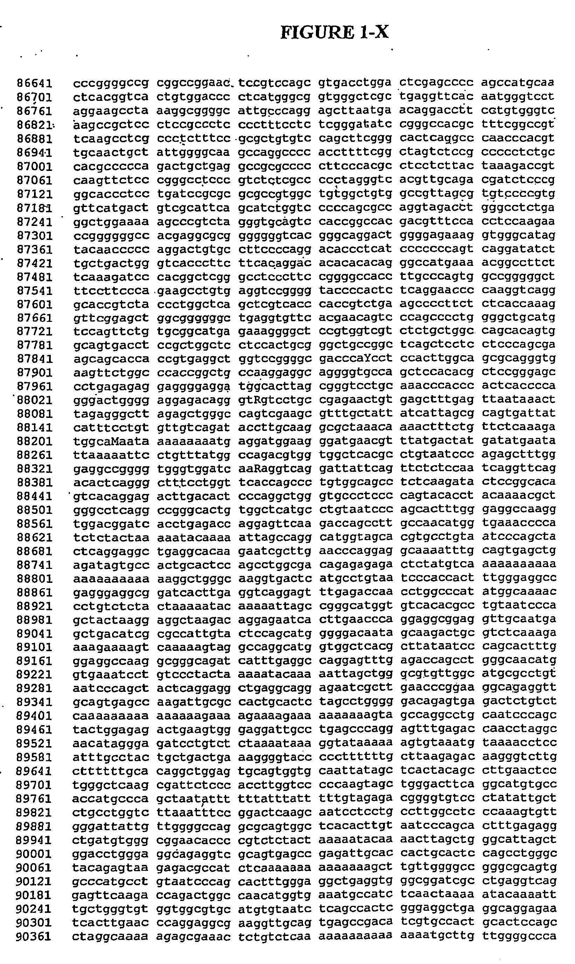

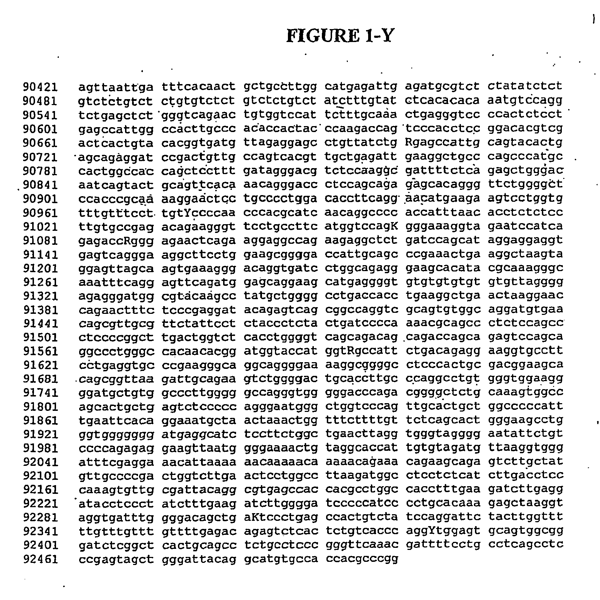

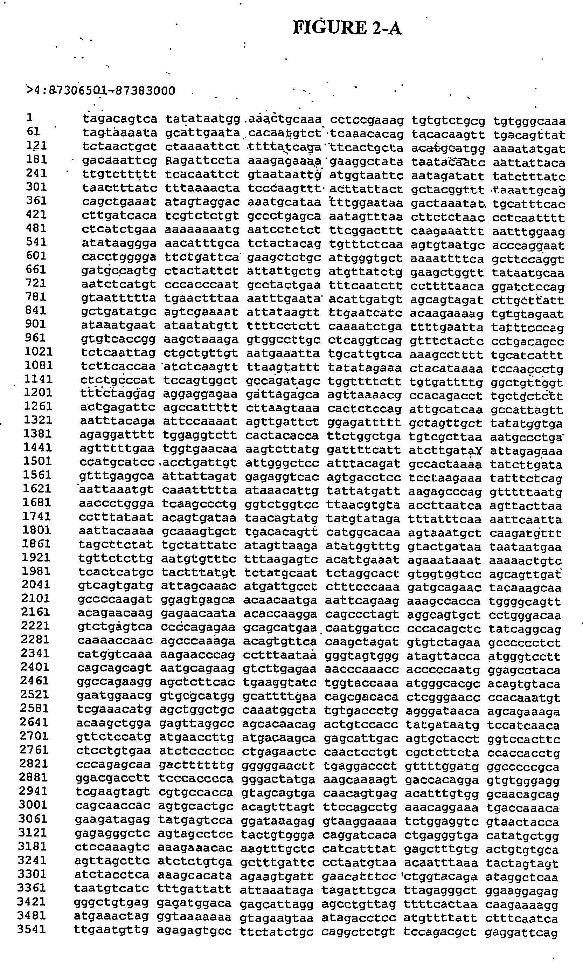

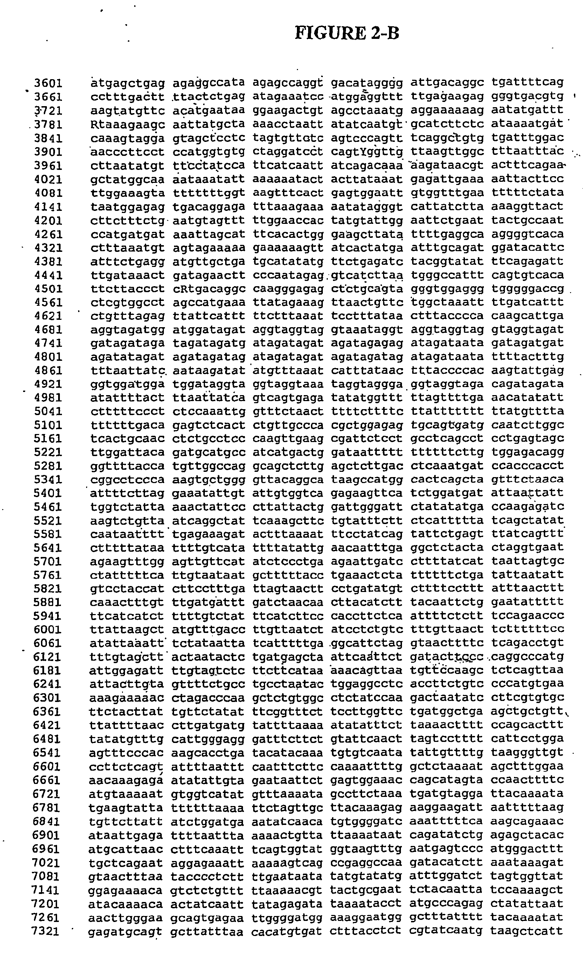

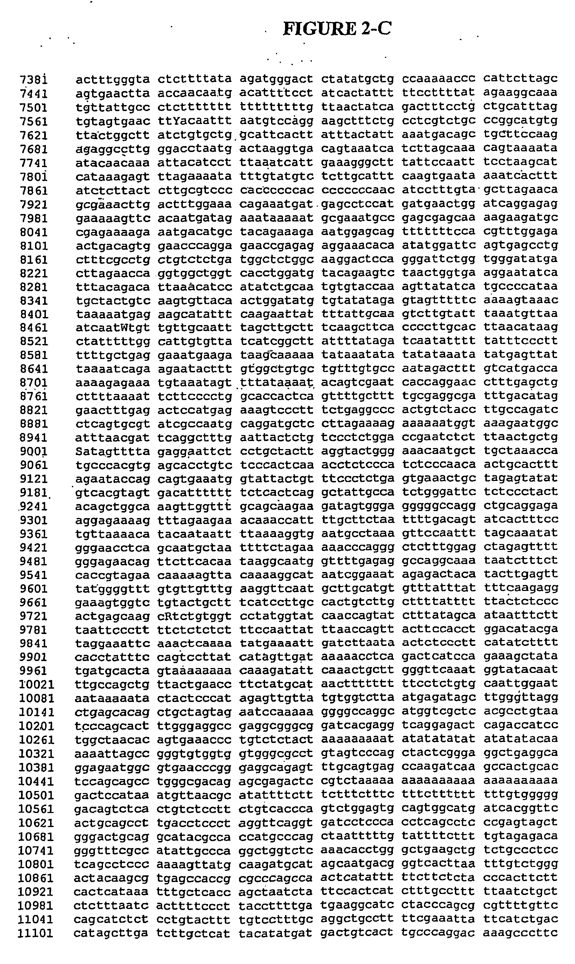

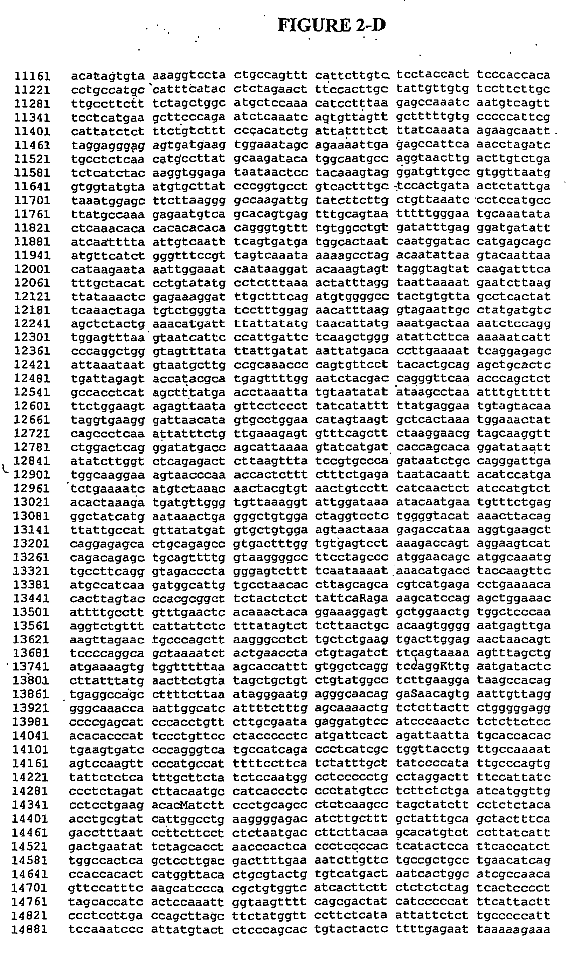

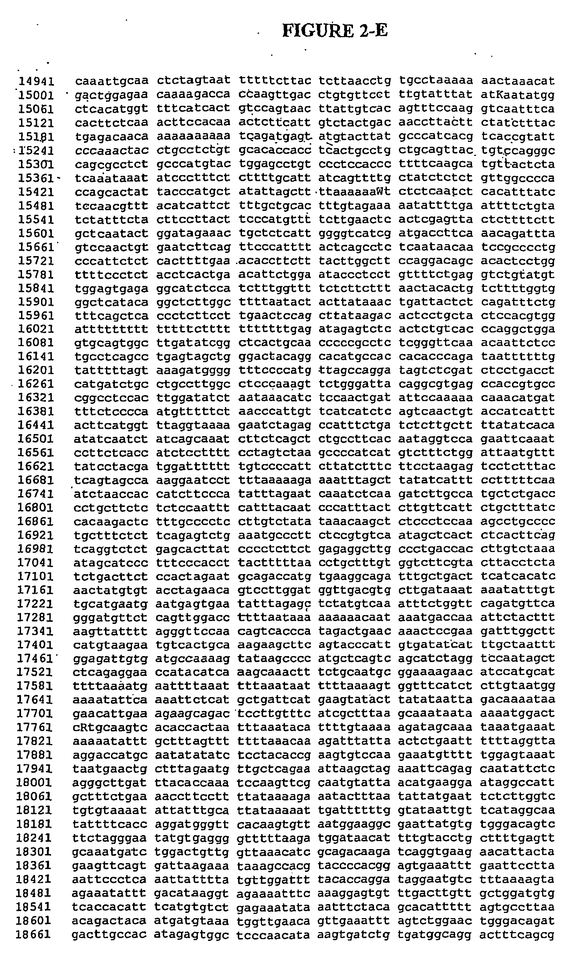

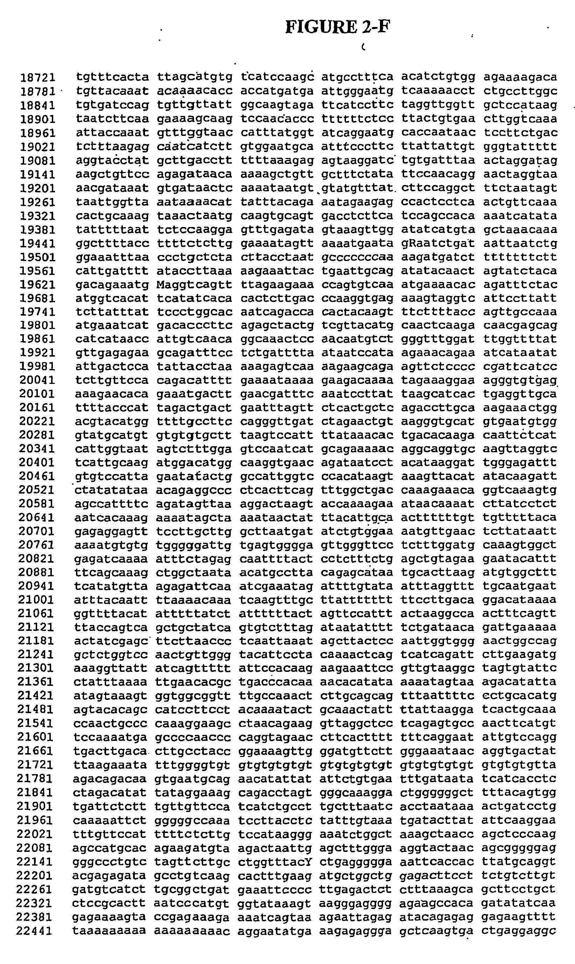

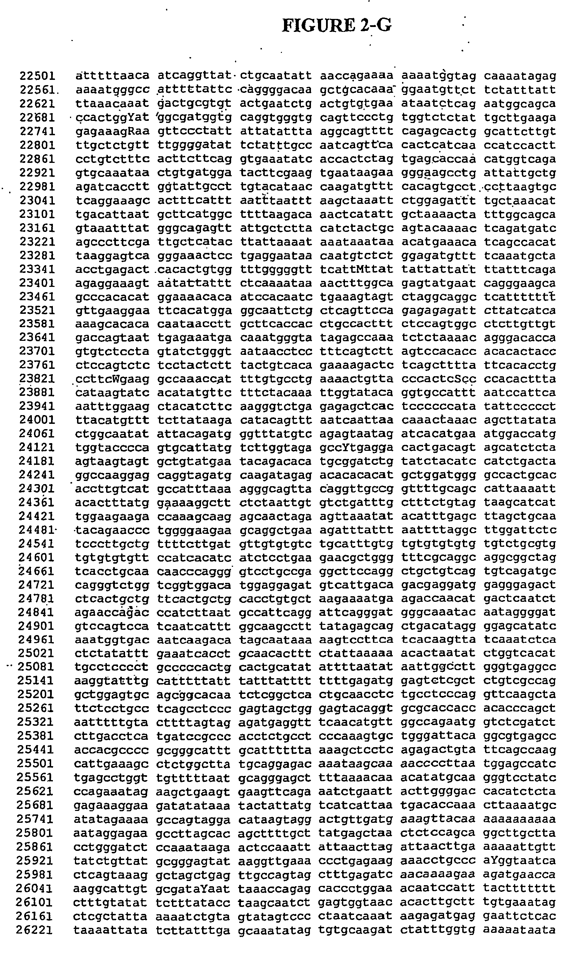

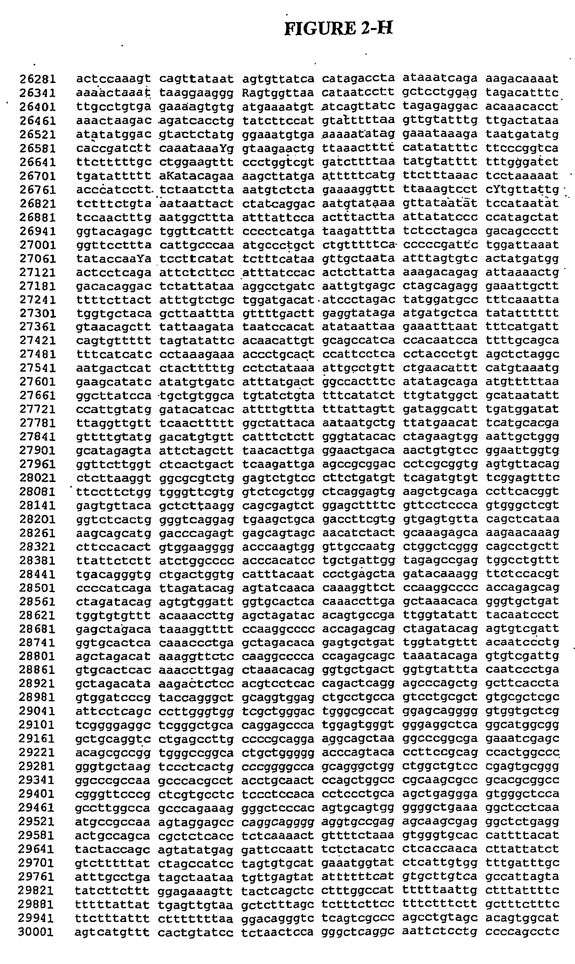

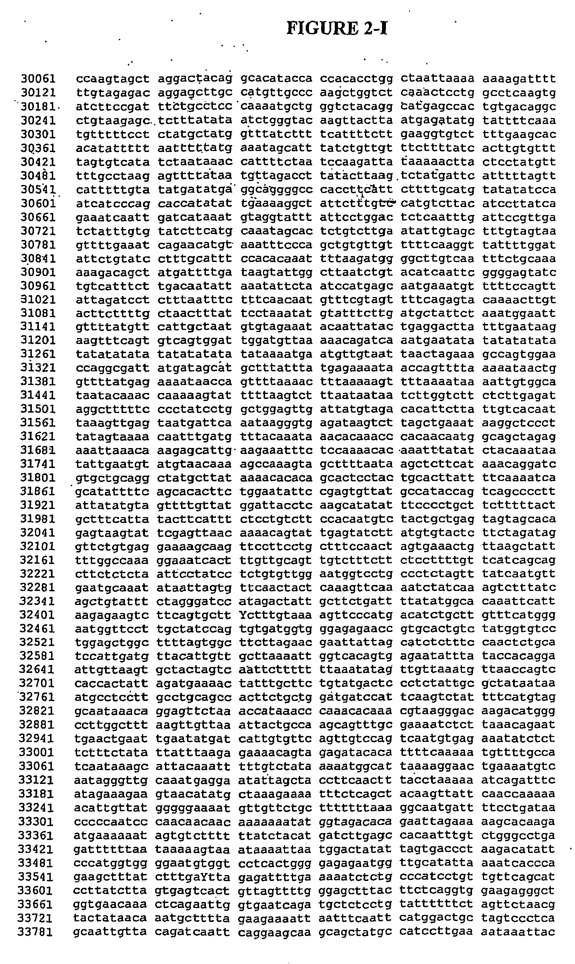

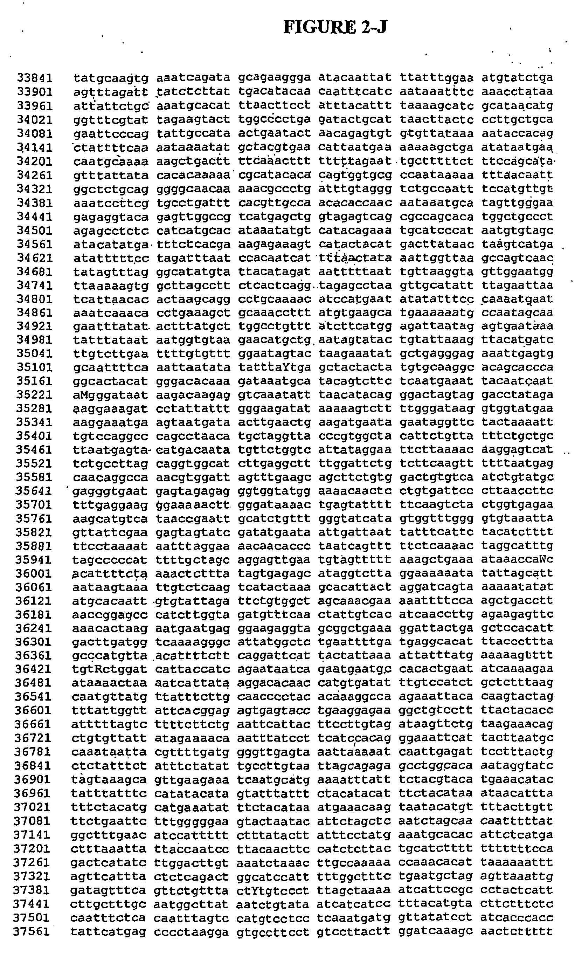

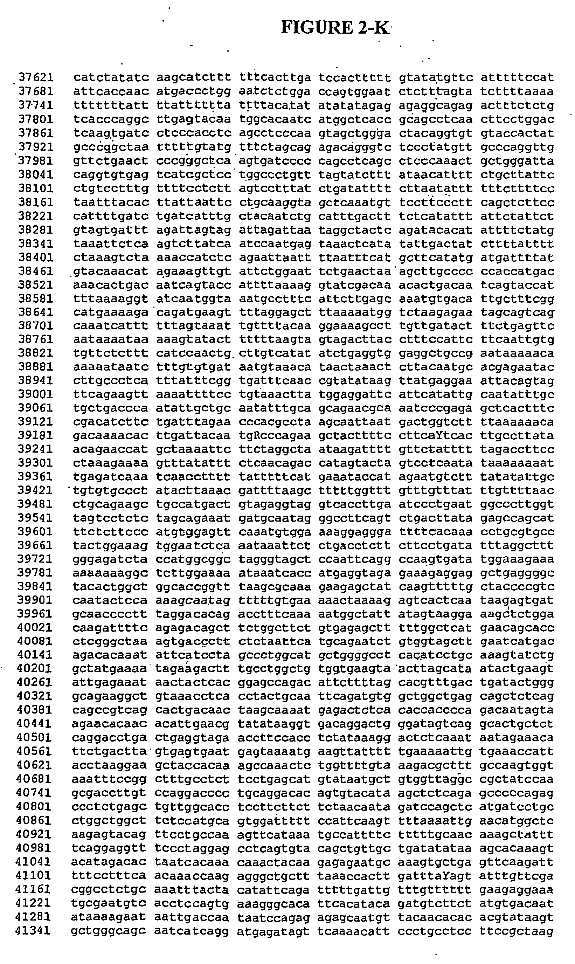

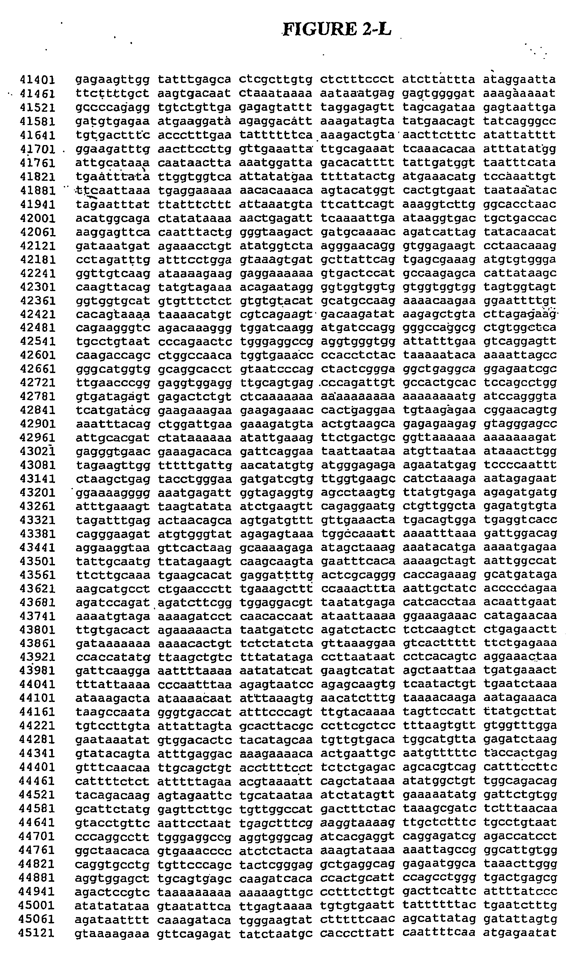

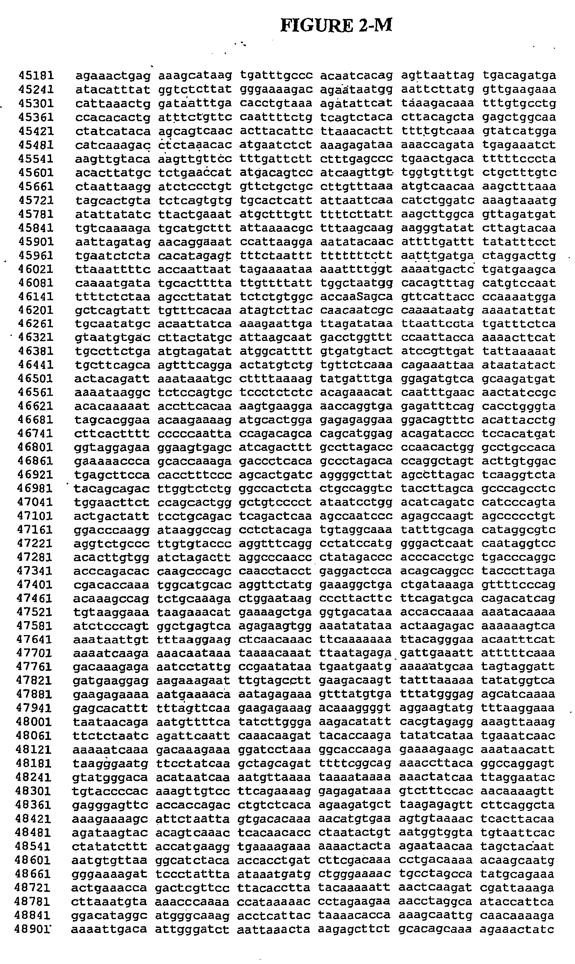

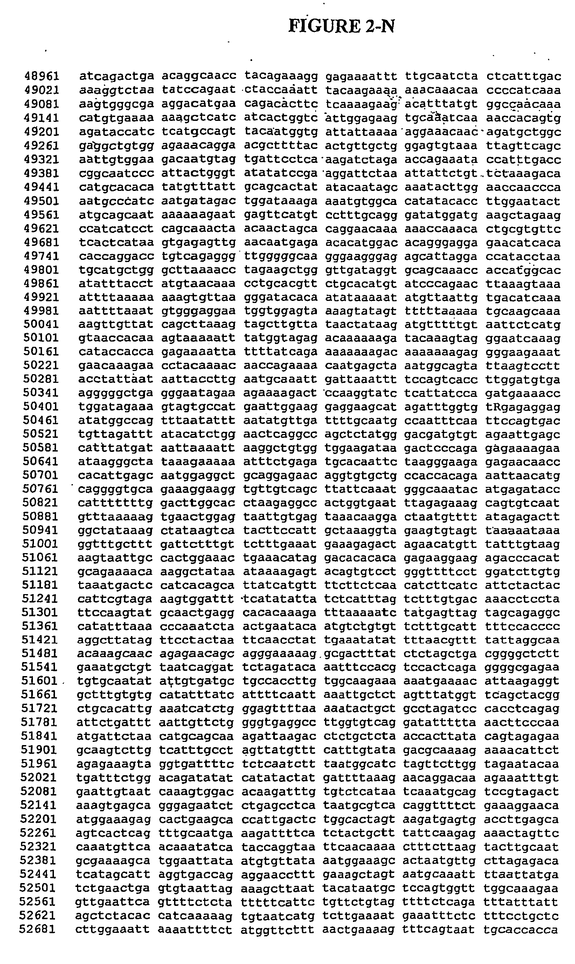

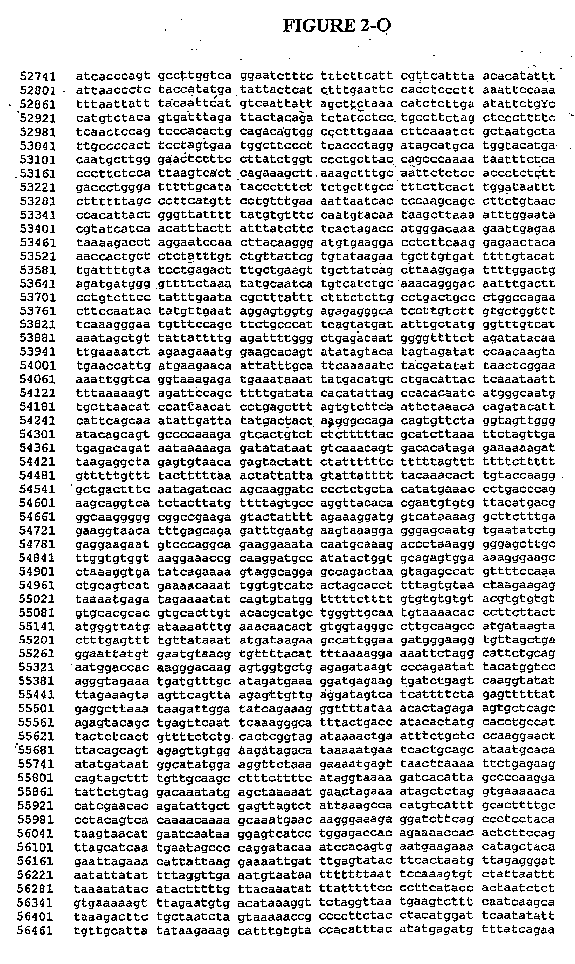

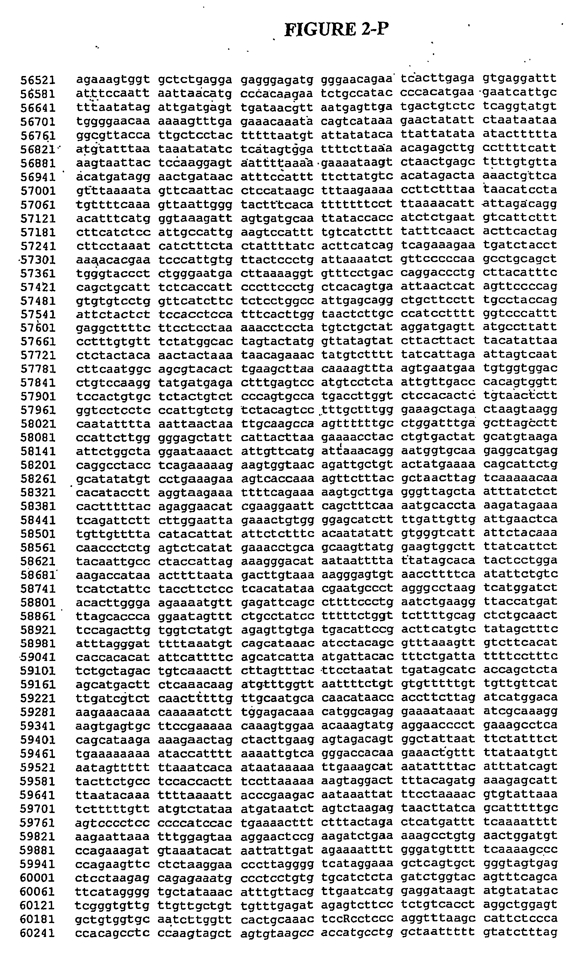

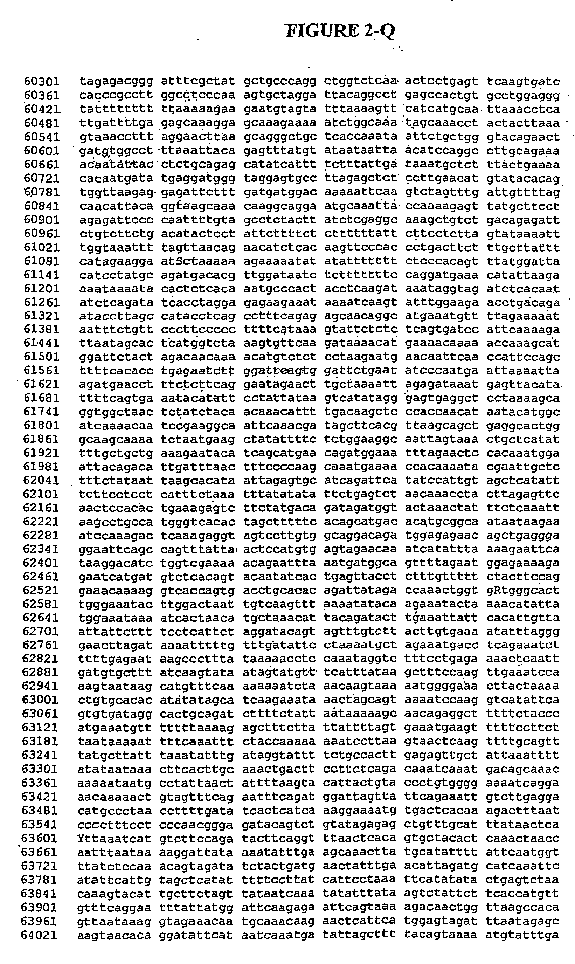

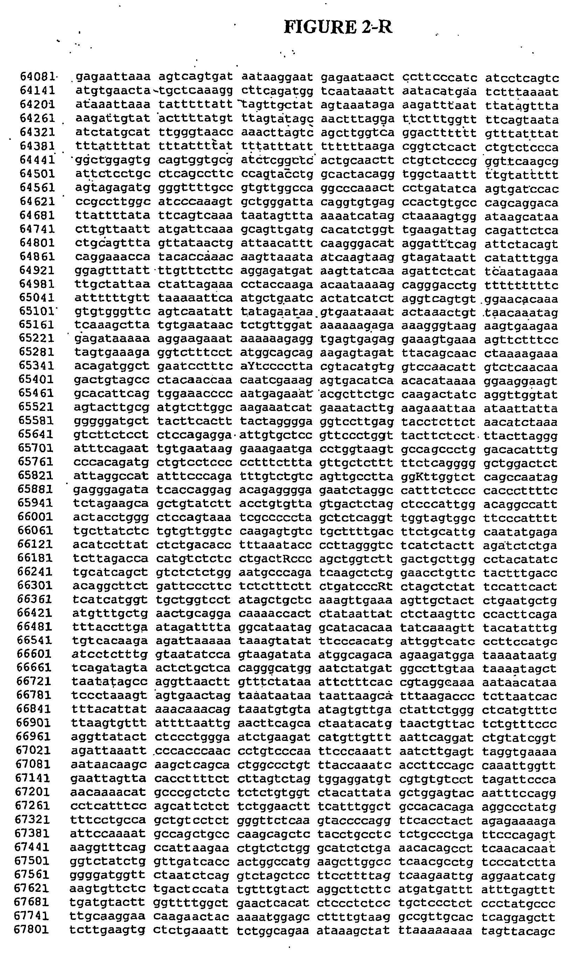

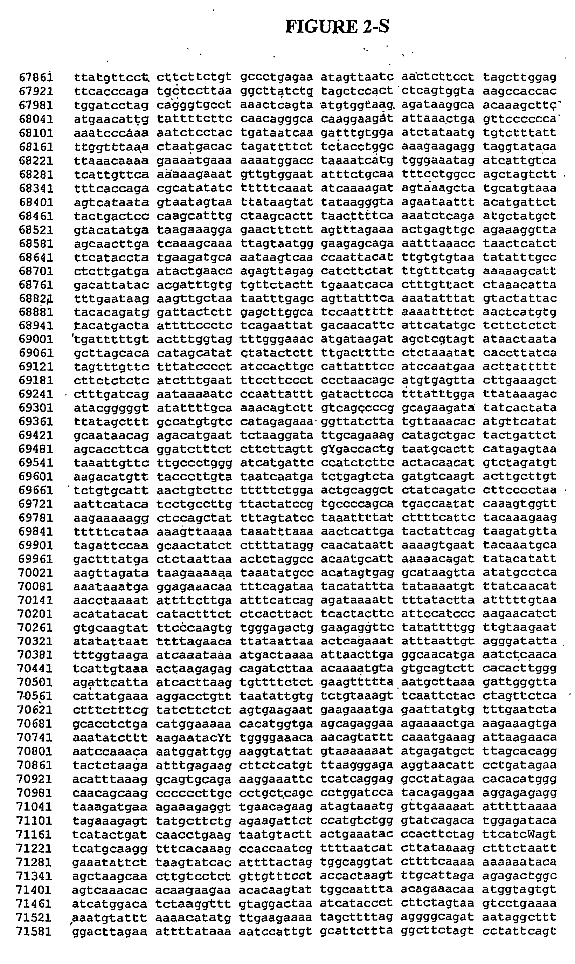

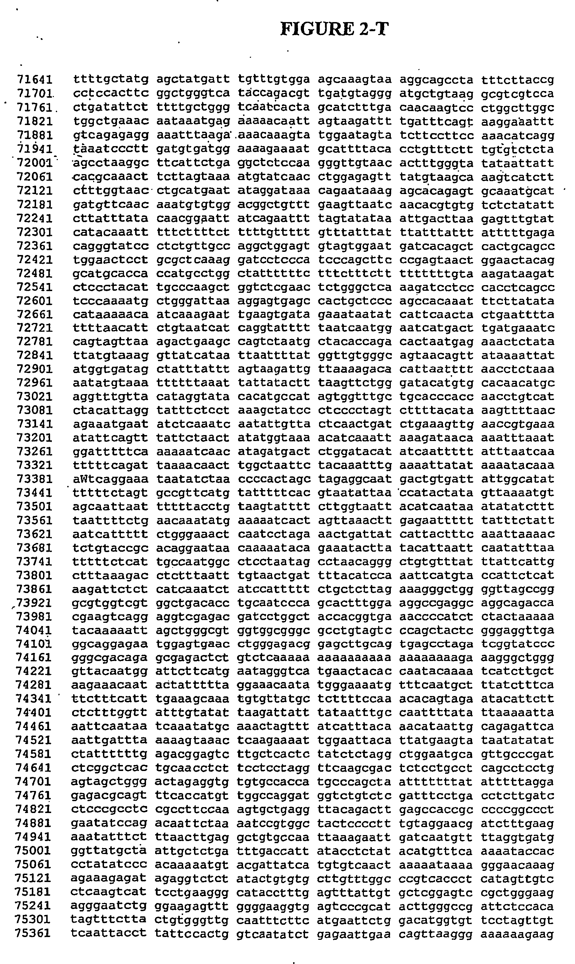

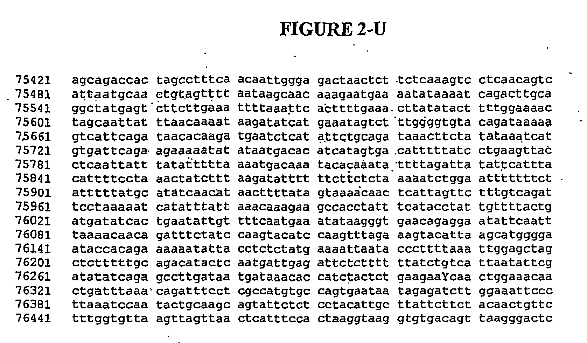

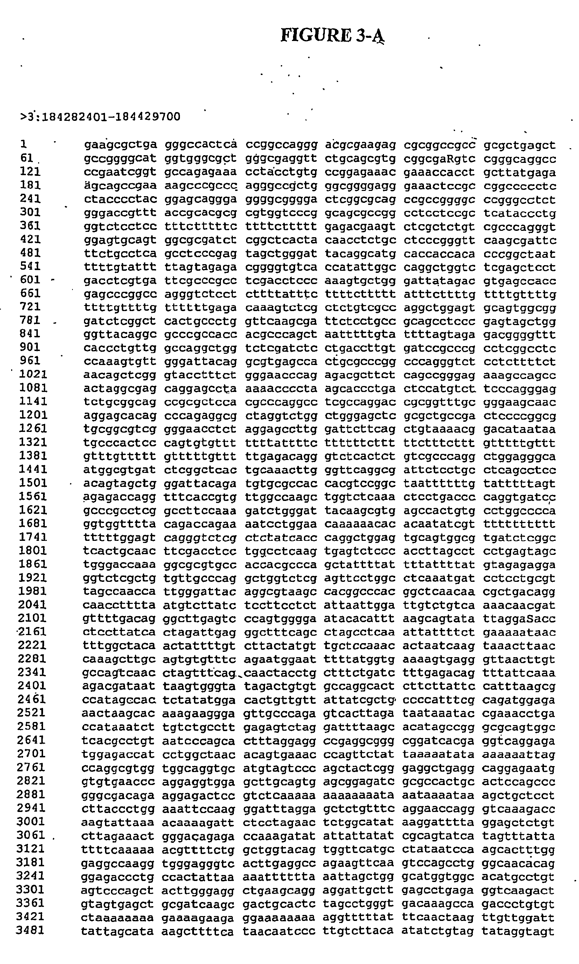

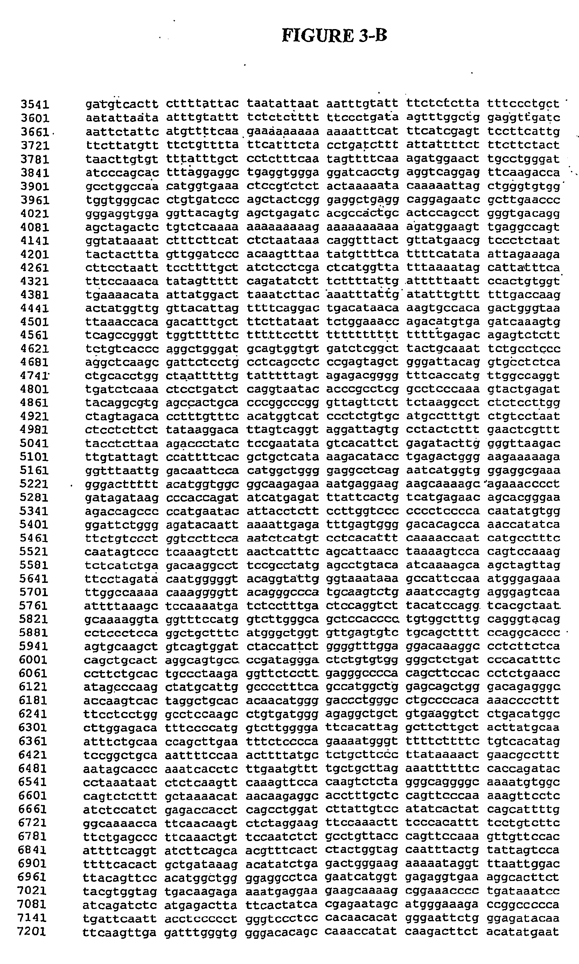

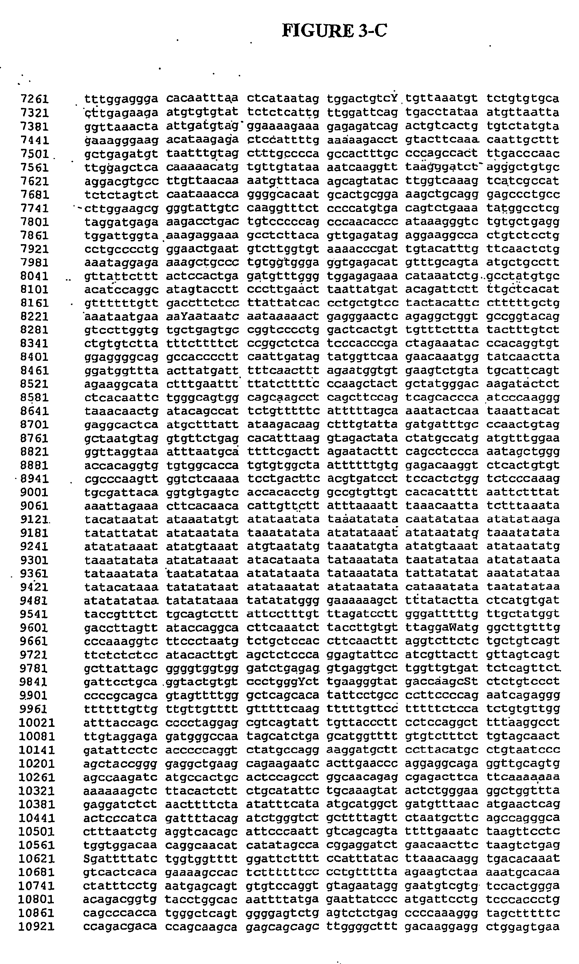

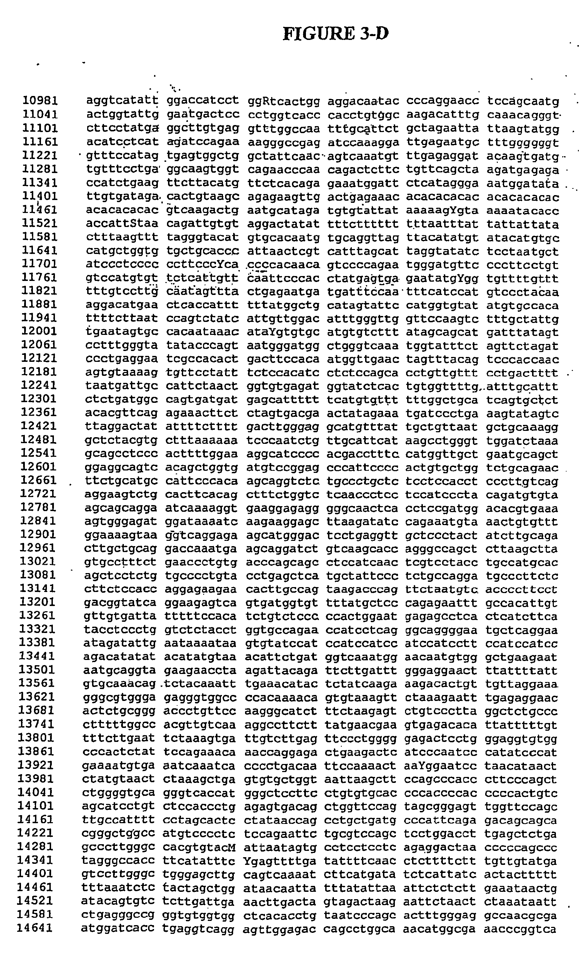

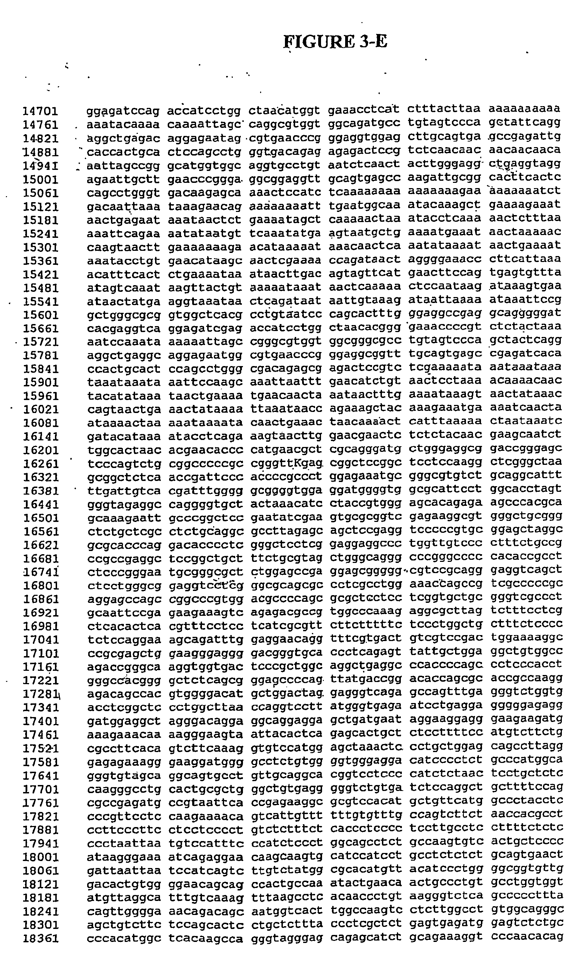

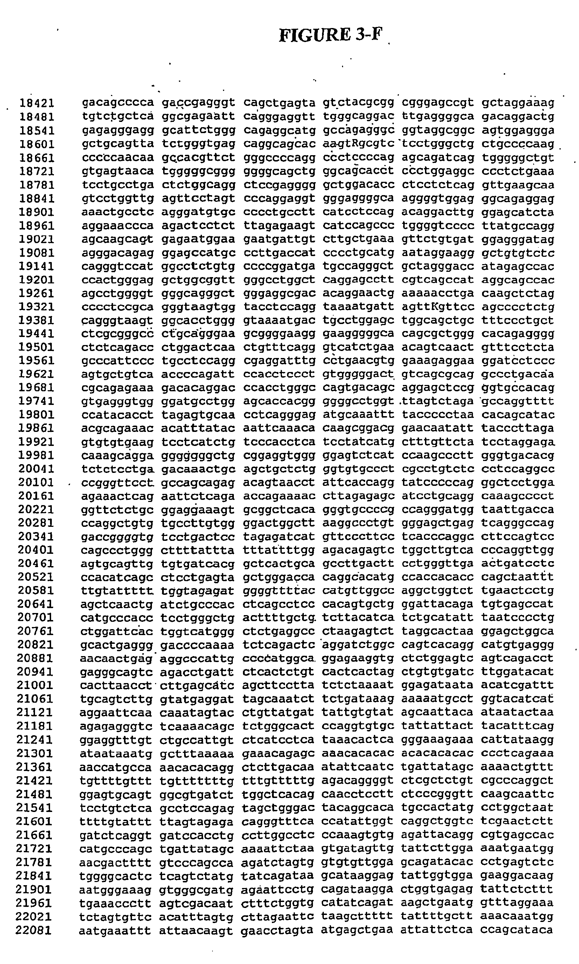

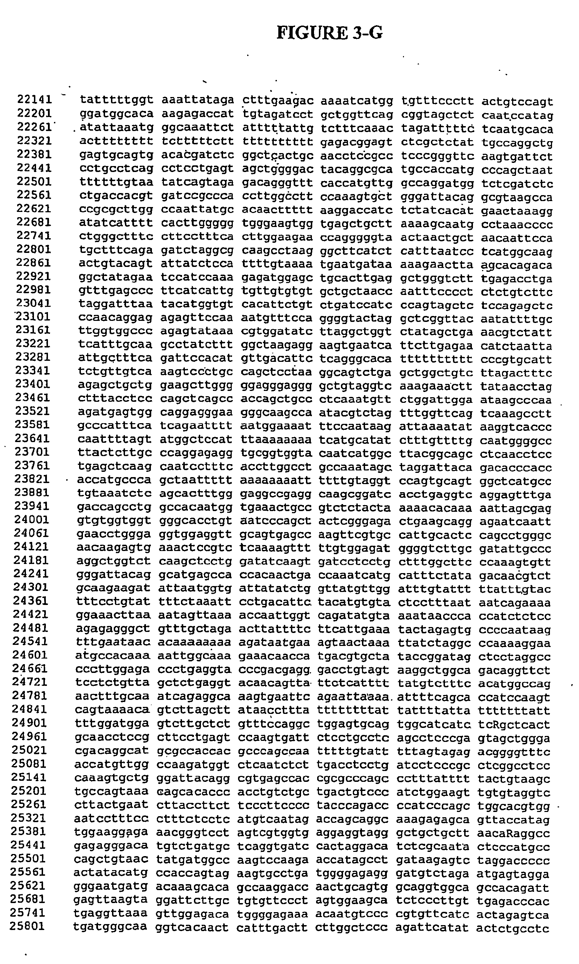

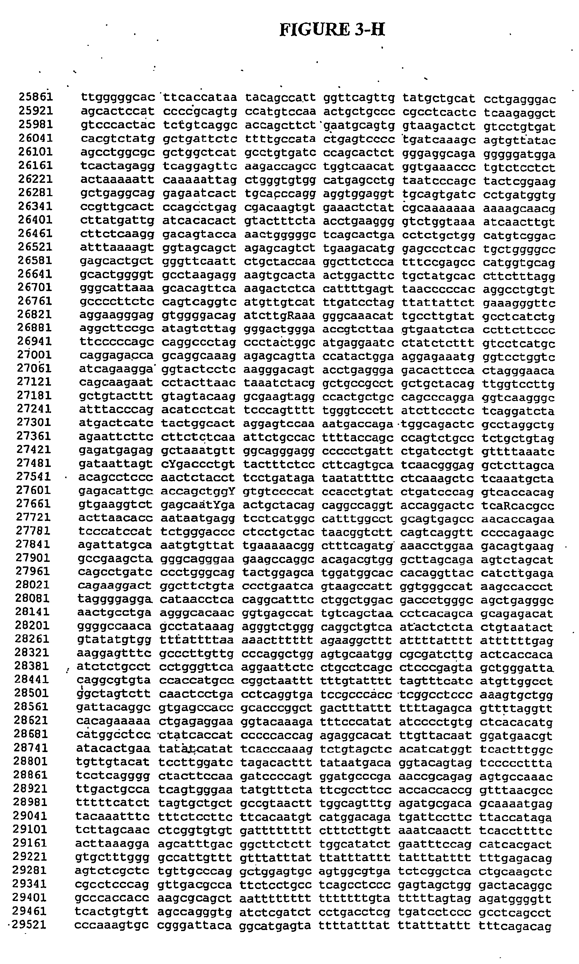

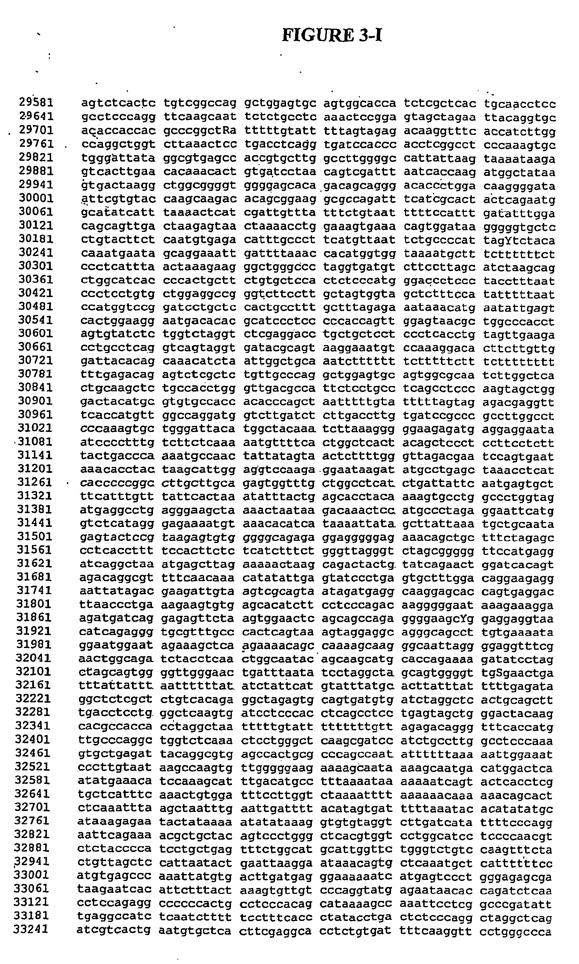

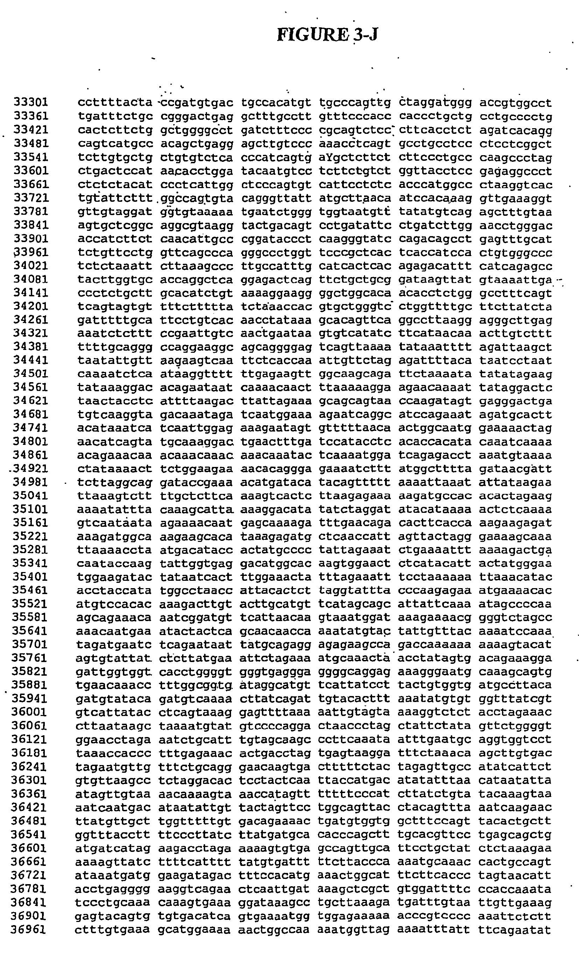

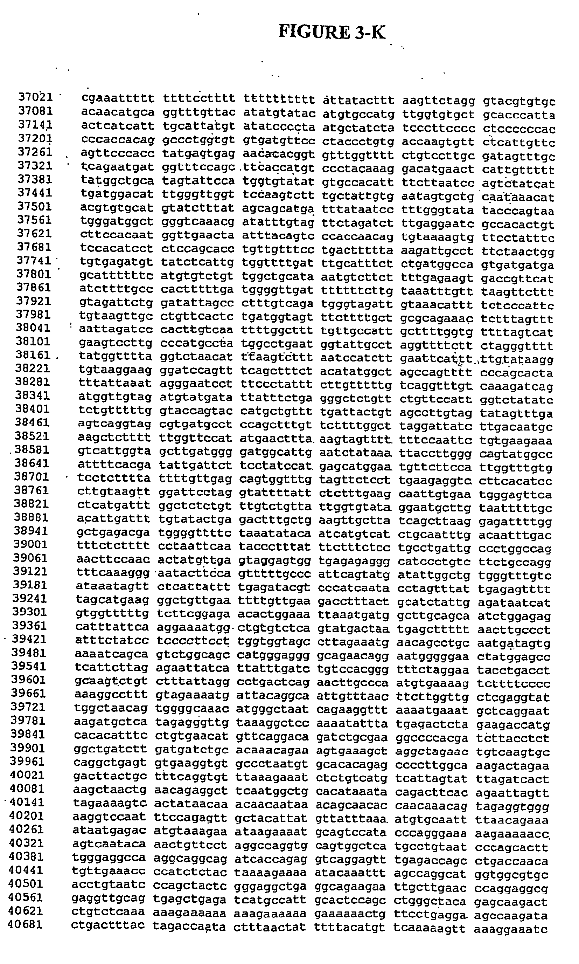

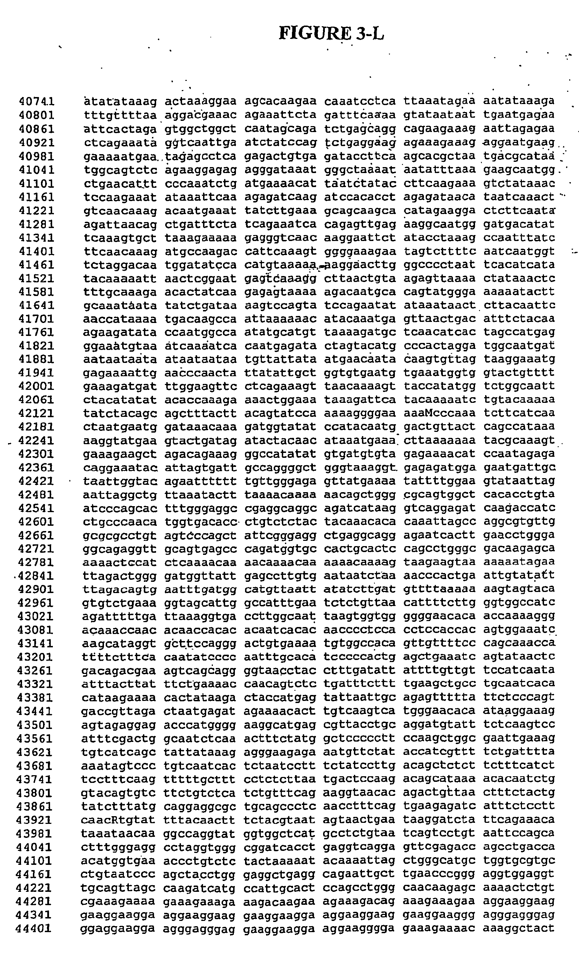

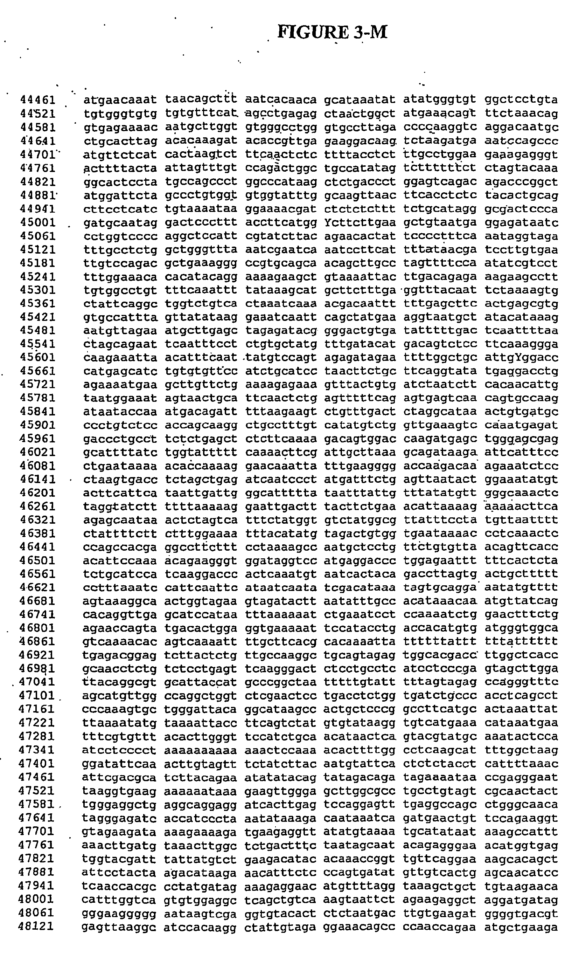

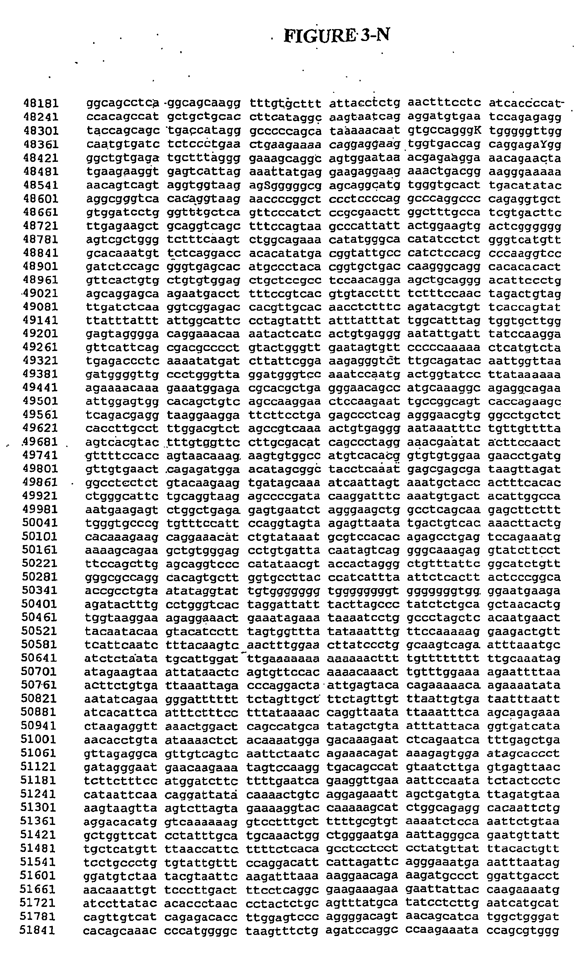

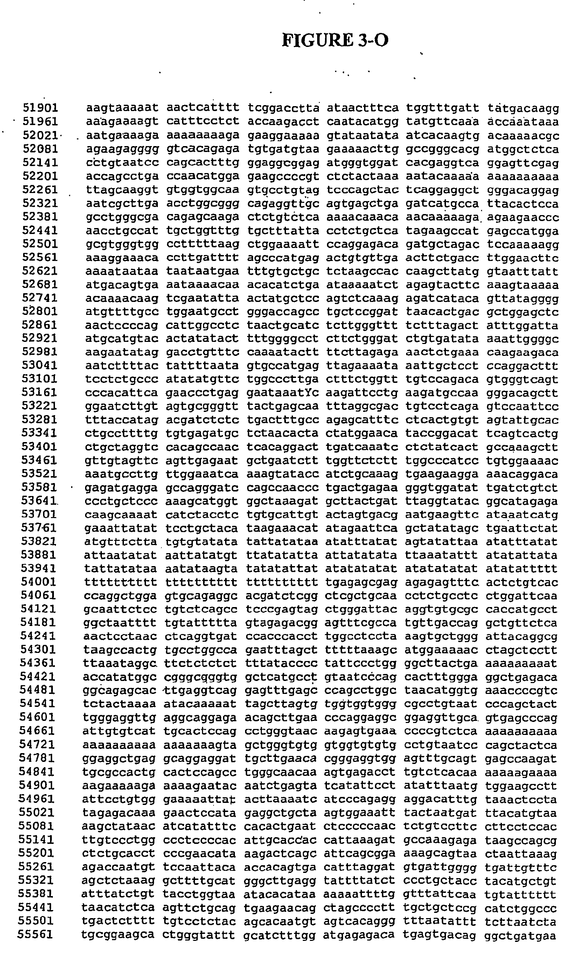

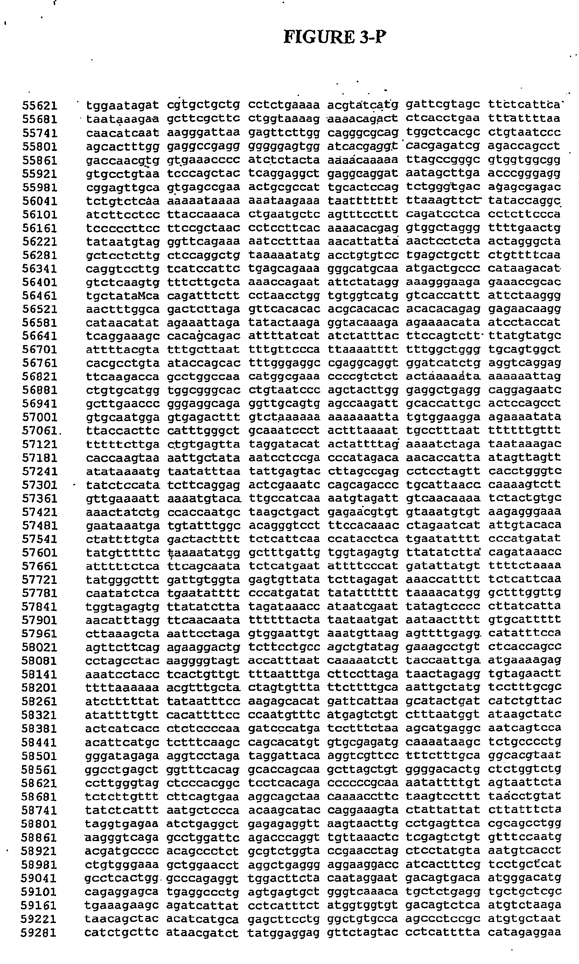

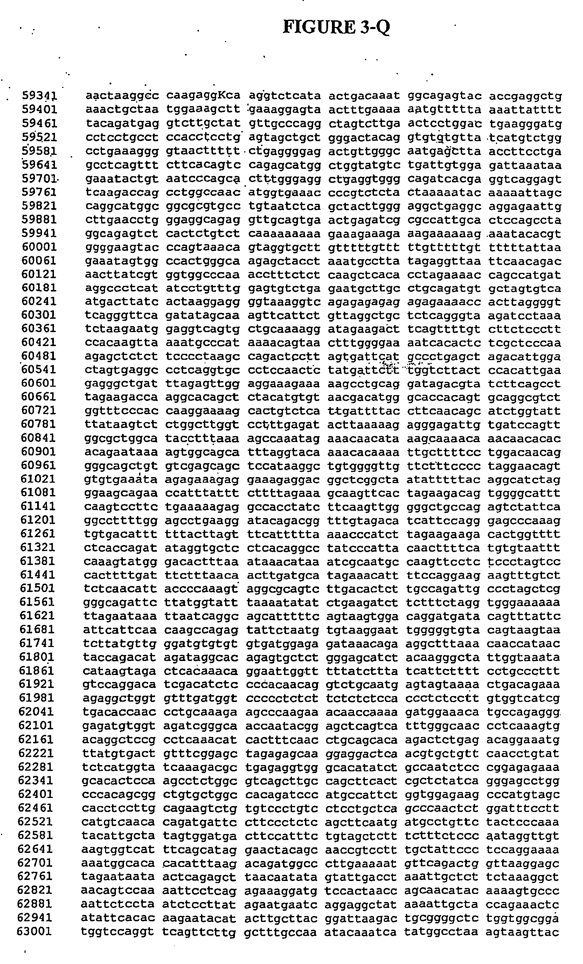

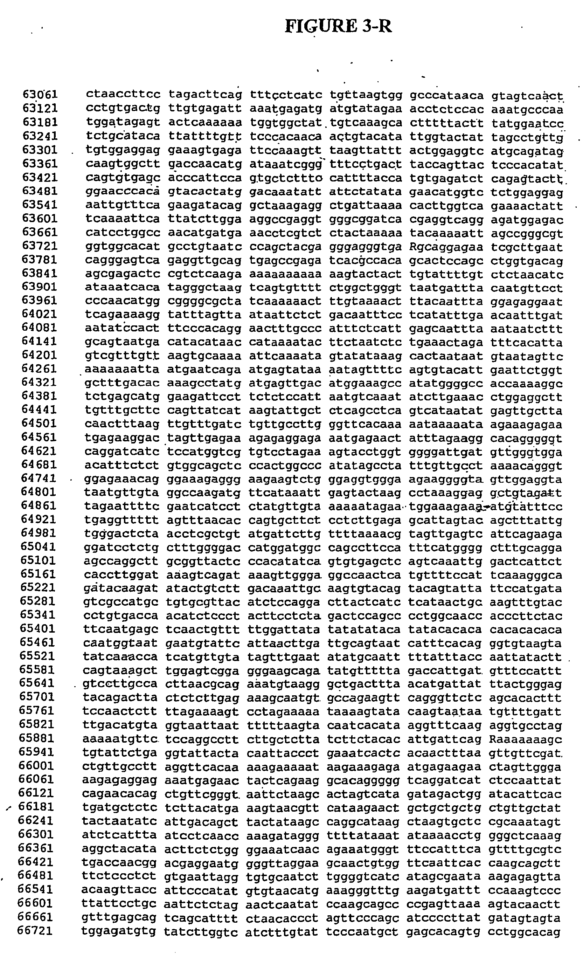

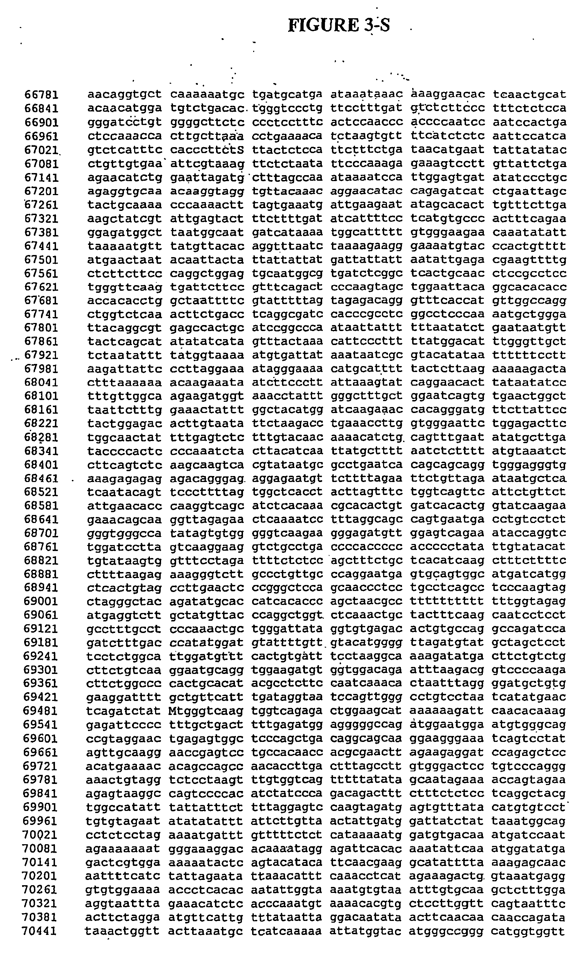

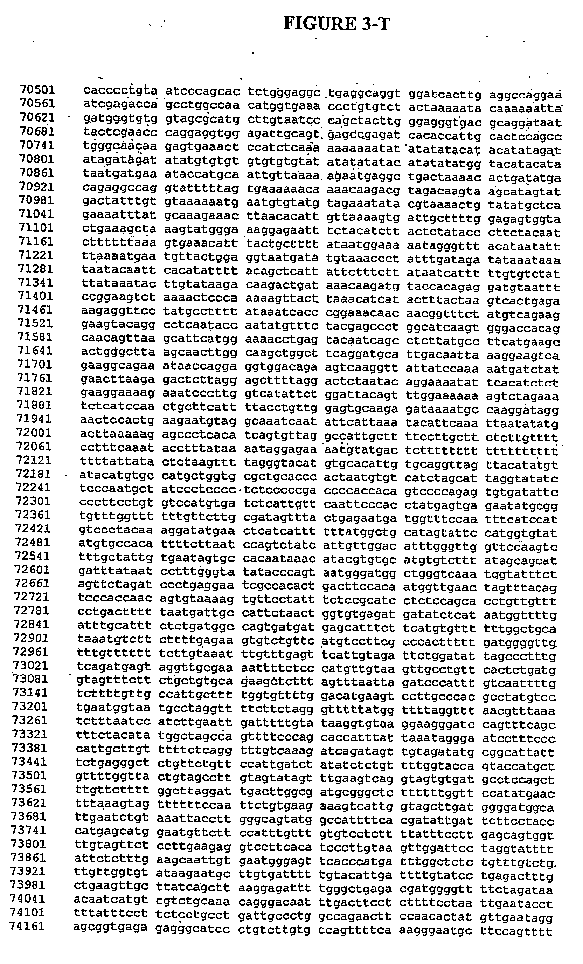

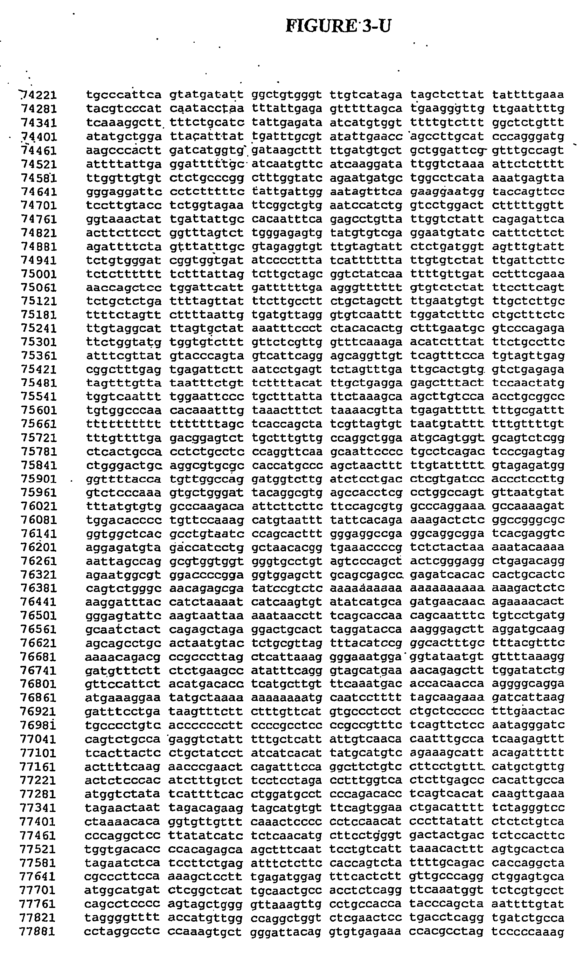

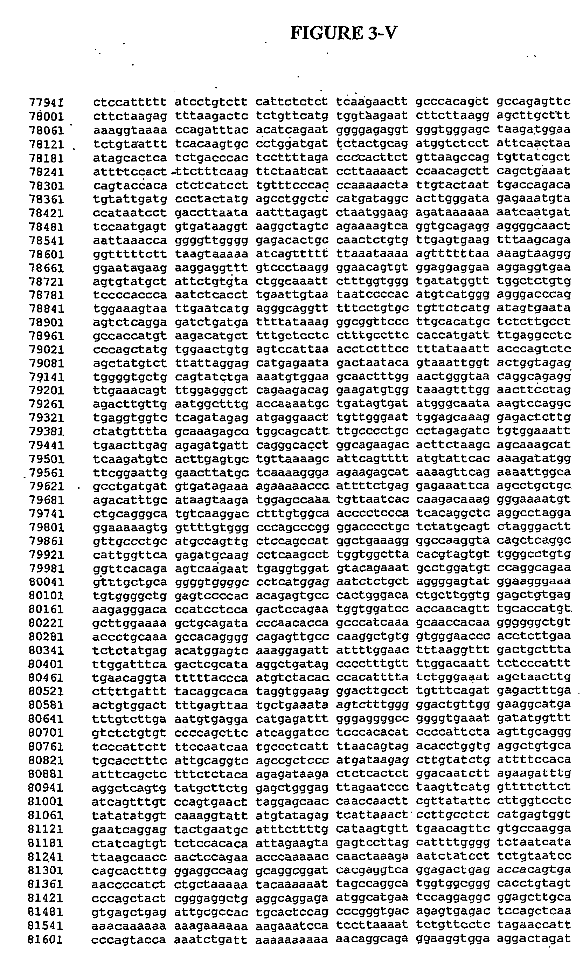

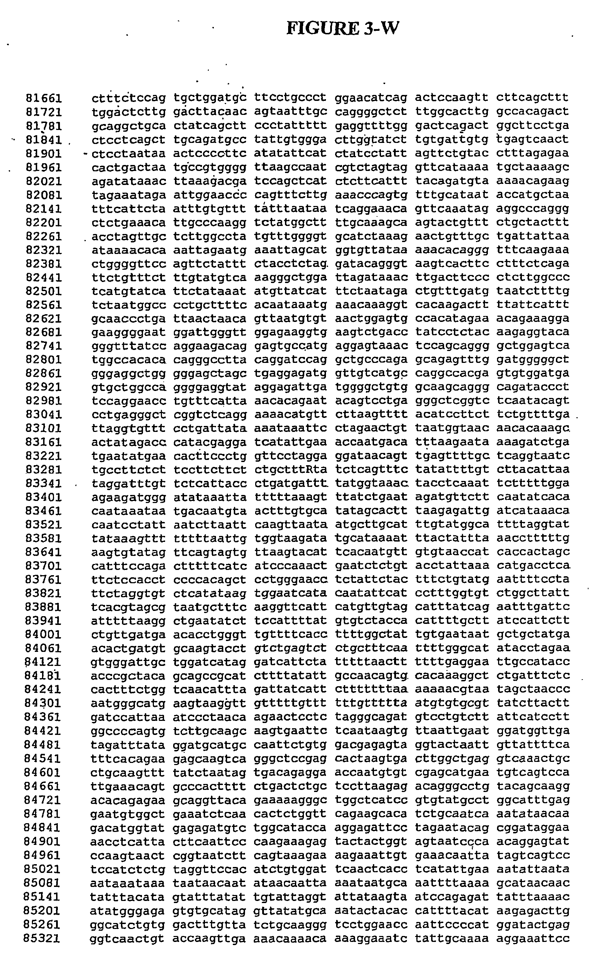

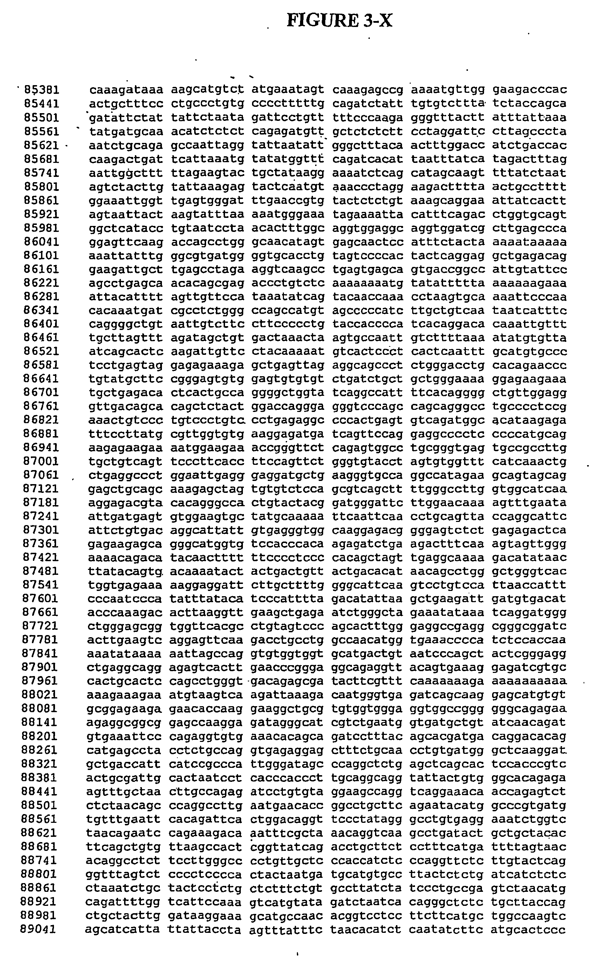

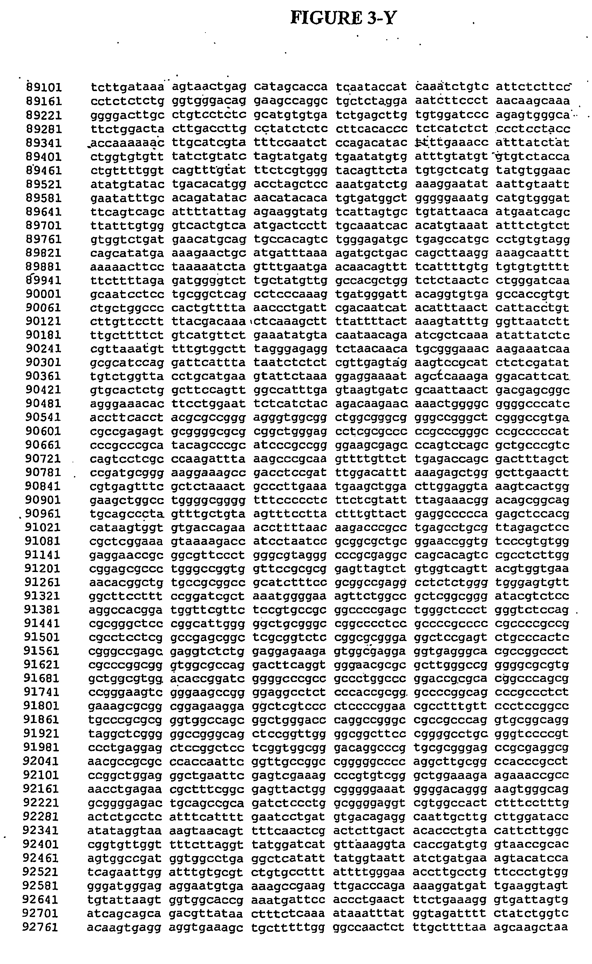

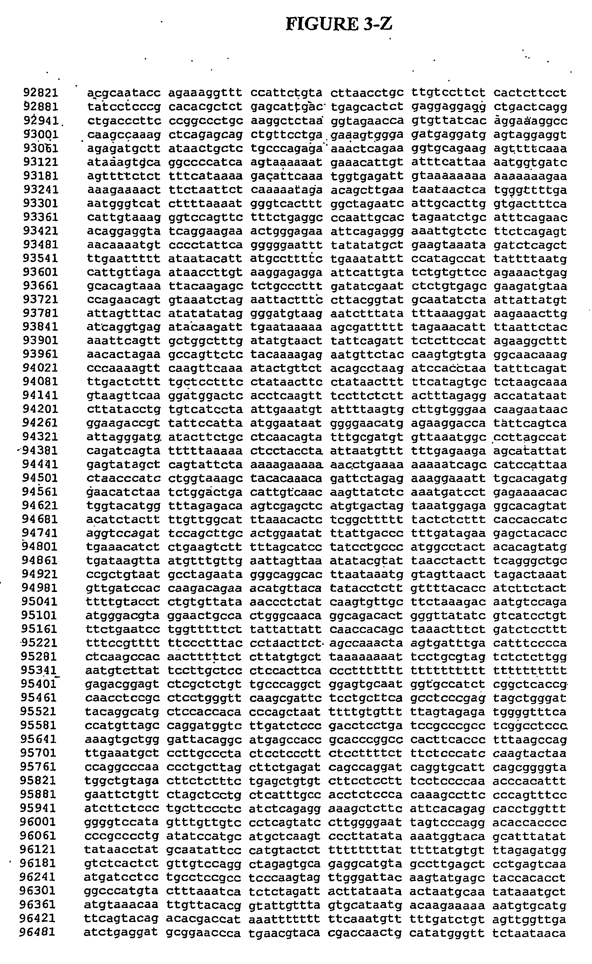

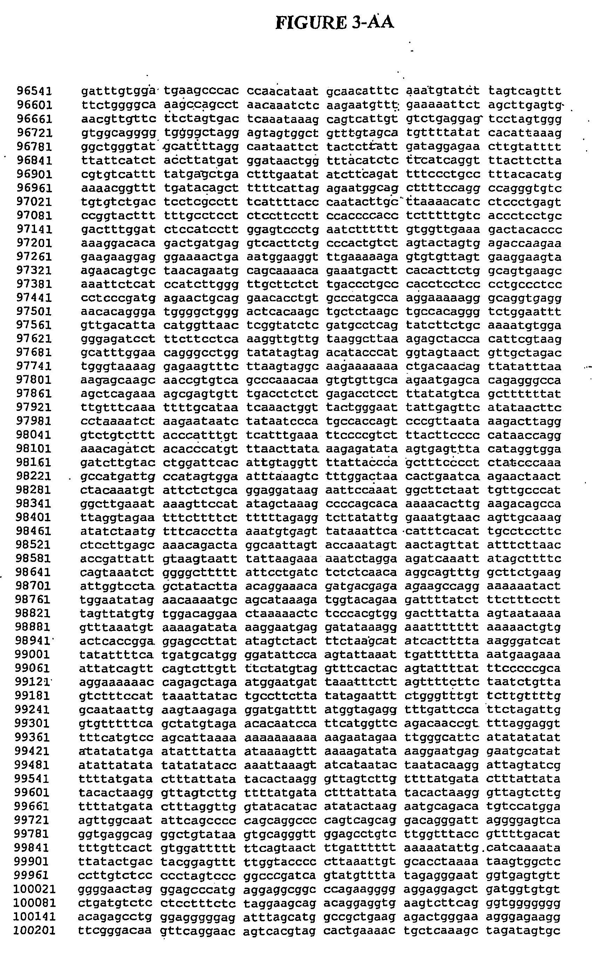

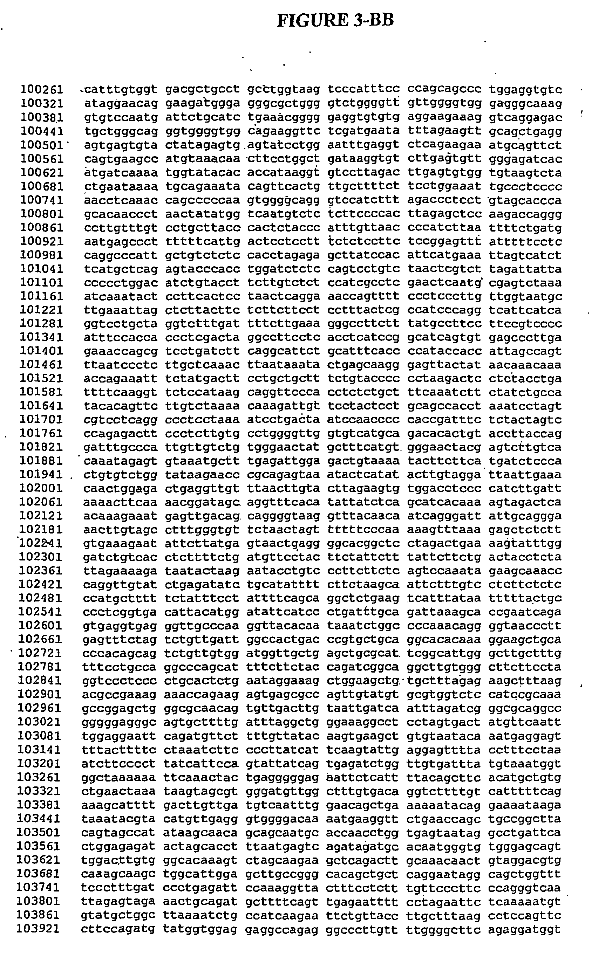

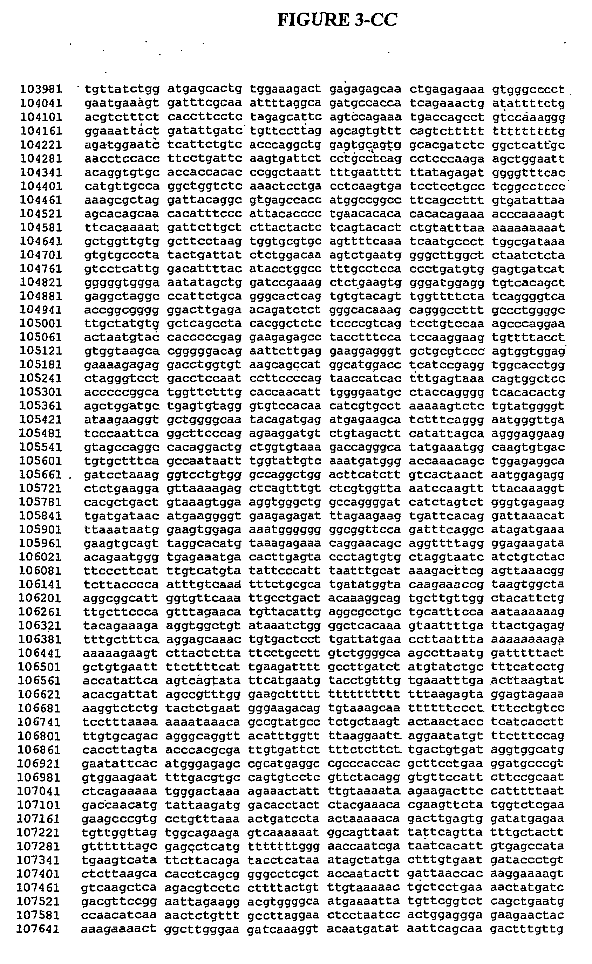

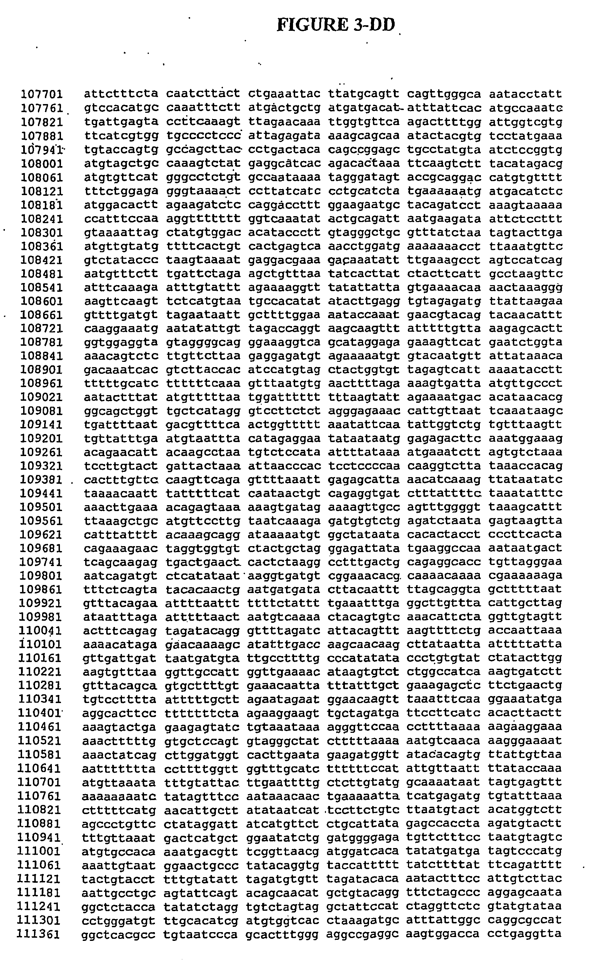

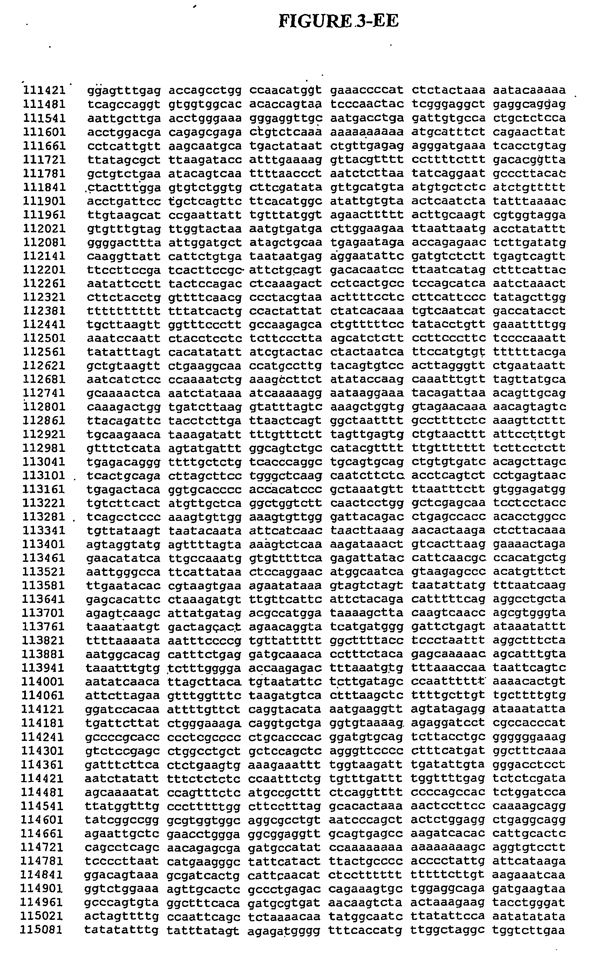

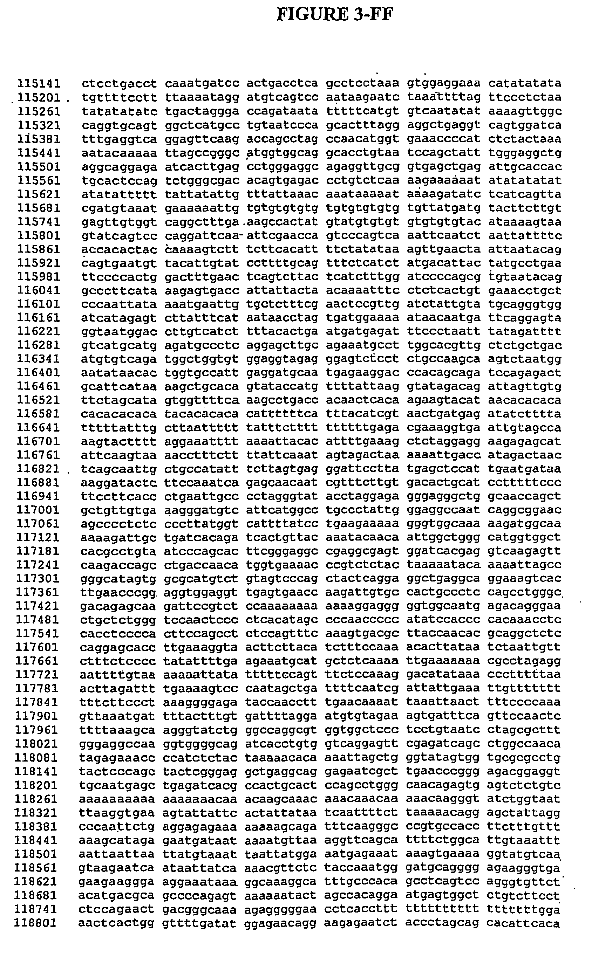

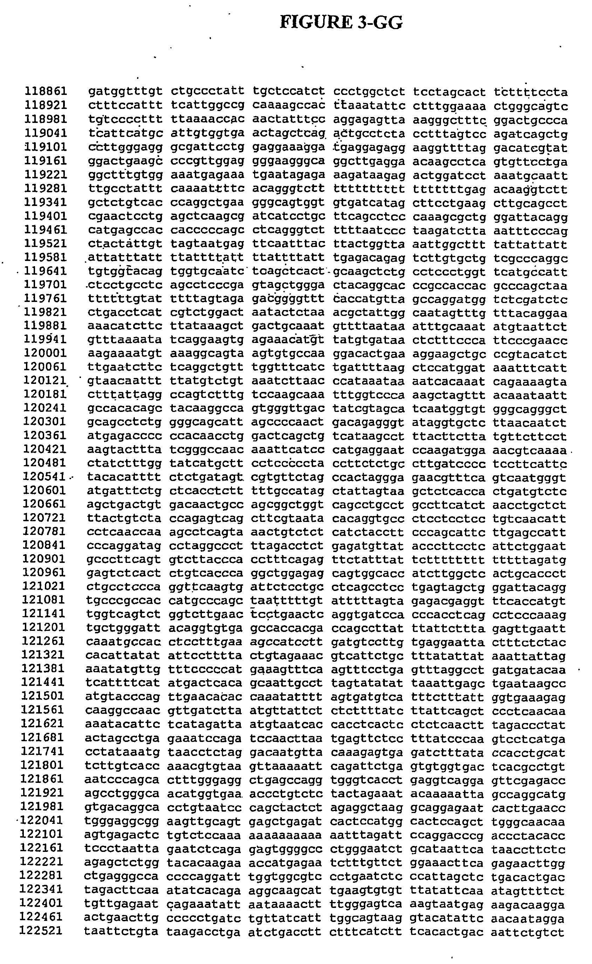

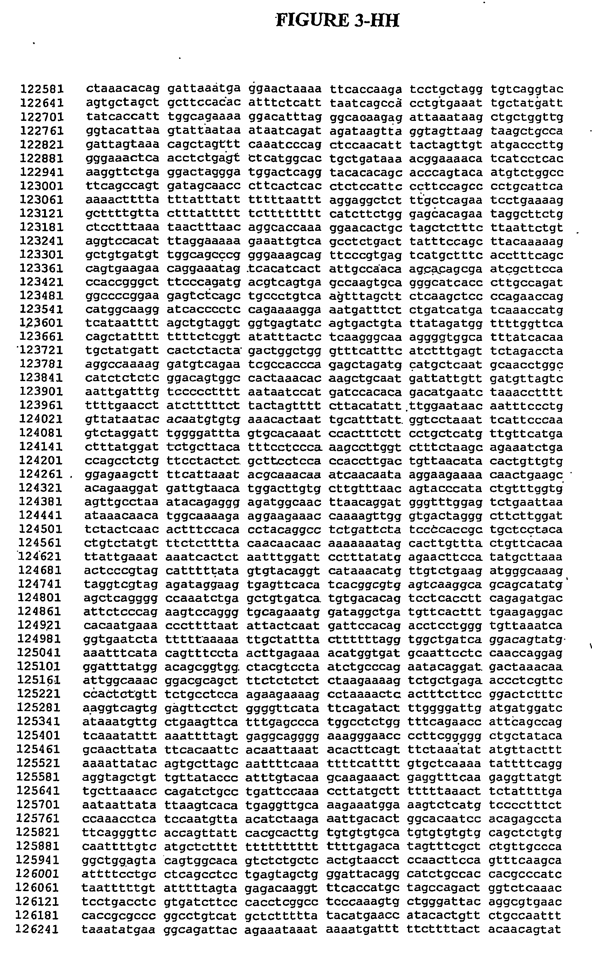

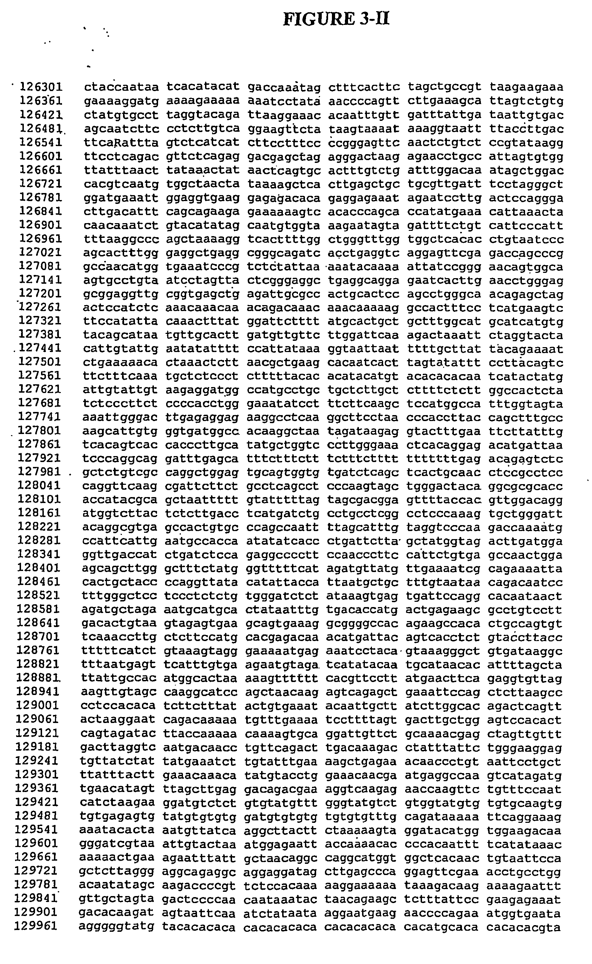

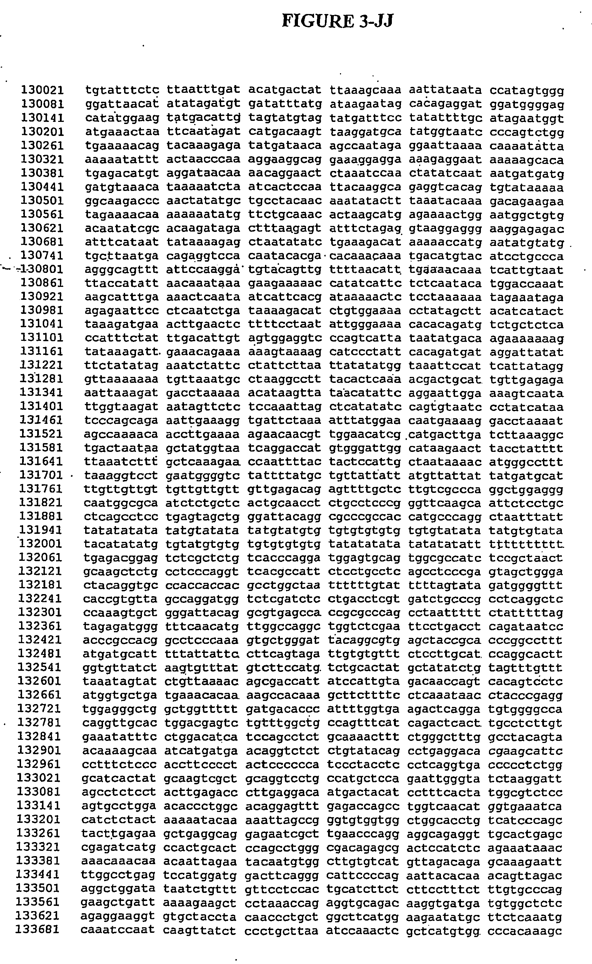

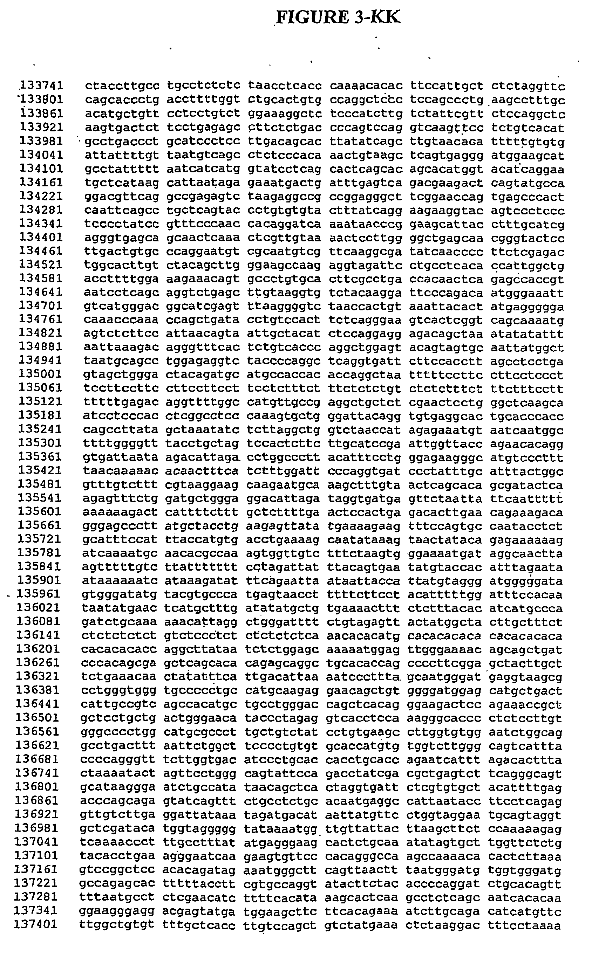

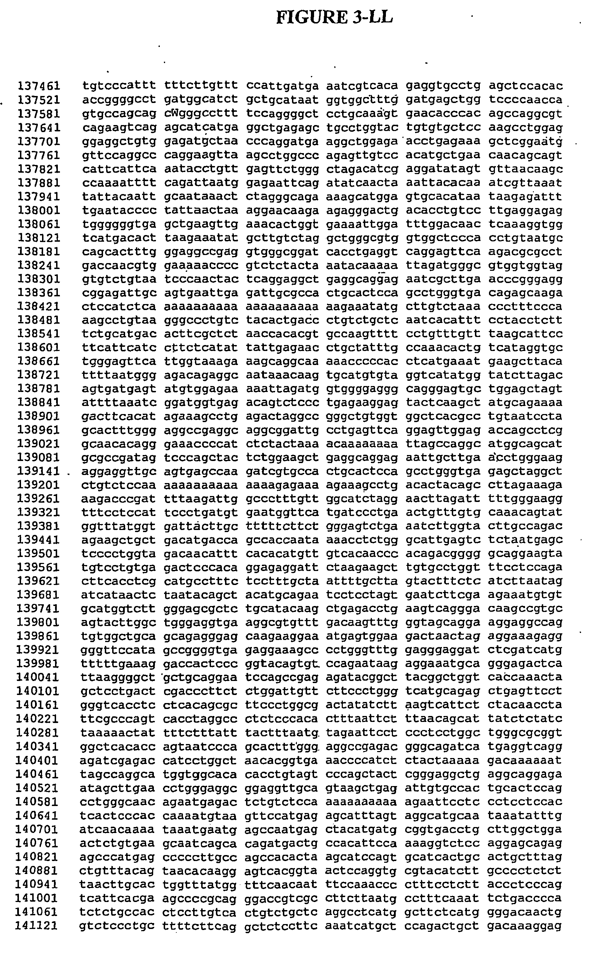

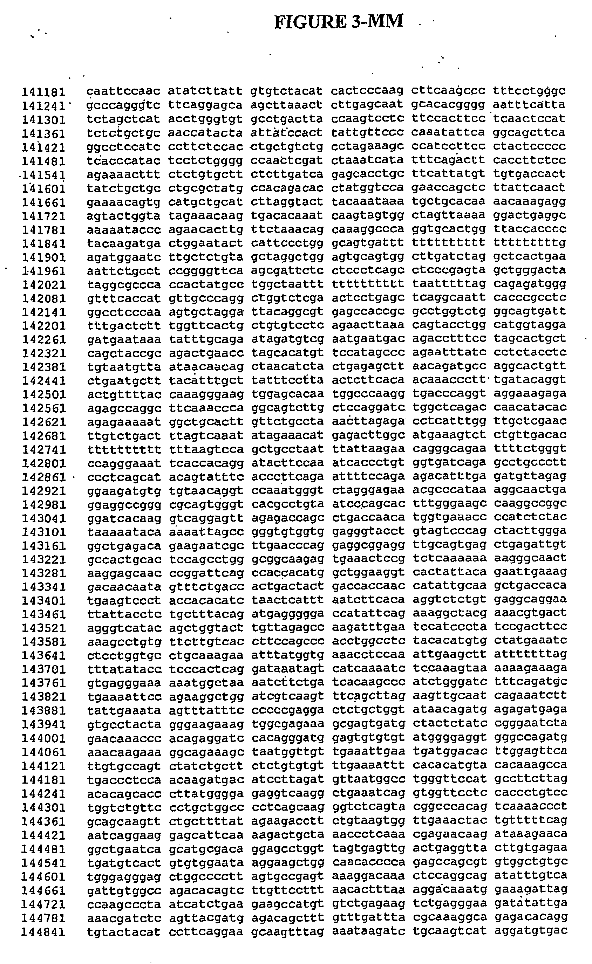

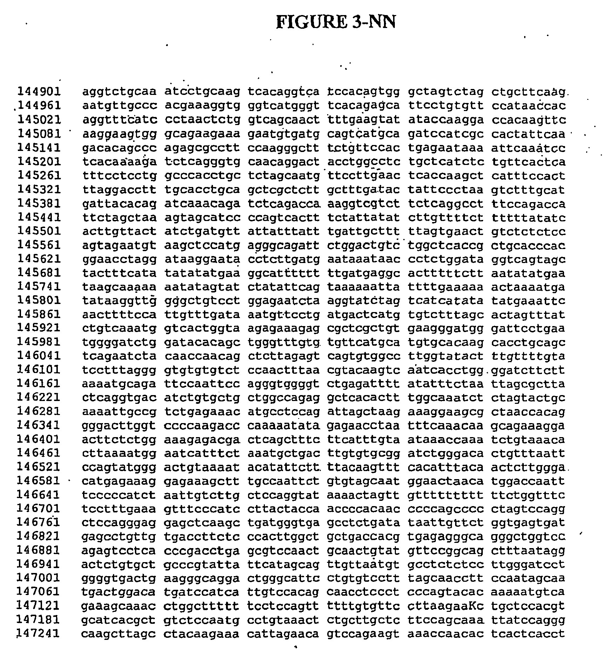

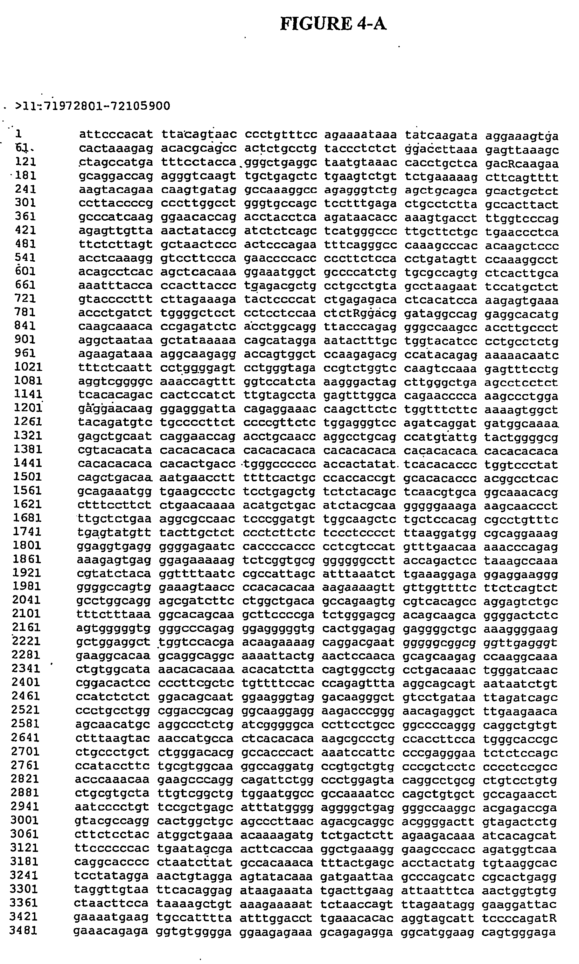

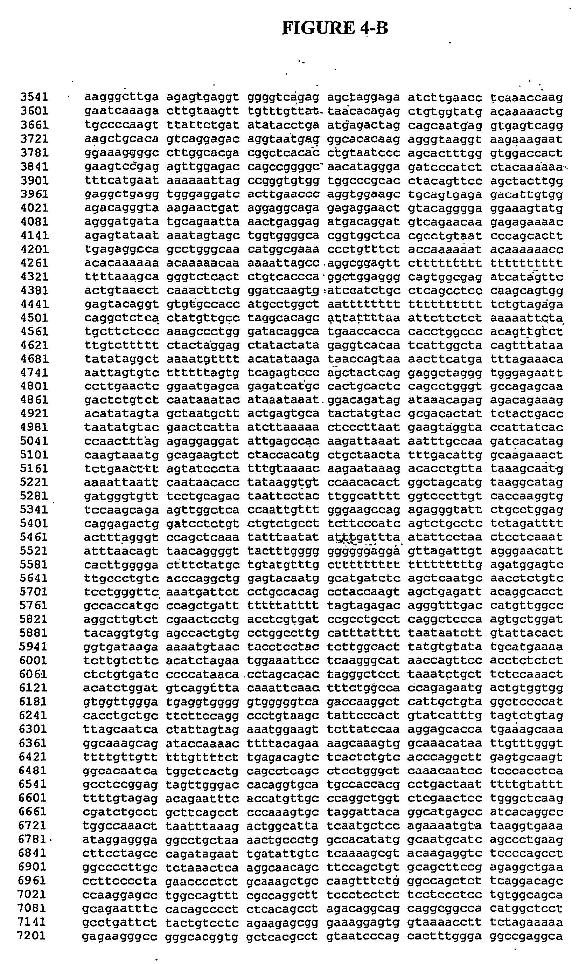

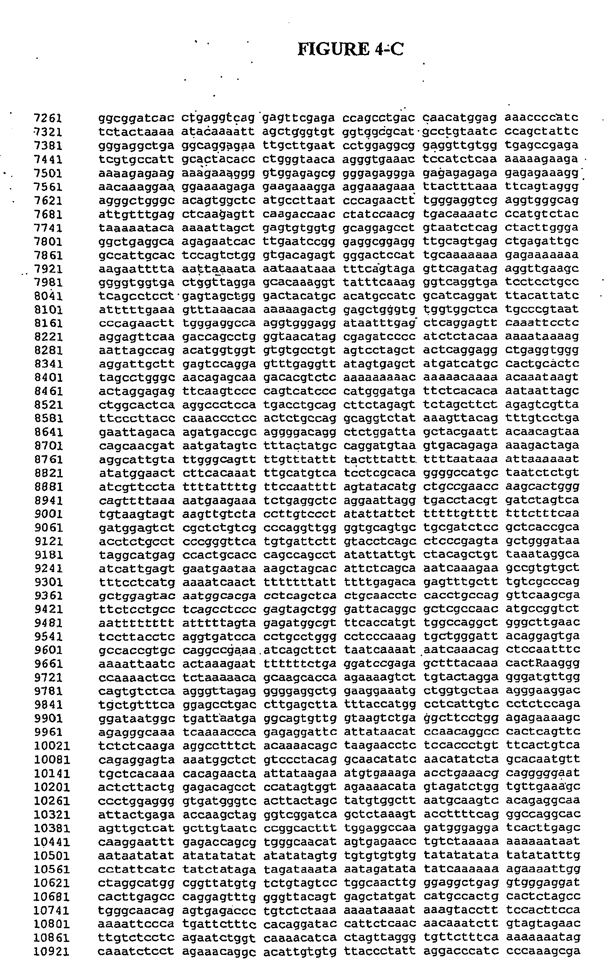

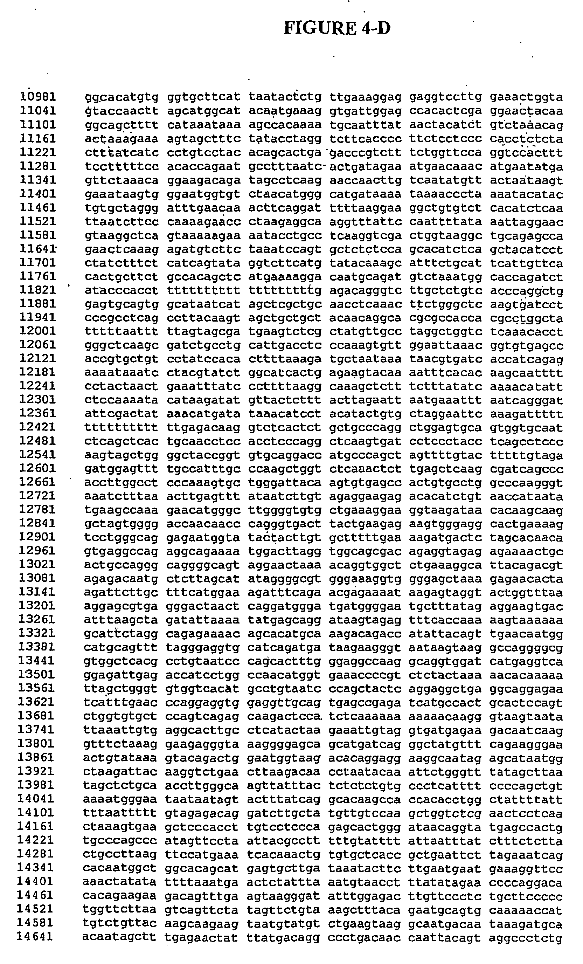

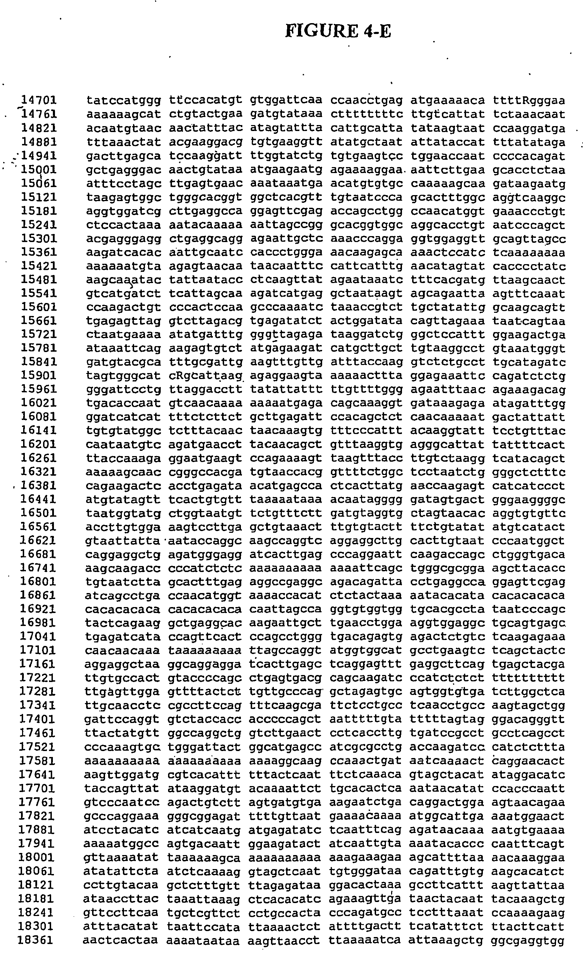

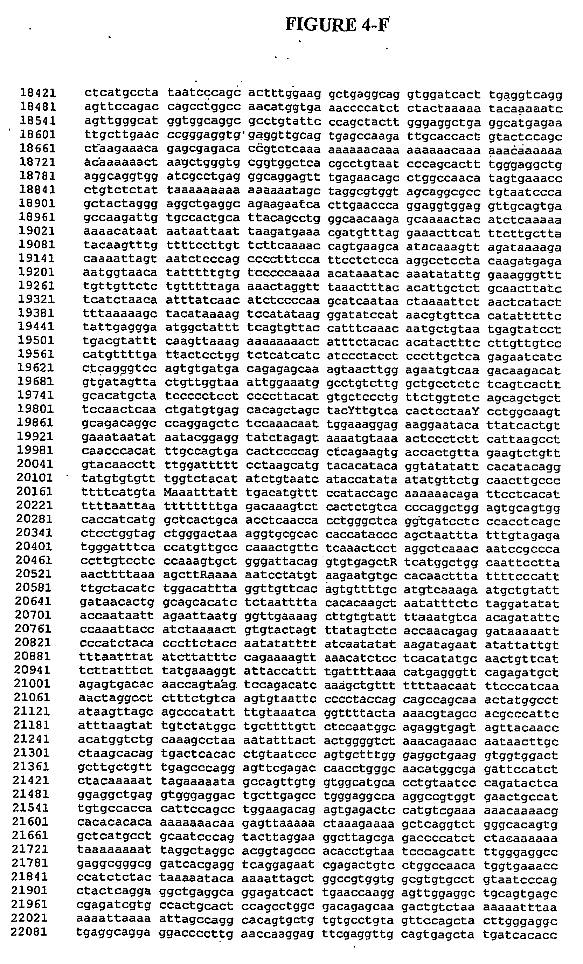

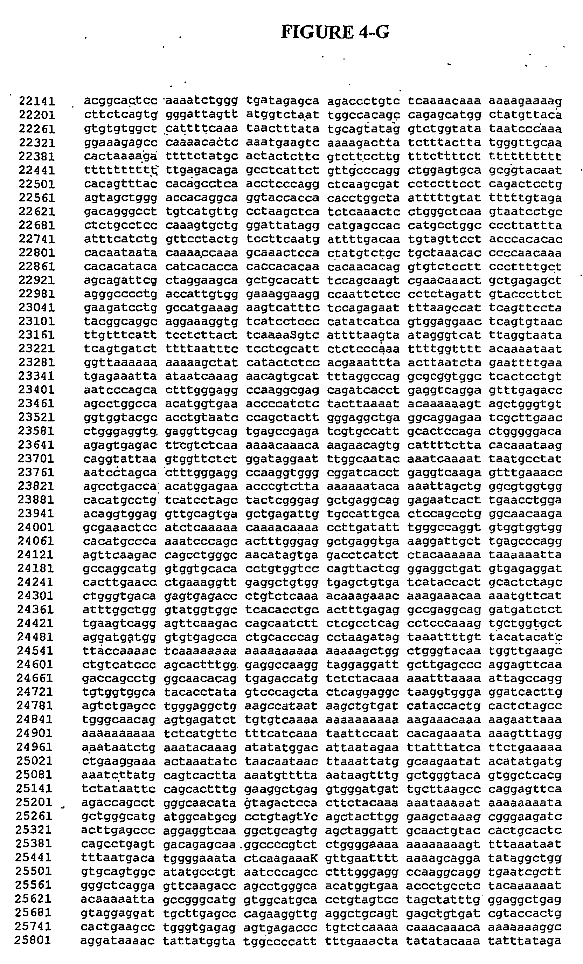

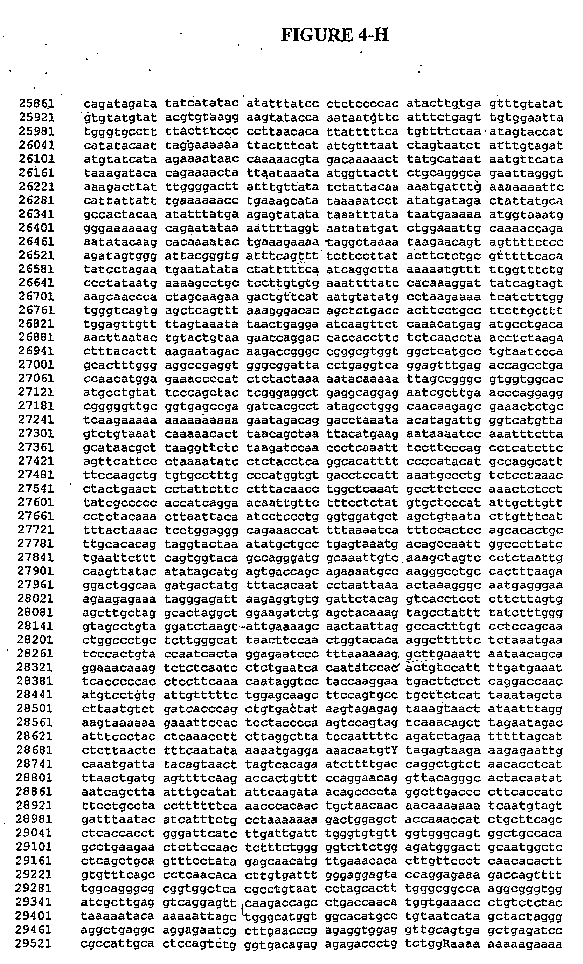

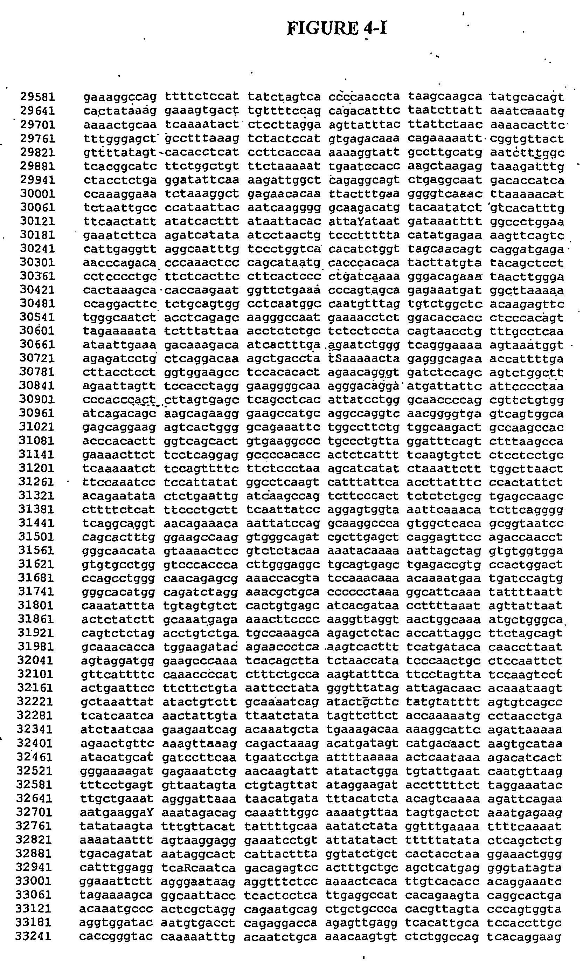

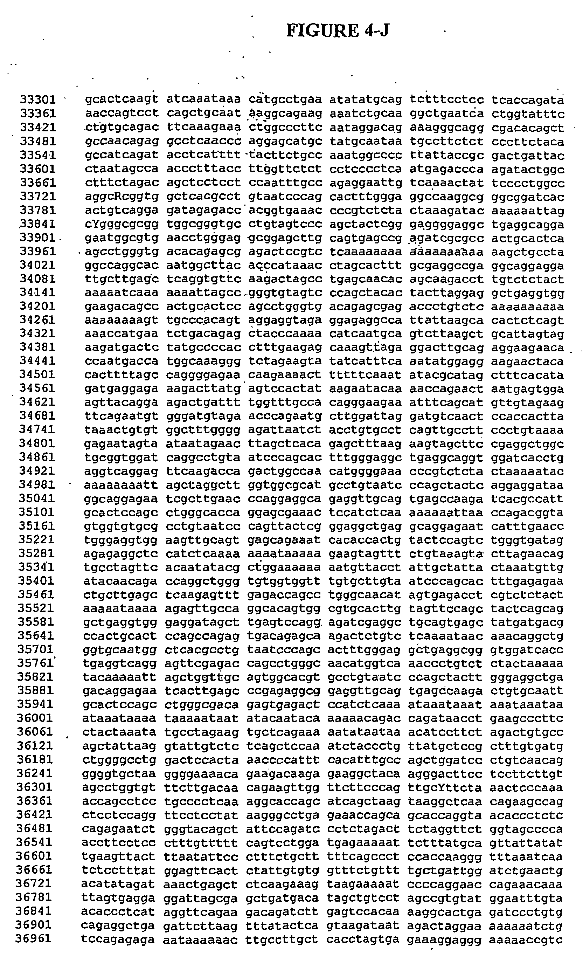

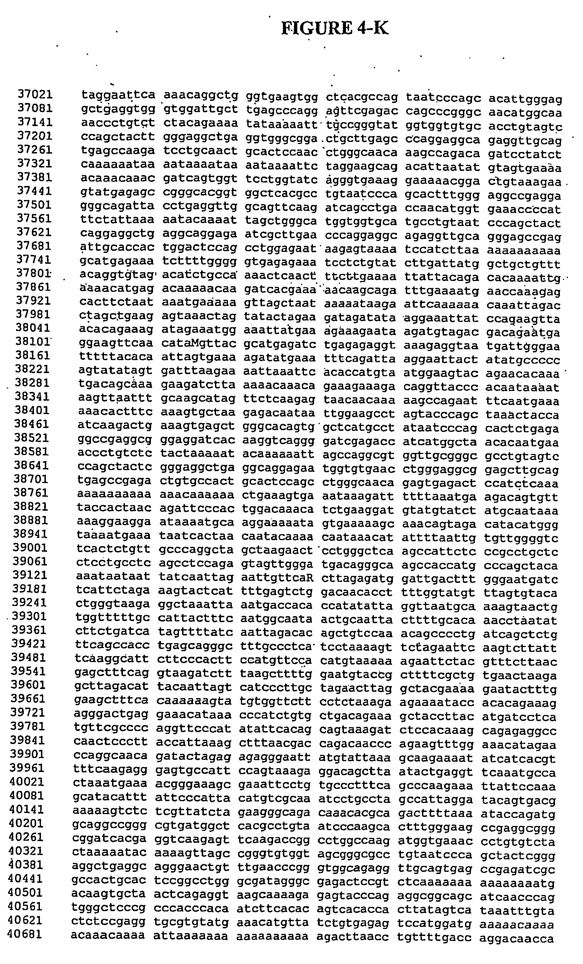

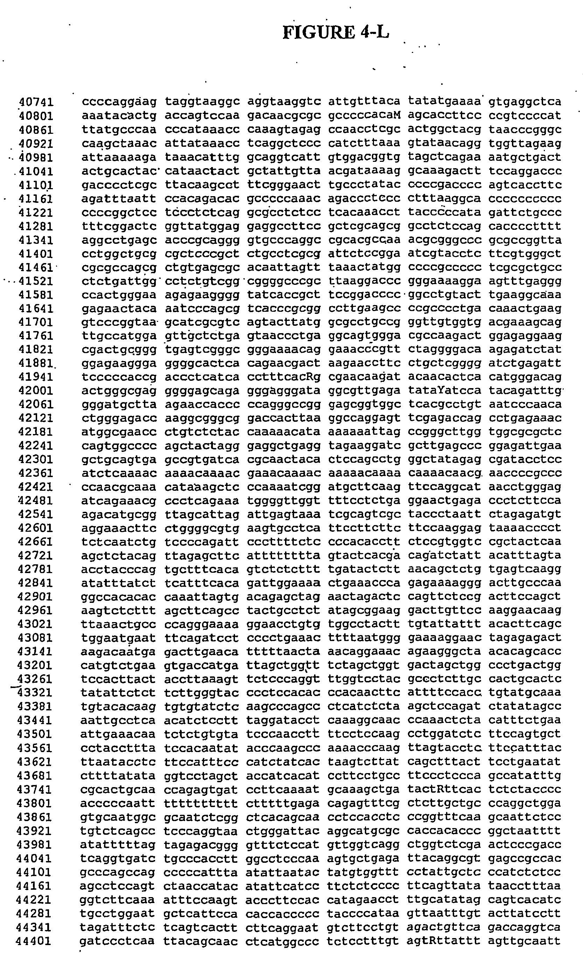

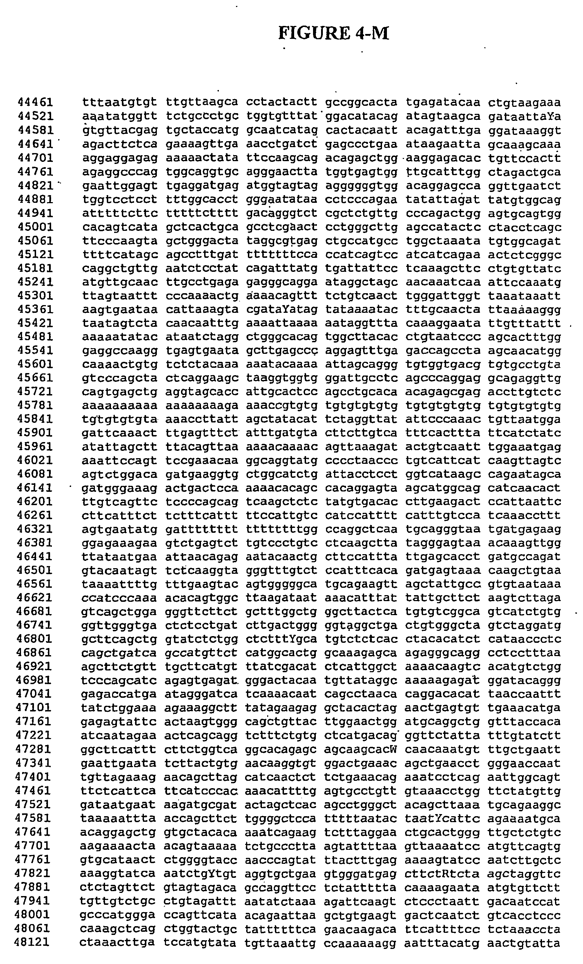

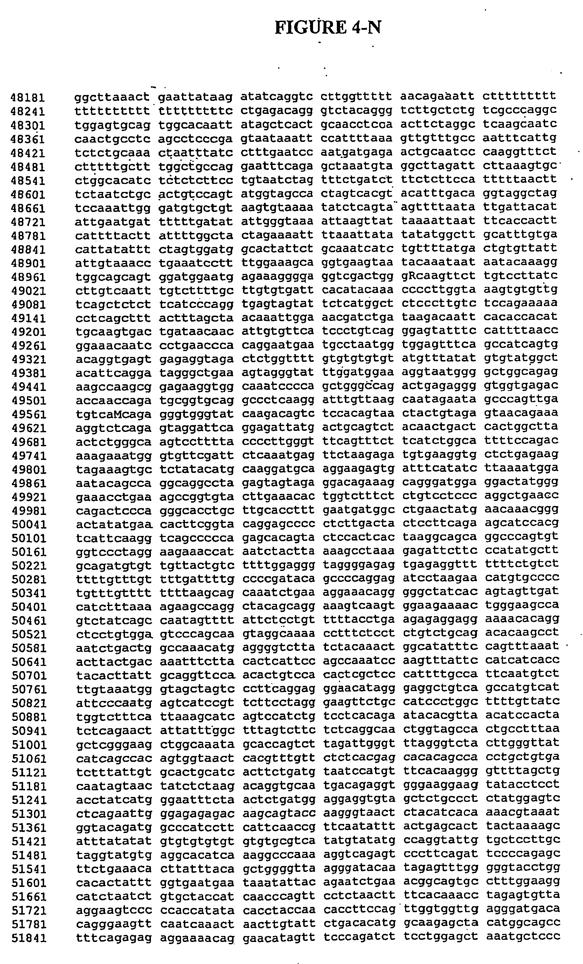

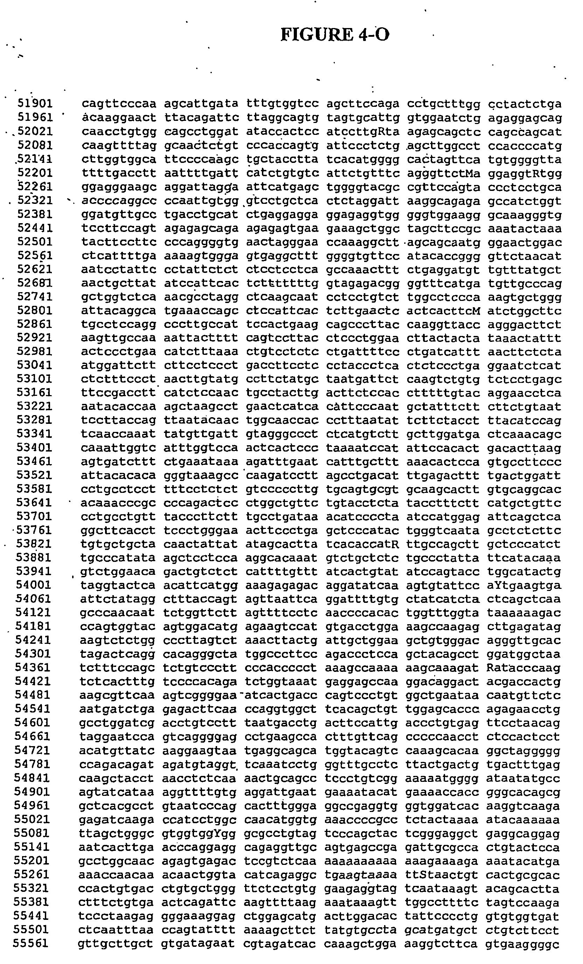

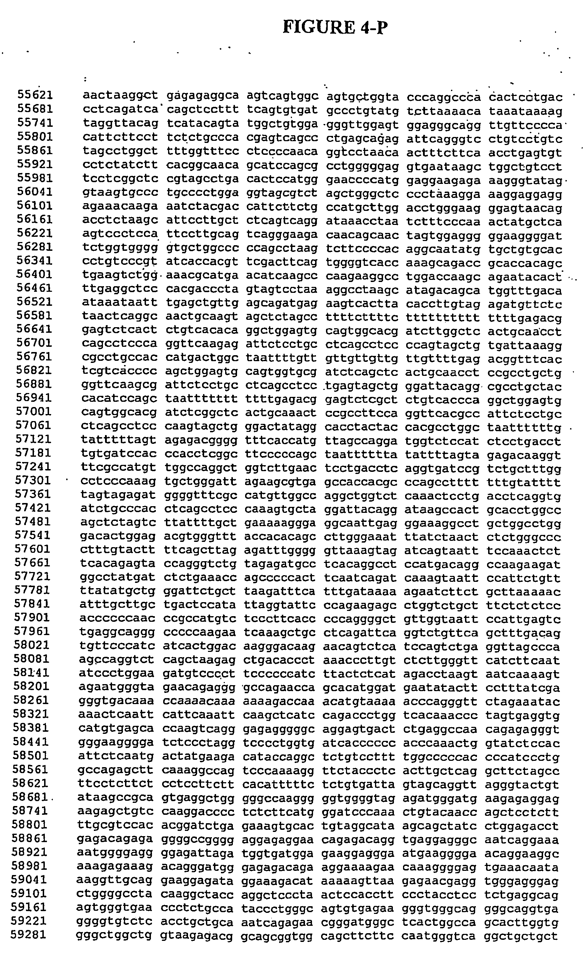

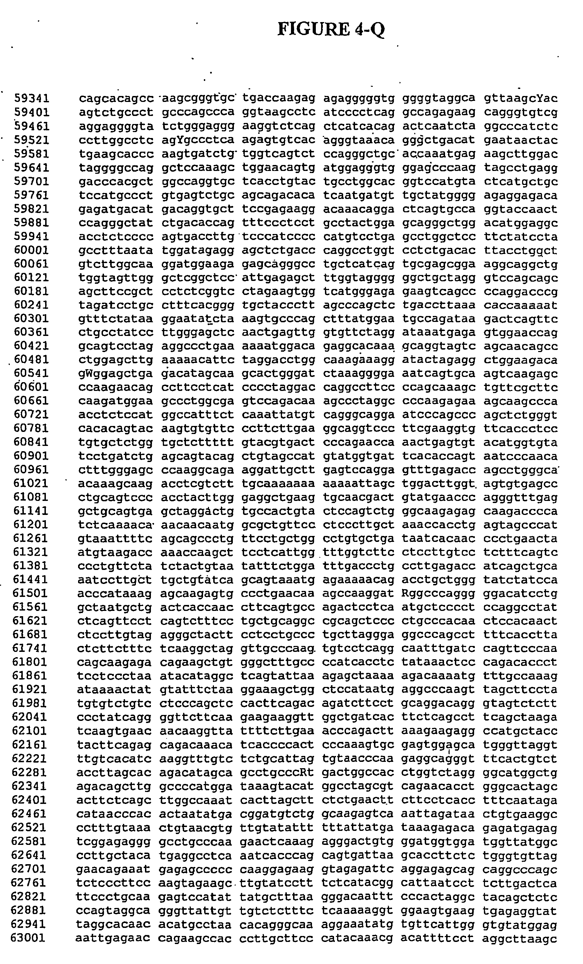

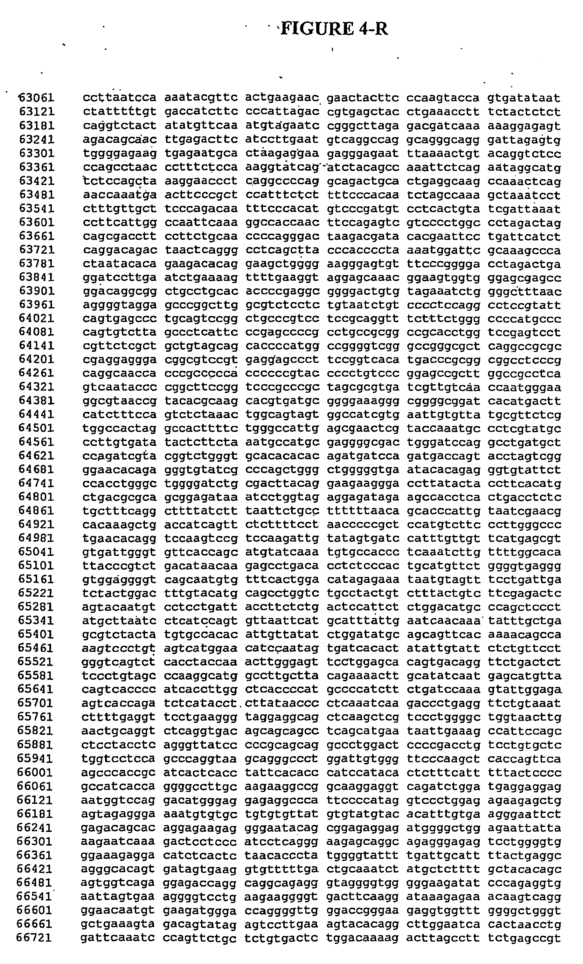

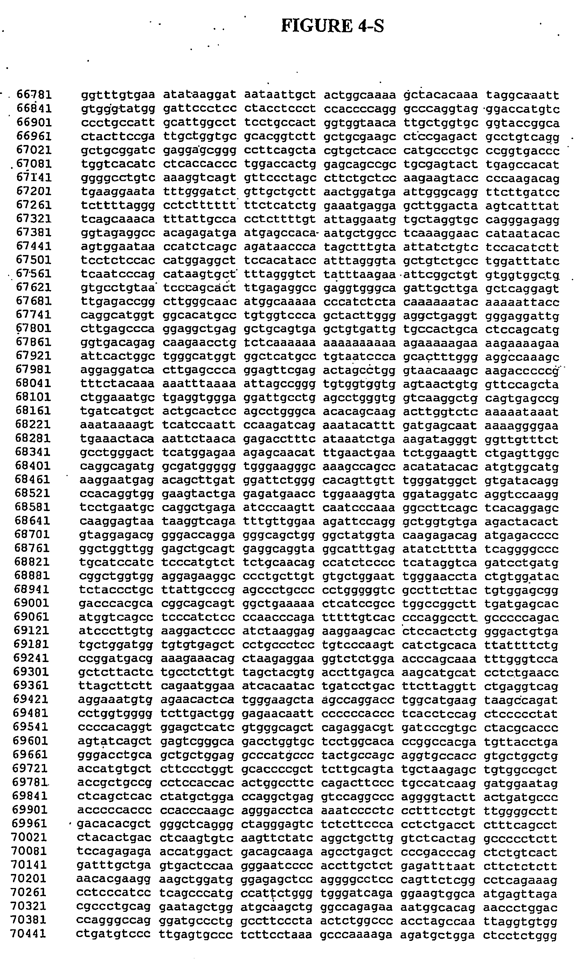

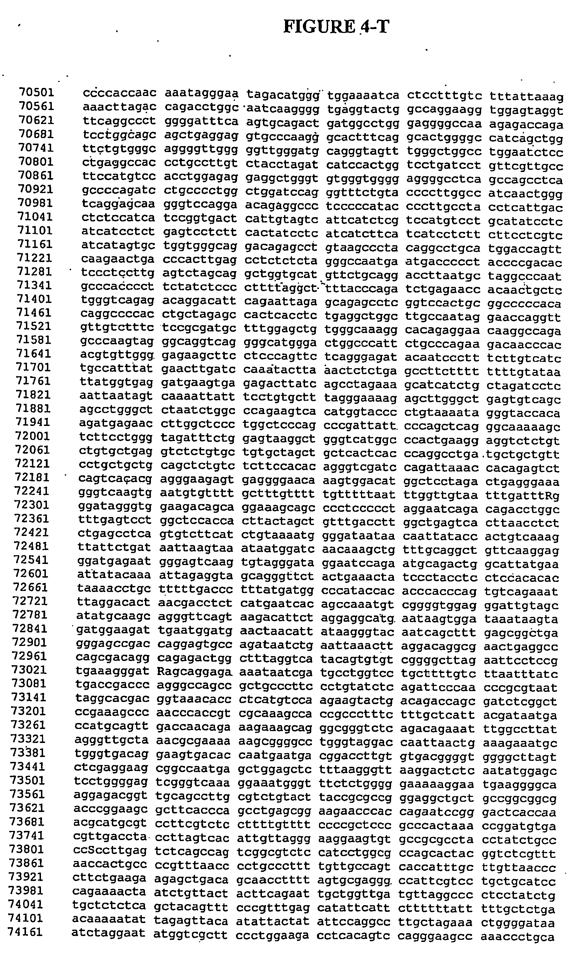

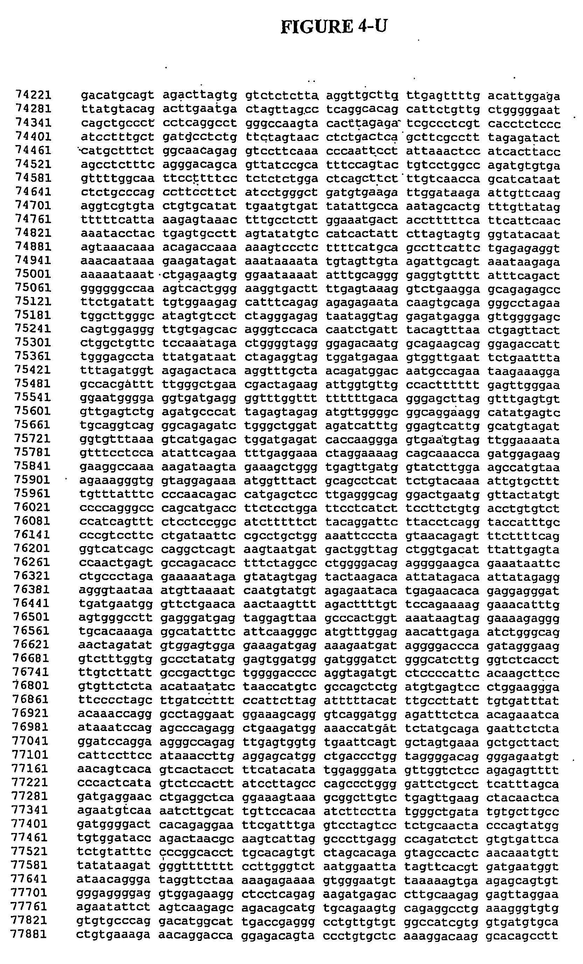

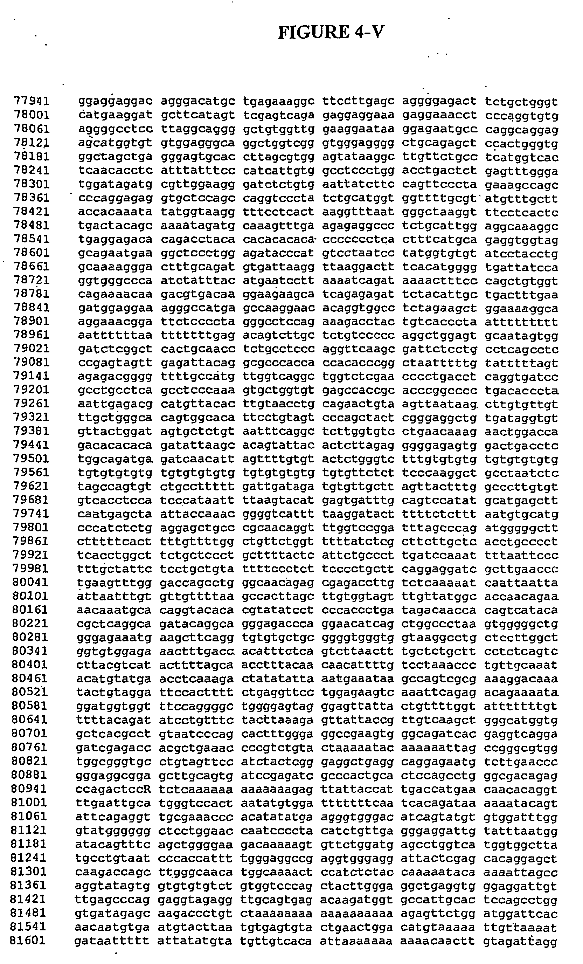

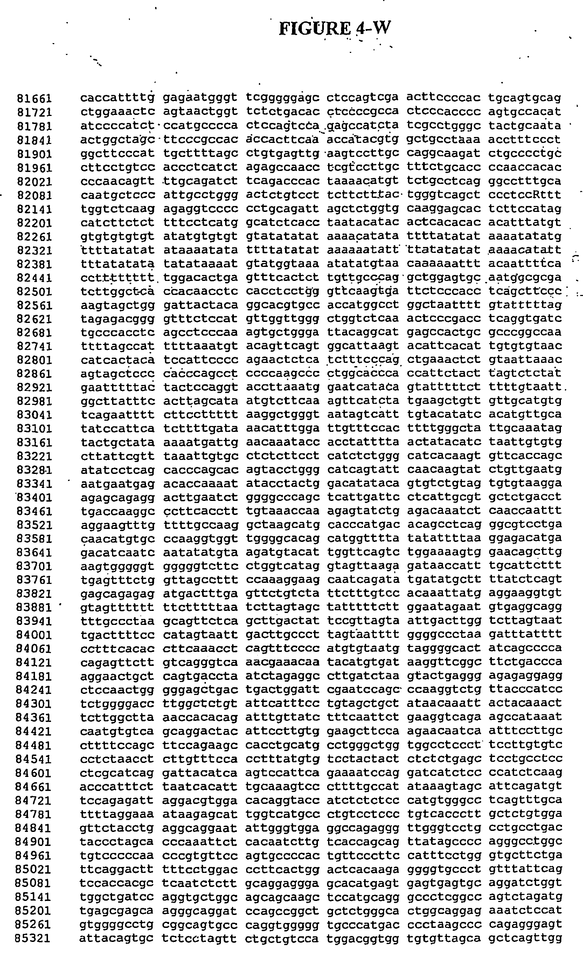

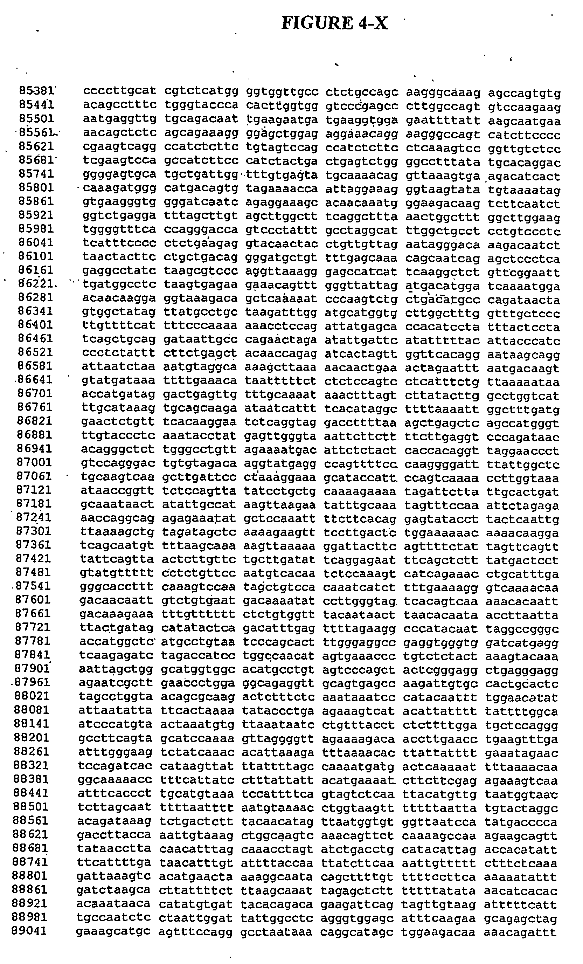

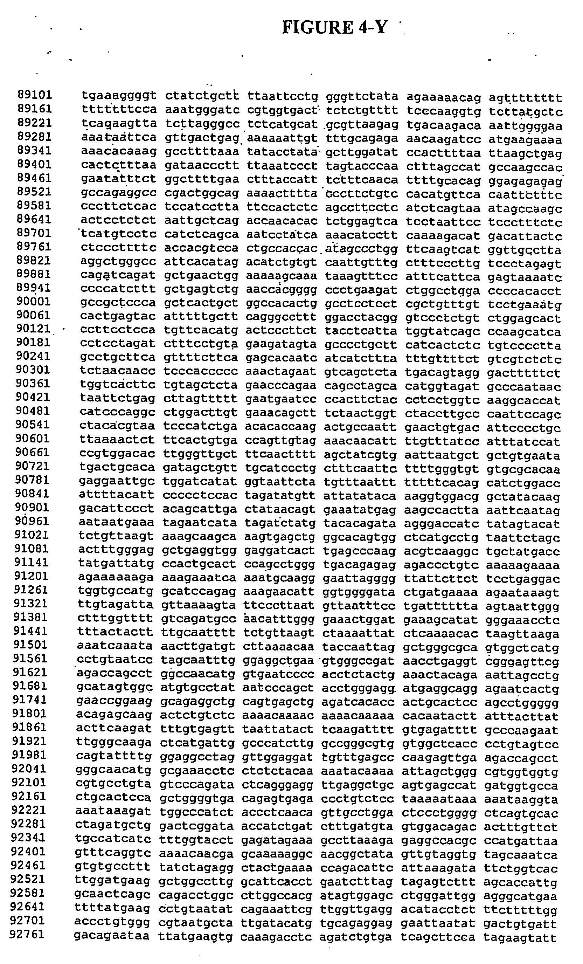

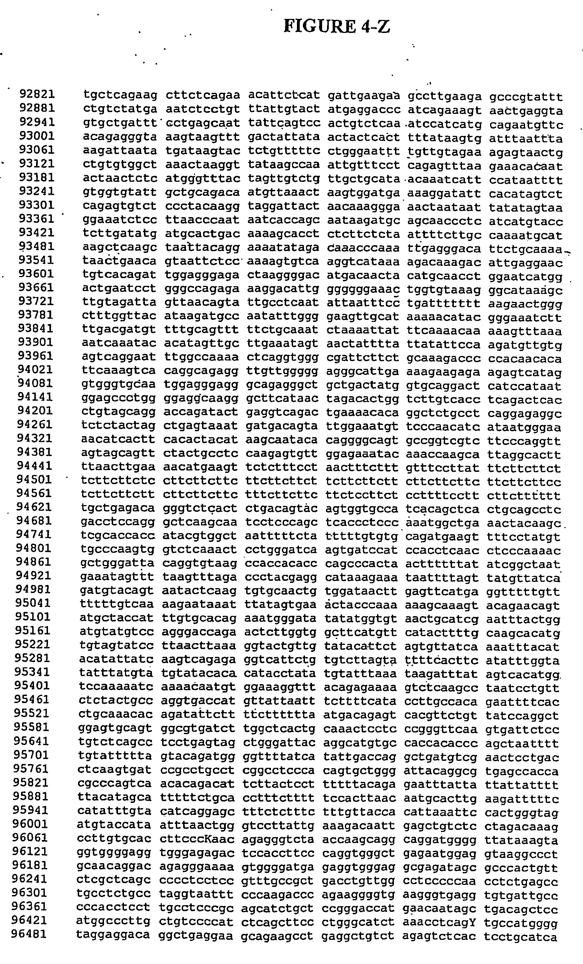

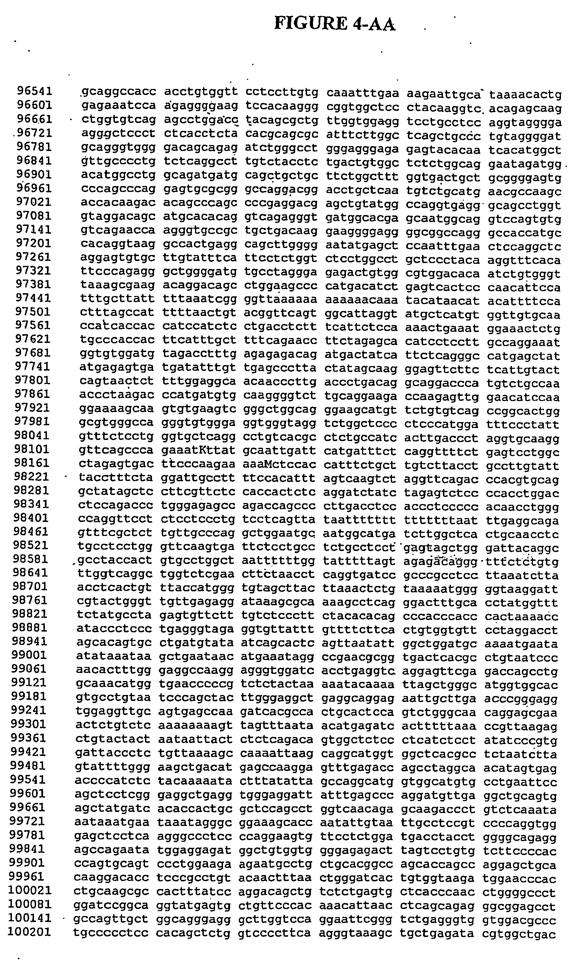

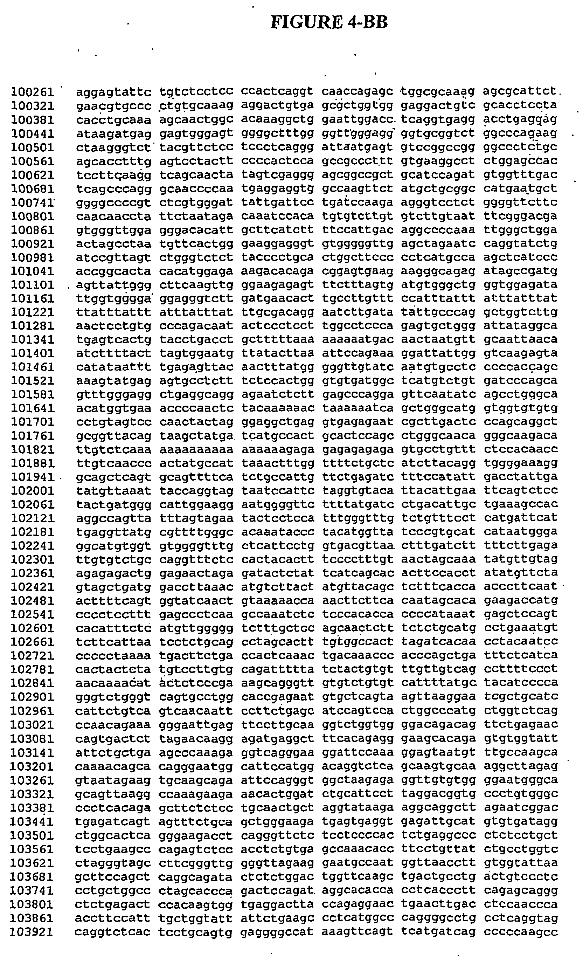

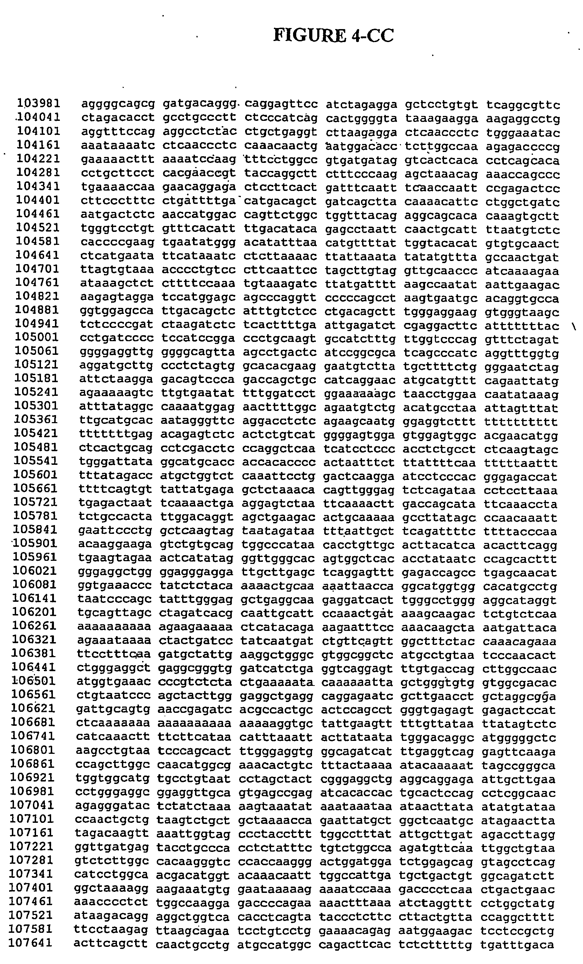

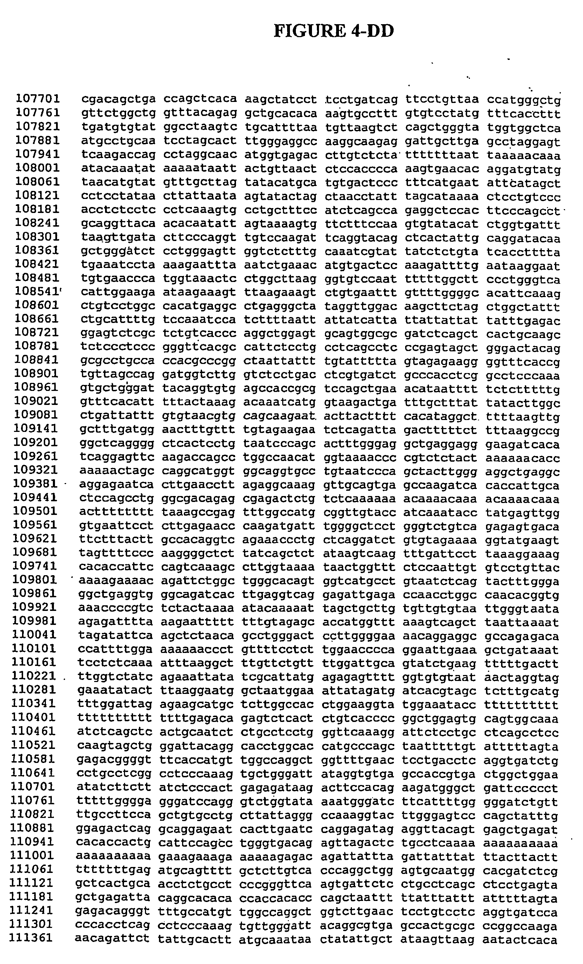

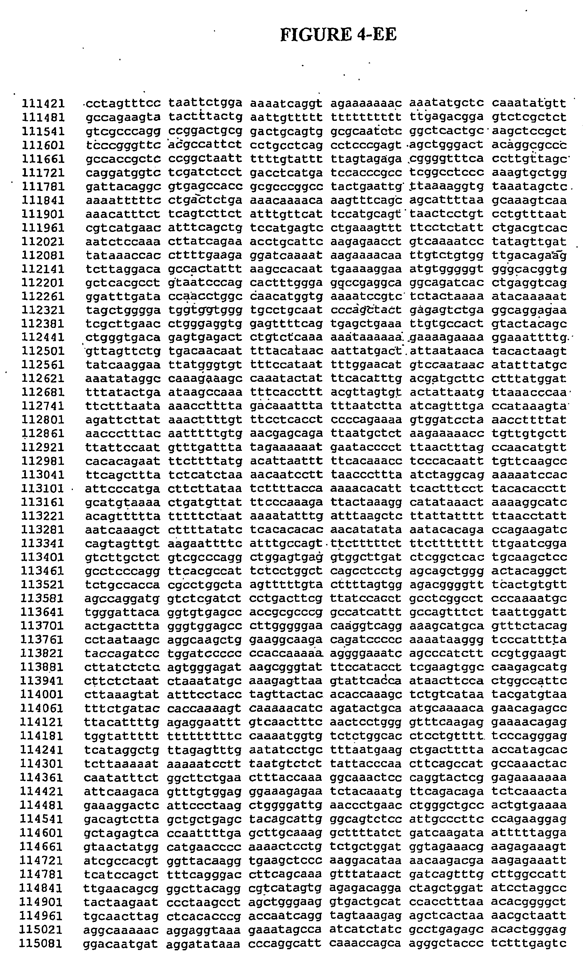

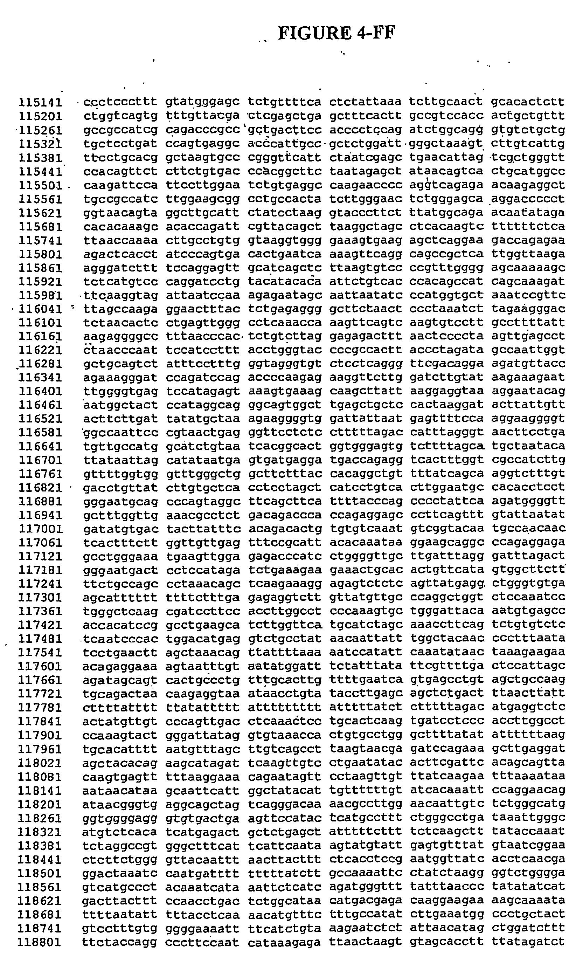

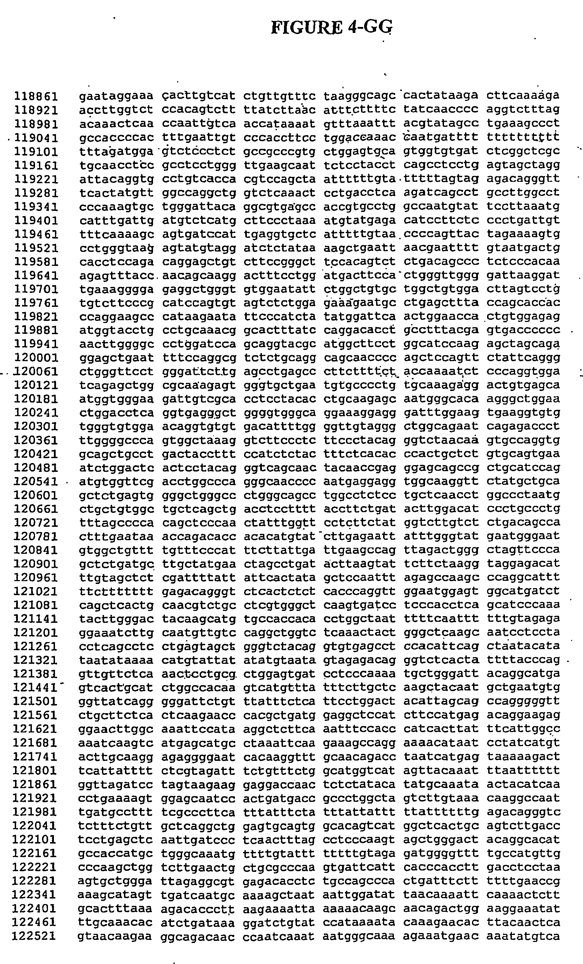

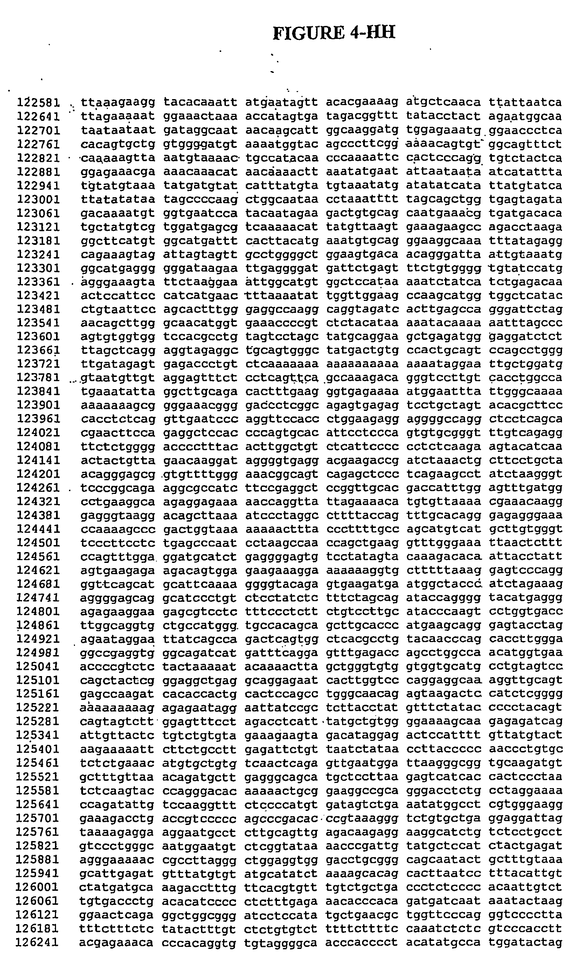

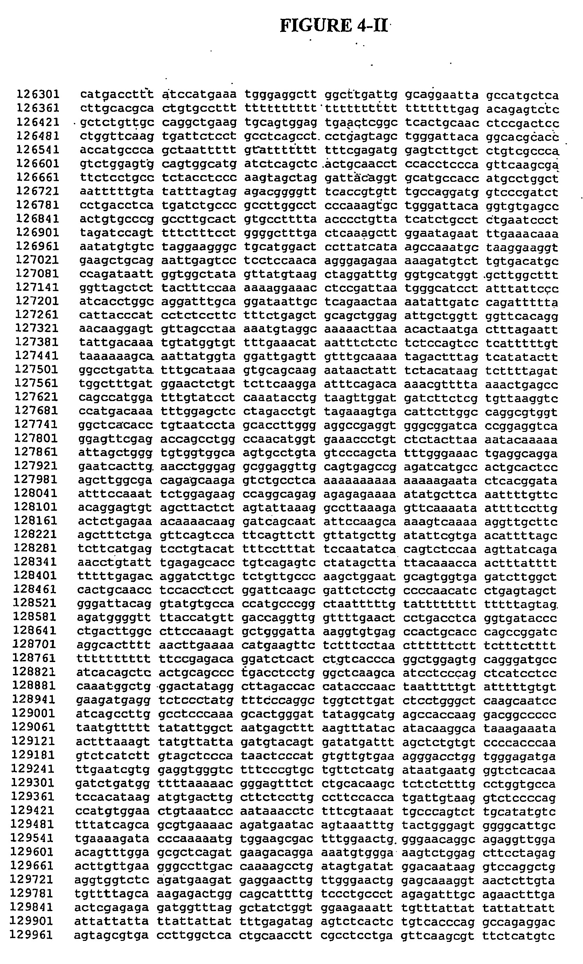

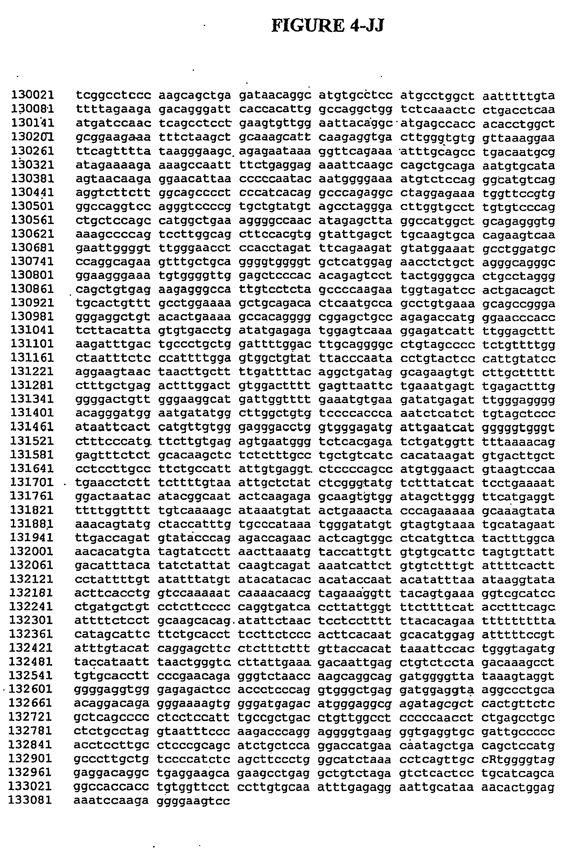

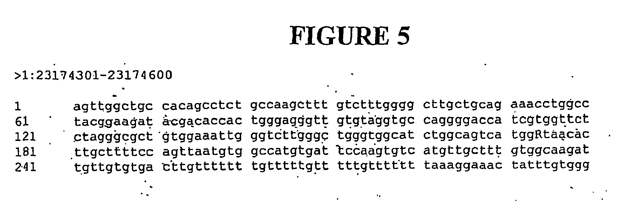

Table 3 includes information pertaining to the incident polymorphic variant associated with breast cancer identified herein. Public information pertaining to the polymorphism and the genomic sequence that includes the polymorphism are indicated. The genomic sequences identified in Table 3 may be accessed at the http address www.ncbi.nih.gov/entrez/query.fcgi, for example, by using the publicly available SNP reference number (e.g., rs1541998). The chromosome position refers to the position of the SNP within NCBI's Genome Build 33, which may be accessed at the following http address: www.ncbinlmnih.gov/mapview/map__search.cgi?chr=hum_chr.inf&query=. The "Contig Position" provided in Table 3 corresponds to a nucleotide position set forth in the contig sequence, and designates the polymorphic site corresponding to the SNP reference number. The sequence containing the polymorphisms also may be referenced by the "Sequence Identification" set forth in Table 3. The "Sequence Identification" corresponds to cDNA sequence that encodes associated target polypeptides (*e.g., NUMA1*) of the invention. The position of the SNP within the cDNA sequence is provided in the "Sequence Position" column of Table 3. Also, the allelic variation at the polymorphic site and the allelic variant identified as associated with breast cancer is specified in Table 3. All nucleotide sequences referenced and accessed by the parameters set forth in Table 3 are incorporated herein by reference. The positions for these SNPs are indicated in the tables below in Figures 1, 2, 3 and 4, and the incident SNP for the *GALE* region is at position 174 in Figure 5.

Assay for Verifying, Allelotyping, and Genotyping SNPs

A MassARRAY™ system (Sequenom, Inc.) was utilized to perform SNP genotyping in a high-throughput fashion. This genotyping platform was complemented by a homogeneous, single-tube assay method (hME™ or homogeneous MassEXTEND™ (Sequenom, Inc.)) in which two genotyping primers anneal to and amplify a genomic target surrounding a polymorphic site of interest. A third primer (the MassEXTEND™ primer), which is complementary to the amplified target up to but not including the polymorphism, was then enzymatically extended one or a few bases through the polymorphic site and then terminated.

For each polymorphism, SpectroDESIGNER™ software (Sequenom, Inc.) was used to generate a set of PCR primers and a MassEXTEND™ primer was used to genotype the polymorphism. Table 4 shows PCR primers and Table 5 shows extension primers used for analyzing polymorphisms. The initial PCR amplification reaction was performed in a 5 μl total volume containing 1X PCR buffer with 1.5 mM $MgCl_2$ (Qiagen), 200 μM each of dATP, dGTP, dCTP, dTTP (Gibco-BRL), 2.5 ng of genomic DNA, 0.1 units of HotStar DNA polymerase (Qiagen), and 200 nM each of forward and reverse PCR primers specific for the polymorphic region of interest.

**Table 4: PCR Primers**

| Reference SNP ID | Forward PCR primer | Reverse PCR primer |
|---|---|---|
| 1056538 | GACAGCCACAGCTAGCGCAGA | TGTTTTCGCCCCCCAGGGTGAC |
| 1541998 | CTGATTATTCTGATGGTAATG | GCCCATGTTAACATTTTCTTC |
| 2001449 | ATGTCAAGTGCACCCACATG | AGGAAGAAACTGACGGAAGG |
| 673478 | TAATACAAAGGTGGCAGCAG | TTGACAAGGATAAGGACAAG |
| 4237 | GCACATGGCCACATTAACTGG | TGGCTGTGGAAATTGGGTCTTG |

Samples were incubated at 95°C for 15 minutes, followed by 45 cycles of 95°C for 20 seconds, 56°C for 30 seconds, and 72°C for 1 minute, finishing with a 3 minute final extension at 72°C. Following amplification, shrimp alkaline phosphatase (SAP) (0.3 units in a 2 μl volume) (Amersham Pharmacia) was added to each reaction (total reaction volume was 7 μl) to remove any residual dNTPs that were not consumed in the PCR step. Samples were incubated for 20 minutes at 37°C, followed by 5 minutes at 85°C to denature the SAP.

Once the SAP reaction was complete, a primer extension reaction was initiated by adding a polymorphism-specific MassEXTEND™ primer cocktail to each sample. Each MassEXTEND™ cocktail included a specific combination of dideoxynucleotides (ddNTPs) and deoxynucleotides (dNTPs) used to distinguish polymorphic alleles from one another. In Table 5, ddNTPs are shown and the fourth nucleotide not shown is the dNTP.

**Table 5: Extend Primers**

| Reference SNP ID | Extend Probe | Term Mix |
|---|---|---|
| 1056538 | CCCAGGGTGACGTTGCAGA | ACG |
| 1541998 | ATTATTCTGATGGTAATGATCCAG | ACG |
| 2001449 | CACATGCCTGCTCGCCCCC | ACT |
| 673478 | AAGGGGAGGTCGACTGGG | ACT |
| 4237 | GGCATCTGGCAGTCATGG | ACT |

The MassEXTEND™ reaction was performed in a total volume of 9 μl, with the addition of 1X ThermoSequenase buffer, 0.576 units of ThermoSequenase (Amersham Pharmacia), 600 nM MassEXTEND™ primer, 2 mM of ddATP and/or ddCTP and/or ddGTP and/or ddTTP, and 2 mM of dATP or dCTP or dGTP or dTTP. The deoxy nucleotide (dNTP) used in the assay normally was complementary to the nucleotide at the polymorphic site in the amplicon. Samples were incubated at 94˚C for 2 minutes, followed by 55 cycles of 5 seconds at 94˚C, 5 seconds at 52˚C, and 5 seconds at 72˚C.

Following incubation, samples were desalted by adding 16 μl of water (total reaction volume was 25 μl), 3 mg of SpectroCLEAN™ sample cleaning beads (Sequenom, Inc.) and allowed to incubate for 3 minutes with rotation. Samples were then robotically dispensed using a piezoelectric dispensing device (SpectroJET™ (Sequenom, Inc.)) onto either 96-spot or 384-spot silicon chips containing a matrix that crystallized each sample (SpectroCHIP® (Sequenom, Inc.)). Subsequently, MALDI-TOF mass spectrometry (Biflex and Autoflex MALDI-TOF mass spectrometers (Bruker Daltonics) can be used) and SpectroTYPER RT™ software (Sequenom, Inc.) were used to analyze and interpret the SNP genotype for each sample.

Genetic Analysis

Variations identified in the target genes are provided in their respective genomic sequences (see Figures 1-5) Minor allelic frequencies for these polymorphisms was verified as being 10% or greater by determining the allelic frequencies using the extension assay described above in a group of samples isolated from 92 individuals originating from the state of Utah in the United States, Venezuela and France (Coriell cell repositories).

Genotyping results are shown for female pools in Table 6A and 6B. Table 6A shows the orginal genotyping results and Table 6B shows the genotyped results re-analyzed to remove duplicate individuals from the cases and controls (*i.e.*, individuals who were erroneously included more than once as either cases or controls). Therefore, Table 6B represents a more accurate measure of the allele frequencies for this particular SNP. In the subsequent tables, "AF" refers to allelic frequency; and "F case" and "F control" refer to female case and female control groups, respectively.

**Table 6A**

| Reference SNP ID | AF F case | AF F control | p-value | Odds Ratio | Breast Cancer Assoc. Allele |
|---|---|---|---|---|---|
| 1056538 | C = 0.651<br>T = 0.349 | C = 0.564<br>T = 0.436 | **0.0038** | 0.69 | C |
| 1541998 | **T** = 0.780<br>C = 0.220 | **T** = 0.839<br>C = 0.161 | **0.0153** | 0.69 | C |
| 2001449 | **G** = 0.703<br>C = 0.297 | **G** = 0.780<br>C = 0.220 | **0.0040** | 1.49 | C |
| 673478 | **T** = 0.919<br>C = 0.081 | **T** = 0.953<br>C = 0.047 | **0.0238** | 1.74 | C |
| 4237 | A = 0.590<br>G = 0.410 | A = 0.530<br>G = 0.470 | **0.0431** | 0.78 | A |

**Table 6B**

| Reference SNP ID | AF F case | AF F control | p-value | Odds Ratio | Breast Cancer Assoc. Allele |
|---|---|---|---|---|---|
| 1056538 | C = 0.658<br>T = 0.342 | C = 0.556<br>T = 0.444 | **0.0012** | 0.65 | C |
| 1541998 | **T** = 0.771<br>C = 0.229 | **T** = 0.839<br>C = 0.161 | **0.0070** | 0.65 | C |
| 2001449 | **G** = 0.693<br>C = 0.307 | **G** = 0.782<br>C = 0.218 | **0.0012** | 1.59 | C |
| 673478 | **T** = 0.916<br>C = 0.084 | **T** = 0.953<br>C = 0.047 | **0.0171** | 1.85 | C |
| 4237 | A = 0.584<br>G = 0.416. | A = 0.527<br>G = 0.473 | 0.0704 | 0.79 | A |

The single marker alleles set forth in Table 3 were considered validated, since the genotyping data for the females, males or both pools were significantly associated with breast cancer, and because the genotyping results agreed with the original allelotyping results. Particularly significant associations with breast cancer are indicated by a calculated p-value of less than 0.05 for genotype results, which are set forth in bold text.

Odds ratio results are shown in Tables 6A and 6B. An odds ratio is an unbiased estimate of relative risk which can be obtained from most case-control studies. Relative risk (RR) is an estimate of the likelihood of disease in the exposed group (susceptibility allele or genotype carriers) compared to the unexposed group (not carriers). It can be calculated by the following equation:

$$\mathbf{RR} = \boldsymbol{I}\mathbf{A}/\boldsymbol{I}\mathbf{a}$$

*I*A is the incidence of disease in the A carriers and *I*a is the incidence of disease in the non-carriers.

**RR > 1 indicates the A allele increases disease susceptibility.**

RR < 1 indicates the a allele increases disease susceptibility.

For example, RR = 1.5 indicates that carriers of the A allele have 1.5 times the risk of disease than non-carriers, *i.e.*, 50% more likely to get the disease.

Case-control studies do not allow the direct estimation of *I*A and *I*a, therefore relative risk cannot be directly estimated. However, the odds ratio (OR) can be calculated using the following equation:

$$\mathbf{OR} = (\mathbf{nDAnda})/(\mathbf{ndAnDa}) = \boldsymbol{p}\mathbf{DA}(1 - \mathbf{pdA})/\boldsymbol{p}\mathbf{dA}(1 - \mathbf{pDA}),$$

or

**OR = ((case f) / (1- case f)) / ((control f) / (1-control f)),** where f = susceptibility allele frequency.

An odds ratio can be interpreted in the same way a relative risk is interpreted and can be directly estimated using the data from case-control studies, i.e., case and control allele frequencies. The higher the odds ratio value, the larger the effect that particular allele has on the development of breast cancer. Possessing an allele associated with a relatively high odds ratio translates to having a higher risk of developing or having breast cancer.

<u>Example 3</u>

Samples and Pooling Strategies for the Replication Samples

The SNPs of Table 3 were genotyped again in a collection of replication samples to further validate its association with breast cancer. Like the original study population described in Examples 1 and 2, the replication samples consisted of females diagnosed with breast cancer (cases) and females without cancer (controls). The case and control samples were selected and genotyped as described below.

Pooling Strategies

Samples were placed into one of two groups based on disease status. The two groups were female case groups and female control groups. A select set of samples from each group were utilized to generate pools, and one pool was created for each group. Each individual sample in a pool was represented by an equal amount of genomic DNA. For example, where 25 ng of genomic DNA was utilized in each PCR reaction and there were 190 individuals in each pool (i.e., 190 cases and 190 controls), each individual would provide 125 pg of genomic DNA. Inclusion or exclusion of samples for a pool was based upon the following criteria: the sample was derived from a female individual characterized as Caucasian from Australia; case samples were derived from individuals diagnosed with breast cancer; control samples were derived from individuals free of cancer and no family history of breast cancer; and sufficient genomic DNA was extracted from each blood sample for all allelotyping and genotyping reactions performed during the study. Samples in the pools also were age-matched. Samples that met these criteria were added to appropriate pools based on gender and disease status.

The selection process yielded the pools set forth in Table 7, which were used in the studies that follow:

**Table 7**

| | **Female CASE** | **Female CONTROL** |
|---|---|---|
| **Pool size** (Number) | 190 | 190 |
| **Pool Criteria** (ex: case/control) | Case | control |
| **Mean Age** (ex: years) | 64.5 | ** |
| **Each case was matched by a control within 5 years of age of the case. | | |

The replication genotyping results are shown in Table 8. The odds ratio was calculated as described in Example 2.

**Table 8**

| **Reference SNP ID** | **AF F case** | **AF F control** | **p-value** | **Odds Ratio** |
|---|---|---|---|---|
| 1056538 | C = 0.650<br>T = 0.350 | C = 0.584<br>T = 0.416 | **0.0624** | 0.75 |
| 1541998 | **T** = 0.820<br>C = 0.180 | **T** = 0.864<br>C=0.136 | 0.1010 | 0.72 |
| 2001449 | **G** = 0.685<br>C = 0.315 | **G** = 0.777<br>C = 0.223 | **0.005** | 1.59 |
| 673478 | **T** = 0.927<br>C = 0.073 | **T** = 0.957<br>C = 0.043 | **0.077** | 1.76 |
| 4237 | A = 0.632<br>G = 0.368 | A = 0.577<br>G = 0.423 | 0.1260 | 1.26 |

The absence of a statistically significant association in the replication cohort should not be interpreted as minimizing the value of the original finding. There are many reasons why a biologically derived association identified in a sample from one population would not replicate in a samples from another population. The most important reason is differences in population history. Due to bottlenecks and founder effects, there may be common disease predisposing alleles present in one population that are relatively rare in another, leading to a lack of association in the candidate region. Also, because common diseases such as breast cancer are the result of susceptibilities in many genes and many environmental risk factors, differences in population-specific genetic and environmental backgrounds could mask the effects of a biologically relevant allele. For these and other reasons, statistically strong results in the original, discovery sample that did not replicate in the replication sample may be further evaluated in additional replication cohorts and experimental systems.

Example 4

*ICAM* Region Proximal SNPs

It has been discovered that a polymorphic variation (rs1056538) in a region that encodes ICAM1, ICAM2 and ICAM5 is associated with the occurrence of breast cancer (see Examples 1 and 2). Subsequently, SNPs proximal to the incident SNP (rs1056538) were identified and allelotyped in breast cancer sample sets and control sample sets as described in Examples 1 and 2. Approximately seventy-five allelic variants located within the ICAM region were identified and allelotyped. The polymorphic variants are set forth in Table 9. The chromosome position provided in column four of Table 9 is based on Genome "Build 33" of NCBI's GenBank.

**Table 9**

| dbSNP rs# | Chromosome | Position in Figure 1 | Chromosome Position | Allele Variants |
|---|---|---|---|---|
| 2884487 | 19 | 139 | 10204039 | T/C |
| 1059840 | 19 | 11799 | 10215699 | A/T |
| 11115 | 19 | 11851 | 10215751 | T/C |
| 1059849 | 19 | 11963 | 10215863 | G/A |
| 3093035 | 19 | 24282 | 10228182 | A/G |
| ICAM_SNPA | 19 | 26849 | 10230749 | A/T |
| 281428 | 19 | 29633 | 10233533 | C/T |
| 281431 | 19 | 31254 | 10235154 | T/C |
| ICAM_SNPB | 19 | 31967 | 10235867 | G/C |
| 2358581 | 19 | 32920 | 10236820 | G/T |
| 281434 | 19 | 33929 | 10237829 | A/G |
| ICAM_SNPC | 19 | 35599 | 10239499 | G/C |
| 1799969 | 19 | 36101 | 10240001 | G/A |
| 3093033 | 19 | 36340 | 10240240 | G/A |
| ICAM_SNPD | 19 | 36405 | 10240305 | A/G |
| ICAM_SNPE | 19 | 36517 | 10240417 | T/C |
| ICAM_SNPF | 19 | 36777 | 10240677 | A/G |
| 5498 | 19 | 36992 | 10240892 | G/A |
| ICAM_SNPG | 19 | 37645 | 10241545 | T/C |
| 1057981 | 19 | 37868 | 10241768 | G/A |
| 281436 | 19 | 38440 | 10242340 | A/G |
| 923366 | 19 | 38532 | 10242432 | T/C |
| 281437 | 19 | 38547 | 10242447 | C/T |
| ICAM_SNPH | 19 | 38712 | 10242612 | T/C |
| 281438 | 19 | 40684 | 10244584 | T/G |
| 3093029 | 19 | 40860 | 10244760 | C/G |
| 2569693 | 19 | 41213 | 10245113 | C/T |
| 281439 | 19 | 41419 | 10245319 | G/C |

(continued)

| dbSNP rs# | Chromosome | Position in Figure 1 | Chromosome Position | Allele Variants |
|---|---|---|---|---|
| 281440 | 19 | 41613 | 10245513 | G/A |
| ICAM_SNPI | 19 | 42407 | 10246307 | C/G |
| 1333881 | 19 | 43440 | 10247340 | T/C |
| 1056538 | 19 | 44247 | 10248147 | T/C |
| 2228615 | 19 | 44677 | 10248577 | A/G |
| 2569702 | 19 | 45256 | 10249156 | T/C |
| 2569703 | 19 | 45536 | 10249436 | C/G |
| ICAM_SNPJ | 19 | 46153 | 10250053 | C/T |
| 2569707 | 19 | 47546 | 10251446 | C/G |
| 2916060 | 19 | 47697 | 10251597 | A/C |
| 885743 | 19 | 47944 | 10251844 | A/T |
| ICAM_SNPK | 19 | 48530 | 10252430 | C/G |
| 892188 | 19 | 51102 | 10255002 | T/C |
| 2291473 | 19 | 57090 | 10260990 | T/C |
| 281416 | 19 | 60093 | 10263993 | A/G |
| 281417 | 19 | 60439 | 10264339 | T/C |
| 281418 | 19 | 62694 | 10266594 | G/C |
| 430092 | 19 | 66260 | 10270160 | C/T |
| 368835 | 19 | 67295 | 10271195 | A/G |
| 2358583 | 19 | 67304 | 10271204 | T/G |
| ICAM_SNPL | 19 | 67731 | 10271631 | G/T |
| 1045384 | 19 | 68555 | 10272455 | C/A |
| 281427 | 19 | 70429 | 10274329 | C/T |
| 3745264 | 19 | 70875 | 10274775 | T/G |
| 281426 | 19 | 72360 | 10276260 | G/A |
| 281424 | 19 | 74228 | 10278128 | C/T |
| 281423 | 19 | 76802 | 10280702 | C/T |
| 281422 | 19 | 77664 | 10281564 | T/C |
| 281421 | 19 | 78803 | 10282703 | A/G |
| 281420 | 19 | 79263 | 10283163 | A/G |
| 3745263 | 19 | 80810 | 10284710 | A/G |
| 3745261 | 19 | 81020 | 10284920 | T/C |
| 3181049 | 19 | 82426 | 10286326 | T/C |
| 281412 | 19 | 82783 | 10286683 | T/C |
| 2230399 | 19 | 85912 | 10289812 | C/G |
| 2278442 | 19 | 86135 | 10290035 | G/A |
| 2304237 | 19 | 87877 | 10291777 | T/C |
| 281413 | 19 | 88043 | 10291943 | G/A |

(continued)

| dbSNP rs# | Chromosome | Position in Figure 1 | Chromosome Position | Allele Variants |
|---|---|---|---|---|
| 1058154 | 19 | 88206 | 10292106 | A/C |
| 3176769 | 19 | 88343 | 10292243 | T/C |
| 2304240 | 19 | 90701 | 10294601 | G/A |
| 3176768 | 19 | 90974 | 10294874 | A/G |
| 3176767 | 19 | 91060 | 10294960 | C/A |
| 3176766 | 19 | 91087 | 10294987 | C/T |
| ICAM_SNPM | 19 | 91594 | 10295494 | G/A |
| 281415 | 19 | 92302 | 10296202 | T/G |
| 3176764 | 19 | 92384 | 10296284 | A/G |

Assay for Verifying and Allelotyping SNPs

The methods used to verify and allelotype the proximal SNPs of Table 9 are the same methods described in Examples 1 and 2 herein. The PCR primers and extend primers used in these assays are provided in Table 10 and Table 11, respectively.

**Table 10**

| dbSNP rs# | Forward PCR primer | Reverse PCR primer |
|---|---|---|
| 5498 | ACGTTGGATGCTCACAGAGCACATTCACGG | ACGTTGGATGAGATCTTGAGGGCACCTACC |
| 11115 | ACGTTGGATGAGGTGACACCTTCCTCGAAG | ACGTTGGATGTGTGAAGCACCTCTTCTGAG |
| 11115 | ACGTTGGATGGTCCAGGTGACACCTTCCTC | ACGTTGGATGAAGCACCTCTTCTGAGCCAG |
| 56901 | ACGTTGGATGGTCCAGGTGACACCTTCCTC | ACGTTGGATGAAGCACCTCTTCTGAGCCAG |
| 240914 | ACGTTGGATGTTCAACAAGCGAGTGACAGC | ACGTTGGATGGTGCAGAGATGGGCTTTCTC |
| 254615 | ACGTTGGATGTGTAGATGGTCACGTTCTCC | ACGTTGGATGATCTGAGTCCTGATGTCACC |
| 254615 | ACGTTGGATGTTGCAGCTTTAAGCTAAGGC | ACGTTGGATGAGCCCAGGAGACTTAATTAC |
| 272539 | ACGTTGGATGTACAGACCCCTCTACCCCTTC | ACGTTGGATGAGGTGACACCTTCCTCGAAG |
| 281412 | ACGTTGGATGTGACCTCAGGTGATTCACCC | ACGTTGGATGGGTATACCTTTAGCTGGCTG |
| 281413 | ACGTTGGATGTCAAAGCTCACAGTTCTCGG | ACGTTGGATGACTTAGCGGGTCCTGCAAAC |
| 281414 | ACGTTGGATGAAGGCACCTTCCTCTGTCAG | ACGTTGGATGTGGGCCACAACACGGATGGTA |
| 281415 | ACGTTGGATGGCACAAAGAGCTAAGGTAGG | ACGTTGGATGGAATCCTGGATAGACAGTGG |
| 281416 | ACGTTGGATGTAACGTAGAGCACAGGTGAG | ACGTTGGATGCAACGCAAACACCAGTGTGG |
| 281417 | ACGTTGGATGAAGAGACAGTGGAGAGGCTG | ACGTTGGATGAGAGCCATCGGGTCCCAGCAA |
| 281418 | ACGTTGGATGTGCGCTCAGTCAGCTTCCTC | ACGTTGGATGAGTGTTAGCCGAGGGCAAGC |
| 281420 | ACGTTGGATGCCAGGACTGTCTCTCTGTTT | ACGTTGGATGATGACACTACAGCCTGAGCA |
| 281421 | ACGTTGGATGAGTGTTGCTTTGTCACCCAG | ACGTTGGATGAGGAGAATCGCTTGTACCTG |
| 281422 | ACGTTGGATGAGAAATCCTCCTACCTTGGC | ACGTTGGATGGCCCGGCCTCTACATAAAAT |
| 281423 | ACGTTGGATGAACCTCAAGCTGCTTCACTG | ACGTTGGATGGAGGAGCCCACCTTTAATGT |
| 281424 | ACGTTGGATGACCTGTGTTTCTAGGTGTGC | ACGTTGGATGCATGCCTGGGAAAAAACTCC |
| 281426 | ACGTTGGATGATCCTCACACCTCAGTCTCC | ACGTTGGATGAATGAGACTCCGTCTCTACC |
| 281427 | ACGTTGGATGGACAATTGTAGTACCCAGCC | ACGTTGGATGAGGAGAATCGCTTGAACCTG |
| 281428 | ACGTTGGATGAGTAGCTGGAATTACAGGCG | ACGTTGGATGGCCAACATGATGAAATCCCG |
| 281431 | ACGTTGGATGACTGGGATTACAGGTGTGAG | ACGTTGGATGGGAGAAATCTTGATGGAGGC |

(continued)

| dbSNP rs# | Forward PCR primer | Reverse PCR primer |
|---|---|---|
| 281432 | ACGTTGGATGAGCTGGGACTTTCCTTCTTG | ACGTTGGATGCAGTAAATCCAGCCTTCAGC |
| 281434 | ACGTTGGATGCCACGCCTGGCTAATTTTTG | ACGTTGGATGGGTCAGGAGTTCAAGACCAG |
| 281436 | ACGTTGGATGCATGGTTCACTGCAGTCTTG | ACGTTGGATGTGTGGTGTTGTGAGCCTATG |
| 281437 | ACGTTGGATGATAGGCTCACAACACCACAC | ACGTTGGATGAACACAAAGGAAGTCTGGGC |
| 281437 | ACGTTGGATGATAGGCTCACAACACCACAC | ACGTTGGATGAACACAAAGGAAGTCTGGGC |
| 281438 | ACGTTGGATGACCTGAGGTTTCCTCACTCAG | ACGTTGGATGAGAGGTTTCTGTGACACCCG |
| 281439 | ACGTTGGATGGCGGAGCCATACCTCTAAGC | ACGTTGGATGTCGCTGGCACTTTCGTCCC |
| 281440 | ACGTTGGATGCTGGCTGAGATGCCATGATA | ACGTTGGATGATGGTGGGAGGAGCTAAATG |
| 281440 | ACGTTGGATGGCCATGATAATAAGCTGGAC | ACGTTGGATGTCTTAGTCCCCAAATGTATC |
| 368835 | ACGTTGGATGGGTGGGAAAAAGACGTGAAG | ACGTTGGATGAGAGGGAATTAAGGAGGTCC |
| 378395 | ACGTTGGATGAATTCCGTGGGATGAGGAAT | ACGTTGGATGACCGTGTTTTCCAGGCTCGCG |
| 378395 | ACGTTGGATGACTTGGCCCCCTGCACTCACA | ACGTTGGATGACCGTGTTTTCCAGGCTCGCG |
| 430092 | ACGTTGGATGGTTGGGATTACAGGCATGAG | ACGTTGGATGATCTGTTGCCTGTCAAGATG |
| 473241 | ACGTTGGATGGCCATGATAATAAGCTGGAC | ACGTTGGATGAAATGTATCCCCGCCCTAAG |
| 547878 | ACGTTGGATGTACTCAGGAGGCTGAGGTG | ACGTTGGATGCATGGTTCACTGCAGTCTTG |
| 827786 | ACGTTGGATGGCGGAGCCATACCTCTAAGC | ACGTTGGATGTCGCTGGCACTTTCGTCCC |
| 827787 | ACGTTGGATGCTGGCTGAGATGCCATGATA | ACGTTGGATGATGGTGGGAGGAGCTAAATG |
| 885743 | ACGTTGGATGTGAGAGAAGGCGATCTTGAC | ACGTTGGATGCCAATTCACAATCCACTGTG |
| 885743 | ACGTTGGATGTGAGAGAAGGCGATCTTGAC | ACGTTGGATGCCAATTCACAATCCACTGTG |
| 892188 | ACGTTGGATGGTTTGTTTTTAGAGACAGGG | ACGTTGGATGGTCAAAGCCACTTCCAGCTA |
| 901886 | ACGTTGGATGCGATCTGGTCGCTCTGCAAG | ACGTTGGATGGCCCCACCTTCTGTTCCAAG |
| 923366 | ACGTTGGATGTCTGGGCAATGTTGCAAGAC | ACGTTGGATGATAGGCTCACAACACCACAC |
| 923366 | ACGTTGGATGTCTGGGCAATGTTGCAAGAC | ACGTTGGATGATAGGCTCACAACACCACAC |
| 1045384 | ACGTTGGATGGTGCAGAGATGGGCTTTCTC | ACGTTGGATGAGATGGGCACAATGTCCGAC |

(continued)

| dbSNP rs# | Forward PCR primer | Reverse PCR primer |
|---|---|---|
| 1056538 | ACGTTGGATGACTGCCACAGCCACAGCTAG | ACGTTGGATGTTTTCGCCCCCCAGGGTGA |
| 1057981 | ACGTTGGATGGTACAACTGTACCTGGTGAC | ACGTTGGATGAATGAACATAGGTCTCTGGC |
| 1058154 | ACGTTGGATGTCCCTTCCATCCTCATTTTT | ACGTTGGATGTGCAAGGCGCTAAACAAAAC |
| 1059840 | ACGTTGGATGTCGGCCTGGCTCAGAAGAGG | ACGTTGGATGACCCCTACCCCACGCTACCCA |
| 1059849 | ACGTTGGATGGGAATGGATGCAGAAGCCCG | ACGTTGGATGAAGCTGAGGCCACAGGGAG |
| 1059849 | ACGTTGGATGAATGGATGCAGAAGCCCGTC | ACGTTGGATGATTCCACGGAGGAAGCTGAG |
| 1333881 | ACGTTGGATGATCAGCTCTACGCGATCTGG | ACGTTGGATGTTCAGGCCCCACCTTCTGTTC |
| 1799969 | ACGTTGGATGTCAACCTCTGGTCCCCCAGTG | ACGTTGGATGAGGGGACCGTGGTCTGTTC |
| 1799969 | ACGTTGGATGTTGCCATAGGTGACTGTGGG | ACGTTGGATGTCCTAGAGGTGGACACGCAG |
| 2075741 | ACGTTGGATGAAGATGCCAGTCCGTGGACC | ACGTTGGATGCTGGAGACCCAGTGTCTCTC |
| 2228615 | ACGTTGGATGGGGCAGATGGTGACAGTAAC | ACGTTGGATGTGGAACTCCCTCCAGTGTGA |
| 2228615 | ACGTTGGATGGGGCAGATGGTGACAGTAAC | ACGTTGGATGTGGAACTCCCTCCAGTGTGA |
| 2230399 | ACGTTGGATGAGCGGCAGTTACCATGTTAG | ACGTTGGATGTTCTTCCCCCATTGCTTCTG |
| 2230399 | ACGTTGGATGAGCGGCAGTTACCATGTTAG | ACGTTGGATGTTCTTCCCCCATTGGTTCTG |
| 2278442 | ACGTTGGATGGGTGATGGACATTGAGGGTG | ACGTTGGATGTCCCTTCTGTCTCCAACCC |
| 2278442 | ACGTTGGATGTCGTGGTGATGGACATTGAG | ACGTTGGATGAAGTCAATATGCGTCCCTTC |
| 2291473 | ACGTTGGATGAAGAGGCTATGTGGCAGATG | ACGTTGGATGAGGGTGAAGCTGGGTTTAAC |
| 2304237 | ACGTTGGATGTGGGCCAGAACTTCACCCTG | ACGTTGGATGAAGCAGCACCACCGTGAGG |
| 2304240 | ACGTTGGATGAATCTCAGCAACGTGACTGG | ACGTTGGATGACACGGTGATGTTAGAGGAG |
| 2304240 | ACGTTGGATGAATCTCAGCAACGTGACTGG | ACGTTGGATGACACGGTGATGTTAGAGGAG |
| 2358581 | ACGTTGGATGTAAGGCAGGAGGATGGAGTG | ACGTTGGATGGACAGAGTCTCACTCTGTCG |
| 2358583 | ACGTTGGATGAAGACGTGAAGAGACACACC | ACGTTGGATGAGAGGGAATTAAGGAGGTCC |
| 2569693 | ACGTTGGATGCTTGTTCTCGCGTGGATGTC | ACGTTGGATGTACTCAGCGTGTGTGAGCTC |
| 2569702 | ACGTTGGATGACCCTCCAGACCTTGAACCA | ACGTTGGATGACGTAACGCTAACGGTGGAG |

(continued)

| dbSNP rs# | Forward PCR primer | Reverse PCR primer |
|---|---|---|
| 2569702 | ACGTTGGATGATACCCTACTCCTACTCTTC | ACGTTGGATGTCAAGGACGTAACGCTAAGG |
| 2569703 | ACGTTGGATGTCAGGAAGCTCCCAGACAGA | ACGTTGGATGATAACCCTTGGACGCCGATC |
| 2569703 | ACGTTGGATGTTAGACGAAAAAGGCGCCAC | ACGTTGGATGTTGTCCCTGCATAACCCTTG |
| 2569707 | ACGTTGGATGTGAGCGTGGCAGGCGCCATG | ACGTTGGATGGCGTGGCGCCCGTGCGCGT |
| 2884487 | ACGTTGGATGTGTGGCAAATGATGGAACAG | ACGTTGGATGCCAGAAGTTTGAGATCTGCC |
| 2916060 | ACGTTGGATGGGCGAGGTATCTGAGAGGG | ACGTTGGATGTACTCTGTCCCACTTCCGTC |
| 3093029 | ACGTTGGATGGGCAGCTCTGATTGGATGTT | ACGTTGGATGCTCCACAGTTGTTTGGCCTC |
| 3093030 | ACGTTGGATGAGAGACCCAGAAGGTCATAG | ACGTTGGATGCCTCCCCCAAGAAAACATTG |
| 3093032 | ACGTTGGATGGGCCACTTCTTCTGTAAGTC | ACGTTGGATGCATGAGGACATACAACTGGG |
| 3093033 | ACGTTGGATGAAAGCCTGGAATAGGCACAC | ACGTTGGATGTGCAGACAGTGACCATCTAC |
| 3093035 | ACGTTGGATGGGAGACATAGCGAGATTCTG | ACGTTGGATGTAGAAAGCAGTGCGATCTGG |
| 3176764 | ACGTTGGATGAAATCGTTTGAACCCGGGAG | ACGTTGGATGGTTTTGAGACAGAGTCTCAC |
| 3176766 | ACGTTGGATGTTTCGGGCTGCAATGGTCCC | ACGTTGGATGTAACACCTCTCTCCTTGTGC |
| 3176767 | ACGTTGGATGCGGTCTCTGATGGATTCTAC | ACGTTGGATGAACAGGCCCCACCATTTAAC |
| 3176768 | ACGTTGGATGGAGAGGTGTTAAATGGTGGG | ACGTTGGATGGGAACATGAAGAAGTCCTGG |
| 3176769 | ACGTTGGATGTTCCTGTTTATGGCCAGACG | ACGTTGGATGGTCTGAACCTGATTGGAGAG |
| 3181049 | ACGTTGGATGATCTTCAGGGATGGTCACTC | ACGTTGGATGGACAAATACAAAGGGACAGG |
| 3745261 | ACGTTGGATGACACACAGCAGGGCATCCGT | ACGTTGGATGCGCAATCAATGCTTTCCACC |
| 3745263 | ACGTTGGATGTACATGAAGAAGGACTCGGC | ACGTTGGATGATCCGTCCAGTGCACGTAGA |
| 3745264 | ACGTTGGATGCAAAGTGCTAGGATCACAGG | ACGTTGGATGACTGCCCCATAGAGTGGCAA |
| FCH-0994 | ACGTTGGATGTTTTCGCCCCCCAGGGTGAC | ACGTTGGATGACAGCCACAGCTAGCGCAGA |

**Table 11**

| dbSNP rs# | Extend Primer | Term Mix |
|---|---|---|
| 5498 | CAGAGCACATTCACGGTCACCT | CGT |
| 11115 | AAGGGTGGGCGTGGGCCT | ACT |
| 11115 | AAGGGTGGGCGTGGGCCT | ACT |
| 56901 | AAGGGTGGGCGTGGGCCT | ACT |
| 240914 | ACAATGTCCGACTCCCACA | ACT |
| 254615 | CCAGGGTGACGTTGCAGA | ACG |
| 254615 | TAAGGCAAAGTTCAGCTACTTA | CGT |
| 272539 | ACCCCGTACCACTGTTGA | CGT |
| 281412 | GCTGGGATTATAAGCGTG | ACT |
| 281413 | GCTCACAGTTCTCGGCAGGAC | ACG |
| 281414 | CCTTCCTCTGTCAGAATGGC | ACG |
| 281415 | GGTGATTTGGGGACAGCTGA | ACT |
| 281416 | GGTCCACACCGACGCCAG | ACT |
| 281417 | CCCCTGCCCAGGACACCCC | ACT |
| 281418 | TCAGCTTCCTCCCTCCCC | ACT |
| 281420 | ACTGTCTCTCTGTTTTTGAGAT | ACT |
| 281421 | GCTTTGTCACCCAGGCTGGA | ACT |
| 281422 | CTGGGGAACTACAGGAATGC | ACT |
| 281423 | GCCCACCCTCCATTCAGC | ACG |
| 281424 | TAGGTGTGCGTGTGTGTGTG | ACG |
| 281426 | GAGCTGGGACCACAGGCA | ACG |
| 281427 | CTTTGTATACAATCTTCCCTC | ACG |
| 281428 | GCGCCCAGCACCACGCC | ACG |
| 281431 | ACAGGTGTGAGCCACTGC | ACT |
| 281432 | GGGAGTCATGGAGGGTTT | ACT |
| 281434 | TAGAGACGGGGTTTCACTAT | ACT |
| 281436 | ACTGCAGTCTTGACCTTTTG | ACT |
| 281437 | TTTTTTTTCCAGAGACGGGGTCT | ACG |
| 281437 | TTTTTCCAGAGACGGGGTCT | ACG |
| 281438 | CGAAGCCCCAGACTCTGTGTA | ACT |
| 281439 | ACCCCTCCGGGTCAGCTCC | ACT |
| 281440 | TAATAAGCTGGACTCCGAGC | ACG |
| 281440 | TAATAAGCTGGACTCCGAGC | ACG |
| 368835 | AGACGTGAAGAGACACACCT | ACT |
| 378395 | GCCCGCGTCCTCCTCTCC | ACT |
| 378395 | GCCCGCGTCCTCCTCTCC | ACT |
| 430092 | ATTACAGGCATGAGCCACTG | ACG |

(continued)

| dbSNP rs# | Extend Primer | Term Mix |
|---|---|---|
| 473241 | ATAATAAGCTGGACTCCGAGC | ACG |
| 547878 | GTGGGAGGATCACTTGAGC | ACG |
| 827786 | ACCCCTCCGGGTCAGCTCC | ACT |
| 827787 | TAATAAGCTGGACTCCGAGC | ACG |
| 885743 | GACCCCTCTCTCCCTCCA | CGT |
| 885743 | GACCCCTCTCTCCCTCCA | CGT |
| 892188 | TGGGCTGGAGCACAATGAC | ACT |
| 901886 | GAGTCCGCAGCTCTTTGAAC | ACT |
| 923366 | TTGCAAGACCCCGTCTCTG | ACT |
| 923366 | TTGCAAGACCCCGTCTCTG | ACT |
| 1045384 | CCAGTCCCCTGCTGTCTGT | CGT |
| 1056538 | GAGGGTGCCAGGCAGCTG | ACT |
| 1057981 | TACCTGGTGACCTTGAATGTGAT | ACG |
| 1058154 | CTTCCATCCTCATTTTTTTTTATT | ACT |
| 1059840 | GCTCAGAAGAGGTGCTTCAC | CGT |
| 1059849 | CAGAAGCCCGTCTGGGCT | ACG |
| 1059849 | CAGAAGCCCGTCTGGGCT | ACG |
| 1333881 | AGAGTCCGCAGCTCTTTGAAC | ACT |
| 1799969 | CCGAGACTGGGAACAGCC | ACG |
| 1799969 | CCGAGACTGGGAACAGCC | ACG |
| 2075741 | GGACCATGGTGCACAGCA | ACT |
| 2228615 | AGTAACCTGCGCAGCTGGG | ACT |
| 2228615 | GTAACCTGCGCAGCTGGG | ACT |
| 2230399 | GTTACCATGTTAGGGAGGAGA | ACT |
| 2230399 | ACCATGTTAGGGAGGAGA | ACT |
| 2278442 | GGACATTGAGGGTGAGCTAA | ACG |
| 2278442 | ACATTGAGGGTGAGCTAA | ACG |
| 2291473 | GGAGTGTCCCTGGACCCC | ACT |
| 2304237 | TGCGCTGCCAAGTGGAGG | ACT |
| 2304240 | GCTCAGTGTACTGCAATGGCTC | ACG |
| 2304240 | AGTGTACTGCAATGGCTC | ACG |
| 2358581 | CTTGCAGTGAGCCCAGATCG | CGT |
| 2358583 | AAGAGACACACCTAATTTGTGG | ACT |
| 2569693 | CGCGTGGATGTCAGGGCC | ACG |
| 2569702 | CAGACCTTGAACCAGATAGAA | ACT |
| 2569702 | ACCTTGAACCAGATAGAA | ACT |
| 2569703 | CTCCCAGACAGAGTGCATG | ACT |

(continued)

| dbSNP rs# | Extend Primer | Term Mix |
|---|---|---|
| 2569703 | TCCCAGACAGAGTGCATG | ACT |
| 2569707 | GGCGAGTACGAGTGCGCA | ACT |
| 2884487 | AGAGACAGGGTCTCGCC | ACT |
| 2916060 | CTCCCTCTCGGTCCCGG | ACT |
| 3093029 | AGTTTCCTATCCCAGCC | ACT |
| 3093030 | CCAGAACCTCAGGGTATG | |
| 3093032 | CTTCTGTAAGTCTGTGGG | |
| 3093033 | GGGTTCAGGTCACACCC | ACG |
| 3093035 | TTCTGTCTCAAAAAACAAAGC | ACT |
| 3176764 | CCCGCCACTGCACTCCA | ACT |
| 3176766 | TCCTTCTGAGTTCTCCC | ACG |
| 3176767 | TGGATTCTACCTTTCCC | CGT |
| 3176768 | TGTTGATGCGTGGGTTGGGG | ACT |
| 3176769 | CGGGGTGGGTGGATCAA | ACT |
| 3181049 | ACTCCCTGCCCTGGCCC | ACT |
| 3745261 | GCAGCTGCACCGACAGTTC | ACT |
| 3745263 | TCGGCTGCCCGTGCCAAGTC | ACT |
| 3745264 | ATACCATGCCAGGCATT | ACT |
| FCH-0994 | CCCAGGGTGACGTTGCAGA | ACG |

Genetic Analysis of Allelotyping Results

Allelotyping results are shown for cases and controls in Table 12. The allele frequency for the A2 allele is noted in the fifth and sixth columns for breast cancer pools and control pools, respectively, where "AF" is allele frequency. The allele frequency for the A1 allele can be easily calculated by subtracting the A2 allele frequency from 1 (A1 AF = 1-A2 AF). For example, the SNP rs2884487 has the following case and control allele frequencies: case A1 (T) = 0.788; case A2 (C) = 0.212; control A1 (T) = 0.758; and control A2 (C) = 0.242, where the nucleotide is provided in paranthesis. SNPs with blank allele frequencies were untyped.

**Table 12**

| dbSNP rs# | Position in Figure 1 | Chromosome Position | A1/A2 Allele | A2 Case AF | A2 Control AF | p-Value |
|---|---|---|---|---|---|---|
| 2884487 | 139 | 10204039 | T/C | 0.212 | 0.242 | 0.2425 |
| 1059840 | 11799 | 10215699 | A/T | 0.809 | 0.805 | 0.8545 |
| 11115 | 11851 | 10215751 | T/C | 0.434 | 0.379 | 0.0644 |
| 1059849 | 11963 | 10215863 | G/A | 0.243 | 0.194 | 0.0468 |
| 3093035 | 24282 | 10228182 | A/G | 0.889 | 0.914 | 0.1592 |
| ICAM SNPA | 26849 | 10230749 | A/T | Not Allelotyped | | |
| 281428 | 29633 | 10233533 | C/T | 0.180 | 0.174 | 0.7908 |
| 281431 | 31254 | 10235154 | T/C | 0.107 | 0.109 | 0.8964 |
| ICAM SNPB | 31967 | 10235867 | G/C | 0.375 | 0.382 | 0.8113 |

(continued)

| dbSNP rs# | Position in Figure 1 | Chromosome Position | A1/A2 Allele | A2 Case AF | A2 Control AF | p-Value |
|---|---|---|---|---|---|---|
| 2358581 | 32920 | 10236820 | G/T | 0.097 | 0.074 | 0.1800 |
| 281434 | 33929 | 10237829 | A/G | 0.818 | 0.831 | 0.5765 |
| ICAM SNPC | 35599 | 10239499 | G/C | Not Allelotyped | | |
| 1799969 | 36101 | 10240001 | G/A | 0.117 | 0.151 | 0.1036 |
| 3093033 | 36340 | 10240240 | G/A | 0.004 | 0.023 | 0.0051 |
| ICAM SNPD | 36405 | 10240305 | A/G | Not Allelotyped | | |
| ICAM SNPE | 36517 | 10240417 | T/C | Not Allelotyped | | |
| ICAM SNPF | 36777 | 10240677 | A/G | Not Allelotyped | | |
| 5498 | 36992 | 10240892 | G/A | 0.554 | 0.487 | 0.0257 |
| ICAM SNPG | 37645 | 10241545 | T/C | 0.684 | 0.732 | 0.0788 |
| 1057981 | 37868 | 10241768 | G/A | 0.978 | 0.994 | 0.0289 |
| 281436 | 38440 | 10242340 | A/G | 0.504 | 0.554 | 0.0977 |
| 923366 | 38532 | 10242432 | T/C | 0.597 | 0.553 | 0.1471 |
| 281437 | 38547 | 10242447 | C/T | 0.195 | 0.151 | 0.0521 |
| ICAM SNPH | 38712 | 10242612 | T/C | 0.448 | 0.398 | 0.0970 |
| 281438 | 40684 | 10244584 | T/G | 0.235 | 0.200 | 0.1589 |
| 3093029 | 40860 | 10244760 | C/G | 0.089 | 0.081 | 0.6267 |
| 2569693 | 41213 | 10245113 | C/T | 0.297 | 0.355 | 0.0389 |
| 281439 | 41419 | 10245319 | G/C | 0.526 | 0.589 | 0.0352 |
| 281440 | 41613 | 10245513 | G/A | 0.736 | 0.746 | 0.7085 |
| ICAM SNPI | 42407 | 10246307 | C/G | 0.325 | 0.394 | 0.0173 |
| 1333881 | 43440 | 10247340 | T/C | 0.336 | 0.360 | 0.3961 |
| 1056538 | 44247 | 10248147 | T/C | 0.592 | 0.489 | 0.0009 |
| 2228615 | 44677 | 10248577 | A/G | 0.595 | 0.519 | 0.0112 |
| 2569702 | 45256 | 10249156 | T/C | 0.294 | 0.357 | 0.0254 |
| 2569703 | 45536 | 10249436 | C/G | 0.438 | 0.476 | 0.2109 |
| ICAM SNPJ | 46153 | 10250053 | C/T | Not Allelotyped | | |
| 2569707 | 47546 | 10251446 | C/G | 0.829 | 0.840 | 0.6238 |
| 2916060 | 47697 | 10251597 | A/C | 0.010 | 0.002 | 0.0702 |
| 885743 | 47944 | 10251844 | A/T | Not Allelotyped | | |
| ICAM SNPK | 48530 | 10252430 | C/G | Not Allelotyped | | |
| 892188 | 51102 | 10255002 | T/C | 0.512 | 0.434 | 0.0104 |
| 2291473 | 57090 | 10260990 | T/C | 0.087 | 0.090 | 0.8770 |
| 281416 | 60093 | 10263993 | A/G | 0.546 | 0.505 | 0.1669 |
| 281417 | 60439 | 10264339 | T/C | 0.471 | 0.476 | 0.8531 |
| 281418 | 62694 | 10266594 | G/C | 0.914 | 0.934 | 0.1968 |
| 430092 | 66260 | 10270160 | C/T | 0.229 | 0.257 | 0.2758 |

(continued)

| dbSNP rs# | Position in Figure 1 | Chromosome Position | A1/A2 Allele | A2 Case AF | A2 Control AF | p-Value |
|---|---|---|---|---|---|---|
| 368835 | 67295 | 10271195 | A/G | 0.703 | 0.727 | 0.3808 |
| 2358583 | 67304 | 10271204 | T/G | 0.304 | 0.326 | 0.4322 |
| ICAM SNPL | 67731 | 10271631 | G/T | 0.705 | 0.669 | 0.2029 |
| 1045384 | 68555 | 10272455 | C/A | 0.180 | 0.187 | 0.7736 |
| 281427 | 70429 | 10274329 | C/T | 0.217 | 0.176 | 0.0916 |
| 3745264 | 70875 | 10274775 | T/G | 0.853 | 0.836 | 0.4285 |
| 281426 | 72360 | 10276260 | G/A | 0.565 | 0.685 | 0.0001 |
| 281424 | 74228 | 10278128 | C/T | 0.246 | 0.250 | 0.8929 |
| 281423 | 76802 | 10280702 | C/T | 0.192 | 0.197 | 0.8585 |
| 281422 | 77664 | 10281564 | T/C | 0.632 | 0.632 | 0.9791 |
| 281421 | 78803 | 10282703 | A/G | 0.920 | 0.925 | 0.7863 |
| 281420 | 79263 | 10283163 | A/G | 0.392 | 0.432 | 0.1774 |
| 3745263 | 80810 | 10284710 | A/G | 0.936 | 0.923 | 0.4005 |
| 3745261 | 81020 | 10284920 | T/C | 0.006 | 0.008 | 0.5979 |
| 3181049 | 82426 | 10286326 | T/C | 0.650 | 0.640 | 0.7183 |
| 281412 | 82783 | 10286683 | T/C | 0.408 | 0.352 | 0.0527 |
| 2230399 | 85912 | 10289812 | C/G | 0.826 | 0.838 | 0.5900 |
| 2278442 | 86135 | 10290035 | G/A | 0.581 | 0.594 | 0.6511 |
| 2304237 | 87877 | 10291777 | T/C | 0.102 | 0.093 | 0.6063 |
| 281413 | 88043 | 10291943 | G/A | Not Allelotyped | | |
| 1058154 | 88206 | 10292106 | A/C | 0.780 | 0.810 | 0.2203 |
| 3176769 | 88343 | 10292243 | T/C | 0.199 | 0.214 | 0.5539 |
| 2304240 | 90701 | 10294601 | G/A | 0.170 | 0.203 | 0.1661 |
| 3176768 | 90974 | 10294874 | A/G | 0.642 | 0.650 | 0.7681 |
| 3176767 | 91060 | 10294960 | C/A | 0.727 | 0.725 | 0.9511 |
| 3176766 | 91087 | 10294987 | C/T | 0.230 | 0.231 | 0.9513 |
| ICAM SNPM | 91594 | 10295494 | G/A | 0.289 | 0.267 | 0.4128 |
| 281415 | 92302 | 10296202 | T/G | 0.754 | 0.766 | 0.6399 |
| 3176764 | 92384 | -10296284 | A/G | 0.899 | 0.894 | 0.8086 |
| 281412 | NOT MAPPED | | | 0.154 | 0.156 | 0.9342 |
| 281413 | NOT MAPPED | | | 0.299 | 0.302 | 0.9195 |
| 281415 | NOT MAPPED | | | 0.664 | 0.684 | 0.4825 |

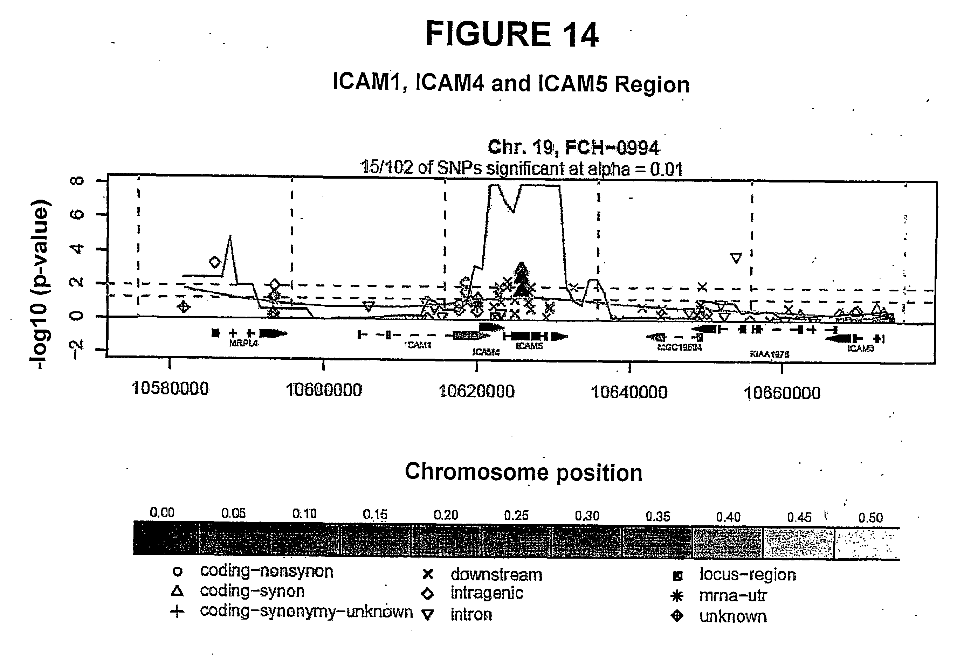

Figure 14 shows the proximal SNPs in and around the *ICAM* region for females. The position of each SNP on the chromosome is presented on the x-axis. The y-axis gives the negative logarithm (base 10) of the p-value comparing the estimated allele in the case group to that of the control group. The minor allele frequency of the control group for each SNP designated by an X or other symbol on the graphs in Figure 14 can be determined by consulting Table 12. By proceeding down the Table from top to bottom and across the graphs from left to right the allele frequency associated with each symbol shown can be determined.

To aid the interpretation, multiple lines have been added to the graph. The broken horizontal lines are drawn at two common significance levels, 0.05 and 0.01. The vertical broken lines are drawn every 20kb to assist in the interpretation of distances between SNPs. Two other lines are drawn to expose linear trends in the association of SNPs to the disease. The light gray line (or generally bottommost curve) is a nonlinear smoother through the data points on the graph using a local polynomial regession method (W.S. Cleveland, E. Grosse and W.M. Shyu (1992) Local regression models. Chapter 8 of Statistical Models in S eds J.M. Chambers and T.J. Hastie, Wadsworth & Brooks/Cole.). The black line (or generally top-most curve, e.g., see peak in left-most graph just to the left of position 92150000) provides a local test for excess statistical significance to identify regions of association. This was created by use of a 10kb sliding window with 1kb step sizes. Within each window, a chi-square goodness of fit test was applied to compare the proportion of SNPs that were significant at a test wise level of 0.01, to the proportion that would be expected by chance alone (0.05 for the methods used here). Resulting p-values that were less than $10^{-8}$ were truncated at that value.

Finally, the gene or genes present in the loci region of the proximal SNPs as annotated by Locus Link (http address: www.ncbi.nlm.nih.gov/LocusLink/) are provided on the graph. The exons and introns of the genes in the covered region are plotted below each graph at the appropriate chromosomal positions. The gene boundary is indicated by the broken horizontal line. The exon positions are shown as thick, unbroken bars. An arrow is place at the 3' end of each gene to show the direction of transcription.

Additional Genotyping

In addition to the ICAM region incident SNP, two other SNPs were genotyped in the discovery cohort. The discovery cohort is described in Example 1. The SNPs (rs1801714 and rs2228615) are located in the ICAM5 encoding portion of the sequence, were associated with breast cancer with a p-value of 0.0734 and 0.00236, respectively, and encoded non-synonymous amino acids (see Table 15).

The methods used to verify and genotype the two proximal SNPs of Table 15 are the same methods described in Examples 1 and 2 herein. The PCR primers and extend primers used in these assays are provided in Table 13 and Table 14, respectively.

**Table 13**

| dbSNP rs# | Second PCR primer | First PCR primer |
|---|---|---|
| 1801714 | ACGTTGGATGAGGGTTGCAGAGCAGGAGAA | ACGTTGGATGAGCCAAGGTGACGCTGAATG |
| 2228615 | ACGTTGGATGAGATGGTGACAGTAACCTGC | ACGTTGGATGTGGCATTTAGCTGAAGCTGG |

**Table 14**

| dbSNP rs# | Extend Primer | Term Mix |
|---|---|---|
| 1801714 | CCTTCAGCAGGAGCTGGGCCCTC | ACT |
| 2228615 | TAACCTGCGCAGCTGGG | ACT |

Table 15, below, shows the case and control allele frequencies along with the p-values for the SNPs genotyped. The disease associated allele of column 4 is in bold and the disease associated amino acid of column 5 is also in bold. The chromosome positions provided correspond to NCBI's Build 33.

**Table 15: Genotpying Results**

| dbSNP rs# | Position in Figure 1 | Chromo some Position | Alleles (A1/A2) | Amino Acid Change | AF F case | AF F control | p-value | Odds Ratio |
|---|---|---|---|---|---|---|---|---|
| 1801714 | 36517 | 10240417 | T/C | L352P | T = 0.010<br>C = 0.990 | T = 0.030<br>C = 0.097 | **0.0734** | 2.260 |
| 2228615 | 44677 | 10248577 | A/G | T348A | A = 0.340<br>G = 0.660 | A = 0.430<br>G = 0.570 | 0**.00236** | 1.470 |

Example 5

*MAPK10* Proximal SNPs

It has been discovered that a polymorphic variation (rs1541998) in a region that encodes MAPK10 is associated with the occurrence of breast cancer (see Examples 1 and 2). Subsequently, SNPs proximal to the incident SNP (rs1541998) were identified and allelotyped in breast cancer sample sets and control sample sets as described in Examples 1 and 2. Approximately sixty-three allelic variants located within the MAPK10 region were identified and allelotyped. The polymorphic variants are set forth in Table 16. The chromosome position provided in column four of Table 16 is based on Genome "Build 33" of NCBI's GenBank.

**Table 16**

| dbSNP rs# | Chromosome | Position in Figure 2 | Chromosome Position | Allele Variants |
|---|---|---|---|---|
| 2575681 | 4 | 191 | 87306691 | C/T |
| 2575680 | 4 | 1490 | 87307990 | A/G |
| 2589505 | 4 | 3781 | 87310281 | C/T |
| 2589504 | 4 | 3935 | 87310435 | G/A |
| 2164538 | 4 | 4512 | 87311012 | T/C |
| 2575679 | 4 | 7573 | 87314073 | A/G |
| MAP_SNP1 | 4 | 8467 | 87314967 | A/T |
| 2869408 | 4 | 9001 | 87315501 | C/G |
| 934648 | 4 | 9732 | 87316232 | T/C |
| 2164537 | 4 | 13477 | 87319977 | T/C |
| 2575678 | 4 | 13787 | 87320287 | A/C |
| 2575677 | 4 | 13903 | 87320403 | G/C |
| 2589509 | 4 | 14355 | 87320855 | T/G |
| 2164536 | 4 | 15053 | 87321553 | A/C |
| 2164535 | 4 | 15459 | 87321959 | T/A |
| MAP_SNP2 | 4 | 17762 | 87324262 | G/A |
| 2589523 | 4 | 19482 | 87325982 | C/T |
| 3755970 | 4 | 19631 | 87326131 | A/C |
| 2575675 | 4 | 22170 | 87328670 | G/A |
| 1202 | 4 | 22688 | 87329188 | T/C |
| 1201 | 4 | 22748 | 87329248 | A/G |
| 2589516 | 4 | 23376 | 87329876 | G/T |
| 2575674 | 4 | 23826 | 87330326 | A/T |
| 2589515 | 4 | 23868 | 87330368 | G/C |
| MAP_SNP3 | 4 | 24154 | 87330654 | C/T |
| 2589506 | 4 | 25972 | 87332472 | G/A |
| 1436524 | 4 | 26057 | 87332557 | A/G |
| 2575672 | 4 | 26361 | 87332861 | C/T |
| 2589518 | 4 | 26599 | 87333099 | G/A |
| 3775164 | 4 | 26712 | 87333212 | T/G |

(continued)

| dbSNP rs# | Chromosome | Position in Figure 2 | Chromosome Position | Allele Variants |
|---|---|---|---|---|
| 2589514 | 4 | 26812 | 87333312 | G/A |
| 3775166 | 4 | 27069 | 87333569 | T/C |
| 3775167 | 4 | 32421 | 87338921 | C/T |
| 3775169 | 4 | 33557 | 87340057 | T/C |
| 2043650 | 4 | 35127 | 87341627 | A/G |
| 2043649 | 4 | 35222 | 87341722 | T/G |
| 3775170 | 4 | 35999 | 87342499 | T/A |
| 1541998 | 4 | 36424 | 87342924 | C/T |
| 2043648 | 4 | 37403 | 87343903 | A/G |
| 2282598 | 4 | 39203 | 87345703 | C/T |
| 2282597 | 4 | 39226 | 87345726 | G/A |
| 3775173 | 4 | 41147 | 87347647 | T/C |
| 1469870 | 4 | 46176 | 87352676 | G/C |
| 1436522 | 4 | 50452 | 87356952 | T/C |
| 1946733 | 4 | 52919 | 87359419 | G/A |
| 1436525 | 4 | 60214 | 87366714 | G/A |
| 3822037 | 4 | 61093 | 87367593 | C/G |
| 3775176 | 4 | 62572 | 87369072 | G/A |
| 1436527 | 4 | 63601 | 87370101 | C/T |
| 1436529 | 4 | 65362 | 87371862 | T/C |
| 3775182 | 4 | 65863 | 87372363 | T/G |
| 3775183 | 4 | 66207 | 87372707 | G/A |
| 3775184 | 4 | 66339 | 87372839 | A/G |
| 3775187 | 4 | 69512 | 87376012 | T/C |
| 1010778 | 4 | 70759 | 87377259 | A/G |
| 2282596 | 4 | 71217 | 87377717 | T/A |
| 2118044 | 4 | 73382 | 87379882 | A/T |
| 1469869 | 4 | 76307 | 87382807 | C/T |
| 1046706 | 4 | Not mapped | | G/T |
| 2060588 | 4 | Not mapped | | G/A |
| 2289490 | 4 | Not mapped | | C/T |
| 2289491 | 4 | Not mapped | | C/T |
| 729511 | 4 | Not mapped | | T/C |

Assay for Verifying and Allelotyping SNPs

The methods used to verify and allelotype the proximal SNPs of Table 16 are the same methods described in Examples 1 and 2 herein. The PCR primers and extend primers used in these assays are provided in Table 17 and Table 18, respectively.

Table 17

| dbSNP rs# | Forward PCR primer | Reverse PCR primer |
|---|---|---|
| 958 | ACGTTGGATGATCCGCATGTGTCTGTATTC | ACGTTGGATGCCCAGTGCATTATGTCTTGG |
| 1201 | ACGTTGGATGTGCCAGTGCTCTGAAAACTG | ACGTTGGATGCCTGTGGTCTCTATTGCTTG |
| 1201 | ACGTTGGATGACAAGAATGCCAGTGCTCTG | ACGTTGGATGCCTGTGGTCTCTATTGCTTG |
| 1202 | ACGTTGGATGTAATCTCAGAATGGCAGCAC | ACGTTGGATGTCAAGCAATAGAGACCACAG |
| 10305 | ACGTTGGATGTTCAAGAATTATTTTATTGCAA GTC | ACGTTGGATGGGTGAAGCTTGAAAGCAAGC |
| 729511 | ACGTTGGATGTTAATGTAGTAAAAAGCACG | ACGTTTGGATGCTAGAGATCGGTTTTACACC |
| 934648 | ACGTTGGATGACTGGTTGATACCATAGGAC | ACGTTGGATGTGTACTGCTTTCATCCTTGC |
| 934648 | ACGTTGGATGACTGGTTGATACCATAGGAC | ACGTTGGATGTGTACTGCTTTCATCCTTGC |
| 1010778 | ACGTTGGATGCAGAGGAAAGAAAACTGAAAG | ACGTTGGATGGGATTTGTTCTTAATCTTTC |
| 1046706 | ACGTTGGATGCAAATGGGAGTCAAGTCCTC | ACGTTGGATGTTTTGCTCCTAAGCTGAAGG |
| 1436522 | ACGTTGGATGGGAATTGAAATTGGCATTGC | ACGTTGGATGATTGGAAGGAGGAAGCATAG |
| 1436524 | ACGTTGGATGGAGTTGCCAGTAGCTTTGAG | ACGTTGGATGATTGTTTCCAGGGTGCTCTG |
| 1436525 | ACGTTGGATGGTGCAATCTTGGTTCACTGC | ACGTTGGATGGCTTACACTAGCTACTTGGG |
| 1436527 | ACGTTGGATGAGCACTGTGAGTTAAACCTG | ACGTTGGATGCTGTATAGAGAGCTGTTTGC |
| 1436529 | ACGTTGGATGCTATGGCAGCAGAAGAGTAG | ACGTTGGATGAATGTTGGACCACATGTACG |
| 1469869 | ACGTTGGATGCATGGCGAGGAAATCTGTTT | ACGTTGGATGTTCGATATATCAGAGCCTTG |
| 1469870 | ACGTTGGATGATACTGAGCTCCATTTTGGG | ACGTTGGATGATGGCACAGTTTAGCATGTC |
| 1541998 | ACGTTGGATGGCCCATGTTAACATTTTCTTC | ACGTTGGATGCTGATTATTCTGATGGTAATG |
| 1946733 | ACGTTGGATGGCAGGAGGATAGATCTGTAG | ACGTTGGATGTAGCTTCTAAACATCTCTTG |
| 2043648 | ACGTTGGATGTGGCTTTCTGAATGCTAGAG | ACGTTGGATGAGGGCGGAATGATTTTTAGC |
| 2043649 | ACGTTGGATGGCACTACATGGGACACAAAG | ACGTTGGATGGTCCTACTAGTCCCTGTATG |
| 2043650 | ACGTTGGATGGCTGAGGGAGAAATTGAGTG | ACGTTGGATGCTGTGCCTTGCACATAGTAG |
| 2060588 | ACGTTGGATGTTTCATTGCTCATGGATTAG | ACGTTGGATGGATAAGTATTGGCTTAATCTG |
| 2118044 | ACGTTGGATGAACAACTTGGCTAATTCTAC | ACGTTGGATGGTCATTGCCTCTAGCTAGTG |
| 2164535 | ACGTTGGATGACCAGCACTATTACCCATGC | ACGTTGGATGGAATGATGTAAACGTTGGAG |
| 2164536 | ACGTTGGATGGTGATGAAAACCATGTGAGC | ACGTTGGATGCTGGAGAACAAAAGACCACC |
| 2164537 | ACGTTGGATGCAAGGCAAAATGTTTCCAGC | ACGTTGGATGAACACACTTAGTACCCACGC |
| 2164538 | ACGTTGGATGTACTGCAGAGCTCTCCCTTG | ACGTTGGATGAGAGGTCATCTTAATGGGCC |
| 2282596 | ACGTTGGATGTCATACTGATCAACCTGAAG | ACGTTGGATGGGTGGCTTTGTGAAACCTTG |
| 2282597 | ACGTTGGATGGCATGGTTCTGTTATAAGGC | ACGTTGGATGACACTTGATTACAATGGCCC |
| 2282598 | ACGTTGGATGCACGCCTAAGCAATTAATGAC | ACGTTGGATGGTGAATGAAGGAAAAGTAGC |
| 2289490 | ACGTTGGATGTGATTACTGGATTGGCTGGG | ACGTTGGATGAAATGCCCTGAAGACCCAGC |
| 2289491 | ACGTTGGATGGGAATGCATTGTAAACCAGG | ACGTTGGATGACCTAGCCTTGCAGGAGGAC |
| 2575672 | ACGTTGGATGATAGTGTTATCACATAGACC | ACGTTGGATGCTCCAGGAGCAAGGATTATG |
| 2575674 | ACGTTGGATGGTGGGTAACAGTTTTCAGGC | ACGTTGGATGCTCTCCTACTCTTTACTGTC |
| 2575675 | ACGTTGGATGTCGTACCTGCATAAGTGGTG | ACGTTGGATGTTGGGAAGGTACTAACAGCG |

(continued)

| dbSNP rs# | Forward PCR primer | Reverse PCR primer |
|---|---|---|
| 2575677 | ACGTTGGATGGATGCCAATTTGGTTTGCCC | ACGTTGGATGGAAGGATAAGCCACAGTGAG |
| 2575678 | ACGTTGGATGCTTCAAGAGGCCATACAGAC | ACGTTGGATGAAGCACCATTTGTGGCTCAG |
| 2575679 | ACGTTGGATGCTTTCCTGCTGCATTTAGTG | ACGTTGGATGTAAGCCAGTAACACATGCCG |
| 2575680 | ACGTTGGATGGCCCTGAAGTTTTTGAATGG | ACGTTGGATGGAGCCCAATACAATCAGGTG |
| 2575681 | ACGTTGGATGTTCACTGCTAACATGCATGG | ACGTTGGATGTTATATAGCCTTCTTTTCTC |
| 2589504 | ACGTTGGATGGGATAGGAAACATATTAAGG | ACGTTGGATGCTGTGTGATTTGGACAACCC |
| 2589505 | ACGTTGGATGAGACTGTAGCCTAAATGAGG | ACGTTGGATGCATTTTATGAGAAGATGCAC |
| 2589506 | ACGTTGGATGGCAACTCAGCTAGCCTTTAC | ACGTTGGATGTGTTATGCGGGAGTATAAGG |
| 2589509 | ACGTTGGATGTGAATCATGGTTGCCTCCTG | ACGTTGGATGATACGCAGGTTGTAGAGAGG |
| 2589514 | ACGTTGGATGTATACATTGTCCTGATAGAG | ACGTTGGATGCTTAAATGTCTCTAGAAAAGG |
| 2589515 | ACGTTGGATGCACCTGTATACCAATTTGTAG | ACGTTGGATGGCCAAACCATTTTGTGCCTG |
| 2589516 | ACGTTGGATGCATACTCTGCCAAAGTTTTA | ACGTTGGATGACTCACACTGTGGTTTGGGG |
| 2589518 | ACGTTGGATGCCAGGCAAAAAGAATGACCG | ACGTTGGATGAATGATATGCACCGATCTTC |
| 2589523 | ACGTTGGATGTCATGTAGCTAAACAAAGGC | ACGTTGGATGAGCAGGGTTAAATTTCCCAG |
| 2589525 | ACGTTGGATGAAGAACATTGAAAGAAGCAG | ACGTTGGATGGTATTTAAATTAGTGGTGTG |
| 2869408 | ACGTTGGATGTCCCAGTACCTAAGTAGCAG | ACGTTGGATGGCTTTGAATTACTCTGTCCC |
| 3755970 | ACGTTGGATGTACAACTAGTATCTACAGAC | ACGTTGGATGGTGACCATGTAGAAATCTGTG |
| 3775164 | ACGTTGGATGGAACATGAAAAATTCATAAGC | ACGTTGGATGAAGTTTCCCTGGTCGTGATC |
| 3775166 | ACGTTGGATGCTGTTTTTCACCCCCGATTC | ACGTTGGATGCTGAGGAGTCCATCATAGTG |
| 3775167 | ACGTTGGATGGAAACAAGCAGATGTCATGG | ACGTTGGATGGCTTCTGATTTTATATGGCAC |
| 3775169 | ACGTTGGATGGGGAGAGAATGGTTGCATAT | ACGTTGGATGATGCTGAACAACAGGATGGG |
| 3775170 | ACGTTGGATGCCTAAGACCTATGCTCTCAC | ACGTTGGATGCCCATTTTTGCTAGCAGGAG |
| 3775173 | ACGTTGGATGCAAGAGGGCTGCTTTAAACC | ACGTTGGATGTAAATTTGCAGAGGCCGTCG |
| 3775176 | ACGTTGGATGAAAAGGTCACCAGTGACCTG | ACGTTGGATGTAGTCCAAGTATTTCCCAAG |
| 3775182 | ACGTTGGATGGATATCTCCCTCCTATTGGC | ACGTTGGATGGCTGGACTCTATTAGGCCAT |
| 3775183 | ACGTTGGATGGATCTCTGATCTTAGACCAC | ACGTTGGATGTGCAGATATGTAGGCCAAGC |
| 3775184 | ACGTTGGATGGACCAGCAACCATGATGAAG | ACGTTGGATGGTTCTACTTTGACCACAGGC |
| 3775187 | ACGTTGGATGTAGCACCTTCAGGATCTTTC | ACGTTGGATGAATCATGATCCCAGGGCAAG |
| 3822037 | ACGTTGGATGGTAATCCATAAACTGTGGGAG | ACGTTGGATGTCCCACCCTGACTTCTTTGC |

**Table 18**

| dbSNP rs# | Extend Primer | Term Mix |
|---|---|---|
| 958 | TTATGTCTTGGTAGAGCC | ACG |
| 1201 | TCTATTGCTTGAAGAGAGAAAG | ACT |
| 1201 | TTGCTTGAAGAGAGAAAG | ACT |
| 1202 | CCACCTGCACCATCGCCAT | ACT |

(continued)

| dbSNP rs# | Extend Primer | Term Mix |
|---|---|---|
| 10305 | AGCTAAATTGCAACAACA | ACG |
| 729511 | ATTGAACTGTATACTTAAAAATGC | ACT |
| 934648 | ACTCTCCCACTGAGCAAGC | ACT |
| 934648 | ACTCTCCCACTGAGCAAGC | ACT |
| 1010778 | TTGAAATACTGTTTGTTTCCCCAA | ACT |
| 1046706 | TCCTAAGCTGAAGGGAATGC | CGT |
| 1436522 | GAGGAAGCATAGATTTGGTGT | ACT |
| 1436524 | CCAGGGTGCTCTGGTTTAATT | ACT |
| 1436525 | GGCTTAAACCTGGGAGG | ACG |
| 1436527 | GAGCTGTTTGCATTTATAACTCA | ACG |
| 1436529 | ACCACATGTACGTAAGGGGA | ACT |
| 1469869 | AAACACCATCTACTCTGAAGAA | ACG |
| 1469870 | CTTATATTCTCTGTGGCACCAA | ACT |
| 1541998 | ATTATTCTGATGGTAATGATCCAG | ACG |
| 1946733 | CTAAACATCTCTTGAATATTCTG | ACG |
| 2043648 | TGATTTTTAGCTAAAGGGGACA | ACT |
| 2043649 | CCTCTTGTCTTATTATCCC | ACT |
| 2043650 | GCACATAGTAGTAGCTCA | ACT |
| 2060588 | ATTGGCTTAATCTGTACATCAATT | ACG |
| 2118044 | GTGGGGTTAGATATTATTTCCTGA | CGT |
| 2164535 | GATAAATGTGAGATTGAGAGA | CGT |
| 2164536 | CCTGTGTTCCTTTGTATTTATAT | ACT |
| 2164537 | CGGCTTCTACTCTCTTATTCA | ACT |
| 2164538 | GTCACATTCTTACCCTC | ACT |
| 2282596 | GAAACCTTGCATGAACT | CGT |
| 2282597 | CAGAAGCTACTTTTCCTTCA | ACG |
| 2282598 | AGGAAAAGTAGCTTCTGGG | ACG |
| 2289490 | GCTAGACTCCTGATACC | ACG |
| 2289491 | GGCTTGCTCCTGGTAATTTA | ACG |
| 2575672 | CAAGGATTATGTTAACCACT | ACG |
| 2575674 | TATTCACACCTGCCTTC | CGT |
| 2575675 | GTTCTTGCCTGGTTTAC | ACG |
| 2575677 | GGAATGAGGGCAACAGGA | ACT |
| 2575678 | TGTGGCTCAGGTCCAGG | ACT |
| 2575679 | CTTCCTGGACATTAAATTGT | ACT |
| 2575680 | GGATGCATGGTTTCTCTAAT | ACT |
| 2575681 | TTCTTTTCTCTTTTAGGAATCT | ACG |

(continued)

| dbSNP rs# | Extend Primer | Term Mix |
|---|---|---|
| 2589504 | GTGCTAGGATCCTCAGT | ACG |
| 2589505 | GTTTTAGCATAATTGCTTCTTTA | ACG |
| 2589506 | GAGAAGAAACCTGCCCA | ACG |
| 2589509 | AGGGCTGCAGGGAAGAT | ACT |
| 2589514 | AGAAAAGGTTTTTAAAGTCCTC | ACG |
| 2589515 | GAAAACTGTTACCCACTC | ACT |
| 2589516 | GGTTTGGGGGTTTCATT | CGT |
| 2589518 | TGCACCGATCTTCAAATAAA | ACG |
| 2589523 | TTTCCCAGATTAATTATCAGATT | ACG |
| 2589525 | TTAGTGGTGTGACTTGCA | ACG |
| 2869408 | CGAATCTCTTTAACTGCTG | ACT |
| 3755970 | GGTTTCTTCTAAAACTGACCT | ACT |
| 3775164 | TTTTTTGGGATCTTGATATTTTTA | ACT |
| 3775166 | AACTTATGAAAGAATATGAAGGAT | ACT |
| 3775167 | TAAGAGAAGTCTTCAGTGCTT | ACG |
| 3775169 | GCAGAGATTTTTCAAAATCTCTAA | ACT |
| 3775170 | TTTTTAAAGCTGAAAATAAACCA | CGT |
| 3775173 | GCCGTCGAACAAATACT | ACT |
| 3775176 | TATTTCCCAAGTGCCCA | ACG |
| 3775182 | CTGTCAGTTGCCTTAGG | ACT |
| 3775183 | AGTCAAGACCAGCTGGG | ACG |
| 3775184 | CTCTTTCTTCTGATCCC | ACT |
| 3775187 | AGTGCATTACAGTGGTC | ACT |
| 3822037 | TTTGCTTATTTCATAGAAGGAAT | ACT |

Genetic Analysis of Allelotyping Results

Allelotyping results are shown for cases and controls in Table 19. The allele frequency for the A2 allele is noted in the fifth and sixth columns for breast cancer pools and control pools, respectively, where "AF" is allele frequency. The allele frequency for the A1 allele can be easily calculated by subtracting the A2 allele frequency from 1 (A1 AF =1-A2 AF). For example, the SNP rs2575681 has the following case and control allele frequencies: case A1 (C) = 0.611; case A2 (T) = 0.389; control A1 (C) = 0.632; and control A2 (T) = 0.368, where the nucleotide is provided in paranthesis. SNPs with blank allele frequencies were untyped.

**Table 19**

| dbSNP rs# | Position in Figure 2 | Chromosome Position | A1/A2 Allele | A2 Case AF | A2 Control AF | p-Value |
|---|---|---|---|---|---|---|
| 2575681 | 191 | 87306691 | C/T | 0.389 | 0.368 | 0.483 |
| 2575680 | 1490 | 87307990 | A/G | 0.599 | 0.585 | 0.646 |
| 2589505 | 3781 | 87310281 | C/T | 0.484 | 0.493 | 0.753 |
| 2589504 | 3935 | 87310435 | G/A | 0.258 | 0.274 | 0.563 |

(continued)

| dbSNP rs# | Position in Figure 2 | Chromosome Position | A1/A2 Allele | A2 Case AF | A2 Control AF | p-Value |
|---|---|---|---|---|---|---|
| 2164538 | 4512 | 87311012 | T/C | 0.403 | 0.412 | 0.784 |
| 2575679 | 7573 | 87314073 | A/G | 0.020 | 0.003 | 0.006 |
| MAP_SNP1 | 8467 | 87314967 | A/T | 0.704 | 0.682 | 0.441 |
| 2869408 | 9001 | 87315501 | C/G | 0.708 | 0.716 | 0.777 |
| 934648 | 9732 | 87316232 | T/C | 0.655 | 0.664 | 0.741 |
| 2164537 | 13477 | 87319977 | T/C | 0.262 | 0.306 | 0.109 |
| 2575678 | 13787 | 87320287 | A/C | 0.110 | 0.078 | 0.065 |
| 2575677 | 13903 | 87320403 | G/C | 0.920 | 0.991 | 0.000 |
| 2589509 | 14355 | 87320855 | T/G | 0.198 | 0.209 | 0.668 |
| 2164536 | 15053 | 87321553 | A/C | 0.623 | 0.605 | 0.534 |
| 2164535 | 15459 | 87321959 | T/A | 0.573 | 0.571 | 0.944 |
| MAP_SNP2 | 17762 | 87324262 | G/A | 0.389 | 0.401 | 0.693 |
| 2589523 | 19482 | 87325982 | C/T | 0.779 | 0.813 | 0.156 |
| 3755970 | 19631 | 87326131 | A/C | 0.118 | 0.107 | 0.563 |
| 2575675 | 22170 | 87328670 | G/A | 0.656 | 0.694 | 0.176 |
| 1202 | 22688 | 87329188 | T/C | 0.764 | 0.762 | 0.933 |
| 1201 | 22748 | 87329248 | A/G | 0.128 | 0.117 | 0.579 |
| 2589516 | 23376 | 87329876 | G/T | 0.427 | 0.478 | 0.086 |
| 2575674 | 23826 | 87330326 | A/T | 0.583 | 0.666 | 0.004 |
| 2589515 | 23868 | 87330368 | G/C | 0.413 | 0.461 | 0.106 |
| MAP_SNP3 | 24154 | 87330654 | C/T | 0.175 | 0.158 | 0.430 |
| 2589506 | 25972 | 87332472 | G/A | 0.435 | 0.491 | 0.063 |
| 1436524 | 26057 | 87332557 | A/G | 0.660 | 0.756 | 0.001 |
| 2575672 | 26361 | 87332861 | C/T | 0.274 | 0.185 | 0.001 |
| 2589518 | 26599 | 87333099 | G/A | 0.194 | 0.130 | 0.004 |
| 3775164 | 26712 | 87333212 | T/G | 0.073 | 0.080 | 0.644 |
| 2589514 | 26812 | 87333312 | G/A | 0.445 | 0.358 | 0.004 |
| 3775166 | 27069 | 87333569 | T/C | 0.249 | 0.167 | 0.001 |
| 3775167 | 32421 | 87338921 | C/T | 0.156 | 0.152 | 0.882 |
| 3775169 | 33557 | 87340057 | T/C | 0.169 | 0.130 | 0.067 |
| 2043650 | 35127 | 87341627 | A/G | 0.697 | 0.787 | 0.001 |
| 2043649 | 35222 | 87341722 | T/G | 0.698 | 0.763 | 0.016 |
| 3775170 | 35999 | 87342499 | T/A | 0.207 | 0.220 | 0.596 |
| 1541998 | 36424 | 87342924 | C/T | 0.715 | 0.772 | 0.029 |
| 2043648 | 37403 | 87343903 | A/G | 0.424 | 0.466 | 0.159 |
| 2282598 | 39203 | 87345703 | C/T | 0.022 | 0.031 | 0.324 |
| 2282597 | 39226 | 87345726 | G/A | 0.817 | 0.802 | 0.541 |

(continued)

| dbSNP rs# | Position in Figure 2 | Chromosome Position | A1/A2 Allele | A2 Case AF | A2 Control AF | p-Value |
|---|---|---|---|---|---|---|
| 3775173 | 41147 | 87347647 | T/C | 0.158 | 0.148 | 0.645 |
| 1469870 | 46176 | 87352676 | G/C | 0.118 | 0.063 | 0.002 |
| 1436522 | 50452 | 87356952 | T/C | 0.165 | 0.120 | 0.036 |
| 1946733 | 52919 | 87359419 | G/A | 0.240 | 0.226 | 0.588 |
| 1436525 | 60214 | 87366714 | G/A | 0.054 | 0.039 | 0.212 |
| 3822037 | 61093 | 87367593 | C/G | 0.956 | 0.918 | 0.010 |
| 3775176 | 62572 | 87369072 | G/A | 0.969 | 0.909 | 0.000 |
| 1436527 | 63601 | 87370101 | C/T | 0.288 | 0.251 | 0.175 |
| 1436529 | 65362 | 87371862 | T/C | 0.555 | 0.534 | 0.481 |
| 3775182 | 65863 | 87372363 | T/G | 0.858 | 0.870 | 0.568 |
| 3775183 | 66207 | 87372707 | G/A | 0.565 | 0.617 | 0.080 |
| 3775184 | 66339 | 87372839 | A/G | 0.174 | 0.185 | 0.634 |
| 3775187 | 69512 | 87376012 | T/C | 0.307 | 0.291 | 0.575 |
| 1010778 | 70759 | 87377259 | A/G | 0.330 | 0.275 | 0.048 |
| 2282596 | 71217 | 87377717 | T/A | 0.735 | 0.738 | 0.892 |
| 2118044 | 73382 | 87379882 | A/T | 0.352 | 0.319 | 0.248 |
| 1469869 | 76307 | 87382807 | C/T | 0.388 | 0.335 | 0.069 |
| 1046706 | Not mapped | | G/T | 0.538 | 0.533 | 0.866 |
| 2060588 | Not mapped | | G/A | 0.188 | 0.135 | 0.016 |
| 2289490 | Not mapped | | C/T | 0.780 | 0.812 | 0.187 |
| 2289491 | Not mapped | | C/T | 0.960 | 0.971 | 0.297 |
| 729511 | Not mapped | | T/C | 0.864 | 0.866 | 0.914 |

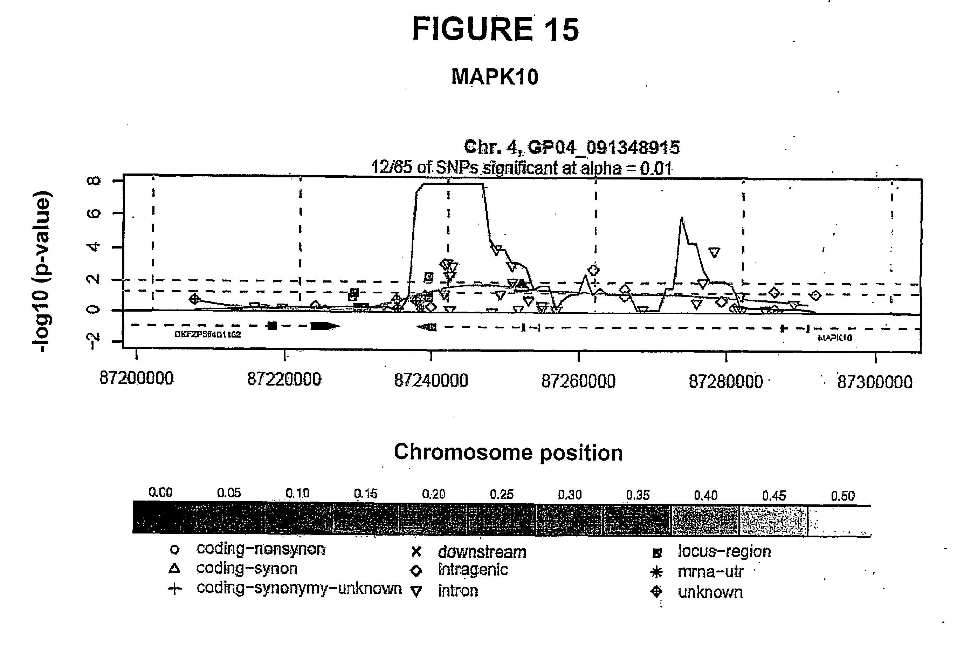

Figure 15 shows the proximal SNPs in and around the MAPK10 region for females. The position of each SNP on the chromosome is presented on the x-axis. The y-axis gives the negative logarithm (base 10) of the p-value comparing the estimated allele in the case group to that of the control group. The minor allele frequency of the control group for each SNP designated by an X or other symbol on the graphs in Figure 15 can be determined by consulting Table 19. By proceeding down the Table from top to bottom and across the graphs from left to right the allele frequency associated with each symbol shown can be determined.

To aid the interpretation, multiple lines have been added to the graph. The broken horizontal lines are drawn at two common significance levels, 0.05 and 0.01. The vertical broken lines are drawn every 20kb to assist in the interpretation of distances between SNPs. Two other lines are drawn to expose linear trends in the association of SNPs to the disease. The light gray line (or generally bottommost curve) is a nonlinear smoother through the data points on the graph using a local polynomial regression method (W.S. Cleveland, E. Grosse and W.M. Shyu (1992) Local regression models. Chapter 8 of Statistical Models in S eds J.M. Chambers and T.J. Hastie, Wadsworth & Brooks/Cole.). The black line (or generally top-most curve, *e.g.*, see peak in left-most graph just to the left of position 92150000) provides a local test for excess statistical significance to identify regions of association. This was created by use of a 10kb sliding window with 1kb step sizes. Within each window, a chi-square goodness of fit test was applied to compare the proportion of SNPs that were significant at a test wise level of 0.01, to the proportion that would be expected by chance alone (0.05 for the methods used here). Resulting p-values that were less than $10^{-8}$ were truncated at that value.

Finally, the gene or genes present in the loci region of the proximal SNPs as annotated by Locus Link (http address: www.ncbi.nlm.nih-gov/LocusLink/) are provided on the graph. The exons and introns of the genes in the covered region are plotted below each graph at the appropriate chromosomal positions. The gene boundary is indicated by the broken horizontal line. The exon positions are shown as thick, unbroken bars. An arrow is place at the 3' end of each gene to show the direction of transcription.

Example 6

*KIAA0861* Proximal SNPs

It has been discovered that a polymorphic variation (rs2001449) in a gene encoding KIAA0861 is associated with the occurrence of breast cancer (see Examples 1 and 2). Subsequently, SNPs proximal to the incident SNP (rs2001449) were identified and allelotyped in breast cancer sample sets and control sample sets as described in Examples 1 and 2. A total of sixty-three allelic variants located within or nearby the KIAA0861 gene were identified and fifty-severn allelic variants were allelotyped. The polymorphic variants are set forth in Table 20. The chromosome position provided in column four of Table 20 is based on Genome "Build 33" of NCBI's GenBank.

**Table 20**

| dbSNP rs# | Chromosome | Position in Figure 3 | Chromosome Position | Allele Variants |
|---|---|---|---|---|
| 3811729 | 3 | 107 | 184282507 | A/G |
| 693208 | 3 | 2157 | 184284557 | C/G |
| 488277 | 3 | 7300 | 184289700 | T/C |
| 645039 | 3 | 8233 | 184290633 | T/C |
| 670232 | 3 | 9647 | 184292047 | A/T |
| 575326 | 3 | 9868 | 184292268 | T/C |
| 575386 | 3 | 9889 | 184292289 | C/G |
| 471365 | 3 | 10621 | 184293021 | G/C |
| 496251 | 3 | 11003 | 184293403 | G/A |
| 831246 | 3 | 11507 | 184293907 | T/C |
| 831247 | 3 | 11527 | 184293927 | G/C |
| 831249 | 3 | 11718 | 184294118 | C/T |
| 831250 | 3 | 11808 | 184294208 | T/C |
| 831252 | 3 | 12024 | 184294424 | T/C |
| 512071 | 3 | 13963 | 184296363 | C/T |
| 1502761 | 3 | 14300 | 184296700 | A/C |
| 681516 | 3 | 14361 | 184296761 | C/T |
| 619424 | 3 | 16287 | 184298687 | T/G |
| 529055 | 3 | 18635 | 184301035 | A/G |
| 664010 | 3 | 19365 | 184301765 | T/G |
| 2653845 | 3 | 24953 | 184307353 | G/A |
| 472795 | 3 | 25435 | 184307835 | G/A |
| 507079 | 3 | 26847 | 184309247 | G/A |
| 534333 | 3 | 27492 | 184309892 | T/C |
| 831242 | 3 | 27620 | 184310020 | T/C |
| 536111 | 3 | 27678 | 184310078 | C/T |
| 536213 | 3 | 27714 | 184310114 | G/A |
| 831245 | 3 | 29719 | 184312119 | A/G |
| 639690 | 3 | 30234 | 184312634 | T/C |

(continued)

| dbSNP rs# | Chromosome | Position in Figure 3 | Chromosome Position | Allele Variants |
|---|---|---|---|---|
| 684174 | 3 | 31909 | 184314309 | T/C |
| 571761 | 3 | 32153 | 184314553 | C/G |
| 1983421 | 3 | 33572 | 184315972 | T/C |
| 2314415 | 3 | 42164 | 184324564 | T/G |
| 2103062 | 3 | 43925 | 184326325 | A/G |
| 6804951 | 3 | 45031 | 184327431 | C/T |
| 1403452 | 3 | 45655 | 184328055 | T/C |
| 903950 | 3 | 48350 | 184330750 | C/A |
| 2017340 | 3 | 48418 | 184330818 | A/G |
| 2001449 | 3 | 48563 | 184330963 | G/C |
| 3821522 | 3 | 53189 | 184335589 | A/G |
| 1390831 | 3 | 56468 | 184338868 | T/G |
| 1353566 | 3 | 59358 | 184341758 | C/A |
| 1813856 | 3 | 63761 | 184346161 | C/T |
| 2272115 | 3 | 65931 | 184348331 | G/A |
| 3732603 | 3 | 67040 | 184349440 | G/C |
| 940055 | 3 | 69491 | 184351891 | A/C |
| 2314730 | 3 | 83308 | 184365708 | A/G |
| KIAA0861_373 2602 | 3 | 126545 | 184408945 | C/T |
| KIAA0861_229 3203 | 3 | 137592 | 184419992 | A/T |
| 7639705 | 3 | 147169 | 184429569 | G/T |

Assay for Verifying and Allelotyping SNPs

The methods used to verify and allelotype the sixty-three proximal SNPs of Table 20 are the same methods described in Examples 1 and 2 herein. The PCR primers and extend primers used in these assays are provided in Table 21 and Table 22, respectively.

**Table 21**

| dbSNP rs# | Forward PCR primer | Reverse PCR primer |
|---|---|---|
| 471365 | ACGTTGGATGTGAGTGACATTTGTGTCACC | ACGTTGGATGCGGAGGATCTGAACAACTTC |
| 472795 | ACGTTGGATGTCACCTGAGCATCAGACATG | ACGTTGGATGATAGTGGAAGGAGAAACGGG |
| 484315 | ACGTTGGATGGTTCTAATGTCACCCCTTCC | ACGTTGGATGCAATGTGGCAAATTCTCTGG |
| 488277 | ACGTTGGATGCACACATTCTTCTCAAGTGC | ACGTTGGATGGGAGGGACACAATTTAACTC |
| 496251 | ACGTTGGATGGGGAGTCATTCCAATACCAG | ACGTTGGATGGGAGTGAAAGGTCATATTGG |
| 502289 | ACGTTGGATGATCACTGCAACCTCCACCTC | ACGTTGGATGTGTGGCATGAGCCTGTAATC |
| 507079 | ACGTTGGATGAAGCCTCAGATGAGGCATAC | ACGTTGGATGTCTGAAAGGGTTCAGGAAGG |
| 512071 | ACGTTGGATGCAAATCACCCCTGACAATTC | ACGTTGGATGACCAGCACACTCAGCTTTAG |
| 519088 | ACGTTGGATGTCACCTGAGGTCAGGAGTTG | ACGTTGGATGAGGTTTCACCATGTTAGCCG |

(continued)

| dbSNP rs# | Forward PCR primer | Reverse PCR primer |
|---|---|---|
| 529055 | ACGTTGGATGCTGCAGTTATCTGGGTGAGC | ACGTTGGATGCCAGAACGTGGCTTGTTGGG |
| 534333 | ACGTTGGATGCGTTGATGCACTGAAGGGAG | ACGTTGGATGAGAGGCTAAATGTTGGCAGG |
| 536111 | ACGTTGGATGTGTATCTGATCCCAGGTCAC | ACGTTGGATGATTGGTGTTAAGTGGCGTGC |
| 536213 | ACGTTGGATGTGAGGACCTCATTATTGGTG | ACGTTGGATGCTGAGCAATCGAACTGCTAC |
| 571761 | ACGTTGGATGAATATCCTAGGCTAGCAGTG | ACGTTGGATGGTGCATAAATACATGAATAG |
| 575326 | ACGTTGGATGACAGAGAGGCTTGGTCATAC | ACGTTGGATGGGTGCTTGGTTGTGATTCTC |
| 575386 | ACGTTGGATGATTCCTGCAGGTACTGTGTC | ACGTTGGATGTGAGCCCAAAACTACTGCTG |
| 578886 | ACGTTGGATGATGAAGTCTCGCTCTGTTGC | ACGTTGGATGAATCACTTGAACCCAGGAGG |
| 602646 | ACGTTGGATGTCTGGGACCGTTTACCGCA | ACGTTGGATGGAGGAGACCCAGGGTATGAG |
| 619424 | ACGTTGGATGACCGGGAGCTCCCAGTCTG | ACGTTGGATGTGGGAATCGGTTGAGAGCCG |
| 620722 | ACGTTGGATGTAAGGCGCCTGCAGAGGCGA | ACGTTGGATGGCAGCAAAGAATTGCCCGGC |
| 631755 | ACGTTGGATGATTTGTAGCTTTGCCCCAGC | ACGTTGGATGTTTGTGAGCTCCAAGTTGGG |
| 639690 | ACGTTGGATGGCATTTTACCACCATGTGGTT | ACGTTGGATGCCTTCATGTTAATTCTGCCC |
| 645039 | ACGTTGGATGCCTCTGAGTTCCCTCAGTTT | ACGTTGGATGTTATCACCCTGCTGTCCTAC |
| 664010 | ACGTTGGATGTGGTACCTCCAGGTAAAATG | ACGTTGGATGTCCAGGCAGTCATTTTACCC |
| 670232 | ACGTTGGATGGAAGGTGGAGCAGACATTAG | ACGTTGGATGACCTTAGTTATACCAGGCAC |
| 678454 | ACGTTGGATGTTAAGCCAGTCCCCACAAGG | ACGTTGGATGTTCTCTGCGGAGGAAAGTGC |
| 681516 | ACGTTGGATGCTCCTCCTCAGAGGACTAAC | ACGTTGGATGAGCCCAAGGACTCATACAAC |
| 683302 | ACGTTGGATGACCACGCCTGGCTAATTTTG | ACGTTGGATGAAACATGGCGAAACCCGGTC |
| 684174 | ACGTTGGATGCTTTACTGAGTGGGCAAACG | ACGTTGGATGTCTAAGTGGAACTCAGCAGC |
| 684846 | ACGTTGGATGAAGTTCCTCTGGTGGACAAC | ACGTTGGATGACCACCAGATAAAATCCCTC |
| 693208 | ACGTTGGATGTTTTGACAGGGCTTGAGTCC | ACGTTGGATGGCTGAAAGCCCTCAATCTAG |
| 831242 | ACGTTGGATGCAATTGCTCAGACCTTCACC | ACGTTGGATGAATGCTAGAGACATTGCACC |
| 831245 | ACGTTGGATGCTAGAATTACAGGTGCACAC | ACGTTGGATGGCCAAGATGGTGAAACCTTG |
| 831246 | ACGTTGGATGCACAATCTGTTAGAATGGTGG | ACGTTGGATGCGTCAAGACTGAATGCATAG |
| 831247 | ACGTTGGATGGAAAATATAGTCCTACACAA | ACGTTGGATGCGTCAAGACTGAATGCATAG |
| 831249 | ACGTTGGATGTCTCCTAATGCTATCCCTCC | ACGTTGGATGAACACATGGACACAGGAAGG |
| 831250 | ACGTTGGATGAGGGACATGGATGAAATTGG | ACGTTGGATGAATTCCCACCTATGAGTGAG |
| 831252 | ACGTTGGATGTGGGTATATACCCAAAGGAC | ACGTTGGATGGGTTGGTTCCAAGTCTTTGC |
| 903950 | ACGTTGGATGCTTCAGTTCAGGGAGAGATC | ACGTTGGATGATAGGGCCCCCAGCATAAAA |
| 940054 | ACGTTGGATGTGGTAGAGATGAGGTCTTGC | ACGTTGGATGAAAGGCAGGAGGATTGCTTG |
| 940055 | ACGTTGGATGTATGCTTCCAGTCTCTGACC | ACGTTGGATGATAGGTAATCCAGTTGGGCC |
| 1353566 | ACGTTGGATGGGTGTACTCTGCCATTTGTC | ACGTTGGATGTGGAGGAGGTTCTAGTACCC |
| 1390831 | ACGTTGGATGGTCTGCCAAAGTTCCCTTAG | ACGTTGGATGAGGAAAGGGAAGAGAAACCG |
| 1403452 | ACGTTGGATGCAGAAGTTAGGATGCAGATG | ACGTTGGATGCCAGTAGAGATAGAATTTTGG |
| 1502761 | ACGTTGGATGCAGAAATATGAAGGTGGCCC | ACGTTGGATGACCTTGAGCTCTGAGCCCTT |
| 1629673 | ACGTTGGATGAAGGATCACGTGAAGTCAGG | ACGTTGGATGGGCACCATGTGTGGCTAATT |

(continued)

| dbSNP rs# | Forward PCR primer | Reverse PCR primer |
|---|---|---|
| 1813856 | ACGTTGGATGTCTGACTCCCTGATTCAAGC | ACGTTGGATGACAAAAATTAGCCGGGCGTG |
| 1983421 | ACGTTGGATGTCCAGGTGTTATGGAGTCAG | ACGTTGGATGGGCTTCTTGTGCTGCTGTGT |
| 2001449 | ACGTTGGATGATGTCAAGTGCACCCACATG | ACGTTGGATGAGGAAGAAACTGACGGAAGG |
| 2017340 | ACGTTGGATGTATTCCACTGCCTGCTTTCC | ACGTTGGATGGAAAACAGGAGGAAGTGGTG |
| 2030578 | ACGTTGGATGTTCTCCACTTTCTGGTCAAC | ACGTTGGATGAACAACCTTACTTCATGCCC |
| 2049280 | ACGTTGGATGCTTCCCAACATTTTCGGCTC | ACGTTGGATGTGGATACTGAGGGTCAACTG |
| 2103062 | ACGTTGGATGTGCAGCCCTCAACCTTTCAG | ACGTTGGATGCCTTATTCAGTTACTATTACG |
| 2272115 | ACGTTGGATGAGTTGTGAGTGATTTCAGGG | ACGTTGGATGCAGGCCTTCTTGCTCTTATC |
| 2272116 | ACGTTGGATGATCTGTTGCCTTAGGTTCAC | ACGTTGGATGCTGTGCCTTCTGAGTAGTTC |
| 2314415 | ACGTTGGATGGGCTGAGTAACAGTCCATTG | ACGTTGGATGCTTACAGTATCCAAAAAGGG |
| 2314730 | ACGTTGGATGCTCAGGTAATCTGCCTTCTC | ACGTTGGATGCAGGGATAATGAGAACAAATC |
| 2653845 | ACGTTGGATGATCACTTGGACTCAGGAAGC | ACGTTGGATGAGTCTTGCTCTGTTTCCAGG |
| 3732603 | ACGTTGGATGCTCTCAATTCCATCAGTCTC | ACGTTGGATGCTTTACGAATTTCACAACAGG |
| 3811728 | ACGTTGGATGACGCGCCACACCTCCCTAC | ACGTTGGATGACGTGTCGGTCCCCTTTCAT |
| 3811729 | ACGTTGGATGTGGGCGAGGTTCTGCAGCGT | ACGTTGGATGGTTTCGTTTCTCCGGCACAG |
| 3811731 | ACGTTGGATGTGCGGTAAACGGTCCCAGAG | ACGTTGGATGAACTCCGCCGGCCCCCTCCTA |
| 3821522 | ACGTTGGATGAACCCGCACTACAAGATTCC | ACGTTGGATGGTCAGTCCCACATTCAGAAC |

**Table 22**

| dbSNP rs# | Extend Primer | Term Mix |
|---|---|---|
| 471365 | TCCAAAACCACCAGATAAAATC | ACT |
| 472795 | GACATGTCCCTCTCGGCCT | ACG |
| 484315 | GGTATCAGGAAGAGTCA | ACT |
| 488277 | AGTGCACACAGAACATTTAACA | ACT |
| 496251 | GTATTGTCCTCCAGTGA | ACG |
| 502289 | CTGTAATCCCAGCTACTC | ACT |
| 507079 | GGCAATGTTTGCCCTTT | ACG |
| 512071 | CCCTGACAATTCCAAAACTAA | ACG |
| 519088 | TTTCGCCATGTTTGCCAGG | ACG |
| 529055 | GAGCAGGCAGCACAAGT | ACT |
| 534333 | GGGAGAAAGTAACAGGGTC | ACT |
| 536111 | GTGAAGGTCTGAGCAAT | ACG |
| 536213 | TGGTGTTAAGTGGCGTG | ACG |
| 571761 | CTAGGCTAGCAGTGGGGTTG | ACT |
| 575326 | TGGTCATACCCTTCAAG | ACT |
| 575386 | GAAGGGTATGACCAAGC | ACT |

(continued)

| dbSNP rs# | Extend Primer | Term Mix |
|---|---|---|
| 578886 | TGAGCCAAGATCATGCC | CGT |
| 602646 | CCAGGGTATGAGCGGAGGA | ACT |
| 619424 | TGCGGCCCCCGCCGGGTT | ACT |
| 620722 | GAATTGCCCGGCTCCGAAT | ACT |
| 631755 | TCCAAGTTGGGTCAAAG | ACT |
| 639690 | CTGCTATTCATTTGTGTAGA | ACT |
| 645039 | CCCTCAGTTTTTATTGATTATT | ACT |
| 664010 | ACCTCCAGGTAAAATGATTAGTT | ACT |
| 670232 | TGGGCAAACAAGCCCAT | CGT |
| 678454 | CAGGGATGGTAATTGAC | ACG |
| 681516 | GGCCACCTTCATATTTC | ACG |
| 683302 | CAGGAGATCCAGACCATCCC | ACG |
| 684174 | CTCTGATGTTACCTCCTCC | ACT |
| 684846 | AGTTGTTCAGATCCTCC | ACT |
| 693208 | TCAATCTAGTGATAAGGAGGGT | ACT |
| 831242 | CAGGTGGATGGGGACAC | ACT |
| 831245 | CACACCACCACGCCCGGCT | ACT |
| 831246 | AGAATGGTGGTGTATTTTTAC | ACT |
| 831247 | TAGTCCTACACAATCTGTTA | ACT |
| 831249 | GCTATCCCTCCCCCCTTCCC | ACG |
| 831250 | GACAAAAAACCAAACACC | ACT |
| 831252 | CTATAAAGACACATGCACAC | ACT |
| 903950 | AGATCACATTGCCAACCCCCA | CGT |
| 940054 | AAAGTAGCAGTTTGAGACCA | ACT |
| 940055 | GTCTCTGACCACTTGACCCA | ACT |
| 1353566 | TTGTCAGTTATGAGACCTTG | CGT |
| 1390831 | GGTTAGGAAGAAATCTGTG | ACT |
| 1403452 | CACAGATGCTCATGGGTCC | ACT |
| 1502761 | GGAGGAGGCACTATTAAT | ACT |
| 1629673 | TGTGGAGACAAGGTCTCACT | ACT |
| 1813856 | TCAAGCGATTCTCCTGC | ACG |
| 1983421 | GGCAGGGAAGAGAAGAGC | ACT |
| 2001449 | CACATGCCTGCTCGCCCCC | ACT |
| 2017340 | CCCTAAAGCATCTCACAGCCCC | ACT |
| 2030578 | TCATGCCCATTGGGTTAG | ACT |
| 2049280 | GGGTCAACTGTACCAAG | ACG |
| 2103062 | GAGATCATTTCTCCTTCAAC | ACT |

(continued)

| dbSNP rs# | Extend Primer | Term Mix |
|---|---|---|
| 2272115 | ATACCTCAGAATACAGCTTTTTTT | ACG |
| 2272116 | TCTCATTTCTCCTCTCTTTC | ACG |
| 2314415 | TAGTTGATGAAGATTTGGG | ACT |
| 2314730 | TCCTTCTTCTCTGCTTT | ACT |
| 2653845 | AAGCGGAGGTTGCAGTGAGC | ACG |
| 3732603 | CTCATTTCCACCCTTCT | ACT |
| 3811728 | GTCCCCTTTCATCTAAAC | ACT |
| 3811729 | TCTGCAGCGTGCGGCGA | ACT |
| 3811731 | CCTACCCCTACGGAGCC | ACT |
| 3821522 | GCATCTTCAGGAATCTTG | ACT |

Genetic Analysis of Allelotyping Results

Allelotyping results are shown for cases and controls in Table 23. The allele frequency for the A2 allele is noted in the fifth and sixth columns for breast cancer pools and control pools, respectively, where "AF" is allele frequency. The allele frequency for the A1 allele can be easily calculated by subtracting the A2 allele frequency from 1 (A1 AF = 1-A2 AF). For example, the SNP in row 2 of Table 13 (rs3811729) has the following case and control allele frequencies: case A1 (A) = 0.976; case A2 (G) = 0.024; control A1 (A) = 0.948; and control A2 (G) = 0.052, where the nucleotide is provided in paranthesis. SNPs with blank allele frequencies were untyped ("not AT").

**Table 23**

| dbSNP rs# | Position in Fig 3 | Chrom Position | Alleles (A1/A2) | A2 Case AF | A2 Control AF | p-Value |
|---|---|---|---|---|---|---|
| 3811729 | 107 | 184282507 | A/G | 0.024 | 0.052 | 0.017 |
| 693208 | 2157 | 184284557 | C/G | 0.186 | 0.207 | 0.368 |
| 3811731 | not mapped | | A/G | 0.690 | 0.641 | 0.084 |
| 602646 | not mapped | | C/G | 0.693 | 0.660 | 0.244 |
| 488277 | 7300 | 184289700 | T/C | 0.099 | 0.103 | 0.848 |
| 645039 | 8233 | 184290633 | T/C | 0.014 | 0.008 | 0.316 |
| 1629673 | not mapped | | T/C | 0.064 | 0.093 | 0.069 |
| 670232 | 9647 | 184292047 | A/T | 0.865 | 0.863 | 0.932 |
| 575326 | 9868 | 184292268 | T/C | 0.128 | 0.129 | 0.949 |
| 575386 | 9889 | 184292289 | C/G | 0.776 | 0.779 | 0.905 |
| 684846 | not mapped | | C/G | 0.799 | 0.745 | 0.033 |
| 471365 | 10621 | 184293021 | G/C | 0.746 | 0.740 | 0.815 |
| 496251 | 11003 | 184293403 | G/A | 0.156 | 0.160 | 0.853 |
| 831246 | 11507 | 184293907 | T/C | 0.773 | 0.802 | 0.243 |
| 831247 | 11527 | 184293927 | G/C | 0.829 | 0.826 | 0.879 |
| 831249 | 11718 | 184294118 | C/T | 0.071 | 0.051 | 0.160 |
| 831250 | 11808 | 184294208 | T/C | 0.682 | 0.697 | 0.589 |
| 831252 | 12024 | 184294424 | T/C | 0.752 | 0.762 | 0.695 |

(continued)

| dbSNP rs# | Position in Fig 3 | Chrom Position | Alleles (A1/A2) | A2 Case AF | A2 Control AF | p-Value |
|---|---|---|---|---|---|---|
| 512071 | 13963 | 184296363 | C/T | 0.616 | 0.642 | 0.367 |
| 1502761 | 14300 | 184296700 | A/C | 0.596 | 0.593 | 0.933 |
| 681516 | 14361 | 184296761 | C/T | 0.240 | 0.189 | 0.037 |
| 619424 | 16287 | 184298687 | T/G | 0.076 | 0.070 | 0.704 |
| 620722 | not mapped | | C/T | 0.779 | 0.819 | 0.100 |
| 529055 | 18635 | 184301035 | A/G | 0.601 | 0.637 | 0.219 |
| 664010 | 19365 | 184301765 | T/G | 0.455 | 0.394 | 0.039 |
| 678454 | not mapped | | T/G | 0.000 | 0.004 | 0.117 |
| 2653845 | 24953 25435 | 184307353 | G/A | 0.175 | 0.168 | 0.775 |
| 472795 | | 184307835 | G/A | 0.082 | 0.077 | 0.756 |
| 502289 | not mapped | | T/G | 0.003 | 0.000 | 0.172 |
| 507079 | 26847 | 184309247 | G/A | 0.833 | 0.835 | 0.937 |
| 534333 | 27492 | 184309892 | T/C | 0.496 | 0.509 | 0.675 |
| 831242 | 27620 | 184310020 | T/C | 0.728 | 0.776 | 0.064 |
| 536111 | 27678 | 184310078 | C/T | 0.800 | 0.812 | 0.632 |
| 536213 | 27714 | 184310114 | G/A | 0.271 | 0.281 | 0.710 |
| 831245 | 29719 | 184312119 | A/G | 0.020 | 0.012 | 0.314 |
| 639690 | 30234 | 184312634 | T/C | 0.117 | 0.106 | 0.577 |
| 684174 | 31909 | 184314309 | T/C | 0.304 | 0.298 | 0.826 |
| 571761 | 32153 | 184314553 | C/G | 0.406 | 0.425 | 0.525 |
| 1983421 | 33572 | 184315972 | T/C | 0.433 | 0.425 | 0.791 |
| 2314415 | 42164 | 184324564 | T/G | 0.014 | 0.050 | 0.001 |
| 2103062 | 43925 | 184326325 | A/G | 0.328 | 0.361 | 0.256 |
| 6804951 | 45031 | 184327431 | C/T | no AT | no AT | - |
| 1403452 | 45655 | 184328055 | T/C | 0.025 | 0.072 | 0.001 |
| 903950 | 48350 | 184330750 | C/A | 0.577 | 0.594 | 0.556 |
| 2017340 | 48418 | 184330818 | A/G | 0.033 | 0.054 | 0.089 |
| 2001449 | 48563 | 184330963 | G/C | 0.262 | 0.205 | 0.025 |
| 3821522 | 53189 | 184335589 | A/G | 0.500 | 0.480 | 0.508 |
| 1390831 | 56468 | 184338868 | T/G | 0.944 | 0.923 | 0.160 |
| 1353566 | 59358 | 184341758 | C/A | 0.545 | 0.533 | 0.692 |
| 1813856 | 63761 | 184346161 | C/T | 0.040 | 0.041 | 0.933 |
| 2272115 | 65931 | 184348331 | G/A | 0.324 | 0.370 | 0.106 |
| 3732603 | 67040 | 184349440 | G/C | 0.228 | 0.209 | 0.429 |
| 940055 | 69491 | 184351891 | A/C | 0.225 | 0.198 | 0.272 |
| 2314730 | 83308 | 184365708 | A/G | 0.649 | 0.691 | 0.135 |
| 484315 | not mapped | | C/G | 0.256 | 0.234 | 0.404 |

(continued)

| dbSNP rs# | Position in Fig 3 | Chrom Position | Alleles (A1/A2) | A2 Case AF | A2 Control AF | p-Value |
|---|---|---|---|---|---|---|
| KIAA0861_ 3732602 | 126545 | 184408945 | C/T | no AT | no AT | - |
| KIAA0861_ 2293203 | 137592 | 184419992 | A/T | no AT | no AT | - |
| 7639705 | 147169 | 184429569 | G/T | no AT | no AT | - |

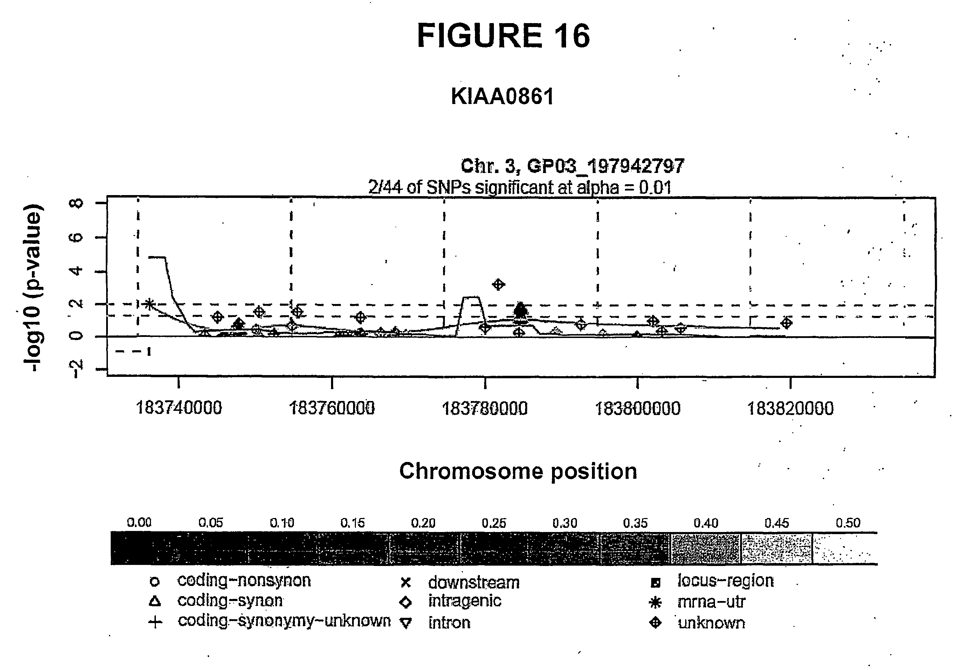

Figure 16 shows the proximal SNPs in and around the KIAA0861 gene for females. As indicated, some of the SNPs were untyped. The position of each SNP on the chromosome is presented on the x-axis. The y-axis gives the negative logarithm (base 10) of the p-value comparing the estimated allele in the case group to that of the control group. The minor allele frequency of the control group for each SNP designated by an X or other symbol on the graphs in Figure 16 can be determined by consulting Table 23. By proceeding down the Table from top to bottom and across the graphs from left to right the allele frequency associated with each symbol shown can be determined.

To aid the interpretation, multiple lines have been added to the graph. The broken horizontal lines are drawn at two common significance levels, 0.05 and 0.01. The vertical broken lines are drawn every 20kb to assist in the interpretation of distances between SNPs. Two other lines are drawn to expose linear trends in the association of SNPs to the disease. The light gray line (or generally bottommost curve) is a nonlinear smoother through the data points on the graph using a local polynomial regression method (W.S. Cleveland, E. Grosse and W.M. Shyu (1992) Local regression models. Chapter 8 of Statistical Models in S eds J.M. Chambers and T.J. Hastie, Wadsworth & Brooks/Cole.). The black line (or generally top-most curve, *e.g.*, see peak in left-most graph just to the left of position 92150000) provides a local test for excess statistical significance to identify regions of association. This was created by use of a 10kb sliding window with 1kb step sizes. Within each window, a chi-square goodness of fit test was applied to compare the proportion of SNPs that were significant at a test wise level of 0.01, to the proportion that would be expected by chance alone (0.05 for the methods used here). Resulting p-values that were less than $10^{-8}$ were truncated at that value.

Finally, the gene or genes present in the loci region of the proximal SNPs as annotated by Locus Link (http address: www.ncbi.nlm.nih.gov/LocusLink/) are provided on the graph. The exons and introns of the genes in the covered region are plotted below each graph at the appropriate chromosomal positions. The gene boundary is indicated by the broken horizontal line. The exon positions are shown as thick, unbroken bars. An arrow is place at the 3' end of each gene to show the direction of transcription.

Additional Genotyping

A total of five SNPs, including the incident SNP, were genotyped in the discovery cohort. The discovery cohort is described in Example 1. Four of the SNPs are non-synonomous, coding SNPs. Two of the SNPs (rs2001449 and rs6804951) were found to be significantly associated with breast cancer with a p-value of 0.001 and 0.007, respectively. See Table 26.

The methods used to verify and genotype the five proximal SNPs of Table 26 are the same methods described in Examples 1 and 2 herein. The PCR primers and extend primers used in these assays are provided in Table 24 and Table 25, respectively.

**Table 24**

| dbSNP rs# | Forward PCR primer | Reverse PCR primer |
|---|---|---|
| rs7639705 | ACGTTGGATGTGTCAGAAAGCAAACCTGGC | ACGTTGGATGTTACAGGCATTGGAGACAGC |
| rs2293203 | ACGTTGGATGCTGCATAATGGTGGCTTTGG | ACGTTGGATGTGTGGGTGTTCACTTTGCAG |
| rs3732602 | ACGTTGGATGCCCTCTTGTCAGGAAGTTCT | ACGTTGGATGGAGACAGAGTTGAACTCCCG |
| rs2001449 | ACGTTGGATGAGGAAGAAACTGACGGAAGG | ACGTTGGATGATGTCAAGTGCACCCACATG |
| rs6804951 | ACGTTGGATGAAGATACGAATGGAGCCTGG | ACGTTGGATGGCAATAGGACTCCCTTTACC |

**Table 25**

| dbSNP rs# | Extend Primer | Term Mix |
|---|---|---|
| rs7639705 | TGATGCACGTGGAGCAG | CGT |
| rs2293203 | GCCCCTGGAAAAGGCCC | CGT |
| rs3732602 | GGAAGATGATGAGACTAAAT | ACG |
| rs2001449 | CACATGCCTGCTCGCCCCC | ACT |
| rs6804951 | TCCCTTTACCTTCATGG | ACG |

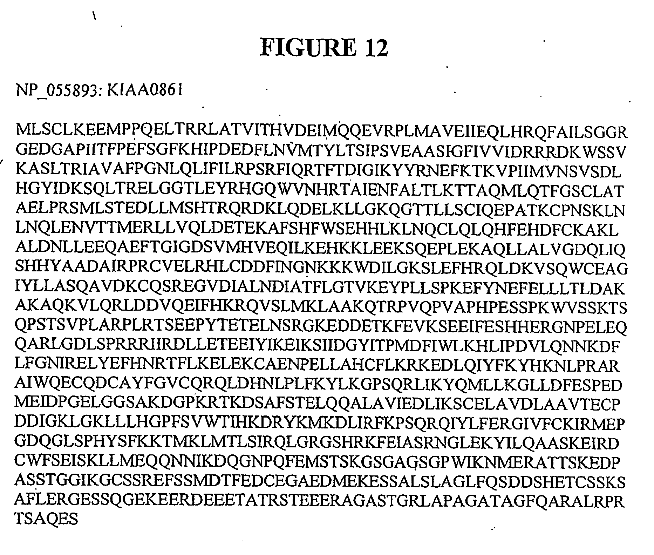

Table 26, below, shows the case and control allele frequencies along with the p-values for all of the SNPs genotyped. The disease associated allele of column 4 is in bold and the disease associated amino acid of column 5 is also in bold. The chromosome positions provided correspond to NCBI's Build 33. The amino acid change positions provided in column 5 correspond to KIAA0861 polypeptide sequence of Figure 12.

**Table 26: Genotpying Results**

| Rs number | Position in Figure 1 | Location within Gene | Alleles (A1/A2) | Amino Acid Change | A2 Case AF | A2 Control AF | p-Value | Odds Ratio |
|---|---|---|---|---|---|---|---|---|
| rs7639705 | 147169 | Exon 7 | G/T | I276L | 0.805 | 0.811 | 0.794 | 1.04 |
| rs2293203 | 137592 | Exon 8 | A/T | Q295L | 0.990 | 0.980 | 0.685 | 1.25 |
| rs3732602 | 126545 | Exon 11 | C/T | S506F | monomorphic | | | |
| rs2001449 | 48563 | Intron 19 | G/C | - | 0.307 | 0.218 | 0.001 | 1.59 |
| rs6804951 | 45031 | Exon 20 | C/T | A819T | 0.044 | 0.085 | 0.007 | 2.02 |

Example 7

*NUMA1* Proximal SNPs

It has been discovered that a polymorphic variation (rs673478) in the NUMA1/FLJ20625/LOC220074 region is associated with the occurrence of breast cancer (see Examples 1 and 2). Subsequently, SNPs proximal to the incident SNP (rs673478) were identified and allelotyped in breast cancer sample sets and control sample sets as described in Examples 1 and 2. Approximately sixty-three allelic variants located within the NUMA1/FLJ20625/LOC220074 region were identified and allelotyped. The polymorphic variants are set forth in Table 27. The chromosome position provided in column four of Table 27 is based on Genome "Build 33" of NCBI's GenBank.

**Table 27**

| dbSNP rs# | Chromosome | Position in Figure 4 | Chromosome Position | Allele Variants |
|---|---|---|---|---|
| 1894003 | 11 | 174 | 71972974 | T/C |
| 2390981 | 11 | 815 | 71973615 | G/A |
| 1939242 | 11 | 3480 | 71976280 | C/T |
| 1894004 | 11 | 9715 | 71982515 | T/C |
| 645603 | 11 | 14755 | 71987555 | G/A |
| 661290 | 11 | 15912 | 71988712 | A/G |
| 679926 | 11 | 19834 | 71992634 | A/G |
| 567026 | 11 | 19850 | 71992650 | G/A |
| 678193 | 11 | 20171 | 71992971 | T/G |

(continued)

| dbSNP rs# | Chromosome | Position in Figure 4 | Chromosome Position | Allele Variants |
|---|---|---|---|---|
| 560777 | 11 | 20500 | 71993300 | C/T |
| 676721 | 11 | 20536 | 71993336 | C/T |
| 585228 | 11 | 23187 | 71995987 | C/G |
| 674319 | 11 | 25289 | 71998089 | C/T |
| 675185 | 11 | 25470 | 71998270 | T/G |
| 575871 | 11 | 28720 | 72001520 | A/G |
| 547208 | 11 | 29566 | 72002366 | C/T |
| 2511075 | 11 | 30155 | 72002955 | T/C |
| 642573 | 11 | 30752 | 72003552 | C/G |
| 671681 | 11 | 32710 | 72005510 | C/T |
| 541022 | 11 | 32954 | 72005754 | A/G |
| 2511076 | 11 | 33725 | 72006525 | G/A |
| 3018308 | 11 | 33842 | 72006642 | T/C |
| 671132 | 11 | 36345 | 72009145 | G/A |
| 552966 | 11 | 38115 | 72010915 | A/C |
| 607446 | 11 | 39150 | 72011950 | C/T |
| 3018302 | 11 | 40840 | 72013640 | T/G |
| 3018301 | 11 | 41969 | 72014769 | A/G |
| 2511114 | 11 | 42045 | 72014845 | C/T |
| 548961 | 11 | 43785 | 72016585 | G/A |
| 575831 | 11 | 44444 | 72017244 | A/G |
| 577435 | 11 | 44579 | 72017379 | T/C |
| 495567 | 11 | 45386 | 72018186 | C/T |
| 493065 | 11 | 46827 | 72019627 | A/G |
| 597513 | 11 | 47320 | 72020120 | A/T |
| 598835 | 11 | 47625 | 72020425 | T/C |
| 610004 | 11 | 47837 | 72020637 | T/C |
| 610041 | 11 | 47866 | 72020666 | A/G |
| 673478 | 11 | 49002 | 72021802 | T/C |
| 670802 | 11 | 49566 | 72022366 | T/G |
| 2511116 | 11 | 52058 | 72024858 | C/T |
| NUMA1_SNP1 | 11 | 52249 | 72025049 | A/C |
| 517837 | 11 | 52257 | 72025057 | C/T |
| 615000 | 11 | 52850 | 72025650 | T/G |
| 482013 | 11 | 53860 | 72026660 | C/T |
| NUMA1_SNP2 | 11 | 54052 | 72026852 | T/C |
| 2250866 | 11 | 54411 | 72027211 | T/C |
| 2511078 | 11 | 55098 | 72027898 | G/A |

(continued)

| dbSNP rs# | Chromosome | Position in Figure 4 | Chromosome Position | Allele Variants |
|---|---|---|---|---|
| 2508858 | 11 | 55303 | 72028103 | C/G |
| 681069 | 11 | 59398 | 72032198 | A/G |
| 595062 | 11 | 59533 | 72032333 | A/G |
| 542752 | 11 | 60542 | 72033342 | A/T |
| 2508856 | 11 | 61541 | 72034341 | C/T |
| 832658 | 11 | 62309 | 72035109 | G/A |
| 3750908 | 11 | 72299 | 72045099 | C/T |
| 3793938 | 11 | 73031 | 72045831 | C/T |
| 2276396 | 11 | 73803 | 72046603 | G/C |
| 1806778 | 11 | 80950 | 72053750 | T/C |
| 4073394 | 11 | 82137 | 72054937 | A/G |
| 471547 | 11 | 96077 | 72068877 | G/T |
| 606136 | 11 | 96470 | 72069270 | A/G |
| 532360 | 11 | 98116 | 72070916 | G/T |
| 703781 | 11 | 98184 | 72070984 | A/C |
| 476753 | 11 | 132952 | 72105752 | A/G |

Assay for Verifying and Allelotyping SNPs

The methods used to verify and allelotype the proximal SNPs of Table 27 are the same methods described in Examples 1 and 2 herein. The PCR primers and extend primers used in these assays are provided in Table 28 and Table 29, respectively.

**Table 28**

| dbSNP rs# | Forward PCR primer | Reverse PCR primer |
|---|---|---|
| 744293 | ACGTTGGATGTCTGCAGACAGTGGCCAATG | ACGTTGGATGAGGGCCCAGGATCACAATAG |
| 750789 | ACGTTGGATGTTCATCTGGTAAGTCCCACC | ACGTTGGATGTGAAACAAGAGAGGCCCTTC |
| 1939110 | ACGTTGGATGTCTTTAGGTCCAGGATTCCC | ACGTTGGATGTATAGTCAGCATCGTCCCTG |
| 2005192 | ACGTTGGATGCCCTCAGAGTTTGGACATAT | ACGTTGGATGTATCCAAAATGCAGACACAG |
| SNP0000 4859 | ACGTTGGATGGTGTTTATCCCAACCCTTCC | ACGTTGGATGGGAGGAAATACAGCCTGTTC |
| 744292 | ACGTTGGATGATCCTAGAGGACTGGGAAAG | ACGTTGGATGCTGCTTCTGTTCCCACAATG |
| 754490 | ACGTTGGATGAAGGGTGGAGAACTCATGGG | ACGTTGGATGACCCCTATTTTGAAGCAGGC |
| 872619 | ACGTTGGATGTTCACACCAAGGTGTTACTG | ACGTTGGATGCACAATAATGTGTTCAGGGC |
| 1807014 | ACGTTGGATGCTGGGCAACAAGAGTGAAAC | ACGTTGGATGGCCCAAAACCACTGAGATTC |
| 1815753 | ACGTTGGATGTAGAGTGAAGACAGAGCTCC | ACGTTGGATGATAAACCCAGGCATTCGAGC |
| 1892893 | ACGTTGGATGTCCTATGAAGATTCATCTGC | ACGTTGGATGGTCCAGAGTTTTAGACTCAAG |
| 1939111 | ACGTTGGATGTCCTTAACCTTATTGGTGGC | ACGTTGGATGGTTGGGTTCAGTAGAAGAGA |
| 1939112 | ACGTTGGATGAGCCACCAATAAGGTTAAGG | ACGTTGGATGTGTCTCTCACTTCCTCAACC |
| 1939113 | ACGTTGGATGAGACACACAAGGCAAGGTTC | ACGTTGGATGCCAGAGAGGAGTCTGTCTAG |

(continued)

| dbSNP rs# | Forward PCR primer | Reverse PCR primer |
|---|---|---|
| 1939114 | ACGTTGGATGGAAAACATTGGTCCAGGCAG | ACGTTGGATGCAAGAACCCAGGCATCAATG |
| 1939115 | ACGTTGGATGGACCACGGAATCCTTTTTTCA | ACGTTGGATGGCTCAAATTCTGTTCTTTAG |
| 1939116 | ACGTTGGATGACATAGGTAGTCAGGCACTC | ACGTTGGATGGCAGCTCTTTTTTTCCTACC |
| 1939117 | ACGTTGGATGGGGAACTTTTCACATTACAC | ACGTTGGATGGAGAGTTTGCATTTGGTGATC |
| 1939118 | ACGTTGGATGATGTTGCTGTATGGTCCTCC | ACGTTGGATGGAAAACATTGCGCTAGGCAC |
| 1954769 | ACGTTGGATGTGAGTGACCAAGTTGCTCTG | ACGTTGGATGTCTACCTTCATGATGTCCCC |
| 2000537 | ACGTTGGATGGGTCTTTTATGAGGTTTCTCC | ACGTTGGATGGTTAAACTTACAAATCTAGC |
| 2011913 | ACGTTGGATGGCTGAGTGTGGATTGCTCTG | ACGTTGGATGAGTAAACCAACACCCAGAAC |
| 2015747 | ACGTTGGATGTGAAGCAGGCTTTCCCAATG | ACGTTGGATGGGTAGTGAAGGGTGGAGAAC |
| 2105587 | ACGTTGGATGAAGAAATACCAGGCCGGGAG | ACGTTGGATGCTCAAGTATCCTCCCTTCTC |
| 2155081 | ACGTTGGATGAGGCAATGCTTCCATTGTTC | ACGTTGGATGTCATAGCATTTTACCCCTGG |
| 2186617 | ACGTTGGATGGCTACATATGGATCTTGGTC | ACGTTGGATGGACCAGCACTAACTCTAAAC |
| 2508423 | ACGTTGGATGCTCCTCTGTAAAACCAGGAC | ACGTTGGATGAGAAACTCTCCTAAGCACAC |
| 2511880 | ACGTTGGATGGTTCCCTGATGGAAAATGCC | ACGTTGGATGCCAGAATGCCTTATCCACAG |
| 2511881 | ACGTTGGATGTGACTCTGCTGTGAGATTGG | ACGTTGGATGACATCGGTTTCACCTCCAAC |
| 2512990 | ACGTTGGATGAGCCAGCAGAGAAAACAGTC | ACGTTGGATGGCCACTTACTACCTGTTGTC |
| 2555537 | ACGTTGGATGGGACATAACCATAGGCCATC | ACGTTGGATGCATTGACAGCTGTATTGCAC |
| 3016250 | ACGTTGGATGTTTTTGAGACGGAGTCTCGC | ACGTTGGATGAGGCAGGAGAATGGCGTGAA |
| 3016251 | ACGTTGGATGAGCTTGCAGTGAGCCGAGAT | ACGTTGGATGTTTTTGAGACGGAGTCTCGC |
| 3016252 | ACGTTGGATGTGGTGAAGAGAAGTCAAAGC | ACGTTGGATGAGGCTGAATGATTCCCCTTC |
| 3781614 | ACGTTGGATGTGGTCAGTCAGTTAGCCAGG | ACGTTGGATGCCCTAATGATGGTAGACTGC |
| 3809048 | ACGTTGGATGACCACCAAGATAACGACCGC | ACGTTGGATGAGCCACCTCCTTGTCCAGTG |
| 4128368 | ACGTTGGATGGGACAATATTTAGTTATGCAC | ACGTTGGATGTTCAAGGTCATCCCGTTATC |

**Table 29**

| dbSNP rs# | Extend Primer | Term Mix |
|---|---|---|
| 744293 | GATGGCCCAGTTCCCTGCC | ACG |
| 750789 | AGAGGCCCTTCCAGGGCT | ACT |
| 1939110 | CGTCCCTGACCTGGACTTA | ACG |
| 2005192 | AATGCAGACACAGTTCTGGG | CGT |
| SNP00004859 | CTGAAAAATAGCTAGTTC | ACG |
| 744292 | ACTCACCTCTACCCATAAGG | ACT |
| 754490 | TTGAAGCAGGCTTTCCCA | ACT |
| 872619 | TGTGTTCAGGGCTTTCTCAT | ACT |
| 1807014 | GTGTTTTTTTTTTCCCCC | ACG |
| 1815753 | CAGGCATTCGAGCCAGCAAT | ACT |

(continued)

| dbSNP rs# | Extend Primer | Term Mix |
|---|---|---|
| 1892893 | ATGTTTTATTCTTTCACAAAAGT | ACT |
| 1939111 | GGAGGAGGCAGTAAGGAA | ACT |
| 1939112 | CTTCCAACTTTTTTCTCTTG | ACT |
| 1939113 | GTCTAGTCCTCCAAGCC | ACG |
| 1939114 | ATCAATGGGGTGGTGCA | ACT |
| 1939115 | TCTGTTCTTTAGAAGGCT | CGT |
| 1939116 | TGTACCAATATGACAATTTAACC | ACT |
| 1939117 | CCTGACACATAGTTCATGCTC | ACT |
| 1939118 | GCTAGGCACAAAATTAAAGAGAT | ACT |
| 1954769 | TCCCCGCCTTTCCCTCC | CGT |
| 2000537 | ACAAATCTAGCACCGAAGG | ACT |
| 2011913 | ATATAAGCAATTCACAAGTAATGT | ACT |
| 2015747 | AAGGGTGGAGAACTCATGG | ACT |
| 2105587 | TATCCTCCCTTCTCAGCAAG | ACT |
| 2155081 | CATTTTACCCCTGGATTATA | ACT |
| 2186617 | CTCAACCTCAACTCAACT | CGT |
| 2508423 | TCTCCTAAGCACACTATGTATAT | ACG |
| 2511880 | AGGATATTAGTCATGCTGGG | ACT |
| 2511881 | CACCTCCAACACGGTCCCC | CGT |
| 2512990 | GTTGTCTTCCCAACTCC | ACT |
| 2555537 | ACTGTGGACATTGGTGT | ACT |
| 3016250 | GGCGTGAACCCGGGAGG | ACG |
| 3016251 | CTGTCGCCCAGGCCGGA | ACT |
| 3016252 | GATTCCCCTTCTTCTAAA | ACT |
| 3781614 | TAGACTGCAGAGTAGCA | ACT |
| 3809048 | TGGGCCTACTTCCCTGA | ACT |
| 4128368 | TTTTCATCACATAGCTCATCT | CGT |

Genetic Analysis of Allelotyping Results

Allelotyping results are shown for cases and controls in Table 30. The allele frequency for the A2 allele is noted in the fifth and sixth columns for breast cancer pools and control pools, respectively, where "AF" is allele frequency. The allele frequency for the A1 allele can be easily calculated by subtracting the A2 allele frequency from 1 (A1 AF = 1-A2 AF). For example, the SNP rs1894003 has the following case and control allele frequencies: case A1 (T) = 0.192; case A2 (C) = 0.808; control A1 (T) = 0.115; and control A2 (C) = 0.885, where the nucleotide is provided in paranthesis. SNPs with blank allele frequencies were untyped.

**Table 30**

| dbSNP rs# | Position in Figure 4 | Chromosome Position | A1/A2 Allele | A2 Case AF | A2 Control AF | p-Value |
|---|---|---|---|---|---|---|
| 1894003 | 174 | 71972974 | T/C | 0.808 | 0.885 | 0.00061 |

(continued)

| dbSNP rs# | Position in Figure 4 | Chromosome Position | A1/A2 Allele | A2 Case AF | A2 Control AF | p-Value |
|---|---|---|---|---|---|---|
| 2390981 | 815 | 71973615 | G/A | 0.013 | 0.002 | 0.02306 |
| 1939242 | 3480 | 71976280 | C/T | 0.902 | 0.943 | 0.01186 |
| 1894004 | 9715 | 71982515 | T/C | 0.020 | 0.009 | 0.12637 |
| 645603 | 14755 | 71987555 | G/A | 0.029 | 0.021 | 0.37479 |
| 661290 | 15912 | 71988712 | A/G | 0.813 | 0.833 | 0.39013 |
| 679926 | 19834 | 71992634 | A/G | 0.077 | 0.039 | 0.00741 |
| 567026 | 19850 | 71992650 | G/A | 0.059 | 0.038 | 0.09767 |
| 678193 | 20171 | 71992971 | T/G | 0.868 | 0.920 | 0.00597 |
| 560777 | 20500 | 71993300 | C/T | 0.070 | 0.041 | 0.03071 |
| 676721 | 20536 | 71993336 | C/T | 0.901 | 0.947 | 0.00419 |
| 585228 | 23187 | 71995987 | C/G | 0.842 | 0.914 | 0.00043 |
| 674319 | 25289 | 71998089 | C/T | 0.027 | 0.027 | 0.96556 |
| 675185 | 25470 | 71998270 | T/G | 0.763 | 0.853 | 0.00031 |
| 575871 | 28720 | 72001520 | A/G | 0.924 | 0.932 | 0.61199 |
| 547208 | 29566 | 72002366 | C/T | 0.042 | 0.023 | 0.07555 |
| 2511075 | 30155 | 72002955 | T/C | 0.894 | 0.944 | 0.00256 |
| 642573 | 30752 | 72003552 | C/G | 0.047 | 0.022 | 0.02382 |
| 671681 | 32710 | 72005510 | C/T | 0.072 | 0.043 | 0.03643 |
| 541022 | 32954 | 72005754 | A/G | 0.070 | 0.040 | 0.02829 |
| 2511076 | 33725 | 72006525 | G/A | 0.223 | 0.256 | 0.20380 |
| 3018308 | 33842 | 72006642 | T/C | 0.442 | 0.439 | 0.92279 |
| 671132 | 36345 | 72009145 | G/A | 0.970 | 0.971 | 0.96469 |
| 552966 | 38115 | 72010915 | A/C | 0.845 | 0.903 | 0.00393 |
| 607446 | 39150 | 72011950 | C/T | 0.861 | 0.918 | 0.00279 |
| 3018302 | 40840 | 72013640 | T/G | 0.767 | 0.827 | 0.01378 |
| 3018301 | 41969 | 72014769 | A/G | 0.734 | 0.837 | 0.00011 |
| 2511114 | 42045 | 72014845 | C/T | 0.080 | 0.036 | 0.00222 |
| 548961 | 43785 | 72016585 | G/A | 0.852 | 0.905 | 0.00833 |
| 575831 | 44444 | 72017244 | A/G | 0.946 | 0.961 | 0.22995 |
| 577435 | 44579 | 72017379 | T/C | 0.013 | 0.007 | 0.34863 |
| 495567 | 45386 | 72018186 | C/T | 0.891 | 0.951 | 0.00045 |
| 493065 | 46827 | 72019627 | A/G | 0.823 | 0.904 | 0.00022 |
| 597513 | 47320 | 72020120 | A/T | 0.890 | 0.936 | 0.00667 |
| 598835 | 47625 | 72020425 | T/C | 0.074 | 0.038 | 0.00994 |
| 610004 | 47837 | 72020637 | T/C | 0.088 | 0.041 | 0.00209 |
| 610041 | 47866 | 72020666 | A/G | 0.872 | 0.933 | 0.00102 |
| 673478 | 49002 | 72021802 | T/C | 0.173 | 0.094 | 0.00026 |

(continued)

| dbSNP rs# | Position in Figure 4 | Chromosome Position | A1/A2 Allele | A2 Case AF | A2 Control AF | p-Value |
|---|---|---|---|---|---|---|
| 670802 | 49566 | 72022366 | T/G | 0.876 | 0.920 | 0.01646 |
| 2511116 | 52058 | 72024858 | C/T | 0.898 | 0.945 | 0.00437 |
| NUMA1_SNP1 | 52249 | 72025049 | A/C | 0.901 | 0.924 | 0.17421 |
| 517837 | 52257 | 72025057 | C/T | 0.095 | 0.061 | 0.03504 |
| 615000 | 52850 | 72025650 | T/G | 0.812 | 0.916 | 0.00001 |
| 482013 | 53860 | 72026660 | C/T | 0.884 | 0.924 | 0.02391 |
| NUMA1_SNP2 | 54052 | 72026852 | T/C | 0.066 | 0.034 | 0.01392 |
| 2250866 | 54411 | 72027211 | T/C | 0.855 | 0.918 | 0.00132 |
| 2511078 | 55098 | 72027898 | G/A | 0.299 | 0.295 | 0.86946 |
| 2508858 | 55303 | 72028103 | C/G | 0.898 | 0.944 | 0.00509 |
| 681069 | 59398 | 72032198 | A/G | 0.835 | 0.878 | 0.04069 |
| 595062 | 59533 | 72032333 | A/G | 0.925 | 0.942 | 0.25198 |
| 542752 | 60542 | 72033342 | A/T | 0.853 | 0.915 | 0.00192 |
| 2508856 | 61541 | 72034341 | C/T | 0.074 | 0.060 | 0.33745 |
| 832658 | 62309 | 72035109 | G/A | 0.047 | 0.023 | 0.02994 |
| 3750908 | 72299 | 72045099 | C/T | 0.912 | 0.944 | 0.04342 |
| 3793938 | 73031 | 72045831 | C/T | 0.084 | 0.045 | 0.00763 |
| 2276396 | 73803 | 72046603 | G/C | 0.892 | 0.937 | 0.00799 |
| 1806778 | 80950 | 72053750 | T/C | 0.041 | 0.034 | 0.50886 |
| 4073394 | 82137 | 72054937 | A/G | 0.547 | 0.579 | 0.28705 |
| 471547 | 96077 | 72068877 | G/T | 0.490 | 0.522 | 0.28304 |
| 606136 | 96470 | 72069270 | A/G | 0.444 | 0.468 | 0.43474 |
| 532360 | 98116 | 72070916 | G/T | 0.043 | 0.021 | 0.03475 |
| 703781 | 98184 | 72070984 | A/C | 0.078 | 0.080 | 0.89053 |
| 476753 | 132952 | 72105752 | A/G | 0.922 | 0.936 | 0.39563 |

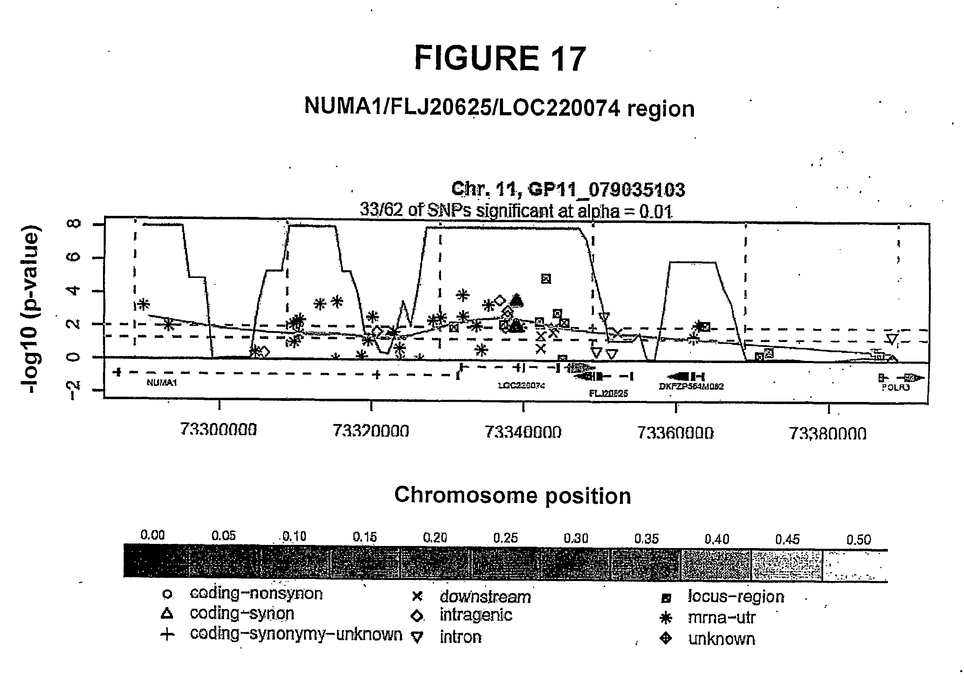

Figure 17 shows the proximal SNPs in and around the *NUMA1* region for females. The position of each SNP on the chromosome is presented on the x-axis. The y-axis gives the negative logarithm (base 10) of the p-value comparing the estimated allele in the case group to that of the control group. The minor allele frequency of the control group for each SNP designated by an X or other symbol on the graphs in Figure 17 can be determined by consulting Table 30. By proceeding down the Table from top to bottom and across the graphs from left to right the allele frequency associated with each symbol shown can be determined.

To aid the interpretation, multiple lines have been added to the graph. The broken horizontal lines are drawn at two common significance levels, 0.05 and 0.01. The vertical broken lines are drawn every 20kb to assist in the interpretation of distances between SNPs. Two other lines are drawn to expose linear trends in the association of SNPs to the disease. The light gray line (or generally bottom most curve) is a nonlinear smoother through the data points on the graph using a local polynomial regression method (W.S. Cleveland, E. Grosse and W.M. Shyu (1992) Local regression models. Chapter 8 of Statistical Models in S eds J.M. Chambers and T.J. Hastie, Wadsworth & Brooks/Cole.). The black line (or generally top-most curve, *e.g.*, see peak in left-most graph just to the left of position 92150000) provides a local test for excess statistical significance to identify regions of association. This was created by use of a 10kb sliding window with 1kb step sizes. Within each window, a chi-square goodness of fit test was applied to compare the proportion of SNPs that were significant at a test wise level of 0.01, to the proportion that would be expected by chance alone (0.05 for the methods used here). Resulting p-values that were less than $10^{-8}$ were truncated at that value.

Finally, the gene or genes present in the loci region of the proximal SNPs as annotated by Locus Link (http address: www.ncbi.nlm.nih.gov/LocusLink/) are provided on the graph. The exons and introns of the genes in the covered region are plotted below each graph at the appropriate chromosomal positions. The gene boundary is indicated by the broken horizontal line. The exon positions are shown as thick, unbroken bars. An arrow is place at the 3' end of each gene to show the direction of transcription.

Example 8

Meta Analysis of Incident SNPs

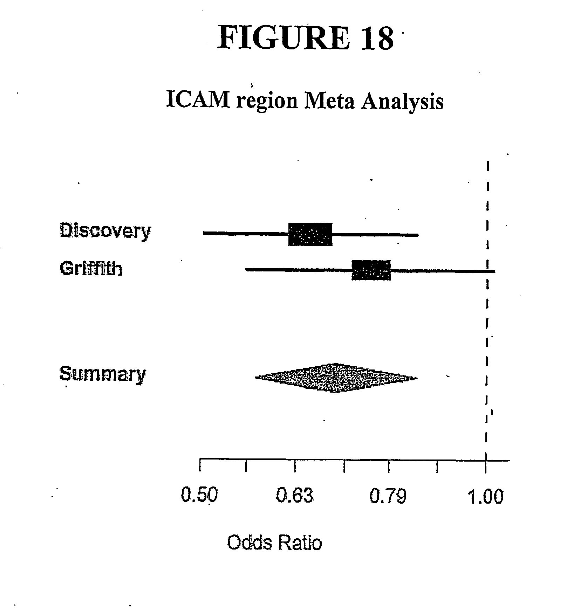

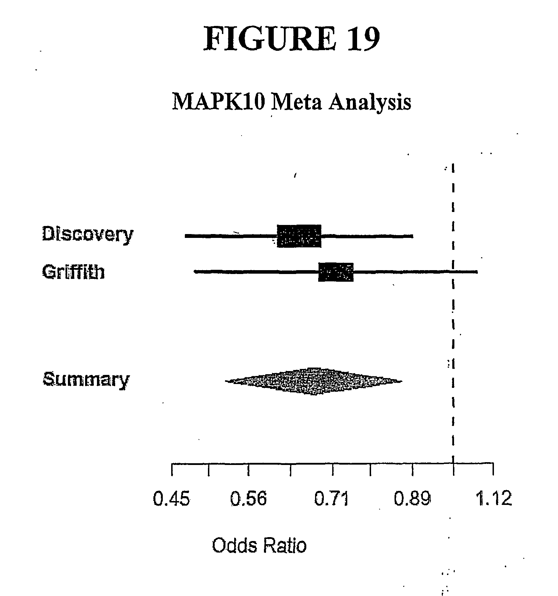

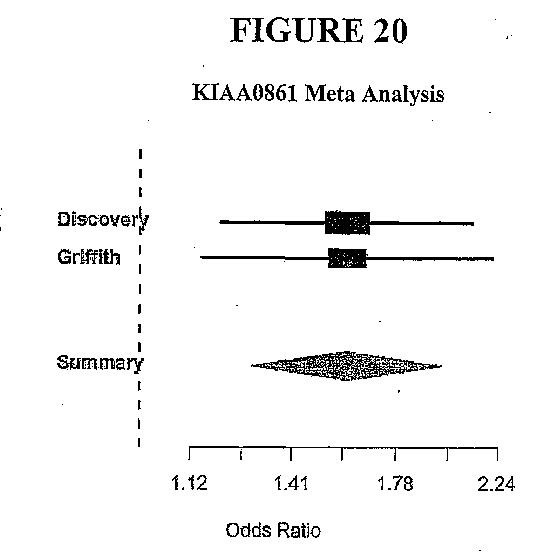

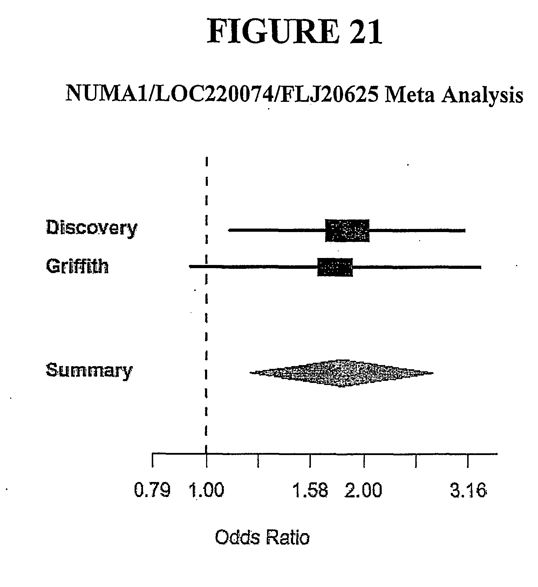

Meta-analysis was performed of five of the incident SNPs disclosed in Table 3 (ICAM region (ICAM_SNP), MAPK10 (rs1541998), KIAA0861 (rs2001449), NUMA1 region (rs673478) and GALE region (rs4237)) based on genotype results provided in Table 6B. Figures 18-21 depict odds ratios for the discovery samples and replication samples (see Example 3) individually, and the combined meta analysis odds ratio for the named SNP. The boxes are centered over the odds ratio for each sample, with the size of the box correlated to the contribution of each sample to the combined meta analysis odds ratio. The lines extending from each box are the 95% confidence interval values. The diamond is centered over the combined meta analysis odds ratio with the ends of the diamond depicting the 95% confidence interval values. The meta-analysis further illustrates the strong association each of the incident SNPs has with breast cancer across multiple case and control samples.

The subjects available for discovery from Germany included 272 cases and 276 controls. The subjects available for replication from Australia included 190 breast cancer cases and 190 controls. Meta analyses, combining the results of the German discovery sample and the Australian replication sample, were carried out using a random effects (Der-Simonian-Laird) procedure.

Example 9

Description of development of predictive breast cancer models

The five SNPs reported in Example 3 were identified as being significantly associated with breast cancer according to the replication analysis discussed therein. These five SNPs are a subset of the panel of SNPs associated with breast cancer in the German chort referenced in Example 1 and reported in provisional patent application no. 60/429,136 filed November 25, 2002 and provisional patent application no. 60/490,234 filed July 24, 2003, having attorney docket number 524593004100 and 524593004101, respectively.

The clinical importance of these SNPs was estimated by combining them into a single logistic regression model. The coefficients of the model were used to estimate penetrance, relative risk and odds ratio values for estimating a subject's risk of having or developing breast cancer according to the subject's genotype. Penetrance is a probability that an individual has or will have breast cancer given their genotype (e.g., a value of 0.01 in the tables is equal to a 1% chance of having or developing breast cancer). The relative risk of breast cancer is based upon penetrance values, and is expressed in two forms. One form, noted as RR in the tables below, is expressed as a risk with respect to the lowest risk group (e.g., the most protected group being the 00000 genotype listed in Table 33). The other form is expressed as a risk with respect to a population average risk of breast cancer, which is noted as RR(Pop) in Table 35 below. Both of these expressions of relative risk are useful to a clinician for assessing risk of breast cancer in an individual and targeting appropriate detection, prevention and/or treatment regimens to the subject. Both expressions of relative risk also are useful to an insurance company to assess population risks of breast cancer (e.g., for developing actuarial tables), where individual genotypes often are provided to the company on an anonymous basis. Odds ratios are the odds one group has or will develop breast cancer with respect to another group, the other group often being the most protective group or the group having a population average risk of breast cancer. Relative risk often is a more reliable assessment of risk in comparison to an odds ratio when the disease or condition at issue is more prevalent.

To fit the single logistic model, all cases and controls from the German and Australian samples were used (see Examples 1 and 3, respectively). Controls were coded as 0 and cases were coded as 1. Based on the genotype penetrance estimates of each SNP (Table 31), GP01.025495354 (rs4237), GP03.197942797 (rs2001449), GP11.079035103 (rs673478) were modeled as additive by coding the genotypes 0, 1, or 2 for the low risk homozygote, the heterozygote, or high risk homozygote, respectively. The SNP FCH.0994 (ICAM_SNP1) was modeled as recessive coding the genotypes 0, 0, or 2 for the low risk homozygote, heterozygote, or high risk homozygote, respectively. The SNP GP04.091348915 (rs1541998) was modeled as dominant coding the genotypes 0, 2, or 2 for the low risk homozygote, the heterozygote, or high risk homozygote, respectively. Table 31 summarizes this analysis.

**Table 31**

| SNP: Genotype | N | Case (N=254) | Control (N=268) | P(D\|G) (%) | P-value |
|---|---|---|---|---|---|
| ICAM_SNP1: CC CT TT | 497 | 45%(103) 42%(98) 13% (30) | 32% (85) 47% (126) 21% (13) | 4.140 2.700 1.910 | 0.006210 |
| rs4237: AA AG GG | 494 | 34% (79) 49% (113) 17% (40) | 29% (75) 48% (126) 23% (61) | 3.550 3.040 2.240 | 0.186000 |
| rs2001449: GG GC CC | 508 | 46% (112) 48% (117) 7% (17) | 60% (158) 36% (94) 4% (10) | 2.280 3.940 5.300 | 0.002930 |
| rs673478: TT TC CC | 509 | 84% (206) 14% (35) 1% (3) | 91% (240) 9% (25) 0% (0) | 2.800 4.490 100.00 | 0.040700 |
| rs1541998: CC CT TT | 493 | 5% (12) 36% (87) 59% (143) | 4% (10) 24% (61) 72% (180) | 3.710 4.370 2.490 | 0.012100 |

Based on this coding, there are a total of 108 unique genotype codes from the 243 unique five SNP genotypes. The relationship between the five SNP genotypes and the case-control status was fit using logistic regression. Many models were fit and compared including the five SNPs and all possible interaction among SNPs and study center. Only statistically significant terms from this complete model were included in the final model, shown in Table 32.

**Table 32**

| | Estimate | Std. Error | z value | Pr(>\|z\|) |
|---|---|---|---|---|
| (Intercept) | -1.34446 | 0.25972 | -5.177 | 2.26e-07 |
| FCH.0994 | 0.77607 | 0.19835 | 3.913 | 9.13e-05 |
| 4237 | 0.54525 | 0.17666 | 3.086 | 0.002025 |
| 2001449 | 0.60383 | 0.28487 | 2.120 | 0.034033 |
| 1541998 | 0.22051 | 0.07849 | 2.809 | 0.004963 |
| 673478 | 0.59961 | 0.21737 | 2.758 | 0.005807 |
| FCH.0994c:4237 | -0.52636 | 0.14516 | -3.626 | 0.000288 |
| FCH.0994c:2001449 | -0.35613 | 0.24503 | -1.453 | 0.146113 |
| 4237c: 2001449 | -0.15685 | 0.20191 | -0.777 | 0.437257 |
| FCH.0994c: 4237c2001449 | 0.41305 | 0.18391 | 2.246 | 0.024705 |
| Null deviance: 1136.7 on 820 degrees ot freedom<br>Residual deviance: 1069.6 on 811 degrees of freedom | | | | |

AIC: 1089.6

The penetrance was calculated for each of the 108 unique genotype codes using this model and an assumed disease prevalence of 0.03 (prev), the cumulative incidence for the age range of the sample in question. This was calculated from the logistic model as follows:

$$\text{penetrance} = \exp(y + adj)/(1 + \exp(y + adj))$$

where

$$y = 1/(1 + \exp(-1.344 + 0.776*A + 0.545*B + 0.604*C + 0.221*D + 0.600*E - 0.526*A*B - 0.356*A*C - 0.157*B*C + 0.413*A*B*C))$$

and

$$adj = \ln(prev/(1 - prev) * freq(case)/(1 - freq(case)).$$

Here A, B, C, D, and E refer to the genotype codes for the SNPs FCH.0994, 4237, 2001449, 1541998, and 673478, respectively.

Table 33 summarizes statistics of interest for each genotype code. "Geno" shows each genotype code with the five integer codes formatted as an integer string. "N Case" and "N Control" is the number of cases and controls with the specified code, respectively. "Frequency" is the expected percent of individuals in the population having that code calculated as the average of the case and control frequencies weighted by the probability of disease in this sample (0.03). "OR" is the odds ratio comparing the odds of the specified code to the odds of the most protective code (00000) using the parameter estimates from the logistic regression model. "OR (Frq)" is an odds ratio estimated using the frequency of cases and control with the specified genotype code and the most protective code. "RR" is the relative risk comparing the probability of disease of the specified code to the probability of disease of the most protective code. "Penetrance" is the probability of disease given the genotype code, followed by "Lower" and "Upper" which give the 95% confidence interval for the penetrance. As can be seen by the ratios for OR and RR, the 00000 genotype was the most protective against breast cancer occurrence.

**Table 33**

| | | | | | | | | Confidence Interval | |
|---|---|---|---|---|---|---|---|---|---|
| **Geno** | **N Case** | **N Control** | **Frequency** | **OR** | **OR (Frq)** | **RR** | **Penetrance** | **Lower** | **Upper** |
| 00000 | 6 | 26 | 5.94% | 1.00 | 1.00 | 1.00 | 0.010 | 0.007 | 0.014 |
| 00001 | 0 | 3 | 0.68% | 1.75 | 0.00 | 1.74 | 0.017 | 0.011 | 0.029 |
| 00002 | 0 | 0 | 0.00% | 3.08 | | 3.01 | 0.030 | 0.013 | 0.069 |
| 00020 | 3 | 9 | 2.06% | 1.61 | 1.44 | 1.60 | 0.016 | 0.011 | 0.023 |
| 00021 | 0 | 3 | 0.68% | 2.83 | 0.00 | 2.78 | 0.028 | 0.017 | 0.047 |
| 00022 | 0 | 0 | 0.00% | 4.97 | | 4.78 | 0.048 | 0.021 | 0.108 |
| 00100 | 9 | 20 | 4.60% | 1.67 | 1.95 | 1.66 | 0.017 | 0.012 | 0.023 |
| 00101 | 2 | 1 | 0.24% | 2.93 | 8.67 | 2.87 | 0.029 | 0.018 | 0.047 |
| 00102 | 0 | 0 | 0.00% | 5.13 | | 4.93 | 0.050 | 0.022 | 0.110 |
| 00120 | 7 | 6 | 1.41% | 2.69 | 5.06 | 2.65 | 0.027 | 0.018 | 0.038 |
| 00121 | 0 | 0 | 0.00% | 4.73 | | 4.56 | 0.046 | 0.028 | 0.075 |
| 00122 | 0 | 0 | 0.00% | 8.29 | | 7.72 | 0.078 | 0.034 | 0.168 |
| 00200 | 1 | 4 | 0.91% | 2.78 | 1.08 | 2.74 | 0.027 | 0.018 | 0.042 |
| 00201 | 0 | 0 | 0.00% | 4.88 | | 4.70 | 0.047 | 0.027 | 0.082 |

(continued)

| | | | | | | | | Confidence Interval | |
|---|---|---|---|---|---|---|---|---|---|
| Geno | N Case | N Control | Frequency | OR | OR (Frq) | RR | Penetrance | Lower | Upper |
| 00202 | 0 | 0 | 0.00% | 8.57 | | 7.96 | 0.080 | 0.034 | 0.178 |
| 00220 | 1 | 1 | 0.23% | 4.50 | 4.33 | 4.34 | 0.044 | 0.027 | 0.070 |
| 00221 | 1 | 0 | 0.01% | 7.89 | | 7.38 | 0.074 | 0.041 | 0.129 |
| 00222 | 0 | 0 | 0.00% | 13.83 | | 12.25 | 0.123 | 0.052 | 0.263 |
| 01000 | 24 | 47 | 10.84% | 1.26 | 2.21 | 1.26 | 0.013 | 0.010 | 0.016 |
| 01001 | 3 | 1 | 0.25% | 2.21 | 13.00 | 2.18 | 0.022 | 0.014 | 0.034 |
| 01002 | 0 | 0 | 0.00% | 3.87 | | 3.77 | 0.038 | 0.017 | 0.083 |
| 01020 | 18 | 22 | 5.12% | 2.03 | 3.55 | 2.01 | 0.020 | 0.015 | 0.027 |
| 01021 | 4 | 4 | 0.94% | 3.57 | 4.33 | 3.48 | 0.035 | 0.022 | 0.055 |
| 01022 | 0 | 0 | 0.00% | 6.26 | | 5.94 | 0.060 | 0.027 | 0.129 |
| 01100 | 21 | 33 | 7.64% | 2.10 | 2.76 | 2.08 | 0.021 | 0.017 | 0.026 |
| 01101 | 2 | 4 | 0.92% | 3.69 | 2.17 | 3.59 | 0.036 | 0.024 | 0.055 |
| 01102 | 0 | 0 | 0.00% | 6.47 | | 6.13 | 0.062 | 0.028 | 0.130 |
| 01120 | 15 | 6 | 1.47% | 3.39 | 10.83 | 3.31 | 0.033 | 0.025 | 0.045 |
| 01121 | 0 | 0 | 0.00% | 5.95 | | 5.67 | 0.057 | 0.036 | 0.089 |
| 01122 | 0 | 0 | 0.00% | 10.44 | | 9.54 | 0.096 | 0.044 | 0.198 |
| 01200 | 5 | 4 | 0.94% | 3.51 | 5.42 | 3.42 | 0.034 | 0.023 | 0.050 |
| 01201 | 0 | 1 | 0.23% | 6.15 | 0.00 | 5.85 | 0.059 | 0.035 | 0.097 |
| 01202 | 0 | 0 | 0.00% | 10.79 | | 9.82 | 0.099 | 0.044 | 0.209 |
| 01220 | 1 | 0 | 0.01% | 5.66 | | 5.41 | 0.054 | 0.035 | 0.083 |
| 01221 | 0 | 0 | 0.00% | 9.93 | | 9.12 | 0.092 | 0.054 | 0.152 |
| 01222 | 0 | 0 | 0.00% | 17.42 | | 14.95 | 0.150 | 0.067 | 0.304 |
| 02000 | 22 | 39 | 9.01% | 1.59 | 2.44 | 1.58 | 0.016 | 0.012 | 0.021 |
| 02001 | 2 | 1 | 0.24% | 2.78 | 8.67 | 2.73 | 0.027 | 0.017 | 0.043 |
| 02002 | 1 | 0 | 0.01% | 4.88 | | 4.70 | 0.047 | 0.021 | 0.103 |
| 02020 | 16 | 10 | 2.39% | 2.56 | 6.93 | 2.52 | 0.025 | 0.018 | 0.035 |
| 02021 | 2 | 2 | 0.47% | 4.49 | 4.33 | 4.34 | 0.044 | 0.027 | 0.070 |
| 02022 | 2 | 0 | 0.02% | 7.88 | | 7.37 | 0.074 | 0.033 | 0.158 |
| 02100 | 21 | 18 | 4.24% | 2.65 | 5.06 | 2.60 | 0.026 | 0.020 | 0.035 |
| 02101 | 5 | 3 | 0.72% | 4.64 | 7.22 | 4.48 | 0.045 | 0.029 | 0.070 |
| 02102 | 0 | 0 | 0.00% | 8.14 | | 7.60 | 0.076 | 0.035 | 0.160 |
| 02120 | 11 | 8 | 1.90% | 4.28 | 5.96 | 4.14 | 0.042 | 0.030 | 0.058 |
| 02121 | 1 | 0 | 0.01% | 7.50 | | 7.04 | 0.071 | 0.044 | 0.112 |
| 02122 | 0 | 0 | 0.00% | 13.15 | | 11.72 | 0.118 | 0.054 | 0.239 |
| 02200 | 4 | 4 | 0.94% | 4.42 | 4.33 | 4.27 | 0.043 | 0.028 | 0.065 |

(continued)

| | | | | | | | | Confidence Interval | |
|---|---|---|---|---|---|---|---|---|---|
| Geno | N Case | N Control | Frequency | OR | OR (Frq) | RR | Penetrance | Lower | Upper |
| 02201 | 3 | 1 | 0.25% | 7.75 | 13.00 | 7.26 | 0.073 | 0.043 | 0.121 |
| 02202 | 0 | 0 | 0.00% | 13.59 | | 12.06 | 0.121 | 0.053 | 0.252 |
| 02220 | 2 | 1 | 0.24% | 7.13 | 8.67 | 6.72 | 0.068 | 0.043 | 0.106 |
| 02221 | 0 | 0 | 0.00% | 12.51 | | 11.21 | 0.113 | 0.065 | 0.189 |
| 02222 | 0 | 0 | 0.00% | 21.94 | | 18.13 | 0.182 | 0.082 | 0.358 |
| 20000 | 9 | 6 | 1.43% | 1.58 | 6.50 | 1.57 | 0.016 | 0.011 | 0.023 |
| 20001 | 0 | 0 | 0.00% | 2.76 | | 2.72 | 0.027 | 0.016 | 0.045 |
| 20002 | 0 | 0 | 0.00% | 4.85 | | 4.67 | 0.047 | 0.020 | 0.105 |
| 20020 | 8 | 4 | 0.97% | 2.54 | 8.67 | 2.51 | 0.025 | 0.017 | 0.037 |
| 20021 | 0 | 0 | 0.00% | 4.46 | | 4.31 | 0.043 | 0.026 | 0.072 |
| 20022 | 0 | 0 | 0.00% | 7.83 | | 7.33 | 0.074 | 0.032 | 0.161 |
| 20100 | 5 | 6 | 1.40% | 2.63 | 3.61 | 2.59 | 0.026 | 0.018 | 0.037 |
| 20101 | 4 | 1 | 0.26% | 4.61 | 17.33 | 4.45 | 0.045 | 0.027 | 0.072 |
| 20102 | 0 | 0 | 0.00% | 8.09 | | 7.55 | 0.076 | 0.033 | 0.163 |
| 20120 | 4 | 1 | 0.26% | 4.25 | 17.33 | 4.11 | 0.041 | 0.028 | 0.060 |
| 20121 | 1 | 0 | 0.01% | 7.45 | | 6.99 | 0.070 | 0.042 | 0.115 |
| 20122 | 0 | 0 | 0.00% | 13.06 | | 11.65 | 0.117 | 0.052 | 0.242 |
| 20200 | 0 | 1 | 0.23% | 4.39 | 0.00 | 4.24 | 0.043 | 0.027 | 0.066 |
| 20201 | 1 | 0 | 0.01% | 7.70 | | 7.21 | 0.072 | 0.041 | 0.124 |
| 20202 | 0 | 0 | 0.00% | 13.50 | | 11.99 | 0.121 | 0.052 | 0.255 |
| 20220 | 0 | 0 | 0.00% | 7.09 | | 6.68 | 0.067 | 0.041 | 0.108 |
| 20221 | 0 | 0 | 0.00% | 12.43 | | 11.15 | 0.112 | 0.063 | 0.192 |
| 20222 | 0 | 0 | 0.00% | 21.80 | | 18.03 | 0.181 | 0.080 | 0.361 |
| 21000 | 22 | 25 | 5.83% | 1.99 | 3.81 | 1.97 | 0.020 | 0.015 | 0.026 |
| 21001 | 3 | 4 | 0.93% | 3.48 | 3.25 | 3.40 | 0.034 | 0.022 | 0.053 |
| 21002 | 1 | 0 | 0.01% | 6.11 | | 5.81 | 0.058 | 0.026 | 0.125 |
| 21020 | 11 | 14 | 3.26% | 3.21 | 3.40 | 3.14 | 0.032 | 0.023 | 0.043 |
| 21021 | 1 | 2 | 0.46% | 5.62 | 2.17 | 5.37 | 0.054 | 0.034 | 0.085 |
| 21022 | 0 | 0 | 0.00% | 9.86 | | 9.05 | 0.091 | 0.041 | 0.190 |
| 21100 | 26 | 24 | 5.64% | 3.31 | 4.69 | 3.24 | 0.033 | 0.025 | 0.042 |
| 21101 | 1 | 2 | 0.46% | 5.81 | 2.17 | 5.54 | 0.056 | 0.036 | 0.085 |
| 21102 | 1 | 0 | 0.01% | 10.19 | | 9.33 | 0.094 | 0.043 | 0.191 |
| 21120 | 16 | 6 | 1.48% | 5.35 | 11.56 | 5.12 | 0.051 | 0.037 | 0.071 |
| 21121 | 4 | 0 | 0.03% | 9.38 | | 8.65 | 0.087 | 0.055 | 0.135 |
| 21122 | 0 | 0 | 0.00% | 16.45 | | 14.24 | 0.143 | 0.067 | 0.281 |

(continued)

| | | | | | | | | Confidence Interval | |
|---|---|---|---|---|---|---|---|---|---|
| Geno | N Case | N Control | Frequency | OR | OR (Frq) | RR | Penetrance | Lower | Upper |
| 21200 | 3 | 1 | 0.25% | 5.53 | 13.00 | 5.29 | 0.053 | 0.036 | 0.078 |
| 21201 | 3 | 0 | 0.02% | 9.69 | | 8.92 | 0.090 | 0.054 | 0.146 |
| 21202 | 0 | 0 | 0.00% | 17.00 | | 14.65 | 0.147 | 0.067 | 0.295 |
| 21220 | 2 | 2 | 0.47% | 8.93 | 4.33 | 8.27 | 0.083 | 0.053 | 0.127 |
| 21221 | 1 | 0 | 0.01% | 15.65 | | 13.65 | 0.137 | 0.081 | 0.223 |
| 21222 | 0 | 0 | 0.00% | 27.46 | | 21.69 | 0.218 | 0.101 | 0.409 |
| 22000 | 13 | 23 | 5.31% | 2.50 | 2.45 | 2.46 | 0.025 | 0.018 | 0.034 |
| 22001 | 4 | 1 | 0.26% | 4.39 | 17.33 | 4.24 | 0.043 | 0.027 | 0.068 |
| 22002 | 0 | 1 | 0.23% | 7.69 | 0.00 | 7.21 | 0.072 | 0.032 | 0.154 |
| 22020 | 3 | 10 | 2.29% | 4.04 | 1.30 | 3.92 | 0.039 | 0.027 | 0.056 |
| 22021 | 1 | 0 | 0.01% | 7.08 | | 6.67 | 0.067 | 0.041 | 0.107 |
| 22022 | 0 | 0 | 0.00% | 12.42 | | 11.14 | 0.112 | 0.051 | 0.230 |
| 22100 | 15 | 5 | 1.25% | 4.17 | 13.00 | 4.04 | 0.041 | 0.030 | 0.055 |
| 22101 | 1 | 0 | 0.01% | 7.32 | | 6.88 | 0.069 | 0.044 | 0.107 |
| 22102 | 0 | 0 | 0.00% | 12.83 | | 11.47 | 0.115 | 0.053 | 0.232 |
| 22120 | 3 | 5 | 1.16% | 6.74 | 2.60 | 6.37 | 0.064 | 0.045 | 0.091 |
| 22121 | 3 | 1 | 0.25% | 11.82 | 13.00 | 10.66 | 0.107 | 0.066 | 0.168 |
| 22122 | 0 | 0 | 0.00% | 20.72 | | 17.30 | 0.174 | 0.081 | 0.333 |
| 22200 | 4 | 0 | 0.03% | 6.96 | | 6.57 | 0.066 | 0.043 | 0.100 |
| 22201 | 0 | 0 | 0.00% | 12.21 | | 10.97 | 0.110 | 0.065 | 0.181 |
| 22202 | 0 | 0 | 0.00% | 21.42 | | 17.77 | 0.179 | 0.081 | 0.348 |
| 22220 | 4 | 1 | 0.26% | 11.24 | 17.33 | 10.19 | 0.102 | 0.064 | 0.160 |
| 22221 | 0 | 0 | 0.00% | 19.72 | | 16.60 | 0.167 | 0.097 | 0.271 |
| 22222 | 0 | 0 | 0.00% | 34.58 | | 25.86 | 0.260 | 0.122 | 0.470 |

To simplify the interpretation of genotype risk, the 243 unique genotypes were divided into five risk classes on the basis of each estimated penetrance. The levels selected for risk class definitions and the resulting assignment of genotypes into five risk classes is shown in Table 34. The frequency percent of each genotype combination is given in parentheses.

**Table 34**

| Class 1 (0, 0.013] | Class 2 (0.013, 0.025] | Class 3 (0.025, 0.042] | Class 4 (0.042, 0.1] | Class 5 (0.1,1) |
|---|---|---|---|---|
| 00000 (5.94) | 00001 (0.68) | 00022 (0.00) | 00102 ( 0.00) | 00222 (0.00) |
| 00020 (2.06) | 00002 (0.00) | 00121 (0.00) | 00122 ( 0.00) | 01222 (0.00) |
| 01000 (10.84) | 00021 (0.68) | 00220 (0.23) | 00201 (0.00) | 02022 (0.02) |
| 22000 (5.31) | 00100 (4.60) | 01002 (0.00) | 00202 ( 0.00) | 02122 (0.00) |
| | 00101 (0.24) | 01021 (0.94) | 00221 (0.01) | 02202 (0.00) |

(continued)

| Class 1 (0, 0.013] | Class 2 (0.013, 0.025] | Class 3 (0.025, 0.042] | Class 4 (0.042, 0.1] | Class 5 (0.1,1) |
|---|---|---|---|---|
| | 00120 (1.41) | 01101 (0.92) | 01022 (0.00) | 02221 (0.00) |
| | 00200 (0.91) | 01120 (1.47) | 01102 (0.00) | 02222 (0.00) |
| | 01001 (0.25) | 01200 (0.94) | 01121 (0.00) | 20002 (0.00) |
| | 01020 (5.12) | 02001 (0.24) | 01122 (0.00) | 20022 (0.00) |
| | 01100 (7.64) | 02020 (2.39) | 01201 (0.23) | 20122 (0.00) |
| | 02000 (9.01) | 02100 (4.24) | 01202 (0.00) | 20222 (0.00) |
| | 21000 ( 5.83) | 02200 (0.94) | 01220 (0.01) | 21102 (0.01) |
| | 22001 (0.26) | 20000 (1.43) | 01221 (0.00) | 21122 (0.00) |
| | 22020 (2.29) | 20100 (1.40) | 02002 (0.01) | 21201 (0.02) |
| | | 20200 (0.23) | 02021 (0.47) | 21202 (0.00) |
| | | 20220 (0.00) | 02101 (0.72) | 21221 (0.01) |
| | | 21001 (0.93) | 02102 ( 0.00) | 21222 (0.00) |
| | | 21020 (3.26) | 02120 (1.90) | 22102 (0.00) |
| | | 21100 (5.64) | 02121 (0.01) | 22121 (0.25) |
| | | 22002 (0.23) | 02201 (0.25) | 22122 (0.00) |
| | | 22021 (0.01) | 02220 (0.24) | 22200 (0.03) |
| | | 22100 (1.25) | 20001 (0.00) | 22201 (0.00) |
| | | | 20020 (0.97) | 22202 (0.00) |
| | | | 20021 (0.00) | 22220 (0.26) |
| | | | 20101 (0.26) | 22221 (0.00) |
| | | | 20102 (0.00) | 22222 (0.00) |
| | | | 20120 (0.26) | |
| | | | 20121 (0.25) | |
| | | | 20201 (0.01) | |
| | | | 20202 (0.00) | |
| | | | 20221 (0.00) | |
| | | | 21002 (0.01) | |
| | | | 21021 (0.46) | |
| | | | 21022 (0.00) | |
| | | | 21101 (0.46) | |
| | | | 21120 (1.48) | |
| | | | 21121 (0.03) | |
| | | | 21200 (0.25) | |
| | | | 21220 (0.47) | |
| | | | 22022 (0.00) | |
| | | | 22101 (0.00) | |
| | | | 22120 (1.16) | |

With this classification, each genotype was recoded as belonging to their respective class and a logistic regression model was fit with the genotype risk class as a categorical variable. Key summary statistics are summarized in Table 35. Each group is described by the number of cases, number of controls, the estimated risk class population frequency, the odds ratio comparing the odds of the given risk class compared to the odds of the lowest risk class, the penetrance, the relative risk (risk class penetrance divided by most protective risk class penetrance), and the population relative risk (risk class penetrance divided by the disease prevalence: 0.03).

**Table 35**

| Risk Class | N Case | N Control | Frequency (%) | OR | Penetrance | RR | RR (Pop) |
|---|---|---|---|---|---|---|---|
| G1 | 46 | 105 | 24.2 | 1.0 | 0.012 | 1.0 | 0.41 |
| G2 | 112 | 168 | 38.9 | 1.5 | 0.019 | 1.5 | 0.62 |
| G3 | 140 | 113 | 26.7 | 2.8 | 0.034 | 2.8 | 1.13 |
| G4 | 77 | 40 | 9.7 | 4.4 | 0.052 | 4.2 | 1.73 |
| G5 | 18 | 2 | 0.06 | 20.5 | 0.204 | 16.6 | 6.79 |

Example 10

Inhibition of ICAM Gene Expression by Transfection of Specific siRNAs

RNAi-based gene inhibition was selected as a rapid way to inhibit expression of ICAM1 in cultured cells. siRNA reagents were selectively designed to target the ICAM1 gene. Algorithms useful for designing siRNA molecules specific for ICAM1 gene are disclosed at the http address www.dharmacon.com. siRNA molecules up to 21 nucleotides in length were utilized.

Table 31 summarizes the features of the duplexes that were used in the assays to target ICAM1. A non-homologous siRNA reagent (siGL2 control) was used as a negative control, and a non-homologous siRNA reagent (siRNA_RAD21_1175 control) shown to inhibit the expression of RAD21 and subsequently inhibit cell proliferation was used as a positive control in all of the assays described herein.

**Table 36**

| siRNA | siRNA Target | Sequence Specificity | SEQ ID NO: |
|---|---|---|---|
| ICAM1_293 | ICAM1 | ACAACCGGAAGGUGUAUGA | |
| ICAM1_335 | ICAM1 | GCCAACCAAUGUGCUAUUC | |
| ICAM1_604 | ICAM1 | GAUCACCAUGGAGCCAAUU | |
| ICAM1_1409 | ICAM1 | CUGUCACUCGAGAUCUUGA | |
| siRNA_RAD21_1175 positive control | RAD21 | GAGUUGGAUAGCAAGACAA | |
| siGL2 negative control | GL2 | CGUACGCGGAAUACUUCGA | |

The siRNAs were transfected in cell lines MCF-7 and T-47D using Lipofectamine™ 2000 reagent from Invitrogen, Corp. 2.5 μg or 5.0 μg of siRNA was mixed with 6.25 μl or 12.5 μl lipofectamine, respectively, and the mixture was added to cells grown in 6-well plates. Their inhibitory effects on ICAM1 gene expression were confirmed by precision expression analysis by MassARRAY (quantitativeRT-PCR hME), which was performed on RNA prepared from the transfected cells. See Chunming & Cantor, PNAS 100(6):3059-3064 (2003). Cell viability was measured at 1, 2, 4 and 6 days post-transfection. Absorbance values were normalized relative to Day 1. RNA was extracted with Trizole reagent as recommended by the manufacturer (Invitrogen, Corp.) followed by cDNA synthesis using SuperSeript™ reverse transcriptase.

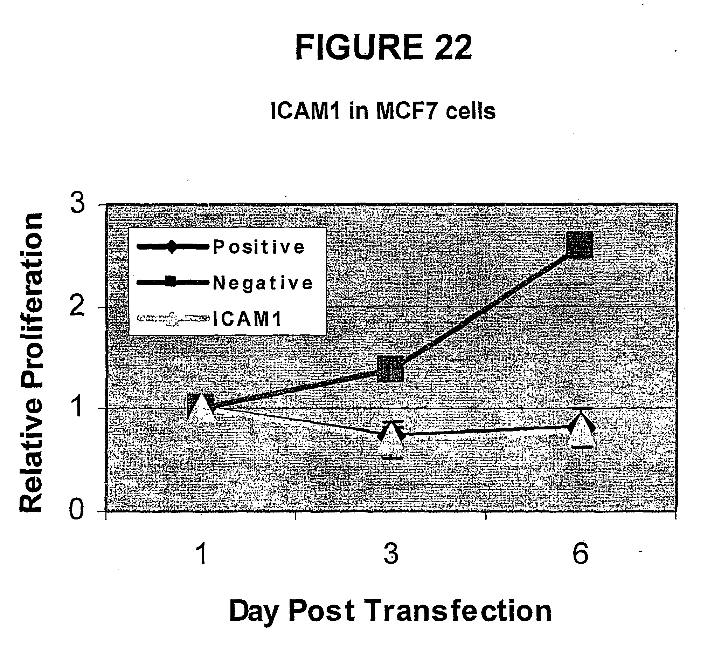

A cocktail of siRNA molecules described in Table 28 (that target ICAM1) strongly inhibited proliferation of breast cancer cell line (MCF-7), as shown in in Figure 22. These effects are consistent in all six experiments performed. Each data point is an average of 3 wells of a 96-well plate normalized to values obtained from day 1 post transfection. The specificity of the active siRNAs, was confirmed with a negative, non-homologous control siRNA (siGL2), and a positive control, siRNA_RAD21_1175, that targets a known cancer-associated gene, RAD21.

Example 11

*In Vitro* Production of Target Polypeptides

cDNA is cloned into a pIVEX 2.3-MCS vector (Roche Biochem) using a directional cloning method. A cDNA insert is prepared using PCR with forward and reverse primers having 5' restriction site tags (in frame) and 5-6 additional nucleotides in addition to 3' gene-specific portions, the latter of which is typically about twenty to about twenty-five base pairs in length. A Sal I restriction site is introduced by the forward primer and a Sma I restriction site is introduced by the reverse primer. The ends of PCR products are cut with the corresponding restriction enzymes (*i.e.*, Sal I and Sma I) and the products are gel-purified. The pIVEX 2.3-MCS vector is linearized using the same restriction enzymes, and the fragment with the correct sized fragment is isolated by gel-purification. Purified PCR product is ligated into the linearized pIVEX 2.3-MCS vector and *E. coli* cells transformed for plasmid amplification. The newly constructed expression vector is verified by restriction mapping and used for protein production.

*E. coli* lysate is reconstituted with 0.25 ml of Reconstitution Buffer, the Reaction Mix is reconstituted with 0.8 ml of Reconstitution Buffer; the Feeding Mix is reconstituted with 10.5 ml of Reconstitution Buffer; and the Energy Mix is reconstituted with 0.6 ml of Reconstitution Buffer. 0.5 ml of the Energy Mix was added to the Feeding Mix to obtain the Feeding Solution. 0.75 ml of Reaction Mix, 50 μl of Energy Mix, and 10 μg of the template DNA is added to the *E. coli* lysate.

Using the reaction device (Roche Biochem), 1 ml of the Reaction Solution is loaded into the reaction compartment. The reaction device is turned upside-down and 10 ml of the Feeding Solution is loaded into the feeding compartment. All lids are closed and the reaction device is loaded into the RTS500 instrument. The instrument is run at 30°C for 24 hours with a stir bar speed of 150 rpm. The pIVEX 2.3 MCS vector includes a nucleotide sequence that encodes six consecutive histidine amino acids on the C-terminal end of the target polypeptide for the purpose of protein purification. Target polypeptide is purified by contacting the contents of reaction device with resin modified with $Ni^{2+}$ ions. Target polypeptide is eluted from the resin with a solution containing free $Ni^{2+}$ ions.

Example 12

Cellular Production of Target Polypeptides

Nucleic acids are cloned into DNA plasmids having phage recombination cites and target polypeptides are expressed therefrom in a variety of host cells. Alpha phage genomic DNA contains short sequences known as attP sites, and *E. coli* genomic DNA contains unique, short sequences known as attB sites. These regions share homology, allowing for integration of phage DNA into *E. coli* via directional, site-specific recombination using the phage protein Int and the *E. coli* protein IHF. Integration produces two new att sites, L and R, which flank the inserted prophage DNA. Phage excision from *E. coli* genomic DNA can also be accomplished using these two proteins with the addition of a second phage protein, Xis. DNA vectors have been produced where the integration/excision process is modified to allow for the directional integration or excision of a target DNA fragment into a backbone vector in a rapid *in vitro* reaction (Gateway™ Technology (Invitrogen, Inc.)).

A first step is to transfer the nucleic acid insert into a shuttle vector that contains attL sites surrounding the negative selection gene, ccdB (*e.g.* pENTER vector, Invitrogen, Inc.). This transfer process is accomplished by digesting the nucleic acid from a DNA vector used for sequencing, and to ligate it into the multicloning site of the shuttle vector, which will place it between the two attL sites while removing the negative selection gene ccdB. A second method is to amplify the nucleic acid by the polymerase chain reaction (PCR) with primers containing attB sites. The amplified fragment then is integrated into the shuttle vector using Int and IHF. A third method is to utilize a topoisomerase- mediated process, in which the nucleic acid is amplified via PCR using gene-specific primers with the 5' upstream primer containing an additional CACC sequence (*e.g.*, TOPO® expression kit (Invitrogen, Inc.)). In conjunction with Topoisomerase I, the PCR amplified fragment can be cloned into the shuttle vector via the attL sites in the correct orientation.

Once the nucleic acid is transferred into the shuttle vector, it can be cloned into an expression vector having attR sites. Several vectors containing attR sites for expression of target polypeptide as a native polypeptide, N-fusion polypeptide, and C-fusion polypeptides are commercially available (*e.g.*, pDEST (Invitrogen, Inc.)), and any vector can be converted into an expression vector for receiving a nucleic acid from the shuttle vector by introducing an insert having an attR site flanked by an antibiotic resistant gene for selection using the standard methods described above. Transfer of the nucleic acid from the shuttle vector is accomplished by directional recombination using Int, IHF, and Xis (LR clonase). Then the desired sequence can be transferred to an expression vector by carrying out a one hour incubation at room temperature with Int, IHF, and Xis, a ten minute incubation at 37°C with proteinase K, transforming bacteria and allowing expression for one hour, and then plating on selective media. Generally, 90% cloning efficiency is achieved by this method. Examples of expression vectors are pDEST 14 bacterial expression vector with att7 promoter, pDEST 15 bacterial expression vector with a T7 promoter and a N-terminal GST tag, pDEST 17 bacterial vector with a T7 promoter and a N-terminal polyhistidine affinity tag, and pDEST 12.2 mammalian expression vector with a CMV promoter and neo resistance gene. These expression vectors or others like them are transformed or transfected into cells for expression of the target polypeptide or polypeptide variants. These expression vectors are often transfected, for example, into murine-transformed a adipocyte cell line 3T3-L1, (ATCC), human embryonic kidney cell line 293, and rat cardiomyocyte cell line H9C2.

Example 13

Haplotype analysis of the *KIAA0861* locus

rs6804951 and rs2001449 are significant at the allele and genotype levels ($P < 0.05$). Moderate LD is observed for markers rs3732602 and rs2293203 (r^2 = 0.646). Chi-squared tests indicate that haplotypes are significantly associated with breast cancer. Cell-specific chi-square values indicate that TTTTG and CTTTC haplotypes are contributors to this relationship. Odds ratios and score tests indicate that individuals carrying the TTTG are less likely to have breast cancer, while individuals with CTTTC are at elevated risk for the disease. Moreover, the odds ratio estimated for the CGTTC indicates more than a two-fold risk of disease among its carriers, although this result must be interpreted with great caution due to the low observed frequency in the population.

A. Summary Statistics of Alleles and Genotypes

1. SNP Locations

| SNP.ID | Type | Location |
|---|---|---|
| rs6804951 | Proximal | 184327431 |
| rs7639705 | Proximal | 184330963 |
| rs3732602 | Proximal | 184408945 |
| rs2293203 | Proximal | 184419992 |
| rs2001449 | Incident | 184429569 |

2. Allele by GYNGroup

| | N | Case (N=544) | Control (N=552) | Test Statistic |
|---|---|---|---|---|
| rs6804951 : T | 1064 | 5% (24) | 9% (46) | Chi-square=6.71 d.f.=1 P=0.009581 |
| rs7639705 : T | 1086 | 80% (434) | 81% (441) | Chi-square=0.03 d.f.=1 P=0.868 |
| rs3732602 : T | 1074 | 99% (532) | 99% (532) | Chi-square=0.4 d.f.=1 P=0.529 |
| rs2293203 : T | 1088 | 99% (536) | 99% (538) | Chi-square=0.27 d.f.=1 P=0.6 |
| rs2001449 : C\|1084 | | 30% (161) | 22% (119) Chi-square=8.49 d.f.=1 P=0.00356 | |

3. Genotype by GYNGroup

| N Case | | Control Test (N=272) | Test (N=276) | Statistic |
|---|---|---|---|---|
| rs6809951 : CC | 5321 | 91% (238) | 83% (225) | Chi-square=7.13 d.f.=2 P=0.02831 |

(continued)

| N Case | | Control Test (N=272) | | Test (N=276) | | Statistic |
|---|---|---|---|---|---|---|
| CT | | 9% | (24) | 16% | (44) | |
| TT | | 0% | (0) | 0% | (1) | |
| rs7639705 : GG | 541 | 3% | (9) | 5% | (14) | Chi-square=2.03 d.f.=2 P=0.362 |
| GT | | 33% | (88) | 28% | (77) | |
| TT | | 64% | (173) | 67% | (182) | |
| rs3732602 : TT | 531 | 99% | (264) | 98% | (263) | Chi-square=0.4 d.f. =1P=0.527 |
| rs2293203 : TT | 544 | 98% | (265) | 97% | (265) | Chi-square-0.28 d.f.=1 P=0.598 |
| rs2001449 : GG15421 | | 47% | (128) | 60% (162) Chi-square=9.29 d.f.=2 P=0.00961 | | |
| GC | | 46% | (125) | 37% | (99) | |
| CC | | 7% | (18) | 4% | (10) | |

4. Genotype QC: Test of Hardy-Weinberg Equilibrium

a. Cases

| | A.freq | D | ChiSq | Pvalue |
|---|---|---|---|---|
| rs6804951 | 0.936 | -0.002280 | 0.7870 | 0.3750 |
| rs7639705 | 0.807 | 0.004790 | 0.5150 | 0.4730 |
| rs3732602 | 0.990 | -0.000101 | 0.0565 | 0.8120 |
| rs2293203 | 0.987 | -0.000164 | 0.0921 | 0.7620 |
| rs2001449 | 0.744 | -0.014500 | 3.1400 | 0.0763 |

b. Controls

| | A.freq | D | ChiSq | Pvalue |
|---|---|---|---|---|
| rs6804951 | 0.916 | -0.003400 | 0.5350 | 0.465 |
| rs7639705 | 0.808 | 0.014400 | 2.3600 | 0.124 |
| rs3732602 | 0.989 | -0.000120 | 0.0336 | 0.855 |
| rs2293203 | 0.985 | -0.000213 | 0.0601 | 0.806 |
| rs2001449 | 0.783 | -0.010700 | 1.0800 | 0.299 |

B. Summary Statistics: Linkage Disequilibrium

1. PHASE Haplotype Frequencies

| | H.freq | H.relfreq |
|---|---|---|
| CGTTC | 13 | 0.012 |
| CGTTG | 191 | 0.175 |

(continued)

| | H.freq | H.relfreq |
|---|---|---|
| CTCAG | 10 | 0.009 |
| CTCTG | 1 | 0.001 |
| CTTAG | 4 | 0.004 |
| CTTTC | 265 | 0.243 |
| CTTTG | 538 | 0.493 |
| TGTTG | 7 | 0.006 |
| TTTTC | 2 | 0.002 |
| TTTTG | 61 | 0.056 |

2. Linkage Disequilibrium Between Markers

a. r^2

| | rs6804951 | rs7639705 | rs3732602 | rs2293203 | rs2001449 |
|---|---|---|---|---|---|
| rs6804951 | 1.000000 | 0.00382 | 0.000697 | 0.00089 | 0.01860 |
| rs7639705 | 0.003820 | 1.00000 | 0.002440 | 0.00311 | 0.04770 |
| rs3732602 | 0.000697 | 0.00244 | 1.000000 | 0.64600 | 0.00351 |
| rs2293203 | 0.000890 | 0.00311 | 0.646000 | 1.00000 | 0.00448 |
| rs2001449 | 0.018600 | 0.04770 | 0.003510 | 0.00448 | 1.00000 |

b. D'

| | rs6804951 | rs7639705 | rs3732602 | rs2293203 | rs2001449 |
|---|---|---|---|---|---|
| rs6804951 | 1.0000 | 0.116 | 0.0685 | 0.0685 | 0.306 |
| rs7639705 | 0.1160 | 1.000 | 0.2400 | 0.2400 | 0.262 |
| rs3732602 | 0.0685 | 0.240 | 1.0000 | 0.9080 | 0.345 |
| rs2293203 | 0.0685 | 0.240 | 0.9080 | 1.0000 | 0.345 |
| rs2001449 | 0.3060 | 0.262 | 0.3450 | 0.3450 | 1.000 |

c. P-value

| | rs6804951 | rs7639705 | rs3732602 | rs2293203 | rs2001449 |
|---|---|---|---|---|---|
| rs6804951 | 1.00e+00 | 4.12e-02 | 0.3830 | 0.3240 | 6.40e-06 |
| rs7639705 | 4.12e-02 | 1.00e+00 | 0.1030 | 0.0653 | 5.41e-13 |
| rs3732602 | 3.83e-01 | 1.03e-01 | 1.0000 | 0.0000 | 5.03e-02 |
| rs2293203 | 3.24e-01 | 6.53e-02 | 0.0000 | 1.0000 | 2.70e-02 |
| rs2001449 | 6.40e-06 | 5.41e-13 | 0.0503 | 0.0270 | 1.00e+00 |

3. Haplotype by GYNGroup

a. PHASE Haplotypes (All)

| | Case | Case(%) | Case.X^2 | Control | Control(%) | Control.X^2 | OR | ln.OR |
|---|---|---|---|---|---|---|---|---|
| TTTTG | 20 | 1.83 | 3.55 | 41 | 3.75 | 3.53 | 0.4782 | -0.7377 |
| CTCAG | 4 | 0.37 | 0.19 | 6 | 0.55 | 0.19 | 0.6654 | -0.4074 |
| TGTTG | 3 | 0.27 | 0.07 | 4 | 0.37 | 0.07 | 0.7493 | -0.2886 |
| CTTTG | 259 | 23.72 | 0.30 | 279 | 25.55 | 0.30 | 0.9060 | -0.0987 |
| CGTTG | 94 | 8.61 | 0.01 | 97 | 8.88 | 0.01 | 0.9662 | -0.0344 |
| CTTAG | 2 | 0.18 | 0.00 | 2 | 0.18 | 0.00 | 1.0000 | 0.0000 |
| TTTTC | 1 | 0.09 | 0.00 | 1 | 0.09 | 0.00 | 1.0000 | 0.0000 |
| CTTTC | 151 | 13.83 | 2.73 | 114 | 10.44 | 2.71 | 1.3766 | 0.3196 |
| CGTTC | 9 | 0.82 | 0.98 | 4 | 0.37 | 0.98 | 2.2604 | 0.8155 |
| CTCTG | 1 | 0.09 | 0.51 | 0 | 0.00 | 0.50 | Inf | Inf |

Pearson Chi-squared Test = 16.6377, DF = 9, P-value = 0.0547

b. PHASE Haplotypes (Low Frequency Removed)

| | Case | Case(%) | Case.X^2 | Control | Control(%) | Control.X^2 | OR | ln.OR |
|---|---|---|---|---|---|---|---|---|
| TTTTG | 20 | 1.86 | 3.55 | 41 | 3.80 | 3.52 | 0.4781 | -0.7379 |
| CTCAG | 4 | 0.37 | 0.19 | 6 | 0.56 | 0.19 | 0.6654 | -0.4074 |
| CTTTG | 259 | 24.03 | 0.30 | 279 | 25.88 | 0.30 | 0.9056 | -0.0992 |
| CGTTG | 94 | 8.72 | 0.01 | 97 | 9.00 | 0.01 | 0.9661 | -0.0345 |
| CTTTC | 151 | 14.01 | 2.73 | 114 | 10.58 | 2.71 | 1.3774 | 0.3202 |
| CGTTC | 9 | 0.83 | 0.98 | 4 | 0.37 | 0.98 | 2.2605 | 0.8156 |

Pearson Chi-squared Test = 15.4946, DF = 5, P-value = 0.008445

c. haplo.score Haplotypes

| | Hap.Freq | Score | P. X^2 | P.Sim |
|---|---|---|---|---|
| TTTTG | 0.0529 | -2.1206 | 0.0340 | 0.0342 |
| TGTTG | 0.0101 | -2.0668 | 0.0388 | 0.0236 |
| CTCAG | 0.0073 | -1.2914 | 0.1966 | 0.2902 |
| CTTTG | 0.5221 | -1.2275 | 0.2196 | 0.2195 |
| CGTTG | 0.1448 | -0.1441 | 0.8854 | 0.8834 |
| CTTTC | 0.2267 | 2.3422 | 0.0192 | 0.0192 |
| CGTTC | 0.0307 | 2.6994 | 0.0069 | 0.0050 |

Global Score = 20.343, DF = 7, Global P.X^2 = 0.0049, Global P.Sim = 0.0022

Example 14

Haplotype analysis of the *NUMA1* locus

All markers noted below except 2276396 are associated with breast cancer at the allele level (P < 0.05). Marker 675185 does not maintain this relationship at the genotype level. Strong LD is observed across the entire region but is particular strong between and among 1894003, 675185, 673478, and 615000. Pearson chi-squared statistics suggest that haplotypes are significantly associated with breast cancer. Haplotype TTCTC contributes the most to this relationship. Odds ratios and score statistics indicate that individuals with haplotype TTCTC are 2.6 times more likely to have breast cancer than individuals with other haplotypes.

Statistics

Chi-squared statistics are estimated to assess whether 1) alleles and genotypes are associated with breast cancer status and 2) marker genotype frequencies deviate significantly from Hardy-Weinberg equilibrium (HWE). Haplotype frequencies and relative frequencies are estimated, as well as several statistics ($r^2$, D', and p-value) that gauge the extent and stability of linkage disequilibrium between markers in each region. Chi-squared statistics and score tests are estimated to determine whether reconstructed haplotypes are significantly associated with breast cancer status (P < 0.05). P-values are estimated for 1) the full set of reconstructed haplotypes and 2) a reduced set that excludes haplotypes with observed frequencies less than 10. Results are presented by chromosome order.

Results

Summary Statistics: Alleles and Genotypes

**SNP Locations**

| SNP.ID | Type | Location |
|---|---|---|
| 1894003 | Proximal | 71972974 |
| 675185 | Proximal | 71998270 |
| 673478 | Incident | 72021802 |
| 615000 | Proximal | 72025650 |
| 2276396 | Proximal | 72046603 |

**Allele by GYNGroup**

| | N | Case(N=510) | Control (N-538) | Test Statistic |
|---|---|---|---|---|
| 1894003:C | 1026 | 91%(450) | 96%(510) | Chi-square=6.95 d.f.=1 P=0.00838 |
| 675185:G | 1010 | 92%(451) | 95%(498) | Chi-square=3.96 d.f=1 P=0.0466 |
| 673478:C | 1022 | 8%(41) | 5%(25) | Chi-square=5.68 d.f.=1 P=0.0171 |
| 615000:G | 1010 | 92%(434) | 96%(513) | Chi-square=7.4 d.f=1 P=0.00652 |
| 2276396:C | 1028 | 97%(478) | 98%(523) | Chi-square=0.18 d.f.=1 P=0.674 |

**Genotype by GYNGroup**

| | N | Case (N=255) | Control (N=269) | Test Statistic |
|---|---|---|---|---|
| 1894003:TT | 513 | 1 %(3) | 0%(0) | Chi-square=7.43 d.f.=2 P=0.0243 |
| TC | | 15%(36) | 9%(24) | |
| CC | | 84%(207) | 91 %(243) | |
| 675185:TT | 505 | 0%(1) | 0%(0) | Chi-square=4.37 d.f.=2 P=0.112 |
| TG | | 14%(35) | 9%(24) | |
| GG | | 85%(208) | 91%(237) | |
| 673478:TT | 511 | 84%(207) | 91%(241) | Chi-square=6.39 d.f.=2 P=0.0409 |

(continued)

| | N | Case (N=255) | Control (N=269) | Test Statistic |
|---|---|---|---|---|
| TC | | 14%(35) | 9%(25) | |
| CC | | 1%(3) | 0%(0) | |
| 615000:TT | 505 | 1%(3) | 0%(0) | Chi-square=7.8 d.f.=2 P=0.0202 |
| TG | | 14%(34) | 9%(23) | |
| GG | | 84%(200) | 91%(245) | |
| 2276396:CC | 514 | 4%(232) | 95%(255) | Chi-square=0.18 d.f.=1 P=0.67 |

**Genotype QC: Test of Hardy-Weinberg Proportions**

**All**

| | A.freq | D | ChiSq | Pvalue |
|---|---|---|---|---|
| 1894003 | 0.935 | 0.00159 | 0.350 | 0.554 |
| 675185 | 0.935 | 0.00159 | 0.350 | 0.554 |
| 673478 | 0.935 | 0.00159 | 0.350 | 0.554 |
| 615000 | 0.937 | 0.00184 | 0.495 | 0.482 |
| 2276396 | 0.974 | -0.00069 | 0.374 | 0.541 |

**Control**

| | A.freq | D | ChiSq | Pvalue |
|---|---|---|---|---|
| 1894003 | 0.953 | -0.002190 | 0.644 | 0.422 |
| 675185 | 0.953 | -0.002190 | 0.644 | 0.422 |
| 673478 | 0.953 | -0.002190 | 0.644 | 0.422 |
| 615000 | 0.957 | -0.001860 | 0.541 | 0.462 |
| 2276396 | 0.976 | -0.000593 | 0.166 | 0.683 |

Summary Statistics: Linkage Disequilibrium

**Haplotype Frequencies**

| | H.freq | H.relfreq |
|---|---|---|
| CGTGC | 961 | 0.935 |
| TTCGC | 1 | 0.001 |
| TTCGG | 1 | 0.001 |
| TTCTC | 39 | 0.038 |
| TTCTG | 26 | 0.025 |

Linkage Disequilibrium Between Markers

**$r^2$**

| | 1894003 | 675185 | GP11.079035103 | 615000 | 2276396 |
|---|---|---|---|---|---|
| 1894003 | 1.000 | 1.000 | 1.000 | 0.968 | 0.387 |
| 675185 | 1.000 | 1.000 | 1.000 | 0.968 | 0.387 |
| 673478 | 1.000 | 1.000 | 1.000 | 0.968 | 0.387 |
| 615000 | 0.968 | 0.968 | 0.968 | 1.000 | 0.369 |
| 2276396 | 0.387 | 0.387 | 0.387 | 0.369 | 1.000 |

**D'**

| | 1894003 | 675185 | GP11.079035103 | 615000 | 2276396 |
|---|---|---|---|---|---|
| 1894003 | 1 | 1 | 1 | 1.00 | 1.00 |
| 675185 | 1 | 1 | 1 | 1.00 | 1.00 |
| 673478 | 1 | 1 | 1 | 1.00 | 1.00 |
| 615000 | 1 | 1 | 1 | 1.00 | 0.96 |
| 2276396 | 1 | 1 | 1 | 0.96 | 1.00 |

**P-value**

| X | 1894003 | 675185 | GP11.079035103 | 615000 | 2276396 |
|---|---|---|---|---|---|
| 1894003 | 1 | 0 | 0 | 0 | 0 |
| 675185 | 0 | 1 | 0 | 0 | 0 |
| GP11.079035103 | 0 | 0 | 1 | 0 | 0 |
| 615000 | 0 | 0 | 0 | 1 | 0 |
| 2276396 | 0 | 0 | 0 | 0 | 1 |

**Haplotype by GYNGroup**

**PHASE Haplotypes (All)**

| | Case | Case(%) | Case.X^2 | Control | Control(%) | Control.X^2 | OR | In.OR |
|---|---|---|---|---|---|---|---|---|
| TTCGC | 0 | 0.00 | 0.48 | 1 | 0.10 | 0.44 | 0.0000 -Inf | -Inf |
| TTCGG | 0 | 0.00 | 0.48 | 1 | 0.10 | 0.44 | 0.0000 -Inf | -Inf |
| CGTGC | 452 | 43.97 | 0.21 | 509 | 49.51 | 0.19 | 0.8001 | -0.2230 |
| TTCTG | 14 | 1.36 | 0.18 | 12 | 1.17 | 0.17 | 1.1690 | 0.1561 |

(continued)

| | Case | Case(%) | Case.X^2 | Control | Control(%) | Control.X^2 | OR | In.OR |
|---|---|---|---|---|---|---|---|---|
| TTCTC | 28 | 2.72 | 4.57 | 11 | 1.07 | 4.23 | 2.5887 | 0.9512 |
| Pearson Chi-squared Test = 11.4058, DF = 4, P-value = 0.02236<br>Permutation Test P-value = 0.14 | | | | | | | | |

**PHASE Haplotypes (Low Frequency Excluded)**

| | Case | Case(%) | Case.X^2 | Control | Control(%) | Control.X^2 | OR | In.OR |
|---|---|---|---|---|---|---|---|---|
| CGTGC | 452 | 44.05 | 0.25 | 509 | 49.61 | 0.23 | 0.7998 | -0.2234 |
| TTCTG | 14 | 1.36 | 0.18 | 12 | 1.17 | 0.16 | 1.1690 | 0.1561 |
| TTCTC | 28 | 2.73 | 4.53 | 11 | 1.07 | 4.21 | 2.5888 | 0.9512 |
| Pearson Chi-squared Test = 9.5506, DF = 2, P-value = 0.008435 | | | | | | | | |

**haplo.score Haplotypes**

| | Hap.Freq | Score | P.X^2 | P.Sim |
|---|---|---|---|---|
| CGTGC | 0.9410 | -2.0316 | 0.0422 | 0.0531 |
| TTCTG | 0.0248 | 0.3232 | 0.7465 | 0.8344 |
| TTCTC | 0.0321 | 2.6973 | 0.0070 | 0.0093 |
| Global Score = 9.1386, DF = 3, Global P.X^2 = 0.0275,<br>Global P.Sim = 0.0212 | | | | |

Modifications may be made to the foregoing without departing from the basic aspects of the invention. Although the invention has been described in substantial detail with reference to one or more specific embodiments, those of skill in the art will recognize that changes may be made to the embodiments specifically disclosed in this application, yet these modifications and improvements are within the scope and spirit of the invention, as set forth in the claims which follow. All publications or patent documents cited in this specification are incorporated herein by reference as if each such publication or document was specifically and individually indicated to be incorporated herein by reference.

Citation of the above publications or documents is not intended as an admission that any of the foregoing is pertinent prior art, nor does it constitute any admission as to the contents or date of these publications or documents. U.S. patents, documents and other publications referenced herein are hereby incorporated by reference.

The present invention will now be described by way of reference to the following clauses

1. A method for identifying a subject at risk of breast cancer, which comprises detecting the presence or absence of one or more polymorphic variations associated with breast cancer in a nucleic acid sample from a subject, wherein the one or more polymorphic variations are detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c);

whereby the presence of the polymorphic variation is indicative of the subject being at risk of breast cancer.

2. The method of clause 1, which further comprises obtaining the nucleic acid sample from the subject.

3. The method of clause 1, wherein the one or more polymorphic variations are detected at one or more positions in SEQ ID NO: 1 selected from the group consisting of 139, 11799, 11851, 11851, 11963, 24282, 26849, 29633, 31254, 31967, 32920, 33929, 35599, 36101, 36101, 36340, 36405, 36517, 36777, 36992, 37645, 37868, 38440, 38440, 38532, 38532, 38547, 38547, 38712, 40684, 40860, 41213, 41419, 41613, 42407, 43440, 43440, 44247, 44247, 44247, 44247, 44677, 44677, 45256, 45256, 45536, 45536, 46153, 47546, 47697, 47944, 47944, 48530, 51102, 57090, 60093, 60439, 62694, 66260, 67295, 67295, 67304, 67731, 67731, 68555, 68555, 70429, 70875, 72360, 74228, 76802, 77664, 78803, 79263, 80810, 81020, 82426, 82783, 85912, 85912, 86135, 86135, 87877, 87877, 88043, 88043, 88206, 88343, 90701, 90701, 90974, 91060, 91087, 91594, 91594, 92302, 92384, 36517, and 44677.

4. The method of clause 1, wherein the one or more polymorphic variations are detected at one or more positions in SEQ ID NO: 1 selected from the group consisting of 11963, 36340, 36992, 37868, 41213, 41419, 41613, 42407, 44247, 44677, 45256, 45536, 51102, 72360, 36517, and 44677.

5. The method of clause 1, wherein the one or more polymorphic variations are detected at one or more positions in a region spanning positions 11851-24282, 36340-37868, 41213-41613, 70875-74228, 42407-45536, and 42407-51102 in SEQ ID NO: 1.

6. The method of clause 1, wherein the one or more polymorphic variations are detected at one or more positions in SEQ ID NO: 2 selected from the group consisting of 191, 1490, 3781, 3935, 4512, 7573, 8467, 9001, 9732, 13477, 13787, 13903, 14355, 15053, 15459, 17762, 19482, 19631, 22170, 22688, 22748, 23376, 23826, 23868, 24154, 25972, 26057, 26361, 26599, 26712, 26812, 27069, 32421, 33557, 35127, 35222, 35999, 36424, 37403, 39203, 39226, 41147, 46176, 50452, 52919, 60214, 61093, 62572, 63601, 65362, 65863, 66207, 66339, 69512, 70759, 71217, 73382, and 76307.

7. The method of clause 1, wherein the one or more polymorphic variations are detected at one or more positions in SEQ ID NO: 2 selected from the group consisting of 7573, 13903, 23826, 26057, 26361, 26599, 26812, 27069, 35127, 35222, 36424, 46176, 50452, 61093, 62572, and 70759.

8. The method of clause 1, wherein the one or more polymorphic variations are detected at one or more positions in a region spanning positions 23826-36424, 46176-62572, 4512-8467 or 13787-14355 in SEQ ID NO: 2.

9. The method of clause1, wherein the one or more polymorphic variations are detected at one or more positions in SEQ ID NO: 3 selected from the group consisting of 107, 2157, 7300, 8233, 9647, 9868, 9889, 10621, 11003, 11507, 11527, 11718, 11808, 12024, 13963, 14300, 14361, 16287, 18635, 19365, 24953, 25435, 26847, 27492, 27620, 27678, 27714, 29719, 30234, 31909, 32153, 33572, 42164, 43925, 45031, 45655, 48350, 48418, 48563, 53189, 56468, 59358, 63761, 65931, 67040, 69491, 83308, 126545, 137592, and 147169.

10. The method of clause 1, wherein the one or more polymorphic variations are detected at one or more positions in SEQ ID NO: 3 selected from the group consisting of 107, 42164, 45031, 45655, 48563, 19365 and 14361.

11. The method of clause 1, wherein the one or more polymorphic variations are detected at one or more positions in a region spanning positions 42164-48563 in SEQ ID NO: 3.

12. The method of clause 1, wherein the one or more polymorphic variations are detected at one or more positions in SEQ ID NO: 4 selected from the group consisting of 174, 815, 3480, 9715, 14755, 15912, 19834, 19850, 20171, 20500, 20536, 23187, 25289, 25470, 28720, 29566, 30155, 30752, 32710, 32954, 33725, 33842, 36345, 38115, 39150, 40840, 41969, 42045, 43785, 44444, 44579, 45386, 46827, 47320, 47625, 47837, 47866, 49002, 49566, 52058, 52249, 52257, 52850, 53860, 54052, 54411, 55098, 55303, 59398, 59533, 60542, 61541, 62309, 72299, 73031, 73803, 80950, 82137, 96077, 96470, 98116,98184, and 132952.

13. The method of clause 1, wherein the one or more polymorphic variations are detected at one or more positions in SEQ ID NO: 4 selected from the group consisting of 174, 815, 3480, 19834, 19850, 20171, 20500, 20536, 23187, 25470, 30155, 30752, 32710, 32954, 38115, 39150, 40840, 41969, 42045, 43785, 45386, 46827, 47320, 47625, 47837, 47866, 49002, 49566, 52058, 52257, 52850, 53860, 54052, 54411, 55303, 59398, 60542, 62309, 72299, 73031, 73803, and 98116.

14. The method of clause 1, wherein the one or more polymorphic variations are detected at one or more positions in a region spanning positions 174-32954, 38115-43785, 45386-52058, 52257-54411, 55303-73803 or 96470-98184 in SEQ ID NO: 4.

15. The method of clause 1, wherein the a polymorphic variation is detected at position 174 in SEQ ID NO: 5.

16. The method of clause1, wherein two or more polymorphic variants are detected in two or more nucleotide sequences.

17. The method of clause 16, wherein polymorphic variants are detected at one or more positions selected from the group consisting of position 44247 in SEQ ID NO: 1, position 36424 in SEQ ID NO: 2, position 48563 in SEQ ID NO: 3, position 49002 in SEQ ID NO: 4 and position 174 in SEQ ID NO: 5.

18. The method of clause 1, wherein the one or more polymorphic variations are detected at one or more positions in linkage disequilibrium with one or more positions in claim 3, 6, 9, 12, or 15.

19. The method of clause 1, wherein detecting the presence or absence of the one or more polymorphic variations comprises:

hybridizing an oligonucleotide to the nucleic acid sample, wherein the oligonucleotide is complementary to a nucleotide sequence in the nucleic acid and hybridizes to a region adjacent to the polymorphic variation;
extending the oligonucleotide in the presence of one or more nucleotides, yielding extension products; and
detecting the presence or absence of a polymorphic variation in the extension products.

20. The method of clause 1, wherein the subject is a human.

21. A method for identifying a polymorphic variation associated with breast cancer proximal to an incident polymorphic variation associated with breast cancer, which comprises:

identifying a polymorphic variation proximal to the incident polymorphic variation associated with breast cancer, wherein the polymorphic variation is detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c) comprising the polymorphic variation;

determining the presence or absence of an association of the proximal polymorphic variant with breast cancer.

22. The method of clause 21, wherein the incident polymorphic variation is at a position in claim 3, 6, 9, 12 or 15.

23. The method of clause 21, wherein the proximal polymorphic variation is within a region between about 5 kb 5' of the incident polymorphic variation and about 5 kb 3' of the incident polymorphic variation.

24. The method of clause 21, which further comprises determining whether the proximal polymorphic variation is in linkage disequilibrium with the incident polymorphic variation.

25. The method of clause 21, which further comprises identifying a second polymorphic variation proximal to the identified proximal polymorphic variation associated with breast cancer and determining if the second proximal polymorphic variation is associated with breast cancer.

26. The method of clause 25, wherein the second proximal polymorphic variant is within a region between about 5 kb 5' of the incident polymorphic variation and about 5 kb 3' of the proximal polymorphic variation associated with breast cancer.

27. An isolated nucleic acid comprising a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c); and
(e) a nucleotide sequence complementary to the nucleotide sequences of (a), (b), (c), or (d);

wherein the nucleotide sequence comprises one or more polymorphic variants associated with breast cancer selected from the group consisting of an adenine at position 11963 in SEQ ID NO: 1, a guanine at position 36340 in SEQ ID NO: 1, an adenine at position 36992 in SEQ ID NO: 1, a guanine at position 37868 in SEQ ID NO: 1, a cytosine at position 41213 in SEQ ID NO: 1, a guanine at position 41419 in SEQ ID NO: 1, a guanine at position 41613 in SEQ ID NO: 1, a cytosine at position 42407 in SEQ ID NO: 1, a cytosine at position 44247 in SEQ ID NO: 1, an adenine or cytosine at position 44677 in SEQ ID NO: 1, a thymine at position 45256 in SEQ ID NO: 1, a guanine at position 45536 in SEQ ID NO: 1, a cytosine at position 51102 in SEQ ID NO: 1, a guanine at position 72360 in SEQ ID NO: 1, a cytosine at position 36517 in SEQ ID NO: 1, a guanine at position 44677 in SEQ ID NO: 1, a guanine at position 7573 in SEQ ID NO: 2, a cytosine at position 13903 in SEQ ID NO: 2, an adenine at position 23826 in SEQ ID NO: 2, an adenine at position 26057 in SEQ ID NO: 2, a thymine at position 26361 in SEQ ID NO: 2, an adenine at position 26599 in SEQ ID NO: 2, an adenine at position 26812 in SEQ ID NO: 2, a cytosine at position 27069 in SEQ ID NO: 2, an adenine at position 35127 in SEQ ID NO: 2, a thymine at position 35222 in SEQ ID NO: 2, a cytosine at position 36424 in SEQ ID NO: 2, a cytosine at position 46176 in SEQ ID NO: 2, a cytosine at position 50452 in SEQ ID NO: 2, a guanine at position 61093 in SEQ ID NO: 2, an adenine at position 62572 in SEQ ID NO: 2, a guanine at position 70759 in SEQ ID NO: 2, an adenine at position 107 in SEQ ID NO: 3, a thymine at position 14361 in SEQ ID NO: 3, a guanine at position 19365 in SEQ ID NO: 3, a thymine at position 42164 in SEQ ID NO: 3, a cytosine at position 45031 in SEQ ID NO: 3, a thymine at position 45655 in SEQ ID NO: 3, a cytosine at position 48563 in SEQ ID NO: 3, a thymine at position 174 in SEQ ID NO: 4, an adenine at position 815 in SEQ ID NO: 4, a cytosine at position 3480 in SEQ ID NO: 4, a guanine at position 19834 in SEQ ID NO: 4, an adenine at position 19850 in SEQ ID NO: 4, a thymine at position 20171 in SEQ ID NO: 4, a thymine at position 20500 in SEQ ID NO: 4, a cytosine at position 20536 in SEQ ID NO: 4, a cytosine at position 23187 in SEQ ID NO: 4, a thymine at position 25470 in SEQ ID NO: 4, a thymine at position 30155 in SEQ ID NO: 4, a guanine at position 30752 in SEQ ID NO: 4, a thymine at position 32710 in SEQ ID NO: 4, a guanine at position 32954 in SEQ ID NO: 4, an adenine at position 38115 in SEQ ID NO: 4, a cytosine at position 39150 in SEQ ID NO: 4, a thymine at position 40840 in SEQ ID NO: 4, an adenine at position 41969 in SEQ ID NO: 4, a thymine at position 42045 in SEQ ID NO: 4, a guanine at position 43785 in SEQ ID NO: 4, a cytosine at position 45386 in SEQ ID NO: 4, an adenine at position 46827 in SEQ ID NO: 4, an adenine at position 47320 in SEQ ID NO: 4, a cytosine at position 47625 in SEQ ID NO: 4, a cytosine at position 47837 in SEQ ID NO: 4, an adenine at position 47866 in SEQ ID NO: 4, a cytosine at position 49002 in SEQ ID NO: 4, a thymine at position 49566 in SEQ ID NO: 4, a cytosine at position 52058 in SEQ ID NO: 4, a thymine at position 52257 in SEQ ID NO: 4, a thymine at position 52850 in SEQ ID NO: 4, a cytosine at position 53860 in SEQ ID NO: 4, a cytosine at position 54052 in SEQ ID NO: 4, a thymine at position 54411 in SEQ ID NO: 4, a cytosine at position 55303 in SEQ ID NO: 4, an adenine at position 59398 in SEQ ID NO: 4, an adenine at position 60542 in SEQ ID NO: 4, an adenine at position 62309 in SEQ ID NO: 4, a cytosine at position 72299 in SEQ ID NO: 4, a thymine at position 73031 in SEQ ID NO: 4, a guanine at position 73803 in SEQ ID NO: 4, and a thymine at position 98116, and an adenine at position 174 in SEQ ID NO: 5.

28. An oligonucleotide comprising a nucleotide sequence complementary to a portion of the nucleotide sequence of (a), (b), (c), or (d) in clause 27, wherein the 3' end of the oligonucleotide is adjacent to a polymorphic variation associated with breast cancer.

29. A microarray comprising an isolated nucleic acid of clause 27 linked to a solid support.

30. An isolated polypeptide encoded by the isolated nucleic acid sequence of clause27.

31. A method for identifying a candidate molecule that modulates cell proliferation, which comprises:

(a) introducing a test molecule to a system which comprises a nucleic acid comprising a nucleotide sequence selected from the group consisting of:

(i) a nucleotide sequence in SEQ ID NO: 1-5;
(ii) a nucleotide sequence which encodes a polypeptide encoded by a nucleotide sequence in SEQ ID NO: 1-5;
(iii) a nucleotide sequence which encodes a polypeptide that is 90% or more identical to the amino acid sequence encoded by a nucleotide sequence in SEQ ID NO: 1-5;
(iv) a fragment of a nucleotide sequence of (i), (ii), or (iii); or

introducing a test molecule to a system which comprises a protein encoded by a nucleotide sequence of (i), (ii), (iii), or (iv); and
(b) determining the presence or absence of an interaction between the test molecule and the nucleic acid or protein,

whereby the presence of an interaction between the test molecule and the nucleic acid or protein identifies the test molecule as a candidate molecule that modulates cell proliferation.

32. The method of clause 31, wherein the system is an animal.

33. The method of clause 31, wherein the system is a cell.

34. The method of clause 31, wherein the nucleotide sequence comprises one or more polymorphic variations associated with breast cancer.

35. The method of clause 31, wherein the one or more polymorphic variations associated with breast cancer are at one or more positions inclause 3, 6, 9, 12 or 15.

36. A method for treating breast cancer in a subject, which comprises administering a candidate molecule identified by the method of clause 31 to a subject in need thereof, whereby the candidate molecule treats breast cancer in the subject.

37. A method for identifying a candidate therapeutic for treating breast cancer, which comprises:

(a) introducing a test molecule to a system which comprises a nucleic acid comprising a nucleotide sequence selected from the group consisting of:

(i) a nucleotide sequence in SEQ ID NO: 1-5;
(ii) a nucleotide sequence which encodes a polypeptide encoded by a nucleotide sequence in SEQ ID NO: 1-5;
(iii) a nucleotide sequence which encodes a polypeptide that is 90% or more identical to the amino acid sequence encoded by a nucleotide sequence in SEQ ID NO: 1-5;
(iv) a fragment of a nucleotide sequence of (i), (ii), or (iii); or

introducing a test molecule to a system which comprises a protein encoded by a nucleotide sequence of (i), (ii), (iii), or (iv); and
(b) determining the presence or absence of an interaction between the test molecule and the nucleic acid or protein,

whereby the presence of an interaction between the test molecule and the nucleic acid or protein identifies the test molecule as a candidate therapeutic for treating breast cancer.

38. The method of clause37, wherein the test molecule inhibits cell proliferation or cell metastasis.

39. A method for treating breast cancer in a subject, which comprises contacting one or more cells of a subject in need thereof with a nucleic acid, wherein the nucleic acid comprises a nucleotide sequence selected from the group consisting of:

(a) a nucleotide sequence in SEQ ID NO: 1-5;

(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c); and
(e) a nucleotide sequence complementary to the nucleotide sequences of (a), (b), (c), or (d);

whereby contacting the one or more cells of the subject with the nucleic acid treats breast cancer in the subj ect.

40. The method of clause 39, wherein the nucleic acid is RNA or PNA.

41. The method of clause 40, wherein the nucleic acid is duplex RNA.

42. The method of clause 41, wherein a strand of the RNA comprises a nucleotide sequence selected from the group consisting of ACAACCGGAAGGUGUAUGA (SEQ ID NO: ); GCCAACCAAUGUGCUAUUC (SEQ ID NO: ); GAUCACCAUGGAGCCAAUU (SEQ ID NO: ); CUGUCACUCGAGAUCUUGA (SEQ ID NO: ); GAGUUGGAUAGCAAGACAA (SEQ ID NO: ) and CGUACGCGGAAUACUUCGA (SEQ ID NO: ).

43. A method for treating breast cancer in a subject, which comprises:

detecting the presence or absence of one or more polymorphic variations associated with breast cancer in a nucleic acid sample from a subject, wherein the one or more polymorphic variation are detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c) comprising the polymorphic variation; and

administering a breast cancer treatment to a subject in need thereof based upon the presence or absence of the one or more polymorphic variations in the nucleic acid sample.

44. The method of clause 43, wherein the one or more polymorphic variations are detected at one or more positions in clause 3, 6, 9, 12 or 15.

45. The method of clause43, wherein the breast cancer treatment comprises a nucleic acid comprising a nucleotide sequence complementary to a nucleotide sequence in SEQ ID NO: 1-5.

46. The method of clause 45, wherein the nucleic acid is a double stranded RNA.

47. The method of clause 46, wherein a strand of the RNA comprises a nucleotide sequence selected from the group consisting of ACAACCGGAAGGUGUAUGA (SEQ ID NO: ); GCCAACCAAUGUGCUAUUC (SEQ ID NO: ); GAUCACCAUGGAGCCAAUU (SEQ ID NO: ); CUGUCACUCGAGAUCUUGA (SEQ ID NO: ); GAGUUGGAUAGCAAGACAA (SEQ ID NO: ) and CGUACGCGGAAUACUUCGA (SEQ ID NO: ).

48. The method of clause 43, which further comprises extracting and analyzing a tissue biopsy sample from the subject.

49. The method ofclause43, wherein the treatment is chemotherapy, surgery, radiation therapy, and combinations of the foregoing.

50. The method ofclause49, wherein the chemotherapy is selected from the group consisting of cyclophosphamide (Cytoxan), methotrexate (Amethopterin, Mexate, Folex), fluorouracil (Fluorouracil, 5-Fu, Adrucil), cyclophosphamide, doxorubicin (Adriamycin), and combinations of the foregoing.

51. The method of clause 50, wherein the combinations are selected from the group consisting of cyclophosphamide (Cytoxan), methotrexate (Amethopterin, Mexate, Folex), and fluorouracil (Fluorouracil, 5-Fu, Adrucil); cyclophosphamide, doxorubicin (Adriamycin), and fluorouracil; and doxorubicin and cyclophosphamide.

52. The method of clause 43, wherein the breast cancer treatment reduces breast cancer metastasis.

53. A method for detecting or preventing breast cancer in a subject, which comprises:

detecting the presence or absence of one or more polymorphic variations associated with breast cancer in a nucleic acid sample from a subject, wherein the polymorphic variation is detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c) comprising the polymorphic variation; and

administering a breast cancer prevention procedure or detection procedure to a subj ect in need thereof based upon the presence or absence of the one or more polymorphic variations in the nucleic acid sample.

54. The method of clause 53, wherein the one or more polymorphic variations are detected at one or more positions in wherein the one or more polymorphic variations are detected at one or more positions in claim 3, 6, 9, 12 or 15.

55. The method of clause 53, wherein the breast cancer detection procedure is selected from the group consisting of a mammography, an early mammography program, a frequent mammography program, a biopsy procedure, a breast biopsy and biopsy from another tissue, a breast ultrasound and optionally ultrasound analysis of another tissue, breast magnetic resonance imaging (MRI) and optionally MRI analysis of another tissue, electrical impedance (T-scan) analysis of breast and optionally of another tissue, ductal lavage, nuclear medicine analysis (e.g., scintimammography), *BRCA1* and/or *BRCA2* sequence analysis results, thermal imaging of the breast and optionally of another tissue, and a combination of the foregoing.

56. The method of clause 53, wherein the breast cancer prevention procedure is selected from the group consisting of one or more selective hormone receptor modulators, one or more compositions that prevent production of hormones, one or more hormonal treatments, one or more biologic response modifiers, surgery, and drugs that delay or halt metastasis.

57. The method of clause 56, wherein the selective hormone receptor modulator is selected from the group consisting of tamoxifen, reloxifene, and toremifene; the composition that prevents production of hormones is an aramotase inhibitor selected from the group consisting of exemestane, letrozole, anastrozol, groserelin, and megestrol; the hormonal treatment is selected from the group consisting of goserelin acetate and fulvestrant; the biologic response modifier is an antibody that specifically binds herceptin/HER2; the surgery is selected from the group consisting of lumpectomy and mastectomy; and the drug that delays or halts metastasis is pamidronate disodium.

58. A method of targeting information for preventing or treating breast cancer to a subject in need thereof, which comprises:

detecting the presence or absence of one or more polymorphic variations associated with breast cancer in a nucleic acid sample from a subject, wherein the polymorphic variation is detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c) comprising the polymorphic variation; and

directing information for preventing or treating breast cancer to a subject in need thereof based upon the presence or absence of the one or more polymorphic variations in the nucleic acid sample.

59. The method of clause 58, wherein the one or more polymorphic variations are detected at one or more positions in wherein the one or more polymorphic variations are detected at one or more positions in claim 3, 6, 9, 12 or 15.

60. The method of clause 58, wherein the information comprises a description of a breast cancer detection procedure, a chemotherapeutic treatment, a surgical treatment, a radiation treatment, a preventative treatment of breast cancer, and combinations of the foregoing.

61. A method of selecting a subject that will respond to a treatment of breast cancer, which comprises:

detecting the presence or absence of one or more polymorphic variations associated with breast cancer in a nucleic acid sample from a subject, wherein the polymorphic variation is detected in a nucleotide sequence selected from the group consisting of:

(a) the nucleotide sequence of SEQ ID NO: 1-5;
(b) a nucleotide sequence which encodes a polypeptide consisting of an amino acid sequence encoded by a nucleotide sequence in SEQ ID NO: 1-5 ;
(c) a nucleotide sequence which encodes a polypeptide that is 90% or more identical to an amino acid sequence encoded by a nucleotide sequence in SEQ ID NO: 1-5 ; and
(d) a fragment of a nucleotide sequence of (a), (b), or (c) comprising the polymorphic variation; and

selecting a subject that will respond to the breast cancer treatment based upon the presence or absence of the one or more polymorphic variations in the nucleic acid sample.

62. The method of clause 61, wherein the one or more polymorphic variations are detected at one or more positions in clause 3, 6, 9, 12 or 15.

63. A composition comprising a breast cancer cell and an antibody that specifically binds to a protein, polypeptide or peptide encoded by a nucleotide sequence identical to or 90% or more identical to a nucleotide sequence in SEQ ID NO: 1-12.

64. The composition of clause 63, wherein the antibody specifically binds to an epitope that comprises a leucine at amino acid position 359 in SEQ ID NO: 17, a leucine at amino acid position 378 in SEQ ID NO: 17, or an alanine at amino acid position 857 in SEQ ID NO: 17, or a ICAM5 polypeptide comprises a proline at amino acid position 352 or an alanine at amino acid position 348 in SEQ ID NO: 15.

65. A composition comprising a breast cancer cell and a RNA, DNA, PNA or ribozyme molecule comprising a nucleotide sequence identical to or 90% or more identical to a portion of a nucleotide sequence in SEQ ID NO: 1-12.

66. The composition of clause 63, wherein the RNA molecule is a short inhibitory RNA molecule.

67. A method for determining a risk of breast cancer in a subject, which comprises detecting the presence or absence of two or more polymorphic variations associated with breast cancer in a nucleic acid sample from a subject, wherein two or more of the polymorphic variations are detected in a nucleotide sequence selected from the group consisting of:

(a) a nucleotide sequence in SEQ ID NO: 1-5;
(b) a nucleotide sequence which encodes a polypeptide encoded by a nucleotide sequence in SEQ ID NO: 1-5;
(c) a nucleotide sequence which encodes a polypeptide that is 90% or more identical to the amino acid sequence encoded by a nucleotide sequence in SEQ ID NO: 1-5;
(d) a fragment of a nucleotide sequence of (a), (b), or (c);

whereby the presence of the polymorphic variation is indicative of the subj ect being at risk of breast cancer.

68. The method of clause 67, wherein two or more polymorphic variants are detected in two or more nucleotide sequences.

69. The method ofclause 68, wherein the two or more polymorphic variations are at one or more positions in wherein the one or more polymorphic variations are detected at one or more positions in clause 3, 6, 9, 12 or 15.

70. The method of clause 69, wherein polymorphic variants are detected at one or more positions selected from the group consisting of position 44247 in SEQ ID NO: 1, position 36424 in SEQ ID NO: 2, position 48563 in SEQ ID NO: 3, position 49002 in SEQ ID NO: 4 and position 174 in SEQ ID NO: 5.

**Claims**

**1.** A method for identifying a subject at risk of breast cancer, which comprises detecting the presence or absence of one or more polymorphic variations associated with breast cancer in a nucleic acid sample from a human subject, wherein one or more of the polymorphic variations are detected at a position selected from the group consisting of a guanine corresponding to position 7573, a cytosine corresponding to position 13903, an adenine corresponding to position 23826, an adenine corresponding to position 26057, a thymine corresponding to position 26361, an adenine corresponding to position 26599, an adenine corresponding to position 26812, a cytosine corresponding to position 27069, an adenine corresponding to position 35127, a thymine corresponding to position 35222, a cytosine corresponding to position 36424, a cytosine corresponding to position 46176, a cytosine corresponding to position 50452, a guanine corresponding to position 61093, an adenine corresponding to position 62572, a guanine corresponding to position 70759 of Figure 2, and a complement of the foregoing; whereby the presence of the one or more polymorphic variations is indicative of the subject being at risk of breast cancer.

**2.** The method of claim 1, wherein detecting the presence or absence of the one or more polymorphic variations comprises:

hybridizing an oligonucleotide to the nucleic acid sample, wherein the oligonucleotide is complementary to a nucleotide sequence in the nucleic acid and hybridizes to a region adjacent to the polymorphic variation;
extending the oligonucleotide in the presence of one or more nucleotides, yielding extension products; and
detecting the presence or absence of a polymorphic variation in the extension products.

**3.** The method of claim 1, wherein the one or more polymorphic variations comprise a guanine at position 7573.

**4.** The method of claim 1, wherein the one or more polymorphic variations comprise a cytosine at position 13903.

**5.** The method of claim 1, wherein the one or more polymorphic variations comprise an adenine at position 23826.

**6.** The method of claim 1, wherein the one or more polymorphic variations comprise an adenine at position 26057.

**7.** The method of claim 1, wherein the one or more polymorphic variations comprise a thymine at position 26361.

**8.** The method of claim 1, wherein the one or more polymorphic variations comprise an adenine at position 26599.

**9.** The method of claim 1, wherein the one or more polymorphic variations comprise an adenine at position 26812.

**10.** The method of claim 1, wherein the one or more polymorphic variations comprise a cytosine at position 27069.

**11.** The method of claim 1, wherein the one or more polymorphic variations comprise an adenine at position 35127.

**12.** The method of claim 1, wherein the one or more polymorphic variations comprise a thymine at position 35222.

**13.** The method of claim 1, wherein the one or more polymorphic variations comprise a cytosine at position 36424.

**14.** The method of claim 1, wherein the one or more polymorphic variations comprise a cytosine at position 46176.

**15.** The method of claim 1, wherein the one or more polymorphic variations comprise a cytosine at position 50452.

**16.** The method of claim 1, wherein the one or more polymorphic variations comprise a guanine at position 61093.

**17.** The method of claim 1, wherein the one or more polymorphic variations comprise an adenine at position 62572.

**18.** The method of claim 1, wherein the one or more polymorphic variations comprise a guanine at position 70759.

**19.** A method for identifying a polymorphic variation associated with breast cancer in the nucleotide sequence of Figure 2 proximal to an incident polymorphic variation associated with breast cancer, which comprises identifying a polymorphic variation in the nucleotide sequence of Figure 2 proximal to the incident polymorphic variation associated with breast cancer in a nucleic acid sample from a human subject and determining the presence or absence of an association of the proximal polymorphic variant with breast cancer, wherein:

the incident polymorphic variation is at a position selected from the group consisting of positions 7573, 13903, 23826, 26057, 26361, 26599, 26812, 27069, 35127, 35222, 36424, 46176, 50452, 61093, 62572, 70759 in Figure 2 and a complement of the foregoing.

# FIGURE 1-A

ICAM REGION GENOMIC

>19:10203901-10296400

```
1     agcgagactc cgtctcaaaa aaaaaaaaaa aaaaaaaaaa gccaggcgag gtgactcaca
61    cccataatcg cagcactttg ggaggccaag gcgggcagat cgcttgagcc cagaagtttg
121   agatctgcct gggcaacaYg gcgagaccct gtctctatga aacaagaaaa aagaaagaaa
181   cgaaatactg ttccatcatt tgccacataa aatctaaact ctctggtgca gaacagactt
241   gcaaatctgc ctgtaaaatc atacaccgga ttttggagta gacacacagg aggctgattg
301   tagtggtggc ttctgggaga agaactgggg ggtagggggc acagataaga gtagacagaa
361   ttttcactct agtaatcttt gtaatttttg aaattctctc taccaggtgt atgaatgacc
421   aatgaaaata tacaataggc caggcgcagt ggctcacgcc tataatccca gcactttggg
481   aggctgaggc gggtggatca catgaggtca ggagtccgag gccagcctga ccaacatggt
541   gaaaccttgt ctctacgaaa aatacaaaca ttagtcaggt agggagcatc acttgagaac
601   aggagttcaa gaccagcctg gacaacaaag caagacctct gcctctacaa aaaaaaaaaa
661   aaaaaaaaaa aattgctggg catggtggaa catgcctgtg gcctcagcta cttaggtggc
721   tgcagcagag gatcatctga ggccaggaat tcaagactac agtgaactat aatcgttaca
781   ctgcactaca gactggtaac agggtgagat cctgtctcaa aaacaacagc aacaaaataa
841   acgattaaaa gacctttaa agttgcaaaa tacatagagt cttaaacatg catccacagt
901   tatttattta tttatttaga aacggagttt cactcttgtt gcccaggctg gagtgcaatg
961   gtgcggtatc agctcactgc aacctccgcc tcccaagtta aaggaattct gcctcagcct
1021  tccaagtagc tggaattaca ggcatgcgcc accatgctcg gctaattttg tatttttagt
1081  aaagatgggg ttttggcata ttggccagga tggtttcgaa cccctgacct caggtgatcc
1141  acccgcctcg gactcccaaa gtgctaggat tataggcatg agccaccacg cccggccgca
1201  ttcagttatt cttgcaggga taaaatacac aatgaaatac actatacttt ttgttttctt
1261  tctttctttt tttttttttt ttttgagaca gagtctcact cgtcgcatgg gctggagtgc
1321  ggtgcgggat ctcggctcac tgcaacctct gcctcctggg ttgaagcgat tctcctgcgt
1381  cagcctcccg agtagctggg attacaggtg cccgccacta cgcccagcta atttttttgt
1441  attttttagta gagataaggt ttcaccatgt tggccaggct ggtctcgaac tcctgactcg
1501  tgattcgccc accttggcct cccaaagtgc tgggattaca ggcgtgagcc accgcacccg
1561  gccacatttt tttttttttt tttttgagac aggatcttag tctgtcaccc aggctggagt
1621  acagtggcat gaacatggct cactgcagcc tcaatctctt gggttcaggt gatccttctg
1681  ccttagcctc cccattagct gggaccacag gcatatacca tcacacctag ctaattttta
1741  aatttttggt agaggccagg cgcggtggct cacgcctgta atcccagcac tttgggaggc
1801  cgagtcgggt ggatcacctg aggtcgggag ttcgagacca gcctggccaa aatggtgaaa
1861  ccctgtctct actaaaaata aaaaataaaa ataaaaatta gctgggcgta gtggcgggcg
1921  cctgtaatcc cagctactcg ggaggctgag gcaggagaat cgcttgaacc caggaggcag
1981  aggttgcagt gagccaagat cgcgctactg cactccagcc tgggcaacag agtgagattc
2041  tgtctcaaaa aaaaaataat aataatgcta tttattgact ttacaccttg taccaggcat
2101  gggaagcttt gcctccatta cgtcactgaa tctcataacc tccttttcca gcagaggaaa
2161  atgaggttgg ttcacagacc acgttgtcag ctgtgctctg tccagaacgc actggcctcc
2221  aagtagacag ccctggactg gtagggaagc cggctatgat ccggtggcgc cccctggagg
2281  tctatcggga acatggtaaa gaacctaaaa atgggtgggc cacagtagct catgcctgta
2341  atcccagcac tttgggggac caatgcggga gaattgcttg agcccaggag ttcaagacca
2401  gcctgggcaa cattgggaga cccacccccc gccatctcta caaaaaaaaa attaggccgg
2461  gcgcggtggc tcaggcctgt aatcccagca ctttgggagg ccgaggcggg tggatcacct
2521  gaggtcagga gttcaagacc agcctggcca acgtggtgcc acactgtctc taataaaaac
2581  acgaaaatta gggccgggct cggtggctca cgcctgtaat cccagcactt tgggaagccg
2641  aggcgggcgg atcacgaggt caggagattg agaccatcct ggctaacacg gtgaaacccc
2701  ctctctacta aaaatacaaa aagttagcca ggtgtggtgg cgggcgcctg tagtcccagc
2761  tactcggtaa gctgaggcag ggaatcgctt gaacccggga ggccgagatg tgcgatctca
2821  caccactgca ctccggcctg ggcgacagag cgatactcca tctcaaaata caaatacaaa
2881  ataaaaaaat acaatacaaa aatgagccgg gcttgcgtac ctgtagacca gctactcagg
2941  agacagaggc aggagaatcc cttgagctct ggaggtcgag gctgcagtaa gccatgatct
3001  tgcccattgc actccagcct gggcgacaga ggaagacctt gtctctaaaa accaaaacaa
3061  agaaactaaa aataagcatt cggatttgtt agggggcgac aagggaggca ctccaggatc
3121  tgtggactcc ccactttgtt ctgtccttgg agagccctgg aaggtctgag aggggacggg
3181  acctggttta agggggtagg ggagaggacc ctggtctagg gggtaaggga cacaaatacc
3241  taatctgagg ggtttcgggg ggacatggcc ctgccctggg gaatctaaac tgggaggcat
3301  ggtctggcag gcccaatcct gaaggctttc tgagggcaat aaggccctgc ctttaaacaa
3361  atgaaaaaac gccaggcacg gtggctcacg cctgtaatcc cagcactttg gaaggctgag
3421  gcgggaggat cacgaggtca ggagatcgag accatcctgg ctaacgcggt gatacccgtc
```

# FIGURE 1-B

3481 tctactaaaa atacaaaaaa ttagccaggc gtggtggcgg gcgcctgcag tcccagctac
3541 tcgggaggct gaggcaggag aatggcgtga acccaggagg cggagcttgc agtgagccga
3601 gatcgcgcca ctgcactcca gcctggcaa cagagcaaga ctccgtctca aaaaaaacaa
3661 aaacaaaaac aaaaacaaaa acaaaaaaac aaataaaaaa acaaaaccac ataactggat
3721 aaagaaaaaa ccatctccga cagccttgga ggcgggatga aggcgtggcc cggtgggcgt
3781 gaccaacagc aaaagtttaa gcgttattgg ctgtattcct tagttgctca ctccagaact
3841 gcccacttat gggcgggggt cactccttca ggcttaacag tcattggctg aattgggcca
3901 gagaggtctc attggctgaa ttcctgcacc ggctcgtcgg aggcgggacc caaagtaggc
3961 taggcctacg gaagctgggt cttcttgctg tgaggtcgcg ttccccagtg ttacggaggg
4021 tccttgaggc aggagtgaaa attgggtctg gggttagtc ctggggtgga ggtctgggca
4081 cgccgggtcg gacccctcc atcttcggtt ttgcacaccc cgctttccag cgcggagtcg
4141 cggcgggtag ggcggcgtcg cgtgcgtgac gtcatccagc ggcgcctcgc gaggctccag
4201 tggccttgac ctcccgcggc gtgggaggct gcgcggcgat gctgcagttc gtccgggccg
4261 gggcgcgggc ctggcttcgg cctaccggca gccaggtgag gccaggggct ggaggcgtgg
4321 tcgaaggatg aaatttgggg gtgtccaggg gtcgtctctc actttcgccc aacccttgca
4381 gggcctgagt tccctggcgg aagaggcagc gcgtgcgacc gagaacccgg agcaggtggc
4441 gagcgagggt aaggcaaccg gggtggctcc aggaggggcg gcgacagaga ggtctgaccc
4501 ttgaccctaa cctctgaccc ccgcaatcgc tccaggtctc ccggagcccg tgctgcgcaa
4561 agtcgagctc ccggtaccca ctcatcgacg cccagtgcag gcctgggtcg agtccttgcg
4621 gggcttcgag caggagcgcg tgggcctggc cgacctgcac cccgatgttt tcgccaccgc
4681 gcccaggtga gcgagggctg taatggtgaa ctgagtggca gagggatgaa gagcgggatt
4741 tcaggagtca cgatgacttt gggcttgtac ccttgggaaa gtgctgtatt tctacagcct
4801 ccgtttctcc acctgtcaaa ggggaatgat gacagtttcc cctgctgtag cgctgtgtga
4861 gattgaagcc tgagaggtga catcatctaa gggttaggga gacagaattc tggagcccga
4921 ctgattaggt tcaaatcctg ccttcccctc ttgtccctca gtgtccctat tttgtcagcg
4981 gtcgggaggt tgctgtgatg aataaatgac ttaattctgg cacataataa gttctataga
5041 aatgttgata atctttgtta actggttttt gcaaataaga gcactaaaaa gactaaacca
5101 ttcctcggtg cctggaagag gctgtttgca ttttagttac cctgctgttc ataacatctc
5161 taagaaaatg taggggccac cctgggcgca gtggctcacg cctgtaatcc cagcactttg
5221 ggaggccaag gcgggcggat cacgaggtca ggagatcgag accatcctgg ctaacatggt
5281 gaaaccccgt ctctactaaa aatacaaaaa aaaattagcc gggcgtggtg gcgggcgtct
5341 gtagtcccag gtactctgga ggctgaggca agagaatggc gtgaacccgg gaggcggagc
5401 ttgcagtgag ccgagatcgc gccactgcac tccatcctgg gcaacactct gtctcaaaaa
5461 aaaaaaaaaa aaagaaagaa aagaaaatgt aggggccagt tactgtggct cacatctgta
5521 atcccagcac attgggaggc cgaggtgggc ggatcacttg aaaccaggag ttgcagacca
5581 gcctggccaa catgatgaaa ccccgtctct accaaaaata caaaaattag ccggacgtct
5641 tagtgcaagc ctgtagtccc agctactcag gaggctgagg catgagaatc gcttgcacct
5701 ggggagatgg aggttgcagt gagccgtgat tatactactg cactccagcc tgggcgacag
5761 agacagactc catctcaaaa aaaaaaacag gtgaaattga tttcaataat gtatttaacc
5821 tgtatttaaa actatgtcga aatcacattg tagcatgggg cactggccat gtttcagatg
5881 ttgagtatgt gactgtatgg aatggtatag aactagagag gaaaaccagt ccctgaagaa
5941 ggtggcaata agtgaagtgt aatagcagga aaaaagtaat ggtaggaaaa acaaggaaga
6001 aggggtggct tttttttct gagatgaagt ttcgctcttg tcgcccaggc tggagtgcaa
6061 tggcatgctc tcagctcact tcaacctccg cctcctgggt tctactaatt ctcatgcctc
6121 agcctcccga gtagccagaa tgacagacat gtaccaccgt gcccagctaa tctttgtata
6181 ttttggagag acatcacttt gccatgttgc ccaggctggt cttactcctt gcctcaagtg
6241 atccacctgc cttggcctcc caaagtgcca ggattccagg catgagccac tgcacctggt
6301 cagggtggct ctttctttag aaggtacctt tcagcagtta tggagctgc tacctgtagg
6361 ctgagaaaga accatccagg agaagagtgt ttcaggcaga gggaacagca agtgccaagg
6421 ccctgaggca gaatttcaag atggggtcag tgaggggcag aggcaaatcg cccagggccc
6481 tggaggcaga agggagaatc ctgggtttc ctatagtggg gtgggagcgt ttgaagcaga
6541 gttgggcttc tcatgtgtcc ttcctccccg caggctggac atactgcacc aggttgctat
6601 gtggcagaag aacttcaaga gaattgtgag tgcctaaatg gagcaaggtg gtgggaagga
6661 gcttcctggg gaggttgggg ataggaccca gaggaagccc atcgctgggt ttctctgga
6721 ctgctcggct ggggcctcat ctgtctcctg aactattcac cgatgggtcc atttttggtt
6781 ctctttttt tgtttgtttt tgagatggag atggagtctc actctgtcac ccaggctgga
6841 gtgcagtggc gcaatcttgg ttcactgcaa cctccgcttc ctgggttcaa gcgattctcc
6901 tgcctctgcc tcctgagtag ctgggattac aggtgtgcac caccatgcag gctaattttt
6961 gtatttttag tagagatgga gtttcaccat gttggtcagg ctggtctcaa actcctgacc
7021 tcaaggaacc tacctgcctt ggcctcccaa agtgctggga ttacaggcgt gagccaccgt
7081 gcccagctca ttttcagttg ttttctgatc actcactgat gtgggcattg tggtgggtga
7141 gaggatatag cagggaccac gaggacaaaa caggcaaggt ccggctgtgt gcaagtggct
7201 caggcctgta atcccagcac tggaagctga ggtgggtggg ttgcttgggg ccaggaaaga

# FIGURE 1-C

```
7261  ccagcctggg aaacagcaag acccagtctc taccccttcc cccacaaccc caagaaaaag
7321  atgggcaaag tccctattct aatgaaggtc acagtgtcat gggaggagaa gtgaaggtga
7381  gtcagatggt catacgtaat gtgtaattat gagcatgtcc cagagaatgg gacgttcaac
7441  cttgggtcta ctgtagaagc taaccagatc aaggaggcag tggggctagt gtggagataa
7501  caggaacagg gtgtgcaaag gccctgtggc agggaccaca aggaagccag tgttgccaca
7561  gaggccagtg ggagcagtgg ggaggagtct gggtttgtg ccaagaacat tgaagaatcc
7621  attaccagtc agggtcttat ccatccaccc cagaggtctg gccagtcctc cccctgctca
7681  caccttccct ggctccccat caccctagga ataaagtcat cagtctgcta ttctgagttc
7741  ttcctgattt ccttgccctg ctcctcttc ctgattctcc ctgtctgtgt catttcccat
7801  ggccctccct ccttgtggct acaaccacat taaaattgtt caaaaaatag gccggcacgg
7861  tggctcacac ctgtaatctc agcactttgg gaggccaagg caggtggatc acttgaggcc
7921  aggagttcca aaccagccta gtcaacgtgg caaaacccca tctctactga aaatacaaaa
7981  aattagctgg ggccaggtgc ggtggctcac acctgtaatc ccagtacttt gggaggccga
8041  ggcggatgga tcacgaggtc gggagatcga gaccagcctg accaatatgg tgaaacccca
8101  tctctactaa aaatacaaat attagttggg tgtggcagcg ggcgcctgta gtcccagcta
8161  ctctggaggc tgagacagga gaattgcttg aacctgggag gcggaggttg cagtgagccg
8221  agatcgcgcc actgcactcc agcgtgggcg acagagcgag actccatctc aaaaaaaaaa
8281  attagccggg catggtgaca ggcacccgta atctcagcta ctagggaggc tgaggcagga
8341  gaatcgcttg aatctaggaa tcagaggttg cagtgagcca aggtcacacc acttgcactc
8401  cacagcctgg gtgacagagt gagactgcct caaaaaaaaa aatcaatagt tcagaaaata
8461  ccgagcaccc cctgcgtgcc cggtggcagg gcccctgcct ttgtgaggct ggcatggggc
8521  aggagctgtg acttctttac cctccccctc gctccccaag tactctgtgc ttggccagag
8581  agagcccgtc atcatggtgc ctcctgtctg acttccccgt ggcaggactg atctgcccgg
8641  ctccctgaca cctgcctcgt ggacctgacc cccctcctct ttgtgcctcc agagctatgc
8701  caagaccaag acgagagccg aggtgcgggg cggtggccgg aagccttggc cgcagaaagg
8761  cactgggcgg gcccggcatg gcagcatccg ctctccgctc tggcgaggag gtaacaggac
8821  agggtggagg gggcggggag gggtgggggg gccagggaag ggcctgggtg tttactcaca
8881  cacagctgcg cacatctggc atggtattat gtcagccctg ttccctccac ctcatggaac
8941  cacctgggct ggtgacatcg gaactgaggc ccttggacct cactacccat ataaggggac
9001  agagatctgg gagccatcca ctcctccctc tcgcatgccc tgtctcttca gcctgtggcc
9061  acagcccctc ttgaccccac ctcctgtcac cctctcctga cccacacttc ccacagcacc
9121  cagagagctc ttctaaatcg tggaacctga actccggacc tcggcctttg tgtggaacct
9181  gaactcctga ccttgtcatt gtgggccctg gtggctgcac acctttcccc ctcatcccct
9241  cctttcctc tctgaccagg cagagatgac tctctcttgt tttttcggtt gtgtttgttt
9301  ttaactttt attttcttga atgctaacaa gatgactcag ggtggtgcca gccaccccat
9361  cacctgttta cctggccagc tctcctcacc tttcagggtt ctgggccaca cctcctccag
9421  gaagcccccc ttgatctcct ttccttccac atcccccgga gctaccctga tttcttctac
9481  agctgagcct cttttctgcc ctgccggaat gtgaatggca tgagggcagg gaccatgtct
9541  gttgtcttct ctgctgcatt tccaaggccc aggcgagggc agacaccaac acatggtgct
9601  tgcaggggtc tccctgactg ttgttgctcc cagatcatac tgcttgctcc accggagcat
9661  gtgcctgatg ccttcctctc ccgcttgacc tgaaagttcg aacctcctga taacttcagc
9721  attaacagcg tgcttgagtt aagttcacac tctagccact ctatggaatc cacaccataa
9781  ctcatggtgt cctatggggc aggaactgtc cgatctcaag gtggtttgtt tttttattg
9841  tttgcttttg agacaagatc tcgctctgtt gcccaggctg tagtgcagtg gtgcaatcat
9901  agctcactgc agccttgagc tcctgggctc aagtgatcct ccatcctcag cctcccaaaa
9961  tgctggtgtg agccaccttc gccagccctg tctcagagtg tgacagctgg gaaaactgaa
10021 gcccagtgaa gcaaagtcac tggtcccatg ttgtccctga tccagcctcc cctgaggccc
10081 cacccctctct gccttgttcc tggcagcgcc ccctctctcg tcaccccatg ccagccactg
10141 cagcagattg ctgacctcca ggctctgcag tggcccctac ctgctcacat gcctctgtcc
10201 ccgcaggagg tgttgcccat ggcccccggg gccccacaag ttactactac atgctgccca
10261 tgaaggtgcg ggcgctgggt ctcaaagtgg cactgaccgt caagctggcc caggtacagc
10321 catggggggg cccagacagc tgctagaggt ggggctgctc tggacccagg gttcaaacca
10381 tcctttcctt ccaccaggac gacctgcaca tcatggactc cctagagctg cccaccggag
10441 acccacagta cctgacagag ctggcgcact accgccgctg gggggactcc gtactcctcg
10501 tggacttgtg agggcacagg gcagagcagg ggcagggggc cctgagctcc gtactctgag
10561 ggttcaaccc ccactccctg gcctctctta cagaacacac gaggagatgc cacagagcat
10621 cgtggaggcc acctctaggc ttaagacctt caacttgatc ccggctgttg gtgagcaaag
10681 agcccaggcc cctagagtgc gcatgtgcag gctccgctgt tagaatcaca gcggttcaaa
10741 tccggcatct ggtcgctgag tggcctcagg cagtgaccac gctcccggac ccaaccttca
10801 gcttgcccaa agcaataatc tttcctaaag aagtgcttgg ctggggatgg tggctcacgc
10861 ttgtaatccc agtactttgg gaggccaagg cagtctgggc aatatagtga ggctcccatc
10921 tgtactaaaa ataaaaaagt taggcgtggc gatgtgcacc tgtagtccca gctactcggg
10981 ggctgagcca ggcggatagc ttgagctcag ggggccaagg ctgcagtgag tcatgatcgc
```

# FIGURE 1-D

11041 accactgcac ccaaacctgg ggagagagct agactcttgt ctcaaaaaaa aaaaaaaaaa
11101 aaaaaaaaaa gctccaaagt cacctctgtc aaagccacag tctgttcctt ccacagccac
11161 aagatggcga catgagccta agtcaagtcc tgtccctcaa cccacgcccc tcgctggctt
11221 ccccacctct ctcaggatga aagcccaagt catcagggtg gcacgtcagg ccctgcacga
11281 tgtgccccgt cacctccctg ccctcccgtc atttgccctg ggtctccccc accagaatgg
11341 gagccaggag tcccagccag gcacaggacc gagcctggct ctcggtcagc aggtgatgag
11401 ctgggagcag ctccttggcc agaggcttac aggcaacaag cggccaggca gggtctggcc
11461 ccgggctgct gggctcacaa agtcacacta gaccacagtg acgatctctg taagcacaaa
11521 gggactccga tgtgggtggg gtgaggagag aggcagcccc ggcctgaccg gccccccgcc
11581 ccgcccccac cccgccccca ggcctaaatg tgcacagcat gctcaagcac cagacgctgg
11641 tcctgacgct gcccaccgtc gccttcctgg aggacaagct gctctggcag gactcacgtt
11701 acagaccccct ctaccccttc agcctgccct acagcgactt cccccgaccc ctacccccacg
11761 ctacccaggg cccagcggcc accccgtacc actgttgaWg tgaagcacct cttctgagcc
11821 aggccgagcc cctggccgac ttgggagcct Yaggcccacg cccacccttc gaggaaggtg
11881 tcacctggac cccttcattc cacggaggaa gctgaggcca cagggagcgg ccatcgccat
11941 tgggaagggg cgactccacg gaRagcccag acgggcttct gcatccattc cctcttttg
12001 tttttaaaat aaattgtatt tttgaatcaa ggaggataaa gataacttct cagtgtcatt
12061 tttgataatt gcattgagaa cgatgagctc cttcccaggt tctgggcact gttggggatc
12121 cgccgtctca agaggctcac ggtctggttt aggggaaccc cagggctgtt tgtggaaatt
12181 acaagaattc acttacccgg tgagtcagac tccaggagct cacaggtgcg tgggcgctgg
12241 cacttcctag gagctgactc ctgccacatc cctctcctga gcactgctgc cagccattct
12301 caccccctgag agggtttgca gtcctgtctg cgacagtact tcatgcagcc tgagagtgtg
12361 tgcttgattg tacaagtaga tgttgtgtaa acctgagttt tcagagtgac ttcctgtaag
12421 cacagtcaga aaagtaaatg tctctctgta agtgagcaga taagcacatg cttaaaaaca
12481 ccagctgggc gtggtgactc gcacctgtaa ccctagcact ttgggaggtc aaggtggaag
12541 gatcgtttga gctcagagct tccagaccag cctgggcaac atggtgaaac cccatctctg
12601 caaaaaatat aaaaattaag tgggcgtgtt ggcgtccacc tgtggtccca gctacttggg
12661 aggctgaggt gggaggatca cctgagccca ggaagcagag gttgcagtga gctgagactg
12721 agccaccaca caccagcctg ggtgacagag tgagacactg tcttaaaacc ccaatatccc
12781 aatttttgag tttgctgagg ttgccagcca agttaatttt tcttaacagc cataacagac
12841 tataatatag acaatgtgat cgatttattt aaagtcaacc agctgggcgc ggtggctcac
12901 gcctataatc ccagaatttt aggaggctga gccaggtgga tcaccggaag tcaggagttc
12961 gagaccagcc tggccaagcc gggagtggtg gcgggcacct gtaatcccag ctacttagga
13021 ggctgaggtg ggagaatcgc ttgaacccag gaggcagagg ttgcagtgag ccaagatagc
13081 gccactgcgc tccagcctgg gtgacagagc aagattccat ctataaataa ataaataaat
13141 aaagtcaatc aggactgcct tctgcctgtg tgggcctggc caggctaagc agccacacaa
13201 ccctccctac ctgctgggcc acccctggct gaaaacctct ctggaatgcc tctcttggga
13261 ttgccttcag agattgcaac acattctcca aacaccctca gtggacatag actttgatcc
13321 ttgaagcagc agatttgact aagtaaaaaa gaaaaagcta ttaaaaccag ttggctgggc
13381 gcggtggctc acgcctgtaa tcccagcact ttgggaggcc gaggcaggcg gatcacgagg
13441 tcaggagatc gagactatcc tggctaacac agtgaaaccc catctctacc aaaaatacaa
13501 aaaattagcc gggcgtggtg gtgggcgcct gtagtcccag ctactcagga gactgaggca
13561 ggagaatggt gtgaacccgg gaggtggagc ttgcagtgag ccgagatcgc gccactgcac
13621 tccactccag cctggcgaca gagctagact gtctcaaaaa aaaaaacaga ggcgtgccaa
13681 agctcagcag aaaatgccgc ctcatcactc ttcctcttgc cagtcttgtg ggggcaccaa
13741 ggcctagagt aacacccagc tgttggcctg acagtgcctg gcccagcctg gagagttgca
13801 gccagaagta taaatgtggt tcagtctgcg tcatacctgt caggaaacat ggtgagacca
13861 actgctgccc agacggattt tcaaaagaaa tgggtcagaa cgtattcccc cacactggaa
13921 tccctcagcc agattgagga taaaaacagg catcagaaaa aaatgataca ggcaggcctg
13981 gtggctcatg cctgtaatcc cagcactttg ggaggctgag gcgggaggat cgcctgagca
14041 caggagtttg agaccagcct gggcaacaca gtgagaacct atctctacta aaaatagaac
14101 aattagccag gcacggtggt gtggctgtgg tcccagctac tctggaggct gaggtgggag
14161 gatcacttga gccctagggg tagaggctgc agtgagcgga gatcacccca ctgcaatcca
14221 gcctgggcaa cagatcaaga ccctgtctca aaaaaaaaaa aaagaaaaga aaacaaaaga
14281 aaaatatgat acactgacta gaatgtgctt taaaataatt ggcatagttg ggtatggtgg
14341 catgcacctg cagtctcacc tacttggaaa gctgtggcca ggagtttgag accagcctgg
14401 gcaacacagc aagacctcat ctctataaaa aataggcggg gagcagtggc tcacgcctgt
14461 aatcccagca ctttgggagg ccaaggcagg cggatcacta gaggacagga gttcaagacc
14521 agcctggcca acatggtgaa accccatctc tactaaaaat acaaaaattg gcaggatgtg
14581 gtggcgggtg cctataatcc cagctacttg ggaggctgag gcaggagaat cgcttgaacc
14641 tgggaggcag aggttgcagt gagccgaggt catgacactg ccctcaaacc tgggtgacag
14701 agcaagactc ggtctcgaaa aaaaaaataa tgaatgaatg aattaattca ttaattaaat
14761 agggggggta tatgagtttg ttagggctgc cgtaggagtg ccacaaactg caggggttgg

# FIGURE 1-E

14821 ggggttagt caacagaaat ttattctgtc ctgtttctgg aggctggaag tccaaggtga
14881 agaacagggt tagctcctte taagggaaaa tctgttccag atccctctcc tagcgtctgg
14941 tggtttgctt tgctgactat ctttgacatt ccttggcttg tagccacact gcttcagtct
15001 ccaccttcat ctttacatga tgttgtccct gtgggtatgt gtctgtttct gtgtctaaat
15061 ttctcctttt tatttttttc ttttttctct atcttttttt ttagacggag tcgcgctctg
15121 ttgcccaggc tggagtgcag tggctcgatc tcggctcact gcaacttctg cctcctgggt
15181 tcaagcgatt ctcctgcctc agcctcccaa gtagctggga ttacaggcgc ccaccaccat
15241 gcctggctaa tttttgtgtt tttagtagag aaggggtttc gccatattgg ccaggctggt
15301 aacctcaggt gatccacccg ctttggcctc ccaaagtact gggattacaa gcgtgagcca
15361 ccgaacctgg cctatttttt catttatttt tagacagagt tttgctcttc ttgtccaggc
15421 tagagtacaa tggcgcaatc tcggctaatc tcaacctccg cctcccgggt tcaggcgatt
15481 ctcctgcctc agcctcccaa gtagctggga ttacaggcat acgccacaat gcctggctaa
15541 ttctgttttg ttagcagaga tggggtttca ccatgttggt caggctggtc tcgaactccc
15601 gacctcaggt gatccaccca cctcagcctc ccaaagtgct gagattacag acacgagcca
15661 ctgagcctgg ccctctcctt tttattttta aaatatattt tgtggacggc agcagtggct
15721 cacacctgta atcccagcac tttgggaggc cgaggcgggt ggatcacaag gtcaggaact
15781 cgagaccagc ctggccaata tggtgaagcc ccatctctac taaaaatata aaaattagcc
15841 aggcatggcc gggcgcggtg gctcacgcct gtaatcccag cactttggga ggccgaggcg
15901 ggcggatcac gaggtcagga gatcgagacc atcctggtta atacggtgaa accccgtctc
15961 tactaaaaat acaaaaaaaa aattagctgg gcgaggtggc aggtgcctgt agtcccagct
16021 actcgggagg ctgaggcatg agaatggcgt gaacccggga ggcggagctt gcagtgagct
16081 gagatcgcgc cactgcactc cagcctgggt gacagagtga gactccatct caaaaaaaaa
16141 aaaaaaaaat ttagccaggc gtggtgtcag aagcctgtag tcgcaactac ttgggaggct
16201 gaggcaggag aatcacttga acccaggagg tggaggttgc agtgagccga gaccacatca
16261 cggcacatct aaaaaaaaaa aaagtttctt gtttttgttt tttgcaaaac tactgaaata
16321 aatacagtga gatatttatt tataaatgag aacgaattaa taatgagccg taggctgggt
16381 gtggtggctc atgcctataa tcccagcatt ttggggggcc aaggcaggtg gaacacttga
16441 ggtcaagagt tcgagaccag gctgaccaat atagtggaac cccatctcta ctaaaaatac
16501 aaaagaatta gccgggcatg gtgccgggcg cctgtaatct cagctatggg aggctgaggt
16561 aggagaatcg cttaaaccct ggaggcggag gttgcagtga ggcgatatca cgccactgca
16621 ctccagcctg gggacagag cgagactcca tctctaaata aataaataag gagctgtatt
16681 tcaaaatttg gagaaggtga cactgagagt actgaataca cagtttttg ttttttggt
16741 ttttttgag acagagtctc gctctgtcac ccaggctgga gtgcagtggt gcaacctcgg
16801 ctcactgcaa tctctgcctc ccggttcaag caaatctcct gcctcagcct cccgcgtagc
16861 tgggattaca ggcacgcatc cccatgcctg gctaattttt gtatttttag tagagacggg
16921 atttcaccat attggtcagg ctgatctcaa actcctgacc tcgtgatctg cctgcctcgg
16981 cctcccaaag tgctgggatt acaggcgtga gccaccgagc ctggccccaa aaaatattta
17041 tcaaaactat gttaatgctg gccgggtgcg gtggctcatg cctgtaatcc cagcactttg
17101 ggaggccgag gcaggtggaa cacgaggtca ggagattgag accatcctgg ctaacacggt
17161 gaaaccccgt ctctactaaa aaatacaaaa aattagccgg gcgcggtggt gggtgcctgt
17221 agtcccagct actcaggagg ctgaagcagg agaatggcag gaaccgggga ggcagaggtt
17281 gtagtgagct gagatcgcgc cattgcactc cagcctgggc gacagagcga gaatccgtct
17341 cgaaaaaaaa aaaaaaatac acacacacac acaaaaactg tgttaatgct taactacaca
17401 aaaatgataa tcagataaat atgcatttat ttagagaact gcatgttggt cagtccagtc
17461 cctgcagagg gaattcccag catgacctca ttcacttgtg aagacagagc aatccttgtg
17521 ttttattttt ttaagatgga tctcactctg ttgcccagac tggagtgcag tggcatgatc
17581 tcagccctct gccacctcca ccttccgggt tcaagagatt ctcatgcctc agcctcctga
17641 gtagctgaga ttacaggctt gtgcctccat gcccagctaa ttttttttatt tttactagag
17701 atgaggtttc accagtttgg gcaggccggt ctcaaactcc tgacctcaag tgatccaccc
17761 acctcggcct cccgaagtgc tgggatgaca ggtgcctggt cagcaactgt tgtttagaca
17821 tacacatttt atctgctcgt ccagcatggt cagccctcca cttttaaat tttattttat
17881 ttattttttt gagacagagt ctcactctgt tgtccaggtt ggagtccagt ggcgtgattt
17941 cggctcactg caacctctac ttcccaggtt cgagcaattc tcctgcctca gcttcccgag
18001 tagctgggat tacaggcccg cgtccccaca cctagctaat ttttgtattt ttagtagaga
18061 cagggtttca ccatgttggc caggctggtc tcgaactcct gacctcaggt gattctcctg
18121 ccttggcctc ccaaagtgct gagattacag gtgtgagcca ctgcacacgg ccttaaattt
18181 tatttattat ttatttattt atttatttag agacttagtc tcactctgtt gcccaggctg
18241 gagtgcagtg gcatggtctc ggctcactgc actccacctc ctgggttcac gccattctcc
18301 tgcctcagcc tcccgagtag ctgggactac aggcgcccac caccactccc ggctaatttt
18361 tgtattttta gtagagatgg ggtttcactg tgttagccag gatagtctcg atctcctgac
18421 ctcgtgatcc gcctgcctcg gcctcccaaa gtgctgggat tacttatttt gtttttgta
18481 gagacaggtt ctcactgtgt tgcccaggct ggtcttgaac tcctgatctc aagtgatctt
18541 cccacctcag tctctcaaag ggctgggatt acaggggtga gccactgcac cccaccttcc

# FIGURE 1-F

```
18601  ctctactttt tgacggtttc cttctgctat gaatgtgcat gtccagttgt ctgcttctta
18661  gaactgatat ttaccttcct catccatcag ccattggagg aggactggga ccgctcagat
18721  tattgatctg acccattctt tcggcagggt ttcctggtgg ctgtcttcca tcaccaaaac
18781  tggaatcaga agagtttcca tagccctttt tttttcccca catctttgct gaagcagagt
18841  tttgaaaaac aaaaccacaa actaagctat tccccagaag aaatctgtaa tcaaagataa
18901  gctctgccgg gcacagtggc tcacgccttt tggaggccaa ggcgggcgga tcacctgagg
18961  tcaggagttc tagacctgcc aggccaacat ggtaaaacct catctctact aaaaatacaa
19021  aaattagcta gatgtggtgg tgggtacctg tagtctcagc tacctgggag gctgaggcaa
19081  gagaatcgct tgaacctggg aagtagaggt tgcagtgagc cgagattgca ccactgcact
19141  ccagcctggg cgacggagtg agacgacctc acaaaaattt acataaataa aatgaaaagt
19201  aaaataaaaa tacaaaagtt ggccgggtgc gtttgctcac gcctgtaatc ccagcacttt
19261  gggagggtga ggcaggcaga taatgaggta agaagatcga gaccatcctg gctaacacgg
19321  tgaaaccctg tctctactaa aaatacaaaa aattagctgt gcgtggtgac acgcacctgt
19381  agtcccagct atttgggagg ctgaggcagg agaatcactt gaacctggga ggtggaggtt
19441  gcagtgagcc gagatcgcac cactgcactc cagcctgggc cacagagtga gactccatct
19501  tgaaaaaaaa aaaaaataca aaagttagcc aggggtgttg gtgggtgcct gtaatcccag
19561  ctatttggga ggctaaggca gaagaatttc ttgaacctag gaaacggagg ttgcagtgag
19621  ccgagatcac acctctgtac tccagcctgg acaacagagc gagactttgt ctcaaaaaaa
19681  aaaaaaaaaa aaaaactaaa taggccggga gcagtggctc atgcctataa tcccagccct
19741  ttgggaggcc aaggcaggtg gatcacttga ggtcaggagt ttgagaccag gctggccaac
19801  atggtgtaac cccgtctcta ctaaaaacac aaaaattagc cgggtctggt ggcgtatgtc
19861  tgtaatccca gctactcggg aggctgaggc aggagaatca cttaaacctg ggaggcaggg
19921  gttgcagtga gctgagatcg tgccactgca ctctagccag ggtgacagag tgaaactctg
19981  tctcaaaaaa ttaaaaaaga aattcagcaa gtaatgagtt aaggaattcg aatattaagg
20041  cgagtgacaa ggaacgccca ggatgtggcc caggatggag taggggggac actcatttag
20101  gagaaagctc aggccacaag acaggaggag ccagccttgt tggggttgaa gggaagagca
20161  ttccaggctg agggaactgc aaggcgtttg catgggacac tatgggatgg cttctgccct
20221  tggtgggcag cctctggtct gaggccattc tttggcctgc ctgactgtct ggcaaccggg
20281  aggaagccct gcccttcctg gagacagaaa caaaggtcta ggaaatatct gcttcccttt
20341  tccttgaaaa acgcttaagg gaacggagga ctgggaggtg ccgtctctct ctgccagcct
20401  gccccctacc atagccatcc cactcccatc tcagaaagtg acccgccatc ctccaaaagg
20461  ctcggaccct gatcaaggag tcatccccct tgtcccagca cctccagttg gcccagcctc
20521  caaaacggat gtcaaattca gccctttctc caaggacact gcccagtcca ggccccacta
20581  tcattcatct ggactagaac agtcacctcc tctcccatct cctggctgca gctcttgaag
20641  cctcaactgg gcccctgtga acacttgagt tagggcaagg tccttcctct gctcagaacc
20701  ctctatacct cccacctcgc tgggcataaa agccaaagtc ctggccaggc acggtggctc
20761  acatctgtta tcccagcact ttgggaggcc aaggggggcg gatcactaga ggtcaggagt
20821  tagagaccaa catggtgaaa ccccatctct actaaaaata caaaaattag ctaggcgtgg
20881  tgacgcaccc ctgtagtacc agctactcgg taggctgagg tgggagaatc gcttgaacct
20941  gggaggcaga gtttgcagtg agccgagatc acaccactgt gctccagcct gggtgacaga
21001  acgagactgg ggttcagaaa caaacaaaca aaacaacaaa gtcctcctca ggtgacagga
21061  acttgcacct atctgccctg tcatctccct gcccgctcct ctcctcgaat ctctcctttg
21121  ctaagcctgc tccagccaca ctgttctcct ggctgttcct tttttttttt tttttttttt
21181  ttttttgagt ctcactctca cccaggctgg agtgcagtgc ctctatcttg gctcactgca
21241  acctccgcct gccgggttca agagattctc ctgcatcagc ctcccaagta ggtggaatta
21301  caggtgtgca ccaccacacc cggctaattt ttgtattttg catagagatg gggtctcccc
21361  tatgttgccc aggctggtct tgaactcctg ggctcaagtg atcctcccat ctcggcctcc
21421  caaaatgctg ggattacagg tgggagccgc gcccaggtgg atttttgtct gactctgttc
21481  attcctgtgt ccccagtacc tggaaggacg ccaagcacac agtaggcgct taaaaaacat
21541  tgagccacat gttgagaaaa gaacggcacc attgtggctg caagtgggac ttgggccgcg
21601  cgggggagct cgcgcacctc gggccggggc aagagctcag tggaacccgc ccgaggaaga
21661  acccgtggcg caggattttc ccaggccttc tgaggaccag gggcgtcccc cgtcccaccc
21721  tgtgactttg ctcaggccgt tccggggcgg gaattcagaa ctcctcagcc ccccaagaaa
21781  aaaatatccc cgtggaaatt ccttgggaat gaccgaggcg ggggaaatat gcgtctctgg
21841  atggccagtg actcgcagcc cccttccccg ataggaaggg cctgcgcgtc cggggaccct
21901  tcgcttcccc ttctgctgcg cgacctccct ggcccctcgg agatctccat ggcgacgccg
21961  cgcgcgcccc acaacaggaa agccttaggc ggcgcggctt ggtgctcgga gacttaagag
22021  tacccagcct cgacgtggtg gatgtcgagt cttggggtca cacgcacagg cggtggccaa
22081  gcaaacaccc gctcatattt agtgcatgag cctgggttcg agttgccgga gcctcgcgcg
22141  tagggcaggg gttcgagcgc cccttctccc tgcctcgcct ctgcgcctgg gggctgctgc
22201  ctcagtttcc cagcgacagg cagggatttc gagcgtcccc ctcccctccc tcgtcaagat
22261  ccaagctagc tgcctcagtt tccccgcgga gcctgggacg ccagcggagg ggctcggcgc
22321  gtagggatca cgcagcttcc ttcctttttc tgggagctgt aaagacgcct ccgcggccaa
```

# FIGURE 1-G

22381 ggccgaaagg ggaagcgagg aggccgccgg ggtgagtgcc ctcgggtgta gagagaggac
22441 gccgatttcc ccggacgtgg tgagaccgcg cttcgtcact cccacggtta gcggtcgccg
22501 ggaggtgcct ggctctgctc tggccgcttc tcgagaaatg cccgtgtcag ctaggtgtgg
22561 acgtgaccta gggggagggg catccctcag tggagggagc ccggggagga ttcctgggcc
22621 cccacccagg caggggggctc atccactcga ttaaagaggc ctgcgtaagc tggagaggga
22681 ggacttgagt tcggaccccc tcgcagcctg gagtctcagt ttaccgcttt gtgaaatgga
22741 cacaataaca gtctccactc tccggggaag ttggcagtat ttaaaagtac ttaataaacc
22801 gcttagcgcg gtgtagaccg tgattcaagc ttagcctggc cgggaaacgg gaggcgtgga
22861 ggccgggagc agccccgggg gtcatcgccc tgccaccgcc gcccgattgc tttagcttgg
22921 aaattccgga gctgaagcgg ccagcgaggg aggatgaccc tctcggcccg ggcaccctgt
22981 cagtccggaa ataactgcag catttgttcc ggaggggaag gcgcgaggtt tccgggaaag
23041 cagcaccgcc ccttggcccc caggtggcta gcgctataaa ggatcacgcg ccccagtcga
23101 cgctgagctc ctctgctact cagagttgca acctcagcct cgctatggct cccagcagcc
23161 cccggcccgc gctgcccgca ctcctggtcc tgctcggggc tctgttccca ggtgagtcgg
23221 ggtggggatt gccgtcgggc cagttctccg aagccccggg aggaccggct cccgggtcag
23281 gtcatgcatg cttaggtagc tgtttatggg aaggaggggc tagagacagc gattgaaagg
23341 caacagccag taggttcgaa tccagaccct gcatacctcc acgtgtggcc ttgggctata
23401 gattgcagct ttaaaaaagg gtagggggtt ggagatggag gggaggggcg ggcctcgttt
23461 tgttgcccag gccggtcttg aactccgggg gtctagcctt acctcctgcc tcagcctccc
23521 gagtagctgg gatgagaggt gtgaaccacc gccttgcttg gctagattgc gtctcttaca
23581 gtttctcagc tgtaaaacgg gaaacgttat agcggccacc tggcagggta tcttggccca
23641 gcgcagcacc tggccccagg actcgatcat gatggtttgg gaacttggct ctgtgccaac
23701 ccaacaaggc ttaagggacc cccacccccc tcaagatgta tattctgttc ctcatcctct
23761 ctgcccctgg ggaagtccag ggctgcttct acttggggga attccagagc tgacttatcc
23821 gtggcccaaa gctgagaagt gggacgcccc agcacaccct cccccagctc cagcccagct
23881 agggaagagg gaaggggtca gagggtcttt catggtggtg taagtttggg gaaccaggag
23941 ggtgggagat tgacagcttg gttaacagct caacaaagcc tgagatccag gccagcacgg
24001 tagttcatgc cagtaatccc aacactttag gagccccagg cgggcgaatc acttaaggtc
24061 aggagtttga gaccagcctg gccaacatgg caacatcccg tctctactaa aaatacaaaa
24121 attagctggc atggtggtgg gcgcctgtga tcccagctgc tcgggaggct gagggaggaa
24181 aatcccttaa gcccacgagg ctgaggttgc agtgaaccaa gattgtgcca ctgcactcca
24241 gcctgggaga catagcgaga ttctgtctca aaaaacaaag cRttctgatc cggactcaga
24301 cccagatcgc actgctttct agctgagtaa ccatttctct ctatgaaatg ggaatggtcc
24361 cagaatctcc cttggagaat gtatggagcc agtgtcctca cacccccatc caagatagaa
24421 caaatctgag acaggaatct ttgagtgagg cagtgctggg ctcagacatt ttttcccacc
24481 ttcggaggca gcagaatctg agggacctga tccaaataag cccctcttt ctttcttttc
24541 ttttcttttt ttttttttt tttttttgag acggagtctc actctgtcgc ccaggctgga
24601 gtgcagtggc gtgatctcgg ctcactgcaa cctctgcctc ccaggttcaa acgattctcc
24661 tgcctcagcc tccctgagta gctgggacta caggcatgtg ccatcacacc cggctaatca
24721 ctgtgttagc caggatggtc tcgatttcct gacctcatga tctgcccacc ctgcctccca
24781 aagtgctggg attacatgcg tgagccacag tgcccacccc gtaagcccct tctttcttac
24841 ctgcaaggta gccagttgct acccatcctg tgctgagtta cttgtattag caagggatgg
24901 ggtggctata ctcacccacc ttacagatgg ggaaattgag gcccaaagag ggggaaacta
24961 cgtgtctcag ggagtgagga gccagtctga ttcctggagg gctgactgtc tccacctgac
25021 ttcttaggag ggaggagggc accaacttca cattaaaatc tggttggaca cagtggctca
25081 cacctgtaat cctggcattt tgggaggctt aggcgggagg atcacttgag gccaggagtt
25141 tgagaccagc cttagcaaca tagtgagacc ccatctctac aaaaatgttt ttcagggcca
25201 ggcgcggtgg ctcacaccta taatcccagc actttgggag gctgaggcgg gcggattacc
25261 tgaggtcagg agtttgagac cagcctgacc aacatggaga aaccccgtct ctactaaaag
25321 tacaaaatta cccgggcgtg gtggcgcatg cctgtaatcc cagctactcg ggaggctgag
25381 gcaggagaat cgcttgaacc tgggaggcgg aggttgcggt gaactgagat cgtgccattg
25441 cactccagcc tgggcaacaa gagctaaact ccgtctcaaa aaaaaaaaaa tgtttttcaa
25501 atattagccg ggtatggtgg tgtcctgtag tcccagctac ttgggaggct gagatgggag
25561 gatcacttga gcccaggagt tcaaggttac agtgagctat gattgtgcca ctgtattcca
25621 gcctgggtaa cagagggaga cccgtttaaa aaaaaaaaag tgatggctaa agtccttcca
25681 tggctcccta ttgccctcag tataaagaac acatgtggct gggcgtggtg gttcacgcct
25741 gtaatcccag cactttggga ggctgaggcg ggcggatcac ttgaggccag gagtttgaga
25801 gcaggctggc cgacgtggcg aaaccccgtc tctattaaaa atacaaaaat tagctgggcg
25861 tggtggtgct tgcctgtaat cccagatact ctggaggctg aggcaggaga atcacttgaa
25921 cccgggaggc aaaggttgca gtgagctgag attgcgccac tgcactccag tctgagtgac
25981 aaagcgagac tccatctcaa aaaaaaaaaa aataaaagaa cacatcttta gcatggcctt
26041 cagtgctcac gggatcttcc tgaattaatc tcccctctt catccttgct cactcagctc
26101 cagccacccct gccccgggac atctgtactt gcctggaact tatttccctt ttctccggac

# FIGURE 1-H

```
26161 agccagccct ttctcgtcat ttagatctct gctgaaacat taccctgtca ccaaagcact
26221 gtctattcta tcaccctgtt ttgtttttgt caaagctcat attaacatca gttattaatt
26281 atcttgtttg ctcataattt tttttttttt ttttttggag acagagtctc gttctgttgc
26341 tcaggctgga gtgcagtggc acaatcttgg ctcactgtaa cctccacctc ccaggttcaa
26401 gtgattcttg tgcctcagcc tcccaaatag ctaggactac aggcacgtcc caccatgccc
26461 agctaatttt tgtattttta gtggagacgg ggctttgtca tgttggccag gctgatctca
26521 aattcctgac ctcaagtgat ctgcccgcct tggcctccca aagtgctggg attacaggcg
26581 tgagccacca cacccggcct gctcatgaat tttctcttta acttccacat cgaaggcaaa
26641 gtattgtctt gttaaggctg tgcctccagc acccagcaca ggctgggcgc acattccctt
26701 gatgaacctg atttgtaatg cctgtcgcct cttccctcgt ttcttctagg acctggcaat
26761 gcccagacat ctgtgtcccc ctcaaaagtc atcctgcccc ggggaggctc cgtgctggtg
26821 acatgcagca cctcctgtga ccagcccaWg ttgttgggca tagagacccc gttgcctaaa
26881 aaggagttgc tcctgcctgg gaacaaccgg aaggtgtatg aactgagcaa tgtgcaagaa
26941 gatagccaac caatgtgcta ttcaaactgc cctgatgggc agtcaacagc taaaaccttc
27001 ctcaccgtgt actgtgagta actgagcccg gagggctgga ctaggcagac ccggtgggag
27061 agacgtgcag gggcacctgc agaggcctgg gggaatcttt gccacttgct cgtagggtca
27121 aggaggggct ccttgcaggg caggtgggga catccttgga aagtcccttt gtgaatttct
27181 ttgggtacaa ttaaagtatt tacaggctgg gtgcggtggc tcatgcctgt aatcccagca
27241 ctttgagagg ctgaggctgg cggatcacct gagatcagga gtttaagttt cgccaacatg
27301 gcgaaacccc gtctctgctg aaaatacaaa aatcagccgg gcatggtgtc aagcgcctgt
27361 aatcccagct acttggaagg ctgaggcagg agaacgcttg aacctgagag gcagagattg
27421 cagtgagccg cgatcgtgcc agtgcactcc agtctggata acagagcaag attccatctc
27481 aagaaaaaaa aaatgccatc tctctatgcc tcactctttg aacatatgac acggtcctgc
27541 ttcagacact ttaataaaag atgcaaatta agccaagtgt ggtggcttgt acctataatc
27601 ccaactactc cagaggctga ggcagaagga tggtttgagc ccaggagttt gagaccagcc
27661 tgggcaacag agtgagaccc tgtttctttc tttttttttt tttttttttt gagacggagt
27721 ctcactctgt cgcccaggct ggagtgcagt ggtgtgatct cggctcactg caagctccgc
27781 ctcccgggtt cacgccattc tcctgcctca gcctcccgag tagctgggac tacaggcgcc
27841 tgccaccatg cccggctcat ttttttgtat ttttagtaga gacggggttt cactgtgtta
27901 gccaggatgg tctcaatctc ctgacctagt ggtccgcccg cctcggcctc ccaaagtgct
27961 gggattacag gtgtaagcca ctgtgcccat ccaagaccct gtttctaccg gaaaaaaaaa
28021 gtaaataatt tagctgggca tcgtggtgtg cacctgtaat cccagctgct cctgaggctg
28081 tgatgggagg attgctttaa cccaggggtt cgaatcctag gagttcgaat ccatcctagg
28141 caacatagca aaaccccatt tttatttaaa aaaaaaaaaa aagatatgag ttaaaactag
28201 ccctgggatg gcatttttca catattggta acaaacaaaa gaattgatgg ccgggcgcag
28261 tggctcacgc ctgtaatccc agcactttgg gaggccgagg cgggcggatc acaaggtcag
28321 gagatcgaga ccatcctggc taacatggtg aaaccccgtc tctactaaaa ctacaaaaaa
28381 ttagccgggc atggtggcag gcgcctgtgg tcccagctac tcaggaggct gaggcaggag
28441 aatggcatga acccgggagg cagagcttgc agtgagccaa gatcgtgcca ctgcactcca
28501 gcctgggcga cagagcaaga ctccatctca aaaaaaaaaa aaaaaaaaag aattgataac
28561 agctgtgctg ccaaggctat tggaacgtag gaggtcctag gacagtgctg ttgggagcat
28621 aaataagccc aaccctgtgg cgggaaattg ggcatcagtt ctcaaaatgt catgggctgg
28681 gcacggtggc tcacgcctgt aatcccagca ctttgggagg ctgagggagg cggatcactt
28741 gaggtcagga gttcgagacc agcctgacca ccatggagaa accccgtctc tattaaaaat
28801 acaaaaaaaa ctagccaggc atagtggcac atacctgtaa tcccagctac tcgggaggct
28861 gaggcaggag aatctcttga aactgggagg cagaggttgc ggtgagctga gattgcgcca
28921 ctgcactcca gcttgggcaa caagagcaaa actccatctc aaaaaaaaaa aaaaaagaaa
28981 taaaagaagg tatgttgaat atgagtggta tgccaccctc acattaggga agggcagttt
29041 cggggaggct gtatttatgt ataaaatagc cctaaaagga agtgggagaa atgacaatat
29101 tagctggcta tgagaagaga ggctgggagg ctgtgggaga gggcttgggt gtggagaatt
29161 ctttttgttt tttccttttt ttgagacaga gtttcactct tgttgcccag gctggagtgc
29221 aatggcacga tctcagctca ccgcaacctt cacctcctga gtttaagtga ttctccggcc
29281 tcagcctccc gactagctgg gattacaggc atgggccact acgcctggca aattttgtat
29341 ttttagtaga gacagggttt ctccatgttg gtcaggctgg tcttgaactc tgacctcagg
29401 tgatccgccc gcctcggcct cccaaagtgc tgggattaca acgtgagcca ctttgcctgg
29461 ctgagaattc ttttttgtt gttgtctttt tgagatggag ttttgctgtg tccccagcct
29521 ggagtgcaat ggtgtaatct cagctcactg caacctctcc ctcccgggtt caagcaattc
29581 tcctgcctca gcctcccaag tagctggaat tacaggcgcc cagcaccacg ccYggctaat
29641 ttttgtattt ttagtagaga cgggatttca tcatgttggc caggctggtc ttgatctcct
29701 gaccttgtga tctgcccgcc tcggcctccc aaagtgctgg gattacaggc gtgagccact
29761 gcccccagcc gagaatttct ctttgcgtcc ttcctacttt ggggacttcg aatggtggga
29821 aagagttatc aaggccaaaa taaggaattc aaatgaaaac aaaacaaaat caaagaagaa
29881 aaaacagaag agcactgggc aggctaggca cgtggctcat gcctataatc ccagtgattt
```

# FIGURE 1-I

29941 agaaggccga agtagaagga tcgcttggag gccaggagtt ggacaccagc ctgggcaaca
30001 tagcaagacc ccatatctac aaaaaataaa aaacctaacc aagcgtgctg gcatactagt
30061 agtcccagct actcaggagg ctgaggtggg aggatcacct gagcctggga ggtccaggct
30121 gtagtgagcc gtgatgacac cactgcactc cagcctgggt gacagagaaa gaccctgtct
30181 ctaaaaaata aaaactggcc aagtagcttt gggattagcc ttgggttcca gtcccagcaa
30241 ggcctttaat agcttgggac atgacttctg catttacttt gcaatcaggt gagacctcct
30301 ctgatgggga aaatgacacg gtgagtgaca aaggatgttc tcctatcatt gtgtcagggc
30361 aaggaagcct ctgggtaaat gatcaaatga tcagctttgc ttctgatttg gagggtgggt
30421 gagcagatgc tgaccttccc aggtgaggga agtcccogaa cattcccagc agcttctgga
30481 aaccccaggg aaacctcttt gaaggtcttt tctgcatctc tgcctgatag gtcttttttt
30541 tttttttttt tttttctttt tttttgacac acagtcttgc tctgtcgccc aggctggatc
30601 actgcaacct ccatctcccg ggttcaagca gttctcctgc ctcatcctct ccagtagctg
30661 ggattacagg cacctggcac cacgcctggc taatttttgt atttttagta gagacagggt
30721 ttcgccaagt tggccaggct ggtctcgaac tctagacctc tggtgatcca cccgcctcag
30781 cctcccgaag tgctgggatt acaggcttga gccaccacgc gcggctctgc ctgatagctg
30841 agagcataga actccaggtt tgagacctgg ctctgccaca tttctccctc tatgactgtg
30901 ggtgccccac tttgccttag tttttacctc tgtgaaatgg agcagatggc tggcacaggt
30961 agcaaaggag taaaagttat gtgggagggt ggtacctgag agagactcta gcttggtctt
31021 gccccacccc tggtgtaaac ataaagaagc ctccctggat ggctcaatct tctccaaaaa
31081 ggttagaggt gtaattccta gaggaggcga ccactagctg ggctttgaag gatgtgtagg
31141 agttcataag gacaggcatt ctgggcagga ggaacagcct gggcaaaagt tgggagcagg
31201 gagaaatctt gatggaggca ggaggaggag gaggtaggtt ggtgtaggcc aggYgcagtg
31261 gctcacacct gtaatcccag tgctttggga ggccaaagca agaggattgt ttgagcccag
31321 gagttcgaga ccagcctggg caacatagcg agaccctgtc tctaagaaaa aataaaaaaa
31381 ttagggtaca gtggcatatg cttgtattct caactactct ggaggctcaa gtgggaggat
31441 cacttgaacc caggaatttt tttgtttttg tttttgtttt tttgagatgg agtctcactc
31501 tgtggcccag gctggagtgc agtggtgcca tcttggctca ctgcagcctc ctccacctcc
31561 tgggttcaac cgattctgct gcctgagcct cccgagttgc tgggattaag gtgcccacca
31621 tcatgcccag ctaatttttt tgtattttta gtagagatag ggtttaccat gttagccagg
31681 ctggtcttga acgcctgacc gcaagagatc ctcctgcctc agtttcccaa agtgctggga
31741 ttataggtgt gagccactga gcctggtcaa gcccaggaat ttgaggttac agtcagctat
31801 gattgcacca ctgcattcca gcccaggtga cagagagaga cactgcccct aaaaaaaaaa
31861 aaaaaattga ttgatgggag gaagggtgag gttggcagag ccttgaatgc caggtggagg
31921 agctgggact ttccttcttg ggtgatagg gagtcatgga gggtttStga gcaggccagg
31981 gattagatag ctgaaggctg gatttactgg aagccaatga gcagttggct atggtccttg
32041 tccacgcggc ccatgttgtg ggcagtgacc gtattcaaga agggaaggac agacaagtat
32101 ttgaatactt cagtgaccag gatttggtaa aggactgcag gtcagggtca agaagaggtg
32161 agagcaggac agacttcctc cccgctgcac caggcagctg agctgggttt cctctagggg
32221 ctgaggtttg agggtacctc aagttctgca agagtctata ggaggtggta agagagaaga
32281 gctggaggtc agagttttct tgactatata tatatatatt tttttgtttt tgtttttaac
32341 agcttaacag ctttctgttt tatttttaga gacagggtct cagggtctca ctttgtcacc
32401 caggctggag tgcagtggta caatcgtagc tgactgcagc ctcaaactcc caggctcaag
32461 aaatcctcct cccaccctta gcctcctgag tagcagggac tacaggtgtg agccagcagg
32521 aagcccagct ggtttttttt ttttctttgg tgtttttttgt ttgtttgttt gagaccggag
32581 tttcgctctt attgcccagg ctgaagtgca atggcaggat cttagctcac cacaacctcc
32641 gacccccagg atcaagctat tctcttgcct cagccacctg agtagctggg attacaggca
32701 tgcgacacca cacaaggcta attttgtatt tttagtaaag acagggtttc tccatgttgg
32761 tcaggctggt ctcgaactcc caacctcaga tgatccacct gcctcggcct cccaaagtgc
32821 tgagattaca ggcatgagcc accgtgcccg gccttttttt tttttttttt ttttttgaga
32881 cagagtctca ctctgtcgcc caggcaggag tgcagtggtK cgatctgggc tcactgcaag
32941 ctccgcctcc cgggttcact ccatcctcct gccttagcct cctgagtagc tgggactaca
33001 ggcgcccacc accacgcctg gctaattttt tgtattttta gtagagacgg ggtttcaccg
33061 tgttagccag gatggtcttg atctcctgac ctcgtgatcc gcccgccttg gcctcccaaa
33121 gtgctgggat tacaggcgtg agccaccatg tctggcctgg ccaggctggt cttgaactcc
33181 tgacttccgg tgatccatct gttctggcct cccaaagtgc tgggattaca ggcataagcc
33241 accacgccat gccgaagccc agcttgtttt taattttttt tttttttttt ggagaaatga
33301 ggtcttgcaa tgttgcccaa gctagccttg aactcctggc ctcaaatgac cccgccttgg
33361 catcccaaag tactgggatt acagatgtga gccaccatgc cccagccttg ctttcttgag
33421 atacgattta gaataccata agattcatcc cttttaagca cataattcaa tgacttctgt
33481 acaaacaacc atgactacaa tctaatttta aaatatttca atcactctaa aaaagaaacc
33541 tcctgcttat gtacagcgac tctgtctacc tcttaagtga attctcctac ctttaatagc
33601 cctattttac agttcaggaa actgaggttc agagagacaa agtcacttac ccacagcaaa
33661 gaagcaaggc tgggtatcaa atgcaggacc cccccggtcc tgatgctttt tttttttttt

# FIGURE 1-J

```
33721  tttttttcct ctgagagaga ctctcactct gtcacttagt ctagagtaca gtggcgcgat
33781  ctcagttcac tgcaatctct gcctcctggg ctgaagtaat cctttcctca caagtaaacc
33841  tcagcctctc aagtagctgg gactacaggc acacaacacc acgcctggct aatttttgta
33901  tttttaggta gagacggggt ttcactatRt tggccaggct ggtcttgaac tcctgacctc
33961  aggtgatccg cctgcctcgg cccccaaag tgttgggatt acaggcgtga gccaccacac
34021  gcagcctttt tgttatttaga ctctgtcatt actgactttt ttttttttt aatagaaaca
34081  gggtctttct ttcccaggct aaagtacagt ggcatgatca cagttcacta tagccttaaa
34141  ctcctgggct caagtgatcc tcctgcctca gcctcccaag tagcagggac tacaggtgtg
34201  caccaccaca cccagttaac cattcattca ttcattcatt catttatttt gagatggagt
34261  ctcgctctgt cacctaggct ggagtgcagt ggcacgatct cagctcactg caacctccac
34321  ctcccaggtt caagagattc tcctgcctca gcctcccgag tagctgagac tacaggcgtg
34381  caccaccatg ccagactaat ttttgtattt ttaatagaga cggggtttca ctctgttggt
34441  caggcttatc tcgaactcct gacttcgtgg atccaccctc cttggcctcc caaagtgctg
34501  ggattaaagg cgtgagccac cgcgccctgc caaccttttt ttaatttttc ttagagatgg
34561  gggtctccct atgttgccca ggcttgtctt gaactcctgg cctcaagtga ccctcttgcc
34621  ttggcctcac aaagtgctag gattacagcc tgagccatca cacctggcca acaggttttt
34681  ttttttgttt tgttttgttt tttaaagaat gtctaggcca ggctcattta ctttcacctg
34741  taatcccagc actttgggag gccggggtgg gcagatcact tgaggtcagg aattcgagac
34801  cagcctgggc aacatgctga aaccccgttt ctactaaaaa tacaaaaatt agctgggtgt
34861  ggtgacacgt gcctgtaatc ccagctactc aggaggttga ggcaggagaa ttgcttgaac
34921  ccaggaggca gaggttgcag tgagccaaga tcatgccatc accctccagc ctgggcgaca
34981  gaaggagact cagtctaaaa acttaattaa ttaattaatt aaaaataaaa atacaaaaat
35041  taacctggtg tggtggtgtg tgcctgtaat ctaagctact caggaggctg aggcaggaga
35101  atcccctgaa tcccagaggc agaggttgca gtgagccaag atcgagccac tgtttgccca
35161  gtctagtgca ctgggctgct gaatttattt gaccagacac ctagcaatag actttgaagt
35221  tcttttccac ttttcactct aagatgctgc tgtcatgaat aaggaatatt ttgatcccct
35281  tcacaaacac tcggggccct cttaccagtt ttcactgaag atcttgacat tcctatctgc
35341  ttaggtgtct gggcgtgttt gggggagata ctgaagaggt agggctccca ggcaggtgca
35401  gttcgtctgt taggcaggca gcaaggtcca cttcaccaga cacccccacc tctgttttcc
35461  tgcagggact ccagaacggg tggaactggc acccctcccc tcttggcagc cagtgggcaa
35521  gaaccttacc ctacgctgcc aggtggaggg tggggcaccc cgggccaacc tcaccgtggt
35581  gctgctccgt ggggagaaSg agctgaaacg ggagccagct gtgggggagc ccgctgaggt
35641  cacgaccacg gtgctggtga ggagagatca ccatggagcc aatttctcgt gccgcactga
35701  actggacctg cggccccaag ggctggagct gtttgagaac acctcggccc cctaccagct
35761  ccagaccttt ggtgaggatt gaagaagcca gcagggagaa ggtgggggtg gggtatcctg
35821  caatgcggtg cctgtggcca caggatcttt tgagatgggt gtggccccgg ctaaggggtg
35881  catgtgttct aggcgtatgt gacctaggct gctgagtggc cctggaagag gatctcgcag
35941  gagggggaat gaaatgcccc agagaagggc ttcgggacgt ccatccctgt ctgctcacac
36001  ctttcttctc tccctagtcc tgccagcgac tcccccacaa cttgtcagcc cccgggtcct
36061  agaggtggac acgcagggga ccgtggtctg ttccctggac Rggctgttcc cagtctcgga
36121  ggcccaggtc cacctggcac tgggggacca gaggttgaac cccacagtca cctatggcaa
36181  cgactccttc tcggccaagg cctcagtcag tgtgaccgca gaggacgagg gcacccagcg
36241  gctgacgtgt gcagtaatac tggggaacca gagccaggag acactgcaga cagtgaccat
36301  ctacagtaag aaggggcagg ggcggagtgg ggcttcttgR gggtgtgacc tgaacccggg
36361  gcggggctca ctgtgtgcct attccaggct ttccggcgcc caacRtgatt ctgacgaagc
36421  cagaggtctc agaagggacc gaggtgacag tgaagtgtga ggcccaccct agagccaagg
36481  tgacgctgaa tggggttcca gcccagccac tgggccYgag ggcccagctc ctgctgaagg
36541  ccaccccaga ggacaacggg cgcagcttct cctgctctgc aaccctggag gtggccggcc
36601  agcttataca caagaaccag acccgggagc ttcgtgtcct gtgtgagtgg ggctgctggt
36661  caatggcccc tatcccccaa ggcccaatct ccctgaaggt cccataaggt cttgcctcca
36721  agtcctgccc ccacccacct ccatgtcatc tcatcgtgtt tttccagatg gcccccRact
36781  ggacgagagg gattgtccgg gaaactggac gtggccagaa aattcccagc agactccaat
36841  gtgccaggct tggggaacc cattgcccga gctcaagtgt ctaaaggatg gcactttccc
36901  actgcccatc ggggaatcag tgactgtcac tcgagatctt gagggcacct acctctgtcg
36961  ggccaggagc actcaagggg aggtcacccg cRaggtgacc gtgaatgtgc tctgtgagtg
37021  agccggcggg cagagctggg tgggggcagg ggccatggac ctaatgcaat cctcaccgcc
37081  tgttgtatcc tccccacagc cccccggtat gagattgtca tcatcactgt ggtagcagcc
37141  gcagtcataa tgggcactgc aggcctcagc acgtacctct ataaccgcca gcggaagatc
37201  aagaaataca gactacaaca ggcccaaaaa gggaccccca tgaaaccgaa cacacaagcc
37261  acgcctccct gaacctatcc cgggacaggg cctcttcctc ggccttccca tattggtggc
37321  agtggtgcca cactgaacag agtggaagac atatgccatg cagctacacc taccggccct
37381  gggacgccgg aggacagggc attgtcctca gtcagataca acagcatttg ggccatggt
37441  acctgcacac ctaaaacact aggccacgca tctgatctgt agtcacatga ctaagccaag
```

# FIGURE 1-K

37501 aggaaggagc aagactcaag acatgattga tggatgttaa agtctagcct gatgagaggg
37561 gaagtggtgg gggagacata gccccaccat gaggacatac aactgggaaa tactgaaact
37621 tgctgcctat tgggtatgct gaggYcccac agacttacag aagaagtggc cctccataga
37681 catgtgtagc atcaaaacac aaaggcccac acttcctgac ggatgccagc ttgggcactg
37741 ctgtctactg accccaaccc ttgatgatat gtatttattc atttgttatt ttaccagcta
37801 tttattgagt gtcttttatg taggctaaat gaacataggt ctctggcctc acggagctcc
37861 cagtcctRat cacattcaag gtcaccaggt acagttgtac aggttgtaca ctgcaggaga
37921 gtgcctggca aaaagatcaa atggggctgg gacttctcat tggccaacct gcctttcccc
37981 agaaggagtg atttttctat cggcacaaaa gcactatatg gactggtaat ggttacaggt
38041 tcagagatta cccagtgagg ccttattcct cccttccccc caaaactgac acctttgtta
38101 gccacctccc cacccacata catttctgcc agtgttcaca atgacactca gcggtcatgt
38161 ctggacatga gtgcccaggg aatatgccca agctatgcct tgtcctcttg tcctgtttgc
38221 atttcactgg gagcttgcac tatgcagctc cagtttcctg cagtgatcag ggtcctgcaa
38281 gcagtgggga agggggccaa ggtattggag gactccctcc cagctttgga agcctcatcc
38341 gcgtgtgtgt gtgtgtgtat gtgtagacaa gctctcgctc tgtcacccag gctggagtgc
38401 agtggtgcaa tcatggttca ctgcagtctt gaccttttgR gctcaagtga tcctcccacc
38461 tcagcctcct gagtagctgg gaccataggc tcacaacacc acacctggca aatttgattt
38521 tttttttttt tYcagagacg gggtctYgca acattgccca gacttccttt gtgttagtta
38581 ataaagcttt ctcaactgcc tcagccttgt gtgagttgag gggaggtgtc acatccagct
38641 ggagtccttt ctaagcagcc acagcctgat cctcccactt cctcccccaa gaaaacattg
38701 tgggttgatg gYcataccct gaggttctgg tccaaatcgg actttctatg accttctggg
38761 tctctagtga aaactaaaga ctcctctcca gaaaaaaaca tttggtttct aatgaggcct
38821 ggaatcttat tcttgacctg gggagcggaa tccctttttg cagtactccc gggccctctg
38881 ttggggcctc cccttcctct ccagggtgga gtcgaggagg cggggctgcg ggcctcctta
38941 tctctagagc cggccctggc tctctggcgc ggggcccctt agtccgggct ttttgccatg
39001 gggtctctgt tccctctgtc gctgctgttt tttttggcgg ccgcctaccc gggagttggg
39061 agcgcgctgg gacgccggac taagcgggcg caaagcccca agggtagccc tctcgcgccc
39121 tccgggacct cagtgccctt ctgggtgcgc atgagcccgg agttcgtggc tgtgcagccg
39181 gggaagtcag tgcagctcaa ttgcagcaac agctgtcccc agccgcagaa ttccagcctc
39241 cgcaccccgc tgcggcaagg caagacgctc agagggccgg gttgggtgtc ttaccagctg
39301 ctcgacgtga gggcctggag ctccctcgcg cactgcctcg tgacctgcgc aggaaaaaca
39361 cgctgggcca cctccaggat caccgcctac agtgagggac aggggctcgg tcccggctgg
39421 ggtgagggga ggggctgga agaggtgggg gaagggtagt tgacagtcgc tctatagga
39481 gcgcccgcgg acctcactca gaggctcccc cttgccttag aaccgcccca cagcgtgatt
39541 ttggagcctc cggtcttaaa gggcaggaaa tacactttgc gctgccacgt gacgcaggtg
39601 ttcccggtgg gctacttggt ggtgaccctg aggcatggaa gccgggtcat ctattccgaa
39661 agcctggagc gcttcaccgg cctggatctg gccaacgtga ccttgaccta cgagtttgct
39721 gctggacccc gcgacttctg gcagcccgtg atctgccacg cgcgcctcaa tctcgacggc
39781 ctggtggtcc gcaacagctc ggcacccatt acactgatgc tcggtgaggc acccctgtaa
39841 ccctggggac taggaggaag gggcagaga gagttatgac cccgagaggg cgcacagacc
39901 aagcgtgagc tccacgcggg tcgacagacc tccctgtgtt ccgttcctaa ttctcgcctt
39961 ctgctcccag cttggagccc cgcgcccaca gctttggcct ccggttccat cgctgccctt
40021 gtagggatcc tcctcactgt gggcgctgcg tacctatgca agtgcctagc tatgaagtcc
40081 caggcgtaaa gggggatgtt ctatgccggc tgagcgagaa aaagaggaat atgaaacaat
40141 ctggggaaat ggccatacat ggtggctgac gcctgtaatc ccagcacttt gggaggccga
40201 ggcaggagaa tcgcttgagc ccaggagttc gagaccagcc tggacaacat agtgagaccc
40261 cgtctatgca aaaaatacac aaattagcct ggtgtggtgg cccgcacctg tggtcccagc
40321 tacccgggag gctgagttgg gaggatcctt tgagccctga aagtcgaggt tgcagtgagc
40381 cttgatcgtg ccactgcact ccagcctggg ggacagagca cgaccctgtc tccaaaaata
40441 aaataaaaat aaaaataaat attggcgggg gaacccctctg gaatcaataa aggcttcctt
40501 aaccagcctc tgtcctgtga cctaagggtc cgcattactg cccttcttcg gaggaactgg
40561 tttgtttttg ttgttgttgt tgtttttgcg atcactttct ccaagttcct tgtctccctg
40621 agggcacctg aggtttcctc actcagggcc cacctggggt cccgaagccc cagactctgt
40681 gtaKccccag cgggtgtcac agaaacctct ccttctgctg gccttatcga gtgggatcag
40741 cgcgggccgg ggagagccac gggcaggggc ggggtggggt tcatggtatg gctttcctga
40801 ttggcgccgc cgccaccacg cggcagctct gattggatgt taagtttcct atcccagccS
40861 caccttcaga ccctgtgctt tcctggaggc caaacaactg tggagcgaga actcatctcc
40921 aaaataactt accacgctgg agtgagacca cgaatggtgg ggaggggagg gtcccacgga
40981 catattgagg gacgtggata cgcagaagag gtatccatgt ggtggcagcc gggaaggggt
41041 gatcagatgg tccacaggga atatcacaaa ctcgaattct gacgatgttc tggtagtcac
41101 ccagccagat gagcgcatgg agttggggt gggggtgtc aaagcttggg gcccggaagc
41161 ggagtcaaaa gcatcaccct cggtcccttg ttctcgcgtg gatgtcaggg ccYccaccca
41221 ccgagcagaa ggcggactca ggggcgctcc agggtggctc gagctcacac acgctgagta

# FIGURE 1-L

```
41281   gacacgtgcc cgctgcaccc tgggtaaata cagacccgga gccgagcgga ttctaattta
41341   gacgcccgcg aacgctgcgc gcacgcacac gtgtcctcgg ctcgctggca ctttcgtccc
41401   gccccctccg tcgcgtgcSg gagctgaccc ggaggggtgc ttagaggtat ggctccgcgg
41461   ggtcaaaagg agaaggatca gtgagagagg catccccaca ccctccccta gaactgtcct
41521   ttccccatcc agtgcctccc aaatctctct tagtccccaa atgtatcccc gccctaaggg
41581   gcgctggtgg gaggagctaa atgtgggggc ggRgctcgga gtccagctta ttatcatggc
41641   atctcagcca gggctggggt aggggtttgg gaagggcaac ccagcatccc ccgatcccag
41701   agtcggcggcc ggggatgacg cgagagagcg tggtcgcccc cagaaggccc tgggccatca
41761   tgccggcctc cacgtagacc ccaggggtcg ctcactcctg ccagctcgcc ttcaccaagg
41821   ccaggagctt agcgcacgct cgcctcccgc ccccccgccg cctctgccgc cgccccctcc
41881   ttggaaacca agttaccaac gttaaaccaa tccccaagcg caactctgcc tcccccacac
41941   cccacccgcc gcgccgcgcg gagccgtcct ctagcccagc tcctcggctc gcgctctcct
42001   cgcctcctgt gctttccccg ccgcggcgat gccagggcct tcgccagggc tgcgccgggc
42061   gctactcggc ctctgggctg ctctgggcct ggggctcttc ggcctctcag gtaagagccc
42121   cgctctggtt cggggtggac agggcggggg cggagtccct ggacctgaga aacggcctcc
42181   tgtccctccc agctctgccc tcgcctcgct cccacgcctc tgcccccacc tcgagcctga
42241   gtcttctccc cttccccctc ctccccaaca cacacccgag ccccagcttc ctgactcctc
42301   gatagcccct acccgcttcg agatcctagg tgttcttccg cacccaaccc ttcgccctgg
42361   agacccagtg tctctcctgt ccgctccccg ggtacctcct tacgctStgc tgtgcaccat
42421   ggtccacgga ctggcatctt ccccactcgc ggtccgcgaa gactccatct ctccaactac
42481   cctgactcag aggcgctgtt cccgctccac ccagagccct ggcaaccggg tccctcagct
42541   gttcccgcgg ttccttccat gagcccagcc ttgcgtcccg gctccgtgtt cttcaccggg
42601   tgtagggtcc ttcctgatcc ttgacccagc ctcgtctctc ctttgccctt gccgcaaacg
42661   cactctcctc gtatcccggc attctgccag gaccctgaga actggttcat cccccatccc
42721   ccatcccggg tccccttctc tcagccttgc tgtgttcatc caagaaccca cctttcctct
42781   cctctacgcc ctccccccat gctttcccgc cgctccatcg gcgctttgga gaccatggct
42841   ctctgctacc acgtcccaga gacaccctcg aggtttagac tctgggagtg cgcctttaaa
42901   ccggaggcct gggcaggacg cgggacgcct gggttctttc cctggctgca gcctctcctc
42961   ctcctccccg ccctctgaga acccttgact cgacataggg gcgctaagat gtcagggagt
43021   tggctcccca ggctcagccc gcgtttccct gggcagcggt ctcgcaggag cccttctggg
43081   cggacctgca gcctcgcgtg gcgttcgtgg agcgcggggg ctcgctgtgg ctgaattgca
43141   gcaccaactg ccctcggccg gagcgcggtg gcctggagac ctcgctgcgc cgaaacggga
43201   cccagagggg tttgcgttgg ttggcgcggc agctggtgga cattcgcgag ccggagactc
43261   agcccgtctg cttcttccgc tgcgcgcggc gcacactaca ggcgcgtggg ctcattcgca
43321   ctttccgtga gttctgggtg gccacgcgcg tactccacta ctctccctcc ctcccaggcc
43381   ccgccccctg ggtcccaggg tcctcccctt caggccccac cttctgttcc aagtcccggY
43441   gttcaaagag ctgcggactc ttccccccttg cagagcgacc agatcgcgta gagctgatgc
43501   cgctgcctcc ctggcagccg gtgggcgaga acttcaccct gagctgtagg gtccccggcg
43561   ccgggccccg tgcgagcctc acgctgaccc tgctgcgggg cgcccaggag ctgatccgcc
43621   gcagcttcgc cggtgaacca ccccgagcgc ggggcgcggt gctcacagcc acggtactgg
43681   ctcggaggga ggaccatgga gccaatttct cgtgtcgcgc cgagctggac ctgcggccgc
43741   acggactggg actgtttgaa aacagctcgg cccccagaga gctccgaacc ttctgtgagt
43801   gggtgtgggg aggagatggg gacccagtgg ggtcggtcgg tgtttaggag gtttagaggt
43861   agatacatct gaatgctgac cccgacttca accctcgccg gctgagctgt ttccccctcc
43921   gtgccttgag gatgataata cgataagata gtgcatgtca agtgcttagg acatattgag
43981   cgctcggagt tagttcaaac ttggttcttc gaccccctagc cctgtctccg gatgccccgc
44041   gcctcgctgc tccccggctc ttggaagttg gctcggaaag gcccgtgagc tgcactctgg
44101   acggactgtt tccagcctca gaggccaggg tctacctcgc actgggggac cagaatctga
44161   gtcctgatgt caccctcgaa ggggacgcat tcgtggccac tgccacagcc acagctagcg
44221   cagagcagga gggtgccagg cagctgRtct gcaacgtcac cctggggggc gaaaaccggg
44281   agacccggga gaacgtgacc atctacagta aggaaggagg cggggtctcc gcggctccga
44341   ggtgggacca gaggaatgcg aaggcggggc gaagagtggg cgggacctca gtaccggaac
44401   aggcgtggcc cgaggggcgg ggcaggtggg ggcggagacg taatcgctgg ggaggaggag
44461   cctgtacagc ctgagaggcg gggcgccgta ccctagttcg ttctcagcac cccgaggatt
44521   cgggcagata aggggcgggc cttgaccgga gggaggggta tggtcagtat actacgacca
44581   aatgctccgc ccccaggctt cccggcacca ctcctgaccc tgagcgaacc cagcgtctcc
44641   gaggggcaga tggtgacagt aacctgcgca gctgggRccc aagctctggt cacactggag
44701   ggagttccag ccgcggtccc ggggcagccc gcccagcttc agctaaatgc caccgagaac
44761   gacgacagac gcagcttctt ctgcgacgcc accctcgatg tggacgggga gaccctgatc
44821   aagaacagga gcgcagagct tcgtgtccta tgtgagttgg tgataacccc tcgcccccca
44881   ccttctggtg acttccaagg acccgcctgc tccctcaccg tgtcgtggag gcggagccat
44941   ttcttacgtc taagcctctg taaccccacg ccctgcccgc agacgctccc cggctagacg
45001   attcggactg ccccaggagt tggacgtggc ccgagggccc agagcagacg ctgcgctgcg
```

# FIGURE 1-M

45061 aggcccgcgg gaacccagaa ccctcagtgc actgtgcgcg ctccgacggc ggggccgtgc
45121 tggctctggg cctgctgggt ccagtcactc gggcgctctc aggcacttac cgctgcaagg
45181 cggccaatga tcaaggcgag gcggtcaagg acgtaacgct aacggtggag tgtgagtggg
45241 ggtgcgcagg gtgcaYttct atctggttca aggtctggag ggtggccagc ctccagggaa
45301 gagtaggagt agggtatgag gtgtcccttt gggtgaggtt ttgggaaagg gaagaggctg
45361 gttagtgggg ttggagaaag atcttggagg atggaaggga ccgggtgggc gtgcccctag
45421 cctagggcgt ggtatttggg cggagtcgtg gaaaggcggg cagtccagag tgtttaagtt
45481 tttagacgaa aaaggcgcca ctggtggctc aggaagctcc cagacagagt gcatgSctcg
45541 actagcgtga cacctccttg gatcggcgtc caagggttat gcagggacaa cacttcgtgg
45601 aagccttgcc gcgccaagga gggtctaggg acgtcagatt tgcccccaaa ccccaaagcc
45661 aacaatacac tccctcctcc agacgcacca gcgctggaca gcgtgggctg cccagaacgc
45721 attacttggc tggagggaac agaagcctcg ctgagctgtg tggcgcacgg ggtaccgccg
45781 cctgatgtga tctgcgtgcg ctctggagaa ctcggggccg tcatcgaggg gctgttgcgt
45841 gtggcccggg agcatgcggg cacttaccgc tgcgaagcca ccaaccctcg ggctctgcg
45901 gccaaaaatg tggccgtcac ggtggaatgt gagtaggggc accgcggagt taggcaggat
45961 ctgtgggaca accccggctg gacttcctgg cccccgtgtg agcccctgca atcctgtttc
46021 ccagatggcc ccaggtttga ggagccgagc tgccccagca attggacatg ggtggaagga
46081 tctgggcgcc tgttttcctg tgaggtcgat gggaagccac agccaagcgt gaagtgcgtg
46141 ggctccgggg gcRccactga gggggtgctg ctgccgctgg cacccccaga ccctagtccc
46201 agagctccca gaatccctag agtcctggca cccggtatct acgtctgcaa cgccaccaac
46261 cgccacggct ccgtggccaa aacagtcgtc gtgagcgcgg agtgtgagcg aggcccaggc
46321 gggtagggag caggggtgcc ccacggtcca ggcactccct gacatccccc atggctgctt
46381 tgcagcgcca ccggagatgg atgaatctac ctgcccaagt caccagacgt ggctggaagg
46441 ggctgaggct tccgcgctgg cctgcgccgc ccggggtcgc ccttccccag gagtgcgctg
46501 ctctcgggaa ggcatcccat ggcctgagca gcagcgcgtg tcccgagagg acgcgggcac
46561 ttaccactgt gtggccacca atgcgcatgg cacggactcc cggaccgtca ctgtgggcgt
46621 ggaatgtgag tgggggcagc accggatgga ggggacacgg tcctcggaag aatgactcgc
46681 agcggtggga gcattcaagg gcacctctcc caatcccatt ctcggggaca gggaattcca
46741 gcctaaacca gggggtaatg aaaattctag ccaggcgcag tggctcaggt ctgtaatccc
46801 aacactttgg aaggttgagg cggatgaatc acttgaggcc aggagttcga gaccagcccg
46861 gccaacatgg cgaaaacccg tctctacaaa aattagccgg tcgtggtggt gggcgcctgt
46921 ggtcccagct acttgggagg ctgaggcagg agaatcgctt gagcctggga ggcagaggtt
46981 gcagggagcc gagatcccgc cactgcactc cagcctgggc aacagagtga gactctttct
47041 tagaaaaaca gaaaaagaaa attataggga ataggagcac ggccctcctc aaatcctgga
47101 ttagaacact gacctgggct tcacctcctt ccattcggtg aagaagggcg aggaatttta
47161 gccgcaacag cagcctgatt gtcggggaag gaggctctga taggaggcag gatcctcttc
47221 tgcccatcag aggcgcggtg gtctcccatc gatcgttgtg ggccggagca gggcatttga
47281 tcagtggctg ggccggcgct aagccccact tcaccttctg tgcccttcag accggccagt
47341 ggtggccgaa cttgctgcct cgccccctgg aggcgtgcgc ccaggaggaa acttcacgtt
47401 gacctgccgc gcggaggcct ggcctccagc ccagatcagc tggcgcgcgc ccccgggggc
47461 cctcaacatc ggcctgtcga gcaacaacag cacactgagc gtggcaggcg ccatgggaag
47521 ccacggcggc gagtacgagt gcgcaSccac caacgcgcac gggcgccacg cgcggcgcat
47581 cacggtgcgc gtggccggta agtggcagct ggggagaggc ggggcgaggt atctgagagg
47641 gggcgtgacc tgggtcttgg ggcggccggc cccgcctgcc tccctctcgg tcccggKaga
47701 ctagacggaa gtgggacaga gtagaagtca aaggtgcctt agcgggtggg gctgttgatc
47761 gcactttgag gggtgggaga tggaggtggc agggggactg aattcaaggg gagggaccct
47821 ccggggctgt cactgctgca ggaaaggact ttaaaacttg ggctggattc tgcgtggtgg
47881 aaacttgacc caattcacaa tccactgtgg ggaagattgg ggagggacca aagtcattgg
47941 atgWtggagg gagagagggg tcaagatcgc cttctctcac ttcctgaagc tcctggggct
48001 gggttctctc tggggtcggg ggaacctgac ctggtcctgc cggtcctcat tttttggggg
48061 atggggagag ttgacttgtc gccagggggc taagtgtggt cacgggttta tatccccatg
48121 ggctgaagtg cgatataaat cgggactcgg gtcgccctgg cactgggagt ggccttattg
48181 catgggttgt ctcccttctc ccaaggccag acagtgagct ccccggggca tgaggactga
48241 cttcgtgcct gtgggagtgc ggggggcggt gggagaagcc ccccggagct tggccaccct
48301 ccgcgagggt ctccaccccg caggtccgtg gctatgggtc gccgtgggcg gcgcggcggg
48361 gggcgcggcg ctgctggccg cgggggccgg cctggccttc tacgtgcagt ccaccgcctg
48421 caagaagggc gagtacaacg tgcaggaggc cgagagctca ggcgaggccg tgtgtctgaa
48481 cggagcgggc ggcggcgctg gcggggcggc aggcgcggag ggcggacccS aggcggcggg
48541 gggcgcggcc gagtcgccgg cggagggcga ggtcttcgcc atacagctga catcggcgtg
48601 agcccgctcc cctctccccg cgggccgggg gacgcccccc agactcacac gggggcttat
48661 ttattgcttt atttatttac ttattcattt atttatgtat tcaactccaa gggcgtcacc
48721 cccatttcct acccatcccc tcaataaagt ttttataaag gaactccctg tctccgcttc
48781 tgtttctgca aggtggaaca agcccagggt tccagtcgtg acctcccgag ttattcattc

# FIGURE 1-N

48841 aaaaagcgat ttttgagagg gcctggggtg gtggcttagg cctgtaatcc caggacttgt
48901 gggaggccca ggtgggagga tcccttgcac ccaagagttt cagaccagcc tgggcaacat
48961 agggagaccc tacaaaaaat atttttcaaa aattagccaa ggccggtcgc ggtggctcat
49021 gcgtgtaatc ccaggacttt ggaaggccga ggcggccaga tcacgaggtc gaggtcatga
49081 gatcgagacc atcctggcca atatggtgaa accctgtctc tactaaaaat acaaaaatta
49141 gctgggcatg gtggtgcgcg cctgtagtcc cagctattcg ggaggctgag gcaggagaat
49201 cgctttaacc ccggaggcag aggttgcagt gagccgagat cgtgccactg aactccagcc
49261 tggcgacaga gcgagattcc gtcttaaaaa aaaaaccagg cgtggtggct cacgcctgta
49321 atcccagcac tttgggaggc cgaggcgggc ggatcacgag gtcgggagat cgagaccatc
49381 ctcgctaaca cggtgaaacc ccgtctctac taaaaataca aaaaaaaaaa aaaaaaaaaa
49441 attagccgag cgtggtggcg ggcgcctgta gtcccagcta ctcaggaggc tgaggcagga
49501 gaatggcatg aacccgggga gcagagcttg cagtgagccg agatcgcgcc actgcactcc
49561 accctggcag acagcaagac tccgtctcaa aaaaaaaaaa aaaaattagc cagggccgag
49621 cacagtactc tcgcttgaat cccagaactt tgggaaacta aggcgggggg ggggggtcac
49681 ttgaggtcag gagttcgaga ccaccctggc caacgtggtg aaacaccttc tctaccaaaa
49741 atacaaaaaa attaaggccg ggtgaagtgg ctcacgtctg taatcccagc actttgcgag
49801 gctgaggcgg gcggatcatg gggttagaag atcaagactg tcctggctaa catggtgaaa
49861 ccccgtcact actaaaaaca caaaaattag ccgggcgtgg tggcacatgc ctgtagtccc
49921 agctactcaa gaggctgagg caggagaatc gctttaaccc cggaggcaga ggttgcagtg
49981 agccaagatc gcaccactgc actccagcct gggcaataga gcgagactct gtctcaaaaa
50041 aaaaccaaaa acaaaaacaa aaaaatacaa aaatttagct gggctgtagt ggcacacgcc
50101 ctgtaatccc agctactcgt gaggctgagg caggagaatt gctagaaccc gggaggtaga
50161 gatcttgcca ttgcactcca gcctgggcaa cagaactaga ctctgtctca aaaaaaaaaa
50221 aaaaaaaaaa aaagtgtttt ttgagggggc tgggcatggt ggctcaagcc tgtaatccca
50281 ggacttttgg gaggcccagg tgggaggatc ccttgtgccc acgagtttca gaccagcctg
50341 ggcaacatag cgagacccta caaaaaaaca tgtttcaaaa aaaaattttt tttttgaga
50401 tggagtctcg ctctgtcgcc caggctggac agcagtggca ctatctctgc tcactgcaag
50461 ctccgtctcc cgggttcatg ccattctcct gcctcagcct cccaagtagc tgggattaca
50521 ggcgcctgcc accacaccca gctaattttt ttttgtattt ttagtagaga tgggatttca
50581 ccgtgttcgc caggatggtc tctattcctg acctcgagat ccacccgcct cagcctccca
50641 aagtgctggg attacaggcg tgagccaccg cgcccggcct caaaaaaaaa attttttttt
50701 tgagatggag tcttgctctg tcacccaggc tggagtgctg tggtgtgatc ttggctcact
50761 gcaacctctg cctcctgggt tcaagcaatt cccctgcctt ggcttcccaa atagctggga
50821 ctataggcat gcaccaccac acccagctaa tttttgtatt tttagtagag acagggtttc
50881 accatgttgg ccaggctggt ctcgaactcc tgacctaagg tgatccacct gcctcggtct
50941 cccaaagtgc tgggattaca gctgtaagcc accatgcctg gtgtattttt caaaaattag
51001 ccagacatgg tggtgcatgc ctgtagtccc agctacttgg gaggctgagg caggaggatc
51061 ctttgagcct aggaggtcaa agccacttcc agctatgata tYgtcattgt gctccagccc
51121 aggtgacaga accagaccct gtctctaaaa acaaacaacg aaaaaatcca aaaacttttt
51181 ttgaacacct actatgcatc aggcacaatc taagctgtct ttttcttttt cttttttttt
51241 tttttttgag acggagtctc tctgtcgccc aggctggagt gcagtggtgt aatctcagct
51301 cactgcaagc tctgcctccc acgttcaagc gattttcctg cctcagcctc ctgagtagct
51361 gggattacag gcgcgcgcca ccactacgcc cggctaattt ttgtattttt agtagagaca
51421 gggtttcacc atgttggtca ggctagtctg gaactactga cctcgtgatt cacctgcctc
51481 ggtctcccaa cgtgctggaa ttacaagcct gagccactgt gcctggcccg gctggctgtc
51541 ttttctcagt gtgacctaga gctcgtcctc tctcagtgcc tgttttctgc tctcttcaca
51601 cccttgggca ggggtggggg cctcactcct tcctcctact tatctcttcc acccagcact
51661 gccctctgat ccgggtcact cttggaggtg gggctaggt gcttcccca ggcctggtta
51721 ccggcagagc tgaggccgcc ttgccagggt gggtggaggc cctctccgtt gtgcccctgc
51781 cctgagactt cggtgagacc ttcctgggca ggcactggct gatgctatgg gtgtcagccc
51841 ttagcatgtc ctccctgtta aagggggcac ccctgccccg ccctcaatgc ctcctttgtc
51901 ctgtgtccac ccacagtgct tcagtttacc cccaccctgc ctttccactc agcccatcat
51961 gattcagaac agacacacac agggtatccg ctgcaacccc cactacacgg caccttcccc
52021 cctcatcccc aacagcataa ggcacagcag ctgggcctcc tccccacagc ctcctcctct
52081 gcccctcctc cctcaaaagc aggaacacgg agccctagag aaggaagcca gtcccatgca
52141 aacatgtaat gagcaaagat gagacgggga aatggcacaa gagggcaggc cagtgctcgg
52201 tgctggagcc aggggccagg tataacaccc aaaaaggcac cagccaggtg cagtccctgc
52261 cactaacagc acctcacctt ttctgcctgt gccctcccta cttaatgatc tctctccgct
52321 cctactgacc tcaaggtctc tttcagagcc ttcagatgag atatttattt tagtaggttg
52381 gtgcaaaagt aattgctgtt tttgccattg aaagtaatgg caggcagggc atggtggctc
52441 acgcctgtaa tcccagcact ttgggaggcc gaggtgggca gatcacgagg tcaggagttc
52501 aagaccagcc tggctaacat ggtgaaaccc tgtctctact aacaatacaa aaaaaattag
52561 ccggacatgg tggcacaccc ctgtagtctc agctacttgg gaggctgagg caggagaact

# FIGURE 1-O

```
52621  gcttgaaccc tgaagcggag gctgcagtga gctgaaatca cgccactgca ctccagcctg
52681  ggccacagag tgagactgtc tcagaggaaa aaaaaaaaaa aaaagtaatg gcaaaaactg
52741  caattacttt ttcttttct ttctgttttt tttttgttt gtttgtttgt ttgtttgttt
52801  gtttgagacg gagtctcact gtgtggccca ggctggagtg cagtggcgca atcttggctt
52861  actgcagcct ccgcctccca ggttcaagtg attctctcct gcctcagcct cccaatagga
52921  ttacaggcac ccaccactat gcctggctaa tttttgtatc tttagcagag atggggtttc
52981  accatgtttg ccaggctggt ctcgaactcc tgatccaccc acctcagctt cccaaagtgc
53041  tgggattaca ggcaggcatg agccatggtg cccgacattt tttttttttt ttcttttttt
53101  ttttgagaca gagtctcact ttgtcaccca ggctggagtg caaaggcaag atctcactgc
53161  agcctctgcc tcctgggtta aagcgattct cccaccttag ccccctgagt aggtgagatt
53221  acaggcacat gccaccacac ctgcctaatt tttgtatttt taatagagag ggggtttcac
53281  catttgtcc acgctggtct cgaactcctg acctcaaggg attcgcccac ctcagcctcc
53341  caaagtgctg ggattacagg catgagccac cacgcccgac ctccttaact cttttatttc
53401  cttgcacact ttttctctgt agcccagctc acaacttaac atcctcttag acatacacct
53461  gaggtttttt tttttttttt ttttgagagg gagtctctct ctgtcaccca ggatggaatg
53521  cagtggtgtg ctctcagctc actgcaacct tgcaaccact gcctcccagg ttcaagcaat
53581  tctcctgcct cagcctcccg agtagctggg actacagaca catgccgcca cacctggctg
53641  atttttgtt ttttagtaga gacagggttt caccgtgttg cccaggctgg tctcaaactc
53701  ctgagttcag gcagtccgcc tgccttagcc ttccaaaatg ctgggattac aggcatgagg
53761  caccgcgtcc tgcctctgag tttttttttt tttttttttt ttactgtctc ctagaacatc
53821  agccccagta gagcagggat ctttgttctg ttcaccactg agggtcctca ttgggtccta
53881  gcacacacag ttgctcaata aatgttgaat aagtgggtaa agacagccac gagcttgcag
53941  atatgtgttc aaggtgtgtc cttgcagaga gcttctctat acttggcact ggagaggcct
54001  gtcgtgggca aggacataga tgtggcgacc tcagacttga gaactcctgg ggcagtaggg
54061  gagatggatg tggataatgt aaccataagc cttcctttct atttagact gagtggtcaa
54121  ggaaggcttc cagacaatgg aaattctgat aggttctaag agggagacca gcaaggagct
54181  gaaccggagg ctgacacagg gtgagagtgg gaatgtctat tttatttttt ttttggagac
54241  agggtctcac tctgttgctc aggcagcagt gcagtggcac gatcgtggct cactgcagct
54301  tcaacttccc agactcaagt gatcctccca cctcagcctc ccaagcagct gggaatatag
54361  gtgcatgcca ccacatccgg ctaattttg tatttttggt agaaacgggg tttcaccatg
54421  ttgcccaggc tggtcttgaa ctcctgggtt caagtgatct tgctgccttg ggctcccaaa
54481  gtgcttggat tacaggtgtg agcctgtgag ccacatctgg cctattttat tttttaatta
54541  gtttttgtt tttgtttttg ttttgagatg gagtctctgt ctccaaggct ggagtgcagt
54601  ggcacgatct cggctcactg taacctccac ctcccaagta gctgggatta caggcacatg
54661  ccaccacgcc tggctaattt ttgtatcttt agtagagaca gggtttcccc atgttggcca
54721  ggctggtctc gaactcctga cctccggtga tccacctgcc tcagtttccc aaagtgctgg
54781  gattatagga atgagccact ctgcctggct gggcatgtgt cttgtttttt tcattctgta
54841  gataacaagc aattgtcctc cctcagcctc ccaagtagct ggtactacag gcgcctgcca
54901  ctacgcccgg ctatttttg tagttttagc agaggtgggg tttcaccatg ttaaccagtc
54961  tctaactcct gacctcaggt gatcctcctg ccttggcctc ccaaagtgct ggaattacag
55021  gtgtgagcca ccaagcccag cctcaaactc ctgagtttaa gtgatcctca cacctcagcc
55081  tccctgagtg ctgggattac aggtgtgagc caccacaccc ggcctgagtc gggggaggtg
55141  tctattttag tctgagtggt caaggaaggc ttctctgaag agctgatgtt ggggccaggc
55201  ctcctcaagc gattagaagg tgtcagccat ggggagatgt ggagggaggg tgtttcaggc
55261  caaggaaggg taagatcaaa taattcaggg atctgagggc agaggaatct ggactccagt
55321  tctcccattc agggctgccc aggagagaca aagaggatct cataagggtg tgtggctttg
55381  tggctcactc acgcccacag caaggaccat gcccacccta cttttttttt tttccccaag
55441  tctccctcta ttacccaggc tggagtacag tggtacgatc acggttcact gcagtcttga
55501  cctccagggc tcaagtgatc ctcccacctc agtgtcctga gtagctggca ctacaggcat
55561  gcaccaccac ccctggctca tttttgatt ttttggtaga gacggggtct cactgtgtta
55621  cccaggctgg tctcaaactc ctgggctcaa gcgatcctcc cacctctgcc ttctgaagtg
55681  ttgggattac aggtgtgagc caccatgccc agcccgccac cagtcttgac tctccatcct
55741  ccctccctcc ctgtgagtgc tggtggcctg tgtgtcccgc ctgctgaaag atgcctctga
55801  cttggggctt ttgacttcag gcccgggaga cacatttgg ctggattagg gagatcttaa
55861  aaggggtag gggacacagc aggggctaag gagagtgaca cactgcaggc aggggttggg
55921  tgacagatga aggatccact ggtatatgct gccagtgtgt gccatctact tgtgtgtttt
55981  acgtgtgtct ggtgcatgtg tcctgtgtgg gctgtatctg tgtgtgactc agctacgtgt
56041  cagcatatcc ctgtcgacgt gtgtctctgc tgatgtgtct gtcagcacgc acggctgtct
56101  tgcatgtgtc tttttgaga tagagtctcg ctctgtcacc caggctggag tgcagtggca
56161  cgatcttggc tcattgcaac ctcaacctcc caggttcaag cgattctcct gcctgagcct
56221  cccaagtagc tcggattaca ggcacatgct accacgcctg gctaattttt gcatatttag
56281  tagagacggg gtttcatcat gttggccagg ctggtctcga tctcctgacc tcaggtgatc
56341  cacctgcatc agcctcccaa agtgctggga ttacagaaat gagccactgt gcctggctag
```

# FIGURE 1-P

56401 acatgtgtct ttttttttcc cttctgtata taacagggtc tcactatgtt gcccaggctg
56461 gtctcaaact cgtggcctca agcaatcctc ctgcctcagc ctcccaaagt gttgggatta
56521 caggcgtgag ccactgcacc cggcttgtgt gtgtctttct gtatctgttg tgtgtgtctg
56581 tgggtagtgg ctgtttccct gcatgggaca ttgtggtaaa tgtggctaat tccctgtgtg
56641 ggggctgctg cctgtggata agactgtgtc tctgtgcatg cgcacgtgtg tgcacgcccc
56701 tcacaacccc aacgagaaaa cacctgtcca ccttcctggg ctggcacagg ggcacaggag
56761 gcgggatccc aaatcacagg ctttttctct cggcatatct ctgtacttta ttgtccctgc
56821 tgtaacaatg ctcatccttc ctgagagcgt ctcctgaggg ggcctcggcc aaggctgact
56881 ggagaagggg ctggtgaccc ctagcaggct ctgccacctg aggcctgggt cttcccccgg
56941 gatccttgaa tctggagatg gcagagagga ggcaggccgg ctctttcccc aatcctccta
57001 ggagagctgc ttctgcccat tctcccactg gtgaaacgga ggcagaagca gcagctcagc
57061 agggtgaagc tgggtttaac cttcctgaaY ggggtccagg gacactccac atctgccaca
57121 tagcctcttg ggctggacat ttttcctggg caccagccag cagctggagc agcgagtgcc
57181 gtatttcttg tacctagggt tgggggacaa gaaactgcca tttaggatgc agtgggggcc
57241 cggaaaccgc cacaaggaaa ccactttcc caagaggtag gtgttttgct ttttgctttc
57301 cccacaggcc atcctggtta cacgtggact gatttgggga cccccgcccc aactccctcc
57361 tccattctaa ggacctgatc ccacaggcgt tgcagagagg ggtcccatct tcagcgtctc
57421 tccagagcgg ggtcctctgg gtccgacagg aagcacagcg ccggggctct gaagggtcag
57481 aggtcaaagg gcaggggtca gaggccaagc atgtgagctc gggatgcctg tgcggagtcg
57541 ggcattttgt gggggtctca gtaaaggccc cacctagctc tggatcagcc ctgggagttg
57601 ccagctctaa gccacagcaa agccaggaag ggagacagat ggcagcattc cgcagaggag
57661 gaagctgggg gtgggggtga ctcagcccaa gtggaggggg gtgctgcgac tcctccctga
57721 gggctctaaa tggggaagca agatggagaa gggggggca gggagaaagg cagggaagac
57781 aggaaattgg cccccaaaat atttatagct cttgggtttt caggactcac ccagggcctc
57841 gctgcctgct gagtgggcct cggtgcctcc tgggtgggct gcagggcccc caacagcatc
57901 tgcaggggat tcctgagaac ggctactgca gggcaggctg tggggcagac aaggtattag
57961 cactgggggg gatctgtagc ttgtccccaa cagccctccg aaatgaagat ttagtatcaa
58021 ggtatcagcc atcccccgag gaggcaacta atatctgaat ggcctggctt tgcctctcat
58081 tagtaatatt attattatta ttattattat ttttgcagat ggagtctcgc tctgttgccc
58141 aggctggagt gtagtggtgt catctcagct cactacaacc tccacctccc gggttcaagc
58201 aattcttgag cctcagcctc ccaagcagct gggactacag gcgcgcgaca ccacgcccag
58261 ctaatttttg tatttttagt acagacgggg tttcaccatg ttggccaggc tggtctcgaa
58321 ctcctgacct caagtgatcc acccacctcg gcctcccaaa gtgcagggat tacaggcatg
58381 agctaccgtg cccgacctaa ttattattat tattatttga aaaatagtaa gcacagggac
58441 agcctgccag gttccaatcc cagttctcca tgtccttgct gtgtgagcct ggacacatta
58501 tctcctactc tgtgcctcag tttcctcatc tgtaaaatgg gcttcccaat acaacagttt
58561 tttttctttt gaaaatttga tttattgatt tttaaaaaga gatgggagtc tgggcaatat
58621 agggagaccc cgtctctaca aacaaaaaaa atagccagga gtggtggtac aggcctgcac
58681 tcccagctag ttgggaggct gaggagggag gatggcttgg gcctgggagg tccaggctgc
58741 agtgagccat gattgcgcca ctgcactcca ccctgggtga cagagcaaga ccctgtctca
58801 aaaacaaaca aacaaacaaa agcaaaaaaa agcagccagg catggtggcg gctcactcct
58861 gtaatcccaa cactttggga gggcaaggca gatggatcac ctgaggtcaa gagatcgaga
58921 ccatcccggc caacatggca aaaccccatc tctactaaaa atacaaaaat tagccaggcg
58981 tggtagcagg cacctgtaat cccagctact ccagaggctg aggcaggaga atcgtttgaa
59041 tccgggaggc agaggttgca gtgagccgag attgtgtcat tgcactccag cctgaacgac
59101 agagtgcaac tccatctcaa aaaaaaaaaa aaatattgtt ttggcggcat gattaaaaaa
59161 gtcataatta taatttttat aataacaata aataggtaaa aattagagct gggggtcata
59221 ctatgttgcc caggctggtc ttgaactcct gggctcatgc aatcctccca ccttggcctc
59281 ccaaagtgct gggattacag gtgtgagcca ccaggcccag cttcccaaag cttttttttt
59341 ttttttttta aggggatgga gtcttgctct gtcacccagg ctggagtgca gtggtgccat
59401 ctctgctcag tgcaacctcc acctcctggg ttcaagcgat tctcctgcct cagcctccca
59461 agtagctggg attacaggtg cccattacca ttcccagcta gttttttttt tttttttgag
59521 actgagtccc actctgtggc tcaggctgga gtgaggtggc gctatctctg cttactgaag
59581 acttcacctc ctgagttcaa gcgattctcc tgcctcagcc tcctgagcag ctgggattac
59641 aggcacccac tacaacgccc agctaatttt ttgtattttt agtagacatg ggtttcact
59701 atgttggcca ggctgatctc aaactcctga cttcaggtga tctgctcgcc ttggcctccc
59761 aaagtgatgg gattataggc gtgagccact gtgcccggcc tccccaggct tttgaatgag
59821 cccatacaca taaaatactg cctgttaacc tcttattgtt gtcactacca acaaatagta
59881 gctgttagca ttattgggaa aaatcaccgc agccgcagct gcagcctaag caaggccagg
59941 cctgattcct aaagagacca ggtctcacat tgggagtggg gagccactga gtcaggagcg
60001 gcaggactgt atctggcacc agctcaagtt gtcaaccaga gactgggcca cgtgtaacgt
60061 agagcacagg tgagggtcca caccgacgcc agRgagcacc cctacctccc acactggtgt
60121 ttgcgttgta actgggagaa agggcggcct ggccccagcc ggcccctacc tgtacgtggg

# FIGURE 1-Q

60181 gatgatctgc aggctggagt ccggctttat ctgaaacttc agagtcaccc cctcgaaccc
60241 agggtccact ctctcggtgc cccggcaggg gttcagctgt ttccggggcc ttctctgggg
60301 tggggccgag ggagtccccg ggggctgcag acggcggccc ttttggctga tcctggtctg
60361 tgtgtccttg gaatccctgt ccagcagcag gcggccctga gtccccagag ccatcgggtc
60421 ccagcaaggc cccaggacYg gggtgtcctg ggcaggggac tgacccagcc tctccactgt
60481 ctcttggagg aagcacaggg cggtgaccga ctcctggcat gcaggccaga gggacctggg
60541 gagaagggca gtggggtcac gttttcttgg gtgactcctg ggtttagccg gccagggggt
60601 tccaacttgg gccggctctt tgtgtaccct tggacaggct acacacccca cctgaggttc
60661 agtcatttct aactctgggt ggaaggttta agagttgcag taattgcaac tcacctcgcc
60721 cactgtgggc ctcccaggca cgcccagccc tccacatcaa tcaacccctt acggcacccg
60781 tagatcaccc aggcagggac cgccctaggt gaccgaggca ggatatcaag cctctggctg
60841 caggatcgag aaaaaggtta cttttttct ttgtgtttct tttagagacc gagcctcact
60901 ctgtaccccc gcctggagtg caggggcgtg atcaggactc agtgcagcct agaactcctg
60961 ggctcgaccg atccttccgc ctcagcctcc cgagtagctg ggacttacag gcacgcgcca
61021 ccacgccgct ggcttgccat aatatgatta cgatttttat cattttcatt attattgagt
61081 tgccctccat aacccctgga tgatccctgg ggagtgccta catcagcccc agagacatgg
61141 aattgcatgg cctctgatcc ctagatgacc ccaagtgtct ctgggcacca gaacctcttt
61201 gtaagtttca gtaactatta taaaattgcc acttctcgat aaccccagca aagagaggtc
61261 atgacccctt gatcactgag ccaggtggtg aagtgctagc ggggtccggg gaatggggtt
61321 tggccttacg cacctctcac tgattctgca gcatcctggg gtcctcggct tctcctggga
61381 gggggatcct cccagccgct ccaggttcag acagggcggc gccagaagct ttgctggcat
61441 tgaggagtcc gggattggct cggcctccat tgcgccccag cccagccccc tccctaattt
61501 cacccggccc tgtcccgctg ggcccacccg atgaaaccca cctgggaggg gcgggacctg
61561 tgtggtggga gcgctcttgg tagaggatgc gatgagcccc agctgagagc gaccagcctc
61621 ggcggcacct ttcctctttc tggctttttt caggtcatcc gcgcacccct gcgtggcaag
61681 ggcggcctgg accccaccaa atccctccgc agctgccggg gcggggcctt ccacgctagc
61741 cctccccctc ggaaatgggg gctcccaggc ctctgccttg gatccccagc ggcatcttag
61801 atcccagagg gaaaagagaa ccgggggccc agcacagcag atgcaggtgt ggtttgtttt
61861 tgtttttgtt tttttttttt ttgagacagg gcctcactct gtcacccagg ctggagtgga
61921 atggcctgat caccgctcca ctgcagccta aatcccctgg gctcaagtga tcctcccacc
61981 tcagtctccc gagtagctgg gactacaggc acttaccacc actccacgct aatttttact
62041 gtttttatag agacgaggtc tcgccatgtt gcccaggatg gtcttaagct cctggcctca
62101 agtgatcctc ccatcttggc ttcccaaagt gctaggatta caggtgtgag ccacctgtag
62161 accccagatc tgtcccccaa ggacactcaa catgctgggg cctgtgttat cgatttattg
62221 cagctccaat atgagtccac tcctactcaa accctgattc tcaggggcct ggggaaggcc
62281 accccactag gggccctgtc cccaccagag cctggacatg ggactctctc ccaagcaggg
62341 gtgttgtcct tcacaggggc ttccacgtct ctcccggccc tgtccgcact ctgggcaggg
62401 ctggcacctg gctattccct caatctgggc cctggggcca tggcaatgtg gaatggtcca
62461 ggtgttcatg tcagtggggc ttggggacat ggccatccac gtagaagttc ctggtgatct
62521 tgggcagggt gaattccgct ccttccagct ccggtgtcag cacaatctgg cagcccagcc
62581 gcgagttctc ctggaggagg ggggccatgt ctagcatgtc gtcttcccta gggtggtgac
62641 acgggcagtg ttagccgagg gcaagctagc tgggtgggtg gggggccaga ggtSggggag
62701 ggaggaagct gactgagcgc agggggtagt ggtgggggag gcagctccca ttccaggaac
62761 tggaagtggg gaggatggtg ggggctgctg aatgctccag ggtgaagctg ccagctagac
62821 agggctgacc cactcacctc tcctcgggag gaggcaggag atccaggtgg tcttcactca
62881 catacacatg gcaggtggag caggccaggg aggcttcaca ggcccctagg ggtgggaagt
62941 gaaggggggcg caggtgagtg gcagagtcag gaaggggctg ctatgcgaat ggcgtagagg
63001 agtagacctc tcttccacca cgtgcctcac gtctccctcc tgggcctgca gctccactgt
63061 gtgtaccagg cactgtgtca tgtcctcaat gtcctcagct agctgttccc cagtgtgtag
63121 ggtttacaaa cggacatctg ggaggcccca tttcccaggg actccctagc aggctgagat
63181 gcagaaccat taaggagcag ggacttggct gccaggtagc tgagagggga aaggccaggc
63241 aggctccccg cagtctgtga gcccttgctg tgaccctatc actgtgctgg gctaatgtgt
63301 ccccacctct ccactgctct tggaggatgg accggctgac tcagcagtca gtgtggtggg
63361 ggaaggaatc aggcattagg atttgtttt tttttttttt gagatggagt cttgttctgt
63421 tgcccaggct ggagtgcagt ggcacgatct cggctcactg caagctctgc ctcccgggtt
63481 cacgccattc tcctgcctca gcctcccaag tagctgggac tacaggtgcc tgccaccacg
63541 cctggctaat tttttatat tttttagta gagctggggt ttcaccatgt tagccaggat
63601 ggtcttgatc tcctgacctc atgatctgcc cgcctcagct tcccaaagtg ctgggattac
63661 aggcgtaagc cactgcaccc ggccaggatt tttttttttt ttttttgaga cagggtctca
63721 ctctgttgcc cagcctggag tgccatggct cagtcatagc ttactgcctc aacctcccgg
63781 ctcaagcgat cctcccaccc cagcctcccg agtagctggg actacatgtg cgcaccacca
63841 cacctggcta atttttttt tttttttttt ttgaggtgga gtttcgctct ctttgcccag
63901 gctggagtgc aatggtatga tctcggctca atgcaacctc tgcctcccag gttcaagtga

# FIGURE 1-R

63961 ttctcctgcc tcagcctccc aagtagctgg gattacaggt gcccaccatc acgcccagct
64021 aattttgtat ttttagtaga gatggggttt tgccatgctg gccaggctgg tcttgaactc
64081 ctgacctcag gtgatccacc tatctcggcc tccctaagtg ctgggattac aggtgtgagc
64141 caccgcacct ggccatattt tttttttttt tttttttttt tgtagagacg aggttcacca
64201 tgtggcccag gctggtctca aactcctaag ctcaagcaat ctgcctgcct tggcttccca
64261 aaatgctggg actacaggca tatgccactg caccagacca ggaattaggg ttgaaagaga
64321 cagaacctga agtttctgc tgaccactg cccttggtca cacccccaca tcctctgatc
64381 acagagaccg gtgcttgggg atcatgtccc tcctggccta aaactttcca aagggtttc
64441 acactcagaa taaaatccaa actccttcac ttagtcttgc agttccccca tcccctcat
64501 tcattccttt gtttcctttt gtaatttttt tcttagagat ggagtcccgc tctgttgccc
64561 aggctggact gcagtggcgt gatctcggct cactacaagc tccgcttccc ggattcacgc
64621 cattctcctg cctccgcctc ccagagcagc tgggactaca ggcgcctgcc accatgcctg
64681 gctaatttg tttttgtatt tttagtagag acagggtttc accatgttag ccaggatggt
64741 cttgatctcc tgacctcgtg atccgctcgc ctcagcctcc caaagtgctg ggattacagg
64801 cgtgagccac cacaccttgc caggccactt acctctacta gcccggcaca gtggctcatg
64861 tctgtaatcc cagcactttg ggaggccaag gcaggcagat caactgtggt caggagtttg
64921 agaccaccct ggccaacatg gcgaaacccc atctctatta aaaatacaaa aacttagccg
64981 ggcatggtgg cagttgcctg taatcccagc tactcgggag gctaaggcag gagaatcact
65041 tgaaccctgg aggtagaggt tgcagtaagc caagatcatg ccattgcagt ccagcctggg
65101 tgatagagcg agactctgtc tcagataaat aaatgaacaa ataaatctat ctcacattcc
65161 aatccctgtc ctagactatt ggctgctgga ttccagaaca gaactttagg taggcctttc
65221 ataacccctc ccaacatatc ttccccttta atgataaaat caggatccct gatatattca
65281 tcccaaaagc aaaccctata tattttcttc cagacaaccc ttcccaagtt ggtcataatt
65341 atgtatttac ctgtgggatt attgtcccta ttagaccgaa atggatgtga gatgacagag
65401 gccttatttg ttttgtatat tgctgcctga cgcgcagtag gtattcatag aaaggcagca
65461 cagattagtg gctacaatgg actggtgagt ctagtattgt cactcacttg cccttagacc
65521 aggtctactt aacctggggt ttagttgcct catctataat agggaataac agtctctact
65581 actcaagttg tcatgaagat tcagtcagtg aatatttatt aacatggtta aagacatata
65641 tatatatata tatatatttt tttttttttt tttttttttt tttttttttg agacagtctc
65701 actctgtcgc ccaggctgga gtgcagtggc gtgatcttgg ctcactgcaa gctccacctc
65761 ccgggttcat gccattctcc tgcctcagcc tcccgagtag ctgggactac aggcgcccac
65821 caccacgccc acctaatttt ttgtattttt attagagacg gggtttcacc attttagcca
65881 ggatggtctc tatctcctga cctcgtgatc cgcccgcctc ggcctcccaa agtgctggga
65941 ttacaggcgt gagccaccgc gcctggcctt tttttttgt ttgtttgaga cagagtcccg
66001 ctctgtcatg cagcctggag tgcagtggtg ccatcttggc tcactgcaac ctctgcctcc
66061 caagttcaag tgattcttgt gcctcagcct cccaagtagc tgggattata tgcatgtgcc
66121 accacgccca gctaattttt gtgtttcttg tagagacagg gtttcgccat gttggccaga
66181 ctagtctcaa actcctgtcc tcaagtgagt cgcctgcctc ggcctcccaa agtgttggga
66241 ttacaggcat gagccactgY gcctggacaa gaaatttcta ttattatttt tctctcaaca
66301 aattatcaaa catctgttac atcttgacag gcaacagata agcatgaata aggacatggg
66361 tcaccaatct cactggggga cagatgacaa agtaggaaac aaaacagcct ggcattgtgg
66421 cttgtgccta taatcccagc tacttgggag gctgaggtgg gaggatcact tgaacccagg
66481 agttcaaggc tgcagtgagg tatgattgca tcatggcact ccagcattgg cgacacagca
66541 aggctccgtc tcttgaaaaa aaaaaagtga gaagtgctat atatcatatc acactgataa
66601 gacagagtaa ctagggcatg ggtacttcag ctgggctgag gggaatggga ttgttaggga
66661 catgacattt acactgaggt ctgaaggatg agaaagagcc agaatgtttg ctgggaaagc
66721 acttcagaca gagggaacag caaatgtaaa ggccccgaag caggaatgag cttggttgtt
66781 tgaggaacca caagaaagcc agtgtgggct gggcgcggtg gctcatgcct gtaatcccag
66841 catttgggga ggctgaggcg ggtggatcac ctttcaggag tttgagacca gcctggccaa
66901 tgtggtgaaa ccccgtctct actaaaaata caaaaattag ctgggcttgg tggtgggcgc
66961 ctgtaatccc agctagtctg gaggctgggg caggagaaac gcttgaaccc gggaggtgga
67021 ggttgcagtg agctgaggtg cagtaagcca ttgcactcca gcctgggcaa caagagtgaa
67081 actccatctc aaaacaacaa caacaaaaag aaagccaatg tggctagaat acagggagga
67141 agggggaaga gaacacaaaa tcctgggagc caggagtcct agtgatctct aataaatgct
67201 taacaaataa gagcttggca acagatgagg ccatggtgag gggtttggat tttagtccat
67261 atttggtggg aaaaagacgt gaagagacac acctRatttg tggKtttttt tttttttttt
67321 tttggacaaa tgtataagga cctccttaat tccctctgcc cggttcctac cttccaggtc
67381 caccccgtgg cgctgggcca ggtgaagaac attgtccccg actctgccac tcactgggat
67441 ccgctggcct gagcggtcta cgaacaccac gttcaccctg gggacagatg gaagtgcgaa
67501 tgccgaactg gatgatgggc tcagggcccg ggtagaatct agggttagga agtaggaatt
67561 gaggcttgac gcacaggtac tgaatagggc aaagcagaag ggggacctcg agatgaagct
67621 acagggcaag atggggacca gaggaattcc gtgggatgag gaatgggggga cttggccccc
67681 tgcactcaca cgtccccggg ccgctccggg ccgcccgcgt cctcctctcc Mgccgggcgc

# FIGURE 1-S

67741 gagcctggaa aacacggttc ggtgagcggc tgcgccgagc cccgccccgc cggcatctga
67801 cggattaacg ctgcccgccc cgggcgcccc aggtacctgt cgcttgaaac tttctggttg
67861 tccccagcgc cacccccctcc cccgacccgg aagtgccccc aggtctgttc caccaggtgc
67921 ccctggcagc ctgcagtaga accctggcac tcacgcctcc ccgggccatg gaggcggcca
67981 tgacatgcat cacgtgactc accgactgag catgcgccgc gccagggagg cgagggaaag
68041 cccataatca atggaacata agcgcagatg tttgcttagg gcctgcggtg accaggtggg
68101 aagagcaggg cggaatccat ctcaggtaaa cccgcggggc catcccagac ctcccactca
68161 agcccactgt ttgtcccaag aaccagccaa ctctgatttc tttaaaacca tttacttaca
68221 aactttaatt cagcaaaggt ccgtgtgggg agactggggt ggggtcgggg gaatagtccc
68281 cttggagtgg atgtggaccc ccagagtcaa gggagggaag cttggtggccc agttggctgg
68341 gggcaaggcc cagggtcacc tcaggtcgac aggtcctgct ggtgggcggg cccagagttt
68401 atcttcatgg agtgctggtt tctggcactg ggctggaagg aggccagctc cagggatctg
68461 gccgggggtg ggcaggcaga attcaagaat tcatcttcaa caagcgagtg acagcagagg
68521 ctccgggaga tgggcacaat gtccgactcc cacaKacaga cagcagggga ctggcagaga
68581 aagcccatct ctgcacggag gcccgggtag gagggggtgg tggggccggt tcgccaagat
68641 gaaggctttc cccttctact gtccccaggg tggagatcct gggtagggtg gcccaatccc
68701 taggccagag ctgtttggtc catagtcaag ctcccagagc ttggcatctg tggctctggc
68761 cagcagggcc tggggcccag cttttaaggc atcagaaggg gaggggctg cggcagggac
68821 cccgggcccc acggctgggg tataggccag atgggcaggc aggggcagga gacagccatg
68881 cctgcagcaa atgtgggaaa aacagctgtt tcaaaccgca aggtgtggaa tggtggcccg
68941 gacaggccgg gccttgaagg aatcagagct gggggctgtc cggggtggtt tgaaaaataa
69001 aacttagaaa aggaaacaga agtcagttgt caaagttaaa aaaaaaggag acagtctctg
69061 tatcttcacg ggaggtcagg gaaacctcca aggcactcga aaggccaaaa ttacaggagc
69121 aatgaggcag gagggctccg gagagacggg cacagcggag gaggagatgg ggggagggag
69181 ggagagcagg ccggggcccc tctctcttaa ggctgcaggg tttcagcgtg gggagcaagc
69241 cagagacata atgaggcctc cagactcccc cacacccctt gggctcctgg ggctccggct
69301 gattggtcag taaagtcttt cagagatttt tctattaccg aaagagagaa aatggtttaa
69361 aaaaaacaca aaacaaaaca tcagaaaacc caaaagcgat ttggtgcagg cccttgagtt
69421 atctctggtg ccagccactt agaaaatcct cttccgcttc aggtaggagt ccgcgtagtg
69481 gccgccgagg ccctgggagt gctggcccac atagctgcct tctgggctgg gctcgggcga
69541 gggcagcagg tgggcaaagc tgcgtttctg gccgcccagt ggggtctgga gacagagggc
69601 agggcggggc gggtcagggg ccgctggggg gccggggctt cccagccctg catgtcccca
69661 cccccctgccc gtaccttcag caggtggctg tgaccattag gcccggggct gaggcccagg
69721 agcccttctc cggaccccag cggggaagag ccgattgcct gggagaaatg gagaggtgga
69781 acaggtcact ccctgggggc cagaggaagc acccaccccg cccccctgcc actgcccccc
69841 agccctgagg gaaagcgaac agtttcaggt tcaaatcttg cttctgctac tttctagcta
69901 taagacctag ggccaattgc tccattttat ttgagcctcg gttttctcat ctgtcagatg
69961 gggccagtag cacccacagc ctgggctgta gagagaggaa gtgacagaaa gcccggaatg
70021 ctggcagtgc taggtgcttg atgcagagtg gatggaggac aataggctgt tgctactcca
70081 ccctgagcaa gaccaacccc tgccaccacc ccagctttgg cctgggcacc gagggttaca
70141 gagccccggt ggcaggcacc cccaaatcct agctgtccca gggggcctgt gcttggccag
70201 agaggttcct caccttactg aggtggctct gcttgaggcc agcctgcagg ccgctggtga
70261 agtaggaagt gggcgagcct gaatagaagt gagacagggg gcccccgcca ctcccaccgc
70321 tgctccgttc gccgaagcca ctgggcgggg gggacatctg tagaagaagg gccggtggag
70381 tgaagcagac aattgtagta cccagccctt tgtatacaat cttccctcYg cctcccaggt
70441 tcaagcgatt ctcctgcctc agcctcccga gtagctggga ttacaggcat gcgccaccat
70501 gcccagctaa tttttgtat ttttagtaga gacggggttt ctccatgttg gtcagactgg
70561 tctcacactc acgacctcag gtgatccgcc cacctcggcc tcccaaagtg ctgggattac
70621 aggcgtgagc caccatgccc ggcagttctg ggctttgcaa agactagctt tggctaatag
70681 gactgaggag agctggcacg tgggtacccg cctcctcttc ctcctccagg aacccaggga
70741 tcatcaccgc acgaatgggc acacagaaac tgttccagcc tgtggacgcc aaatatgtga
70801 ggccacatag ctcactgaga cccaggtgag tgccggggac cgcccactgc cccatagagt
70861 ggcaagaagt aataMaatgc ctggcatggt atggtggctc atgcctgtga tcctagcact
70921 ttgggaggcc aaagtgggag gattgcttga gcccaggagt ttgaggccac agtgagctat
70981 catggagcca ctgcactcca gcctgggcaa cacagtgaga tcctgtctta aaaaaaccca
71041 caaagaacac acaaaataca aggtctgctg ttccaggcta ctaagtcatg gcacagcctg
71101 tcatgtagta aaagctgatt gctacacatg ccataccagg tggctcttac cttgtgtcgg
71161 ctgggtccgt gaggcccaag ggctggttcc cgtgggtggg agaagagccg ccgaggtccc
71221 atgtcctgaa tgaaagcggg agaagcacat tgggttggag tctgtcccag cctcacccct
71281 ggctgaactc catggcagag ctgtcatcag ggaagacagt ccccaccact ctgggaccag
71341 gaagaggcaa atttggggct ggatgcagct tggcagggat ggctgctgtg aagggggtg
71401 ccccccatgg gcctaagtgt ttggagtttc ccaggcaagc tgggaattcg agctgtgtgg
71461 actcttgcag ttttcctgtg ttggcgttag ttcctgttgt tatttgtttg aaacacagag

# FIGURE 1-T

71521 ggggccaaac atcacccagg tgacctgtgg gcggccagtt tgcaatttct ttcttttttt
71581 ttttaaacg gagttttgct cttgttgccc aggctggagt gtaatggcac aatcttggct
71641 caccacaacc tccgcctccc aggttcaagt gattctcctg cctcagcctc cctagtaact
71701 gggattacag gcatgtctac cacacctggc taattttgta ttttagtat aaaacataaa
71761 ttatatttta tattatatat gaaatatata ttttattata tattttataa tatataattt
71821 ctccatgttg gtcaggctgg tcttgaactc ttgacctcag gtgatctgcc cgcctcggcc
71881 tcccaaagtg ctgggattat aggcatgagc caccgtgcct ggcccagttt gcaatttctg
71941 acctgccaag gaggttggca tcatagaagg accaaatctc caggggcaca gacaggaatc
72001 aaaggtggga gagagggata gaaagggcaa gccctgtctc tggggagaga cagtgtggcc
72061 taagtcctct tgggagagaa agacagacgc aggatgacag acagactgag acccaagatc
72121 ctccagggct cgagaatgga cagacaggca ggtggaccgg aagcaggggg tgatgtgcag
72181 acagacggga aactttaagg gcatgaggct gggcgcggtg gctcttgcct gtaatcccag
72241 tactttggga ggctgaggcg ggtggctcac ttgaggtcag gagtttgaga ccagcctgga
72301 caacacaatg agactccgtc tctaccacaa atttaaaaat tagctgggca tggaggtgcR
72361 tgcctgtggt cccagctcct caggagactg aggtgtgagg atcgcttgag cccaagaggt
72421 tgaggctgca gtgagctatg atcgcaccac tgcactccag ccagggcagc agagggagac
72481 cctgtctcta aaaaaaaaaa aaaaaaaaag cagagaccat atctggcacg ttccctgccg
72541 cctccctagc acatggcctg gcatagtcaa tcctagagaa atatcagttt ggagaatgat
72601 ttggccttag gggacagaga gctgcagcca ggcactcacc gaggggtagt cgaagctgta
72661 gctgtcagac agtcctggtt cgggggcag gcgggcgctg ctgagggggc tgagcaggcg
72721 ggacttgagc tggaaggctt tgctgctgct gctgccacct ccagggggctg ggggttagt
72781 tgggggcccc tcagctgggc ggcggcttct cccggcgcct ccccagcccc gggcctcctt
72841 acccgccagg ttgctggccg ggagcaggct gtgtaggttc aggtagggat tcaggggaat
72901 ccggtagtcc ttgggggct cccccagcag tgagacctgt ggcgggaagg caggattcga
72961 gagggccccc gggagaggga accctgcgga acaggaggct ggtgtggtgc cgcgcgcagg
73021 gcagggcctt accccagggg gcgtcggcag aggcccactg tctttctgga ggcctctgag
73081 gccagagcct cggagcccca caggggcggg gggaggagtg agctgggccg ctgggggacc
73141 caagcccagg gcctcccggt cacccccagc agggccaagc acggatggca gcaggggtgg
73201 tggcttccct cgccggggcg gcagctccgg gggcaggccc cctcctaggg agggaaggag
73261 gatttcaggg gcccaggctc acaggacccc tctccccagg gccctccagc tcccacctgg
73321 accagggccc aggggtcaag agacaaatcc atgctggcca ctgagcagag ctcatccctg
73381 ggccctgagt ggagggcacc caactgatgg acaagggcgc tgggacccag aggttgggtc
73441 actcacccga ggccacgtag cagggaagga aggggaagtg gctgggggac accgtgtggg
73501 cagggtctgc gttacctgca ggcagctccc cgaggagggg gttggctggc tgggccccag
73561 gctggagggc gcccaggggt gagtctccca ggatgccggg cttctaggga cagagcacag
73621 acagttgcag atgggggcag ggtggcccag ggaagcagac aggtgtggcc cagagtcttg
73681 gcagacacac cccaactcca gggcacggcc gatcctgggc agacaggcac agggttcccc
73741 agtgcccgcc acactccaac tctatcactg tcccatcagc catggtgacc gagggtctcc
73801 tgagctccag gccccagttc cctggccaat gattgcagca cagtatcaac tgcagccaca
73861 tggaatggga cagccacaca gtgtttcagc cacttttagt cacagaaggc cagacccaca
73921 ggctcacata ttctgtcagt taccaccaga cagacacacc tgacttagaa tccagggaac
73981 cacaccagac aggacggagc atgggtgtat gtctatgtgt gtgtgtgtct gtgtgtgtgt
74041 gtgcgtgtat atctgtgtct ttgtttctgt gtgcatgagt ctgtcttgtg tctatatgtg
74101 tgtctgtgtc tatgtgtgtg tctttgtctt atgtctttgt gtgtctatgt ctctgtgtgt
74161 atatttgcat ctttgtatgt gtgagtctat atgtatacct gtgtttctag gtgtgcgtgt
74221 gtgtgtgYgc acacactgag gggatagacc tggagttttt tcccaggcat gtctatagag
74281 cttccgagca gagtctattc acaaactggt gcccagcagc ggcgctccca gcccacaccc
74341 agtttcaggc tgtaaatggg cgtgtccagg tcccctgacc ccaccccagg caccaagtcc
74401 aggggcgctc tcagaatgga gggaaagatg gcgaaccatc atgagcaatt gccagagggg
74461 gatggggatg tgggtgggaa aggcaggggt ttgggggagt cgccctcacc ctggttctgc
74521 gcccccaggg cacagccaga ggtcccccca ccccaccgat cgcaccagct gtgttgaaaa
74581 gagcctgtag gggctgtaga gcagtgggtg aggggatgc gtgtgttacc ttctggccct
74641 gggtctgcag ggcgagctgc aacagcgccg tggacagggc tggcccattg agcagcggca
74701 tggctggggg ggcacccagg aggcctggag ggagacatag gaggatgtgt gggggtccct
74761 gtgtcctccc tgccccacct cacgaaacac agccctgaac ccattgatgg ggcaggaaac
74821 tgacactctg gggccggggg gtgggcagtg gacttgccca aggaagggc ctagaagggg
74881 cagaggctac tccagtgaag gtcagccccc tcttgtcagc accccacctc actgcaggct
74941 gctgtgtggc cctggtcccg gcccccagtt gtggacagca gcagcccccg ctatgactcc
75001 atactccccc tcggccctac tcctggggct cctgtcactg cagccaagat gagcagtttc
75061 gggaagtgcc gggggcagcg ctgcagcccc tgttctgcac agtggggccc ctgtccagcc
75121 ccaccttacc ctgcttcccc cccgcactgc catggagcag ggggttgagc agcagctgga
75181 gggacgcgga tgggcccagg ttgttgagca gctgcaggat gttgggctcg ggaggagtc
75241 ccttcccccg attgagggcc tgcaggaagg aacagaagca gctcagagtg ccgctggggc

# FIGURE 1-U

```
75301 cctgacatcc ccatggggct cccatcccca ccccgccacc tctgcccgca caggctcacc
75361 gtggcctggg cagcgatgag agcggccagc atactgcggc cgggggggcc aggggcgcag
75421 aaggagactc gcaggtggct gccccccagg gacagggcgt ccgcctgctg ctgtgcctcc
75481 tccgccatct cagccgtctc atactccagc accgcgaagc ccttcagctg cccatcctgg
75541 ccgcacgcca gctgccggag gaaggcaggc agtcatgagc atctgggcag cagtgactgc
75601 accaccccgg gaagtgacaa ccgtaaccag actcagctgc cccataaggg tgaactccac
75661 gccaggcctc cactgccctg tgcctcattc ctgcatcagc tcatctgatt ttcccaatga
75721 ccctgggagg ctgcatcata cccatttctc agagaaggca actgaggcac ggagaggtca
75781 gcgcacccaa ggccacacag ctggagggag ggcggagctg ggagccacct caccaacaaa
75841 gccaaggcca cgggcggatg cggtggctca cacctgtaat cccagcactt tgggaggccg
75901 aggcgggcag ataacctgag gtcgggagtt cgagaccagc ctgaccaaca tggagaaacc
75961 ccgtctctac taaaaataca aaattagcca ggcgtggtgg cacatgcctg tattcccagc
76021 tacttgggag gctgaggcag aggcaagaga gtcacttgaa cctgtgaggc agaggttgcg
76081 gtgggccgag atcgcgccac tgcactccag cctgggcaac aagagcaaaa ctccatctca
76141 aaaaaaaaaa aaaaaaagag ataatcagca acgggtgggg aaggaagggc tctcgttcca
76201 gacagtggcc aagccaggta gcagtgctaa gggaagtggt tgggctgggc accggcactt
76261 gctgaatgcc cgggcagcaa aggtgcaggt agcagccctt cacctctgcc cacgccctgt
76321 gccaaggtct gggcacagat ctcccataac caaacctgtc ggcttttgtt ttgttttgtt
76381 tttttgaga cagagtcttg ctctgtcacc ccggagtgca gtgatgtgaa cttagcttac
76441 cgtaacctcc acctcctggg ttcaagtgat tctcctgcct cagcctcctg gtagctggga
76501 ttacaggtgt gcaccaccac gcccagctaa tttttgtatt tttagtagaa atgaggtttc
76561 accatgtcgg caaggctggt cttgaactcc tgacctcaag cgatccgcct gccttggcct
76621 cccaaagtgc tggattacag gcgtgagcca ctgtgcccgg ccataaacac acccatctta
76681 gggatgaaca gagagagact cagaggggtc acagagtgat caaggccaca caattcctaa
76741 gtcgagggac tgatgtgtga acctcaagct gcttcactgt caagcccacc ctccattcag
76801 cYgggtcacc taacattaaa ggtgggctcc tcggaggaca tcacttcaac gcctgctact
76861 catcagatgc tcaacaaatg tttggtgaga ccaggcccag tggctcgaat ctataatccc
76921 agctctttgg gaggcaaagg caggaggatc gtttgagcac aggagtttca gaccagcctg
76981 ggcaacatag ggagacccca tcttcacata attttttttt tttttgaggg agtctcgctc
77041 tgttgcccag gctggagtgc ggtggcatga tcttggctca ctgcaacctc cacctccctg
77101 gttcaatcaa ttctcctgcc tcagcttccc gagtagctgg gattaaaggc acctaccacc
77161 acgcctggct aatttttttt ctatttttaa tagagacagg gtttttgttt tttgttttgt
77221 ttttttgag acggagtctc gctctgccac ccagactgga atgcagtagt gggatcttgg
77281 ctcaccacaa cctctgtctc ccaggttcag gtgattcttc tgcctcagcc tcccaaatag
77341 ctgggaccac aggcacatgc caccacaccc agctaatttt ttttttgta tttttaatgg
77401 agatagggtt tcaccatgtt agccaggatg gcctcgatct ccagacctcg tgatgcaccc
77461 acctcggcct cccaaagtgc tgggattaca ggcgtgagcc accgtgccat gagacagggt
77521 tttaccatgt tggccaggct ggtttcgaac tcctgacctc aagtgatctg cccgccgcag
77581 cctcccaaag tgctgggatt acaggcatga gccactgtgc ccggcctcta cataaaattt
77641 taaaaatcag ctgggcatgg tgaYgcattc ctgtagttcc ccagctacat gggaggccaa
77701 ggtaggagga tttcttgaac ccaggaggtc gaggccggag tgtactcctc cagcctgggt
77761 gattgagaac ctgtctcaaa aaaaaaaaaa attaagttta gtgaatgttc gagtggagga
77821 atgaatgggc agctgttcac aacaacctga tcatgaagca ctgccagccc cattttgaga
77881 tgtggaaaca ggctaggaga gtacctgtag ctggtcatga ccctgtgacc tttgatctcc
77941 acataagaag gtaaggccag gccagcgcag tggctcacac ctgtaatctc agcacttggg
78001 gaggccgagg caggtgtatc acctgaagtc aggagttcaa gaccagcctg gccaacacgg
78061 cgaaacccca tctctattaa aaacacaaaa attagctggg tgtggtgtca ggcgctgtca
78121 tcccagctac tcgggaggct gaagctggag aatcacttga acccaggagg cggaggatgc
78181 agtgagccaa gatcgcgccc ctgcactcca gactgggtga cagagcaaga ctctgtctta
78241 aaaaaatata aaataaaata aaagaaaggt gaggccattg tgctaactca tcttctgtcc
78301 tgagcccgca caggcggcct ggtacatagc aggcctggta cgtagcaggt gtccaagaga
78361 aagcagcaga atggatgaat gataagtaaa cagagagggg gttcccgtgg ctcatggata
78421 ctcagttttt gtttttttg tttctttgag acggagtctc gctctgtcgc ccaggctgga
78481 gtgcagcggc acgatctcca ctaaccgcaa ccgcagtctt ctgggttcaa gcaattctcc
78541 tgtctcagcc tcctgagtag ctgagattac aggcgcgtgg cgccatgcct ggctaccttt
78601 ttttttttgt tgtattttta gtagagacgg gatttctcca tgtttgtcag gctggtctca
78661 aactcctgac ctcaggtgat ccgtccgtct cggcctccaa agagctggca ttacaagcgt
78721 gagccactgc gcctggcctg ttttttttt ttttttttt tctgtttttg agatgcagtg
78781 ttgctttgtc acccaggctg gaRtacagtg gcgtgatctc agctcactgc aacctccacc
78841 tcccaggtac aagcgattct cctgcctcag cctcctgagt agctgggatt acaggcctgc
78901 accaccacac ctggctaatt tttttgtat ttttagtaga gacagggttt caccatgttg
78961 gccaggctgg tcttgaactc ttgacctcaa gtgatccacc tgcctcggcc tctcaaagtg
79021 ctgggattac atgcatgagc cacctcaccc agcctgtttc ttgttttaaa tatagagatg
```

# FIGURE 1-V

```
79081 gtgtcccact atcttgccca ggctagtctt gaactcctgg tctcgagtga tcctcctgcc
79141 tctgcctccc aacagacacc tagtttaaat ggggcaccaa taccccactt acacgagagg
79201 gacccactca agcccacaga gctggggtc cgagcccagg actgtctctc tgtttttgag
79261 atRagggtct agctctgttg ctcaggctgt agtgtcatgg tgctgtggtg cgatcatggc
79321 tcactgcagc ctcggactcc cagaatcagg caatcctccc acctcagcct cctgagtagc
79381 tgggactaca ggcacaccat catacctatc taatttttgt atagagagga tttcaccata
79441 ttgcccaggc tggtctcgaa ctcctgggct cgagggatcc gcccgcctca gcctcccaaa
79501 gtgttagggt tacaggtgtg agtcactgca cctggccagg cctacccctt ttaagcctct
79561 gcactggcct ccgctcactg gcccaagctc tggagctagg ctgcggtgtt ctgatcccag
79621 ctctgccaga ccctggctgt ttaactctag gcaagttact tgacccctct gtgccttggt
79681 atcctcacct gaaaatggga gtgatacctg cctctcaggg ttgctacagg atgaagcaac
79741 tgaaggttac gtactcagag tccacagcga gtgctggggg aatgtcccct ttgggatctc
79801 tggggatcaa gaccttctgg gggctcttag agagtgagtc tggcctgttc tcgccccact
79861 gcacctgtta cagggcgagg cacacacaca gaagtgcctg gcatgtgtgg cttgaatgaa
79921 caagggactg ttttgccaac ccattgtgca agtattgagt ccctgcttat ggaggaggga
79981 aactgcagat cagagagcca gtgactggcc cgagctcgag ggcggggctg ggatctggca
80041 aagcctcagt ctctgacctc aaggctcaaa tagactgtgg gctcctctgc agcctcctca
80101 atgcctggtc caggtctggg accccatggg aggtttgacg agggtaccag cgaatgagga
80161 ggtgagtggt gagcaagcct cgagatccat agttacaaga gtggccttgc atgcatctga
80221 tagagggaca gtgaggcctg aggctgcaga gggattcaaa tcccctggcc aggtgtggtg
80281 gctcacgcat gtaatcccag cactttggga ggatcacctg aggccaggag ttcaagacta
80341 tcctggccaa catggcaaaa ccccatctct actaaaaata taaaaattat ccaggcatgg
80401 gggcggggcg cctataatcc cagctactct ggaggttaag gcaagagaat tgcttgaacc
80461 aaagaggcgg aggttgcagt gaactgagat tgtatcactg cactccagca tgggtgacaa
80521 agtgagactg tctcaaaaaa aaaaaagaaa aggcagatga ctccaaagac tctctcaggt
80581 gctccgggcc tggcacctgc tccccaatgc tctccacagg gctcacctgg cagaaggtgg
80641 ggctgtggac agctgacagc gcccggcaca gagcgtccac atcgttgaag ccaggtggca
80701 ggcggtccac acagaggcag cgggagtgga gaagggcagg cgtcagttgc ccggcatccg
80761 tccagtgcac gtagagggtg cgtggtccca gcggcttgcc cagcaggtcY gacttggcac
80821 gggcagccga gtccttcttc atgtactcag caaagccata gcccttggat tggccagtgc
80881 gctcactgta gaccaggaag cagcgctcca ggctgccgaa gggccgcacc agctcctcga
80941 actgctgctg tgtgaggctg gggggcaggt tggccacaca cagcagggca tccgtgggct
81001 gcagctgcac cgacagttcR cgctcccgca ggcggctctg gtggaaagca ttgattgcgg
81061 cctcggcctg ctccccattc agcagggtca cgaaggctgc ggtgggagga gggcagtgcg
81121 aggtcagccg ggccctgagg gcctgcccct ccaccccgcc accctgcaaa ccaggtggga
81181 cctccaaatg ctgggcatct caggtgcctg ctgatctgag cagttgccag ccttggcgac
81241 tcacacaggc gagagcgcct gccattgact ctaggctcca cgaagcccac tccctctccc
81301 agccctgcgt ccacctgcac ccacagcagc gctcaccaac tgtgggcctg gctctgtcct
81361 aagaacgctg tgggtcactc gggtgatacc aagggcagtg gttaagccca tgctcactgg
81421 ggcccagatt taagccccaa gccccaggtg gctttggggg tatcagtttc ctcatctctc
81481 aaatggatat aacttaatca tcacaatcac cattttgcca gagaggaaac gggcccagag
81541 cagcacccct gtgccccgag taggggagcc tggacttgaa cccaggcagc cggactccag
81601 aggcccactc tacacactgc ccacccctgc cacctttcca ggccaccagt gcccctgaac
81661 cctgctgcca cgtgctgccc cagatttgcg atccacccag cggccgcagg aagctttgtt
81721 caaaacgatc acgccacatc catcttgtgg cttctagctg cagggtgcag tgcagcctgt
81781 gcgggctcgc tgacctcccc tcccatctca ccgctgccac ccggaccacg ccaggcacac
81841 tccagagggg cctttgcagc tgctgtttct tgagcatgga acactgagcc ccacctcctc
81901 acctggttaa cctggaccca cccaggtaga aaccctgtct ctactaaaaa tacaaaaatt
81961 agccgggcat ggtggcacat gcctgtaatc ccagctactc gggaggctga agcaggggaa
82021 tgggctgaac ccaggaggcg gaggttgcag cgagccgagc tcgcgccact gcactccagc
82081 ctggcgacag agcaagactc catctcaaaa aaaaaaaaaa aaagaccaca caagcagggg
82141 tgactggctg agtgaggcaa gaggggtaag aagagaatga acccacggcc aaacgttgtc
82201 aagaattagc tcaatgccaa gcgagtggag taggaagaga atcagcccga tgcccagtgt
82261 tctccaagga gaacatgaag gctgtcccaa gatgacagga tgacaggcag attggggcaa
82321 cctgccatgt tggcctgggg ggcaatactg ggcctgaagc tcagagcagc agacagtgag
82381 ccaggccctc ccatcttcag ggatggtcac tccctgccct ggcccRcccc atggccccac
82441 aaacctgtcc ctttgtattt gtccacaaaa cagtatttga gctcatagtc actgagcagg
82501 tcatgtactt cctgtggaga tacaagacaa aagacaggga gttactggag atggaagggg
82561 gcaacccagt gccgcttcat agctgccacc agctatgaag gcaggtcaat taccaccccc
82621 cattttacca atgaggaaac tgaggctcga tgcagtaatt ttgtcaaggt cattctgctc
82681 atacctggca gagatgggat tcagaaccta gctgcagact gtgctattac ccaggcaaca
82741 tgctcttaga gggtatacct ttagctggct gggcggggtg gcYcacgctt ataatcccag
82801 cattttggga ggccgaggcg ggtgaatcac ctgaggtcag gagttcgaga ccagcctggc
```

# FIGURE 1-W

82861 caacatggtg aaaccttgtc tctactaaaa atacaaaaat tagccaggag tggtggcgca
82921 cacctgtaat cctagctact cgggaggctg aggtaggaga actgcttgca cccaggaggc
82981 agaggttgca gtgtgccact gcactccagc ctgggtgaca aagggagaca ctgtcaacaa
83041 caataacaac aaggccgggc gcggtggctc acgcctgtaa tcccagcact ttgggaggcc
83101 gaggcgggtg gatcacgagg tcaggagatc gagaccatcc tggctaacaa ggtgaaaccc
83161 cgtctctact aaaaatacaa aaaaattagc cgggcgcggt ggcaggcgcc tgtattccca
83221 gctactcggg aggctgaggc aggagaatgg cgtgaacccg ggaagcagag cttgcagtga
83281 gccgagattg cgccactgca gtccgcagtc cagcctgggc gacagagcga gactccgtct
83341 caaaaaaaaa aaaaaaaaaa aaaaaaaata acaacaacaa aaaaaaaaaa aaaaaaagag
83401 gccgggctca cgcctgtaat tccagcactt tggaggctga ggcaggcggg tcacaagatc
83461 aagagatcga gaccatcctg gccaggaagg tgaaacccca tctctactaa aaatacaaaa
83521 atcagctggc tgtggtggcg tgcacctgta gtcctagcta ctcgggagac tgaggcaaga
83581 caattgcttg aacccgaggg gtgaaggttg cagtgagcag acatcaagcc actgcactct
83641 agcctggcga cagagtgaga ctccgtctca aaaaaaaaaa aaaaaaaaaa aagagtatgc
83701 ctttagggaa gccatttaac ctgcatgaac ctccttttgc aagattcact gtttccaaca
83761 actctgtaag tgaagtatta acaaactcat gttgtatatg agaaaactta ggctaggaga
83821 agtgaagaga tctgctggag gccatgcagt tgggcagcgc agccacccat gggtacctga
83881 ctggggagcc caccttctag taagccccac gttgtgtggg ttcacatgcc tcccaatttc
83941 ctcctgctga taaagctgcc ctcttccaag ccagctcatt tatttttctt tttttagag
84001 atagtgtctt gttctgtcac ctaggctaga gtgtagtagc acaatcatag ctcactgcag
84061 cctcaacctc ctaggctcaa gtgatcctcc cacctcagcc tcctgcgtag ctgggactac
84121 aggcacgcac caccatgcct ggctaatttt taaaaatttt ttgtagagat gagggtttca
84181 ctataccgcc caggctagtc tcgaaccctt gggctcaata cagcctcttg cctcagcctc
84241 tcaaagtgct gggattaaag ggatgagcca ccatgcccag cctctcaagc cagctttgaa
84301 ggcaaagtgc tggcgcctgc tgggaccaga cacactgtgc atcttgcagg agagacaatg
84361 tgttcccagc tgagagactg cagacactga gaggaagacc cagaaagaaa cctgaccatc
84421 cttaatgatc cctgtcccga tctgtcccca aattcagccc ctcccaacat ccctggggtt
84481 ccactagcca cgtgcccacc cggtgtgctg gtgggccgga gagcagggaa tctcagcagc
84541 aggaagcgag aacctatttc accgccatag ggcagggcag tatagatgcc atttccctgg
84601 gtccaaagag cattgggaag cgctgcccca tgctggcccc atgagaaggg cactcctagc
84661 aggaagatta gccacgaggg gctccataga acacaggcct cgcttccttc ctgtaggtct
84721 gtaggctggt ggcagaagac cccattttcc caaattaatc ctgaggtttt gggggctgag
84781 acccagggc tgctgttaac ttccggattt atctctgaga gcagtgaata aacgcgtgat
84841 atggagggc gtactctccg cagtcctgaa acttctggat gtgaggtccc gccaccctgt
84901 ggtagagtgg gtggagaaaa gcatcatcca agcaggggc aactggtggt ccaatgtacc
84961 agcccattca caattcccca acaggaccaa agctgtccgc ccccttccat tccccgcccg
85021 cttccatcac ttggtgtcgc ccaagcctgt ggtcgcccct agcaacgccc tccctcactt
85081 cgcagtcggt ttccccttc atcatcgccc ccccacgtcc cgctcttggt ctctcccgat
85141 cccgcgtgga tccggtgctt gggcgccccc gccaacgacc cccgcgcacc tcagcgttgg
85201 tgtcgcccgg ttcccccgc gcacgcgcac cgtggtattt cccgccacgc tcctacaccg
85261 cccccccaa tacctggttg gtcacgtccc ccgggaggcc ccggatcagt atcttgcggc
85321 ggttacggaa ctggcgctcg gtgtgttcca ggcgtttccg gatctcttct ggatctagag
85381 gcggcagctc ttcttccggc gcccggcgct ccgcggcatc gccggcttcg acttcggccc
85441 cagacttagg gctcagcggg ggccggtgag taacggacac gtccgccgcc atcttgggaa
85501 acccggcgcc ttctgggacc agcgagccgg ggcggagcgg catagagcgg caacgagggc
85561 gcgcccgtcg attggctggg agaaacccca cctccttccc gcccccttg ccccgttggc
85621 taagccccag cccacctctc aagagttcaa gctgcgtctg cgcgccacag gccccgcccc
85681 cttccgtcgc agcgacgcta ctcagtggat gtgcacctgg gtgggagaga cttgcagcgc
85741 gggtggaatc aaaccacaga ttagggagtt tgaaggcttt attggtgcgg aatctgaggg
85801 cacagccaag cccccgccaa ctttgatccc ggatcccagc gtcactcagc tctggacggt
85861 tcttcccca ttgcttctgt cggctgcata gacgtgaggg gcagataggt gStctcctcc
85921 ctaacatggt aactgccgct ccgttggtgc tccctgaaga cgtacattaa ggccagtacg
85981 atagtcacca cgcccagggt cagtaacacc gccacgaaga cggggacaaa gtgggagctc
86041 ccagctgtgc agagaaagcg ctaagtcaat atgcgtccct tctgtctcca acccccccgc
86101 cccccggctt accggtccag acccgcagcc ccgtRttagc tcaccctcaa tgtccatcac
86161 cacgaccagg gtgtatttgc ctcgtgagct ggacgcttgg cactgataag taccattatg
86221 tgttacgttg acgaagaacg ggatccccac cggcacctcc cggctggagc cttccttcaa
86281 acaccgcagc tcggggtacg ggttgcccct ggcttggcac tgcaggacgt gtctcgtttt
86341 atctttccat ttcaagtgct gggggcatgt ggctcggtca attttgggac catctgtgga
86401 accaccatgt gtgatcagac acccaacaca cccgaggcac agtggtgcag aggagcgtct
86461 aatctttcca gggcaggggt ggagggatta aaggtcaggg tgaccgactc acacaggact
86521 cgcagctgga cgctactgtt cctgtgcaag aactcgccgt ccacctcgag agtggcactg
86581 cagaagaagc tgcgtccgtc gtcactctcg gtagcattta gctgaagttg agctggctgc

# FIGURE 1-X

```
86641 cccggggccg cggcccggaac tccgtccagc gtgacctgga ctcgagcccc agccatgcaa
86701 ctcacggtca ctgtggaccc ctcatgggcg gtgggctcgc tgaggttcac aatgggtcct
86761 aggaagccta aaggcggggc attgcccagg agcttaatga acaggacctt cgtgtgggtc
86821 aagccgctcc ctccgccctc ccctttcctc tcgggatatc cgggccacgc tttcggccgt
86881 tcaagcctcg ccctctttcc gcgctgtgtc cagcttcggg cactcaggcc caacccacgt
86941 tgcaactgct attggggcaa gccaggcccc accttttcgg ctagtctccg ccccctctgc
87001 cacgccccca gactgctgag gccgcgcccc cttcccacgc ctcctcttac taaagaccgt
87061 caagttctcc cgggcctccc gtctctcgcc ccctagggtc acgttgcaga cgatctcccg
87121 ggcaccctcc tgatccgcgc gcgccgtggc tgtggctgtg gccgttagcg tgtccccgtg
87181 gttcatgact gtcgcattca gcatctggtc ccccagcgcc aggtagacct gggcctctga
87241 ggctggaaaa agcccgtcta gggtgcagtc caccggccac gacgtttcca cctccaagaa
87301 ccggggggcc acgaggcgcg ggggggtcac gggcaggact ggggagaaag gtgggcatag
87361 tacaaccccc aggactgtgc cttccccagg acaccctcat ccccccccagt caggatatct
87421 tgctgactgg gtcacccttc ttcacaggac acacacacag ggccatgaaa acggccttct
87481 tcaaagatcc cacggctcgg gcctcccttc cggggccacc ttgcccagtg gccgggggct
87541 ttccttccca gaagcctgtg aggtccgggg taccccactc tcaggaaccc caaggtcagg
87601 gcaccgtcta ccctggctca gctcgtcacc caccgtctga agccccttct ctcaccaaag
87661 gttcggagct ggcgggggggc tgaggtgttc acgaacagtc ccagcccctg ggctgcatg
87721 tccagttctg tgcggcatga gaaagggggct ccgtggtcgt ctctgctggc cagcacagtg
87781 gcagtgacct ccgctggctc ctccactgcg ggctgccggc tcagctcctc ctcccagcga
87841 agcagcacca ccgtgaggct ggtccggggc gacccaYcct ccacttggca gcgcagggtg
87901 aagttctggc ccaccggctg ccaaggaggc aggggtgcca gctccacacg ctccgggagc
87961 cctgagagag gaggggagga tggcacttag cgggtcctgc aaacccaccc actcacccca
88021 gggactgggg aggagacagg gtRgtcctgc cgagaactgt gagctttgag ttaataaact
88081 tagagggctt agagctgggc cagtcgaagc gtttgctatt atcattagcg cagtgattat
88141 catttcctgt gttgtcagat accttgcaag gcgctaaaca aaactttctg ttctcaaaga
88201 tggcaMaata aaaaaaaatg aggatggaag ggatgaacgt ttatgactat gatatgaata
88261 ttaaaaattc ctgtttatgg ccagacgtgg tggctcacgc ctgtaatccc agagctttgg
88321 gaggccgggg tgggtggatc aaRaggtcag gattattcag ttctctccaa tcaggttcag
88381 acactcaggg ctttcctggt tcaccagccc tgtggcagcc tctcaagata ctccggcaca
88441 gtcacaggag acttgacact cccaggctgg gtgccctccc cagtacacct acaaaacgct
88501 gggcctcagg ccgggcactg tggctcatgc ctgtaatccc agcactttgg gaggccaagg
88561 tggacggatc acctgagacc aggagttcaa gaccagcctt gccaacatgg tgaaacccca
88621 tctctactaa aaatacaaaa attagccagg catggtagca cgtgcctgta atcccagcta
88681 ctcaggaggc tgaggcacaa gaatcgcttg aacccaggag gcaaaatttg cagtgagctg
88741 agatagtgcc actgcactcc agcctggcga cagagagaga ctctatgtca aaaaaaaaaa
88801 aaaaaaaaaa aaggctgggc aaggtgactc atgcctgtaa tcccaccact ttgggaggcc
88861 gagggaggcg gatcacttga ggtcaggagt ttgagaccaa cctggcccat atggcaaaac
88921 cctgtctcta ctaaaaatac aaaaattagc cgggcatggt gtcacacgcc tgtaatccca
88981 gctactaagg aggctaagac aggagaatca cttgaaccca ggaggcggag gttgcaatga
89041 gctgacatcg cgccattgta ctccagcatg ggggacaata gcaagactgc gtctcaaaga
89101 aaagaaaagt caaaaagtag gccaggcatg gtggctcacg cttataatcc cagcactttg
89161 ggaggccaag gcgggcagat catttgaggc caggagtttg agaccagcct gggcaacatg
89221 gtgaaatcct gtccctacta aaaatacaaa aattagctgg gcgtgttggc atgcgcctgt
89281 aatcccagct actcaggagg ctgaggcagg agaatcgctt gaacccggaa ggcagaggtt
89341 gcagtgagcc aagattgcgc cactgcactc tagcctgggg gacagagtga gactctgtct
89401 caaaaaaaaa aaaaaagaaa agaaaagaaa aaaaaaagta gccaggcctg caatcccagc
89461 tactggagag actgaagtgg gaggattgcc tgagcccagg agtttgagac caacctaggc
89521 aacataggga gatcctgtct ctaaaataaa ggtataaaaa agtgtaaatg taaaacctcc
89581 atttgcctac tgctgactga aaggggtacc cccttttttg cttaagagac aagggtcttg
89641 ctttttttgca caggctggag tgcagtggtg caattatagc tcactacagc cttgaactcc
89701 tgggctcaag cgattctccc accttggtcc cccaagtagc tgggacttca ggcatgtgcc
89761 accatgccca gctaatattt tttatttatt tttgtagaga cggggtgtcc ctatattgct
89821 ctgcctggtc ttaaatttcc ggactcaagc aatcctcctg ccttggcctc ccaaagtgtt
89881 gggattattg ttggggccag gcgcagtggc tcacacttgt aatcccagca ctttgagagg
89941 ctgatgtggg cggaacaccc cgtctctact aaaaatacaa aacttagctg ggcattagct
90001 ggacctggga ggcagaggtc gcagtgagcc gagattgcac cactgcactc cagcctgggc
90061 tacagagtaa gagacgccat ctcaaaaaaa aaaaaaagct tgttggggcc gggcgcagtg
90121 gcccatgcct gtaatcccag cactttggga ggctgaggtg ggcggatcgc ctgaggtcag
90181 gagttcaaga ccagactggc caacatggtg aaatgccatc tcaactaaaa atacaaaatt
90241 tgctgggtgt ggtggcgtgc atgtgtaatc tcagccactc gggaggctga ggcaggagaa
90301 tcacttgaac ccaggaggcg aaggttgcag tgagccgaca tcgtgccact gcactccagc
90361 ctaggcaaaa agagcgaaac tctgtctcaa aaaaaaaaaa aaaatgcttg ttggggccca
```

# FIGURE 1-Y

```
90421 agttaattga tttcacaact gctgccttgg catgagattg agatgcgtct ctatatctct
90481 gtctctgtct ctgtgtctct gtctctgtct atctttgtat ctcacacaca aatgtccagg
90541 tctgagctct gggtcagaac tgtggtccat tctttgcaaa ctgagggtcc ccactctcct
90601 gagccattgg ccacttgccc acaccactac ccaagaccag tcccacctcc ggacacgtcg
90661 actcactgta cacggtgatg ttagaggagc ctgttatctg Rgagccattg cagtacactg
90721 agcagaggat ccgactgttg ccagtcacgt tgctgagatt gaaggctgcc cagcccatgc
90781 cactggccac cagctccttt gatagggacg tctccaaggc gatttctcca gagctgggac
90841 aatcagtact gcagttcaca aacagggacc ctccagcaga gagcacaggg ttctggggct
90901 ccacccgcaa aaggaactcc tgcccctgga caccttcagg aacatgaaga agtcctggtg
90961 tttgtttcct tgtYccccaa cccacgcatc aacaggcccc accatttaac acctctctcc
91021 ttgtgccgag acagaagggt tcctgccttc atggtccagK gggaaaggta gaatccatca
91081 gagaccRggg agaactcaga aggaggccag aagaggctct gatccagcat aggaggaggt
91141 gagtcaggga aggcttcctg gaagcgggga ccattgcagc ccgaaactga aggctaagta
91201 ggagttagca agtgaaaggg acaggtgatc ctggcagagg gaagcacata cgcaaagggc
91261 aaatttcagg agttcagatg gagcaggaag catgaggggt gtgtgtgtgt gtgttagggg
91321 agagggatgg cgtacaagcc tatgctgggg cctgaccacc tgaaggctga actaaggaac
91381 cagaactttc tcccgaggat acagagtcag cggccaggtc gcagtgtggc aggatgtgaa
91441 cagcgttgcg ttctattcct ctaccctcta ctgatcccca aaacgcagcc ctctccagcc
91501 ctccccggct tgactggtct cacctggggt cagcagacag cagaccagca gagtccagca
91561 ggccctgggc cacaacacgg atggtaccat ggtRgccatt ctgacagagg aaggtgcctt
91621 cctgaggtgc ccgaagggca ggcaggggaa aaggcggggc ctcccactgc gacggaagca
91681 cagcggttaa gattgcagaa gtctggggac tgcaccttgc ccaggcctgt gggtggaagg
91741 ggatgctgtg gcccttgggg gccagggtgg gggacccaga cggggctctg caaagtggcc
91801 agcactgctg agtctccccc agggaatggg ctggtcccag ttgcactgct ggccccccatt
91861 tgaattcaca ggaaatgcta actaaactgg tttcttttgt tctcagcact gggaagcctg
91921 ggtggggggg atgaggcatc tccttctggc tgaacttagg tgggtagggg aatattctgt
91981 ccccagagag gaagttaatg gggaaaactg taggcaccat tgtgtagatg ttaaggtggg
92041 atttcgagga aacattaaaa aacaaaaaca aaaacagaaa cagaagcaga gtcttgctat
92101 gttgccccga ctggtcttga actcctggcc ttaagatggc ctcctctcat cttgacctcc
92161 caaagtgttg cgattacagg cgtgagccac cacgcctggc cacctttgaa gatcttgagg
92221 atacctccct atctttgaag atcttgggga tcccccatcc cctgcacaaa gagctaaggt
92281 aggtgatttg gggacagctg aKtccctgag ccactgtcta tccaggattc tacttggttt
92341 ttgtttgttt gttttgagac agagtctcac tctgtcaccc aggYtggagt gcagtggcgg
92401 gatctcggct cactgcagcc tctgcctccc gggttcaaac gatttcctg cctcagcctc
92461 ccgagtagct gggattacag gcatgtgcca ccacgcccgg
```

# FIGURE 2-A

>4:87306501-87383000

```
1     tagacagtca tatataatgg aaactgcaaa cctccgaaag tgtgtctgcg tgtgggcaaa
61    tagtaaaata gcattgaata cacaatgtct tcaaacacag tacacaagtt tgacagttat
121   tctaactgct ctaaaattct ttttatcaga ttcactgcta acatgcatgg aaaatatgat
181   gacaaattcg Ragattccta aaagagaaaa gaaggctata taatacaatc aattattaca
241   ttgtcttttt tcacaattct gtaataattg atggtaattc aatagatatt tatctttatc
301   taactttatc tttaaaacta tcccaagttt acttattact gctacggttt taaattgcag
361   cagctgaaat atagtaggac aaatgcataa tttggaataa gactaaatat tgcatttcac
421   cttgatcaca tcgtctctgt gccctgagca aatagtttaa cttctctaac cctcaatttt
481   ctcatctgaa aaaaaaaatg aatcctctct ttcggacttt caagaaattt aatttggaag
541   atataaggga aacatttgca tctactacag tgtttctcaa agtgtaatgc acccaggaat
601   cacctgggga ttctgattca gaagctctgc attgggtgct aaaattttca gcttccaggt
661   gatgccagtg ctactattct attattgctg atgttatctg gaagctggtt tataatgcaa
721   aatctcatgt cccacccaat gcctactgaa tttcaatctt ccttttaaca ggatctccag
781   gtaattttta tgaactttaa aatttgaata acattgatgt agcagtagat cttgcttatt
841   gctgatatgc agtcgaaaat attataagtt ttgaatcatc acaagaaaag tgtgtagaat
901   ataaatgaat ataatatgtt ttttcctctt caaaatctga ttttgaatta tatttcccag
961   gtgtcaccgg aagctaaaga gtggccttgc ctcaggtcag gtttctactc cctgacagcc
1021  tctcaattag ctgctgttgt aatgaaatta tgcattgtca aaagcctttt tgcatcattt
1081  tcttcaccaa atctcaagtt ttaagtattt tatatagaaa ctacataaaa tccaaccctg
1141  ctctgcccat tccagtggct gccagatagc tggttttctt tgtgattttg ggctgttggt
1201  tttctaggag aggaggagaa gattagagca agttaaaacg ccacagacct tgctgctctt
1261  actgagattc agccattttt cttaagtaaa cactctccag attgcatcaa gccattagtt
1321  aatttacaga attccaaaat agttgattct ggagattttt gctagttgct tatatggtga
1381  agaggatttt tggaggtctt cactacacca ttctggctga tgtcgcttaa aatgccctga
1441  agtttttgaa tggtgaacaa aagtcttatg gattttcatt atcttgataY attagagaaa
1501  ccatgcatcc acctgattgt attgggctcc atttacagat gccactaaaa tatcttgata
1561  gtttgaggca attattagat gagaggtcac agtgacctcc tcctaagaaa tatttctcag
1621  aattaaatgt caaattttta ataaacattg tattatgatt aagagcccag gtttttaatg
1681  aaccctggga tcaagccctg ggtctggtcc ttaacgtgta accttaatca agttacttaa
1741  cctttataat acagtgataa taacagtatg tatgtataga tttatttcaa aattcaatta
1801  aattacaaaa gcaaagtgct tgacacagtt catggcacaa agtaaatgct caagatgttt
1861  tagcttctat tgctattatc atagttaaga atatggtttg gtactgataa taataatgaa
1921  tgttctcttg aatgtgtttc tttaagagtc acattgaaat agaaataaat aaaaactgtc
1981  tcactcatgc tactttatgt tctatgcaat tctaggcact gtggtggtcc agcagttgat
2041  gtcagtgatg attagcaaac atgattgcct ctttcccaaa gatgcagaac tacaaagcaa
2101  gccccaagat ggagtgagca acaacaatga aattcagaag aaagccacca tggggcagtt
2161  acagaacaag gagaacaata acaccaagga cagccctagt aggcagtgct cctgggacaa
2221  gtctgagtca ccccagagaa gcagcatgaa caatggatcc cccacagctc tatcaggcag
2281  caaaaccaac agcccaaaga acagtgttca caagctagat gtgtctagaa gcccccctct
2341  catggtcaaa aagaacccag cctttaataa gggtagtggg atagttacca atgggtcctt
2401  cagcagcagt aatgcagaag gtcttgagaa aacccaaacc acccccaatg ggagcctaca
2461  ggccagaagg agctcttcac tgaaggtatc tggtaccaaa atgggcacgc acagtgtaca
2521  gaatggaacg gtgcgcatgg gcattttgaa cagcgacaca ctcgggaacc ccacaaatgt
2581  tcgaaacatg agctggctgc caaatggcta tgtgaccctg agggataaca agcagaaaga
2641  acaagctgga gagttaggcc agcacaacag actgtccacc tatgataatg tccatcaaca
2701  gttctccatg atgaaccttg atgacaagca gagcattgac agtgctacct ggtccacttc
2761  ctcctgtgaa atctccctcc ctgagaactc caactcctgt cgctcttcta ccaccacctg
2821  cccagagcaa gactttttttg gggggaactt tgaggaccct gttttggatg ggcccccgca
2881  ggacgacctt tcccacccca gggactatga aagcaaaagt gaccacagga gtgtgggagg
2941  tcgaagtagt cgtgccacca gtagcagtga caacagtgag acatttgtgg gcaacagcag
3001  cagcaaccac agtgcactgc acagtttagt ttccagcctg aaacaggaaa tgaccaaaca
3061  gaagatagag tatgagtcca ggataaagag gtaaggaaaa tctggaggtc gtaactacca
3121  gagagggctc agtagcctcc tactgtggga caggatcaca ctgagggtga catatgctgg
3181  ctccaaagtc aaagaaacac aagtttgctc catcatttat gagctttgtg actgtgtgca
3241  agttagcttc atctctgtga gctttgattc cctaatgtaa acaatttaaa tactagtagt
3301  atctacctca aaagcacata agaagtgatt gaacatttcc ctggtacaga ataggctcaa
3361  taatgtcatc tttgattatt attaaataga tagatttgca ttagagggct ggaaggagag
3421  gggctgtgag gagatggaca gagcattagg agcctgttag ttttcactaa caagaaaagg
3481  atgaaactag ggtaaaaaaa gtagaagtaa atagacctcc atgttttatt ctttcaatca
3541  ttgaatgttg agagagtgcc ttctatctgc caggctctgt tccagacgct gaggattcag
```

# FIGURE 2-B

```
3601 atgagctgag agaggccata agagccaggt gacatagggg attgacaggc tgattttcag
3661 cctttgactt ttactctgag atagaaatcc atggaggttt ttgagaagag gggtgacgtg
3721 aagtatgttc acatgaataa ggaagactgt agcctaaatg aggaaaaaag aatatgattt
3781 Rtaaagaagc aattatgcta aaacctaatt atatcaatgt gcatcttctc ataaaatgat
3841 caaagtagga gtagctcctc tagtgttctc agtcccagtt tcaggctgtg tgatttggac
3901 aacccttcct ccatggtgtg ctaggatcct cagtYggttg ttaagttggc tttaatttac
3961 cttaatatgt ttcctatcca ttcatcaatt atcagacaaa aagataacgt actttcagaa
4021 gctatggcaa aataaatatt aaaaaatact acttataaat gagattgaaa aattacttcc
4081 ttggaaagta tttttttggt aagtttcact gagtggaatt gtggtttgaa tttttctata
4141 taatggagag tgacaggaga tttaaagaaa aatatagggt cattatctta aaaggttact
4201 cttctttctg aatgtagttt ttggaaccac tatgtattgg aattctgaat tactgccaat
4261 ccatgatgat aaattagcat ttcacactgg gaagcttata ttttgaggca aggggtcaca
4321 ctttaaatgt agtagaaaaa gaaaaaagtt atcactatga atttgcagat ggatacattc
4381 atttctgagg atgttgctga tgcatatatg ttctgagatc tacggtatat ttcagagatt
4441 ttgataaact gatagaactt cccaatagag gtcatcttaa tgggccattt cagtgtcaca
4501 ttcttaccct cRtgacaggc caagggagag ctctgcagta gggtggaggg tgggggaccg
4561 ctcgtggcct agccatgaaa ttatagaaag ttaactgttc tggctaaatt ttgatcattt
4621 ctgtttagag ttattcattt ttctttaaat tcctttataa ctttacccca caagcattga
4681 aggtagatgg atggatagat aggtaggtag gtaaataggt aggtaggtag gtaggtagat
4741 gatagataga tagatagatg atagatagat agatagagag atagataata gatagatgat
4801 agatatagat agatagatag atagatagat agatagatag atagataata ttttactttg
4861 tttaattatc aataagatat atgtttaaat catttataac tttaccccac aagtattgag
4921 ggtggatgga tggataggta ggtaggtaaa taggtaggga ggtaggtaga cagatagata
4981 atattttact ttaattatca gtcagtgaga tatatggttt ttagttttga aacatatatt
5041 ctttttccct ctccaaattg gtttctaact ttttctttc ttattttttt ttatgtttta
5101 tttttgaca gagtctcact ctgttgccca cgctggagag tgcagtgatg caatcttggc
5161 tcactgcaac ctctgcctcc caagttgaag cgattctcct gcctcagcct cctgagtagc
5221 ttggattaca gatgcatgcc atcatgactg gataattttt ttttttcttg tggagacagg
5281 ggttttacca tgttggccag gcagctcttg agctcttgac ctcaaatgat ccacccacct
5341 cggcctccca aagtgctggg gttacaggca taagccatgg cactcagcta gtttctaaca
5401 atttcttag gaaatattgt attgtggtca gagaagttca tctggatgat attaattatt
5461 tggtctatta aaactattcc cttattactg gattgggatt ctatatatga ccaagagatc
5521 aagtctgtta atcaggctat tcaaagcttc tgtatttctt ctcattttta tcagctatat
5581 caataatttt tgagaaagat actttaaaat ttcctatcag tattctgagt ttatcagttt
5641 ctttttataa ttttgtcata ttttatattg aacaatttga ggctctacta ctaggtgaat
5701 agaagtttgg agttgttcat atctccctga agaattgatc ctttatcat taattagtgc
5761 ctatttttca ttgtaataat gctttttacc tgaaactcta ttttttctga tattaatatt
5821 gtcctaccat cttcctttga ttagtaactt cctgatatgt cttttccttt atttaacttt
5881 caaactttgt ttgatgattt gatctaacaa cttacatctt tacaattctg gaatattttt
5941 ttcatcatct ttttgtctat ttcatcttcc caccttctca atttctcttt tccagaaccc
6001 ttattaagct atgtttgacc ttgttaatct atcctctgtc tttgttaact tctttttcc
6061 atattaaatt tctataatta tcatttttga ggcattctag gtaacttttc tcagacctgt
6121 tttgtagctt actaatactc tgatgagcta attcatttct gatacttgcc caggcccatg
6181 attggagatt ttgtagtctc ttcttcataa aaacagttaa tgttccaagc tctcagttaa
6241 attacttgta gtttctgcc tgcctaatac tggaggcctc accttctgtc cccatgtgaa
6301 aaagaaaaac ctagacccaa gctctgtggc ctctatccaa gactaatatc cttcgtgtgc
6361 ttctacttat tgttctatat ttcggtttct tccttggttc tgatggctga agctgctgtt
6421 ttattttaac cttgatgatg tattttaaaa atatatttct taaaactttt ccagcacttt
6481 tatatgtttg cattgggagg gatttcttct gtattcaact tagtcctttt cattcctgga
6541 agtttcccac aagcacctga tacatacaaa tgtgtcaata tattgttttg taagggttgt
6601 ccttctcagt attttaattt caatttcttc caaaattttg gctctaaaat agctttggaa
6661 aacaaagaga atatattgta gaataattct gagtggaaac cagcatagta ccaacttttc
6721 atgtaaaaat gtggtcatat gtttaaaata gccttctaaa tgatgtagga ttacaaaata
6781 tgaagtatta tttttaaaa ttctagttgc ttacaaagag aaggaagatt aatttttaag
6841 tgttcttatt atctggatga aatatcaaca tgtggggatc aaatttttca aagcagaaac
6901 ataattgaga ttttaattta aaaactgtta ttaaaataat cagatatctg agagctacac
6961 atgcattaac ctttcaaatt tcagtggtat ggtaagtttg aatgagtccc atgggacttt
7021 tgctcagaat aggagaaatt aaaaagtcag ccgaggccaa gatacatctt aaataaagat
7081 gtaactttaa tacccctctt ttgaataata tatgtatatg atttggatct tagtggttat
7141 ggagaaaaca gtctctgttt ttaaaaacgt tactgcgaat tctacaatta tccaaaagct
7201 atacaaaaca actatcaatt tatagagata taaaatacct atgcccagag ctatattaat
7261 aacttgggaa gcagtgagaa ttggggatgg aaaggaatgg gctttatttt tacaaaatat
7321 gagatgcagt gcttatttaa cacatgtgat ctttacctct cgtatcaatg taagctcatt
```

# FIGURE 2-C

```
7381   actttgggta ctcttttata agatgggact ctatatgctg ccaaaaaccc cattcttagc
7441   agtgaactta accaacaatg acattttcct atcactattt ttccttttat agaaggcaaa
7501   tgttattgcc ctcttttttt tttttttttg ttaactatca gactttcctg ctgcatttag
7561   tgtagtgaac ttYacaattt aatgtccagg aagctttctg cctcgtctgc ccggcatgtg
7621   ttactggctt atctgtgctg gcattcactt atttactatt aaatgacagc tgcttccaag
7681   agaggccttg ggacctaatg actaaggtga cagtaaatca tcttagcaaa cagtaaaata
7741   atacaacaaa attacatcct ttaaatcatt gaaagggctt tattccaatt tcctaagcat
7801   cataaagagt ttagaaaata tttgtatgtc tcttgcattt caagtgaata aaatcacttt
7861   atctcttact cttgcgtccc caccccccac cccccccaac atcctttgta gcttagaaca
7921   gcgaaacttg actttggaaa cagaaatgat gagcctccat gatgaactgg atcaggagag
7981   gaaaaagttc acaatgatag aaataaaaat gcgaaatgcc gagcgagcaa aagaagatgc
8041   cgagaaaaga aatgacatgc tacagaaaga aatggagcag tttttttcca cgtttggaga
8101   actgacagtg gaacccagga gaaccgagag aggaaacaca atatggattc agtgagcctg
8161   ctttcgcctg ctgtctctga tggctctggc aaggactcca gggattctgg tgggatatga
8221   cttagaacca ggtggctggt cacctggatg tacagaagtc taactggtga aggaatatca
8281   tttacagaca ttaaacatcc atatctgcaa tgtgtaccaa agttatatca tgccccataa
8341   tgctactgtc aagtgttaca actggatatg tgtatataga gtagtttttc aaaagtaaac
8401   taaaaatgag aagcatattt caagaattat tttattgcaa gtcttgtatt taaatgttaa
8461   atcaatWtgt tgttgcaatt tagcttgctt tcaagcttca ccccttgcac ttaacataag
8521   ctatttttgg cattgtgtta tcatcggctt attttataga tcaatatttt tatttccctt
8581   ttttgctgag gaaatgaaga taagcaaaaa tataaatata tatataaata tatgagttat
8641   taaaatcaga agaatacttt gtggctgtgc tgtttgtgcc aatagacttt gtcatgacca
8701   aaaagagaaa tgtaaatagt tttataaaat acagtcgaat caccaggaac ctttgagctg
8761   cttttaaaat tcttcccctg gcaccactca gttttgcttt tgcgaggcga tttgacatag
8821   gaactttgag actccatgag aaagtccctt tctgaggccc actgtctacc ttgccagatc
8881   ctcagtgcgt atcgccaatg caggatgctc cttagaaaag aaaaaatggt aaagaatggc
8941   atttaacgat tcaggctttg aattactctg tccctctgga ccgaatctct ttaactgctg
9001   Satagtttta gaggaattct cctgctactt aggtactggg aaacaatgct tgctaaacca
9061   tgcccacgtg agcacctgtc tcccactcaa acctctccca tctcccaaca actgcacttt
9121   agaataccag cagtgaaatg gtattactgt ttccctctga gtgaaactgc tagagtatat
9181   gtcacgtagt gacatttttt tctcactcag gctattgcca tctgggattc tctccctact
9241   acagctggca aagttggttt gcagcaagaa gatagtggga gggggccagg ctgcaggaga
9301   aggagaaaag tttagaagaa acaaaccatt ttgcttctaa ttttgacagt atcactttcc
9361   tgttaaaaca tacaataatt ttaaaaggtg aatgcctaaa gttccaattt tagcaaatat
9421   gggaacctca gcaatgctaa ttttctagaa aaacccaggg ctctttggag ctagagtttt
9481   gggagaacag ttcttcacaa taaggcaatg gttttgagag gccaggcaaa taatctttct
9541   caccgtagaa caaaaagtta caaaaggcat aatcggaaat agagactaca tacttgagtt
9601   tatggggttt gtgttgtttg aaggttcaat gcttgcatgt gtttatttat tttcaagagg
9661   gaaagtggtc tgtactgctt tcatccttgc cactgtcttg cttttatttt ttactctccc
9721   actgagcaag cRtctgtggt cctatggtat caaccagtat ctttatagca ataatttctt
9781   taattccctt ttctctctct ttccaattat ttaaccagtt acttccacct ggacatacga
9841   taggaaattc aaactcaaaa tatgaaaatt gatcttaata actctccctt catatctttt
9901   cacctatttc cagtccttat catagttgat aaaaacctca gactcatcca gaaagctata
9961   tgatgcacta gtaaaaaaaa caaagatatt caaactgctt gggttcaaat ggtatacaat
10021  ttgccagctg ttactgaacc ttctatgcat aactttttt ttcctctgtg caattggaat
10081  aataaaaata ctactcccat agagttgtta tgtggtctta atgagatagc ttgggttagg
10141  ctgagcacag ctgctagtag aatccaaaaa ggggccaggc atggtcgctc acgcctgtaa
10201  tcccagcact ttgggaggcc gaggcgggcg gatcacgagg tcaggagact cagaccatcc
10261  tggctaacac agtgaaaccc tgtctctact aaaaaaaaat atatatatat atatatacaa
10321  aaaattagcc gggtgtggtg gtgggcgcct gtagtcccag ctactcggga ggctgaggca
10381  ggagaatggc gtgaacccgg gaggcagagt ttgcagtgag ccaagatcaa gccactgcac
10441  tccagcagcc tgggcgacag agcgagactc cgtctaaaaa aaaaaaaaaa aaaaaaaaaa
10501  gactccataa atgttaacgc atattttctt tctttctttc tttctttttt tttgtggggg
10561  gacagtctca ctgtctcctt ctgtcaccca gtctggagtg cagtggcatg atcacggttc
10621  actgcagcct tgacctccct aggttcaggt gatcctccca cctcagcctc ccgagtagct
10681  gggactgcag gcatacgcca ccatgcccag ctaatttttg tattttcttt tgtagagaca
10741  gggtttcgcc atattgccca ggctggtctc aaacacctgg gctgaagctg tctgccctcc
10801  tcagcctccc aaaagttatg caagatgcat agcaatgacg ggtcacttaa tttgtctggg
10861  actacaagcg tgagccaccg cgcccagcca actcatattt ttcttctcta cccacttctt
10921  cactcataaa tttgctcacc agctaatcta ttccactcat ctttgccttt ttaatctgct
10981  ctctttaatc acttttccct taccttttga tgaaggcatc ctacccagcg cgttttgttc
11041  cagcatctct cctgtacttt tgtcctttgc aggctgcctt ttcgaaatta ttcatctgac
11101  catagcttga tcttgctcat tacatatgat gactgtcact tgcccaggac aaagcccttc
```

# FIGURE 2-D

11161 acatagtgta aaaggtccta ctgccagttt cattcttgtc tcctaccact tcccaccaca
11221 cctgccatgc catttcatac ctctagaact ttccacttgc tattgttgtg tccttcttgc
11281 ttgccttctt tctagctggc atgctccaaa catcctttaa gagccaaatc aatgtcagtt
11341 tcctcatgaa gcttcccaga atctcaaatc agtgttagtt gctttttgtg cccccattcg
11401 cattatctct ttctgtcttt cccacatctg attattttct ttatcaaata agaagcaatt
11461 taggaggggag agtgatgaag tggaaatagc agaaaattga gagccattca aacctagatc
11521 tgcctctcaa catgccttat gcaagataca tggcaatgcc aggtaacttg acttgtctga
11581 tctcatctac aaggtggaga taataactcc tacaaagtag ggatgttgcc gtggttaatg
11641 gtggtatgta atgtgcttat cccggtgcct gtcactttgc tccactgata actctattga
11701 taaatggagc ttcttaaggg gccaagattg tatcttcttg ctgttaaatc cctccatgcc
11761 ttatgccaaa gagaatgtca gcacagtgag tttgcagtaa tttttgggaa tgcaaatata
11821 ctcaaacaca cacacacaca cagggtgttt tgtggcctgt gatatttgag ggatgatatt
11881 atcaatttta attgtcaatt tcagtgatga tggcactaat caatggatac catgagcagc
11941 atgttcatct gggtttccgt tagtcaaata aaaagcctag acaatattaa gtacaattaa
12001 cataagaata aattggaaat caataaggat acaaagtagt taggtagtat caagatttca
12061 tttgctacat cctgtatatg cctctttaaa actatttagg taattaaaat gaatcttaag
12121 ttataaactc gagaaaggat ttgctttcag atgtggggcc tactgtgtta gcctcactat
12181 tcaaactaga tgtctgggta tcctttggag aacatttaag gtagaattgc ctatgatgtc
12241 agctctactg aaacatgatt ttattatatg taacattatg aaatgactaa aatctccagg
12301 tggagtttaa gtaatcattc ccattgattc tcaagctggg atattcttca aaaaatcatt
12361 cccaggctgg gtagtttata ttattgatat aattatgaca ccttgaaaat tcaggagagc
12421 attaaataat gtaatgcttg ccgcaaaccc cagtgttcct tacactgcag agctgcactc
12481 tgattagagt accatacgca tgagttttgg aatctacgac cagggttcaa acccagctct
12541 gccacctcat agctttatga acctaaatta tgtaatatat ataagcctaa atttgttttt
12601 ttctggaagt agagttaata gttcctccct tatcatattt ttatgaggaa tgtagtacaa
12661 taggtgaagg gattaacata gtgcctggaa catagtaagt gctcactaaa tggaaactat
12721 cagccctcaa attatttctg ttgaaagagt gtttcagctt ctaaggaacg tagcaaggtt
12781 ctggactcag ggatatgacc agcattaaaa gtatcatgat caccagcaca ggatataatt
12841 atatcttggt ctcagagact cttaagttta tccgtgccca gataatctgc cagggattga
12901 tggcaaggaa agtaacccaa accactcttt ctttctgaga taatacaatt acatccatga
12961 tctgaaaatc atgtctaaac aactacgtgt aactgtcctt catcaactct atccatgtct
13021 acactaaaga tgatgttggg tgttaaaggt attggataaa atacaatgaa tgtttctgag
13081 ggctatcatg aataaactga gggctgtgga ctaggtcctc tggggtacat aaacttacag
13141 ttattgccat gttatatgat gtgctgtgga agtaactaaa gagaccataa aggtgaagct
13201 caggagagca ctgcagagcc gtgactttgg tgtgagtcct aaagaccagt aggaagtcat
13261 cagacagagc tgcagttttg gtaaggggcc ttcctagccc atggaacagc atggcaaatg
13321 tgccttcagg gtagacccta gggagtcttt tcaataaaat aaacatgacc taccaagttc
13381 atgccatcaa gatggcattg tgcctaacac cttagcagca cgtcatgaga cctgaaaaca
13441 cacttagtac ccacgcggct tctactctct tattcaRaga aagcatccag agctggaaac
13501 attttgcctt gtttgaactc acaaactaca ggaaaggagt gctggaactg tggctcccaa
13561 aggtctgttt cattattctc tttatagtct tcttaactgc acaagtgggg aatgagttga
13621 aagttagaac tgcccagctt aagggcctct tgctctgaag tgacttggag aactaacagt
13681 tccccaggca gctaaaatct actgaaccta ctgtagatct ttcagtaaaa agtttagctg
13741 atgaaaagtg tggtttttaa agcaccattt gtggctcagg tccaggKttg aatgatactc
13801 cttatttatg aacttctgta tagctgctgt ctgtatggcc tcttgaagga taagccacag
13861 tgaggccagc cttttcttaa atagggaatg agggcaacag gaSaacagtg aattgttagg
13921 gggcaaacca aattggcatc attttctttg agcaaaactg tctcttactt ctgggggagg
13981 ccccgagcat cccacctgtt cttgcgaata gaggatgtcc atcccaactc tctcttctcc
14041 acacacccat tccctgttcc ctaccccctc atgattcact agattaatta tgcaccacac
14101 tgaagtgatc cccagggtca tgccatcaga ccctcatcgc tggttacctg ttgccaaaat
14161 agtccaagtt cccatgccat ttttccttca tctatttgct tatccccata ttgcccagtg
14221 tattctctca tttgcttcta tctccaatgg cctccccctg cctaggactt ttccattatc
14281 ccctctagat cttacaatgc catcaccctc ccctatgtcc tcttctctga atcatggttg
14341 cctcctgaag acacMatctt ccctgcagcc ctctcaagcc tagctatctt cctctctaca
14401 acctgcgtat cattggcctg aaggggagac atcttgcttt gctatttgca gctactttca
14461 gacctttaat ccttcttcct ctctaatgac cttcttacaa gcacatgtct ccttatcatt
14521 gactgaatat tctagcacct aacccactca ccctccccac tcatactcca ttcaccatct
14581 tggccactca gctccttgac gactttgaa aatcttgttc tgccgctgcc tgaacatcag
14641 ccaccacact catggttaca ctgcgtactg tgtcatgact aatcactggc atcgccaaca
14701 gttccatttc aagcatccca cgctgtggtc atcacttctt ctctctctag tcactcccct
14761 tagcaccatc actccaaatt ggtaagtttt cagcgactat catcccccat ttcattactt
14821 ccctccttga ccagcttagc ttctatggtt ccttctcata attattctct tgcccccatt
14881 tccaaatccc attatgtact ctcccagcac tgtactactc ttttgagaat taaaaagaaa

# FIGURE 2-E

```
14941  caaattgcaa ctctagtaat tttttcttac tcttaacctg tgcctaaaaa aactaaacat
15001  gactggagaa caaaagacca ccaagttgac ctgtgttcct ttgtatttat atKaatatgg
15061  ctcacatggt tttcatcact gtccagtaac ttattgtcac agtttccaag gtcaatttca
15121  cacttctcaa acttccacaa actcttcatt gtctactgac aaccttactt ctatctttac
15181  tgagacaaca aaaaaaaaaa tcagatgagt atgtacttat gcccatcacg tcaccgtatt
15241  cccaaactac ctgcctctgt gcacaccacc tcactgcctg ctgcagttac tgtccagggc
15301  cagcgcctct gcccatgtac tggagcctgt ccctccaccc ttttcaagca tgttactcta
15361  tcaaataaat atccctttct cttttgcatt atcagttttg ctatctctct gttggcccca
15421  ccagcactat tacccatgct atattagctt ttaaaaaaWt ctctcaatct cacatttatc
15481  tccaacgttt acatcattct tttgctgcac tttgtagaaa aatattttga attttctgta
15541  tctatttcta cttccttact tcccatgttt tcttgaactc actcgagtta ctcttttctt
15601  gctcaatact ggatagaaac tgctctcatt ggggtcatcg atgaccttca aacagattta
15661  gtccaactgt gaatcttcag ttcccatttt actcagcctc tcaataacaa tccgcccctg
15721  cccattctct cactttgaa acaccttctt tacttggctt ccaggacagc acactcctgg
15781  ttttccctct acctcactga acattctgga ataccctcct gttttctgag gtctgtatgt
15841  tggagtgaga ggcatctcca tctttggttt tctcttcttt aactacactg tcttttggtg
15901  ggctcataca ggctcttggc ttttaatact acttataaac tgattactct cagatttctg
15961  tttcagctca ccctcttcct tgaactccag cttataagac actcctgcta ctccacgtgg
16021  attttttttt tttttctttt ttttttgag atagagtctc actctgtcac ccaggctgga
16081  gtgcagtggc ttgatatcgg ctcactgcaa cccccgcctc tcgggttcaa acaattctcc
16141  tgcctcagcc tgagtagctg ggactacagg cacatgccac cacacccaga taattttttg
16201  tatttttagt aaagatgggg tttccccatg ttagccagga tagtctcgat ctcctgacct
16261  catgatctgc ctgccttggc ctcccaaagt tctgggatta caggcgtgag ccaccgtgcc
16321  cggcctccac ttggatatct aataaacatc tccaactgat attccaaaaa caaacatgat
16381  tttctcccca atgtttttct aacccattgt tcatcatctc agtcaactgt accatcattt
16441  acttcatggt ttaggtaaaa gaatctagag ccatttctga tctcttgctt ttatatcaca
16501  atatcaatct atcagcaaat cttctcagct ctgccttcac aataggtcca gaattcaaat
16561  ccttctcacc atctcctttt cctagtctaa gccccatcat gtctttctgg attaatgttt
16621  tatcctacga tggatttttt tgtccccatt cttatctttc ttcctaagag tcctctttac
16681  tcagtagcca aaggaatcct tttaaaaaga aaatttagct tatatcattt ccttttttcaa
16741  atctaaccac catcttcccca tatttagaat caaatctcaa gatcttgcca tgctctgacc
16801  cctgcttctc tctccaattt catttacaat cccatttact cttgttcatt ctgctttatc
16861  cacaagactc tttgcccctc cttgtctata taaacaagct ctccctccaa agcctgcccc
16921  tgctttctct tcagagtctg aaatgccctt ctccgtgtca atagctcact ctcacttcag
16981  tcaggtctct gagcacttat cccctcttct gagaggcttg ccctgaccac cttgtctaaa
17041  atagcatccc tttcccacct tactttttaa cctgctttgt ggtcttcgta cttacctcta
17101  tctgacttct ccactagaat gcagaccatg tgaaggcaga tttgctgact tcatcacatc
17161  aactatgtgt acctagaaca gtccttggat ggttgacgtg cttgataaat aaatatttgt
17221  tgcatgaatg aatgagtgaa tatttagagc tctatgtcaa atttctggtt cagatgttca
17281  gggatgttct cagttggacc ttttaataaa aaaaaacaat aaatgaccaa attctacttt
17341  aagttatttt agggttccaa cagtcaccca tagactgaac aaactccgaa gatttggctt
17401  catgtaagaa tgtcactgca aagaagcttc agtacccatt gtgatatcat ttgctaattt
17461  ggagattgtg atgccaaaag tataagcccc atgctcagtc agcatctagg tccaatagct
17521  ctcagaggaa ccatacatca aagcaaactt tctgcaatgc ggaaaagaac atccatgcat
17581  ttttaaaatg aattttaaat tttaaataat ttttaaaagt ggtttcatct cttgtaatgg
17641  aaaatattca aaattctcat gctgattcat gaagtatact tatataatta gacaaaataa
17701  gaacattgaa agaagcagac tccttgtttc atcgctttaa gcaaataata aaaatggact
17761  cRtgcaagtc acaccactaa tttaaataca ttttgtaaaa agatagcaaa taaatgaaat
17821  aaaaatattt gctttagttt ttttaaacaa agatttatta actctgaatt ttttaggtta
17881  aggaccatgc aatatatatc tcctacaccg aagtgtccaa gaaatgtttt tggagtaaat
17941  taatgaactg ctttagaatg ttgctcagaa attaagctag aaattcagag caatattctc
18001  agggcttgat ttacaccaaa tccaagttcg caatgtatta acatgaagga ataggccatt
18061  gctttctgaa aaccttcctt ttataaaaga aatactttaa tattatgaat tctcttggtc
18121  tgtgtaaaat attatttgca ttataaaaat tgattttttg gtataattgt tcataggcaa
18181  tattttcacc aggatgggtt cacaagtgtt aatggaaggc gaattatgtg tgggacagtc
18241  ttctagggaa tatgtgaggg gtttttaaga tggataacat tttgtacctg cttttgagtt
18301  gcaaatgatc tggactgttg gttaaacatc gcagacaaga tcaggtgaag aacattacta
18361  gaagttcagt gattaagaaa taaagccacg taccccacgg agtgaaattt gaattcctta
18421  aattccctca aattatttta tgttggattt tacaccagga taggaatgtc tttaaaagta
18481  agaaatattt gacataaggt agaaaatttc aaaggagtgt ttgacttgtt gctggatgtg
18541  tcaccacatt tcatgtgtct gagaaatata aatttctaca gcacattttt agtgccttaa
18601  acagactaca atgatgtaaa tggttgaaca gttgaaattt agtctggaac tgggacagat
18661  gacttgccac atagagtggc tcccaacata aagtgatctg tgatggcagg actttcagcg
```

# FIGURE 2-F

18721 tgtttcacta ttagcatgtg tcatccaagc atgcctttca acatctgtgg agaaaagaca
18781 tgttacaaat acaaaacacc accatgatga attgggaatg tcaaaaacct ctgccttggc
18841 tgtgatccag tgttgttatt ggcaagtaga ttcatccttc taggttggtt gctccataag
18901 taatcttcaa gaaaagcaag tccaacaccc ttttttctcc ttactgtgaa cttggtcaaa
18961 attaccaaat gtttggtaac catttatggt atcaggaatg caccaataac tccttctgac
19021 tctttaagag caatcatctt gtggaatgca atttcccttc ttattattgt gggtattttt
19081 aggtacctat gcttgacctt ttttaaagag agtaaggatc tgtgatttaa actaggatag
19141 aagctgttcc agagataaca aaaagctgtt gctttctata ttccaacagg aactaggtaa
19201 aacgataaat gtgataactc aaaataatgt gtatgtttat cttccaggct ttctaatagt
19261 taattggtta aataaaacat tatttacaga aatagaagag ccactcctca actgttcaaa
19321 cactgcaaag taaactaatg caagtgcagt gacctcttca tccagccaca aaatcatata
19381 tatttttaat tctccaagga gtttgagata gtaaagttgg atatcatgta gctaaacaaa
19441 ggcttttacc ttttctcttg gaaaatagtt aaaatgaata gRaatctgat aattaatctg
19501 ggaaatttaa ccctgctcta cttacctaat gcccccccaa aagatgatct ttttttctt
19561 cattgatttt ataccttaaa aagaaattac tgaattgcag atatacaact agtatctaca
19621 gacagaaatg Maggtcagtt ttagaagaaa ccagtgtcaa atgaaaacac agatttctac
19681 atggtcacat tcatatcaca cactcttgac ccaaggtgag aaagtaggtc attccttatt
19741 tcttatttat tccctggcac aatcagacca cactacaagt ttcttttacc agttgccaaa
19801 atgaaatcat gacacccttc agagctactg tcgttacatg caactcaaga caacgagcag
19861 catcataacc attgtcaaca ggcaaactcc aacaatgtct gggtttggat ttggttttat
19921 gttgagagaa gcagatttcc tctgatttta ataatccata agaaacagaa atcataatat
19981 attgactcca tattacctaa aaagagtcaa aagaagcaga agttctcccc cgattcatcc
20041 tcttgttcca cagacatttt gaaaataaaa gaagacaaaa tagaaaggaa agggtgtgag
20101 aaagaacaca gaaatgactt gaacgatttc aaatccttat taagcatcac tgaggttgca
20161 ttttacccat tagactgact gaatttagtt ctcactgctc agaccttgca aagaaactgg
20221 acgtacatgg ttttgccttc cagggttgat ctagaactgt aagggtgcat gtgaatgtgg
20281 gtatgcatgt gtgtgtgctt taagtccatt ttataaacac tgacacaaga caattctcat
20341 cattggtaat agtctttgga gtccaatcat gcagaaaaac aggcaggtgc aagttaggtc
20401 tcattgcaag atggacatgg caaggtgaac agataatcct acataaggat tgggagattt
20461 gtgtccatta gaatatactg gccattggtc ccacataagt aaagttacat atacaagatt
20521 ctatatataa acagaggccc ctcacttcag tttggctgac caaagaaaca ggtcaaagtg
20581 agccattttc agatagttaa aggactaagt accaaaagaa ataacaaaat cttatcctct
20641 aatcacaaag aaaatagcta aaataactat ttacattgca actttttgt tgtttttaca
20701 gagaggagtt tccttgcttg gcttaatgat atctgtggaa aatgttgaac tcttataatt
20761 aaaatgtgtg tgggggattg tgagtgggga gttgggttcc tctttggatg caaagtggct
20821 gagatcaaaa atttctagag caattttact cctctttctg agctgtagaa gaatacattt
20881 ttcagcaaag ctggctaata acatgcctta cagagcataa tgcacttaag atgtggcttt
20941 tcatatgtta agagattcaa atcgaaatag attttgtata atttaggttt tgcaatgaat
21001 atttacaatt ttaaaacaaa tcaagtttgc ttattttttt ttccttgaca ggacataaaa
21061 ggttttacat atttttatct atttttact agttccattt actaaggcca actttcagtt
21121 ttaccagtca gctgctatca gtgtctttag ataatatttt tctgataaca gattgaaaaa
21181 actatcgagc ttcttaaccc tcaattaaat agcttactcc aattggtggg aactggccag
21241 gctctggtcc aactgttggg tacattccta caaaactcag tcatcagatt cttgaagatg
21301 aaaggttatt atcagttttt attccacaag aagaaattcc gttgtaaggc tagtgtattc
21361 ctatttaaaa ttgaacacgc tgacccacaa aacacatata aaaatagtaa agacatatta
21421 atagtaaagt ggtggcggtt ttgccaaact cttgcagcag tttaatttc cctgcacatg
21481 agtacacagc catccttcct acaaaatact gcaaactatt ttattaagga tcactgcaaa
21541 ccaactgccc caaaggaagc ctaacagaag gttaggctcc tcagagtgcc aacttcatgt
21601 tccaaaatga gccccaaccc caggtagaac cttcactttt tttcaggaat attgtccagg
21661 tgacttgaca cttgcctacc ggaaaagttg ggatgttctt gggaaataac aggtgactat
21721 ttaagaaata tttggggtgt gtgtgtgtgt gtgtgtgtgt gtgtgtgtgt gtgtgtgtta
21781 agacagacaa gtgaatgcag aacatattat attctgtgaa tttgataata tcatcacctc
21841 ctagacatat tataggaaag cagacctagt gggcaaagga ctgggggct tttacagtgg
21901 tgattctctt tgttgttcca tcatctgcct tgctttaatc acctaataaa actgatcctg
21961 caaaaattct gggggccaaa tccttacctc tatttgtaaa tgatacttat attcaaggaa
22021 tttgttccat ttttctcttg tccataaggg aaatctggct aaagctaacc agctcccaag
22081 agccatgcac agaagatgta agactaattg agctttggga aggtactaac agcgggggag
22141 gggccctgtc tagttcttgc ctggtttacY ctgaggggga aattcaccac ttatgcaggt
22201 acgagagata gcctgtcaag cactttgaag atgctggctg gagacttcct tctgtcttgt
22261 gatgtcatct tgcggctgat gaaattcccc ttgagactct ctttaaagca gcttcctgct
22321 ctccgcactt aatcccatgt ggtataaagt aagggagggg agaagccaca gatatatcaa
22381 gagaaaagta ccgagaaaga aaatcagtaa agaattagag atacagagag gagaagtttt
22441 taaaaaaaaa aaaaaaaaac aggaatatga aagagaggga gctcaagtga ctgaggaggc

# FIGURE 2-G

22501 atttttaaca atcaggttat ctgcaatatt aaccagaaaa aaaatggtag caaaatagag
22561 aaaatgggcc atttttattc caggggacaa gctgcacaaa ggaatgttct tctatttatt
22621 ttaaacaaat gactgcgtgt actgaatctg actgtgtgaa ataatctcag aatggcagca
22681 ccactggYat ggcgatggtg caggtgggtg cagttccctg tggtctctat tgcttgaaga
22741 gagaaagRaa gttccctatt attatattta aggcagtttt cagagcactg gcattcttgt
22801 ttgctctgtt ttggggatat tctatttgcc aatcagttca cactcatcaa ccatccactt
22861 cctgtctttc acttcttcag gtgaaatatc accactctag tgagcaccaa catggtcaga
22921 gtgcaaataa ctgtgatgga tacttcgaag tgaataagaa gggaagcctg attattgctg
22981 agatcacctt ggtattgcct tgtacataac caagatgttt cacagtgcct ccttaagtgc
23041 tcaggaaagc actttcattt aatttaattt aagctaaatt ctggagattt tgctaaacat
23101 tgacattaat gcttcatggc ttttaagaca aactcatatt gctaaaacta tttggcagca
23161 gtaaatttat gggcagagtt attgctctta catctactgc agtacaaaac tcagatgatc
23221 agcccttcga ttgctcatac ttattaaaat aaataaataa acatgaaaca tcagccacat
23281 taaggagtca gggaaactcc tgaggaataa caatgtctct ggagatgttt tcaaatgcta
23341 acctgagact cacactgtgg tttgggggtt tcattMttat tattattatt ttatttcaga
23401 agaggaaagt aatattattt ctcaaaataa aactttggca gagtatgaat cagggaagca
23461 gcccacacat ggaaaacaca atccacaatc tgaaagtagt ctaggcaggc tcattttttt
23521 gttgaaggaa ttcacatgga ggcaattctg ctcagttcca gagagagatt cttatcatca
23581 aaagcacaca caataacctt gcttcaccac ctgccacttt ctccagtggc ctcttgttgt
23641 gaccagtaat tgagaaatga caaatgggta tagagccaaa tctctaaaac agggacacca
23701 gtgtctccta gtatctgggt aataacctcc tttcagtctt agtccacacc acacactacc
23761 ctccagtctc tcctactctt tactgtcaca gaaaagactc tcagctttta ttcacacctg
23821 ccttcWgaag gccaaaccat tttgtgcctg aaaactgtta cccactcScc ccacacttta
23881 cataagtatc acatatgttc tttctacaaa ttggtataca ggtgccattt aatccattca
23941 aatttggaag ctacatcttc aaggtctga gagagctcac tccccccata tattcccccct
24001 ttacatgttt tcttataaga catacagttt aatcaattaa caaactaaac agcttatata
24061 ctggcaatat attacagatg ggtttatgtc agagtaatag atcacatgaa atggaccatg
24121 tggtacccca gtgcattatg tcttggtaga gccYtgagga cactgacagt agcatctcta
24181 agtaagtagt gctgtatgaa tacagacaca tgcggatctg tatctacatc catctgacta
24241 ggccaaggag caggtagatg caagatagag acacacacat gctggatggg gccactgcac
24301 accttgtcat gccatttaaa agggcagtta caggttgccg gttttgcagc cattaaaatt
24361 acactttatg gaaaaggctt ctctaattgt gtctgatttg ctttctgtag taagcatcat
24421 tggaagaaga ccaaagcaag agcaactaga agttaaatat acatttgagc ttagctgcaa
24481 tacagaaccc tggggaagaa gcaggctgaa agatttattt aattttaggc ttggattctc
24541 tcccttgctg ttttcttgat gttgtgtgtc tgcatttgtg tgtgtgtgtg tgtctgcgtg
24601 tgtgtgtgtt ccatcacatc atctcctgaa gaacgctggg tttcgcaggc aggcggctag
24661 tcacctgcaa caacccaggg gtcctgccga ggcttccagg ctgctgtcag tgtcagatgc
24721 cagggtctgg tcggtggaca tggaggagat gtcattgaca gacgaggatg gagggagact
24781 ctcactgctg ttcactgctg cacctgtgct aagaaaatga agaccaacat gactcaatct
24841 agaaccagac ccatcttaat gccattcagg attcagggat gggcaaatac aataggggat
24901 gtccagtcca tcaatcattt ggcaagcctt tatagagcag ctgacatagg ggagcatatc
24961 aaatggtgac aatcaagaca tagcaataaa aagtccttca tcacaagtta tcaaatctca
25021 ctctatattt gaaatcacct gcaacacttt ctattaaaaa acactaatat ctggtcacat
25081 tgcctcccct gccccccactg cactgcatat attttaatat aattggcctt gggtgaggcc
25141 aaggtatttg catttttatt tatttatttt ttttgagatg gagtctcgct ctgtcgccag
25201 gctggagtgc agcggcacaa tctcggctca ctgcaacctc tgcctcccag gttcaagcta
25261 ttctcctgcc tcagcctccc gagtagctgg gagtacaggt gcgcaccacc acacccagct
25321 aatttttgta ctttttagtag agatgaggtt tcaacatgtt ggccagaatg gtctcgatct
25381 cttgacctca tgatccgccc acctctgcct cccaaagtgc tgggattaca ggcgtgagcc
25441 accacgcccc gcgggcattt gcatttttta aaagctcctc agagactgta ttcagccaag
25501 cattgaaagc ctctggctta tgcaggagac aaataagcaa aaccccttaa tggagccatc
25561 tgagcctggt tgtttttaat gcagggagct tttaaaacaa acatatgcaa gggtcctatc
25621 ccagaaatag aagctgaagt gaaggtcaga aatctgaatt acttggggac cacatctcta
25681 gagaaaggaa gatatataaa tactattatg tcatcattaa tgacaccaaa cttaaaatgc
25741 atatagaaaa gccagtagga cataagtagg actgttgatg aaagttacaa aaaaaaaaaa
25801 aataggagaa gccttagcac agcttttgct tatgagctaa ctctccagca ggcttgctta
25861 cctgggatct ccaaataaga actccaaatt attaacttag attaacttga aaaaattgtt
25921 tatctgttat gcgggagtat aaggttgaaa ccctgagaag aaacctgccc aYggtaatca
25981 ctcagtaaag gctagctgag ttgccagtag ctttgagatc aacaaaagaa agatgaacca
26041 aaggcattgt gcgataYaat taaaccagag caccctggaa acaatccatt tacttttttt
26101 ctttgtatat tctttatacc taagcaatct gagtggtaac acacttgctt tgtgaaatag
26161 ctcgctatta aaaatctgta gtatagtccc ctaatcaaat aagagatgag gaattctcac
26221 taaaattata tcttatttga gcaaatatag tgtgcaagat ctatttggtg aaaaataata

# FIGURE 2-H

26281 actccaaagt cagttataat agtgttatca catagaccta ataaatcaga aagacaaaat
26341 aaaactaaat taaggaaggg Ragtggttaa cataatcctt gctcctggag tagacatttc
26401 ttgcctgtga gaaaagtgtg atgaaaatgt atcagttatc tagagaggac acaaacacct
26461 aaactaagac agatcacctg tatcttccat gtattttaa gttgtatttg ttgactataa
26521 atatatggac gtactctatg ggaaatgtga aaaaatatag gaaataaaga taatgatatg
26581 caccgatctt caaataaaYg gtaagaactg ttaaactttt catatatttc ttcccggtca
26641 ttctttttgc ctggaagttt ccctggtcgt gatcttttaa tatgtatttt tttgggatct
26701 tgatattttt aKatacagaa aagcttatga atttttcatg ttctttaaac tcctaaaaat
26761 acccatcctt tctaatctta aatgtctcta gaaaaggttt ttaaagtcct cYtgttattg
26821 tctttctgta aataattact ctatcaggac aatgtataaa gttataatat tccataatat
26881 tccaactttg aatggcttta atttattcca actttactta attatatccc ccatagctat
26941 ggtacagagc tggttcattt cccctcatga taagatttta tctcctagca gacagccctt
27001 ggttccttta cattgcccaa atgccctgct ctgttttca cccccgattc tggattaaat
27061 tataccaaYa tccttcatat tctttcataa gttgctaata atttagtgtc actatgatgg
27121 actcctcaga attctcttcc atttatccac actcttatta aaagacagag attaaaactg
27181 gacacaggac tctattataa aggcctgatc aattgtgagc ctagcagagg gaaattgctt
27241 ttttcttact atttgtctgc tggatgacat atccctagac tatggatgcc tttcaaatta
27301 tggtgctaca gcttaattta gttttgactt gaggtataga atgatgctca tatatttttt
27361 gtaacagctt tattaagata taatccacat atataattaa gaaatttaat tttcatgatt
27421 cagtgttttt tagtatattc acaacattgt gcagccatca ccacaatcca ttttgcagca
27481 tttcatcatc cctaaagaaa accctgcact ccattcctca cctaccctgt agctctaggc
27541 aatgactcat ctacttttg cctctataaa attgcctgtt ctgaacattt catgtaaatg
27601 gaagcatatc atatgtgatc atttatgact ggccactttc atatagcaga atgtttttaa
27661 ggcttatcca tgctgtggca tgtatctgta tttcatatct ttgtatggct gcataatatt
27721 ccattgtatg gatacatcac attttgttta tttattagtt gataggcatt tgatggatat
27781 ttaggttgtt tcaacttttt ggctattaca aataatgctg ttatgaacat tcatgcacga
27841 gttttgtatg gacatgtgtt catttctctt gggtatacac ctagaagtgg aattgctggg
27901 gcatagagta attctagctt taacacttga ggaactgaca aactgtgtcc ggaattggtg
27961 ggttcttggt ctcactgact tcaagattga agccgcggac cctcgcggtg agtgttacag
28021 ctcttaaggt ggcgcgtctg gagtctgtcc cttctgatgt tcagatgtgt tcggagtttc
28081 ttccttctgg tgggttcgtg gtctcgctgg ctcaggagtg aagctgcaga ccttcacggt
28141 gagtgttaca gctcttaagg cagcgagtct ggagcttttc gttcctccca gtgggctcgt
28201 ggtctcactg gggtcaggag tgaagctgca gaccttcgtg gtgagtgtta cagctcataa
28261 aagcagcatg gacccagagt gagcagtagc aacatctact gcaaagagca aagaacaaag
28321 cttccacact gtggaagggg acccaagtgg gttgccaatg ctggctcggg cagcctgctt
28381 ttattctctt atctggcccc acccacatcc tgctgattgg tagagccgag tggcctgttt
28441 tgacagggtg ctgactggtg catttacaat ccctgagcta gatacaaagg ttctccacgt
28501 ccccatcaga ttagatacag agtatcaaca caaaggttct ccaaggcccc accagagcag
28561 ctagatacag agtgtggatt ggtgcactca caaaccttga gctaaacaca gggtgctgat
28621 tggtgtgttt acaaaccttg agctagatac acagtgccga ttggtatatt tacaatccct
28681 gagctagaca taaaggtttt ccaaggcccc accagagcag ctagatacag agtgtcgatt
28741 ggtgcactca caaaccctga gctagacaca gagtgctgat tggtatgttt acaatccctg
28801 agctagacat aaaggttctc caaggcccca ccagagcagc taaatacaga gtgtcgattg
28861 gtgcactcac aaaccttgag ctaaacacag ggtgctgact ggtgtattta caatccctga
28921 gctagacata aagactctcc acgtcctcac cagactcagg agcccagctg gcttcaccta
28981 gtggatcccg taccagggct gcaggtggag ctgcctgcca gtcctgcgct gtgcgctcgc
29041 attcctcagc ccttgggtgg tcgctgggac tgggcgccat ggagcagggg gtggtgctcg
29101 tcggggaggc tcgggctgca caggagccca tggagtgggt gggaggctca ggcatggcgg
29161 gctgcaggtc ctgagccttg ccccgcagga aggcagctaa ggcccggcga gaaatcgagc
29221 acagcgccgg tgggccggca ctgctggggg acccagtaca ccttccgcag ccactggccc
29281 gggtgctaag tccctcactg cccggggcca gcagggctgg ctggctgtcc cgagtgcggg
29341 ggcccgccaa gcccacgcct acctgcaact ccagctggcc cgcaagcgcc gcacgcggcc
29401 cgggttcccg ctcgtgcctc tccctccaca cctccctgca agctgaggga gtgggctcca
29461 gccttggcca gcccagaaag gggctcccac agtgcagtgg ggggctgaaa ggctcctcaa
29521 atgccgccaa agtaggagcc caggcagggg aggtgccgag agcaagcgag ggctctgagg
29581 actgccagca cgctctcacc tctcaaaact gttttctaaa gtgggtgcac cattttacat
29641 tactaccagc agtatatgag gattccaatt tctctacatc ctcaccaaca cttattatct
29701 gtctttttat ctagccatcc tagtgtgcat gaaatggtat ctcattgtgg tttgatttgc
29761 atttgcctga tagctaataa tgttgagtat atttttcat gtgcttgtca gccattagta
29821 tatcttcttt ggagaaagtt tactcagctc ctttggccat tttttaattg ctttattttc
29881 tttttattat tgagttgtaa gctctttagc tctttcttcc tttctttctt gctttctttc
29941 ttctttattt ctttttttaa ggacagggtc tcagtcgccc agcctgtagc acagtggcat
30001 agtcatgttt cactgtatcc tctaactcca gggctcaggc aattctcctg ccccagcctc

# FIGURE 2-I

30061 ccaagtagct aggactacag gcacatacca ccacacctgg ctaattaaaa aaaagatttt
30121 ttgtagagac aggagcttgc catgttgccc aagctggtct caaactcctg gcctcaagtg
30181 atcttccgat ttctgcctcc caaaatgctg ggtctacagg catgagccac tgtgacaggc
30241 ctgtaagagc tctttatata atctgggtac aagttactta atgagatatg tattttcaaa
30301 tgtttttcct ctatgctatg gtttatcttt tcattttctt gaaggtgtct tttgaagcac
30361 acatattttt aatttttatg aaatagcatt tatctgttgt ttcttttatc acttgtgttt
30421 tagtgtcata tctaataaac cattttctaa tccaagatta taaaaactta ctcctatgtt
30481 tttgcctaag agttttataa tgttagacct tatacttaag tctatgattc atttttagtt
30541 catttttgta tatgatatga ggcaggggcc caccttcatt cttttgcatg tatatatcca
30601 atcatcccag caccatatat tgaaaaggct attcttttgtc catgtcttac atccttatca
30661 gaaatcaatt gatcataaat gtaggtattt attcctggac tctcaatttg attccgttga
30721 tctatttgtg tatcttcatg caaatagcac tctgtcttga atattgtagc tttgtagtaa
30781 gttttgaaat cagaacatgt aaatttccca gctgtgttgt ttttcaaggt tattttggat
30841 attctgtatc ctttgcattt ccacacaaat tttaagatgg ggcttgtcaa tttctgcaaa
30901 aaagacagct atgattttga taagtattgg cttaatctgt acatcaattc ggggagtatc
30961 tgtcatttct tgacaatatt aaatattcta atccatgagc aatgaaatgt ttttccagtt
31021 attagatcct ctttaatttc tttcaacaat gtttcgtagt tttcagagta caaaacttgt
31081 acttcttttg ctaactttat tcctaaatat gtatttcttg atgctattct aaatggaatt
31141 gttttatgtt cattgctaat gtgtagaaat acaattatac tgaggactta tttgaataag
31201 aagtttcagt gtcagtggat tggatgttaa aaacagatca aatgaatata tatatatata
31261 tatatatata tatatatata tataaaatga atgttgtaat taactagaaa gccagtggaa
31321 ccaggcgatt atgatagcat gctttattta tgagaaaata accagtttta aaaataactg
31381 gttttatgag aaaataacca gttttaaaac tttaaaaagt tttaaaataa aattgtggca
31441 taatacaaac caaaaagtat ttttaagtct ttaataataa tcttggtctt ctcttgagat
31501 aggctttttc ccctatcctg gctggagttg attatgtaga cacattctta ttgtcacaat
31561 taaagttgag taatgattca aataagggtg agataagtct tagctgaaat aaggctccct
31621 tatagtaaaa caatttgatg tttacaaata aacacaaacc cacaacaatg gcagctagag
31681 aaattaaaca aagagcattg aagaaatttc tccaaaacac aaatttatat ctacaaataa
31741 tattgaatgt atgtaacaaa agccaaagta gcttttaata agctcttcat aaacaggatc
31801 gtgctgcagg ctatgcttat aaaacacaca gcactcctac tgcacttatt ttcaaaatca
31861 gcatattttc agcacacttc tggaatattc cgagtgttat gccataccag tcagcccctt
31921 attatatgta gttttgttat ggattacctc aagcatatat ttcccctgct tctttttact
31981 gctttcatta tacttcattt ctcctgtctt ccacaatgtc tactgctgag tagtagcaca
32041 gagtaagtat tcgagttaac aaaacagtat tgagtatctt atgtgtactc ttctagatag
32101 gttctgtgag gaaaagcaag ttccttcctg ctttccaact agtgaaactg ttaagctatt
32161 tttggccaaa ggaaatcact ttgttgcagt tgtctttctt ctccttttgt tcatcagcag
32221 cttctctcta attcctatcc tctgtgttgg aatggtcctg ccctctagtt tatcaatgtt
32281 gaatgcaaat ataattagtg ttcaactact caaagttcaa aatctatcaa agtctttatc
32341 agctgtattt ctagggatcc atagactatt gcttctgatt ttatatggca caaattcatt
32401 aagagaagtc ttcagtgctt Yctttgtaaa agttcccatg acatctgctt gtttcatggg
32461 aatggttcct tgctatccag tgtgatggtg ggagagaacc gtgcactgtc tatggtgtcc
32521 tggagctggc ttttagtggc ttcttagaac gaattattag catctctttc caactctgca
32581 tccattgatg ttacattgtt gcttaaaatt ggtcacagtg agaatattta taccacagga
32641 attgttaagt gctactagtc aattcttttt ttaaatatag ttgttaaatg ttaaccagtc
32701 caccactatt agatgaaaac tatttgcttc tgtatgactc cctctattgc gctataataa
32761 atgcctcctt gcctgcagcc acttctgctg gatgatccat tcaagtctat tttcatgtag
32821 gcaataaaca ggagttctaa accataaacc caaacacaaa cgtaagggac aagacatggg
32881 ccttggcttt aagttgttaa attactgcca agcagtttgc gaaaatctct taaacagaat
32941 tgaactgaat tgaatatgat cattgtgttc agttgtccag tcaatgtgag aaatatctct
33001 tctttctata ttatttaaga gaaaacagta gagatacaca ttttcaaaaa tgttttgcca
33061 tcaataaagc attacaaatt tttgtctata aaaatggcat taaaaggaac tgaaaatgtc
33121 aatagggttg caaatgagga atattagcta ccttcaactt tacctaaaaa atcagatttc
33181 atagaaagaa gtaacatatg ctaaagaaaa tttctcagct acaagttatt caaccaaaaa
33241 acattgttat gggggaaaat gttgttctgc tttttttaaa ggcaatgatt ttcctgataa
33301 cccccaatcc caacaacaac aaaaaaatat ggtagacaca gaattagaaa aagcacaaga
33361 atgaaaaaat agtgtctttt ttatctacat gatcttgagc cacaatttgt ctgggcctga
33421 gatttttaa taaaaagtaa ataaaattaa tggactatat tagtgaccct aagacatatt
33481 cccatggtgg ggaatgtggt cctcactggg gagagaatgg ttgcatatta aaatcaccca
33541 gaagctttat ctttgaYtta gagattttga aaaatctctg cccatcctgt tgttcagcat
33601 ccttatctta gtgagtcact gttagttttg ggagctttac ttctcaggtg gaagagggct
33661 ggtgaacaaa ctcagaattg gtgaatcaga tgctctcctg tattttttct agttctaacg
33721 tactataaca aatgctttta gaagaaaatt aatttcaatt catggactgc tagtccctca
33781 gcaattgtta cagatcaatt caggaagcaa gcagctatgc catccttgaa aataaattac

# FIGURE 2-J

```
33841   tatgcaagtg aaatcagata gcagaaggga atacaattat ttatttggaa atgtatctga
33901   agtttagatt tatctcttat tgacatacaa caatttcatc aataaatttc aaacctataa
33961   attattctgc aaatgcacat ttaacttcct atttacattt taaaagcatc gcataacatg
34021   ggtttcgtat tagaagtact tggcccctga gatactgcat taacttactc ccttgctgca
34081   gaattcccag tattgccata actgaatact aacagagtgt gtgttataaa aataccacag
34141   ctatttcaa aataaaatat gctacgtgaa cattaatgaa aaaaagctga atataatgaa
34201   caatgcaaaa aagctgactt ttcaaacttt ttttagaat tgctttttct ttccagcata
34261   gtttattata cacacaaaaa cgcatacaca cagtggtgcg ccaataaaaa tttaacaatt
34321   ggctctgcag ggggcaacaa aaacgccctg atttgtaggg tctgccaatt tccatgttgt
34381   aaatccttcg tgcctgattt cacgttgcca acacaccaac aataaatgca tagttgggaa
34441   gagaggtaca gagttggccg tcatgagctg gtagagtcag cgccagcaca tggctgccct
34501   agagcctctc catcatgcac ataaatatgt catacagaaa tgcatcccat aatgtgtagc
34561   atacatatga tttctcacga aagagaaagt catactacat gacttataac taagtcatga
34621   atattttcc tagatttaat ccacaatcat tttaactata aattggttaa gccagtcaac
34681   tatagtttag ggcatatgta ttacatagat aatttttaat tgttaaggta gttggaatgg
34741   ttaaaaagtg gcttagcctt ctcactcagg tagagcctaa gttgcatatt ttagaattaa
34801   tcattaacac actaagcagg cctgcaaaac atccatgaat atatatttcc caaaatgaat
34861   aaatcaaaca cctgaaagct gcaaaccttt atgtgaagca tgaaaaaatg ccaatagcaa
34921   gaatttatat actttatgct tggcctgttt atcttcatgg agattaatag agtgaataaa
34981   tatttataat aatggtgtaa gaacatgctg aatagtatac tgtattaaag ttacatgatc
35041   ttgtcttgaa ttttgtgttt ggaatagtac taagaaatat gctgagggag aaattgagtg
35101   gcaattttca aattaatata tatttaYtga gctactacta tgtgcaaggc acagcaccca
35161   ggcactacat gggacacaaa gataaatgca tacagtcttc tcaatgaaat tacaatcaat
35221   aMgggataat aagacaagag gtcaaatatt taacatacag ggactagtag gacctataga
35281   aaggaaagat cctattattt gggaagatat aaaaagtctt ttgggataag gtggtatgaa
35341   aaggaaatga agtaatgata acttgaactg aagatgaata gaataggttc tactaaaatt
35401   tgtccaggcc cagcctaaca tgctaggtta cccgtggcta cattctgtta tttctgctgc
35461   ttaatgagta catgacaata tgttctggtc attataggaa ttcttaaaac aaggagtcat
35521   tctgccttag caggtggcat cttgaggctt ttggattctg tcttcaagtt ttttaatgag
35581   caacaggcca aacgtggatt agtttgaagc agcttctgtg gactgtgtca atctgtatgc
35641   gagggtgaat gagtagagag ggtggtatgg aaaacaactc ctgtgattcc cttaaccttc
35701   tttgaggaag ggaaaaactt gggataaaac tgagtatttt ttcaagtcta ctggtgagaa
35761   aagcatgtca taaccgaatt gcatctgttt gggtatcata gtggtttggg gtgtaaatta
35821   gttattcgaa gagtagtatc gatatgaata attgattaat tatttcattc tacatctttt
35881   ttcctaaaat aatttaggaa aacaacaccc taatcagttt ttctcaaaac taggcatttg
35941   tagcccccat ttttgctagc aggagttgaa tgtagttttt aaagctgaaa ataaaccaWc
36001   acattttcta aaactcttta tagtgagagc ataggtctta ggaaaaaata tattagcatt
36061   aataagtaaa ttgtctcaag tcatactaaa gcacattact aggatcagta aaaaatatat
36121   atgcacaatt gtgtattaga ttctgtggct agcaaacgaa aaattttcca agctgacctt
36181   aaccggagcc catcttggta gatgtttcaa ctattgtcac atcaaccttg agaagagttc
36241   aaacactaag aatgaatgag ggaagaggta gcggctgaaa ggattactga gctccacatt
36301   gacttgatgg tcaaaagggc attatggctc tgaattttga tgaggcacat ttacccttta
36361   gcccatgtta acatttttctt caggattcat tactattaaa attatttatg aaaaagtttt
36421   tgtRctggat cattaccatc agaataatca gaatgaatgc cacactgaat atcaaaagaa
36481   ataaaactaa aatcattata aggacacaac catgtgatat ttgtccatct gctctttaag
36541   caatgttatg ttatttcttg caacccctac acaaaggcca agaaattaca caagtactag
36601   tttattggtt attcacggag agtgagtacc tgaaggagaa ggctgtcctt ttactacacc
36661   atttttagtc ttttcttctg aattcattac ttccttgtag ataagttctg taagaaacag
36721   ctgtgttatt atagaaaaca aatttatcct tcatccacag ggaaattcat tacttaatgc
36781   caaataatta cgttttgatg ggttgagta aattaaaaat caattgagat tcctttactg
36841   ctctatttct atttctatat tgccttgtaa ttagcagaga gcctggcaca aataggtatc
36901   tagtaaagca gttgaagaaa tcaatgcatg aaaatttatt tctacgtaca tgaaacatac
36961   tatttatttc catatacata gtatttattt ctacatacat ttctacataa ataacattta
37021   tttctacatg catgaaatat ttctacataa atgaaacaag taatacatgt tttacttgtt
37081   ttctgaattc tttgggggaa gtactaatac attctagctc aatctagcaa caatttttat
37141   ggctttgaac atccattttt ctttatactt atttcctatg aaatgcacac attctcatga
37201   ctttaaatta ttaccaatcc ttacaacttc catctcttac tgcatctttt tttttttcca
37261   gactcatatc ttggacttgt aaatctaaac ttgccaaaaa ccaaacacat taaaaaattt
37321   agttcattta ctctcagact ggcatccatt tttggctttc tgaatgctag agttaaattg
37381   gatagtttca gttctgttta ctYtgtccct ttagctaaaa atcattccgc cctactcatt
37441   cttgctttgc aatggcttat aatctgtata atcatcatcc tttacatgta cttctttctc
37501   caatttctca caatttagtc catgtcctcc tcaaatgatg gttatatcct atcacccacc
37561   tattcatgag cccctaagga gtgccttcct gtccttactt ggatcaaagc aactcttttt
```

# FIGURE 2-K

```
37621  catctatatc aagcatcttt tttcacttga tccacttttt gtatatgttc atttttccat
37681  attcaccaac atgaccctgg aatctctgga ccagtggaat ctctttagta tcttttaaaa
37741  ttttttttatt ttattttttta ttttacatat atatatagag agaggcagag actttctctg
37801  tcacccaggc ttgagtacaa tggcacaatc atggctcacc gcagcctcaa cttcctggac
37861  tcaagtgatc ctcccacctc agcctcccaa gtagctggga ctacaggtgt gtaccactat
37921  gccggctaa tttttgtatg tttctagcag agacagggtc tccctatgtt gcccaggttg
37981  gttctgaact cccgggctca agtgatcccc cagcctcagc ctcccaaact gctgggatta
38041  caggtgtgag tcatcgctcc tggccctgtt tagtatcttt ataacatttt ctgcttattc
38101  ctgtcctttg ttttcctctt agtcctttat ctgatatttt cttaatattt tttcttttcc
38161  taatttacac ttattaattc ctgcaaggta gctcaaatgt tccttccctt cagctcttcc
38221  cattttgatc tgatcatttg ctacaatctg catttgactt tctcatattt attctattct
38281  gtagtgattt agattagtag attagattaa taggctactc agatacacat attttctatg
38341  taaattctca agtcttatca atccaatgag taaactcata tattgactat cttttatttt
38401  ctaaagtcta aaaccatctc agaattaatt ttaatttcat gcttcatatg atgattttat
38461  gtacaaacat agaaagttgt attctggaat tctgaactaa agcttgcccc ccaccatgac
38521  aaacactgac aatcagtacc attttaaaag gtatcgacaa acactgacaa tcagtaccat
38581  tttaaaaggt atcaatggta aatgcctttc attcttgagc aaatgtgaca ttgctttcgg
38641  catgaaaaga cagatgaagt tttaggagct ttaaaaatgg tctaagagaa tagcagtcag
38701  caaatcattt tttagtaaat tgttttacaa ggaaaagcct tgttgatact ttctgagttc
38761  aataaaataa aaagtatact tttttaagta gtagacttac ctttccattc ttcaattgtg
38821  tgttctcttt catccaactg cttgtcatat atctgaggtg gaggctgccg aataaaaaca
38881  aaaaataatc tttgtgtgat aatgtaaaca taactaaact cttacaatgc acgagaatac
38941  cttgccctca tttatttcgg tgatttcaac cgtatataag ttatgaggaa attacagtag
39001  ttcagaagtt aaaattttcc tgtaaactta tggaggattc attcatattg caatatttgc
39061  tgctgaccca atattgctgc aatatttgca gcagaacgca aatcccgaga gctcactttc
39121  cgacatcttc tgatttagaa cccacgccta agcaattaat gactggtctt ttaaaaaaca
39181  gacaaaacac ttgattacaa tgRcccagaa gctacttttc cttcaYtcac ttgccttata
39241  acagaaccat gctaaaattc ttctaggcta ataagatttt gttctatttt tagaccttcc
39301  ctaaagaaaa gtttatattt ctcaacagac catagtacta gtcctcaata taaaaaaaat
39361  tgagatcaaa tcaacctttt tatttttcat gaaataccat agaatgtctt tatatattgc
39421  tgtgtgccct atacttaaac gattttaagc tttttggttt gtttgtttat ttgttttaac
39481  ctgcagaagc tgccatgact gtagaggtag gtcaccttga atccctgaat ggcccttggt
39541  tagtcctctc tagcagaaat gatgcaatag ggccttcagt ctgacttata gagccagcat
39601  ttctcttccc atgtggagtt caaatgtgga aaaggaggga ttttcacaaa cctgcgtgcc
39661  tactggaaag tggaatctca aataaattct ctgacctctt cttcctgata tttaggcttt
39721  gggagatcta ccatggcggc tagggtagct ccaattcagg ccaagtgata tggaaagaaa
39781  aaaaaaaggc tcttggaaaa ataaatcacc atgaggtaga gaaagaggag gctgaggggc
39841  tacactggct ggcaccggtt taagcgcaaa gaagagctat caagtttttg ctacccgtc
39901  caatactcca aaagcaatag ttttgtgaa aaactaaaag agtcactcaa taagagtgat
39961  gcaacccctt taggacacag acctttcaaa aatggctatt atagtaagga aagctctgga
40021  caagattttc agagacagct tctggcttct gtgagagctt tttggctcat gaacagcacc
40081  ctcgggctaa agtgacgctt ctctaattca tgcagaatct gtggtagctt gaatcatgac
40141  agacacaaat attcatccta gccctggcat gctggggcct cacatcctgc aaagtatctg
40201  gctatgaaaa tagaagactt tgcctggctg tggtgaagta acttagcata atactgaagt
40261  attgagaaat aactactcac ggagccagac attcttttag cacgtttgac tgatactggg
40321  gcagaaggct gtaaacctca cctactgcaa ttcagatgtg gctggctgag cagctctcag
40381  cagccgtcag cactgacaac taagcaaaat gagactctca caccacccca gacaatagta
40441  agaacacaac acattgaacg tatataaggt gacaggactg ggatagtcag gcactgctct
40501  caggacctga ctgaggtaga accttccacc tctataaagg actctcaaat aatagaaaca
40561  ttctgactta gtgagtgaat gagtaaaatg aagttatttt tgaaaaattg tgaaaccatt
40621  acctaaggaa gctaccacaa agccaaactc tggttttgta aagacgcttt gccaagtggt
40681  aaatttccgg ctttgcctct tcctgagcat gtataatgct gtggttaggc cgctatccaa
40741  gcgaccttgt ccaggacccc tgcaggacac agtgtacata agctctcaga gcccccagag
40801  ccctctgagc tgttggcacc tccttcttct tctaacaata gatccagctc atgatcctgc
40861  ctggctggct tctccatgca gtggattttt ccattcaagt tttaaaattg aacatggctc
40921  aagagtacag ttcctgccaa agttcataaa tgccattttc tttttgcaac aaagctattt
40981  tcaggaggtt tccctaggag cctcagtgta cagctgttgc tgatatataa agcacaaagt
41041  acatagacac taatcacaaa caaactacaa gagagaatgc aaagtgctga gttcaagatt
41101  tttcctttca acaaaccaag agggctgctt taaaccactt gatttaYagt atttgttcga
41161  cggcctctgc aaatttacta catattcaga tttttgattg tttgtttttt gaagaggaaa
41221  tgcgaatgtc acctccagtg aaagggcaca ttcacataca gatgtcttct atgtgacaat
41281  ataaaagaat aattgaccaa taatccagag agagcaatgt tacaacacac acgtataagt
41341  gctgggcagc aatcatcagg atgagatagt tcaaaacatt ccctgcctcc ttccgctaag
```

# FIGURE 2-L

41401 gagaagttgg tatttgagca ctcgcttgtg ctctttccct atcttattta ataggaatta
41461 ttctttttgct aagtgacaat ctaaataaaa aataaatgag gagtggggat aaagaaaaat
41521 gccccagagg tgtctgttga gagagtattt taggagagtt tagcagataa gagtaattga
41581 gatgtgagaa atgaaggata agaggacatt aaagatagta tatgaacagt tatcagggcc
41641 tgtgactttc accctttgaa tattttttca aaagactgta aacttctttc atattatttt
41701 ggaagatttg aacttccttg gttgaaatta ttgcagaaat tcaaacacaa atttatatgg
41761 attgcataaa caataactta aaatggatta gacacatttt tattgatggt taatttcata
41821 tgaatttata ttggtggtca attatatgaa ttttatactg atgaaacatg tccaaattgt
41881 ttcaattaaa tgaggaaaaa aacacaaaca agtacatggt cactgtgaat taataaatac
41941 tagaatttat ttatttcttt attaaatgta ttcattcagt aaaggtcttg ggcacctaac
42001 acatggcaga ctatataaaa aactgagatt tcaaaattga ataaggtgac tgctgaccac
42061 aaggagttca caatttactg ggtaagact gatgcaaaac agatcattag tatacaacat
42121 gataaatgat agaaacctgt atatggtcta agggaacagg gtggagaagt cctaacaaag
42181 cctagatttg atttcctgga gtaaagtgat gcttattcag tgagcgaaag atgtgtggga
42241 ggttgtcaag ataaaagaag gaggaaaaaa gtgactccat gccaagagca cattataagc
42301 caagttacag tatgtagaaa acagaatagg ggtggtggtg gtggtggtgg tagtggtagt
42361 ggtggtgcat gtgtttctct gtgtgtacat gcatgccaag aaaacaagaa ggaattttgt
42421 cacagtaaaa taaaacatgt cgtcagaagt gacaagatat aagagctgta cttagagaag
42481 cagaagggtc agacaaaggg tggatcaagg atgatccagg gggccaggcg ctgtggctca
42541 tgcctgtaat cccagaactc tgggaggccg aggtgggtgg attatttgaa gtcaggagtt
42601 caagaccagc ctggccaaca tggtgaaacc ccacctctac taaaaataca aaaattagcc
42661 gggcatggtg gcaggcacct gtaatcccag ctactcggga ggctgaggca ggagaatcgc
42721 ttgaacccgg gaggtggagg ttgcagtgag cccagattgt gccactgcac tccagcctgg
42781 gtgatagagt gagactctgt ctcaaaaaaa aaaaaaaaaa aaaaaaaatg atccagggta
42841 tcatgatacg gaagaaagaa gaagagaaac cactgaggaa tgtaagagaa cggaacagtg
42901 aaatttacag ctggattgaa gaaagatgta actgtaagca gagagaagag gtagggagcc
42961 attgcacgat ctataaaaaa atattgaaag ttctgactgc ggttaaaaaa aaaaaaagat
43021 gagggtgaac gaaagacaca gattcaggaa taattaataa atgttaataa ataaacttgg
43081 tagaagttgg tttttgattg aacatatgtg atgggagaga agaatatgag tccccaattt
43141 ctaagctgag tacctgggaa gatgatcgtg ttggtgaagc catctaaaga aatagagaat
43201 ggaaaagggg aaatgagatt ggtagaggtg agcctaagtg ttatgtgaga agagatgatg
43261 atttgaaagt taagtatata atctgaagtt cagaggaatg ctgttggcta gagatgtgta
43321 tagatttgag actaacagca agtgatgttt gttgaaacta tgacagtgga tgaggtcacc
43381 cagggaagat atgtgggtat agagagtaaa tggccaaatt aaaatttaaa gattggacag
43441 aggaaggtaa gttcactaag gcaaaagaga atagctaaag aaatacatga aaaatgagaa
43501 tattgcaatg ttatagaagt caagcaagta gaatttcaca aaaagctagt aattggccat
43561 ttcttgcaaa tgaagcacat gaggattttg actcgcaggg caccagaaag gcatgataga
43621 aagcatgcct ctgaacccct tgaaagcttt ccaaacttta aattgctatc acccccagaa
43681 agatccagat agatcttcgg tggaggacgt taatatgaga catcacctaa acaattgaat
43741 aaaatgtaga aaaagatcct caacaccaat ataattaaaa ggaaagaaac catagaacaa
43801 ttgtgacact agaaaaacta taatgatctc agatctactc tctcaagtct ctgagaactt
43861 gataaaaaaa aaaacactgt tctctatcta gttaaaggaa gtcacttttt ttctgagaaa
43921 ccaccatatg ttaagctgtc tttatataga ccttaataat cctcacagtc aggaaactaa
43981 gattcaagga aattttaaaa aatatatcat gaagtcatat agctaattaa tgatgaaact
44041 tttattaaaa cccaatttaa agagtaatcc agagcaagtg tcaatactgt tgaatctaaa
44101 ataaagacta ataaaacaat atttaaagtg aacatctttg taaaacaaga aatagaaaca
44161 taagccaata gggtgaccat atttcccagt ttgtacaaaa tagttccatt ttatgcttat
44221 tgtccttgta attattagta gcacttacgc ccttcgctcc tttaagtgtt gtggtttgga
44281 gaataaatat gtggacactc tacatagcaa tgttgtgaca tggcatgtta gagatctaag
44341 gtatacagta atttgaggac aaagaaaaca actgaattgc aatgtttttc taccactgag
44401 gtttcaacaa ttgcagctgt acctttttcct tctctgagac agcacgtcag catttccttc
44461 catttctct atttttagaa acgtaaaatt cagctataaa atatggctgt tggcagacag
44521 tacagacaag agtagaattc tgcataataa atctatagtt gaaaaatatg gattctgtgg
44581 gcattctatg gagttcttgc tgttggccat gactttctac taaagcgatc tctttaacaa
44641 gtacctgttc aattcctaat tgagctttcg aaggtaaaag ttgctctttc tgcctgtaat
44701 cccaggcctt tgggaggccg aggtgggcag atcacgaggt caggagatcg agaccatcct
44761 ggctaacaca gtgaaacccc atctctacta aaagtataaa aaattagccg ggcattgtgg
44821 caggtgcctg tgttcccagc tactcgggag gctgaggcag gagaatggca taaacttggg
44881 aggtggagct tgcagtgagc caagatcaca ccactgcatt ccagcctggg tgactgagcg
44941 agactccgtc taaaaaaaaa aaaaagttgc cctttcttgt gacttcattc atttatccc
45001 atatatataa gtaatattca ttgagtaaaa tgtgtgaatt tattttttac tgaatctttg
45061 agataatttt caaagataca tgggaagtat ctttttcaac agcattatag gatattagtg
45121 gtaaaagaaa gttcagagat tatctaatgc caccccttatt caattttcaa atgagaatat

# FIGURE 2-M

45181 agaaactgag aaagcataag tgatttgccc acaatcacag agttaattag tgacagatga
45241 atacatttat ggtctcttat gggaaaagac agaataatgg aattcttatg gttgaagaaa
45301 cattaaactg gataatttga cacctgtaaa agatattcat taaagacaaa tttgtgcctg
45361 ccacacactg atttctgttc caattttctg tcagtctaca cttacagcta gagctggcaa
45421 ctatcataca agcagtcaac acttacattc ttaaacactt ttttgtcaaa gtatcatgga
45481 catcaaagac ctctaaacac atgaatctct aaagagataa aaaccagata tgagaaatct
45541 aagttgtaca aagttgttcc tttgattctt ctttgagccc tgaactgaca tttttcccta
45601 acacttatgc tctgaaccat atgacagtcc atcaagttgt tggtgtttgt ctgctttgtc
45661 ctaattaagg atctccctgt gttctgctgc cttgtttaaa atgtcaacaa aagctttaaa
45721 tagcactgta tctcagtgtg tgcactcatt attaattcaa catctggatc aaagtaaatg
45781 atattatatc ttactgaaat atgctttgtt ttttcttatt aagcttggca gttagatgat
45841 tgtcaaaaga tgcatgcttt attaaaacgc tttaagcaag aagggtatat cttagtacaa
45901 aattagatag aacaggaaat ccattaagga aatatacaac attttgattt tatatttcct
45961 tgaatctcta cacatagagt tttctaattt tttttttctt aatttgatga ctaggacttg
46021 ttaaattttc accaattaat tagaaaataa aaattttggt aaaatgactc tgatgaagca
46081 caaaatgata tgcactttta ttgttttatt tggctaatgg cacagtttag catgtccaat
46141 ttttctctaa agccttatat tctctgtggc accaaSagca gttcattacc ccaaaatgga
46201 gctcagtatt tgtttcacaa atagtcttac caacaatcgc caaaataatg aaaatattat
46261 tgcaatatgc acaattatca aaagaattga ttagatataa ttaattccta tgatttctca
46321 gtaatgtgac cttactatgc attaagcaat gacctggttt ccaattacca aaaacttcat
46381 tgccttctga atgtagatat atggcatttt gtgatgtact atccgttgat tattaaaaat
46441 tgcttcagca agtttcagga actatgtctg tgttctcaaa cagaaattaa ataatatact
46501 actacagatt aaataaatgc cttttaaaag tatgatttga ggagatgtca gcaagatgat
46561 aaaataaggc tctccagtgc tccctctctc acagaaacat caatttgaac aactatccgc
46621 acacaaaaat accttcacaa aagtgaagga aaccaggtga gagatttcag cacctgggta
46681 tagcacggaa acaagaaaag atgcactgga gagagaggaa ggacagtttc acattacctg
46741 cttcactttt cccccaatta ccagacagca cagcatggag acagataccc tccacatgat
46801 ggtaggagaa ggaagtgagc atcagacttt gccttagacc ccaacactgg gcctgccaca
46861 gaaaaaccca gcaccaaaga gaccctcaca gccctagaca ccaggctagt acttgtggac
46921 tgagcttcca caccttcccc agcactgatc aggggcttat agccttagac tcaaggtcta
46981 tacagcagac ttggtctctg ggccactcta ctgccaggtc taccttagca gcccagcctc
47041 tggaacttct ccagcactgg gctgtcccct ataatcctgg acatcagatc catcccagta
47101 actgactatt tcctgcagac tcagactcaa agccaatccc agagccaagt agccccctgt
47161 ggacccaagg ataaggccag cctctacaga tgtaggcaaa tatttgcaga cataggcgtc
47221 aggtctgccc ttgtgtaccc aggtttcagg cctatccatg gggactcaat caataggtcc
47281 acacttgtgg atctagactt aggcccaacc ctatagaccc acccacctgc tgacccaggc
47341 acccagacac caagcccagc caacctacct gaggactcca acagcaggcc tacccttaga
47401 cgacaccaaa tggcatgcac aggttctatg gaaaggctga ctgataaaga gttttcccag
47461 acaaagccag tctgcaaaga ctggaataag cccttacttc ttcagatgca cagacatcag
47521 tgtaaggaaa taagaaacat gaaaagctga ggtgacataa accaccaaaa aaatacaaaa
47581 atctcccagt ggctgagtca agagaagtgg aaatatataa actaagagac aaaaaagtca
47641 aaataattgt tttaaggaag ctcaacaaac ttcaaaaaaa ttacagggaa acaatttcat
47701 aaaatcaaga aaacaataaa taaaacaaat ttaatagaga gattgaaatt atttttcaaa
47761 gacaaagaga aatcctattg ccgaatataa tgaatgaatg aaaaatgcaa tagtaggatt
47821 gatgaaggag aagaaagaat ttgtagcctt gaagacaagt tatttaaaaa tatatggtca
47881 gaagagaaaa aatgaaaaca aatagagaaa gtttatgtga tttatgggag agcatcaaaa
47941 gagcacattt tttagttcaa gaagagaaag acaaaggggt aggaagtatg tttaaggaaa
48001 taataacaga aatgttttca tatcttggga aagacatatt cacgtagagg aaagttaaag
48061 ttctctaatc agattcaatt caaacaagat tacaccaaga tatatcataa tgaaatcaac
48121 aaaaatcaaa gacaaagaaa ggatcctaaa ggcaccaaga gaaaagaagc aaataacatt
48181 taagggaatg ttcctatcaa gctagcagat ttttcggcag aaaccttaca ggccaggagt
48241 gtatgggaca acataatcaa aatgttaaaa taaaataaaa aaactatcaa ttaggaatac
48301 tgtaccccac aaagttgtcc ttcagaaagg gagagataaa gtctttccac aacaaaagtt
48361 gagggagttc accaccagac ctgtctcaca agaagatgct taagagagtt cttcaggcta
48421 aaagaaaagc attctaatta gtgacacaaa aacatgtgaa agtgtaaaac tcacttacaa
48481 agataagtac acagtcaaac tcacaacacc ctaatactgt aatggtggta tgtaattcac
48541 ctatatcttt accatgaagg tgaaaagaaa aaaactacta agaataacaa tagctacaat
48601 aatgtgttaa ggcatctaca accacctgat cttcgacaaa cctgacaaaa acaagcaatg
48661 gggaaaagat tccctattta ataaatgatg ctgggaaaac tgcctagcca tatgcagaaa
48721 actgaaacca gactcgttcc ttacacctta tacaaaaatt aactcaagat cgattaaaga
48781 cttaaatgta aaacccaaaa ccataaaaac cctagaagaa aacctaggca ataccattca
48841 ggacataggc atgggcaaag acctcattac taaaacacca aaagcaattg caacaaaaga
48901 aaaattgaca attgggatct aattaaacta aagagcttct gcacagcaaa agaaactatc

# FIGURE 2-N

```
48961 atcagactga acaggcaacc tacagaaagg gagaaaattt ttgcaatcta ctcatttgac
49021 aaaggtctaa tatccagaat ctaccaaatt tacaagaaaa aaacaaacaa ccccatcaaa
49081 aagtgggcga aggacatgaa cagacacttc tcaaaagaag acatttatgt ggccaacaaa
49141 catgtgaaaa aaagctcatc atcactggtc attggagaag tgcaaatcaa aaccacagtg
49201 agataccatc tcatgccagt tacaatggtg attattaaaa aggaaacaac agatgctggc
49261 gaggctgtgg agaaacagga acgcttttac actgttgctg ggagtgtaaa ttagttcagc
49321 aattgtggaa gacaatgtag tgattcctca aagatctaga accagaaata ccatttgacc
49381 cggcaatccc attactgggt atatatccga aggattctaa attattctgt tctaaagaca
49441 catgcacaca tatgtttatt gcagcactat atacaatagc aaatacttgg aaccaaccca
49501 aatgcccatc aatgatagac tggataaaga aaatgtggca catatacacc ttggaatact
49561 atgcagcaat aaaaaagaat gagttcatgt cctttgcagg gatatggatg aagctagaag
49621 ccatcatcct cagcaaacta acaactagca caggaacaaa aaaccaaaca ctgcgtgttc
49681 tcactcataa gtgagagttg aacaatgaga acacatggac acagggagga gaacatcaca
49741 caccaggacc tgtcagaggg ttgggggcaa gggaagggag agcattagga ccatacctaa
49801 tgcatgctgg gcttaaaacc tagaagctgg gttgataggt gcagcaaacc accatggcac
49861 atatttacct atgtaacaaa cctgcacgtt ctgcacatgt atcccagaac ttaaagtaaa
49921 attttaaaaa aaagtgttaa gggatacaca atataaaaat atgttaattg tgacatcaaa
49981 aattttaaat gtgggaggaa tggtggagta aaagtatagt tttttaaaaa tgcaagcaaa
50041 aagttgttat cagcttaaag tagcttgtta taactataag atgtttttgt aattctcatg
50101 gtaaccacaa agtaaaaatt tatggtagag acaaaaaaga tacaaagtag ggaatcaaag
50161 cataccacca gagaaaatta ttttatcaga aaaaaaagac aaaaaaagag gggaagaaat
50221 gaacaaagaa cctacaaaac aaccagaaaa caatgagcta aatggcagta ttaagtcctt
50281 acctattaat aattaccttg aatgcaaatt gattaaattt tccagtcacc ttggatgtga
50341 agggggctga gggaatagaa agaaaagact ccaaggtatc tcattatcca gatgaaaacc
50401 tggatagaaa gtagtgccat gaattggaag gaggaagcat agatttggtg tRgagaggag
50461 atatggccag tttaatattt aatatgttga ttttgcaatg ccaatttcaa ttccagtgac
50521 tgttagattt atacatctgg aactcaggcc agctctatgg gacgatgtgt agaattgagc
50581 catttatgat aattaaaatt aaggctgtgg tggaagataa gactcccaga gagaaaagaa
50641 ataagggcta taaagaaaaa atttctgaga tgcacaattc taagggaaga gagaacaacc
50701 cacattgagc aatggaggct gcaggagaac aggtgtgctg ccaccacaga aattaacatg
50761 caggggtgca gaaaggaagg tgttgtcagc ttattcaaat gggcaaatac atgagatacc
50821 catttttttg gacttggcac ctaagaggcc actggtgaat ttagagaaag cagtgtcaat
50881 gtttaaaaag tgaactggag taattgtgag taaacaagga ctaatgtttt atagagactt
50941 ggctataaag ctataagtca tacttccatt gctaaaggta gaagtgtagt taaaaataaa
51001 ggtttgcttt gattctttgt tctttgaaat gaaagagact agaacatgtt tatttgtaag
51061 aagtaattgc cactggaaac tgaaacatag gacacacaca gagaaggaag agacccacat
51121 gcagaaaaca aaggctataa ataaaagagt acagtgtcct gggttttcct ggatcttgtg
51181 taaatgactc catcacagca ttatcatgtt ttcttctcaa catcttcatc attctactac
51241 cattcgtaga aagtggattt tcatatatta tctcatttag tctttgtgac aaacctccta
51301 ttccaagtat gcaactgagg cacacaaaga tttaaaaatc tatgagttag tagcagaggc
51361 catatttaaa cccaaatcta actgaataca atgtctgtgt tctttgcatt tttccacccc
51421 aggcttatag ttcctactaa ttcaacctat tgaaatatat tttaacgttt tattaggcaa
51481 acaaagcaac agagaacagc agggaaaaag gcgactttat ctctagctga cggggctctt
51541 gaaatgctgt taatcaggat tctagataca aatttccacg tccactcaga ggggcgagaa
51601 tgtgcaatat attgtgatgc tgccaccttg tggcaagaaa aaatgaaaac attaagaggt
51661 gctttgtgtg catatttatc attttcaatt aaattgctct agtttatggt tcagctacgg
51721 ctgcacattg aaatcatctg ggagttttaa aaatactgct gcctagatcc cacctcagag
51781 attctgattt aattgttctg gggtgaggcc ttggtgtcag gatattttta aacttcccaa
51841 atgattctaa catgcagcaa agattaagac ctctgctcta accacttata cagtagagaa
51901 gcaagtcttg tcatttgcct agttatgttt catttgtata gacgcaaaag aaaacattct
51961 agagaaagta ggtgattttc tctcaatctt taatggcatc tagttcttgg tagaatacaa
52021 tgatttctgg acagatatat catatactat gatttaaag aacaggacaa agaaatttgt
52081 gaattgtaat caaagtggac acaagatttg tgtctcataa tcaaatgcag tccgtagact
52141 aaagtgagca gggagaatct ctgagcctca taatgcgtca caggttttct gaaaggaaca
52201 atggaaagag cactgaagca ccattgactc tggcactagt aagatgagtg accttgagca
52261 agtcactcag tttgcaatga aagattttca tctactgctt tattcaagag aaactagttc
52321 caaatgttca acaaatatca taccaggtaa ttcaacaaaa ctttcttaag tacttgcaat
52381 gcgaaaagca tggaattata atgtgttata aatggaaagc actaatgttg cttagagaca
52441 tcatagcatt aggtgaccag aggaaccttt gaaagctagt aatgcaaatt ttaattatga
52501 tctgaactga gtgtaattag aaagcttaat tacataatgc tccagtggtt tggcaaagaa
52561 gttgaattca gtttctctca tttttcattc tgttctgtag ttttctcaga tttatttatt
52621 agctctacac catcaaaaag tgtaatcatg tcttgaaaat gaaatttctc tttcctgctc
52681 cttggaaatt aaaattttct atggttcttt aactgaaaag tttcagtaat tgcaccacca
```

# FIGURE 2-O

52741 atcacccagt gccttggtca ggaatctttc tttcttcatt cgttcattta acacatattt
52801 attaaccctc taccatatga tattactcat ctttgaattc cacctccctt aaattccaaa
52861 tttaattatt tacaattcat gtcaattatt agcttctaaa catctcttga atattctgYc
52921 catgtctaca gtgatttaga ttactacaga tctatcctcc tgccttctag ctccctttc
52981 tcaactccag tcccacactg cagacagtgg ccctttgaaa cttcaaatct gctaatgcta
53041 ttgccccact tcctagtgaa tggcttccct tcaccctagg atagcatgca tggtacatga
53101 caatgcttgg gaactccttc cttatctggt ccctgcttac cagcccaaaa taatttctca
53161 cccttctcca ttaagtcact cagaaagctt aaagctttgc aattctctcc accctctctt
53221 gaccctggga tttttgcata taccctttct tctgcttgcc tttcttcact tggataattt
53281 ctttttagc ccttcatgtt cctgtttgaa aattaatcac tccaagcagc cttctgtaac
53341 ccacattact gggttatttt tatgtgtttc caatgtacaa taagcttaaa atttggaata
53401 cgtatcatca acatttactt atttatcttc tcactagacc atgggacaaa gaattgagaa
53461 taaaagacct aggaatccaa cttacaaggg atgtgaagga cctcttcaag gagaactaca
53521 aaccactgct ctctatttgt ctgttattcg tgtataagaa tgcttgtgat ttttgtacat
53581 tgattttgta tcctgagact ttgctgaagt tgcttatcag cttaaggaga ttttggactg
53641 agatgatggg gttttctaaa tatgcaatca tgtcatctgc aaacagggac aatttgactt
53701 cctgtcttcc tatttgaata cgctttattt ctttctcttg cctgactgcc ctggccagaa
53761 cttccaatac tatgttgaat aggagtggtg agagagggca tccttgtctt gtgctggttt
53821 tcaaagggaa tgtttccagc ttctgcccat tcagtatgat atttgctatg ggtttgtcat
53881 aaatagctgt tattatttg agattttggg ctgagacaat ggggttttct agatatacaa
53941 ttgaaaatct agaagaaatg gaagcacagt atatagtaca tagtagatat ccaacaagta
54001 tgaaccattg atgaagaaca attatttgca ttcaaaaatc tacgatatat taactcggaa
54061 aaattggtca ggtaaagaga tgaaataaat tatgacatgt ctgacattac tcaaataatt
54121 tttaaaaagt agattccagc ttttgatata cacatattag ccacacaatc atgggcaatg
54181 tgcttaacat ccattaacat cctgagcttt agtgtcttca attctaaaca cagatacatt
54241 cattcagcaa atattgatta tatgactact agggccaga cagtgttcta ggtagttggg
54301 atacagcagt gccccaaaga gtcactgtct ctctttttac gcatcttaaa ttctagttga
54361 tgagacagat aataaaaaga gatatataat gtcaaacagt gacacataga gaaaaaagat
54421 taagaggcta gagtgtaaca gagtactatt ctatttttc tttttagttt ttttcttttt
54481 gtttttgttt tactttttaa actattatta gtattatttt tacaaacact tgtaccaagg
54541 gctgactttc aatagatcac agcaaggatc ccctctgcta catatgaaac cctgacccag
54601 aagcaggtca tctacttatg ttttagtgcc aggttacaca cgaatgtgtg ttacatgacg
54661 ggcaaggggg cggccgaaga gtactatttt agaaaggatg gtcataaaag gcttctttga
54721 gaaggtaaca tttgagcaga gatttgaatg aagtaaagga gggagcaatg tgaatatctg
54781 gaggaagaat gtcccaggca gaaggaaata caatgcaaag accctaaagg gggagcttgc
54841 ttggtgtggt aaggaaaccg caaggatgcc atatactggt gcagagtgga aaagggaagc
54901 ctaaaggtga tatcagaaaa gtaggcagga gccagactaa gtagagccat gttttccaaa
54961 ctgcagtcat gaaaacaaat tggtgtcatc actagcacct tttagtgtaa ctaagaagag
55021 taaaatgaga tagaaaatat cagtgtatgg tttttctttt gtgtgtgtgt acgtgtgtgt
55081 gtgcacgcac gtgcacttgt acacgcatgc tgggttgcaa tgtaaaacac ccttcttact
55141 atgggttatg ataaaatttg aaacaacact gtggtagggc cttgcaagcc atgataagta
55201 ctttgagttt tgttataaat atgataagaa gccattggaa gatgggaagg tgttagctga
55261 ggaattatgt gaatgtaacg tgttttacat tttaaaagga aaattctagg cattctgcag
55321 aatggaccac aagggacaag agtggtgctg agagataagt cccagaatat tacatggtcc
55381 agggtagaaa tgatgtttgc atagatgaaa ggatgagaag tgatctgagt caaggtatat
55441 ttagaaagta agttcagtta agagttgttg aggatagtca tcattttcta gagtttttat
55501 gaggcttaaa taagattgga tatcagaaag ggttttataa acactagaga agtgctcagc
55561 agagtacagc tgagttcaat tcaaagggca tttactgacc atacactatg cacctgccat
55621 tactctcact gttttctctg cactcggtag ataaaactga atttctgctc ccaaggaact
55681 ttacagcagt agagttgtgg aagatagaca taaaaatgaa tcactgcagc ataatgcaca
55741 atatgataat ggcatatgga aggttctaaa gaaaatgagt taacttaaaa ttctgagaag
55801 cagtagcttt tgttgcaagc ctttcttttc ataggtaaaa gatcacatta gccccaagga
55861 tattctgtag gacaaatatg agctaaaaat gaactagaaa atagctctag gtgaaaaaca
55921 catcgaacac agatattgct gagttagtct attaaagcca catgtcattt gcacttttgc
55981 cctacagtca caaaacaaaa gcaaatgaac aagggaaaga ggatcttcag ccctcctaca
56041 taagtaacat gaatcaataa ggagtcatcc tggagaccac agaaaaccac actcttccag
56101 ttagcatcaa tgaatagccc caggatacaa atccacagtg aatgaagaaa catagctaca
56161 gaattagaaa cattattaag gaaaattgat ttgagtatac ttcactaatg ttagagggat
56221 aatattatat tttaggttga aatgtaataa ttttttaat tccaaagtgt ctattaattt
56281 taaaatatac atactttttg ttacaaatat ttatttttcc ccttcatacc actaatctct
56341 gtgaaaaagt ttagaatgtg acataaaggt tctaggttaa tgaagtcttt caatcaagca
56401 taaagacttc tgctaatcta gtaaaaaccg ccccttctac ctacatggat tcaatatatt
56461 tgttgcatta tataagaaag catttgtgta ccacatttac atatgagatg tttatcagaa

# FIGURE 2-P

```
56521 agaaagtggt gctctgagga gagggagatg gggaacagaa tcacttgaga gtgaggattt
56581 atttccaatt aattaacatg cccacaagaa tctgccatac cccacatgaa gaatcattgc
56641 tttaatatag attgatgagt tgataacgtt aatgagttga tgactgtctc tcaggtatgt
56701 tggggaacaa aaaagtttga gaaacaaata cagtcataaa gaactatatt ctaataataa
56761 ggcgttacca ttgctcctac tttttaatgt attatataca ttattatata atacttttta
56821 atgtatttaa taaatatatc tcatagtgga ttttcttaaa acagagcttg ccttttcatt
56881 aagtaattac tccaaggagt aattttaaaa gaaaataagt ctaactgagc ttttgtgtta
56941 acatgatagg aactgataac atttccattt ttcttatgtc acatagacta aaactgttca
57001 gtttaaaata gttcaattac ctccataagc tttaagaaaa ccttctttaa taacatccta
57061 tgttttcaaa gttaattggg tactttcaca tttttttcct ttaaaacatt attagacagg
57121 acatttcatg ggtaaagatt agtgatgcaa ttataccacc atctctgaat gtcattcttt
57181 cttcatctcc attgccattg aagtccattt tgtcatcttt tatttcaact acttcactag
57241 cttcctaaat catctttcta ctattttatc acttcatcag tcagaaagaa tgatctacct
57301 aaaacacgaa tcccattgtg ttactccctg attaaaatct gttcccccaa gcctgcagct
57361 tgggtaccct ctgggaatga cttaaaaggt gtttcctgac caggaccctg cttacatttc
57421 cagctgcatt tctcaccatt cccttccctg ctcacagtga attaactcat agttccccag
57481 gtgtgtcctg gttcatcttc tctcctggcc attgagcagg ctgcttcctt tgcctaccag
57541 attctactct tccacctcca tttcacttgg taactcttgc ccatcctttt ggtcccattt
57601 gaggcttttc ttcctcctaa aaacctccta tgtctgctat aggatgagtt atgccttatt
57661 cctttgtgtt tctatggcac tagtactatg gttatagtat cttacttact tacatattaa
57721 ctctactaca aactactaaa taacagaaac tatgtctttt tatcattaga attagtcaat
57781 cttcaatggc agcgtacact tgaagcttaa caaaagttta agtgaatgaa tgtggtggac
57841 ctgtccaagg tatgatgaga ctttgagtcc atgtcctcta attgttgacc cacagtggtt
57901 tccactgtgc tctactgtct cccagtgcca tgaccttggt ctccacactc tgtaactctt
57961 ggtcctcctc ccattgtctg tctacagtcc tttgctttgg gaaagctaga ctaagtaagg
58021 caatatttta aattaactaa ttgcaagcca agtttttgc ctggatttga gcttagcctt
58081 ccattcttgg gggagctatt cattacttaa gaaaacctac ctgtgactat gcatgtaaga
58141 attctggcta ggaataaact attgttcatg attaaacagg aatggtgcaa gaggcatgag
58201 caggcctacc tcagaaaaag aagtggtaac agattgctgt actatgaaaa cagcattctg
58261 gcatatatgt cctgaaagaa agtcaccaaa agttctttac gctaacttag tcaaaaacaa
58321 cacatacctt aggtaagaaa ttttcagaaa aagtgcttga gggttagcta atttatctct
58381 cactttttac agaggaacat cgaaggaatt cagctttcaa aatgcaccta aagatagaaa
58441 tcagattctt cttggaatta gaaactgtgg ggagcatctt ttgattgttg attgaactca
58501 tgttgtttta catacattat attctctttc acaatatatt gtgggtcatt attctacaaa
58561 caaccctctg agtctcatat gaaacctgca gcaagttatg gaagtggctt ttatcattct
58621 tacaattgcc ctaccattag aaagggacat aataatttta ttatagcaca tactcctgga
58681 aagaccataa acttttaata gacttgtaaa aagggagtgt aacctttca atattctgtc
58741 tcatctattc taccttctcc tcacatataa cgaatgccct agggcctaag tcatggatct
58801 acacttggga agaaaatgtt gagattcagc cttttccctg aatctgaagg ttaccatgat
58861 ttagcaccca ggaatagttt ctgcctatcc tttttctggt tcttttgcag ctctgcaact
58921 tccagacttg tggtctatgt agagttgtga tgacattccg acttcatgtc tatagctttc
58981 atttagggat ttttaaatgt cagcataaac atcctacagc gtttaaagtt gtcttcacat
59041 caccacacat attcattttc agcatcatta atgattacac tttctgatta ttttcctttc
59101 tctgctagac tgtcaaactt cttagtttac ttcctaatat tgatagcatc accagctcta
59161 agcatgactt ctcaaacaag atgtttggtt aattttctgt gtgtttttgt tgttgttcat
59221 ttgatcgtct caactttttg ttgcaatgca caacataacc accttcttag atcatggaca
59281 aagaaacaaa caaaaatctt tggagacaaa catggcagag gaaaataaat atcgcaaagg
59341 aagtgagtgc ttccgaaaaa caaagtggaa acaaagtatg aggaacccct gaaagcctca
59401 cagcataaga aaagaactag ctacttgaag agtagacagt ggctattaat ttctatttct
59461 tgaaaaaaaa ataccatttt aaaattgtca gggaccacaa gaaactgttt ttataatgtt
59521 aatagttttt ttaaatcaca ataataaaaa ttgaaagcat aatattttac atttatcagt
59581 tacttctgcc tccaccactt tccttaaaaa aagtaggact tttacagatg aaagagcatt
59641 ttaatacaaa ttttaaaatt acccgaagac aataaattat ttcctaaaac gtgtattaaa
59701 tctttttgtt atgtctataa atgataatct agtctaagag taacttatca gcatttttgc
59761 agtcccctcc ccccatccac tgaaaacttt ctttactaga ctcatgattt tcaaaatttt
59821 aagaattaaa tttggagtaa aggaactccg aagatctgaa aaagcctgtg aactggatgt
59881 ccagaaagat gtaaatacat aattattgat agaaaatttt gggatgtttt tcaaaagccc
59941 ccagaagttc ctctaaggaa ccccttagggg tcataggaaa gctcagtgct gggtagtgag
60001 ctcctaagag cagagaaatg ccctcctgtg tgcatctcta gatctggtac agtttcagca
60061 ttcatagggg tgctataaac atttgttacg ttgaatcatg gaggataagt atgtatatac
60121 tcgggtgttg ttgttgctgt tgtttgagat agagtcttcc tctgtcacct aggctggagt
60181 gctgtggtgc aatcttggtt cactgcaaac tccRcctccc aggtttaagc cattctccca
60241 ccacagcctc ccaagtagct agtgtaagcc accatgcctg gctaattttt gtatctttag
```

# FIGURE 2-Q

60301 tagagacggg atttcgctat gctgcccagg ctggtctcaa actcctgagt tcaagtgatc
60361 cacccgcctt ggcctcccaa agtgctagga ttacaggcct gagccactgt gcctggaggg
60421 tatttttttt ttaaaaagaa gaatgtagta tttaaaagtt catcatgcaa ttaaacctca
60481 ttgattttga gagcaaagga gcaaagaaaa atctggcaaa tagcaaacct actacttaaa
60541 gtaaaccttt aggaacttaa gcagggctgc tcaccaaata attctgctgg gtacagaact
60601 gatgtggcct ttaaattaca gagtttatgt ataataatta acatccaggc cttgcagaaa
60661 acaatattac ctctgcagag catatcattt tctttattga taaatgctct ttactgaaaa
60721 cacaatgata tgaggatggg taggagtgcc ttagagctct ccttgaacat gtatacacag
60781 tggttaagag gagattcttt gatgatggac aaaaattcaa gtctagtttg attgttttag
60841 caacattaca ggtaagcaaa caaggcagga atgcaaatta ccaaaagagt tatgcttcct
60901 agagattccc caattttgta gcctctactt atctcgaggc aaagctgtct gacagagatt
60961 ctgtcttctg acatactcct attcttttct ctttttattt cttcctctta gtataaaatt
61021 tggtaaattt tagttaacag aacatctcac aagttcccac cctgacttct ttgcttattt
61081 catagaagga atSctaaaaa agaaaaatat atattttttt ctcccacagt ttatggatta
61141 catcctatgc agatgacacg ttggataatc tctttttttc caggatgaaa catattaaga
61201 aaataaaata cactctcaca aatgcccact acctcaagat aaataggtag atctcacaat
61261 atctcagata tcacctagga gagaagaaat aaaatcaagt atttggaaga acctgacaga
61321 ataccttagc catacctcag cctttcagag agcaacaggc atgaaatgtt ttagaaaaat
61381 aatttctgtt cccttccccc ttttcataaa gtattctctc tcagtgatcc attcaaaaga
61441 ttaatagcac tcatggtcta aagtgttcaa gataaaacat gaaaacaaaa accaaagcat
61501 ggattctact agacaacaaa acatgtctct cctaagaatg aacaattcaa ccattccagc
61561 ttttcacacc tgagaatctt ggattcagtg gattctgaat atcccaatga attaaaatta
61621 agatgaacct ttctcttcag gaatagaact tgctaaaatt agagataaat gagttacata
61681 ttttcagtga aatacatatt cctattataa gtcatatagg gagtgaggct cctaaaagca
61741 ggtggctaac tctatctaca acaaacattt tgacaagctc ccaccaacat aatacatggc
61801 atcaaaacaa tccgaaggca attcaaacga tagcttcacg ttaagcagct gaggcactgg
61861 gcaagcaaaa tctaatgaag ctatattttc tctggaaggc aattagtaaa ctgctcatat
61921 tttgctgctg aaagaataca tcagcatgaa cagatggaaa tttagaactc cacaaatgga
61981 attacagaca ttgatttaac tttccccaag caaatgaaaa ccacaaaata cgaattgctc
62041 tttctataat taagcacata attagagtgc atcagattca tatccattgt agctcatatt
62101 tcttcctcct catttctaaa tttatatata ttctgagtct aacaaaccta cttagagttc
62161 aactccacac tgaaagagtc ttctatgaca gatagatggt actaaactat ttctcaaatt
62221 aagcctgcca tgggtcacac tagcttttc acagcatgac acatgcggca ataataagaa
62281 atccaaagac tcaaagaggt agtccttgtg gcaggacaga tggagagaac agctgaggga
62341 ggaattcagc cagtttatta actccatgtg agtagaacaa atcatattta aaagaattca
62401 taaggacatc tggtcgaaaa acagaattta aatgatggca gttttagaat ggagaaaaga
62461 gaatcatgat gtctcacagt acaatatcac tgagttacct ctttgttttt ctacttccag
62521 gaaacaaaag gtcaccagtg acctgcacac agattataga ccaaactggt gRtgggcact
62581 tgggaaatac ttggactaat tgtcaagttt aaaatataca agaaatacta aaacatatta
62641 tggaaataaa atcactaaca tgctaaacat tacagatact tgaaattatt cacattgtta
62701 attattcttt tcctcattct aggatacagt agtttgtctt acttgtgaaa atatttaggg
62761 gaacttagat aaaatttttg tttgatattc ctaaaatgct agaaatgacc tcagaaatct
62821 ttttgagaat aagcccttta taaaaacctc caaataggtc tttcctgaga aaactcaatt
62881 gatgtgcttt atcaagtata atagtatgtt tcatttataa gctttccaag ttgaaatcca
62941 aagtaataag catgtttcaa aaaaaatcta aacaagtaaa aatggggaaa cttactaaaa
63001 ctgtgcacac atatatagca tcaagaaata aactagcagt aaaatccaag gtcatattca
63061 gtgtgatagg cactgcagat cttttctatt aataaaagc aacagaggct ttttctaccc
63121 atgaaatgtt tttttaaaag agctttctta ttattttagt gaaatgaagt ttttccttct
63181 taataaaaat tttcaaattt ctaccaaaaa aaatccttaa gtaactcaag ttttgcagtt
63241 tatgcttatt taaatatttg ataggtattt tctgccactt gagagttgct attaaatttt
63301 atataataaa cttcacttgc aaactgactt ccttctcaga caaatcaaat gacagcaaac
63361 aaaaataatg cctattaact attttaagta cattactgta ccctgtgggg aaaatcagga
63421 aacaaaaact gtagtttcag aatttcagat ggattagtta ttcagaaatt gtcttgagga
63481 catgccctaa ccttttgata tcactcatca aaggaaaatg tgactcacaa agactttaat
63541 cccctttcct cccaacggga gatacagtct gtatagagag ctgtttgcat ttataactca
63601 Yttaaatcat gtcttccaga tacttcaggt ttaactcaca gtgctacact caaactaacc
63661 aatttaataa aaggattata aaatatttga agcaaactta tgcatatttt attcaatggt
63721 ttatctccaa acagtagata tctactgatg aactatttga acattagatg catcaaattc
63781 atattcattg tagctcatat ttttccttat cattcctaaa ttcatatata ctgagtctaa
63841 caaagtacat tgcttctagt tataatcaaa tatatttata agtctattct tcaccatgtt
63901 gtttcaggaa tttattatgg attcaagaga attcagtaaa agacaactgg ttaagccaca
63961 gttaataaag gtagaaacaa tgcaaacaag aactcattca tggagtagat ttaatagagc
64021 aagtaacaca ggatattcat aatcaaatga tattagcttt tacagtaaaa atgtatttga

# FIGURE 2-R

64081 gagaattaaa agtcagtgat aataaggaat gagaataact ccttcccatc atcctcagtc
64141 atgtgaacta tgctcaaagg cttcagatgg tcaataaatt aatacatgaa tctttaaaat
64201 ataaattaaa tatttttatt tagttgctat agtaaataga aagatttaat ttatagttta
64261 aagattgtat acttttatgt ttagtatagc aactttagga ttctttggtt ttcagtaata
64321 atctatgcat ttgggtaacc aaacttagtc agcttggtca ggactttttt gtttatttat
64381 tttattttat tttattttat tttatttatt ttttttaaga cggtctcact ctgtctccca
64441 ggctggagtg cagtggtgcg atctcggctc actgcaactt ctgtctcccg ggttcaagcg
64501 attctcctgc ctcagccttc ccagtacctg gcactacagg tggctaattt ttgtattttt
64561 agtagagatg gggttttgcc gtgttggcca ggcccaaact cctgatatca agtgatccac
64621 ccgccttggc atcccaaagt gctgggatta caggtgtgag ccactgtgcc cagcaggaca
64681 ttatttttata ttcagtcaaa taatagttta aaaatcatag ctaaaagtgg ataagcataa
64741 cttgttaatt atgattcaaa gcagttgatg cacatctggt tgaagattag cagattctca
64801 ctgcagttta gttataactg attaacattt caagggacat aggatttcag attctacagt
64861 caggaaacca tacaccaaac aagttaaata atcaagtaag gtagataatt catatttgga
64921 ggagtttatt ttgtttcttc aggagatgat aagttatcaa agattctcat tcaatagaaa
64981 ttgctattaa ctattagaaa cctaccaaga acaataaaag cagggacctg tttttttttc
65041 atttttttgtt taaaaattca atgctgaatc actatcatct aggtcagtgt ggaacacaaa
65101 gtgtgggttc agtcaatatt tatagaataa gtgaataaat actaaactgt taacaaatag
65161 tcaaagctta tgtgaataac tctgttggat aaaaaagaga aaagggtaag aagtgaagaa
65221 gagataaaaa aggaagaaat aaaaaagagg tgagtgagag gaaagtgaaa agttctttcc
65281 tagtgaaaga ggtctttcct atggcagcag aagagtagat ttacagcaac ctaaaagaaa
65341 acagatggct gaatcctttc aYtcccctta cgtacatgtg gtccaacatt gtctcaacaa
65401 gactgtagcc ctacaaccaa caatcgaaag agtgacatca acacataaaa ggaaggaagt
65461 gcacattcag tggaaacccc aatgagaaat acgcttctgc caagactatc aggttggtat
65521 agtacttgcg atgtcttggc aagaaatcat gaaatacttg aagaaattaa ataattatta
65581 gggggatgct tacttcactt tactagggga ggtccttgag tacctcttct aacatctaaa
65641 gtcttctcct ctccagagga attgtgctcc gttccctggt tacttctcct ttacttaggg
65701 atttcagaat tgtgaataag gaaagaatga cctggtaagt gccagccctg gacacatttg
65761 cccacagatg ctgtcctccc cctttcttta gttgctcttt ttctcagggg gctggactct
65821 attaggccat atttcccaga tttgtctgtc agttgcctta ggKttggtct cagccaatag
65881 gagggagata tcaccaggag acagagggga gaatctaggc catttctccc caccctttc
65941 tctagaagca gctgtatctt acctgtgtta gtgactctag ctcccattgg acaggccatt
66001 actacctggg ctccagtaaa tcgcccccta gctctcaggt tggtagtggc ttcccatttt
66061 tgcttatctc tgtgttggtc caagagtgtc tgcttttgac ttctgcattg caatatgaga
66121 acatccttat ctctgacacc tttaaatacc ccttagggtc tcatctactt agatctctga
66181 tcttagacca catgtctctc ctgactRccc agctggtctt gactgcttgg cctacatatc
66241 tgcatcagct gtctctctgg aatgcccaga tcaagctctg gaacctgttc tactttgacc
66301 acaggcttct gattcccttc tctctttctt ctgatcccRt ctagctctat tccattcact
66361 tcatcatggt tgctggtcct atagctgctc aaagttgaaa agttgctact ctgaatgctg
66421 atgtttgctg aactgcagga caaaaccact ctataattat ctctaagttc ccacttcaga
66481 tttaccttga atagatttta ggcataatag gcatacacaa tatcaaagtt tacatatttg
66541 tgtcacaaga agattaaaaa taaagtatat ttcccacatg attggtcatc ccttccatgc
66601 atcctctttg gtaatatcca gtaagatata atggcagaca agaagatgga taaaataatg
66661 tcagatagta actctgctca cagggcatgg aatctatgat ggccttgtaa taaaatagct
66721 taatatagcc aggttaactt gtttctataa attctttcac cgtaggcaaa aataacataa
66781 tccctaaagt agtgaactag taaataataa taattaagca tttaagaccc tcttaatcac
66841 tttacattat aaacaaacag taaatgtgta atagtgttga ctattctggg ctcatgtttc
66901 ttaagtgttt attttaattg aacttcagca ctaatacatg taactgttac tctgtttccc
66961 aggttatact ctccctggga atctgaagat catgttgttt aattcaggat ctgtatcggt
67021 agattaaatt cccacccaac cctgtcccaa ttcccaaatt aatcttgagt taggtgaaaa
67081 aataacaagc aagctcagca ctggccctgt ttaccaaatc accttccagc caaattggtt
67141 gaattagtta caccttttct cttagtctag tggaggatgt cgtgtgtcct tagattccca
67201 aacaaaacat gcccgctctc tctctgtggt ctacattata gctggagtac aatttccagg
67261 cctcatttcc agcattctct tctggaactt tcatttggct gccacacaga aggccctatg
67321 tttcctgcca gctgtcctct gggttctcaa gtaccccagg ttcacctact agagaaaaga
67381 attccaaaat gccagctgcc caagcagctc tacctgcctc tctgccctga ttcccagagt
67441 aaggtttcag ccattaagaa ctgtctctgg gcatctctga aacacagcct tcaacacaat
67501 ggtctatctg gttgatcacc actggccatg aagcttggcc tcaacgcctg tcccatctta
67561 ggggatggtt ctaatctcag gtctagctcc ttcctttag tcaagaattg aggaatcatg
67621 aagtgttctc tgactccata tgtttgtact aggcttcttc atgatgattt atttgagttt
67681 tgatgtactt ggttttggct gaactcacat ctccctctcc tgctccctct ccctatgccc
67741 ttgcaaggaa caagaactac aaaatggagc cttttgtaag gccgttgcac tcaggagctt
67801 tcttgaagtg ctctgaaatt tctggcagaa ataaagctat ttaaaaaaaa tagttacagc

# FIGURE 2-S

```
67861  ttatgttcct cttcttctgt gccctgagaa atagttaatc aactcttcct tagcttggag
67921  ttcacccaga tgctccttaa ggcttatctg tagctccact ctcagtggta aagccaccac
67981  tggatcctag cagggtgcct aaactcagta atgtggtaag agataaggca acaaagcttc
68041  atgaacattg tattttcttc caacagggca caaggaagat attaaactga gttcccccca
68101  aaatcccaaa aatctcctac tgataatcaa gatttgtgga atctataatg tgtctttatt
68161  ttggtttaaa ctaatgacac tagattttct tctacctggc aaagaagagg taggtataga
68221  ttaaacaaaa gaaaatgaaa aaaatggacc taaaatcatg tgggaaatag atcattgtca
68281  tcattgttca aaaaagaaat gttgtggaat atttctgcaa tttcctggcc agctagtctt
68341  tttcaccaga cgcatatatc tttttcaaat atcaaaagat agtaaagcta tgcatgtaaa
68401  agtcataata gtaatagtaa ttataagtat tataagggta agaataattt acatgattct
68461  tactgactcc caagcatttg ctaagcactt taactttttca aaatctcaga atgctatgct
68521  gtacatatga taagaaagga gaactttctt agtttagaaa actgagttgc agaaaggtta
68581  agcaacttga tcaaagcaaa ttagtaatgg gaagagcaga aatttaaacc taactcatct
68641  ttcataccta tgaagatgca aataagtcaa ccaattacat ttgtgtgtaa tatatttgcc
68701  ctcttgatga atactgaacc agagttagag catcttctat ttgtttcatg aaaaagcatt
68761  gacattatac acgatttgtg tgttctactt tgaaatcaca ctttgttact ctaaacatta
68821  tttgaataag aagttgctaa taatttgagc agttatttca aaatatttat gtactattac
68881  tacacagatg gattactctt gagcttggca tccaattttt aaaatttttct aactcatgtg
68941  tacatgacta attttccctc tcagaattat gacaacattc attcatatgc tcttctctct
69001  tgatttttgt actttggtag tttgggaaac atgataagat agctcgtagt ataactaata
69061  gcttagcaca catagcatat ctatactctt ttgacttttc ctctaaatat caccttatca
69121  tagtttgttc tttatcccct atccacttgc cattatttcc atccaatgaa acttattttt
69181  cttctctctc atctttgaat ttccttccct ccctaacagc atgtgagtta cttgaaagct
69241  ctttgatcag aataaaaatc ccaattattt gatacttcca tttatttgga ttataaagac
69301  atacgggggt atattttgca aaacagtctt gtcagccccg gcgaagata tatcactata
69361  ttatagcttt gccatgtgtc catagagaaa ggttatctta tgttaaacac atgttcatat
69421  gcaataacag agacatgaat tctaaggata ttgcagaaag catagctgac tactgattct
69481  agcaccttca ggatctttct cttcttagtt gYgaccactg taatgcactt catagagtaa
69541  taaattgttc ttgccctggg atcatgattc ccatctcttc actacaacat gtctagatgt
69601  aagacatgtt taccccttgta taatcaatga tctgagtcta gatgtcaagt acttgcttgt
69661  tctgtgcatt aactgtcttc tttttctgga actgcaggct ctatcagatc cttcccctaa
69721  aattcataca tcctgccttg ttactatccg tgccccagca tgaccaatat caaagtggtt
69781  aagaaaaagg ctccagctat tttagtatcc taaattttat cttttcattc tacaaagaag
69841  tttttcataa aaagttaaaa taaatttaaa aactcattga tactattcag taagatgtta
69901  tagattccaa gcaactatct cttttatagg caacataatt aaaagtgaat tacaaatgca
69961  gactttatga ctctaattaa actctaggcc acaatgcatt aaaaacagat tatacatatt
70021  aagttagata taagaaaaaa taaatatgcc acatagtgag gcataagtta atatgcctca
70081  aaataaatga ggagaaacaa tttcagataa tacatattta tataaaatgt ttatcaacat
70141  aacctaaaat attttcttga atttcatcag agataaaatt tttatactta atttttgtaa
70201  acatatacat catactttct ctcacttact tcactacttc attccatccc aagaacatct
70261  gtgcaagtat ttcccaagtg tgggagactg gaagaggttc tatattttgg ttgtaagaat
70321  atatattaat ttttagaaca ttataattaa actcagaaat atttaattgt agggatatta
70381  tttggtaaga atcaaataaa atgactaaaa attaacttga ggcaacatga aatctcaaca
70441  tcattgtaaa actaagagag cagatcttaa acaaaatgta gtgcagtctt cacacttggg
70501  agattcatta atcacttaag tgtttctct gaagttttta aatgcttaaa gattgggtta
70561  cattatgaaa aggacctgtt taatattgtg tctgtaaagt tcaattctac ctagttctca
70621  ctttctttcg tatcttctct agtgaagaat gaagaaatga gaattatgtg tttgaatcta
70681  gcacctctga catggaaaaa cacatggtga agcagaggaa agaaaactga aagaaagtga
70741  aaatatcttt aagaatacYt tggggaaaca aacagtattt caaatgaaag attaagaaca
70801  aatccaaaca aatggattgg aaggtattat gtaaaaaaat atgagatgct ttagcacagg
70861  tactctaaga atttgagaag cttctcatgt ttaagggaga aggtaacatt cctgatagaa
70921  acatttaaag gcagtgcaga aaggaaattc tcatcaggag gcctatagaa cacacatggg
70981  caacagcaag ccccccttgc cctgctcagc cctggatcca tacagaggaa aggagagagg
71041  taaagatgaa agaaagaggt tgaacagaag atagtaaatg gttgaaaaat atttttaaaa
71101  tagaaagagt tatgcttctg agaagattct ccatgtctgg gtatcagaca tggagataca
71161  tcatactgat caacctgaag taatgtactt actgaaatac ccacttctag ttcatcWagt
71221  tcatgcaagg tttcacaaag ccaccaatcg ttttaatcat cttataaaag ctttctaatt
71281  gaaatattct taagtatcac atttactag tggcaggtat cttttcaaaa aaaaaataca
71341  agctaagcaa cttgtcctct gttgtttcct accactaagt ttgcattaga agagactggc
71401  agtcaaacac acaagaagaa acacaagtat tggcaattta acagaaacaa atggtagtgt
71461  atcatggaca tctaaggttt gtaggactaa atcataccct cttctagtaa gtcctgaaaa
71521  aaatgtattt aaaacatatg ttgaagaaaa tagcttttag aggggcagat aataggcttt
71581  ggacttagaa attttataaa aatccattgt gcattcttta ggcttctagt cctattcagt
```

# FIGURE 2-T

71641 ttttgctatg agctatgatt tgtttgtgga agcaaagtaa aggcagccta tttcttaccg
71701 cctccacttc ggctgggtca taccagacgt tgatgtaggg atgctgtaag gcgtcgtcca
71761 ctgatattct ttttgctggg tcaatcacta gcatctttga caacaagtcc ctggcttggc
71821 tggctgaaac aataaatgag aaaaacaatt agtaagattt tgatttcagt aaggaattt
71881 gtcagagagg aaatttaaga aaacaaagta tggaatagta tcttccttcc aaacatcagg
71941 taaatccctt gatgtgatgg aaaagaaaat gcattttaca cctgtttctt tgtgtctcta
72001 agcctaaggc ttcattctga ggctctccaa gggttgtaac actttgggta tataattatt
72061 cacgcaaact tcttagtaaa atgtatcaac ctggagagtt tatgtaagca aagtcatctt
72121 ctttggtaac ctgcatgaat ataggataaa cagaataaag agcacagagt gcaaatgcat
72181 gatgttcaac aaatgtgtgg acggctgttt gaagttaatc aacacgtgtg tctctatatt
72241 cttatttata caacggaatt atcagaattt tagtatataa attgacttaa gagtttgtat
72301 catacaaatt tttcttttct ttttgttttt gtttatttat ttatttattt atttttgaga
72361 cagggtatcc ctctgttgcc aggctggagt gtagtggaat gatcacagct cactgcagcc
72421 tggaactcct gcgctcaaag gatcctccca tcccagcttc ccgagtaact ggaactacag
72481 gcatgcacca ccatgcctgg ctattttttc tttctttctt ttttttttgta aagataagat
72541 ctccctacat tgcccaagct ggtctcgaac tctgggctca aagatcctcc cacctcagcc
72601 tcccaaaatg ctgggattaa aggagtgagc cactgctccc agccacaaat ttcttatata
72661 cataaaaaca atcaaagaat tgaagtgata gaaataatat cattcaacta ctgaatttta
72721 ttttaacatt ctgtaatcat caggtatttt taatcaatgg aatcatgact tgatgaaatc
72781 cagtagttaa agactgaagc cagtctaatg ctacaccaga cactaatgag aaactctata
72841 ttatgtaaag gttatcataa ttaattttat ggttgtgggc agtaacagtt ataaaattat
72901 atggtgatag ctatttattt agtaagattg ttaaaagaca cattaatttt aacctctaaa
72961 aatatgtaaa ttttttaaat tattatactt taagttctgg gatacatgtg cacaacatgc
73021 aggtttgtta cataggtata cacatgccat agtggtttgc tgcacccacc aacctgtcat
73081 ctacattagg tatttctcct aaagctatcc ctcccctagt cttttacata aagttttaac
73141 agaaatgaat atctcaaatc aatattgtta ctcaactgat ctgaaagttg aaccgtgaaa
73201 atattcagtt tattctaact atatggtaaa acatcaaatt aaagataaca aaatttaaat
73261 ggatttttca aaaaatcaac atagatgact ctggatacat atcaattttt atttaatcaa
73321 tttttcagat taaaacaact tggctaattc tacaaatttg aaaattatat aaaatacaaa
73381 aWtcaggaaa taatatctaa ccccactagc tagaggcaat gactgtgatt attggcatat
73441 tttttctagt gccgttcatg tatttttcac gtaatattaa ccatactata gttaaaatgt
73501 agcaattaat tttttacctg taagtatttt cttggtaatt acatcaataa atatatcttt
73561 taattttctg aacaaatatg aaaaatcact agttaaactt gagaattttt tatttctatt
73621 aatcattttt ctgggaaact caatcctaga aactgattat cattactttc aaattaaaac
73681 tctgtaccgc acaggaataa caaaaataca gaaatactta tacattaatt caatatttaa
73741 tttttctcat tgccaatggc ctcctaatag cctaacaggg ctgtgtttat ttattcattg
73801 ctttaaagac ctctttaatt tgtaactgat tttacatcca aattcatgta ccattctcat
73861 aagattctct catcaaatct atccattttt ctgctcttag aaagggctgg ggttagccgg
73921 gcgtggtcgt ggctgacacc tgcaatccca gcactttgga aggccgaggc aggcagacca
73981 cgaagtcagg aggtcgagac gatcctggct accacggtga aaccccatct ctactaaaaa
74041 tacaaaaatt agctgggcgt ggtggcgggc gcctgtagtc ccagctactc gggaggttga
74101 ggcaggagaa tggagtgaac ctgggagacg gagcttgcag tgagcctaga tcggtatccc
74161 gggcgacaga gcgagactct gtctcaaaaa aaaaaaaaaa aaaaaaaaga aagggctggg
74221 gttacaatgg attcttcatg aatagggtca tgaactacac caatacaaaa tcatcttgct
74281 aagaaacaat actattttta ggaaacaata tgggaaaatg tttcaatgct ttatctttca
74341 ttctttcatt tgaaagcaaa tgtgttatgc tcttttccaa acacagtaga atacattctt
74401 ctctttggtt atttgtatat taagattatt tataatttgc caattttata ttaaaaatta
74461 aattcaataa tcaaatatgc aaactagttt atcatttaca aacataattg cagagattca
74521 aattgattta aaaagtaaac tcaagaaaat tggaattaca ttatgaagta taatatatat
74581 ctattttttg agacggagtc ttgctcactc tatctctagg ctggaatgca gttgcccgat
74641 ctcggctcac tgcaacctct tcctcctagg ttcaagcgac tctcctgcct cagcctcctg
74701 agtagctggg actagaggtg tgtgccacca tgcccagcta attttttat attttagga
74761 gagacgcagt ttcaccatgt tggccaggat ggtctgtctc gatttcctga cctcttgatc
74821 ctcccgcctc cgccttccaa agtgctgagg ttacagactt gagccaccgc ccccggccct
74881 gaatatccag acaattctaa aatccgtggc tactcccctt tgtaggaacg atctttgaag
74941 aaatatttct ttaacttgag gctgtgccaa ttaaagaatt gatcaatgtt ttaggtgatg
75001 ggttatgcta attgctctga tttgaccatt atacctctat acatgtttca aaaataccac
75061 cctatatccc acaaaaatgt acgattatca tgtgtcaact aaaaataaaa gggaacaaag
75121 agaaagagat agaggtctct atactgtgtg cttgtttggc ccgtcaccct catagttgtc
75181 ctcaagtcat tcctgaaggg catacctttg agtttattgt gctcggagtc cgctgggaag
75241 agggaatctg ggaagagttt ggggaaggtg agtcccgcat acttgggccg attctccaca
75301 tagtttctta ctgtgggttg caatttcttc atgaattctg gacatggtgt tcctagttgt
75361 tcaattacct tattccactg gtcaatatct gagaattgaa cagttaaggg aaaaaagaag

# FIGURE 2-U

```
75421  agcagaccac tagcctttca acaattggga gactaactct tctcaaagtc ctcaacagtc
75481  attaatgcaa ctgtagtttt aataagcaac aaagaatgaa aatataaaat cagacttgca
75541  ggctatgagt cttcttgaaa ttttaaattc acttttgaaa cttatatact tttggaaaac
75601  tagcaattat ttaacaaaat aagatatcat gaaatagtct ttggggtgta cagataaaaa
75661  gtcattcaga taacacaaga tgaatctcat attctgcaga taaacttcta tataaatcat
75721  gtgattcaga agaaaaatat ataatgacac atcatagtga catttttatc ctgaagttac
75781  ctcaattatt tatattttta aaatgacaaa tacacaaata ttttagatta tattcattta
75841  cattttccta aactatcttt aagatatttt ttcttctcta aaaatctgga atttttttct
75901  atttttatgc atatcaacat aacttttata gtaaaacaac tcattagttc tttgtcagat
75961  tcctaaaaat catatttatt aaacaaagaa gccacctatt tcatacctat tgttttactg
76021  atgatatcac tgaatattgt tttcaatgaa atataagggt gaacagagga atattcaatt
76081  taaaacaaca gatttctatc caagtacatc caagtttaga aagtacatta agcatgggga
76141  ataccacaga aaaaatatta cctctctatg aaaattaata cccttttaaa ttggagctag
76201  ctctttttgc agacatactc aatgattgag attctctttt ttatctgtca ttaatattcg
76261  atatatcaga gccttgataa tgataaacac catctactct gaagaaYcaa ctggaaacaa
76321  ctgatttaaa cagatttcct cgccatgtgc cagtgaataa tagagatctt ggaaattccc
76381  ttaaatccaa tactgcaagc agtattctct cctacattgc ttattcttct acaactgttc
76441  tttggtgtta agttagttaa ctcatttcca ctaaggtaag gtgtgacagt taagggactc
```

# FIGURE 3-A

```
>3:184282401-184429700

1     gaagcgctga gggccactca ccggccaggg acgcgaagag cgcggccgcc gcgctgagct
61    gccggggcat ggtgggcgct gggcgaggtt ctgcagcgtg cggcgaRgtc cgggcaggcc
121   ccgaatcggt gccagagaaa cctacctgtg ccggagaaac gaaaccacct gcttatgaga
181   agcagccgaa aagcccgccc agggccgctg ggcggggagg gaaactccgc cggcccccctc
241   ctacccctac ggagcaggga ggggcgggga ctcggcgcag ccgccggggc ccgggcctct
301   gggaccgttt accgcacgcg cgtggtcccg gcagcgccgg cctcctccgc tcataccctg
361   ggtctcctcc tttctttttc ttttcttttt gagacgaagt ctcgctctgt cgcccagggt
421   ggagtgcagt ggcgcgatct cggctcacta caacctctgc ctcccgggtt caagcgattc
481   ttctgcctca gcctcccgag tagctgggat tacaggcatg caccaccaca cccggctaat
541   ttttgtattt ttagtagaga cggggtgtca ccatattggc caggctggtc tcgagctcct
601   gacctcgtga ttcgcccgcc tcgacctccc aaagtgctgg gattatagac gtgagccacc
661   gagcccggcc agggtctcct cttttatttc ttttcttttt atttcttttg ttttgttttg
721   ttttgttttg tttttgagga caaagtctcg ctctgtcgcc aggctggagt gcagtggcgg
781   gatctcggct cactgccctg gttcaagcga ttctcctgcc gcagcctccc gagtagctgg
841   ggttacaggc gcccgccacc acgcccagct aatttttgta ttttagtaga gacggggttt
901   caccctgttg gccaggctgg tctcgatctc ctgaccttgt gatccgcccg cctcggcctc
961   ccaaagtgtt gggattacag gcgtgagcca ctgcgcccgg cccagggtct cctcttttct
1021  aacagctcgg gtacctttct gggaacccag agacgcttct cagccgggag aaagccagcc
1081  actaggcgag caggagccta aaaacccccta agcaccctga ctccatgtct tcccagggag
1141  tctgcggcag ccgcgctcca cgcccaggcc tcgccaggac cgcggtttgc gggaagcaac
1201  aggagcacag cccagaggcg ctaggtctgg ctgggagctc gcgctgccga ctccccggcg
1261  tgcggcgtcg ggaacctct aggagccttg gattcttcag ctgtaaaacg gacataataa
1321  tgcccactcc cagtgtgttt ttttattttc tttttctttt ttctttcttt gtttttgttt
1381  gtttgttttt gtttttgttt ttgagacagg gtctcactct gtcgcccagg ctggagggca
1441  atggcgtgat ctcggctcac tgcaaacttg ggttcaggcg attctcctgc ctcagcctcc
1501  acagtagctg ggattacaga tgtgcgccac cacgtccggc taattttttg tatttttagt
1561  agagaccagg tttcaccgtg ttggccaagc tggtctcaaa ctcctgaccc caggtgatcc
1621  gcccgcctcg gccttccaaa gatctgggat tacaagcgtg agccactgtg cctggcccca
1681  ggtggtttta cagaccagaa aatcctggaa caaaaaacac acaatatcgt tttttttttt
1741  tttttggagt cagggtctcg ctctatcacc caggctggag tgcagtggcg tgatctcggc
1801  tcactgcaac ttcgacctcc tggcctcaag tgagtctccc accttagcct cctgagtagc
1861  tgggaccaaa ggcgcgtgcc accacgccca gctattttat tttattttat gtagagagga
1921  ggtctcgctg tgttgcccag gctggtctcg agttcctggc ctcaaatgat cctcctgcgt
1981  tagccaacca ttgggattac aggcgtaagc cacggcccac ggctcaacaa cgctgacagg
2041  caacctttta atgtcttatc tccttcctct attaattgga ttgtctgtca aaacaacgat
2101  gttttgacag ggcttgagtc ccagtgggga atacacattt aagcagtata ttaggaSacc
2161  ctccttatca ctagattgag ggctttcagc ctagcctcaa attattttct gaaaaataac
2221  tttggctaca actattttgt cttactatgt tgctccaaac actaatcaag taaacttaac
2281  caaagcttgc agtgtgtttc agaatggaat ttttatggtg aaaagtgagg gttaacttgt
2341  gccagtcaac ctagtttcag caactacctg ctttctgatc tttgagacag tttattcaaa
2401  agacgataat taagtgggta tagactgtgt gccaggcact cttcttattc catttaagcg
2461  ccatagccac tctatatgga cactgttgtt attatcgctg ccccatttcg cagatggaga
2521  aactaagcac aaagaaggga gttgcccaga gtcacttaga taataaatac cgaaacctga
2581  ccataaatct tgtctgcctt gagagtctag gattttaagc acatagccgg gcgcagtggc
2641  tcacgcctgt aatcccagca ctttaggagg ccgaggcggg cggatcacga ggtcaggaga
2701  tggagaccat cctggctaac acagtgaaac ccagttctat taaaaatata aaaaaattag
2761  ccaggcgtgg tggcaggtgc atgtagtccc agctactcgg gaggctgagg caggagaatg
2821  gtgtgaaccc aggaggtgga gcttgcagtg agcggagatc gcgccactgc actccagccc
2881  gggcgacaga aggagactcc gtctcaaaaa aaaaaaaata aataaaataa agctgctcct
2941  cttaccctgg aaattccaag ggatttagga gctctgtttc aggaaccagg gtcaaagacc
3001  aagtattaaa acaaaagatt ctcctagaac tctggcatat aaggatttta ggagctctgt
3061  cttagaaact gggacagaga ccaaagatat attattatat cgcagtatca tagtttatta
3121  ttttcaaaaa acgttttctg gctggtacag tggttcatgc ctataatcca agcactttgg
3181  gaggccaagg tgggagggtc acttgaggcc agaagttcaa gtccagcctg ggcaacacag
3241  ggagaccctg ccactattaa aaatttttta aattagctgg gcatggtggc acatgcctgt
3301  agtcccagct acttgggagg ctgaagcagg aggattgctt gagcctgaga ggtcaagact
3361  gtagtgagct gcgatcaagc gactgcactc tagcctgggt gacaaagcca gaccctgtgt
3421  ctaaaaaaaa gaaaagaaga ggaaaaaaaa aggtttttat ttcaactaag ttgttggatt
3481  tattagcata aagcttttca taacaatccc ttgtcttaca atatctgtag tataggtagt
```

# FIGURE 3-B

```
3541  gatgtcactt cttttattac taatattaat aatttgtatt ttctctctta tttccctgct
3601  aatattaata atttgtattt tctctctttt ttccctgata agtttggctg gaggttgatc
3661  aattctattc atgttttcaa gaaaaaaaaa aaaatttcat ttcatcgagt tccttcattg
3721  ttcttatgtt ttctgtttta ttcatttcta cctgatcttt attattttct ttcttctact
3781  taacttgtgt tttatttgct cctctttcaa tagttttcaa agatggaact tgcctgggat
3841  atcccagcac tttaggaggc tgaggtggga ggatcacctg aggtcaggag ttcaagacca
3901  gcctggccaa catggtgaaa ctccgtctct actaaaaata caaaaattag ctgggtgtgg
3961  tggtgggcac ctgtgatccc agctactcgg gaggctgagg caggagaatc gcttgaaccc
4021  gggaggtgga ggttacagtg agctgagatc acgccactgc actccagcct gggtgacagg
4081  agctagactc tgtctcaaaa aaaaaaaaag aaaaaaaaaa agatggaagt tgaggccagt
4141  ggtataaaat ctttcttcat ctctaataaa caggtttact gttatgaacg tccctctaat
4201  tactacttta gttggatccc acaagtttaa tatgttttca ttttcatata attagaaaga
4261  cttcctaatt tccttttgct atctcctcga ctcatggtta tttaaaatag cattatttca
4321  tttccaaaca tatagttttt cagatatctt tcttttattg atttttaatt ccactgtggt
4381  tgaaaacata attatggact taaatcttac aaatttattg atatttgttt tttgaccaag
4441  actatggttg gttacattag ttttcaggac tgacataaca aagtgccaca gactgggtaa
4501  ttaaaccaca gacatttgct ttcttataat tctggaaacc agacatgtga gatcaaagtg
4561  tcagccgggt tggtttttc ttttcctttt tttttttttt ttttgagac agagtctctt
4621  tctgtcaccc aggctgggat gcagtggtgt gatctcggct tactgcaaat tctgcctccc
4681  aggctcaagc gattctcctg cctcagcctc ccgagtagct gggattacag gtgcctctca
4741  ctgcacctgg ctaattttg tatttttagt agagacgggg tttcaccatg ttggccaggt
4801  tgatctcaaa ctcctgatct caggtaatac acccgcctcg gcctcccaaa gtactgagat
4861  tacaggcgtg agccactgca cccggcccgg gttagttctt tctaaggcct ctctccttgg
4921  ctagtagaca cctttgtttc acatggtcat ccctctgtgc atgcctttgt ctgtcctaat
4981  ctcctcttct tataaggaca ttagtcaggt aggattagtg cctactcttt gaactcgttt
5041  tacctcttaa agaccctatc tccgaatata gtcacattct gagatacttg gggttaagac
5101  ttgtattagt ccattttcac gctgctcata aagacatacc tgagactggg aagaaaaaga
5161  ggtttaattg gacaattcca catggctggg gaggcctcag aatcatggtg ggaggcgaaa
5221  gggacttttt acatggtggc ggcaagagaa aatgaggaag aagcaaaagc agaaacccct
5281  gatagataag cccaccagat atcatgagat ttattcactg tcatgagaac agcacgggaa
5341  agaccagccc ccatgaatac attacctctt ccttggtccc cccctcccca caatatgtgg
5401  ggattctggg agatacaatt aaaattgaga tttgagtggg gacacagcca aaccatatca
5461  ttctgtccct ggtccttcca aatctcatgt cctcacattt caaaaccaat catgcctttc
5521  caatagtccc tcaaagtctt aactcatttc agcattaacc taaaagtcca cagtccaaag
5581  tctcatctga gacaaggcct tccgcctatg agcctgtaca atcaaaagca agctagttag
5641  ttcctagata caatgggggt acaggtattg ggtaaataaa gccattccaa atgggagaaa
5701  ttggccaaaa caaagggggt acagggccca tgcaagtctg aaatccagtg agggagtcaa
5761  attttaaagc tccaaaatga tctcctttga ctccaggtct tacatccagg tcacgctaat
5821  gcaaaaggta ggtttccatg gtcttgggca gctccacccc tgtggctttg cagggtacag
5881  cctccctcca ggctgctttc atgggctggt gttgagtgtc tgcagctttt ccaggcaccc
5941  agtgcaagct gtcagtggat ctaccattct ggggtttgga ggacaaaggc cctcttctca
6001  cagctgcact aggcagtgcc ccgataggga ctctgtgtgg gggctctgat cccacatttc
6061  ccttctgcac tgccctaaga ggttctcctt gagggcccca cagcttccac cctctgaacc
6121  atagcccaag ctatgcattg gcccctttca gccatggctg gagcagctgg gacagagggc
6181  accaagtcac taggctgcac acaacatggg gaccctgggc ctgccccaca aaacccccttt
6241  ttcctcctgg gcctccaagc ctgtgatggg agaggctgct gtgaaggtct ctgacatggc
6301  cttggagaca tttccccatg gtcttgggga ttcacattag gcttcttgct acttatgcaa
6361  atttctgcaa ccagcttgaa tttctcccca gaaaatgggt ttttcttttc tgtcacatag
6421  tccggctgca aattttccaa actttatgc tctgcttccc ttataaaact gaacgccttt
6481  aatagcaccc aaatcacctc ttgaatgttt tgctgcttag aaattttttc caccagatac
6541  cctaaataat ctctcaagtt caaagttcca caagtctcta gggcaggggc aaaatgtggc
6601  cagtctcttt gctaaaacat aacaagaggc acctttgctc cagttcccaa aaagttcctc
6661  atctccatct gagaccacct cagcctggat cttattgtcc atatcactat cagcattttg
6721  ggcaaaacca ttcaacaagt ctctaggaag ttccaaactt tcccacattt tcctgtcttc
6781  ttctgagccc ttcaaactgt tccaatctct gcctgttacc cagttccaaa gttgttccac
6841  attttcaggt atcttcagca acgtttcact ctactggtag caatttactg tattagtcca
6901  ttttcacact gctgataaag acatatctga gactgggaag aaaaataggt ttaattggac
6961  ttacagttcc acatggctgg ggaggcctca gaatcatggt gagaggtgaa aggcacttct
7021  tacgtggtag tgacaagaga aaatgaggaa gaagcaaaag cggaaacccc tgataaatcc
7081  atcagatctc atgagactta ttcactatca cgagaatagc atgggaaaga ccggccccca
7141  tgattcaatt acctccccct gggtccctcc cacaacacat gggaattctg ggagatacaa
7201  ttcaagttga gatttgggtg gggacacagc caaaccatat caagacttct acatatgaat
```

# FIGURE 3-C

```
7261   tttggaggga cacaatttaa ctcataatag tggactgtcY tgttaaatgt tctgtgtgca
7321   cttgagaaga atgtgtgtat tctctcattg ttggattcag tgacctataa atgttaatta
7381   ggttaaacta attgatgtag ggaaaagaaa gagagatcag actgtcactg tgtctatgta
7441   gaaagggaag acataagaga ctccatttg aaaaagacct gtacttcaaa caattgcttt
7501   gctgagatgt taatttgtag ctttgcccca gccactttgc cccagccact ttgacccaac
7561   ttggagctca caaaaacatg tgttgtataa aatcaaggtt taagggatct agggctgtgc
7621   aggacgtgcc ttgttaacaa aatgtttaca agcagtatac ttggtcaaag tcatcgccat
7681   tctctagtct caataaacca ggggcacaat gcactgcgga aagctgcagg gagccctgcc
7741   cttggaagcg gggtattgtc caaggtttct ccccatgtga cagtctgaaa tatggcctcg
7801   taggatgaga aagacctgac tgtcccccag cccaacaccc ataaagggtc tgtgctgagg
7861   tggattggta aaagaggaaa gcctcttaca gttgagatag aggaaggcca ctgtctcctg
7921   cctgcccctg ggaactgaat gtcttggtgt aaaacccgat tgtacatttg ttcaactctg
7981   aaataggaga aaagctgccc tgtggtggga ggtgagacat gtttgcagta atgctgcctt
8041   gttattcttt actccactga gatgtttggg tggagagaaa cataaatctg gcctatgtgc
8101   acatccaggc atagtacctt cccttgaact taattatgat acagattctt ttgctcacat
8161   gtttttttgtt gaccttctcc ttattatcac cctgctgtcc tactacattc ctttttgctg
8221   aaataatgaa aaYaataatc aataaaaact gagggaactc agaggctggt gccggtacag
8281   gtccttggtg tgctgagtgc cggtcccctg gactcactgt tgtttcttta tactttgtct
8341   ctgtgtctta tttcttttct ccggctctca tcccacccga ctagaaatac ccacaggtgt
8401   ggaggggcag gccacccctt caattgatag tatggttcaa gaacaaatgg tatcaactta
8461   ggatggttta acttatgatt tttcaacttt agaatggtgt gaagtctgta tgcattcagt
8521   agaaggcata ctttgaattt ttatcttttc ccaagctact gctatgggac aagatactct
8581   ctcacaattc tgggcagtgg cagcaagcct cagcttccag tcagcaccca atcccaaggg
8641   taaacaactg atacagccat tctgtttttc atttttagca aaatactcaa taaattacat
8701   gaggcactca atgctttatt ataagacaag ctttgtatta gatgatttgc ccaactgtag
8761   gctaatgtag gtgttctgag cacatttaag gtagactata ctatgccatg atgtttggaa
8821   ggttaggtaa atttaatgca ttttcgactt agaatacttt cagcctccca aatagctggg
8881   accacaggtg tgtggcacca tgtgtggcta attttttgtg gagacaaggt ctcactgtgt
8941   cgcccaagtt ggtctcaaaa tcctgacttc acgtgatcct tccactctgg tctcccaaag
9001   tgcgattaca ggtgtgagtc accacacctg gccgtgttgt cacacatttt aattctttat
9061   aaattagaaa cttcacaaca cattgttctt atttaaaatt taaacaatta tctttaaata
9121   tacataatat ataaatatgt atataatata taaatatata caatatataa atatataaga
9181   tatattatat atataatata taaatatata atatataaat atataatatg taaatatata
9241   atatataaat atatgtaaat atgtaatatg taaatatgta atatgtaaat atataatatg
9301   taaatatata atatataaat atacataata tataaatata taatatataa atatataata
9361   tataaatata taatatataa atatataata tataaatata tattatatat aaatatataa
9421   tatacataaa tatatataat atataaatat atataatata cataaatata taatatataa
9481   atatatataa tatatataaa tatatatggg gaaaaaagct tttatactta ctcatgtgat
9541   taccgtttct tgcagtcttt attcctttgt ttagatcctt gggatttttg ttgctatggt
9601   gaccttagtt ataccaggca cttcaaatct taccttgtgt ttaggaWatg ggcttgtttg
9661   cccaaaggtc ttccctaatg tctgctccac cttcaacttt aggtcttctc tgctgtcagt
9721   ttctctctcc atacacttgt agctctccca ggagtattcc atcgttactt gttagtcagt
9781   gcttattagc ggggtggtgg gatctgagag gtgaggtgct tggttgtgat tctcagttct
9841   gattcctgca ggtactgtgt ccctgggYct tgaagggtat gaccaagcSt ctctgtccct
9901   ccccgcagca gtagttttgg gctcagcaca tattcctgcc ccttccccag aatcagaggg
9961   tttttgttg ttgttgtttt gtttttcaag tttttgttcc tttttctcca tctgtgttgg
10021  atttaccagc cccctaggag cgtcagtatt tgttaccctt cctccaggct tttaaggcct
10081  ttgtaggaga gatgggccaa tagcatctga gcatggtttt gtgtctttct tgtagcaact
10141  gatattcctc acccccaggt ctatgccagg aaggatgctt ccttacatgc ctgtaatccc
10201  agctaccggg gaggctgaag cagaagaatc acttgaaccc aggaggcaga ggttgcagtg
10261  agccaagatc atgccactgc actccagcct ggcaacagag cgagacttca ttcaaaaaaa
10321  aaaaaagctc ttacactctt ctgcatattc tgcaaagtat actctgggaa ggctggttta
10381  gaggatctct aacttttcta atatttcata atgcatggct gatgtttaac atgaactcag
10441  actcccatca gattttacag atctgggtct gcttttagtt ctaatgcttc agccagggca
10501  ctttaatctg aggtcacagc attcccaatt gtcagcagta ttttgaaatc taagttcctc
10561  tggtggacaa caggcaacat catatagcca cggaggatct gaacaacttc taagtctgag
10621  Sgattttatc tggtggtttt ggattctttt ccatttatac ttaaacaagg tgacacaaat
10681  gtcactcaca gaaaagccac tctttttcc cctgttttta agaagtctaa aaatgcacaa
10741  ctatttcctg aatgagcagt gtgtccaggt gtagaatagg gaatgtcgtg tccactggga
10801  acagacggtg tacctggcac aattttatga gaattatccc atgattcctg tcccaccctg
10861  cagcccacca tgggctcagt ggggagtctg agtctctgag ccccaaaggg tagcttttc
10921  ccagacgaca ccagcaagca gagcagcagc ttggggcttt gacaaggagg ctggagtgaa
```

# FIGURE 3-D

```
10981 aggtcatatt ggaccatcct ggRtcactgg aggacaatac cccaggaacc tccagcaatg
11041 actggtattg gaatgactcc cctggtcacc cacctgtggc aagacatttg caaacagggt
11101 cttcctatga ggcttgtgag gtttggccaa tttgcattct gctagaatta ttaagtatgg
11161 acatcctcat agatccagaa aagggccgag atccaaagga ttgagaatgc tttgggggt
11221 gtttccatag tgagtggctg gctattcaac agtcaaatgt ttgagaggat acaagtgatg
11281 tgtttcctga ggcaagtggt cagaacccaa cagactcttc tgttcagcta agatgagaga
11341 ccatctgaag ttcttacatg ttctcacaga gaaatggatt ctcataggga aatggatata
11401 ttgtgataga cactgtaagc agagaagttg actgagaaac acacacacac acacacacac
11461 acacacacac gtcaagactg aatgcataga tgtgtattat aaaaagYgta aaaatacacc
11521 accattStaa cagattgtgt aggactatat tttcttttt tttaatttat tattattata
11581 ctttaagttt tagggtacat gtgcacaatg tgcaggttag ttacatatgt atacatgtgc
11641 catgctggtg tgctgcaccc attaactcgt catttagcat taggtatatc tcctaatgct
11701 atccctcccc ccttcccYca ccccacaaca gtccccagaa tgggatgttc cccttcctgt
11761 gtccatgtgt tctcattgtt caattcccac ctatgagtga gaatatgYgg tgttttgttt
11821 tttgtccttg caatagttta ctgagaatga tgatttccaa tttcatccat gtccctacaa
11881 aggacatgaa ctcaccattt tttatggctg catagtattc catggtgtat atgtgccaca
11941 ttttcttaat ccagtctatc attgttggac atttgggttg gttccaagtc tttgctattg
12001 tgaatagtgc cacaataaac ataYgtgtgc atgtgtcttt atagcagcat gatttatagt
12061 cctttgggta tatacccagt aatgggatgg ctgggtcaaa tggtatttct agttctagat
12121 ccctgaggaa tcgccacact gacttccaca atggttgaac tagtttacag tcccaccaac
12181 agtgtaaaag tgttcctatt tctccacatc ctctccagca cctgttgttt cctgactttt
12241 taatgattgc cattctaact ggtgtgagat ggtatctcac tgtggttttg atttgcattt
12301 ctctgatggc cagtgatgat gagcattttt tcatgtgttt tttggctgca tcagtgctct
12361 acacgttcag agaaacttct ctagtgacga actatagaaa tgatccctga aagtatagtc
12421 ttaggactat attttctttt gacttgggag gcatgtttat tgctgttaat gctgcaaagg
12481 gctctacgtg ctttaaaaaa tcccaatctg ttgcattcat aagcctgggt tggatctaaa
12541 gcagcctccc acttttggaa aggcatcccc acgacctttc catggttgct gaatgcagct
12601 ggaggcagtc acagctggtg atgtccggag cccattcccc actgtgctgg tctgcagaac
12661 ttctgcatgc cattcccaca agcaggtctc tgccctgctc tcctccacct ccccttgtcag
12721 aggaagtctg cacttcacag ctttctggtc tcaaccctcc tccatcccta cagatgtgta
12781 agcagcagga atcaaaaggt gaaggagagg gggcaactca cctccgatgg acacgtgaaa
12841 agtgggagat ggataaaatc aagaaggagc ttaagatatc cagaaatgta aactgtgttt
12901 ggaaaagtaa ggtcaggaga agcatgggac tcctgaggtt gctccctact atcttgcaga
12961 cttgctgcag gaccaaatga agcaggatct gtcaagcacc agggccagct cttaagctta
13021 gtgcctttct gaaccctgtg acccagcagc ctccatcaac tcgtcctacc tgccatgcac
13081 agctcctctg tgcccctgta cctgagctca tgctattccc tctgccagga tgcccttctc
13141 cttctccacc aggagaagaa cacttgccag taagacccag ttctaatgtc acccccttcct
13201 gacggtatca ggaagagtca gtgatggtgt tttatgctcc cagagaattt gccacattgt
13261 gttgtgatta tttttccaca tctgtctccc ccactggaat gagagcctca ctcatcttca
13321 tacctccctg gtctctacct ggtgccagaa ccatcctcag ggcaggggaa tgctcaggaa
13381 atagatattg aataaaataa gtgtatccat ccatccatcc atccatcctt ccatccatcc
13441 agacatatat acatatgtaa acattctgat ggtcaaatgg aacaatgtgg gctgaagaat
13501 aatgcaggta gaagaaccta agattacaga ttcttgattt gggaggaact ttattttatt
13561 gtgcaaacag tctacaaatt tgaaacatac tctatcaaga aagacactgt tgttaggaaa
13621 gggcgtggga gagggtggcc ccacaaaaca gtgtaaagtt ctaaagaatt tgagaggaac
13681 actctgcggg accctgttcc aagggcatct ttctaagagt ctgtccctta ggctctgccc
13741 ctttttggcc acgttgtcaa aggccttctt tatgaacgaa gtgagacaca ttatttttgt
13801 tttcttgaat tctaaagtga ttgtcttgag ttccctgggg gagactcctg ggaggtgtgg
13861 cccactctat tccagaaaca aaccaggaga ctgaagactc atcccaatcc catatcccat
13921 gaaaatgtga aatcaaatca cccctgacaa ttccaaaact aaYggaatcc taacataact
13981 ctatgtaact ctaaagctga gtgtgctggt aattaagctt ccagcccacc cttcccagct
14041 ctggggtgca gggtcaccat gggctccttc ctgtgtgcac cccaccccac ccccactgtc
14101 agcatcctgt ctccaccctg agagtgacag ctggttccag tagcgggagt tggttccagc
14161 ttgccatttt cctagcactc ctataaccag cctgctgatg cccattcaga gacagcagca
14221 cgggctggcc atgtcccctc tccagaattc tgcgtccagc tcctggacct tgagctctga
14281 gcccttgggc cacgtgtacM attaatagtg cctcctcctc agaggactaa cccccagccc
14341 tagggccacc ttcatatttc Ygagttttga tattttcaac ctcttttctt tgttgtatga
14401 gtccttgggc tgggagcttg cagtcaaaat cttcatgata tctcattatc actacttttt
14461 tttaaatctc tactagctgg ataacaatta tttatattaa attctctctt gaaataactg
14521 atacagtgtc tcttgattga aacttgacta gtagactaag aattctaact ctaaataatt
14581 ctgagggccg ggtgtggtgg ctcacacctg taatcccagc actttgggag gccaacgcga
14641 atggatcacc tgaggtcagg agttggagac cagcctggca aacatggcga aacccggtca
```

# FIGURE 3-E

14701 ggagatccag accatcctgg ctaacatggt gaaacctcat ctttacttaa aaaaaaaaa
14761 aaatacaaaa caaaattagc caggcgtggt ggcagatgcc tgtagtccca gctattcagg
14821 aggctgagac aggagaatag cgtgaacccg ggaggtggag cttgcagtga gccgagattg
14881 caccactgca ctccagcctg ggtgacagag agagactccg tctcaacaac aacaacaaca
14941 aattagccgg gcatggtggc aggtgcctgt aatctcaact acttgggagg ctgaggtagg
15001 agaattgctt gaacccggga ggcggaggtt gcagtgagcc aagattgcgg cacttcactc
15061 cagcctgggt gacaagagca aaactccatc tcaaaaaaaa aaaaaaagaa aaaaaaatct
15121 gacaattaaa taaagaacag aaaaaaaatt tgaatggcaa atacaaagct gaaaagaaat
15181 aactgagaat aaataactct gaaaatagct caaaaactaa atacctcaaa aactctttaa
15241 aaattcagaa aatataatgt tcaaatatga agtaatgctg aaaatgaaat aactaaaac
15301 caagtaactt gaaaaaaaga acataaaaat aaacaactca aatataaaat aactgaaaat
15361 aaatacctgt gaacataagc aactcgaaaa ccagataact aggggaaacc cttcattaaa
15421 acatttcact ctgaaaataa ataacttgac agtagttcat gaacttccag tgagtgttta
15481 atagtcaaat aagttactgt aaaaataaat aactcaaaaa ctccaataag ataaagtgaa
15541 ataactatga aggtaaataa ctcagataat aattgtaaag ataattaaaa ataaattccg
15601 gctgggcgcg gtggctcacg cctgtaatcc cagcactttg ggaggccgag gcaggggat
15661 cacgaggtca ggagatcgag accatcctgg ctaacacggg gaaaccccgt ctctactaaa
15721 aatccaaata aaaaattagc cgggcgtggt ggcgggcgcc tgtagtccca gctactcagg
15781 aggctgaggc aggagaatgg cgtgaacccg ggaggcggtt tgcagtgagc cgagatcaca
15841 ccactgcact ccagcctggg cgacagagcg agactccgtc tcgaaaaata aataaataaa
15901 taaataaata aattccaagc aaattaattt gaacatctgt aactcctaaa acaaaacaac
15961 tacatataaa taactgaaaa tgaacaacta aataactttg aaaataaagt aactataaac
16021 cagtaactga aactataaaa ttaaataacc agaaagctac aaagaaatga aaatcaacta
16081 ataaaactaa aaataaaata caactgaaac taacaaaact catttaaaaa ctaataaatc
16141 gatacataaa atacctcaga aagtaacttg gaacgaactc tctctacaac gaagcaatct
16201 tggcactaac acgaacaccc catgaacgct cgcagggatg ctgggaggcg gaccgggagc
16261 tcccagtctg cggcccccgc cgggttKgag cggctccggc tcctccaagg ctcgggctaa
16321 gcggctctca accgattccc accccgccct ggagaaatgc gggcgtgtct gcaggcattt
16381 ttgattgtca cgatttgggg gcggggtgga ggatggggtg gcgcattcct ggcacctagt
16441 gggtagaggc caggggtgct actaaacatc ctaccgtggg agcacagaga agcccacgca
16501 gcaaagaatt gcccggctcc gaatatcgaa gtgcgcggtc gagaaggcgt gggctgcggg
16561 ctctgctcgc ctctgcaggc gccttagagc agctccgagg tcccccgtgc ggagctaggc
16621 gcgcacccag gacacccctc gggctcctcg gaggaggccc tggttgtccc ctttctgccg
16681 ccgccgaggc tccggctgct ttctgcgtag ctgggcaggg cccgggcccc cacaccgcct
16741 ctcccgggaa tgcgggcgct ctggagccga ggagcggggg cgtccgcagg gaggtcagct
16801 ctcctgggcg gaggtcctcg ggcgcagcgc cctcgcctgg aaaccagccg tcgcccccgc
16861 aggagccagc cggcccgtgg acgccccagc gcgctcctcc tcggtgctgc gggtcgccct
16921 gcaattccga gaagaaagtc agagacgccg tggcccaaag aggcgcttag tctttcctcg
16981 ctcacactca cgtttcctcc tcatcgcgtt cttctttttc tccctggctg ctttctcccc
17041 tctccaggaa agcagatttg gaggaacagg tttcgtgact gtcgtccgac tggaaaaggc
17101 ccgcgagctg gaagggaggg gacgggtgca ccctcagagt tattgctgga ggctgtggcc
17161 agaccgggca aggtggtgac tcccgctggc aggctgaggc ccacccccagc cctcccacct
17221 gggccacggg gctctcagcg ggagccccag ttatgaccgg acaccagcgc accgccaagg
17281 agacagccac gtggggacat gctggactag gagggtcaga gccagtttga gggtctggtg
17341 acctcggctc cctggcttaa ccaggtcctt atgggtgaga atcctgagga gggggagagg
17401 gatggaggct agggacagga ggcaggagga gctgatgaat aggaaggagg gaagaagatg
17461 aaagaaacaa aagggaagta attacactca gagcactgct ctccttttcc atgtcttctg
17521 cgccttcaca gtcttcaaag gtgtccatgg agctaaactc cctgctggag cagccttagg
17581 gagagaaagg gaaggatggg gcctctgtgg ggtgggagga catcccctct gcccatggca
17641 gggtgtagca ggcagtgcct gttgcaggca cggtcctccc catctctaac tcctgctctc
17701 caagggcctg cactgcgctg ggctgtgagg gggtctgtga tctccaggct gcttttccag
17761 cgccgagatg ccgtaattca ccgagaaggc gcgtccacat gctgttcatg gccctacctc
17821 cccgttcctc caagaaaaca gtcattgttt tttgtgtttg ccagtcttct aaccacgcct
17881 ccttcccttc ctcctcccct gtctctttct caccctcccc tccttgcctc cttttctctc
17941 ccctaattaa tgtccatttc ccatctccct ggcagcctct gccaagtgtc actgctcccc
18001 ataagggaaa atcagaggaa caagcaagtg catccatcct gcctctctct gcagtgaact
18061 gattaattaa tccatcagtc ttgtctatgg cgcacatgtt acatccctgg ggcggtgttg
18121 gacactgtgg ggaacagcag ccactgccaa atactgaaca actgccctgt gcctggtggt
18181 atgttaggca tttgtcaaag tttaagcctc acaaccctgt aagggtctca gcccccttta
18241 cagttgggga aacagacagc aatggtcact tggccaagtc ctcttggcct gtggcagggc
18301 agctgtcttc tccagcactc ctgctcttta ccctcgctct gagtgagatg gagtctctgc
18361 cccacatggc tcacaagcca gggtagggag cagagcatct gcagaaaggt cccaacacag

# FIGURE 3-F

```
18421  gacagcccca gaccgagggt cagctgagta gtctacgcgg cgggagccgt gctaggaaag
18481  tgtctgctca ggcgagaatt cagggaggtt tgggcaggac ttgaggggca gacaggactg
18541  gagagggagg gcattctggg cagaggcatg gccagaggc ggtaggcggc agtggaggga
18601  gctgcagtta tctgggtgag caggcagcac aagtRgcgtc tcctgggctg ctgccccaag
18661  cccccaacaa gccacgttct gggccccagg ccctccccag agcagatcag tgggggctgt
18721  gtgagtaaca tgggggcggg ggggcagctg ggcagcacct ccctggaggc ccctctgaaa
18781  tcctgcctga ctctggcagg ctccgagggg gctggacacc ctcctctcag gttgaagcaa
18841  gtcctggttg agttcctagt cccaggaggt gggaggggca aggggtggag ggcagaggag
18901  aaactgcctc agggatgtgc cccctgcctt catcctccag acaggacttg ggagcatcta
18961  aggaaaccca agactcctct ttagagaagt catccagccc tggggtcccc ttatgccagg
19021  agcaagcagt gagaatggaa gaatgattgt cttgctgaaa gttctgtgat ggagggatag
19081  agggacagag ggagccatgc ccttgaccat cccctgcatg aataggaagg gctgtgtctc
19141  cagggtccat ggcctctgtg ccccggatga tgccagggct gctagggacc atagagccac
19201  ccactgggag gctggcggtt gggcctggct caggagcctt cgtcagccat aggcagccac
19261  agcctggggt gggcagggct gggaggcgac acaggaactg aaaaacctga caagctctag
19321  cccctccgca gggtaagtgg tacctccagg taaaatgatt agttKgttcc agcccctctg
19381  cagggtaagt ggcacctggg gtaaaatgac tgcctggagc tggcagctgc tttccctgct
19441  ctcgcgggcc ctgcagggaa gcggggaagg gaagggggca cagcgctggg cacagagggg
19501  ctctcagacc ctggactcaa ctgtttcagg gtcatctgaa acagtcaact gtttcctcta
19561  gcccattccc tgcctccagg cgaggatttg cctgaacgtg gaaagaggaa ggatcctccc
19621  agtgctgtca accccagatt ccacctccct gtgggggact gtcagcgcag gccctgacaa
19681  cgcagagaaa gacacaggac ccacctgggc cagtgacagc aggagctccg ggtgccacag
19741  gtgagggtgg ggatgcctgg agcaccacgg ggggcctggt ttagtctaga gccaggtttt
19801  ccatacacct tagagtgcaa cctcagggag atgcaaattt taccccctaa cacagcatac
19861  acgcagaaac acatttatac aattcaaaca caagcggacg gaacaatatt taccccttaga
19921  gtgtgtgaag tcctcatctg tcccacctca tcctatcatg ctttgttcta tcctaggaga
19981  caaagcagga ggggggctg cggaggtggg ggagtctcat ccaagccctt gggtgacacg
20041  tctctcctga gacaaactgc agctgctctg ggtgtgccct cgcctgtctc cctccaggcc
20101  ccgggttcct gccagcagag acagtaacct attcaccagg tatcccccag ggctcctgga
20161  agaaactcag aattctcaga accagaaaac cttagagagc atcctgcagg caaagcccct
20221  ggttctctgc ggaggaaagt gcggctcaca gggtgccccg ccagggatgg taattgacca
20281  ccaggctgtg tgccttgtgg ggactggctt aaggccctgt gggagctgag tcagggccag
20341  gaccggggtg tcctgactcc tagagatcat gttcccttcc tcacccaggc cttccagtcc
20401  cagccctggg ctttttattta tttattttgg agacagagtc tggcttgtca cccaggttgg
20461  agtgcagttg tgtgatcacg gctcactgca gccttgactt cctgggttga actgatcctc
20521  ccacatcagc ctcctgagta gctgggacca caggcacatg ccaccacacc cagctaattt
20581  ttgtattttt tggtagagat ggggtttcac catgttggcc aggctggtct tgaactcctg
20641  agctcaactg atctgcccac ctcagcctcc cacagtgctg ggattacaga tgtgagccat
20701  catgcccacc tcctgggctg acttttgctg tcttacatca tctgcatatt taatcccctg
20761  ctggattcac tggtcatggg ctctgaggcc ctaagagtct taggcactaa ggagctggca
20821  gcactgaggg gaccccaaaa tctcagactc aggatctggc cagtcacagg catgtgaggg
20881  aacaactgag aggcccattg ccccatggca ggagaaggtg ctctggagtc agtcagacct
20941  gagggcagtc agacctgatt ctcactctgt cactcactag ctgtgtgatc ttggatacat
21001  cacttaacct cttgagcatc agcttcctta tctctaaaat ggagataata acatcgattt
21061  tgcagtcttg gtatgaggat tagcaaatct tctgataaag aaaaatgcct ggtacatcat
21121  aggaattcaa caaatagtac ctgttatgat tattgtgtat agcaattaca ataatactaa
21181  agagagggtc tcaaaacagc tctgggcact ccaggtgtgc tattattact tacatttcag
21241  ggaggtttgt ctgccattgt ctcatcctca taaacactca gggaaagaaa cattataagg
21301  ataataaatg gctttaaaaa gaaacagagc aaacacacac acacacacac ccctcagaaa
21361  aaccatgcca aacacacagg ctcttgacaa atattcaatc tgattatagc aaaactgttt
21421  tgttttgttt tgttttttg tttgttttg agacaggggt ctcgctctgt cgcccaggct
21481  ggagtgcagt ggcgtgatct tggctcacag caacctcctt ctcccgggtt caagcaattc
21541  tcctgtctca gcctccagag tagctgggac tacaggcaca tgccactatg cctggctaat
21601  ttttgtattt ttagtagaga cagggtttca ccatattggt caggctggtc tcgaactctt
21661  gatctcaggt gatccacctg ccttggcctc ccaaagtgtg agattacagg cgtgagccac
21721  catgcccagc tgattatagc aaaattctaa gtgatagttg tattcttgga aaatgaatgg
21781  aacgactttt gtcccagcca agatctagtg gtgtgttgga gcagatacac cctgagtctc
21841  tggggcactc tcagtctatg tatcagataa gcataaggag tattggtgga gaaggacaag
21901  aatgggaaag gtgggcgatg agaattcctg cagataagga ctggtgagag tattctcttt
21961  tgaaaccctt agtcgacaat ctttctggtg catatcagat aagctgaatg gtttaggaaa
22021  tctagtgttc acatttagtg cttagaattc taagcttttt tattttgctt aaacaaatgg
22081  aatgaaattt attaacaagt gaacctagta atgagctgaa attattctca ccagcataca
```

# FIGURE 3-G

```
22141  tatttttggt aaattataga ctttgaagac aaaatcatgg tgtttccctt actgtccagt
22201  ggatggcaca aagagaccat tgtagatcct gctggttcag cggtagctct caatccatag
22261  atattaaatg ggcaaattct attttattg tctttcaaac tagattttc tcaatgcaca
22321  acttttttt tctttttctt ttttttttt gagacggagt ctcgctctat tgccaggctg
22381  gagtgcagtg acacgatctc ggctcactgc aacctccgcc tcccgggttc aagtgattct
22441  cctgcctcag cctcctgagt agctggggac tacaggcgca tgccaccatg cccagctaat
22501  ttttttgtaa tatcagtaga gacagggttt caccatgttg gccaggatgg tctcgatctc
22561  ctgaccacgt gatccgccca ccttggcctt ccaaagtgct gggattacag gcgtaagcca
22621  ccgcgcttgg ccaattatgc acaactttt aaggaccatc tctatcacat gaactaaagg
22681  atatcatttt cacttggggg tgggaagtgg tgagctgctt aaaagcaatg cctaaacccc
22741  ctgggctttc cttcctttca cttggaagaa ccagggggta actaactgct aacaattcca
22801  tgctttcaga gatctaggcg caagcctaag ggcttcatct catttaatcc tcatggcaag
22861  actgtacagt attatctcca ttttgtaaaa tgaatgataa aaagaactta agcacagaca
22921  ggctatagaa tccatccaaa gagatggagc tgcacttgag gctgggtctt tgagacctga
22981  gtttgagccc ttcatcattg tgttgtgtgt gctgctaacc aatttcccct ctctgtcttc
23041  taggatttaa tacatggtgt cacattctgt ctgatccatc ccagtagctc tccagagctc
23101  ccaacaggag agagttccaa aatgtttcca ggggtactag gctcggttac aatattttgc
23161  ttggtggccc agagtataaa cgtggatatc ttaggctggt ctatagctga aacgtctatt
23221  tcatttgcaa gcctatcttt ggctaagagg aagtgaatca ttcttgagaa catctaatta
23281  attgctttca gattccacat gttgacattc tcagggcaca ttttttttt cccgtgcatt
23341  tctgttgtca aagtccctgc cagctcctaa ggcagtctga gctggctgtc ttagactttc
23401  agagctgctg gaagcttggg ggagggaggg gctgtaggtc aaagaaactt tataacctag
23461  ctttacctcc cagctcagcc accagctgcc ctcaaatgtt ctggattgga ataagcccaa
23521  agatgagtgg caggagggaa gggcaagcca atacgtctag tttggttcag tcaaagcctt
23581  gcccatttca tcagaatttt aatggaaaat ttccaataag attaaaatat aaggtcaccc
23641  caattttagt atggctccat ttaaaaaaa tcatgcatat ctttgttttg caatggggcc
23701  ttactcttgc ccaggagagg tgcggtggta caatcatggc ttacggcagc ctcaacctcc
23761  tgagctcaag caatcctttc accttggcct gccaaatagc taggattaca gacacccacc
23821  accatgccca gctaattttt aaaaaaaatt ttttgtaggt ccagtgcagt ggctcatgcc
23881  tgtaaatctc agcactttgg gaggccgagg caagcggatc acctgaggtc aggagtttga
23941  gaccagcctg gccacaatgg tgaaactgcc gtctctacta aaaacacaaa aattagcgag
24001  gtgtggtggt gggcacctgt aatcccagct actcgggaga ctgaagcagg agaatcaatt
24061  gaacctggga ggtggaggtt gcagtgagcc aagttcgtgc cattgcactc cagcctgggc
24121  aacaagagtg aaactccgtc tcaaaagttt ttgtggagat ggggtcttgc gatattgccc
24181  aggctggtct caagctcctg gatatcaagt gatcctcctg ctttggcttc ccaaagtgtt
24241  gggattacag gcatgagcca ccacaactga ccaaatcatg catttctata gacaacgtct
24301  gcaagaagat attaatggtg attatatctg gttatgttgg atttgtattt ttatttgtac
24361  tttcctgtat tttctaaatt cctgacattc tacatgtgta ctcctttaat aatcagaaaa
24421  ggaaacttaa aatagttaaa accaattggt cagatatgta aaataaccca ccatctctcc
24481  agagagggct gtttgctaga acttatttc ttcattgaaa tactagagtg ccccaataag
24541  tttgaataac acaaaaaaaa agataatgaa agtaactaaa ttatctaggc ccaaaaggaa
24601  atgccacaaa aattggcaaa gaaacaacca tgacgtgcta taccggatag ctcctaggcc
24661  cccttggaga ccctgaggta cccgacgagg gacctgtagt aaggctggca gacaggttct
24721  tcctctgtta gctctgaggt acaacagtta ttctcatttt tatgtctttc acatggccag
24781  aactttgcaa atcagaggca aagtgaattc agaattaaaa atttcagca ccatccaagt
24841  cagtaaaaca gtcttagctt ataaccttta ttttttttat tattttatta tttttttatt
24901  tttggatgga gtcttgctct gtttccaggc tggagtgcag tggcatcatc tcRgctcact
24961  gcaacctccg cttcctgagt ccaagtgatt ctcctgcctc agcctcccga gtagctggga
25021  cgacaggcat gcgccaccac gcccagccaa tttttgtatt tttagtagag acggggtttc
25081  accatgttgg ccaagatggt ctcaatctct tgacctcctg atcctcccgc ctcggcctcc
25141  caaagtgctg ggattacagg cgtgagccac cgcgcccagc cctttatttt tactgtaagc
25201  tgccagtaaa cagcacaccc acctgtctgc tgactgtccc atctggaagt tgtgtaggtc
25261  cttactgaat cttaccttct tcccttcccc tacccagacc ccatcccagc tggcacgtgg
25321  aatcctttcc ctttctcctc atgtcaatag accagcaggc aaagagagca gttaccatag
25381  tggaaggaga aacgggtcct agtcgtggtg aggaggtagg gctgctgctt aacaRaggcc
25441  gagagggaca tgtctgatgc tcaggtgatc cactaggaca tctcgcaata ctcccatgcc
25501  cagctgtaac tatgatggcc aagtccaaga accatagcct gataagagtc taggaccccc
25561  actatacatg ccaccagtag aagtgcctga tggggagagg gatgtctaga atgagtagga
25621  gggaatgatg acaaagcaca gccaaggacc aactgcagtg gcaggtggca gccacagatt
25681  gagttaagta ggattcttgc tgtgttccct agtggaagca tctccccttgt tgagacccac
25741  tgaggttaaa gttggagaca tggggagaaa acaatgtccc cgtgttcatc actagagtca
25801  tgatgggcaa ggtcacaact catttgactt cttggctccc agattcatat actctgcctc
```

# FIGURE 3-H

```
25861  ttgggggcac ttcaccataa tacagccatt ggttcagttg tatgctgcat cctgagggac
25921  agcactccat ccccgcagtg ccatgtccaa actgctgccc cgcctcactc tcaagaggct
25981  gtcccactac tctgtcaggc accagcttct gaatgcagtg gtaagactct gtcctgtgat
26041  cacgtctatg gctgattctc ttttgccata ctgagtcccc tgatcaaagc agtgttatac
26101  agcctggcgc gctggctcat gcctgtgatc ccagcactct gggaggcaga gggggatgga
26161  tcactagagg tcaggagttc aagaccagcc tggtcaacat ggtgaaaccc tgtctcctct
26221  actaaaaatt caaaaattag ctgggtgtgg catgagcctg taatcccagc tactcggaag
26281  gctgaggcag gagaatcact tgcacccagg aggtggaggt tgcagtgatc cctgatggtg
26341  ccgttgcact ccagcctgag cgacaagtgt gaaactctat cgcaaaaaaa aaaagcaacg
26401  cttatgattg atcacacact gtactttcta acctgaaggg gtctggtaaa atcaacttgt
26461  cttctcaagg gacagtacca aactgggggc tcagcactga cctctgctgg catgtcggac
26521  atttaaaagt ggtagcagct agagcagtct tgaagacatg gagccctcac tgctggggcc
26581  gagcactgct gggttcaatt ctgctaccaa ggcttctcca tttccgagcc catggtgcag
26641  gcactggggt gcctaagagg aagtgcacta actggacttc tgctatgcac cttctttagg
26701  gggcattaaa gcacagttca aagactctca cattttgagt taaccccccac aggcctgtgt
26761  gccccttctc cagtcaggtc atgttgtcat ttgatcctag ttattattct gaaagggttc
26821  aggaagggag gtggggacag atcttgRaaa gggcaaacat tgccttgtat gcctcatctg
26881  aggcttccgc atagtcttag gggactggga accgtcttaa gtgaatctca ccttcttccc
26941  ttcccccagc caggccctag ccctactggc atgaggaatc ctatctcttt gtcctcatgc
27001  caggagacca gcaggcaaag agagcagtta ccatactgga aggagaaatg ggtcctggtc
27061  atcagaagga ggtactcctc aagggacagt acctgaggga gacacttcca ctagggaaca
27121  cagcaagaat cctacttaac taaatctacg gctgccgcct gctgctacag ttggtccttg
27181  gctgtacttt gtagtacaag gcgaagtagg ccactgctgc cagcccagga ggtcaagggc
27241  atttacccag acatcctcat tcccagtttt tgggtccctt atcttccctc tcaggatcta
27301  atgactcatc tactggcact aggagtccaa aatgaccaga tggcagactc gcctaggctg
27361  agaattcttc cttctctcaa attctgccac ttttaccagc ccagtctgcc tctgctgtag
27421  gagatgagag gctaaatgtt ggcagggagg ccccctgatt ctgatcctgt gttttaaatc
27481  gataattagt cYgaccctgt tactttctcc cttcagtgca tcaacgggag gctcttagca
27541  acagcctccc aactctacct tcctgataga taatattttc ctcaaagctc tcaaatgcta
27601  gagacattgc accagctggY gtgtccccat ccacctgtat ctgatcccag gtcaccacag
27661  gtgaaggtct gagcaatYga actgctacag caggccaggt accaggactc tcaRcacgcc
27721  acttaacacc aataatgagg tcctcatggc catttggcct gcagtgagcc aacaccagaa
27781  tcccatccat tctgggaccc ctcctgctac taacggtctt cagtcaggtt ccccagaagc
27841  agattatgca aatgtgttat tgaaaaacgg ctttcagatg aaacctggaa gacagtgaag
27901  gccgaagcta gggcagggaa gaagccaggc acagacgtgg gcttagcaga agtctagcat
27961  cagcctgatc ccctgggcag tactggagca tggatggcac cacaggttac catcttgaga
28021  cagaaggact ggcttctgta ccctgaatca gtaagccatt ggtgggccat aagccacccct
28081  taggggagga cataacctca caggcatttc ctggctggac gaccctgggc agctgagggc
28141  aactgcctga agggcacaac ggtgagccat tgtcagctaa cctcacagca gcagagacat
28201  ggggccaaca gcctataaag agggtctggg caggctgtca atactctcta ctgtaatact
28261  gtatatgtgg tttattttaa aaactttttt agaaggcttt attttatttt atttttttgag
28321  aaggagtttc gcccttgttg cccaggctgg agtgcaatgg cgcgatcttg actcaccaca
28381  atctctgcct cctgggttca aggaattctc ctgcctcagc ctcccgagta gctgggatta
28441  caggcgtgta ccaccatgcc cggctaattt tttgtatttt tagtttcatc atgttggcct
28501  ggctagtctt caactcctga cctcaggtga tccgcccacc tcggcctccc aaagtgctgg
28561  gattacaggc gtgagccacc gcacccggct gactttattt ttttagagca gttttaggtt
28621  cacagaaaaa ctgagaggaa ggtacaaaga tttcccatat atcccctgtg ctcacacatg
28681  catggcctcc ctatcaccat cccccaccag agaggcacat ttgttacaat ggatgaacgt
28741  atacactgaa tatatcatat tcacccaaag tctgtagctc acatcatggt tcactttggc
28801  tgttgtacat tccttggatc tagacacttt tataatgaca ggtacagtag tccccccttta
28861  tcctcagggg ctacttccaa gatccccagt ggatgcccga aaccgcagag agtgccaaac
28921  ttgactgcca tcagtgggaa tatgtttcta ttcgccttcc accaccaccg gtttaacgcc
28981  tttttcatct tagtgctgct gccgtaactt tggcagtttg agatgcgaca gcaaaatgag
29041  tacaaatttc tttctccttc ttcacaatgt catggacaga tgattccttc ttaccataga
29101  tcttagcaac ctcggtgtgt gatttttttt ctttcttgtt aaatcaactt tcacctttttc
29161  acttaaagga agcatttgac ggcttctctt tggcatatct gaatttccag catcacgact
29221  gtgctttggg gccattgttt gtttatttat ttatttattt tatttatttt tttgagacag
29281  agtctcgctc tgttgcccag gctggagtgc agtggcgtga tctcggctca ctgcaagctc
29341  cgcctcccag gttgacgcca ttctcctgcc tcagcctccc gagtagctgg gactacaggc
29401  gcccaccacc aagcgcagct aattttttttt tttttttgta ttttagtag agatggggtt
29461  tcactgtgtt agccagggtg atctcgatct cctgacctcg tgatcctccc gcctcagcct
29521  cccaaagtgc cgggattaca ggcatgagta ttttatttat ttatttattt tttcagacag
```

# FIGURE 3-I

```
29581  agtctcactc tgtcggccag gctggagtgc agtggcacca tctcgctcac tgcaacctcc
29641  gcctcccagg ttcaagcaat tctctgcctc aaactccgga gtagctagaa ttacaggtgc
29701  acaccaccac gcccggctRa tttttgtatt tttagtagag acaaggtttc accatcttgg
29761  ccaggctggt cttaaactcc tgacctcagg tgatccaccc acctcggcct cccaaagtgc
29821  tgggattata ggcgtgagcc accgtgcttg gccttggggc cattattaag taaaataaga
29881  gtcacttgaa cacaaacact gtgatcctaa cagtcgattt aatcaccaag atggctataa
29941  gtgactaagg ctggcggggt ggggagcaca gacagcaggg acaccctgga caaggggata
30001  attcgtgtac caagcaagac acagcggaag gcgccagatt tcatcgcact actcagaatg
30061  gcatatcatt taaaactcat cgattgttta tttctgtaat ttttccattt gatatttgga
30121  cagcagttga ctaagagtaa ctaaaacctg gaaagtgaaa cagtggataa gggggtgctc
30181  ctgtacttct caatgtgaga catttgccct tcatgttaat tctgccccat tagYtctaca
30241  caaatgaata gcaggaaatt gattttaaac cacatggtgg taaaatgctt tctttttct
30301  ccctcattta actaaagaag ggctgggccc taggtgatgt cttccttagc atctaagcag
30361  ctggcatcac cccactgctt ctgtgctcca ctctcccatg ggaccctccc tacctttaat
30421  ccctcctgtg ctggaggccg ggtcttcctt gctagtggta gctctttcca tatttttaat
30481  ccatggtccg gatcctgctc cactgccttt gctttagaga aataaacatg aatattgagt
30541  cactggaagg aatgacacac gcatccctcc cccaccagtt ggagtaacgc tggcccacct
30601  agtgtatctc tggtctaggt ctcgaggacc tgctgctcct ccctcacctg tagttgaaga
30661  cctgcctcag gtcagtaggt gatacgcagt aaggaaatgt ccaaaggaca cttcttgttg
30721  gattacacag caaacatcta attggctgca aatctttttt tctttttctt tcttttttt
30781  tttgagacag agtctcgctc tgttgcccag gctggagtgc agtggcgcaa tcttggctca
30841  ctgcaagctc tgccacctgg gttgacgcca ttctcctgcc tcagcctccc aagtagctgg
30901  gactacatgc gtgtgccacc acacccagct aatttttgta ttttagtag agacgaggtt
30961  tcaccatgtt ggccaggatg gtcttgatct cttgaccttg tgatccgccc gccttggcct
31021  cccaaagtgc tgggattaca tggctacaaa tcttaaaggg ggaagagatg aggaggaata
31081  atccccttg tcttctcaaa aatgttttca ctggctcact acagctccct ccttcctctt
31141  tactgaccca aaatgccaac tattatagta actcttttgg gttagacgaa tccagtgaat
31201  aaacacctac taagcattgg aggtccaaga ggaataagat atgcctgagc taaacctcat
31261  caccccggc cttgcttgca gagtggtttg ctggcctcat ctgattattc aatgagtgct
31321  ttcatttgtt tattcactaa atatttactg agcacctaca aaagtgcctg gccctggtag
31381  atgaggcctg agggaagcta aaactaataa gacaaactcc atgccctaga ggaattcatg
31441  gtctcatagg gagaaaatgt aaacacatca taaaattata gcttattaaa tgctgcaata
31501  gagtactccg taagagtgtg ggggcagaga ggaggggag aaacagctgc tttctagagc
31561  cctcaccttt tccacttctc tcatctttct gggttagggt ctagcggggg ttccatgagg
31621  atcaggctaa atgagcttag aaaaactaag cagactactg tatcagaact ggatcacagt
31681  agacaggcgt tttcaacaaa catatattga gtatccctga gtgctttgga caggaagagg
31741  aattatagac gaagattgta agtcgcagta atagatgagg caaggagcac cagtgaggac
31801  ttaaccctga aagaagtgtg agcacatctt cctcccagac aaggggaat aaagaaagga
31861  agatgatcag gagagttcta agtggaactc agcagccaga ggggaagcYg gaggaggtaa
31921  catcagaggg tgcgtttgcc cactcagtaa agtaggaggc agggcagcct tgtgaaaata
31981  ggaatggaat agaaagctca agaaaacagc caaaagcaag ggcaattagg ggaggtttcg
32041  aactggcaga tctacctcaa ctggcaatac agcaagcatg caccagaaaa gatatcctag
32101  ctagcagtgg ggttgggaac tgatttaata tcctaggcta gcagtggggt tgSgaactga
32161  tttattattt aatttttat atctattcat gtatttatgc acttatttat ttttgagata
32221  ggctctcgct ctgtcacaga ggctagagtg cagtgatgtg atctaggctc actgcagctt
32281  tgacctcctg ggctcaagtg atcctcccac ctcagcctcc tgagtagctg ggactacaag
32341  cacgccacca cctaggctaa tttttgtatt tttttttgtt agagacaggg tttcaccatg
32401  ttgcccaggc tggtctcaaa ctcctgggct caagcgatcc atctgccttg gcctcccaaa
32461  gtgctgagat tacaggcgtg agccactgcg cccagccaat atttttaaa aattggaaat
32521  ccccttgtaat aagccaagtg ttgggggaag aaaagcaata aaagcaatga catggactca
32581  atatgaaaca tccaaagcat ttgacatgcc tttaaaataa aaaaatcagt actcacctcg
32641  tgctcatttc aaactgtgga tttccttggt ctaaaatttt aaaaaacaaa aaacagcact
32701  ctcaaattta agctaatttg aattgatttt acatagtgat ttttaaatac acatatatgc
32761  ataaagagaa tactataaaa atatataaag gtgtgtaggt cttgatcata ttttcccagg
32821  aattcagaaa acgctgctac agtccctggg ctcacgtggt cctggcatcc tccccaacgt
32881  ctctacccca tcctgctgag tttctggcat gcattggttc tgggtctgtc caagtttcta
32941  ctgttagctc cattaatact gaattaagga ataaacagtg ctcaaatgct catttttcc
33001  atgtgagccc aaattatgtg acttgatgag ggaaaaaatc atgagtccct gggagagcga
33061  taagaatcac attctttact aaagtgttgt cccaggtatg agaataacac cagatctcaa
33121  cctccagagg ccccccactg cctcccacag cataaaagcc aaattcctcg gcccgatatt
33181  tgaggccatc tcaatctttt tcctttcacc ctatacctga ctctcccagg ctaggctcag
33241  atcgtcactg aatgtgctca cttcgaggca cctctgtgat tttcaaggtt cctgggccca
```

# FIGURE 3-J

33301 cctttacta ccgatgtgac tgccacatgt tgcccagttg ctaggatggg accgtggcct
33361 tgatttctgc cgggactgag gctttgcctt gtttcccacc caccctgctg cctgcccctg
33421 cactcttctg gctggggcct gatctttccc cgcagtctcc cttcacctct agatcacagg
33481 cagtcatgcc acagctgagg agcttgtccc aaacctcagt gcctgcctcc ctcctcggct
33541 tcttgtgctg ctgtgtctca cccatcagtg aYgctcttct cttccctgcc caagccctag
33601 ctgactccat aacacctgga tacaatgtcc tcttctgtct ggttacctcc gagaggccct
33661 ctctctacat ccctcattgg ctcccagtgt cattcctctc acccatggcc ctaaggtcac
33721 tgtattcttt ggccagtgta cagggttatt atgcttaaca atccacaaag gttgaaaggt
33781 gttgtaggat ggtgtaaaaa tgaatctggg tggtaatgtt tatatgtcag agctttgtaa
33841 agtgctcggc aggcgtaagg tactgacagt cctgatattc ctgatcttgg aacctgggac
33901 accatcttct caacattgcc cggataccct caagggtatc cagacagcct gagtttgcat
33961 tctgttcctg gttcagccca gggccctggt tcccgctcac tcaccatcca ctgtgggccc
34021 tctctaaatt cttaaagccc ttgccatttg catcactcac agagacattt catcagagcc
34081 tacttggtgc accaggctca ggagactcag ttctgctgcg gataagttat gtaaaattga
34141 ccctctgctt gcacatctgt aaaaggaagg ggctggcaca acacctctgg gcctttcagt
34201 tcagtagtgt tttcttttta tctaaaccac gtgctgggtc ctggttttgc ttcttatcta
34261 gatttttgca ttcctgtcac aacctataaa gcacagttca ggccttaagg agggcttgag
34321 aaatctcttt ccgaattgtc aactgaataa gtgtcatatc ttcataacaa acttgtcttt
34381 ttttgcaggg ccaggaaggc agcaggggag tcagttaaaa tataaatttt agattaagct
34441 taatattgtt aagaagtcaa ttctcaccaa attgttctag agattttaca taatcctaat
34501 caaaatctca ataaggtttt ttgagaagtt ggcaagcaga ttctaaaata tatatagaag
34561 tataaaggac acagaataat caaaacaact ttaaaaagga agaacaaaat tataggactc
34621 taactacctc attttaagac ttattagaaa gcagcagtaa ccaagatagt gagggactga
34681 tgtcaaggta gacaaataga tcaatggaaa agaatcaggc atccagaaat agatgcactt
34741 acataaatca tcaattggag aaagaatagt gtttttaaca actggcaatg gaaaaactag
34801 aacatcagta tgcaaaggac tgaactttga tccatacctc acaccacata caaatcaaaa
34861 acagaaacaa acaaacaaac aaacaaatac tcaaaatgga tcagagacct aaatgtaaaa
34921 ctataaaact tctggaagaa aacacaggga gaaaatcttt atggctttta gataacgatt
34981 tcttaggcag gataccgaaa acatgataca tacagttttt aaaattaaat attataagaa
35041 ttaaagtctt ttgctcttca aaagtcactc ttaagagaaa aagatgccac acactagaag
35101 aaaatattta caaagcatta aaaggacata tatctaggat atacataaaa actctcaaaa
35161 gtcaataata agaaaacaat gagcaaaaga tttgaacaga cacttcacca aagaagagat
35221 aaagatggca aagaagcaca taaagagatg ctcaaccatt agttactagg gaaaagcaaa
35281 ttaaaaccta atgacatacc actatgcccc tattagaaat ctgaaaattt aaaagactga
35341 caataccaag tattggtgag gacatggcac aagtggaact ctcatacatt actatgggaa
35401 tggaagatac tataatcact ttggaaacta tttagaaatt tcctaaaaaa ttaaacatac
35461 acctaccata tggcctaacc attacactct taggtattta cccaagagaa atgaaaacac
35521 atgtccacac aaagacttgt acttgcatgt tcatagcagc attattcaaa atagccccaa
35581 agcagaaaca aatcggatgt tcattaacaa gtaaatggat aaagaaaacg gggtctagcc
35641 aaacaatgaa atactactca gcaacaacca aaatatgtac tattgtttac aaaatccaaa
35701 tagatgaatc tcagaataat tatgcagagg agagaagcca gaccaaaaaa aaaagtacat
35761 agtgtattat ctcttatgaa attctagaaa atgcaaacta acctatagtg acagaaagga
35821 gattggtggt cacctggggt gggtgaggga ggggcaggag aaagggaatg caaagcagtg
35881 tgaacaaacc tttggcggtg ataggcatgt tcattatcct tactgtggtg atgccttaca
35941 gatgtataca gatgtcaaaa cttatcagat tgtacacttt aaatatgtgt ggtttatcgt
36001 gtcattatac ctcagtaaag gagttttaaa aattgtagta aaaggtctct acctagaaac
36061 cttaataagc taaaatgtat gtccccagga ctaaccctag ctattctata gttctggggt
36121 ggaacctaga aatctgcatt tgtagcaagc ccttcaaata atttgaatgc aggtggtcct
36181 taaaccaccc tttgagaaac actgacctag tgagtaagga tttctaaaca agcttgtgac
36241 tagaatgttg tttctgcagg gaacaagtga ctttttctac tagagttgcc atatcattct
36301 gtgttaagcc tctaggacac tcctactcaa ttaccatgac atatatttaa cataatatta
36361 atagttgtaa aacaaaagta aaccatagtt tttttcccat cttatctgta tacaaagtaa
36421 aatcaatgac ataatattgt tactagttcc tggcagttac ctacagttta aatcaagaac
36481 ttatgttgct tggtttttgt gacagaaaac tgatgtggtg gctttccagt tacactgctt
36541 ggtttacctt ttcccttatc ttatgatgca caccccagctt tgcacgttcc tgagcagctg
36601 atgatcatag aagacctaga aaaagtgtga gccagttgca ttcctgctat ctctaaagaa
36661 aaaagttatc ttttcatttt tatgtgattt ttcttaccca aaatgcaaac cactgccagt
36721 ataaatgatg gaagatagac tttccacatg aaactggcat ttcttcaccc tagtaacatt
36781 acctgagggg aaggtcagaa ctcaattgat aaagctcgct gtggattttc ccaccaaata
36841 tccctgcaaa caaagtgaaa ggataaagcc tgcttaaaga tgatttgtaa ttgttgaaag
36901 gagtacagtg tgtgacatca gtgaaaatgg tggagaaaaa acccgtcccc aaattctctt
36961 ctttgtgaaa gcatggaaaa aactggccaa aaatggttag aaaatttatt ttcagaatat

# FIGURE 3-K

37021 cgaaattttt ttttcctttt tttttttttt attatacttt aagttctagg gtacgtgtgc
37081 acaacatgca ggtttgttac atatgtatac atgtgccatg ttggtgtgct gcacccatta
37141 actcatcatt tgcattatgt atatccccta atgctatcta tcccttcccc ctccccccac
37201 cccaccacag gccctggtgt gtgatgttcc ctaccctgtg accaagtgtt ctcattgttc
37261 agttcccacc tatgagtgag aacacacggt gtttggtttt ctgtccttgc gatagtttgc
37321 tcagaatgat ggtttccagc ttcaccatgt ccctacaaag gacatgaact cattgttttt
37381 tatggctgca tagtattcca tggtgtatat gtgccacatt ttcttaatcc agtctatcat
37441 tgatggacat ttgggttggt tccaagtctt tgctattgtg aatagtgctg caataaacat
37501 acgtgtgcat gtatctttat agcagcatga tttataatcc tttgggtata tacccagtaa
37561 tgggatggct gggtcaaacg atatttgtag ttctagatct ttgaggaatc gccacactgt
37621 cttccacaat ggttgaacta atttacagtc ccaccaacag tgtaaaagtg ttcctatttc
37681 tccacatcct ctccagcacc tgttgtttcc tgacttttta aagattgcct ttctaactgg
37741 tgtgagatgt tatctcattg tggttttgat ttgcatttct ctgatggcca gtgatgatga
37801 gcatttttc atgtgtctgt tggctgcata aatgtcttct tttgagaagt gaccgttcat
37861 atctttgcc cactttttga tggggttgat ttttttcttg taaatttgtt taagttcttt
37921 gtagattctg gatattagcc ctttgtcaga tgggtagatt gtaaacattt tctcccattc
37981 tgtaagttgc ctgttcactc tgatggtagt ttcttttgct gcgcagaaac tctttagttt
38041 aattagatcc cacttgtcaa ttttggcttt tgttgccatt gcttttggtg ttttagtcat
38101 gaagtccttg cccatgccta tggcctgaat ggtattgcct aggttttctt ctagggtttt
38161 tatggtttta ggtctaacat ttaagtcttt aatccatctt gaattcattt ttgtataagg
38221 tgtaaggaag ggatccagtt tcagctttct acatatggct agccagtttt cccagcacta
38281 tttattaaat agggaatcct ttccctattt cttgtttttg tcaggtttgt caaagatcag
38341 atggttgtag atgtatgata ttatttctga gggctctgtt ctgttccatt ggtctatatc
38401 tctgtttttg gtaccagtac catgctgttt tgattactgt agccttgtag tatagtttga
38461 agtcaggtag cgtgatgcct ccagctttgt tcttttggct taggattatc ttgacaatgc
38521 aagctctttt ttggttccat atgaacttta aagtagtttt tttccaattc tgtgaagaaa
38581 gtcattggta gcttgatggg gatggcattg aatctataaa ttaccttggg cagtatggcc
38641 attttcacga tattgattct tcctatccat gagcatggaa tgttcttcca ttggtttgtg
38701 tcctctttta ttttgttgag cagtggtttg tagttctcct tgaagaggtc cttcacatcc
38761 cttgtaagtt ggattcctag gtattttatt ctctttgaag caattgtgaa tgggagttca
38821 ctcatgattt ggctctctgt ttgtctgtta ttggtgtata ggaatgcttg taatttttgc
38881 acattgattt tgtatactga gactttgctg aagttgctta tcagcttaag gagattttgg
38941 gctgagacga tggggttttc taaatataca atcatgtcat ctgcaatttg acaatttgac
39001 tttctctttt cctaattcaa taccctttat ttctttctcc tgcctgattg ccctggccag
39061 aacttccaac actatgttga gtaggagtgg tgagagaggg catccctgtc ttctgccagg
39121 tttcaaaggg aatacttcca gtttttgccc attcagtatg atattggctg tgggtttgtc
39181 ataaatagtt ctcattattt tgagatacgt cccatcaata cctagtttat tgagagtttt
39241 tagcatgaag ggctgttgaa ttttgttgaa gacctttact gcatctattg agataatcat
39301 gtggtttttg tcttcggaga acactggaaa ttaaatgatg gcttgcagca atctggagag
39361 catttattca aggaaaatgg ctgtgtctca gtatgactaa tgagcttttt aacttgccct
39421 atttctatcc tccccttcct tggtggtagc cttagaaatg aacagcctgc aatgatagtg
39481 aaaatcagca gtctggcagc catgggaggg gcagaacagg aatgggggaa ctatggagcc
39541 tcattcttag agaattatca ttatttgatc tgtccacggg tttctaggaa tacctgacct
39601 gcaagtctgt ctttattagg cctgactcag aacttgccca atgtgaaaag tcttttcccc
39661 aaaggccttt gtagaaaatg attacaggca attgtttaac ttcttggttg ctcgaggtat
39721 tggctaacag tggggcaaac atgggctaat cagaaggttt aaaatgaaat gctcaggaat
39781 aagatgctca tagagggttg taaaggctcc aaaatattta tgagactcta gaagaccatg
39841 cacacatttc ctgtgaacat gttcaggaca gatctgcgaa ggccccacga tcttacctct
39901 ggctgatctt gatgatctgc acaaacagaa agtgaaagct aggctagaac tgtcaagtgc
39961 caggctgagt gtgaaggtgt gccctaatgt gcacacagag ccccttggca aagactagaa
40021 gacttactgc tttcaggtgt ttaaagaaat ctctgtcatg tcattagtat ttagatcact
40081 aagctaactg aacagaggct tcaatggctg cacataaata cagacttcac agaattagtt
40141 tagaaaagtc actataacaa acaacaataa acagcaacac caacaaacag tagaggtggg
40201 aaggtccaat ttccagagtt gctacattat gttatttaaa atgtgcaatt ttaacagaaa
40261 ataatgagac atgtaaagaa ataagaaaat gcagtccata cccagggaaa aagaaaaacc
40321 agtcaataca aactgttcct aggccaggtg cagtggctca tgcctgtaat cccagcactt
40381 tgggaggcca aggcaggcag atcaccagag gtcaggagtt tgagaccagc ctgaccaaca
40441 tgttgaaacc ccatctctac taaaagaaaa atacaaattt agccaggcat ggtggcgtgc
40501 acctgtaatc ccagctactc gggaggctga ggcagaagaa ttgcttgaac ccaggaggcg
40561 gaggttgcag tgagctgaga tcatgccatt gcactccagc ctgggctaca gagcaagact
40621 ctgtctcaaa aaagaaaaaa aaagaaaaaa gaaaaaactg ttcctgagga agccaagata
40681 ctgactttac tagaccagta ctttaactat ttttacatgt tcaaaaagtt aaaggaaatc

# FIGURE 3-L

```
40741   atatataaag actaaaggaa agcacaagaa caaatcctca ttaaatagaa aatataaaga
40801   tttgttttaa aggacgaaac agaaattcta gatttcaaaa gtataataat tgaatgagaa
40861   attcactaga gtggctggct caatagcaga tctgagcagg cagaagaaag aattagagaa
40921   ctcagaaata ggtcaattga atctatccag tctgaggaag agaaagaaag aggaatgaag
40981   gaaaaatgaa tagagcctca gagactgtga gataccttca agcacgctaa tgacgcataa
41041   tggcagtctc agaaggagag agggataaat gggctaaaat aatatttaaa gaagcaatgg
41101   ctgaacattt cccaaatctg atgaaaacat taatctatac cttcaagaaa gtctataaac
41161   tccaagaaat ataaattcaa agagatcaag atccacacct agagataaca taatcaaact
41221   gtcaacaaag acaatgaaat tatcttgaaa gcagcaagca catagaagga ctcttcaata
41281   agattaacag ctgatttcta tcagaaatca cagagttgag aaggcaatgg gatgacatat
41341   tcaaagtgct taaagaaaaa gagggtcaac aaggaattct atacctaaag ccaatttatc
41401   ttcaacaaag atgccaagac cattcaaagt ggggaaagaa tagtcttttc aatcaatggt
41461   tctaggacaa tggatatcca catgtaaaaa aaggaacttg ggcccctaat tcacatcata
41521   tacaaaaatt aactcggaat gagtcaaagg cttaactgta agagttaaaa ctataaactc
41581   tttgcaaaga acactatcaa gagagtaaaa agacaatgca cagtatggga aaaaatactt
41641   gcaaataata tatctgataa aagtccagta tccagaatat ataaataact cttacaattc
41701   aaccataaaa tgacaagcca attaaaaaac atacaaatga gttaactgac atttctacaa
41761   agaagatata ccaatggcca atatgcatgt taaaagatgc tcaacatcac tagccatgag
41821   ggaaatgtaa atcaaaatca caatgagata ctagtacatg cccactagga tggcaatgat
41881   aataataata ataataataa tgttattata atgaacaata caagtgttag taaggaaatg
41941   gagaaaattg aacccaacta ttatattgct ggtgtgaatg tgaaatggtg gtactgtttt
42001   gaaagatgat ttggaagttc ctcagaaagt taacaaaagt taccatatgg tctggcaatt
42061   ctacatatat acaccaaaga aaactggaaa taaagattca tacaaaaatc tgtacaaaaa
42121   tatctacagc agctttactt acagtatcca aaaaggggaa aaaMcccaaa tcttcatcaa
42181   ctaatgaatg gataaacaaa gatggtatat ccatacaatg gactgttact cagccataaa
42241   aaggtatgaa gtactgatag atactacaac ataaatgaaa cttaaaaaaa tacgcaaagt
42301   gaaagaagct agacagaaag ggccatatat gtgatgtgta gagaaaacat ccaatagaga
42361   caggaaatac attagtgatt gccaggggct gggtaaaggt gagagatgga gaatgattgc
42421   taattggtac agaatttttt tgttgggaga gttatgaaaa tattttggaa gtataattag
42481   aattaggctg ttaaatactt taaaacaaaa aacagctggg cgcagtggct cacacctgta
42541   atcccagcac tttgggaggc cgaggcaggc agatcataag gtcaggagat caagaccatc
42601   ctgcccaaca tggtgacacc ctgtctctac tacaaacaca caaattagcc aggcgtgttg
42661   gcgcgcctgt agtcccagct attcgggagg ctgaggcagg agaatcactt gaacctggga
42721   ggcagaggtt gcagtgagcc cagatggtgc cactgcactc cagcctgggc gacaagagca
42781   aaaactccat ctcaaaacaa aacaaaacaa aaaacaaaag taagaagtaa aaaaatagaa
42841   ttagactggg gatggttatt gagccttgtg aataatctaa aacccactga attgtatatt
42901   ttagacagtg aatttgatgg catgttaatt atatcttgat gttttaaaaa aagtagtaca
42961   gtgtctgaaa ggtagcattg gccatttgaa tctctgttaa cattttcttg ggtggccatc
43021   agatttttga ttaaaggtga ccttggcaat taagtggtgg ggggaacaca accaaaaggg
43081   acaaaccaac acaaccacac acaatcacac aacccctcca cctccaccac agtggaaatc
43141   aagcataggt gcttccaggg actgtgaaaa tgtggccaca gttgttttcc cagcaaacca
43201   ttttctttca caatatcccc aatttgcaca tccccccactg agctgaaatc agtataactc
43261   gacagacgaa agtcagcagg ggtaacctac ctttgatatt attttgttgt tccatcaata
43321   atttacttat ttctgaaaac caacagtctc tgatttcttt tgaagctgcc tgcaatcaca
43381   cataagaaaa cactataaga ctaccatgag tattaattgc agagttttta ttctcccagt
43441   gaccgttaga ctaatgagat agaaaacact tgtcaagtca tgggaacaca ataaggaaag
43501   agtagaggag acccatgggg aaggcatgag cgttacctgc aggatgtatt tctcaagtcc
43561   atttcgactg gcaatctcaa actttctatg gctccccctt ccaagctggc gaattgaaag
43621   tgtcatcagc tattataaag agggaagaga aatgttctat accatcgttt tctgatttta
43681   aaatagtccc tgtcaatcac tctaatcctt tctatccttg acagctctct tctttcatct
43741   tcctttcaag tttttgcttt cctctcttaa tgactccaag acagcataaa acacaatctg
43801   gtacagtgtc ttctgtctca tctgtttcag aaggtaacac agactgttaa ctttctactg
43861   tatctttatg caggaggcgc tgcagccctc aacctttcag tgaagagatc atttctcctt
43921   caacRtgtat tttacaactt tctacgtaat agtaactgaa taaggatcta ttcagaaaca
43981   taaataacaa ggccaggtat ggtggctcat gcctctgtaa tcagtcctgt aattccagca
44041   ctttgggagg cctaggtggg cggatcacct gaggtcagga gttcgagacc agcctgacca
44101   acatggtgaa accctgtctc tactaaaaat acaaaattag ctgggcatgc tggtgcgtgc
44161   ctgtaatccc agctacctgg gaggctgagg cagaattgct tgaacccggg aggtggaggt
44221   tgcagttagc caagatcatg ccattgcact ccagcctggg caacaagagc aaaactctgt
44281   cgaaagaaaa gaaagaaaga aagacaagaa agaaagacag aaagaaagaa aggaaggaag
44341   gaaggaagga aggaaggaag gaaggaagga aggaaggaag gaaggaaggg agggagggag
44401   ggaggaagga agggagggag gaaggaagga aggaagggga gaaagaaaac aaaggctact
```

# FIGURE 3-M

44461 atgaacaaat taacagcttt aatcacaaca gcataaatat atatgggtgt ggctcctgta
44521 tgtgggtgtg tgtgtttcat agcctgagag ctaactggct atgaaacagt ttctaaacag
44581 gtgagaaaac aatgcttggt gtgggcctgg gtgccttaga ccccaaggtc aggacaatgc
44641 ctgcacttag acacaaagat acaccgttga gaaggacaag tctaagatga aatccagccc
44701 atgttctcat cactaagtct ttcaactctc ttttacctct ttgcctggaa gaaagagggt
44761 acttttacta attagtttgt ccagactggc tgccatatag tctttttct ctagtacaaa
44821 ggcactccta tgccagccct ggcccataag ctctgaccct ggagtcagac agacccggct
44881 atggattcta gccctgtggt gtggtatttg gcaagttaac ttcacctctc tacactgcag
44941 cttcctcatc tgtaaaataa ggaaaacgat ctctctcttt tctgcatagg gcgactccca
45001 gatgcaatag gactcccttt accttcatgg Ycttcttgaa gctgtaatga ggagataatc
45061 cctggtcccc aggctccatt cgtatcttac agaacactat tcccctttca aataggtaga
45121 tttgcctctg gctgggttta aatcgaatca aatccttcat tttataacga tccttgtgaa
45181 ttgtccagac gctgaaaggg ccgtgcagca acagcttgcc tagttttcca atatcgtcct
45241 tttggaaaca cacatacagg aaaagaagct gtaaaattac ttgacagaga aagaagcctt
45301 tgtggcctgt tttcaaattt tataaagcat gcttctttga ggtttacaat tctaaaagtg
45361 ctattcaggc tggtctgtca ctaaatcaaa acgacaattt tttgagcttc actgagcgtg
45421 gtgccattta gttatataag gaaatcaatt cagctatgaa aggtaatgct atacataaag
45481 aatgttagaa atgcttgagc tagagatacg gggactgtga tatttttgac tcaattttaa
45541 ctagcagaat tcaatttcct ctgtgctatg tttgatacat gacagtctcc ttcaaaggga
45601 caagaaatta acatttcaat tatgtccagt agagatagaa ttttggctgc attgYggacc
45661 catgagcatc tgtgtgttcc atctgcatcc taacttctgc ttcaggtata tgaggacctg
45721 agaaaatgaa gcttgttctg aaaagagaaa gtttactgtg atctaatctt cacaacattg
45781 taatggaaat agtaactgca ttcaactctg agtttttcag agtgagtcaa cagtgccaag
45841 ataataccaa atgacagatt tttaagaagt ctgtttgact ctaggcataa actgtgatgc
45901 ccctgtctcc accagcaagg ctgcctttgt catatgtctg gttgaaagtc caaatgagat
45961 gaccctgcct tctctgagct ctcttcaaaa gacagtggac caagatgagc tgggagcgag
46021 gcattttatc tggtattttt caaaacttcg attgcttaaa gcagataaga attcatttcc
46081 ctgaataaaa acaccaaaag gaacaaatta tttgaagggg accaagacaa agaaatctcc
46141 ctaagtgacc tctagctgag atcaatccct atgatttctg agttaatact ggaaatatgt
46201 acttcattca taattgattg ggcattttta taatttattg tttatatgtt gggcaaactc
46261 taggtatctt ttttaaaaag gaattgactt tacttctgaa acattaaaag aaaaacttca
46321 agagcaataa actctagtca tttctatggt gtctatggcg ttatttccta tgttaatttt
46381 ctatttcttt ctttggaaaa tttacatatg tagactgtgg tgaataaaac cctcaaactc
46441 ccagccacga ggccttcttt cctaaaagcc aatgctcctg ttctgtgtta acagttcacc
46501 acattccaaa acagaagggt ggataggtcc atgaggaccc tggagaattt tttcactcta
46561 tctgcatcca tcaaggaccc actcaaatgt aatcactaca gaccttagtg actgcttttt
46621 cctttaaatc cattcaattc ataatcaata tcgacataaa tagtgcagga aatatgtttt
46681 agtaaaggca actggtagaa gtagatactt aatatttgcc acataaacaa atgttatcag
46741 cacaggttga gcatccataa tttaaaaaat ctgaaatcct ccaaaatctg gaactttctg
46801 agaaccagta tgacactgga ggtgaaaaat tccatacctg accacatgtg atgggtggca
46861 gtcaaaacac agtcaaaatt ttgcttcacg cacaaaatta tttttattt tttattttt
46921 tgagacggag tcttactctg ttgccaaggc tgcagtagag tggcacgacc ttggctcacc
46981 gcaacctctg tctcctgagt tcaagggact ctcctgcctc atcctcccga gtagcttgga
47041 ttacaggcgt gcattaccat gcccggctaa tttttgtatt tttagtagag ccagggtttc
47101 agcatgttgg ccaggctggt ctcgaactcc tgacctctgg tgatctgccc acctcagcct
47161 cccaaagtgc tgggattaca ggcataagcc actgctcccg gccttcatgc actaaattat
47221 ttaaaatatg taaaattacc ttcagtctat gtgtataagg tgtcatgaaa cataaatgaa
47281 tttcgtgttt acacttgggt tccatctgca acataactca gtacgtatgc aaatactcca
47341 atcctcccct aaaaaaaaaa aaactccaaa acactttttgg cctcaagcat tttggctaag
47401 ggatattcaa acttgtagtt tctatcttac aatgtattca ctctctacct cattttaaac
47461 attcgacgca tcttacagaa atatatacag tatagacaga tagaaaataa ccgagggaat
47521 taaggtgaag aaaaaataaa gaagttggga gcttggcgcc tgcctgtagt cgcaactact
47581 tgggaggctg aggcaggagg atcacttgag tccaggagtt tgaggccagc ctgggcaaca
47641 tagggagatc accatcccta aatataaaga caataaatca gatgaactgt tccagaaggt
47701 gtagaagata aaagaaaaga tgaagaggtt atatgtaaaa tgcatataat aaagccattt
47761 aaacttgatg taaacttggc tctgactttc taatagcaat acagagggaa acatggtgag
47821 tggtacgatt tattatgtct gaagacatac acaaaccggt tgttcaggaa aagcacagct
47881 attcctacta agacataaga aacatttctc ccagtgatat gttgtcactg agcaacatcc
47941 tcaaccacgc cctatgatag aaagaggaac atgttttagg taaagctgct tgtaagaaca
48001 catttggtca gtgtggaggc tcagctgtca aagtaattct agaagaggct aggatgatag
48061 gggaaggggg aataagtcga ggtgtacact ctctaatgac ttgtgaagat ggggtgacgt
48121 gagttaaggc atccacaagg ctattgtaga ggaaacagcc ccaaccagaa atgctgaaga

# FIGURE 3-N

```
48181   ggcagcctca ggcagcaagg tttgtgcttt attacctctg aactttcctc atcaccccat
48241   ccacagccat gctgctgcac cttcataggc aagtaatcag aggatgtgaa tccagagagg
48301   taccagcagc tgaccatagg gcccccagca taaaacaat gtgccagggK tggggggttgg
48361   caatgtgatc tctccctgaa ctgaagaaaa caggaggaag tggtgaccag caggagaYgg
48421   ggctgtgaga tgctttaggg gaaagcaggc agtggaataa acgagaagga aacagaacta
48481   tgaagaaggt gagtcattag aaattatgag gaagaggaag aaactgacgg aagggaaaaa
48541   aacagtcagt aggtggtaag agSgggggcg agcaggcatg tgggtgcact tgacatatac
48601   aggcgggtca cacaggtaag aaccccggct ccctccccag gcccaggccc cagaggtgct
48661   gtggatcctg ggtttgctca gttcccatct ccgcgaactt ggctttgcca tcgtgacttc
48721   ttgagaagct gcaggtcagc tttccagtaa gcccattatt actggaagtg actcgggggg
48781   agtcgctggg tctttcaagt ctggcagaaa catatgggca catatcctct gggtcatgtt
48841   gcacaaatgt tctcaggacc acacatatga cggtattgcc catctccacg cccaaggtcc
48901   gatctccagc gggtgagcac atgccctaca cggtgctgac caagggcagg cacacacact
48961   gttcactgtg ctgtgtggag ctgctccgcc tccaacagga agctgcaggg acattccctg
49021   agcaggagca agaatgacct tttccgtcac gtgtaccttt tctttccaac tagactgtag
49081   ttgatctcaa ggtcggagac cacgttgcac aacctctttc agatacgtgt tcaccagtat
49141   ttatttattt attggcattc cctagtattt atttatttat tggcatttag tggtgcttgg
49201   gagtagggga caggaaacaa aatactcatc actgtgaggg aatattgatt tatccaagga
49261   gttcattcag cgacgcccct gtactgggtt gaatagtgtt cccccaaaaa ctcatgtcta
49321   tgagaccctc aaaatatgat cttattcgga aagagggtct ttgcagatac aattggttaa
49381   gatggggttg ccctgggtta ggatgggtcc aaatccaatg actggtatcc ttataaaaaa
49441   agaaaacaaa gaaatggaga cgcacgctga gggaacagcc atgcaaaggc agaggcagaa
49501   attggagtgg cacagctgtc agccaaggaa ctccaagaat tgccggcagt caccagaagc
49561   tcagacgagg taaggaagga ttcttcctga gagccctcag agggaacgtg ggcctgctct
49621   caccttgcct ttggacgtct agccgtcaaa actgtgaggg aataaatttc tgttgtttta
49681   agtcacgtac tttgtggttc cttgcgacat cagccctagg aaacgaatat acttccaact
49741   gttttccacc agtaacaaag aagtgtggcc atgtcacacg gtgtgtggaa gaacctgatg
49801   gttgtgaact cagagatgga acatagcggc tacctcaaat gagcgagcga taagttagat
49861   ggcctcctct gtacaagaag tgatagcaaa atcaattagt aaatgctacc actttcacac
49921   ctgggcattc tgcaggtaag agccccgata caaggatttc aaatgtgact acattggcca
49981   aatgaagagt ctggctgaga gagtgaatct agggaagctg gcctcagcaa gagcttcttt
50041   tgggtgcccg tgtttccatt ccaggtagta agagttaata tgactgtcac aaacttactg
50101   cacaaagaag caggaaacat ctgtataaat gcgtccacac agagcctgag tccagaaatg
50161   aaaagcagaa gctgtgggag cctgtgatta caatagtcag gggcaaagag gtatcttcct
50221   ttccagcttg agcaggtccc catataacgt accactaggg ctgtttattc ggcatctgtt
50281   gggcgccagg cacagtgctt ggtgccttac ccatcattta attctcactt actcccggca
50341   accgcctgta atataggtat tgtggggggg tgggggggt ggggggtgg ggaatgaaga
50401   agatactttg cctgggtcac taggattatt tacttagccc tatctctgca gctaacactg
50461   tggtaaggaa agaggaaact gaaatagaaa taaaatcctg gccctagctc acaatgaact
50521   tacaatacaa gtacatcctt tagtggttta tataaatttg ttccaaaaag gaagactgtt
50581   tcattcaatc tttacaagtc aactttggaa cttatccctg gcaagtcaga atttaaatgc
50641   atctctaata tgcattggat ttgaaaaaaa aaaaaacttt tgtttttttt ttgcaaatag
50701   atagaagtaa attataactc agtgttccac aaaacaaact tgtttggaaa agaattttaa
50761   acttctgtga ttaaattaga cccaggacta attgagtaca cagaaaaaca agaaaatata
50821   aatatcagaa gggatttttt tctagttgct ttctagttgt ttaattgtga taatttaatt
50881   atcacattca atttctttcc tttataaaac caggttaata ttaaatttca agcagagaaa
50941   ctaagaggtt aaactggact cagccatgca tatagctgta atttattaca ggtgatcata
51001   aacacctgta ataaaactct acaaaatgga gacaaagaat ctcagaatca tttgagctga
51061   gttagaggca gttgtcagtc aattctaatc agaaacagat aaagagtgga atagcaccct
51121   gatagggaat gaacaagaaa tagtccaagg tgacagccat gtaatcttga gtgagttaac
51181   tcttctttcc atggatcttc ttttgaatca gaaggttgaa aattccaata tctactcctc
51241   cataattcaa caggattata caaaactgtc aggagaaatt agctgatgta ttagatgtaa
51301   aagtaagtta agtcttagta gaaaaggtac caaaaagcat ctggcagagg cacaattctg
51361   aggacacatg gtcaaaaaag gtcctttgct ttttgcgtgt aaaatctcca aattctgtaa
51421   gctggttcat cctatttgca tgcaaactgg ctgggaatga aattagggca gaatgttatt
51481   tgctcatgtt ttaaccattc ttttctcaca gcctcctcct cctatgttat ttacactgtt
51541   tcctgccctg tgtattgttt ccaggacatt cattagattc agggaaatga aatttaatag
51601   ggatgtctaa tacgtaattc aagatttaaa aaggaacaga aagatgccct ggattgacct
51661   aacaaattgt tcccttgact ttcctcaggc gaagaaagaa gaattattac caagaaaatg
51721   atccttatac acaccctaac cctactctgc agtttatgca tatcctcttg aatcatgcat
51781   cagttgtcat cagagacacc ttggagtccc aggggacagt aacagcatca tggctgggat
51841   cacagcaaac cccatggggc taagtttctg agatccaggc ccaagaaata ccagcgtggg
```

# FIGURE 3-O

```
51901  aagtaaaaat aactcatttt tcggacctta ataactttca tggtttgatt tatgacaagg
51961  aaagaaaagt catttcctct accaagacct caatacatgg tatgttcaaa accaaataaa
52021  aatgaaaaga aaaaaaaaga gaaggaaaaa gtataatata atcacaagtg acaaaaacgc
52081  agaagagggg gtcacagaga tgtgatgtaa gaaaaacttg gccgggcacg atggctctca
52141  cctgtaatcc cagcactttg ggaggcggag atgggtggat cacgaggtca ggagttcgag
52201  accagcctga ccaacatgga gaagccccgt ctctactaaa aatacaaaaa aaaaaaaaaa
52261  ttagcaaggt gtggtggcaa gtgcctgtag tcccagctac tcaggaggct gggacaggag
52321  aatcgcttga acctggcggg cagaggttgc agtgagctga gatcatgcca ttacactcca
52381  gcctgggcga cagagcaaga ctctgtctca aaaacaaaca aacaaaaaga agaagaaccc
52441  aacctgccat tgctggtttg tgctttatta cctctgctca tagaagccat gagccatgga
52501  gcgtgggtgg cctttttaag ctggaaaatt ccaggagaca gatgctagac tccaaaaagg
52561  aaaggaaaca ccttgatttt agcccatgag actgtgttga acttctgacc ttggaacttc
52621  aaaataataa taataatgaa tttgtgctgc tctaagccac caagcttatg gtaatttatt
52681  atgacagtga aataaaacaa acacatctga ataaaaatct agagtacttc aaagtaaaaa
52741  acaaaacaag tcgaatatta actatgctcc agtctcaaag agatcataca gttatagggg
52801  atgttttgcc tggaatgcct gggaccagcc tgctccggat taacactgac gctggagctc
52861  aactccccag cattggcctc taactgcatc tcttgggttt tctttagact atttggatta
52921  atgcatgtac actatatact tttggggcct cttctgggat ctgtgatata aaattggggc
52981  aagaatatag gacctgtttc caaaatactt ttcttagaga aactctgaaa caagaagaca
53041  aatcttttac tattttaata gtgccatgag ttagaaaata aattgctcct ccaggacttt
53101  tcctctgccc atatatgttc tggcccttga ctttctggtt tgtccagaca gtgggtcagt
53161  cccacattca gaaccctgag gaataaatYc aagattcctg aagatgccaa gggacagctt
53221  ggaatcttgt agtgcgggtt tactgagcaa tttaggcgac tgtcctcaga gtccaattcc
53281  tttaccatag acgatctctc tgactttgcc agagcatttc ctcactgtgt agtattgcac
53341  ctgccttttg tgtgagatgc tctaacacta ctatggaaca taccggacat tcagtcactg
53401  ctgctaggtc cacagccaac tcacaggact tgatcaaatc ctctatcact gccaaagctt
53461  gttgtagttc agttgagaat gctgaatctt tggttctctt tggcccatcc tgtggaaaac
53521  aaatgccttg ttggaaatca aaagtatacc atctgcaaag tgaagaagga aaacaggaca
53581  gagatgagga gccagggatc cagccaaccc tgactgagaa gggtggatat tgatctgtct
53641  ccctgctccc aaagcatggt ggctaaagat gcttactgat ttaggtatac ggcatagaga
53701  caagcaaaat catctacctc tgtgcattgt actagtgacg aatgaagttc ataaatcatg
53761  gaaattatat tcctgctaca taagaaacat atagaattca gctatatagc tgaattctat
53821  atgtttctta tgtgtatata tattatataa atatttatat agtatattaa atatttatat
53881  attaatatat aattatatgt ttatatatta attatatata ttaaatattt atatattata
53941  tattatataa aatataagta tatatattat atatatatat atatatatat atatattttt
54001  ttttttttt ttttttttt ttttttttt tgagagcgag agagagtttc actctgtcac
54061  ccaggctgga gtgcagaggc acgatctcgg ctcgctgcaa cctctgcctc ctggattcaa
54121  gcaattctcc tgtctcagcc tcccgagtag ctgggattac aggtgtgcgc caccatgcct
54181  ggctaatttt tgtattttta gtagagacgg agtttcgcca tgttgaccag gctgttctca
54241  aactcctaac ctcaggtgat ccacccacct tggcctccta aagtgctggg attacaggcg
54301  taagccactg tgcctggcca gaatttagct tttttaaagc atggaaaaac ctagctcctt
54361  ttaaataggc ttctctctct tttatacccc tattccctgg ggcttactga aaaaaaaaat
54421  accatatggc cgggcgggtg gctcatgcct gtaatcccag cactttggga ggctgagaca
54481  ggcagagcac ttgaggtcag gagtttgagc ccagcctggc taacatggtg aaaccccgtc
54541  tctactaaaa atacaaaaat tagcttagtg tggtggtggg cgcctgtaat cccagctact
54601  tgggaggttg aggcaggaga acagcttgaa cccaggaggc ggaggttgca gtgagcccag
54661  attgtgtcat tgcactccag cctgggtaac aagagtgaaa ccccgtctca aaaaaaaaaa
54721  aaaaaaaaaa aaaaaaagta gctgggtgtg gtggtgtgtg cctgtaatcc cagctactca
54781  ggaggctgag gcaggaggat tgcttgaaca cgggaggtgg agtttgcagt gagccaagat
54841  tgcgccactg cactccagcc tgggcaacaa agtgagacct tgtctcacaa aaaaagaaaa
54901  aagaaaaaga aaaagaatac aatctgagta tcatattcct atatttaatg tggaagcctt
54961  attcctgtgg gaaaaattat acttaaaatc atcccagagg aggacatttg taaactccta
55021  tagagacaaa gaactccata gaggctgcta agtggaaatt tactaatgat ttacatgtaa
55081  aagctataac atcatatttc cacactgaat ctcccccaac tctgtccttc cttcctccac
55141  ttgtccctgg ccctccccac attgcaccac cattaaagat gccaaagaga taagccagcg
55201  ctctgcacct cccgaacata aagactcagc attcagcgga aaagcagtaa ctaattaaag
55261  agaccaatgt tccaattaca accacagtga catttaggat gtgattgggg tgattgtttc
55321  agctctaaag gcttttgcat gggcttgagg tattttatct ccctgctacc tacatgctgt
55381  atttatctgt tacctggtaa atacacataa aaaaattttg gtttattcaa tgtatttttt
55441  taacatctca agttctgcag tgaagaacag ctagcccctt tgctgctccg catctggccc
55501  tgactctttt tgtcctctac agcacaatgt agtcacaggg tttaatattt tcttaatcta
55561  tgcggaagca ctgggtattt gcatctttgg atgagagaca tgagtgacag ggctgatgaa
```

# FIGURE 3-P

55621 tggaatagat cgtgctgctg cctctgaaaa acgtatcatg gattcgtagc ttctcattca
55681 taataaagaa gcttcgcttc ctggtaaaag aaaacagact ctcacctgaa tttattttaa
55741 caacatcaat aagggattaa gagttcttgg cagggcgcag tggctcacgc ctgtaatccc
55801 agcactttgg gaggccgagg gggggagtgg atcacgaggt cacgagatcg agaccagcct
55861 gaccaacgtg gtgaaacccc atctctacta aaacaaaaa ttagccgggc gtggtggcgg
55921 gtgcctgtaa tcccagctac tcaggaggct gaggcaggat aatagcttga acccgggagg
55981 cggagttgca gtgagccgaa actgcgccat tgcactccag tctgggtgac agagcgagac
56041 tctgtctcaa aaaaataaaa aaataagaaa taattttttt ttaaagttct tataccaggc
56101 atcttcctcc ttaccaaaca ctgaatgctc agtttccttt cagatcctca cctcttccca
56161 tccccccttcc ttccgctaac cctccttcac aaaacacgag gtggctaggg ttttgaactg
56221 tataatgtag ggttcagaaa aatcctttaa aacattatta aactcctcta actagggcta
56281 gctcctcttg ctccaggctg taaaaatatg acctgtgtcc tgagctgctt ctgttttcaa
56341 caggtccttg tcatccattc tgagcagaaa gggcatgcaa atgactgccc cataagacat
56401 gtctcaagtg tttcttgcta aaaccagaat attctatagg aaagggaaga gaaaccgcac
56461 tgctataMca cagatttctt cctaacctgg tgtggtcatg gtcaccattt attctaaggg
56521 aactttggca gactcttaga gttcacacac acgcacacac acacacagag gagaacaagg
56581 cataacatat agaaattaga tatactaaga ggtacaaaga agaaaacata atcctaccat
56641 tcaggaaagc cacagcagac attttatcat atctatttac ttccagtctt ttatgtatgc
56701 attttacgta tttgcttaat tttgttccca ttaaaatttt tttggctggg tgcagtggct
56761 cacgcctgta ataccagcac tttgggaggc cgaggcaggt ggatcatctg aggtcaggag
56821 ttcaagacca gcctggccaa catggcgaaa ccccgtctct actaaaaata aaaaaattag
56881 ctgtgcatgg tggcgggcac ctgtaatccc agctacttgg gaggctgagg caggagaatc
56941 gcttgaaccc gggaggcaga ggttgcagtg agccaagatt gcaccattgc actccagcct
57001 gtgcaatgga gtgagacttt gtctaaaaaa aaaaaaatta tgtggaagga agaaaatata
57061. ttaccacttc catttgggct gcaaatccct actttaaaat tgcctttaat tttttttgttt
57121 tttttcttga ctgtgagtta taggatacat actattttag aaaatctaga taataaagac
57181 caccaagtaa aattgctata aatcctccga cccatagaca aacaccatta atagttagtt
57241 atataaaatg taatatttaa tattgagtac cttagccgag cctcctagtt cacctgggtc
57301 tatctccata tcttcaggag actcgaaatc cagcagaccc tgcattaacc caaaagtctt
57361 gttgaaaatt aaaatgtaca ttgccatcaa aatgtagatt gtcaacaaaa tctactgtgc
57421 aaactatctg ccaccaatgc taagctgact gagaacgtgt gtaaatgtgt aagagggaaa
57481 gaataaatga tgtatttggc acagggtcct ttccacaaac ctagaatcat attgtacaca
57541 ctatttgta gactactttt tctcattcaa ccatacctca tgaatatttt cccatgatat
57601 tatgttttc taaaatatgg gctttgattg tggtagagtg ttatatctta cagataaacc
57661 atttttctca ttcagcaata tctcatgaat attttcccat gatattatgt ttttctaaaa
57721 tatgggcttt gattgtggta gagtgttata tcttagagat aaaccatttt tctcattcaa
57781 caatatctca tgaatatttt cccatgatat tatatttttt taaaacatgg gctttggttg
57841 tggtagagtg ttatatctta tagataaacc ataatcgaat tatagtcccc cttatcatta
57901 aacatttagg ttcaacaata ttttttacta taataatgat aataactttt gtgcattttt
57961 cttaaagcta aattcctaga gtggaattgt aaatgttaag agttttgagg catatttcca
58021 agttcttcag agaaggactg tcttcctgcc agctgtatag gaaagcctgt ctcaccagcc
58081 cctagcctac aaggggtagt accatttaat caaaaatctt taccaattga atgaaaagag
58141 aaatcctacc tcactgttgt tttaatttga cttccttaga taactagagg tgtagaactt
58201 ttttaaaaaa acgtttgcta ctagtgttta ttcttttgca aattgctatg tcctttgcgc
58261 atctttttat tataatttcc aagagcacat gattcattaa gcatactgat catctgttac
58321 atattttgtt cacattttcc ccaatgtttc atgagtctgt ctttaatggt ataagctatc
58381 actcatcacc ctctccccaa gatcccatga tcctttctaa agcatgaggc aatcagtcca
58441 acattcatgc tctttcaagc cagcacatgt gtgcgagatg caaaataagc tctgcccctg
58501 gggatagaga aggtcctaga taggattaca aggtcgttcc tttctttgca ggcacgtaat
58561 ggcctgagct ggtttcacag gcaccagcaa gcttagctgt ggggacactg ctctggtctg
58621 ccttgggtag ctcccacggc tcctcacaga cccccgcaa aatattttgt agtaattcta
58681 tctcttgttt cttcagtgaa aggcagctaa caaaaccttc taagtccttt taacctgtat
58741 tatctcattt aatgctccca acaagcatac caggaaagta ctattattat cttatttcta
58801 taggtgagaa atctgaggct gagagaggtt aagtaacttg cctgagttca cgcagcctgg
58861 aagggtcaga gcctggattc agacccaggt tgttaaactc tcgagtctgt gtttccaatg
58921 acgatgcccc acagccctct gcgtctggta ccgaacctag ctcctatgta aatgtcacct
58981 ctgtgggaaa gctggaacct aggctgaggg aggaaggacc atcactttcg tcctgctcat
59041 gcctcactgg gcccagaggt tggacttcta caataggaat gacagtgaca atgggacatg
59101 cagaggagca tgaggccctg agtgagtgct gggtcaaaca tgctctgagg tgctgctcgc
59161 tgaaagaagc agatcattat cctcatttct atggtggtgt gacagtctca atgtctaaga
59221 taacagctac acatcatgca gagcttcctg ggctgtgcca agccctccgc atgtgctaat
59281 catctgcttc ataacgatct tatggaggag gttctagtac cctcatttta catagaggaa

# FIGURE 3-Q

```
59341   aactaaggcc caagaggKca aggtctcata actgacaaat ggcagagtac accgaggctg
59401   aaactgctaa tggaaagctt gaaaggagta actttgaaaa aatgttttta aaattatttt
59461   tacagatgag gtcttgctat gttgcccagg ctagtcttga actcctggac tgaagggatg
59521   cctcctgcct ccacctcctg agtagctgct gggactacag gtgtgtgtta tcatgtctgg
59581   cctgaaaggg gtaacttttt ctgaggggag actgttgggc aatgagctta accttcctga
59641   gcctcagttt cttcacagtc cagagcatgg ctggtatgtc tgattgtgga gattaaataa
59701   gaaatactgt aatcccagca ctttgggagg ctgaggtggg cagatcacga ggtcaggagt
59761   tcaagaccag cctggccaac atggtgaaac cccgtctcta ctaaaaatac aaaaattagc
59821   caggcatggc ggcgcgtgcc tgtaatctca gctacttggg aggctgaggc aggagaattg
59881   cttgaacctg ggaggcagag gttgcagtga actgagatcg cgccattgca ctccagccta
59941   ggcagagtct cactctgtct caaaaaaaaa gaaagaaaga aagaaaaaag aaatacacgt
60001   ggggaagtac ccagtaaaca gtaggtgctt gtttttgttt ttgtttttgt tttttattaa
60061   gaaatagtgg ccactgggca agagctacct aaatgcctta tagaggttaa ttcaacagac
60121   aacttatcgt ggtggcccaa acctttctct caagctcaca cctagaaaac cagccatgat
60181   aggccctcat atcctgtttg gagtgtctga gaatgcttgc ctgcagatgt gctagtgtca
60241   atgacttatc actaaggagg ggtaaaggtc agagagagag agagaaaacc acttaggggt
60301   tcagggttca gatatagcaa agttcattct gttaggctgc tctcagggta agatcctaaa
60361   tctaagaatg gaggtcagtg ctgcaaaagg atagaagact tcagttttgt cttctccctt
60421   ccacaagtta aaatgcccat aaaacagtaa ctttggggaa aatcacactc tcgctcccaa
60481   agagctctct tcccctaagc cagactcctt agtgattcat gccctgagct agacattgga
60541   ctagtgaggc cctcaggtgc cctccaactc tatgattctt tggtcttact ccacattgaa
60601   gagggctgat ttagagttgg aggaaagaaa aagcctgcag gatagacgta tcttcagcct
60661   tagaagacca aggcacagct ctacatgtgt aacgacatgg gcaccacagt gcaggcgtct
60721   ggtttcccac caaggaaaag cactgtctca ttgattttac cttcaacagc atctggtatt
60781   ttataagtct ctggcttggt cctttgagat acttaaaaag agggagattg tgatccagtt
60841   ggcgctggca taccttttaaa agccaaatag aaacaacata aagcaaaaca aacaacacac
60901   acagaataaa agtggcagca tttaggtaca aaacacaaaa ttgctttttcc tggacaacag
60961   gggcagctgt gtcgagcagc tccataaggc tgtggggttg ttctttcccc taggaacagt
61021   gtgtgaaata agagaaagag gaaagaggac ggctcggcta atatttttac aggcatctag
61081   ggaagcagaa ccatttattt cttttagaaa gcaagttcac tagaagacag tggggcattt
61141   caagtccttc tgaaaaagag gccacctatc ttcaagttgg gggctgccag agtctattca
61201   ggccttttgg agcctgaagg atacagacgg tttgtagaca tcattccagg gagcccaaag
61261   tgtgacattt tttacttagt ttcattttta aaacccatct tagaagaaga cactggtttt
61321   ctcaccagat ataggtgctc ctcacaggcc tatcccatta caacttttca tgtgtaattt
61381   caaagtatgg gacactttaa ataaacataa atcgcaatgc caagttcctc tccctagtcc
61441   cacttttgat ttctttaaca acttgatgca tagaaacatt ttccaggaag aagtttgtct
61501   tctcaacatt accccaaagt aggcgcagtc ttgacactct tgccagattg ccctagctcg
61561   gggcagattc ttatggtatt taaaatatat ctgaagatct tctttctagg tgggaaaaaa
61621   ttagaataaa ttaatcaggc agcatttttc agtaagtgga caggatgata cagtttattc
61681   attcattcaa caagccagag tattctaatg tgtaaggaat tgggggtgta cagtaagtaa
61741   tcttatgttg ggatgtgtgt gtgatggaga gataaacaga aggctttaaa caaccataac
61801   taccagacat agataggcac agagtgctct gggagcatct acaagggcta ttggtaaata
61861   cataagtaga ctcacaaaca ggaattggtt tttatcttta tcattctttt cctgcccttt
61921   gtccaggaca tcgacatctc cccacaacag gtctgcaatg agtagtaaaa ctgacagaaa
61981   agaggctggt gtttgatggt ccccctctct tctctctcca ccctctcctt gtggtcatcg
62041   tgacaccaac cctgcaaaga agcccaagaa acaaccaaaa gatggaaaca tgccagaggg
62101   gagatgtggt agatcgggca accaatacgg agctcagtca tttgggcaac cctcaaagtg
62161   acaggctccg cctcaaacat cactttcaac ctgcagcaca agactctgag acaggaaatg
62221   ttatgtgact gtttcggagc tagagagcaa ggaggactca acgtgctgtt caacctgtat
62281   tctcatggta tcaaagacgc tgagaggtgg gcacatatct gccaatctcc cggagagaaa
62341   gcacactcca agcctctggc gtcagcttgc cagcttcact cgctctatca gggagcctgg
62401   cccacagcgg ctgtgctggc cacagatccc atgccattct ggtggagaag cccatgtagc
62461   cacctccttg cagaagtctg tgtccctgtc ctcctgctca gcccaactct ggatttcctt
62521   catgtcaaca cagatgattc cttccctctc agcttcaatg atgcctgttc tactcccaaa
62581   tacattgcta tagtggatga cttccatttc tgtagctctt tctttctccc aataggttgt
62641   aagtggtcat ttcagcatag gaactacagc aaccgtcctt tgctattccc tccaggaaaa
62701   aaatggcaca cacatttaag acagatggcc cttgaaaaat gttcagactg gttaaggagc
62761   tagaataata actcagagct taacaatata gtattgacct aaattgctct tctaaaggct
62821   aacagtccaa aattcctcag agaaaggatg tccactaacc agcaacatac aaaagtgccc
62881   aattctccta atctccttat agaatgaatc aggaggctat aaaattgcta ccagaaactc
62941   atattcacac aagaatacat acttgcttac ggattaagac tgcggggctc tggtggcgga
63001   tggtccaggt tcagttcttg gctttgccaa atacaaatca tatggcctaa agtaagttac
```

# FIGURE 3-R

```
63061   ctaaccttcc tagacttcag tttcctcatc tgttaagtgg gcccataaca gtagtcaact
63121   cctgtgactg ttgtgagatt aaatgagatg atgtatagaa acctctccac aaatgcccaa
63181   tggatagagt actcaaaaaa tggtggctat tgtcaaagca cttttactt tatggaatcc
63241   tctgcataca ttattttgtt tcccacaaca actgtacata ttggtactat tagcctgttg
63301   tgtggaggag gaaagtgaga ttccaaagtt taagttattt actggaggtc atgcagatag
63361   caagtggctt gaccaacatg ataaatcggg tttcctgact taccagttac tcccacatat
63421   cagtgtgagc acccattcca gtgctcttto cattttacca tgtgagatct cagagtactt
63481   ggaacccaca gtacactatg gacaaatatt attctatata gaacatggtc tctggaggag
63541   aattgtttca gaagatacag gctaaagagg ctgattaaaa cacttggtca gaaaactatt
63601   tcaaaattca ttatcttgga aggccgaggt gggcggatca cgaggtcagg agatggagac
63661   catcctggcc aacatgatga aacctcgtct ctactaaaaa tacaaaaatt agccgggcgt
63721   ggtggcacat gcctgtaatc ccagctacga gggagggtga Rgcaggagaa tcgcttgaat
63781   cagggagtca gaggttgcag tgagccgaga tcacgccaca gcactccagc ctggtgacag
63841   agcgagactc cgtctcaaga aaaaaaaaaa aagtactact tgtattttgt ctctaacatc
63901   ataaatcaca tagggctaag tcagtgtttt ctggctgggt taatgattta caatgttcct
63961   cccaacatgg cggggcgcta tcaaaaaact ttgtaaaact ttacaattta ggagaggaat
64021   tcagaaaagg tatttagtta ataattctct gacaatttcc tcatatttga acaatttgat
64081   aatatccact ttcccacagg aactttgccc atttctcatt gagcaattta aataatcttt
64141   gcagtaatga catacataac cataaaatac ttctaatctc tgaaactaga tttcacatta
64201   gtcgtttgtt aagtgcaaaa attcaaaata gtatataaag cactaataat gtaatagttc
64261   aaaaaaatta atgaatcaga atgagtataa aatagtttc agtgtacatt gaattctggt
64321   gctttgacac aaagcctatg atgagttgac atggaaagcc atatggggcc accaaaaggc
64381   tctgagcatg gaagattcct tctctccatt aatgtcaaat atcttgaaac ctggaggctt
64441   tgtttgcttc cagttatcat aagtattgct ctcagcctca gtcataatat gagttgctta
64501   caactttaag ttgtttgatc tgttgccttg ggttcacaaa aataaaaata agaaagagaa
64561   tgagaaggac tagttgagaa agagaggaga aatgagaact atttagaagg cacaggggt
64621   caggatcatc tccatggtcg tgtcctagaa agtacctggt ggggattgat gttgggtgga
64681   acatttctct gtggcagctc ccactggccc atatagccta tttgttgcct aaaacagggt
64741   ggagaaacag ggaaagaggg aagaagtctg ggaggtggga agaaggggta gttggaggta
64801   taatgttgta ggccaagatg ttcataaatt gagtactaag cctaaaggag gctgtagatt
64861   tagaatttc gaatcatcct ctatgttgta aaaaatagaa tggaaagaag atgtatttcc
64921   tgaggttttt agtttaacac cagtgcttct cctcttgaga gcattagtac agctttattg
64981   tgggactcta acctcgctgt atgattcttg ttttaaaacg tagttgagtc attcagaaga
65041   ggatcctctg ctttggggac catggatggc cagccttcca tttcatgggg ctttgcagga
65101   agccaggctt gcggttactc ccacatatca gtgtgagctc agtcaaattg gactcattct
65161   caccttggat aaagtcagat aaagttggga ggccaactca tgttttccat tcaaagggca
65221   gatacaagat atactgtctt gacaaattgc aagtgtacag tacagtatta ttccatgata
65281   gtcgccatgc tgtgcgttac atctccagga cttactcatc tcataactgc aagtttgtac
65341   cctgtgacca acatctccct acttcctcta gactccagcc cctggcaacc accccttctac
65401   ttcaatgagc tcaactgttt ttggattata tatatataca tatacacaca cacacacaca
65461   caatggtaat gaatgtattc attaacttga ttgcagtaat catttcacag ggtgtaagta
65521   tatcaaacca tcatgttgta tagtttgaat atatgcaatt tttatttacc aattatactt
65581   cagtaaagct tggagtcgga gggaagcaga tatgttttta gaccattgat gtttccattt
65641   gtccttgcca cttaacgcag aaatgtaagg gctgacttta acatgattat ttactgggag
65701   tacagactta ctctcttgag aaagcaatgt gccagaagtt cagggttctc agcacacttt
65761   tccaactctt ttagaaaagt cctagaaaaa taaaagtata caagtaataa tgttttgatt
65821   ttgacatgta ggtaattaat tttttaagta caatcacata aggtttcaag aggtgcctag
65881   aaaaatgttc tccaggcctt cttgctctta tcttctacac attgattcag Raaaaaaagc
65941   tgtattctga ggtattacta caattaccct gaaatcactc acaactttaa gttgttcgat
66001   ctgttgcctt aggttcacaa aaagaaaaat aagaaagaga atgagaagaa ctagttggga
66061   aagagaggag aaatgagaac tactcagaag gcacaggggg tcaggatcat ctccaattat
66121   cagaacacag ctgttcgggt aattctaagc actagtcata gatagactgg atacattcac
66181   tgatgctctc tcttacatga aagtaacgtt cataagaact gctgctgctg ctgttgctat
66241   tactaatatc attgacagct tactataagc caggcataag ctaagtgctc cgcaaatagt
66301   atctcattta atcctcaacc aaagataggg ttttataaat ataaaacctg gggctcaaag
66361   aggctacata acttctctgg ggaaatcaac agaaatgggt ttccatttca gttttgcgtc
66421   tgaccaacgg acgaggaatg gggttaggaa gcaactgtgg ttcaattcac caagcagctt
66481   ttctccctct gtgaattagg tgtgcaatct tggggtcatc atagcgaata aagagagtta
66541   acaagttacc attcccatat gtgtaacatg aaagggtttg aagatgattt ccaaagtccc
66601   ttattcctgc aattctctag aactcaatat ccaagcagcc ccgagttaaa agtacaactt
66661   gtttgagcag tcagcatttt ctaacaccct agttcccagc atccccttat gatagtagta
66721   tggagatgtg tatcttggtc atctttgtat tcccaatgct gagcacagtg cctggcacag
```

# FIGURE 3-S

66781 aacaggtgct caaaaaatgc tgatgcatga ataaataaac aaaggaacac tcaactgcat
66841 acaacatgga tgtctgacac tgggtccctg ttcctttgat gtctcttccc tttctctcca
66901 gggatcctgt ggggcttctc ccctcctttc actccaaccc accccaatcc aatccactga
66961 ctccaaacca cttgcttaaa cctgaaaaca tctaagtgtt ttcatctctc aattccatca
67021 gtctcatttc caccctt ctS ttactctcca ttctttctga taacatgaat tattatatac
67081 ctgttgtgaa attcgtaaag ttctctaata ttcccaaaga gaaagtcctt gttattctga
67141 agaacatctg gaattagatg ctttagccaa ataaaatcca ttggagtgat atatccctgc
67201 agaggtgcaa acaaggtagg tgttacaaac aggaacatac cagagatcat ctgaattagc
67261 tactgcaaaa cccaaaactt tagtgaaatg attgaagaat atagcacact tgtttcttga
67321 aagctatcgt attgagtact ttcttttgat atcattttcc tcatgtgccc actttcagaa
67381 ggagatggct taatggcaat gatcataaaa tggcattttt gtgggaagaa caaatatatt
67441 taaaaatgtt tatgttacac aggtttaatc taaaagaagg gaaaatgtac ccactgtttt
67501 atgaactaat acaattacta ttattattat gattattatt aatattgaga cgaagttttg
67561 ctcttcttcc caggctggag tgcaatggcg tgatctcggc tcactgcaac ctccgcctcc
67621 tgggttcaag tgattcttcc gtttcagact cccaagtagc tggaattaca ggcacacacc
67681 accacacctg gctaattttc gtatttttag tagagacagg gtttcaccat gttggccagg
67741 ctggtctcaa acttctgacc tcaggcgatc cacccgcctc ggcctcccaa aatgctggga
67801 ttacaggcgt gagccactgc atccggccca ataattattt tttaatatct gaataatgtt
67861 tactcagcat atatatcata gtttactaaa cattcccttt ttatggacat ttgggttgct
67921 tctaatattt tatggtaaaa atgtgattat aaataatcgc gtacatataa ttttttcctt
67981 aagattattc ccttaggaaa atagggaaaa catgcatttt tactcttaag aaaaagacta
68041 ctttaaaaaa acaagaaata atcttccctt attaaagtat caggaacact tataatatcc
68101 tttgttggca agaagatggt aaacctattt gggctttgct ggaatcagtg tgaactggct
68161 taattctttg gaaactattt ggctacatgg atcaagaaac cacagggatg ttcttattcc
68221 tactggagac acttgtaata ttctaagacc tgaaaccttg gtgggaattc tggagacttc
68281 tggcaactat tttgagtctc tttgtacaac aaaacatctg cagtttgaat atatgcttga
68341 taccccactc cccaaatcta cttacatcaa ttatgctttt aatctctttt atgtaaatct
68401 cttcagtctc aagcaagtca cgtataatgc gcctgaatca cagcagcagg tgggagggtg
68461 aaagagagag agacagggag aggagaatgt tcttttagaa ttctgttaga ataatgctca
68521 tcaatacagt tccctttttag tggctcacct acttagtttc tggtcagttc attctgttct
68581 attgaacacc caaggtcagc atctcacaaa cgcacactgt gatcacactg gtatcaagaa
68641 gaaacagcaa ggttagagaa ctcaaaatcc tttaggcagc cagtgaatga cctgtcctct
68701 gggtgggcca tatagtgtgg gggtcaagaa gggagatgtt ggagtcagaa ataccaggtc
68761 tggatcctta gtcaaggaag gtctgcctga ccccaccccc acccccctata ttgtatacat
68821 tgtataagtg gtttcctaga ttttctctcc agctttctgc tcacatcaag ctttctttttc
68881 cttttaagag aaagggtctt gccctgttgc ccaggaatga gtgcagtggc atgatcatgg
68941 ctcactgtag ccttgaactc ccgggctcca gcaaccctcc tgcctcagcc tcccaagtag
69001 ctagggctac agatatgcac catcacaccc agctaacgcc tttttttttt tttggtagag
69061 atgaggtctt gctatgttac ccaggctggt ctcaaactgc tactttcaag caatcctcct
69121 gcctttgcct cccaaactgc tgggattata ggtgtgagac actgtgccag gccagatcca
69181 gatctttgac ccatatggat gtattttgtt gtacatgggg ttagatgtat gctagctcct
69241 tcctctggca ttggatgttt cactgtgatt tcctaaggca aaagatatga cttctgtctg
69301 cttctgtcaa ggaatgcagg tggaagatgt ggtggacaga atttaagacg gtccccaaga
69361 cttctggccc cactgcacat acgcctcttc caatcaaaca ctaatttagg ggatgctgtg
69421 gaaggatttt gctgttcatt tgataggtaa tccagttggg cctgtcctaa tcatatgaac
69481 tcagatctat Mtgggtcaag tggtcagaga ctggaagcat aaaaaagatt caacacaaag
69541 gagattcccc tttgctgact tttgagatgg agggggccag atggaatgga atgtgggcag
69601 ccgtaggaac tgagagtggc tcccagctga caggcagcaa ggaagggaaa tcagtcctat
69661 agttgcaagg aaccgagtcc tgccacaacc acgcgaactt agaagaggat ccagagctcc
69721 acatgaaaac acagccagcc aacaccttga ctttagcctt gtgggactcc tgtcccaggg
69781 aaactgtagg tctcctaagt ttgtggtcag tttttatata gcaatagaaa accagtagaa
69841 agagtaaggc cagtccccac atctatccca gacagacttt ctttctctcc tcaggctacg
69901 tggccatatt tattatttct tttaggagtc caagtagatg agtgtttata catgtgtcct
69961 tgtgtagaat atatatattt attcttgtta actattgatg gattatctat taaatggcag
70021 cctctcctag aaaatgattt gtttttctct cataaaaatg gatgtgacaa atgatccaat
70081 agaaaaaaat gggaaaggac acaaaatagg agattcacac aaatattcaa atggatatga
70141 gactcgtgga aaaaatactc agtacataca ttcaacgaag gcatatttta aaagagcaac
70201 aattttcatc tattagaata ttaaacattt caaacctcat agaaagactg gtaaatgagg
70261 gtgtggaaaa accctcacac aatattggta aaatgtgtaa atttgtgcaa gctctttgga
70321 aggtaattta gaaacatctc acccaaatgt aaaacacgtg ctccttggtt cagtaatttc
70381 acttctagga atgttcattg tttataatta ggacaatata acttcaacaa caaccagata
70441 taaactggtt acttaaatgc tcatcaaaaa attatggtac atgggccggg catggtggtt

# FIGURE 3-T

```
70501   cacccctgta atcccagcac tctgggaggc tgaggcaggt ggatcacttg aggccaggaa
70561   atcgagacca gcctggccaa catggtgaaa ccctgtgtct actaaaaata caaaaaatta
70621   gatgggtgtg gtagcgcatg cttgtaatcc cagctacttg ggagggtgac gcaggataat
70681   tactcgaacc caggaggtgg agattgcagt gagccgagat cacaccattg cactccagcc
70741   tgggcaacaa gagtgaaact ccatctcaaa aaaaaaatat atatatacat acatatagat
70801   atagatagat atatgtgtgt gtgtgtgtat atatatatac atatatatgg tacatacata
70861   taatgatgaa ataccatgca attgttaaaa agaatgaggc tgactaaaac actgatatga
70921   cagaggccag gtatttttag tgaaaaaaca aaacaagacg tagacaagta agcatagtat
70981   gactatttgt gtaaaaaatg aatgtgtatg tagaaatata cgtaaaactg tatatgctca
71041   gaaaatttat gcaaagaaac ttaacacatt gttaaaagtg attgcttttg gagagtggta
71101   ctgaaagcta aagtatggga aaggagaatt tctacatctt actctatacc cttctacaat
71161   ctttttaaa gtgaaacatt tactgctttt ataatggaaa aatagggttt acataatatt
71221   ttaaaatgaa tgttactgga ggtaatgata tgtaaaccct atttgataga tataaataaa
71281   taatacaatt cacatatttt acagctcatt attctttctt ataatcattt ttgtgtctat
71341   ttataaatac ttgtataaga caagactgat aaacaagatg taccacagag gatgtaattt
71401   ccggaagtct aaaactccca aaaagttact taaacatcat actttactaa gtcactgaga
71461   aagaggttcc tatgcctttt ataaatcacc cggaaacaac aacggtttct atgtcagaag
71521   gaagtacagg cctcaatacc aatatgtttc tacgagccct ggcatcaagt gggaccacag
71581   caacagttaa gcattcatgg aaaacctgag tacaatcagc ctcttatgcc ttcatgaagc
71641   actggggctta agcaacttgg caagctggct tcaggatgca ttgacaatta aaggaagtca
71701   gaaggcagaa ataaccagga ggtggacaga agtcaaggtt attatccaaa aatgatctat
71761   gaacttaaga gactcttagg agcttttagg actctaatac aggaaaatat tcacatctct
71821   gaaggaaaag aaatcccttg gtcatattct ggattacagt ttggaaaaaa agtctagaaa
71881   tctcatccaa ctgcttcatt ttacctgttg gagtgcaaga gataaaatgc caaggatagg
71941   aactccactg aagaatgtag gcaaatcaat attcattaaa tacattcaaa ttaatatatg
72001   acttaaaaag agccctcaca tcagtgttag gccattgctt ttccttgctt ctcttgtttt
72061   cctttcaaat acctttataa aataggagaa aatgtatgac tctttttttt tttttttttt
72121   ttttattata ctctaagttt tagggtacat gtgcacattg tgcaggttag ttacatatgt
72181   atacatgtgc catgctggtg cgctgcaccc actaatgtgt catctagcat taggtatatc
72241   tcccaatgct atccctcccc tctcccccga ccccaccaca gtccccagag tgtgatattc
72301   cccttcctgt gtccatgtga tctcattgtt caattcccac ctatgagtga gaatatgcgg
72361   tgtttggttt tttgttcttg cgatagttta ctgagaatga tggtttccaa tttcatccat
72421   gtccctacaa aggatatgaa ctcatcattt tttatggctg catagtattc catggtgtat
72481   atgtgccaca ttttcttaat ccagtctatc attgttggac atttgggttg gttccaagtc
72541   tttgctattg tgaatagtgc cacaataaac atacgtgtgc atgtgtcttt atagcagcat
72601   gatttataat cctttgggta tatacccagt aatgggatgg ctgggtcaaa tggtatttct
72661   agttctagat ccctgaggaa tcgccacact gacttccaca atggttgaac tagtttacag
72721   tcccaccaac agtgtaaaag tgttcctatt tctccgcatc ctctccagca cctgttgttt
72781   cctgactttt taatgattgc cattctaact ggtgtgagat gatatctcat aatggttttg
72841   atttgcattt ctctgatggc cagtgatgat gagcatttct tcatgtgttt tttggctgca
72901   taaatgtctt cttttgagaa gtgtctgttc atgtccttcg cccacttttt gatggggttg
72961   tttgtttttt tcttgtaaat ttgtttgagt tcattgtaga ttctggatat tagccctttg
73021   tcagatgagt aggttgcgaa aattttctcc catgttgtaa gttgcctgtt cactctgatg
73081   gtagtttctt ctgctgtgca gaagctcttt agtttaatta gatcccattt gtcaattttg
73141   tcttttgttg ccattgcttt tggtgttttg gacatgaagt ccttgcccac gcctatgtcc
73201   tgaatggtaa tgcctaggtt ttcttctagg gtttttatgg ttttaggttt aacgtttaaa
73261   tctttaatcc atcttgaatt gatttttgta taaggtgtaa ggaagggatc cagtttcagc
73321   tttctacata tggctagcca gttttcccag caccatttat taaataggga atcctttccc
73381   cattgcttgt ttttctcagg tttgtcaaag atcagatagt tgtagatatg cggcattatt
73441   tctgagggct ctgttctgtt ccattgatct atatctctgt tttggtacca gtaccatgct
73501   gttttggtta ctgtagcctt gtagtatagt ttgaagtcag gtagtgtgat gcctccagct
73561   ttgttctttt ggcttaggat tgacttggcg atgcgggctc tttttggtt ccatatgaac
73621   tttaaagtag ttttttccaa ttctgtgaag aaagtcattg gtagcttgat ggggatggca
73681   ttgaatctgt aaattacctt gggcagtatg gccattttca cgatattgat tcttcctacc
73741   catgagcatg gaatgttctt ccatttgttt gtgtcctctt ttatttcctt gagcagtggt
73801   ttgtagttct ccttgaagag gtccttcaca tcccttgtaa gttggattcc taggtatttt
73861   attctctttg aagcaattgt gaatgggagt tcacccatga tttggctctc tgtttgtctg
73921   ttgttggtgt ataagaatgc ttgtgatttt tgtacattga ttttgtatcc tgagactttg
73981   ctgaagttgc ttatcagctt aaggagattt tgggctgaga cgatggggtt ttctagataa
74041   acaatcatgt cgtctgcaaa cagggacaat ttgacttcct cttttcctaa ttgaatacct
74101   tttatttcct tctcctgcct gattgccctg gccagaactt ccaacactat gttgaatagg
74161   agcggtgaga gagggcatcc ctgtcttgtg ccagttttca aagggaatgc ttccagtttt
```

# FIGURE 3-U

```
74221  tgcccattca gtatgatatt ggctgtgggt ttgtcataga tagctcttat tattttgaaa
74281  tacgtcccat caatacctaa tttattgaga gtttttagca tgaaggttg ttgaattttg
74341  tcaaaggctt tttctgcatc tattgagata atcatgtggt ttttgtcttt ggctctgttt
74401  atatgctgga ttacatttat tgatttgcgt atattgaacc agccttgcat cccagggatg
74461  aagcccactt gatcatggtg gataagcttt ttgatgtgct gctggattcg gtttgccagt
74521  atttattga ggattttgc atcaatgttc atcaaggata ttggtctaaa attctctttt
74581  ttggttgtgt ctctgcccgg ctttggtatc agaatgatgc tggcctcata aaatgagtta
74641  gggaggattc cctctttttc tattgattgg aatagtttca gaaggaatgg taccagttcc
74701  tccttgtacc tctggtagaa ttcggctgtg aatccatctg gtcctggact ctttttggtt
74761  ggtaaactat tgattattgc cacaatttca gagcctgtta ttggtctatt cagagattca
74821  acttcttcct ggtttagtct tgggagagtg tatgtgtcga ggaatgtatc catttcttct
74881  agatttcta gtttatttgc gtagaggtgt ttgtagtatt ctctgatggt agtttgtatt
74941  tctgtgggat cggtggtgat atccccttta tcattttta ttgtgtctat ttgattcttc
75001  tctcttttt tctttattag tcttgctagc ggtctatcaa ttttgttgat cctttcgaaa
75061  aaccagctcc tggattcatt gatttttga agggttttt gtgtctctat ttccttcagt
75121  tctgctctga ttttagttat ttcttgcctt ctgctagctt ttgaatgtgt ttgctcttgc
75181  ttttctagtt cttttaattg tgatgttagg gtgtcaattt tggatctttc ctgctttctc
75241  ttgtaggcat ttagtgctat aaatttccct ctacacactg ctttgaatgc gtcccagaga
75301  ttctggtatg tggtgtcttt gttctcgttg gtttcaaaga acatctttat ttctgccttc
75361  atttcgttat gtacccagta gtcattcagg agcaggttgt tcagtttcca tgtagttgag
75421  cggctttgag tgagattctt aatcctgagt tctagtttga ttgcactgtg gtctgagaga
75481  tagtttgtta taatttctgt tcttttacat ttgctgagga gagctttact tccaactatg
75541  tggtcaattt tggaattccc tgctttatta ttctaaagca agcttgtcca acctgcggcc
75601  tgtggcccaa cacaaatttg taaactttct taaaacgtta tgagattttt tttgcgattt
75661  ttttttttt tttttttagc tcaccagcta tcgttagtgt taatgtattt tttgttttgt
75721  tttgttttga gacggagtct tgctttgttg ccaggctgga atgcagtggt gcagtctcgg
75781  ctcactgcca cctctgcctc ccaggttcaa gcaattcccc tgcctcagac tcccgagtag
75841  ctgggactgc aggcgtgcgc caccatgccc agctaacttt ttgtattttt gtagagatgg
75901  ggttttacca tgttggccag gatggtcttg atctcctgac ctcgtgatcc accctccttg
75961  gtctcccaaa gtgctgggat tacaggcgtg agccacctcg cctggccagt gttaatgtat
76021  tttatgtgtg gcccaagaca attcttcttc ttccagcgtg gcccaggaaa gccaaaagat
76081  tggacacccc tgttccaaag catgtaattt tattcacaga aaagactctc ggccgggcgc
76141  ggtggctcac gcctgtaatc ccagcacttt gggaggccga ggcaggcgga tcacgaggtc
76201  aggagatgta gaccatcctg gctaacacgg tgaaaccccg tctctactaa aaatacaaaa
76261  aattagccag gcgtggtggt gggtgcctgt agtcccagct actcgggagg ctgagacagg
76321  agaatggcgt ggaccccgga ggtggagctt gcagcgagcc gagatcacac cactgcactc
76381  cagtctgggc aacagagcga tatccgtctc aaaaaaaaa aaaaaaaaaa aaagactctc
76441  aaggatttac catctaaaat catcaagtgt atatcatgca gatgaacaac agaaaacact
76501  gggagtattc aagtaattaa aaataacctt tcagcaccaa cagcaatttc tgtcctgatg
76561  gcaatctact cagagctaga ggactgcact taggatacca aagggagctt aggatgcaag
76621  agcagcctgc actaatgtac tctgcgttag tttacatccg ggcactttgc tttacgtttc
76681  aaaacagacg ccgcccttag ctcattaaag gggaaatgga ggtataatgt gttttaaagg
76741  gatgtttctt ctctgaagcc atatttcagg gtagcatgaa aacagagctt tggatatctg
76801  gttccattct acatgacacc tcatgcttgt ttcaaatgac accacaacca aggggcagga
76861  atgaaaggaa tatgctaaaa aaaaaaaatg caatcctttt tagcaagaaa gatcattaag
76921  gatttcctga taagtttctt ctttgttcat gtgccctcct ctgctccccc tttgaactac
76981  tgcccctgtc accccccctt ccccgcctcc ccgccgtttc tcagttctcc aatagggatc
77041  cagtctgcca gaggtctatt tttgctcatt attgtcaaca caatttgcca tcaagagttt
77101  tcacttactc ctgctatcct atcatcacat tatgcatgtc agaaagcatt acagattttt
77161  acttttcaag aacccgaact cagatttcca ggcttctgtc cttcctgttt catgctgttg
77221  actctcccac atctttgtct tcctcctaga cctttggtca ctcttgagcc cacattgcca
77281  atggtctata tcattttcac ctggatgcct cccagacacc tcagtcacat caagttgaaa
77341  tagaactaat tagacagaag tagcatgtgt ttcagtggaa ctgacatttt tctagggtcc
77401  ctaaaacaca ggtgttgttt caaactcccc cctccaacat cccttatatt ctctctgtca
77461  cccaggctcc ttatatcatc tctcaacatg cttcctgggt gactactgac tctccacttc
77521  tggtgacacc ccacagagca agctttcaat tcctgtcatt taaacacttt agtgcactca
77581  tagaatctca tccttctgag atttctcttc caccagtcta ttttgcagac caccaggcta
77641  cgcccttcca aaagctcctt tgagatggag tttcactctt gttgcccagg ctggagtgca
77701  atggcatgat ctcggctcat tgcaactgcc acctctcagg ttcaaatggt tctcgtgcct
77761  cagcctcccc agtagctggg gttaaagttg cctgccacca tacccagcta aattttgtat
77821  taggggtttt accatgttgg ccaggctggt ctcgaactcc tgacctcagg tgatctgcca
77881  cctaggcctc ccaaagtgct gggattacag gtgtgagaaa ccacgcctag tcccccaaag
```

# FIGURE 3-V

```
77941    ctccattttt atcctgtctt cattctctct tcaagaactt gcccacagct gccagagttc
78001    cttctaagag tttaagactc tctgttcatg tggtaagaat cttcttaagg agcttgcttt
78061    aaaggtaaaa ccagatttac acatcagaat ggggagaggt gggtgggagc taagatggaa
78121    tctgtaattt tcacaagtgc cctggatgat tctactgcag atggtctcct attcaactaa
78181    atagcactca tctgacccac tcctttaga ccccacttct gttaagccag tgttatcgct
78241    attttccact ttctttcaag ttctaatcat ccttaaaact ccaacagctt cagctgaaat
78301    cagtaccaca ctctcatcct tgtttcccac ccaaaaacta ttgtactaat tgaccagaca
78361    tgtattgatg ccctactatg agcctggctc catgataggc acttgggata gagaaatgta
78421    ccataatcct gaccttaata aatttagagt ctaatggaag agataaaaaa aatcaatgat
78481    tccaatgagt gtgataaggt aaggctagtc agaaaagtca ggtgcagagg aggggcaact
78541    aattaaacca ggggttgggg gagacactgc caactctgtg ttgagtgaag tttaagcaga
78601    ggtttttctt taagtaaaaa atcagttttt ttaaataaaa agtttttaa aaagtaaggg
78661    ggaatagaag aaggaggttt gtccctaagg ggaacagtgt ggaggaggaa aggaggtgaa
78721    agtgtatgct attctgtgta ctggcaaatt ctttggtggg tgatatggtt tggctctgtg
78781    tccccaccca aatctcacct tgaattgtaa taatccccac atgtcatggg agggacccag
78841    tggaaagtaa ttgaatcatg agggcaggtt tttcctgtgc tgttctcatg atagtgaata
78901    agtctcagga gatctgatga ttttataaag ggcggttccc ttgcacatgc tctcttgcct
78961    gccaccatgt aagacatgct tttgctcctc ctttgccttc caccatgatt ttgaggcctc
79021    cccagctatg tggaactgtg agtccattaa acctctttcc tttataaatt acccagtctc
79081    agctatgtct ttattaggag catgagaata gactaataca gtaaattggt actggtagag
79141    tggggtgctg cagtatctga aaatgtggaa gcaactttgg aactgggtaa caggcagagg
79201    ttgaaacagt ttggagggct cagaagacag gaagatgtgg taaagtttgg aacttcctag
79261    agacttgttg aatggctttg accaaaatgc tgatagtgat atgggcaata aagtccaggc
79321    tgaggtggtc tcagatagag atgaggaact tgttgggaat tggagcaaag gagactcttg
79381    ctatgtttta gcaaagagca tggcagcatt ttgcccctgc cctagagatc tgtggaaatt
79441    tgaacttgag agagatgatt cagggcacct ggcagaagac acttctaagc agcaaagcat
79501    tcaagatgtc acttgagtgc tgttaaaagc attcagtttt atgtattcac aaagatatgg
79561    ttcggaattg gaacttatgc tcaaaaggga agaagagcat aaaagttcag aaaattggca
79621    gcctgatgat gtgatagaaa agaaaaaccc attttctgag gagaaattca agcctgctgc
79681    agacatttgc ataagtaaga tggagccaaa tgttaatcac caagacaaag gggaaaatgt
79741    ctgcagggca tgtcaaggac ctttgtggca accctcccca tcacaggctc aggcctagga
79801    ggaaaaagtg gttttgtggg cccagcccgg ggacccctgc tctatgcagt ctagggactt
79861    gttgccctgc atgccagttg ctccagccat ggctgaaagg ggccaaggta cagctcaggc
79921    cattggttca gagatgcaag cctcaagcct tggtggctta cacgtagtgt tgggcctgtg
79981    ggttcacaga agtcaagaat tgaggtggat gtacagaaat gcctggatgt ccaggcagaa
80041    gtttgctgca ggggtggggc cctcatggag aatctctgct aggggagtat ggaagggaaa
80101    tgtggggctg gagtccccac acagagtgcc cactgggaca ctgcttggtg gagctgtgag
80161    aagagggaca ccatcctcca gactccagaa tggtggatcc accaacagtt tgcaccatgt
80221    gcttggaaaa gctgcagata cccaacacca gcccatcaaa gcaaccacaa ggggggctgt
80281    accctgcaaa gccacagggg cagagttgcc caaggctgtg gtgggaaccc acctcttgaa
80341    tctctatgag acatggagtc aaaggagatt attttggaac tttaaggttt gactgcttta
80401    ttggatttca gactcgcata aggctgatag ccccttttgtt ttggacaatt tctcccattt
80461    tgaacaggta tttttaccca atgtctacac ccacatttta tctgggaaat agctaacttg
80521    cttttgattt tacaggcaca taggtggaag ggacttgcct tgtttcagat gagactttga
80581    actgtggact tttgagttaa tgctgaaata agtctttggg ggactgttgg gaaggcatga
80641    tttgtcttga aatgtgagga catgagattt gggaggggcc ggggtgaaat gatatggttt
80701    gtctctgtgt ccccagcttc atcaggatcc tcccacacat ccccattcta agttgcaggg
80761    tcccattctt ttccaatcaa tgccctcatt ttaacagtag acacctggtg aggctgtgca
80821    tgcacctttc attgcaggtc agccgctccc atgataagag cttgtatctg attttccaca
80881    atttcagctc tttctctaca agagataaga ctctcactct ggacaatctt agaagatttg
80941    aggctcagtg tatgcttctg gagctgggag ttagaatccc taagttcatg gttttcttct
81001    atcagtttgt ccagtgaact taggagcaac caaccaactt cgttatattc cttggtcctc
81061    tatatatggt caaaggtatt atgtatagag tcattaaact ccttgcctct catgagtggt
81121    gaatcaggag tactgaatgc atttcttttg cataagtgtt tgaacagttc gtgccaagga
81181    ctatcagtgt tctccacaca attagaagta gagtccttag cattttgggg tctaatcata
81241    ttaagcaacc aactccagaa acccaaaaac caactaaaga aatctatcct tctgtaatcc
81301    cagcactttg ggaggccaag gcaggcggat cacgaggtca ggagactgag accacagtga
81361    aaccccatct ctgctaaaaa tacaaaaaat tagccaggca tggtggcggg cacctgtagt
81421    cccagctact cgggaggctg aggcaggaga atggcatgaa tccaggaggc ggagcttgca
81481    gtgagctgag attgcgccac tgcactccag cccgggtgac agagtgagac tccagctcaa
81541    aaacaaaaaa aaagaaaaaa aagaaatcca tccttaaaat tctgttcctc tagaaccatt
81601    cccagtacca aaatctgatt aaaaaaaaaa aacaggcaga ggaaggtgga aggactagat
```

# FIGURE 3-W

```
81661  ctttctccag tgctggatgc ttcctgccct ggaacatcag actccaagtt cttcagcttt
81721  tggactcttg gacttacaac agtaatttgc caggggctct ttggcacttg gccacagact
81781  gcaggctgca ctatcagctt ccctattttt gaggttttgg gactcagact ggcttcctga
81841  ctcctcagct tgcagatgcc tattgtggga cttggtatct tgtgattgtg tgagtcaact
81901  ctcctaataa actccccttc atatattcat ctatcctatt agttctgtac ctttagagaa
81961  cactgactaa tgccgtgggg ttaagccaat cgtctagtag gttcataaaa tgctaaaagc
82021  agatataaac ttaaagacga tccagctcat ctcttcattt tacagatgta aaaacagaag
82081  tagaaataga attggaaccc cagtttcttg aaacccagtg tttgcataat accatgctaa
82141  tttcattcta atttgtgttt tatttaataa tcaggaaaca gttcaaatag aggcccaggg
82201  ctctgaaaca ttgcccaagg tctatggctt ttgcaaagca agtactgttt ctgctacttt
82261  acctagttgc tcttggccta tgtttggggt gcatctaaag aactgtttgc tgattattaa
82321  ataaaacaca aattagaatg aaattagcat ggtgttataa aaacacaggg tttcaagaaa
82381  ctggggttcc agttctattt ctacctctag gatacagggt aagtcacttc ctttctcaga
82441  ttctgtttct ttgtatgtca aagggctgga ttagataaac ttgacttccc ctcttggccc
82501  tcatgtatca ttctataaat atgttatcat ttctaataga ctgtttgatg taatcttttg
82561  tctaatggcc cctgcttttc acaataaatg aaacaaaggt cacaagactt ttattcattt
82621  gcaaccctga ttaactaaca gttaatgtgt aactggagtg ccacatagaa acagaaagga
82681  gaaggggaat ggattgggtt ggagaaggtg aagtctgacc tatcctctac aagaggtaca
82741  gggtttatcc aggaagacag gagtgccatg aggagtaaac tccagcaggg gctggagtca
82801  tggccacaca cagggcctta caggatccag gctgcccaga gcagagtttg gatgggggct
82861  gggaggctgg gggagctagc tgaggagatg gttgtcatgc caggccacga gtgtggatga
82921  gtgctggcca ggggaggtat aggagattga tggggctgtg gcaagcaggg cagataccct
82981  tccaggaacc tgtttcatta aacacagaat acagtcctga gggctcggtc tcaatacagt
83041  cctgagggct cggtctcagg aaaacatgtt cttaagtttt acatccttct tctgtttga
83101  ttaggtgttt cctgattata aaataaattc ctagaactgt taatggtaac aacacaaagc
83161  actatagacc catacgagga tcatattgaa accaatgaca tttaagaata aaagatctga
83221  tgaatatgaa cacttccctg gttcctagga ggataacagt tgagttttgc tcaggtaatc
83281  tgccttctct tccttcttct ctgctttRta tctcagtttc tatattttgt cttacattaa
83341  taggatttgt tctcattacc ctgatgattt tatggtaaac tacctcaaat tctttttgga
83401  agaagatggg atataaatta tttttaaagt ttatctgaat agatgttctt caatatcaca
83461  caataaataa tgacaatgta actttgtgca tatagcactt taagagattg atcataaaca
83521  caatcctatt aatcttaatt caagttaata atgcttgcat ttgtatggca ttttaggtat
83581  tataaagttt tttttaattg tggtaagata tgcataaaat ttactattta aacctttttg
83641  aagtgtatag ttcagtagtg ttaagtacat tcacaatgtt gtgtaaccat caccactagc
83701  catttccaga ctttttcatc atcccaaact gaatctctgt acctattaaa catgacctca
83761  ttctccacct ccccacagct cctgggaacc tctattctac tttctgtatg aatttcccta
83821  ttctaggtgt ctcatataag tggaatcata caatattcat cctttggtgt ctggcttatt
83881  tcacgtagcg taatgctttc aaggttcatt catgttgtag catttatcag aatttgattc
83941  atttttaagg ctgaatatct tccattttat gtgtctacca cattttgctt atccattctt
84001  ctgttgatga acacctgggt tgttttcacc ttttggctat tgtgaataat gctgctatga
84061  acactgatgt gcaagtacct gtctgagtct ctgctttcaa ttttgggcat atacctagaa
84121  gtgggattgc tggatcatag gatcattcta tttttaactt ttttgaggaa ttgccatacc
84181  acccgctaca gcagccgcat cttttatatt gccaacagtg cacaaaggct ctgatttctc
84241  cactttctgg tcaacattta gattatcatt cttttttttaa aaaaacgtaa tagctaaccc
84301  aatgggcatg aagtaaggtt gtttttgttt tttgttttta atgtgtgcgt tatcttactt
84361  gatccattaa atccctaaca agaactcctc tagggcagat gtcctgtctt attcatcctt
84421  ggccccagtg tcttgcaagc aagtgaattc tcaataagtg ttaattgaat ggatggttga
84481  tagatttata ggatgcatgc caattctgtg gacgagagta ggtactaatt gttattttca
84541  tttcacagaa gagcaagtca gggctccgag cactaagtga cttggctgag gtcaaactgc
84601  ctgcaagttt tatctaatag tgacagagga accaatgtgt cgagcatgaa tgtcagtcca
84661  ttgaaacagt gcccactttt ctgactctgc tccttaagag acagggcctg tacagcaagg
84721  acacagagaa gcaggttaca gaaaaagggc tggctcatcc gtgtatgcct ggcatttgag
84781  gaatgtggct gaaatctcaa cactctggtt cagaagcaca tctgcaatca aatataacaa
84841  gacatggtat gagagatgtc tggcatacca aggagattcc tagaatacag cggataggaa
84901  aacctcatta cttcaattcc caagaaagag tactactggt agtaatccca acaggagtat
84961  ccaagtaact cggtaatctt cagtaaagaa aagaaattgt gaaacaatta tagtcagtcc
85021  tccatctctg taggttccac atctgtggat tcaactcacc tcatattgaa aatattaata
85081  aataaataaa taataacaat ataacaatta aaataatgca aattttaaaa gcataacaac
85141  tatttacata gtatttatat tgtattaggt attataagta atccagagat tatttaaaac
85201  atatgggaga gtgtgcatag gttatatgca aatactacac cattttacat aagagacttg
85261  ggcatctgtg gactttgtta tctgcaaggg tcctggaacc aattccccat ggatactgag
85321  ggtcaactgt accaagttga aaacaaaaca aaaggaaatc tattgcaaaa aggaaattcc
```

# FIGURE 3-X

85381 caaagataaa aagcatgtct atgaaatagt caaagagccg aaaatgttgg gaagacccac
85441 actgctttcc ctgccctgtg ccccttttttg cagatctatt tgtgtcttta tctaccagca
85501 gatattctat tattctaata gattcctgtt tttcccaaga gggtttactt atttattaaa
85561 tatgatgcaa acatctctct cagagatgtt gctctctctt cctaggattc cttagcccta
85621 aatctgcaga gccaattagg tattaatatt gggctttaca actttggacc atctgaccac
85681 caagactgat tcattaaatg tatatggttt cagatcacat taatttatca tagactttag
85741 aattggcttt ttagaagtac tgctataagg aaaatctcag catagcaagt tttatctaat
85801 agtctacttg tattaaagag tactcaatgt aaaccctagg aagactttta actgcctttt
85861 ggaaattggt tgagtgggat ttgaaccgtg tactctctgt aaagcaggaa attatcactt
85921 agtaattact aagtatttaa aaatgggaaa tagaaaatta catttcagac ctggtgcagt
85981 ggctcatacc tgtaatccta acactttggc aggtggaggc aggtggatcg cttgagccca
86041 ggagttcaag accagcctgg gcaacatagt gagcaactcc atttctacta aaaataaaaa
86101 aaattatttg ggcgtgatgg ggtgcacctg tagtccccac tactcaggag gctgagacag
86161 gaagattgct tgagcctaga aggtcaagcc tgagtgagca gtgaccggcc attgtattcc
86221 agcctgagca acacagcgag accctgtctc aaaaaaaatg tatattttta aaaaaagaaa
86281 attacatttt agttgttcca taaatatcag tacaaccaaa cctaagtgca aaattcccaa
86341 cacaaatgat cgcctctggg ccagccatgt agccccccatc ttgctgtcaa taatcatttc
86401 caggggctgt aattgtcttc cttccccctg taccacccca tcacaggaca caaattgttt
86461 tgcttagttt agatagctgt gactaaacta agtgccaatt gtcttttaaa atatgtgtta
86521 atcagcactc aagattgttc ctacaaaaat gtcactccct cactcaattt gcatgtgccc
86581 tcctgagtag gagagaaaga gctgagttag aggcagccct ctgggacctg cacagaaccc
86641 tgtatgcttc cgggagtgtg gagtgtgtgt ctgatctgct gctgggaaaa ggagaagaaa
86701 tgctgagaca ctcactgcca ggggctggta tcaggccatt ttcacagggg ctgttggagg
86761 gttgacagca cagctctact ggaccaggga gggtcccagc cagcagggcc tgcccctccg
86821 aaactgtccc tgtccctgtc cctgagaggc cccactgagt gtcagatggc acataagaga
86881 tttccttatg cgttggtgtg aaggagatga tcagttccag gaggcccctc ccccatgcag
86941 aagagaagaa aatggaagaa accgggttct cagagtggcc tgcgggtgag tgccgccttg
87001 tgctgtcagt tcccttcacc ttccagttct gggtgtacct agtgtggttt catcaaactg
87061 ctgaggccct ggaattgagg gaggatgctg aagggtgcca ggccatagaa gcagtagcag
87121 gagctgcagc aaagagctag tgtgtctcca gcgtcagctt ttgggccttg gtggcatcaa
87181 aggagacgta cacagggcca ctgtactacg gatgggattc ttggaacaaa agtttgaata
87241 attgatgagt gtggaagtgc tatgcaaaaa ttcaattcaa cctgcagtta ccaggcattc
87301 attctgtgac aggcattatt gtgagggtgg caaggagacg gggagtctct gagagactca
87361 gagaagagca gggcatggtg tccacccaca agagatctga agactttcaa agtagttggg
87421 aaaacagaca tacaactttt ttcccctccc cacagctagt tgaggcaaaa gacatataac
87481 ttatacagtg acaaaatact actgactgtt actgacacat aacagcctgg gctgggtcac
87541 tggtgagaaa aaggaggatt cttgcttttg gggcattcaa gtcctgtcca ttaaccattt
87601 cccaatccca tatttataca tcccatttta gacatattaa gctgaagatt gatgtgacat
87661 acccaaagac acttaaggtt gaagctgaga atctgggcta gaaatataaa tcaggatggg
87721 ctgggagcgg tggttcacgc ctgtagtccc agcactttgg gaggccgagg cggcggatc
87781 acttgaagtc aggagttcaa gacctgcctg gccaacatgg tgaaacccca tctccaccaa
87841 aaatataaaa aattagccag gtgtggtggt gcatgactgt aatcccagct actcgggagg
87901 ctgaggcagg agagtcactt gaacccggga ggcagaggtt acagtgaaag gagatcgtgc
87961 cactgcactc cagcctgggt gacagagcga tacttcgttt caaaaaaaga aaaaaaaaa
88021 aaagaaagaa atgtaagtca agattaaaga caatgggtga gatcagcaag gagcatgtgt
88081 gcggagaaga gaacaccaag gaaggctgcg tgtggtggga ggtggccggg gggcagagaa
88141 agaggcggcg gagccaagga gatagggcat cgtctgaatg gtgatgctgt atcaacagat
88201 gtgaaattcc cagaggtgtg aaacacagca gatcctttac agcacgatga caggacacag
88261 catgagccta cctctgccag gtgagaggag ctttctgcaa cctgtgatgg gctcaaggat
88321 gctgaccatt catccgccca ttgggatagc ccaggctctg agctcagcac tccacccgtc
88381 actgcgattg cactaatcct cacccaccct tgcaggcagg tattactgtg ggcacagaga
88441 agtttgctaa cttgccagag atcctgtgta ggaagccagg tcaggaaaca accagagtct
88501 ctctaacagc ccaggccttg aatgaacacc ggcctgcttc agaatacatg gccccgtgatg
88561 tgtttgaatt cacagattca ctggacaggt tccctatagg gcctgtgagg aaatctggtc
88621 taacagaatc cagaaagaca aatttcgcta aacaggtcaa gcctgatact gctgctacac
88681 ttcagctgtg ttaagccact cggttatcag acctgcttct cctttcatga ttttagtaac
88741 acaggcctct tccttgggcc cctgttgctc ccaccatctc ccaggttctc ttgtactcag
88801 ggtttagtct cccctcccca ctactaatga tgcatgtgcc ttactctctg atcatctctc
88861 ctaaatctgc tactcctctg ctctttctgt gccttatcta tccctgccga gtctaacatg
88921 cagattttgg tcattccaaa gtcatgtata gatctaatca cagggctctc tgcttaccag
88981 ctgctacttg gataaggaaa gcatgccaac acggtcctcc ttcttcatgc tggccaagtc
89041 agcatcatta ttattaccta agtttatttc taacacatct caatatcttc atgcactccc

# FIGURE 3-Y

89101 tcttgataaa agtaactgag catagcacca tcaataccat caaatctgtc attctcttcc
89161 cctctctctg ggtgggacag gaagccaggc tgctctagga aatcttccct aacaagcaaa
89221 ggggacttgc ctgtcctctc gcatgtgtga tctgagcttg tgtggatccc agagtgggca
89281 ttctggacta cttgaccttg cctatctctc cttcacaccc tctcatctct ccctcctacc
89341 accaaaaaac ttgcatcgta tttccaatct ccagacatac tcttgaaacc atttatctat
89401 ctggtgtgtt tatctgtatc tagtatgatg tgaatatgtg atttgtatgt gtgtctacca
89461 ctgttttggt cagtttgtat ttctcgtggg tacagttcta tgtgctcatg tatgtggaac
89521 atatgtatac tgacacatgg acctagctcc aaatgatctg aaaggaatat aattgtaatt
89581 gaatatttgc acagatatac aacatacaca tgtgatggct gggggaaatg catgtgggat
89641 ttcagtcagc attttattag agaaggtatg tcattagtgc tgtattaaca atgaatcagc
89701 ttatttgtgg gtcactgtca atgactcctt tgcaaatcac acatgtaaat atttctgtct
89761 gtggtctgat gaacatgcag tgccacagtc tgggagatgc tgagccatgc cctgtgtagg
89821 cagcatatga aaagaactgc atgatttaaa agatgctgac cagcttaagg aaagcaattt
89881 aaaaacttcc taaaaatcta gtttgaatga acaacagttt tcattttgtg tgtgtgtttt
89941 ttcttttaga gatggggtct tgctatgttg gccacgctgg tctctaactc ctgggatcaa
90001 gcaatcctcc tgcggctcag cctcccaaag tgatgggatt acaggtgtga gccaccgtgt
90061 ctgctggccc cactgtttta aaccctgatt cgacaatcat acatttaact cattacctgt
90121 cttgttcctt ttacgacaaa ctcaaagctt ttatttttact aaagtatttg ggttaatctt
90181 ttgcttttct gtcatgttct gaaatatgta caataacaga atcgctcaaa atattatctc
90241 cgttaaatgt tttgtggctt tagggagagg tctaacaaca tgcgggaaac aagaaatcaa
90301 gcgcatccag gattcattta taatctctct cgttgagtag aagtccgcat ctctcgatat
90361 tgtctggtta cctgcatgaa gtattctaaa ggaggaaaat agctcaaaga ggacattcat
90421 gtgcactctg gcttccagtt ggccatttga gtaagtgatc gcaattaact gacgagcggc
90481 agggaaacac ttcctggaat tctcatctac agacaagaac aaactggggc ggggcccatc
90541 accttcacct acgcgccggg agggtggcgg ctggcgggcg gggccgggct cgggccgtga
90601 cgccgagagt gcggggcgcg cggctgggag cctcgcgccc ccgcccgggc ccgcccccat
90661 cccgcccgca tacagcccgc atcccgccgg ggaagcgagc ccagtctagc gctgcccgtc
90721 cagtcctcgc ccaagattta aagcccgcaa gttttgttct tgagaccagc gactttagct
90781 ccgatgcggg aaggaaagcc gacctccgat ttggacattt aaagagctgg gcttgaactt
90841 cgtgagtttc gctctaaact gcccttgaaa tgaagctgga cttggaggta aagtcactgg
90901 gaagctggcc tggggcgggg tttccccctc ttctcgtatt ttagaaacgg acagcggcag
90961 tgcagcccta gtttgctgta agtttcctta ctttgttact gaggccccca gagctccacg
91021 cataagtggt gtgaccagaa acctttttaac aagacccgcc tgagcctgcg ttagagctcc
91081 cgctcggaaa gtaaaagacc atcctaatcc gcggcgctgc ggaaccggtg tcccgtgtgg
91141 gaggaaccgc ggcgttccct gggcgtaggg cccgcgaggc cagcacagtc cgcctcttgg
91201 cggagcgccc tgggccggtg gttccgcgcg gagttagtct gtggtcagtt acgtggtgaa
91261 aacacggctg tgccgcggcc gcatctttcc gcggccgagg cctctctggg tgggagtgtt
91321 ggcttccttt ccggatcgct aaatggggaa agttctggcc gctcggcggg atacgtctcc
91381 aggccacgga tggttcgttc tccgtgccgc ggccccgagc tgggctccct gggtctccag
91441 cgcgggctcc cggcattggg ggctgcgggc cggcccctcc gccccgcccc cgccccgccg
91501 cgcctcctcg gccgagcggc tcgcggtctc cggcgcggga ggctccgagt ctgcccactc
91561 cgggccgagc gaggtctctg gaggagaaga gtggcgagga ggtgagggca cgccggccct
91621 cgcccggcgg gtggcgccag gacttcaggt gggaacgcgc gcttgggccg ggggcgcgtg
91681 gctggcgtgg acaccggatc ggggcccgcc gccctggccc ggaccgcgca cggcccagcg
91741 ccgggaagtc gggaagccgg ggaggcctct cccaccgcgg gccccggcag cccgccctct
91801 gaaagcgcgg cggagaagga ggctcgtccc ctccccggaa cgcctttgtt ccctccggcc
91861 tgcccgcgcg ggtggccagc ggctgggacc caggccgggc cgccgcccag gtgcggcagg
91921 taggctcggg ggccgggcag ctccggttgg ggcggcttcc cggggcctgc gggtccccgt
91981 ccctgaggag ctccggctcc tcggtggcgg gacaggcccg tgcgcgggag ccgcgaggcg
92041 aacgccgcgc ccaccaattc ggttgccggc cgggggcccc aggcttgcgg ccacccgcct
92101 ccggctggag ggctgaattc gagtcgaaag cccgtgtcgg gctggaaaga agaaaccgcc
92161 aacctgagaa cgctttcggc gagttactgg cgggggaaat ggggacaggg aagtgggcag
92221 gcggggagac tgcagccgca gatctccctg gcggggaggt cgtggccact ctttcctttg
92281 actctgcctc atttcatttt gaatcctgat gtgacagagg caattgcttg cttggatacc
92341 atataggtaa aagtaacagt tttcaactcg actcttgact acaccctgta cattcttggc
92401 cggtgttggt tttcttaggt tatggatcat gttaaaggta caccgatgtg gtaaccgcac
92461 agtggccgat ggtggcctga ggctcatatt tatggtaatt atctgatgaa agtacatcca
92521 tcagaattgg atttgtgcgt ctgtgccttt attttgggaa accttgcctg ttccctgtgg
92581 gggatgggag aggaatgtga aaagccgaag ttgacccaga aaaggatgat tgaaggtagt
92641 tgtattaagt ggtggcaccg aaatgattcc accctgaact ttctgaaagg gtgattagtg
92701 atcagcagca gacgttataa ctttctcaaa ataaatttat ggtagatttt ctatctggtc
92761 acaagtgagg aggtgaaagc tgctttttgg ggccaactct ttgcttttaa agcaagctaa

# FIGURE 3-Z

92821 acgcaatacc agaaaggttt ccattctgta cttaacctgc ttgtccttct cactcttcct
92881 tatcctcccg cacacgctct gagcatgac tgagcactct gaggaggagg ctgactcagg
92941 ctgacccttc ccggccctgc aaggctctaa ggtagaacca gtgttatcac aggaaaggcc
93001 caagccaaag ctcagagcag ctgttcctga gaaagtggga gatgaggatg agtaggaggt
93061 agagatgctt ataactgctc tgcccagaga aaactcagaa ggtgcagaag agttttcaaa
93121 ataaagtgca ggccccatca agtaaaaaat gaaacattgt atttcattaa aatggtgatc
93181 agttttctct tttcataaaa gacattcaaa tggtgagatt gtaaaaaaaa aaaaaaagaa
93241 aaagaaaact ttctaattct caaaaataga acagcttgaa taataactca tgggttttga
93301 aatgggtcat cttttaaaat gggtcacttt ggctagaatc attgcacttg gtgactttca
93361 cattgtaaag ggtccagttc tttctgaggc ccaattgcac tagaatctgc atttcagaac
93421 acaggaggta tcaggaagaa actgggagaa attcagaggg aaattgtctc ttctcagagt
93481 aacaaaatgt cccctattca gggggaattt tatatatgct gaagtaaata gatctcagct
93541 ttgaattttt ataatacatt atgccttttc tgaaatattt ccatagccat tattttaatg
93601 cattgttaga ataaccttgt aaggagagga attcattgta tctgtgttcc agaaactgag
93661 gcacagtaaa ttacaagagc tctgcccttt gatatcgaat ctctgtgagc gaagatgtaa
93721 ccagaacagt gtaaatctag aattactttc cttacggtat gcaatatcta attattatgt
93781 attagtttac atatatatag gggatgtaag aatctttata tttaaaggat aagaaacttg
93841 atcaggtgag atacaagatt tgaataaaaa agcgattttt tagaaacatt ttaattctac
93901 aaattcagtt gctggctttg atatgtaact tattcagatt tctcttccat agaaggcttt
93961 aacactagaa gccagttctc tacaaaagag aatgttctac caagtgtgta ggcaacaaag
94021 cccaaaagtt caagttcaaa atactgttct acagcctaag atccacctaa tatttcagat
94081 ttgactcttt tgctcctttc ctataacttc ctataacttt ttcatagtgc tctaagcaaa
94141 gtaagttcaa ggatggactc acctcaagtt tccttctctt actttagagg accatataat
94201 cttatacctg tgtcatccta attgaaatgt attttaagtg cttgtgggaa caagaataac
94261 ggaagaccgt tattccatta atggaataat ggggaacatg agaaggacca tattcagtca
94321 attagggatg atacttctgc ctcaacagta tttgcgatgt gttaaatggc ccttagccat
94381 cagatcagta tttttaaaaa ctcctaccta attaatgttt tttgagaaga agcatattat
94441 gagtatagct cagtattcta aaaagaaaaa aacctgaaaa aaaaatcagc catccattaa
94501 ctaacccatc ctggtaaagc tacacaaaca gattctagag aaaggaaatt tgcacagatg
94561 gaacatctaa tctggactga cattgtcaac aagttatctc aaatgatcct gagaaaacac
94621 tggtacatgg tttagagaca agtcgagctc atgtgactag taaatggaga ggcacagtat
94681 acatctactt ttgttggcat ttaaacactc tcggcttttt tactctcttt caccaccatc
94741 aggtccagat tccagcttgc actggaatat ttattgaccc tttgatagaa gagctacacc
94801 tgaaacatct ctgaagtctt tttagcatcc tatcctgccc atggcctact acacagtatg
94861 tgataagtta atgtttgttg aattagttaa atatacgtat taacctactt tcagggctgc
94921 ccgctgtaat gcctagaata gggcaggcac ttaataaatg gtagttaact tagactaaat
94981 gttgatccac caagacagaa acatgttaca tatacctctt gttttacacc atcttctact
95041 ttttgtacct ctgtgttata aaccctctat caagtgttgc ttctaaagac aatgtccaga
95101 atgggacgta ggaactgcca ctgggcaaca ggcagacact gggttatatc gtcatcctgt
95161 ttctgaatcc tggtttttct tattattatt caaccacagc taaactttct gatctccttt
95221 tttccgtttt ttccctttac cctaacttct agccaaacta agtgatttga catttcccca
95281 ctcaagccac aactttttct cttatgtgct taaaaaaaat tcctgcgtag tctctcttgg
95341 aatgtcttat tccttgctcc ctccacttca cccttttttt tttttttttt tttttttttt
95401 gagacggagt ctcgctctgt tgcccaggct ggagtgcaat ggtgccatct cggctcaccg
95461 caacctccgc ctcctgggtt caagcgattc tcctgcttca gcctcccgag tagctgggat
95521 tacaggcatg ctccaccaca cccagctaat ttttgtgttt ttagtagaga tggggtttca
95581 ccatgttagc caggatggtc ttgatctccc gacctcctga tccgcccgcc tcggcctccc
95641 aaagtgctgg gattacaggc atgagccacc gcacccggcc cacttcaccc tttaagccag
95701 ttgaaatgct ccttgcccta ctcctccctt ctcctttctt ttctcccatc caagtactaa
95761 ccaggcccaa ccctgcttag cttctgagat cagccaggat caggtgcatt cagcggggta
95821 tggctgtaga cttctctttc tgagctgtgt cttcctcctt tcctccccaa acccacattt
95881 gaattctgtt ctagctcctg ctcatttgcc acctctccca caaagccttc cccagtttcc
95941 atcttctccc tgcttccctc atctcagagg aaagctcttc attcacagag cacctggttt
96001 ggggtccata gtttgttgtc cctcagtatc cttggggaat tagtcccagg acaccacccc
96061 cccgccccctg atatccatgc atgctcaagt cccttatata aaatggtaca gcatttatat
96121 tataacctat gcaatattcc catgtactct tttttttat ttttatgtgt ttagagatgg
96181 gtctcactct gttgtccagg ctagagtgca gaggcatgta gccttgagct cctgagtcaa
96241 atgatcctcc tgcctccgcc tcccaagtag ttgggattac aagtatgagc taccacacct
96301 ggcccatgta ctttaaatca tctctagatt acttataata actaatgcaa tataaatgct
96361 atgtaaacaa ttgttacacg gtattgttta gtgcataatg acaagaaaaa aatgtgcatg
96421 ttcagtacag acacgaccat aaattttttt ttcaaatgtt tttgatctgt agttggttga
96481 atctgaggat gcggaaccca tgaacgtaca cgaccaactg catatgggtt tctaataaca

# FIGURE 3-AA

```
96541  gatttgtgga tgaagcccac ccaacataat gcaacatttc aaatgtatct tagtcagttt
96601  ttctggggca aagccagcct aacaaatctc aagaatgttt gaaaaattct agcttgagtg
96661  aacgttgttc ttctagtgac tcaaataaag cagtcattgt gtctgaggag tcctagtggg
96721  gtggcagggg tggggctagg agtagtggct gtttgtagca tgttttatat cacattaaag
96781  ggctgggtat gcattttagg caataattct tactctttatt gataggagaa cttgtatttt
96841  ttattcatct accttatgat ggataactgg tttacatctc ttcatcaggt ttacttctta
96901  cgtgtcattt tatgagctga ctttgaatat atcttcagat tttccctgcc tttacacatg
96961  aaaacggttt tgatacagct ttttcattag agaatggcag cttttccagg ccagggtgtc
97021  tgtgtctgac tcctcgcctt tcattttacc caatacttgc ttaaaacatc ctccctgagt
97081  ccggtacttt tttgcctcct ctccttcctt ccaccccacc tctttttgtc accctcctgc
97141  gactttggat ctccatcctt ggagtccctg aatctttttt gtggttgaaa gactacaccc
97201  aaaggacaca gactgatgag gtcacttctg cccactgtct agtactagtg agaccaagaa
97261  gaagaaggag ggaaaactga aatggaaggt ttgaaaaaga gtgtgttagt gaaggaagta
97321  agaacagtgc taacagaatg cagcaaaaca gaaatgactt cacacttctg gcagtgaagc
97381  aaattctcat ccatcttggg ttgcttctct tgaccctgcc cacctcctcc cctgccctcc
97441  cctcccgatg agaactgcag gaacacctgt gcccatgcca aggaaaaagg gcaggtgagg
97501  aacacaggga tggggctggg actcacaagc tgctctaagc tgccacaggg tctggaattt
97561  gttgacatta catggttaac tcggtatctc gatgcctcag tatcttctgc aaaatgtgga
97621  gggagatcct ttcttcctca aaggttgttg taaggcttaa agagctacca cattcgtaag
97681  gcatttggaa cagggcctgg tatatagtag acatacccat ggtagtaact gttgctagac
97741  tgggtaaaag gagaagtttc ttaagtaggc aagaaaaaaa ctgacaacag ttatatttaa
97801  aagagcaagc aaccgtgtca gcccaaacaa gtgtgttgca agaatgagca cagagggcca
97861  agctcagaaa agcgagtgtt tgacctctct gagacctcct ttatatgtca gctttttttat
97921  ttgtttcaaa ttttgcataa tcaaactggt tactgggaat tattgagttc atataacttc
97981  cctaaaatct aagaataatc tataatccca tgccaccagt cccgttaata aagacttagg
98041  gtctgtcttt acccatttgt tcatttgaaa ttccccgtct ttacttcccc cataaccagg
98101  aaacagatct acacccatgt ttaacttata aagagatata agtgagttta cataggtgga
98161  gatcttgtac ctggattcac attgtaggtt ttattaccca gctttcccct ctatcccaaa
98221  gccatgattg ccatagtgga atttaaagtc tttggactaa cactgaatca agaactaact
98281  ctacaaatgt attctctgca ggaggataag aattccaaat ggcttctaat tgttgcccat
98341  ggcttgaaat aaagttccat atagctaaag ccccagcaca aaaacacttg aagacagcca
98401  ttaggtagaa tttcttttct tttttagagg tcttatattg gaaatgtaac agttgcaaag
98461  atatctaatg tttcacctta aaatgtgagt tataaattca catttcacat tgcctccttc
98521  ctccttgagc aaacagacta ggcaattagt accaaatagt aactagttat atttcttaac
98581  accgattatt gtaagtaatt tattaagaaa aaatctagga agatcaaatt atagcttttc
98641  cagtaaatct ggggcttttt attcctgatc tctctcaaca aggcagtttg gcttctgaag
98701  attggtccta gctatactta acaggaaaca gatgacgaga agaagccagg aaaaaatact
98761  tggaatatag aacaaaatgc agcataaaga tggtacagaa gattttatct ttctttcctt
98821  tagttatgtg tggacaggaa ctaaaaactc tcccacgtgg gactttatta agtaataaaa
98881  gtttaaatgt aaaagatata aaggaatgag gatataaagg aaatttttt aaaaactgtg
98941  actcaccgga ggagccttat atagtctact ttctaagcat atcactttta aagggatcat
99001  tatatttca tgatgcatgg ggatattcca agtattaaat tgattttta aatgaagaaa
99061  attatcagtt cagtcttgtt ttctatgtag gtttcactac agtattttat ttcccccgca
99121  aggaaaaaac cagagctaga atggaatgat taaatttctt agtttcttc taatctgtta
99181  gtctttccat taaattatac tgccttctta tatagaattt ctgggtttgt tcttgttttg
99241  gcaataattg aagtaagaga ggatgatttt atggtagagg tttgattcca ttctagattg
99301  gtgtttttca gctatgtaga acacaatcca ttcatggttc agacaaccgt tttaggaggt
99361  tttcatgtcc agcattaaaa aaaaaaaaaa aagaatagaa ttgggcattc atatatatat
99421  atatatatga atatttatta ataaaagttt aaaagatata aaggaatgag gaatgcatat
99481  atattatata tatatatacc aaattaaagt atcataatac taatacaagg attagtatcg
99541  ttttatgata ctttattata tacactaagg gttagtcttg ttttatgata ctttattata
99601  tacactaagg gttagtcttg ttttatgata ctttattata tacactaagg gttagtcttg
99661  ttttatgata ctttaggttg gtatacatac atatactaag aatgcagaca tgtccatgga
99721  agttggcaat attcagcccc cagcaggccc cagtcagcag gacagggatt aggggagtca
99781  ggtgaggcag ggctgtataa gtgcagggtt ggagcctgtc ttggtttacc gttttgacat
99841  tttgttcact gtggattttt ttcagtaact ttgatttttt aaaaatattg catcaaaata
99901  ttatactgac tacggagttt ttggtacccc cttaaattgt gcacctaaaa taagtggctc
99961  ccttgtctcc ccctagtccc ggcccgatca gtatgtttta tagagggaat ggtgagtgtt
100021 ggggaactag ggagcccatg aggaggcggc ccagaagggg aggaggagct gatggtgtgt
100081 ctgatgtctc ctcctttctc taggaagcag acaggaggtg aagtcttcag ggtggggggg
100141 acagagcctg ggagggggag atttagcatg gccgctgaag agactgggaa agggagaagg
100201 ttcgggacaa gttcaggaac agtcacgtag cactgaaaac tgctcaaagc tagatagtgc
```

# FIGURE 3-BB

100261 catttgtggt gacgctgcct gcttggtaag tcccatttcc ccagcagccc tggaggtgtc
100321 ataggaacag gaagatggga gggcgctggg gtctggggtt gttggggtgg gagggcaaag
100381 gtgtccaatg attctgcatc tgaaacgggg gaggtgtgtg aggaagaaag gtcaggagac
100441 tgctgggcag ggtggggtgg cagaaggttc tcgatgaata tttagaagtt gcagctgagg
100501 agtgagtgta ctatagagtg agtatcctgg aatttgaggt ctcagaagaa atgcagttct
100561 cagtgaagcc atgtaaacaa cttcctggct gataaggtgt cttgagtgtt gggagatcac
100621 atgatcaaaa tggtatacac accataaggt gtccttagac ttgagtgtgg tgtaagtcta
100681 ctgaataaaa tgcagaaata cagttcactg ttgcttttct tcctggaaat tgccctcccc
100741 aacctcaaac cagcccccaa gtggggcagg gtccatcttt agaccctcct gtagcaccca
100801 gcacaaccct aactatatgg tcaatgtctc tcttccccac ttagagctcc aagaccaggg
100861 ccttgtttgt cctgcttacc cactctaccc atttgttaac cccatcttaa ttttctgatg
100921 aatgagccct tttttcattg actcctcctt tctctccttc tccggagttt atttttcctc
100981 caggcccatt gctgtctctc cacctagaga gcttatccac attcatgaaa ttagtcatct
101041 tcatgctcag agtacccacc tggatctctc cagtcctgtc taactcgtct tagattatta
101101 ccccctggac atctgtacct tcttgtctct ccatcgcctc gaactcaatg cgagtctaaa
101161 atcaaatact ccttcactcc taactcagga aaccagtttt ccctcccttg ttggtaatgc
101221 ttgaaattag ctcttacttc tcttcttcct cctttactcg ccatcccagg tcattcatca
101281 ggtcctgcta ggtctttgat ttcttgaaa gggccttctt tatgccttcc ttccgtcccc
101341 atttccacca ccctcgacta ggccttcctc acctcatccg gcatcagtgt gagcccttga
101401 gaaaccagcg tcctgatctt caggcattct gcatttcacc ccataccacc attagccagt
101461 ttaatccctc ttgctcaaac ttaataaata ctgagcaagg gagttactat aacaaacaaa
101521 accagaaatt tctatgactt cctgctgctt tctgtacccc cctaagactc ctctacctga
101581 ttttcaaggt tctccataag caggttccca cctctctgct ttcaaatctt ctatctgcca
101641 tacacagttc ttgtctaaaa caaagattgt tcctactcct gcagccacct aaatcctagt
101701 cgtcctcagg ccctcctaaa atcctgacta atccaacccc caccgatttc tctactagtc
101761 ccagagactt ccctcttgtg cctggggttg gtgtcatgca gacacactgt accttaccag
101821 gatttgccca ttgttgtctg tgggaactat gctttcatgt gggaactacg agtcttgtca
101881 caaatagagt gtaaatgctt tgagattgga gactgtaaaa tacttcttca tgatctccca
101941 ctgtgtctgg tataagaacc cgcagagtaa atactcatat acttgtagga ttaattgaaa
102001 caactggaga ctgaggttgt ttaacttgta cttagaagtg tggacctccc catcttgatt
102061 aaaacttcaa aacggatagc aggtttcaca tattatctca gcatcacaaa agtagactca
102121 acaaagaaat gagttgacag caggggtaag gtttacaaca atcagggatt attgcaggga
102181 aacttgtagc ctttgggtgt tctaactagt tttttcccaa aaagtttaaa gagctctctt
102241 gtgaaagaat attcttatga gtaactgagg ggcacggctc ctagactgaa aagtatttgg
102301 gatctgtcac ctcttttctg atgttcctac ttctattctt tattcttctg actacctcta
102361 ttagaaaaga taatactaag aatacctgtc ccttcttctc agtccaaata gaagcaaacc
102421 caggttgtat ctgagatatc tgcatatttt cttctaagca attctttgtc ctcttctctc
102481 ccatgctttt tctatttcct attttcagca ggctctgaag tcatttataa tttttactgc
102541 ccctcggtga cattacatgg atattcatcc ctgatttgca gattaaagca ccgaatcaga
102601 gtgaggtgag ggttgcccaa ggttacacaa taaatctggc cccaaacagg ggtaaccctt
102661 gagtttctag tctgttgatt ggccactgac ccgtgctgca ggcacacaaa ggaagctgca
102721 cccacagcag tctgttgtgg atggttgctg agctgcgcat tcggcattgg gcttgctttg
102781 tttcctgcca ggcccagcat tttcttctac cagatcggca ggcttgtggg cttcttccta
102841 ggtccctccc ctgcactctg aataggaaag ctggaagctg tgctttagag aagctttaag
102901 acgccgaaag aaaccagaag agtgagcgcc agttgtatgt gcgtggtctc catccgcaaa
102961 gccggagctg ggcgcaacag tgttgacttg taattgatca atttagatcg ggcgcaggcc
103021 gggggagggc agtgcttttg atttaggctg ggaaaggcct cctagtgact atgttcaatt
103081 tggaggaatt cagatgttct tttgttatac aagtgaagct gtgtaataca aatgaggagt
103141 tttacttttc ctaaatcttc cccttatcat tcaagtattg aggagtttta cctttcctaa
103201 atcttcccct tatcattcca gtattatcag tgagatctgg ttgtgattta tgtaaatggt
103261 ggctaaaaaa ttcaaactac tgagggggag aattctcatt ttacagcttc acatgctgtg
103321 ctgaactaaa taagtagcgt gggatgttgg ctttgtgaca ggtcttttgt cattttttcag
103381 aaagcatttt gacttgttga tgtcaatttg gaacagctga aaaaatacag gaaaataaga
103441 taaatacgta catgttgagg gtggggacaa aatgaaggtt ctgaaccagc tgccggctta
103501 cagtagccat ataagcaaca gcagcaatgc accaacctgg tgagtaatag gcctgattca
103561 ctggagagat actagcacct ttaatgagtc agatagatgc acaatgggtg tgggagcagt
103621 tggacttgtg ggcacaaagt ctagcaagaa gctcagactt gcaaacaact gtaggacgtg
103681 caaagcaagc tggcattgga gcttgccggg cacagctgct caggaatagg cagctggttt
103741 tccctttgat ccctgagatt ccaaaggtta ctttcctctt tgttcccttc ccagggtcaa
103801 ttagagtaga aactgcagat gcttttcagt tgagaatttt cctagaattc tcaaaaatgt
103861 gtatgctggc ttaaaatctg ccatcaagaa ttctgttacc ttgctttaag cctccagttc
103921 cttccagatg tatggtggag gaggccagag ggcccttgtt ttggggcttc agaggatggt

# FIGURE 3-CC

```
103981 tgttatctgg atgagcactg tggaaagact gagagagcaa ctgagagaaa gtgggcccct
104041 gaatgaaagt gatttcgcaa attttaggca gatgccacca tcagaaactg atattttctg
104101 acgtctttct caccttcctc tagagcattc agtccagaaa tgaccagcct gtccaaaggg
104161 ggaaattact gatattgatc tgttccttag agcagtgttt cagtcttttt tttttttttg
104221 agatggaatc tcattctgtc acccaggctg gagtgcagtg gcacgatctc ggctcattgc
104281 aacctccacc ttcctgattc aagtgattct cctgcctcag cctcccaaga agctggaatt
104341 acaggtgtgc accaccacac ccggctaatt tttgaatttt ttatagagat ggggtttcac
104401 catgttgcca ggctggtctc aaactcctga cctcaagtga tcctcctgcc tcggcctccc
104461 aaagcgctag gattacaggc gtgagccacc atggccggcc ttcagccttt gtgatattaa
104521 agcacagcaa cacatttccc attacacccc tgaacacaca cacacagaaa acccaaaagt
104581 ttcacaaaat gattcttgct cttactactc tcagtacact ctgtatttaa aaaaaaaaat
104641 gctggttgtg gcttcctaag tggtgcgtgc agttttcaaa tcaatgccct tggcgataaa
104701 gtgtgcccta tactgattat ctctggacaa agtctgaatg gggcttggct ctaatctcta
104761 gtcctcattg gacatttac atacctggcc tttgcctcca ccctgatgtg gagtgatcat
104821 gggggtggga aatatagctg gatccgaaag ctctgaagtg gggatggagg tgtcacagct
104881 gaggctaggc ccattctgca gggcactcag tgtgtacagt tggttttcta tcaggggtca
104941 accggcgggg ggacttgaga acagatctct gggcacaaag cagggccttt gccctggggc
105001 ttgctatgtg gctcagccta cacggctctc tccccgtcag tcctgtccaa agcccaggaa
105061 actaatgtac cacccccgag gaagagagcc tacctttcca tccaaggaag tgttttacct
105121 gtggtaagca cgggggacag aattcttgag gaaggagggt gctgcgtccc agtggtggag
105181 gaaaagagag gacctggtgt aagcagccat ggcatggacc tcatccgagg tggcacctgg
105241 ctagggtcct gacctccaat ccttccccag taaccatcac tttgagtaaa cagtggctcc
105301 acccccggca tggttctttg caccaacatt tggggaatgc ctaccagggg tcacacactg
105361 agctggatgc tgagtgtagg gtgtccacaa catcgtgcct aaaaagtctc tgtatggggt
105421 ataagaaggt gctggggcaa tacagatgag atgagaagca tctttcaggg aatgggttga
105481 tcccaattca ggcttcccag agaaggatgt ctgtagactt catattagca agggaggaag
105541 gtagccaggc cacaggactg ctggtgtaaa gaccagggca tatgaaatgg caagtgtgac
105601 tgtgctttca gccaataatt tggtattgtc aaatgatggg accaaacagc tggagaggca
105661 gatcctaaag ggtcctgtgg gccaggctgg acttcatctt gtcactaact aatggagagg
105721 ctctgaagga gttaaaagag ctcagtttgt ctcgtggtta aatccaagtt ttacaaaggt
105781 cacgctgact gtaaagtgga aggtgggctg gccaggggat catctagtct gggtgagaag
105841 tgatgataac atgaaggggt gaagagagat ttagaagaag tgattcacag gattaaacat
105901 ttaaataatg gaagtggaga aaatgggggg ggcggttcca gatttcaggc atagatgaaa
105961 gaagtgcagt taggcacatg taaagagaaa caggaacagc aggttttagg ggagaagata
106021 acagaatggg tgagaaatga cacttgagta ccctagtgtg ctaggtaatc atctgtctac
106081 ttcccttcat ttgtcatgta tattcccatt taatttgcat aaagacttcg agttaaacgg
106141 tcttacccca atttgtcaaa tttctgcgca tgatatggta caagaaaccg taagtggcta
106201 aggcggcatt ggtgttcaaa ttgcctgact acaaaggcag tgcttgttgg ctacattctg
106261 ttgcttccca gtttagaaca tgttacattg aggcgcctgc tgcatttcca aataaaaaag
106321 tacagaaaga aggtggctgt ataaatctgg ggctcacaaa gtaattttga ttactgagag
106381 tttgctttca aggagcaaac tgtgactcct tgattatgaa ccttaattta aaaaaaaaga
106441 aaaaagaagt cttactctta ttcctgcctt gtctggggca agccttaatg gatttttact
106501 gctgtgaatt ttctttttcat tgaagatttt gccttgatct atgtatctgc tttcatcctg
106561 accatattca agtcagtata ttcatgaatg tacctgtttg tgaaatttga acttaagtat
106621 acacgattat agccgtttgg gaagcttttt tttttttttt tttaagagta ggagtagaaa
106681 aaggtctctg tactctgaat gggaagacag tgtaaagcaa tttttccct tttcctgtcc
106741 tcctttaaaa aaaataaaca gccgtatgcc tctgctaagt actaactacc tcatcacctt
106801 ttgtgcagac agggcaggtt acatttggtt ttaaggaatt aggaatatgt ttctttccag
106861 caccttagta acccacgcga ttgtgattct tttctcttct tgactgtgat aggtggcatg
106921 gaatattcac atgggagagc cgcatgaggc cgcccaccac gcttcctgaa ggatgcccgt
106981 gtggaagaat tttgacgtgc cagtgtcctc gttctacagg gtgttccatt cttccgcaat
107041 ctcagaaaaa tgggactaaa agaaactatt ttgtaaaata agaagacttc catttttaat
107101 gaccaacatg tattaagatg gacacctact ctacgaaaca cgaagttcta tggtctcgaa
107161 gaagcccgtg cctgtttaaa actgatccta actaaaaaca gacttgagtg gatatgagaa
107221 tgttggttag tggcagaaga gtcaaaaaat ggcagttaat tattcagtta tttgctactt
107281 gtttttagc gagcctcatg tttttttggg aaccaatcga taatcacatt gtgagccata
107341 tgaagtcata ttcttacaga tacctcataa atagctatga ctttgtgaat gataccctgt
107401 ctcttaagca cacctcagcg gggcctcgct accaatactt gattaaccac aaggaaaagt
107461 gtcaagctca agacgtcctc cttttactgt ttgtaaaaac tgctcctgaa aactatgatc
107521 gacgttccgg aattagaagg acgtggggca atgaaaatta tgttcggtct cagctgaatg
107581 ccaacatcaa aactctgttt gccttaggaa ctcctaatcc actggaggga gaagaactac
107641 aaagaaaact ggcttgggaa gatcaaaggt acaatgatat aattcagcaa gactttgttg
```

# FIGURE 3-DD

```
107701  attctttcta caatcttact ctgaaattac ttatgcagtt cagttgggca aatacctatt
107761  gtccacatgc caaatttctt atgactgctg atgatgacat atttattcac atgccaaatc
107821  tgattgagta ccttcaaagt ttagaacaaa ttggtgttca agacttttgg attggtcgtg
107881  ttcatcgtgg tgcccctccc attagagata aaagcagcaa atactacgtg tcctatgaaa
107941  tgtaccagtg gccagcttac cctgactaca cagccggagc tgcctatgta atctccggtg
108001  atgtagctgc caaagtctat gaggcatcac agacactaaa ttcaagtctt tacatagacg
108061  atgtgttcat gggcctctgt gccaataaaa tagggatagt accgcaggac catgtgtttt
108121  tttctggaga gggtaaaact ccttatcatc cctgcatcta tgaaaaaatg atgacatctc
108181  atggacactt agaagatctc caggaccttt ggaagaatgc tacagatcct aaagtaaaaa
108241  ccatttccaa aggttttttt ggtcaaatat actgcagatt aatgaagata attctccttt
108301  gtaaaattag ctatgtggac acataccctt gtagggctgc gtttatctaa tagtacttga
108361  atgttgtatg ttttcactgt cactgagtca aacctggatg aaaaaaacct ttaaatgttc
108421  gtctataccc taagtaaaat gaggacgaaa gacaaatatt ttgaaagcct agtccatcag
108481  aatgtttctt tgattctaga agctgtttaa tatcacttat ctacttcatt gcctaagttc
108541  atttcaaaga atttgtattt agaaaaggtt tatattatta gtgaaaacaa aactaaaggg
108601  aagttcaagt tctcatgtaa tgccacatat atacttgagg tgtagagatg ttattaagaa
108661  gttttgatgt tagaataatt gcttttggaa aataccaaat gaacgtacag tacaacattt
108721  caaggaaatg aatatattgt tagaccaggt aagcaagttt atttttgtta aagagcactt
108781  ggtggaggta gtaggggcag ggaaaggtca gcataggaga gaaagttcat gaatctggta
108841  aaacagtctc ttgttcttaa gaggagatgt agaaaaatgt gtacaatgtt attataaaca
108901  gacaaatcac gtcttaccac atccatgtag ctactggtgt tagagtcatt aaaatacctt
108961  tttttgcatc tttttcaaa gtttaatgtg aacttttaga aaagtgatta atgttgccct
109021  aatactttat atgtttttaa tggatttttt tttaagtatt agaaaatgac acataacacg
109081  ggcagctggt tgctcatagg gtccttctct agggagaaac cattgttaat tcaaataagc
109141  tgattttaat gacgttttca actggttttt aaatattcaa tattggtctg tgtttaagtt
109201  tgttatttga atgtaattta catagaggaa tataataatg gagagacttc aaatggaaag
109261  acagaacatt acaagcctaa tgtctccata attttataaa atgaaatctt agtgtctaaa
109321  tccttgtact gattactaaa attaacccac tcctccccaa caaggtctta taaaccacag
109381  cactttgttc caagttcaga gttttaaatt gagagcatta aacatcaaag ttataatatc
109441  taaaacaatt tatttttcat caataactgt cagaggtgat ctttatttc taaatatttc
109501  aaacttgaaa acagagtaaa aaagtgatag aaaagttgcc agtttggggt taaagcattt
109561  ttaaagctgc atgttccttg taatcaaaga gatgtgtctg agatctaata gagtaagtta
109621  catttatttt acaaagcagg ataaaaatgt ggctataata cacactacct cccttcacta
109681  cagaaagaac taggtggtgt ctactgctag ggagattata tgaaggccaa aataatgact
109741  tcagcaagag tgactgaact cactctaagg cctttgactg cagaggcacc tgttagggaa
109801  aatcagatgt ctcatataat aaggtgatgt cggaaacacg caaaacaaaa cgaaaaaaga
109861  tttctcagta tacacaactg aatgatgata cttacaattt ttagcaggta gctttttaat
109921  gtttacagaa attttaattt ttttctattt tgaaatttga ggcttgttta cattgcttag
109981  ataatttaga atttttaact aatgtcaaaa ctacagtgtc aaacattcta ggttgtagtt
110041  actttcagag tagatacagg gttttagatc attacagttt aagttttctg accaattaaa
110101  aaaacataga gaacaaaagc atatttgacc aagcaacaag cttataatta atttttatta
110161  gttgattgat taatgatgta ttgccttttg cccatatata ccctgtgtat ctatacttgg
110221  aagtgtttaa ggttgccatt ggttgaaaac ataagtgtct ctggccatca aagtgatctt
110281  gtttacagca gtgcttttgt gaaacaatta tttatttgct gaaagagctc ttctgaactg
110341  tgtcctttta atttttgctt agaatagaat ggaacaagtt taaatttcaa ggaaatatga
110401  aggcacttcc tttttttcta agaaggaagt tgctagatga ttccttcatc acacttactt
110461  aaagtactga gaagagtatc tgtaaataaa agggttccaa ccttttaaaa aagaaggaaa
110521  aaactttttg gtgctccagt gtagggctat ctttttaaaa aatgtcaaca aagggaaaat
110581  aaactatcag cttggatggt cacttgaata gaagatggtt atacacagtg ttattgttaa
110641  aattttttta ccttttggtt ggtttgcatc tttttccat attgttaatt ttataccaaa
110701  atgttaaata tttgtattac ttgaattttg ctcttgtatg gcaaaataat tagtgagttt
110761  aaaaaaaatc tatagtttcc aataaacaac tgaaaaatta tcatgagatg tgtatttaaa
110821  ctttttcatg aacattgctt atataatcat tccttctgtc ttaatgtact acatggtctt
110881  agccctgttc ctataggatt atcatgttct ctgcattata gagccaccta agatgtactt
110941  tttgttaaat gactcatgct ggaatatctg gatggggaga tgttctttcc taatgtagtc
111001  atgtgccaca aaatgacgtt tcggttaacg atggatcaca tatatgatga tagtcccatg
111061  aaattgtaat ggaactgccc tatacaggtg taccattttt tatcttttat ttcagatttt
111121  tactgtacct tttgtatatt tagatgtgtt tagatacaca aatactttcc attgtcttac
111181  aattgcctgc agtattcagt acagcaacat gctgtacagg tttctagccc aggagcaata
111241  ggctctacca tatatctagg tgtctagtag gctattccat ctaggttctc gtatgtataa
111301  cctgggatgt ttgcacatcg atgtggtcac ctaaagatgc atttattggc caggcgccat
111361  ggctcacgcc tgtaatccca gcactttggg aggccgaggc aagtggacca cctgaggtta
```

# FIGURE 3-EE

```
111421  ggagtttgag accagcctgg ccaacatggt gaaaccccat ctctactaaa aatacaaaaa
111481  tcagccaggt gtggtggcac acaccagtaa tcccaactac tcgggaggct gaggcaggag
111541  aattgcttga acctgggaaa gggaggttgc aatgacctga gattgtgcca ctgctctcca
111601  acctggacga cagagcgaga ctgtctcaaa aaaaaaaaaa atgcatttct cagaacttat
111661  cctcattgtt aagcaatgca tgactataat ctgttgagag agggatgaaa tcacctgtag
111721  ttatagcgct ttaagatacc atttgaaaag gttacgtttt ccttttcttt gacacggtta
111781  gctgtctgaa atacagtcaa ttttaaccct aatctcttaa tatcaggaat gcccttacac
111841  ctactttgga gtgtctggtg cttcgatata gttgcatgta atgtgctctc atctgttttt
111901  acctgattcc tgctcagttc ttcacatggc atattgtgta actcaatcta tatttaaaac
111961  ttgtaagcat ccgaattatt tgtttatggt agaacttttt acttgcaagt cgtggtagga
112021  gtgtttgtag ttggtactaa aatgtgatga cttggaagaa ttaattaatg acctatattt
112081  ggggacttta attggatgct atagctgcaa tgagaataga accagagaac tcttgatatg
112141  caaggttatt cattctgtga taataatgag aggaatattc gatgtctctt tgagtcagtt
112201  ttccttccga tcacttccgc attctgcagt gacacaatcc ttaatcatag ctttcattac
112261  aatattcctt tactccagac ctcaaagact cctcactgcc tccagcatca aatctaaact
112321  cttctacctg gttttcaacg ccctacgtaa acttttcctc cttcattccc tatagcttgg
112381  tttttttttt tttatcactg ccactattat ctatcacaaa tgtcaatcat gaccatacct
112441  tgcttaagtt ggtttccctt gccaagagca ctgtttttcc tatacctgtt gaaatttttgg
112501  aaatccaatt ctacctcctc tcttccctta agcatctctt ccttcccttc tccccaaatt
112561  tatatttagt cacatatatt atcgtactac ctactaatca ttccatgtgt tttttttacga
112621  gctgtaagtt ctgaaggcaa ccatgccttg tacagtgtcc acttagggtt ctgaataatt
112681  aatcatctcc ccaaaatctg aaagccttct atataccaag caaatttgtt tagttatgca
112741  gcaaaactca aatctataaa atcaaaaagg aataaggaaa tacagattaa acagttgcag
112801  caaagactgg tgatcttaag gtatttagtc aaagctggtg gtagaacaaa aacagtagtc
112861  ttacagattc tacctcttga ttaactcagt ggctaatttt gccttttctc aaagttcttt
112921  tgcaagaaca taaagatatt tttgtttctt tagttgagtg ctgtaacttt attcctttgt
112981  gtttctcata agtatgattt ggcagtctgc catacgtttt ttgttttttt tcttcctctt
113041  tgagacaggg ttttgctctg tcacccaggc tgcagtgcag ctgtgtgatc acagcttagc
113101  tcactgcaga cttagcttcc tgggctcaag caatcttctc acctcagtct cctgagtaac
113161  tgagactaca ggtgcacccc accacatccc gctaaatgtt ttaatttctt gtggagatgg
113221  tgtcttcact atgttgctca ggctggtctt caactcctgg gctcgagcaa tcctcctacc
113281  tcagcctccc aaagtgttgg aaagtgttgg gattacagac ctgagccacc acacctggcc
113341  tgttataagt taatacaata attcatcaac taacttaaag aacactaaga ctcttacaaa
113401  agtaggtatg agttttagta aaagtctcaa aagataaact gtcacttaag gaaaactaga
113461  gaacatatca ttgccaaatg gtgtttttca gagattatac cattcaacgc ccacatgctg
113521  aattgggcca ttcattataa ctccaggaac atggcaatca gtaagagccc acatgtttct
113581  ttgaatacac cgtaagtgaa agaatataaa gtagtctagt taatattatg tttaatcaag
113641  gagcacattc ctaaagatgt ttgttcattc attctacaga catttttcag aggcctgcta
113701  agagtcaagc attatgatag acgccatgga taaaagctta caagtcaacc agcgtgggta
113761  taaataatgt gactagcact agaacaggta tcatgatggg gattctgagt ataaatattt
113821  ttttaaaata aatttccccg tgttattttt ggcttttacc tccctaattt aggctttcta
113881  aatggcacag catttctgag gatgcaaaca cctttctaca gagcaaaaac agcatttgta
113941  taaatttgtg tctttgggga accaagagac tttaaatgtg tttaaaccaa taattcagtc
114001  aatatcaaca ttagcttaca tgtaatattc tcttgatagc ccaatttttt aaaacactgt
114061  attcttagaa gtttggtttc taagatgtca ctttaagctc ttttgcttgt tgcttttgtg
114121  ggatccacaa attttgttct caggtacata aatgaaggtt agtatagagg ataaatatta
114181  tgattcttat ctgggaaaga caggtgctga ggtgtaaaag agaggatcct cgccacccat
114241  gccccgcacc ccctcgcccc ctgcacccac ggatgtgcag tcttacctgc ggggggaaag
114301  gtctccgagc ctggcctgct gctccagctc agggttcccc ctttcatgat ggctttcaaa
114361  gatttcttca ctctgaagtg aaagaaattt tggtaagatt tgatattgta gggacctcct
114421  aatctatatt tttctctctc ccaatttctg tgtttgattt tggttttgag tctctcgata
114481  agcaaaatat ccagtttctc atgccgcttt ctcaggtttt ccccagccac tctggatcca
114541  ttatggtttg cccttttttgg cttcctttag gcacactaaa aactccttcc caaaagcagg
114601  tatcggccgg gcgtggtggc aggcgcctgt aatcccagct actctggagg ctgaggcagg
114661  agaattgctc gaacctggga ggcggaggtt gcagtgagcc aagatcacac cattgcactc
114721  cagcctcagc aacagagcga gatgccatat ccaaaaaaaa aaaaaaaagc aggtgtcctt
114781  tccccttaat catgaagggc tattcatact ttactgcccc acccctattg attcataaga
114841  ggacagtaaa gcgatcactg cattcaacat ctcctttttt tttttcttgt aagaaatcaa
114901  ggtctggaaa agttgcactc gccctgagac cagaaagtgc tggaggcaga gatgaagtaa
114961  gcccagtgta ggctttcaca gatgcgtgat aacaagtcta actaaagaag tacctgggat
115021  actagttttg ccaattcagc tctaaaacaa tatggcaatc ttatattcca aatatatata
115081  tatatatttg tatttatagt agagatgggg tttcaccatg ttggctaggc tggtcttgaa
```

# FIGURE 3-FF

```
115141  ctcctgacct caaatgatcc actgacctca gcctcctaaa gtggaggaaa catatatata
115201  tgttttcctt ttaaaatagg atgtcagtcc aataagaatc taaattttag ttccctctaa
115261  tatatatatc tgactaggga ccagataata tttttcatgt gtcaatatat aaaagttggc
115321  caggtgcagt ggctcatgcc tgtaatccca gcactttagg aggctgaggt cagtggatca
115381  tttgaggtca ggagttcaag accagcctag ccaacatggt gaaaccccat ctctactaaa
115441  aatacaaaaa ttagccgggc atggtggcag gcacctgtaa tccagctatt tgggaggctg
115501  aggcaggaga atcacttgag cctgggaggc agaggttgcg gtgagctgag attgcaccac
115561  tgcactccag tctgggcgac acagtgagac cctgtctcaa aagaaaaaat atatatatat
115621  atatattttt tattatattg tttattaaac aaataaaaat aaaagatatc tcatcagtta
115681  cgatgtaaat gaaaaaattg tgtgtgtgtg tgtgtgtgtg tgttatgatg tacttcttgt
115741  gagttgtggt caggctttga aagccactat gtatgtgtgt gtgtgtgtac ataaaagtaa
115801  gtatcagtcc caggattcaa attcgaacca gtcccagtca aattcaatct aattattttc
115861  accacactac caaaagtctt tcttcacatt ttctatataa agttgaacta attaatacag
115921  cagtgaatgt tacattgtat ccttttgcag tttctcatct atgacattac tatgcctgaa
115981  ttccccactg gactttgaac tcagtcttac tcatctttgg atccccagcg tgtaatacag
116041  gcccttcata aagagtgacc attattacta acaaaatttc ctctcactgt gaaacctgct
116101  cccaattata aaatgaattg tgctctttcg aactccgttg atctattgta tgcagggtgg
116161  atcatagagt cttatttcat aataacctag tgatggaaaa ataacaatga ttcaggagta
116221  ggtaatggac cttgtcatct tttacactga atgatgagat ttccctaatt tatagatttt
116281  gtcatgcatg agatgccctc aggagcttgc agaaatgcct tggcacgttg ctctgctgac
116341  atgtgtcaga tggctggtgt ggaggtagag ggagtctcct ctgccaagca agtctaatgg
116401  aatataacac tggtgccatt gaggatgcaa tgagaaggac ccacagcaga tccagagact
116461  gcattcataa aagctgcaca gtataccatg ttttattaag gtatagacag attagttgtg
116521  ttctagcata gtggttttca aagcctgacc acaactcaca agaagtacat aacacacaca
116581  cacacacaca tacacacaca catttttca tttacatcgt aactgatgag atatctttta
116641  tttttatttg cttaattttt tatttctttt ttttttgaga cgaaaggtga attgtagcca
116701  aagtactttt aggaaatttt aaaattacac attttgaaag ctctaggagg aagagagcat
116761  attcaagtaa aacctttctt ttattcaaat agtagactaa aaaattgacc atagactaac
116821  tcagcaattg ctgccatatt tcttagtgag ggattcctta tgagctccat tgaatgataa
116881  aaggatactc ttccaaatca gagcaacaat cgtttcttgt gacactgcat ccttttttccc
116941  ttccttcacc ctgaattgcc cctagggtat acctaggaga gggagggctg gcaaccagct
117001  gctgttgtga aagggatgtc attcatggcc tgccctattg ggaggccaat caggcggaac
117061  agcccctctc cccttatggt cattttatcc tgaagaaaaa gggtggcaaa aagatggcaa
117121  aaaagattgc tgatcacaga tcactgttac aaatacaaca attggctggg catggtggct
117181  cacgcctgta atcccagcac ttcgggaggc cgaggcgagt ggatcacgag gtcaagagtt
117241  caagaccagc ctgaccaaca tggtgaaaac ccgtctctac taaaaataca aaaattagcc
117301  gggcatagtg gcgcatgtct gtagtcccag ctactcagga ggctgaggca ggaaagtcac
117361  ttgaacccgg aggtggaggt tgagtgaacc aagattgtgc cactgccctc cagcctgggc
117421  gacagagcaa gattccgtct ccaaaaaaaa aaaaggaggg ggtggcaatg agacagggaa
117481  ctgctctggg tccaactccc ctcacatagc cccaaccccc atatccaccc cacaaacctc
117541  cacctcccca cttccagcct ctccagtttc aaagtgacgc ttaccaacac gcaggctctc
117601  caggagcacc ttgaaaggta acttcttaca tctttccaaa acacttataa tctaattgtt
117661  ctttctcccc tatattttga agaaatgcat gctctcaaaa ttgaaaaaaa cgcctagagg
117721  aattttgtaa aaaaattata tttttccagt ttctccaaag gacatataaa cccttttttaa
117781  acttagattt tgaaaagtcc caatagctga ttttcaatcg attattgaaa ttgttttttt
117841  tttcttccct aaagggggaga taccaacctt tgaacaaaat taaattaact tttccccaaa
117901  gttaaatgat tttactttgt gattttagga atgtgtagaa agtgatttca gttccaactc
117961  ttttaaagca agggtatctg ggccaggcgt ggtggctccc tcctgtaatc ctagcgcttt
118021  gggaggccaa ggtggggcag atcacctgtg gtcaggagtt cgagatcagc ctggccaaca
118081  tagagaaacc ccatctctac taaaaacaca aaattagctg ggtatagtgg tgcgcgcctg
118141  tactcccagc tactcgggag gctgaggcag gagaatcgct tgaacccggg agacggaggt
118201  tgcaatgagc tgagatcacg ccactgcact ccagcctggg caacagagtg agtctctgtc
118261  aaaaaaaaaa aaaaaaacaa acaagcaaac aaacaaacaa aaacaagggt atctggtaat
118321  ttaaggtgaa agtattattc actattataa tcaattttct taaaaacagg agctattagg
118381  cccaattctg aggagagaaa aaaaagcaga tttcaagggc ccgtgccacc ttctttgttt
118441  aaagcataga gaatgataat aaaatgttaa aggttcagca ttttctggca ttgtaaattt
118501  aattaattaa ttatgtaaat taattatgga aatgagaaat aaagtgaaaa ggtatgtcaa
118561  gtaagaatca ataattatca aaacgttctc taccaaatgg gatgcagggg agaagggtga
118621  gaagaaggga aggaaataaa ggcaaaggca tttgcccaca gcctcagtcc agggtgttct
118681  acatgacgca gccccagagt aaaaaatact agccacagga atgagtggct ctgtcttcct
118741  ctccagaact gacgggcaaa agagggggaa cctcaccttt tttttttttt tttttttgga
118801  aactcactgg gttttgatat ggagaacagg aagagaatct accctagcag cacattcaca
```

# FIGURE 3-GG

```
118861 gatggtttgt ctgccctatt tgctccatct ccctggctct tcctagcact tcttttccta
118921 ctttccattt tcattggccg caaaagccac ttaaatattc ctttggaaaa ctgggcagtc
118981 tgtccccttt ttaaaaccac aactatttcc aggagagtta aagggctttc ggactgccca
119041 tcattcatgc attgtggtga actagctcag actgcctcta cctttagtcc agatcagctg
119101 ccttgggagg gcgattcctg gaggaaagga tgaggagagg aaggttttag gacatcgtat
119161 ggactgaagc cccgttggag gggaagggca ggcttgagga acaagcctca gtgttcctga
119221 ggctttgtgg aaatgagaaa tgaatagaga aagataagag actggatcct aaatgcaatt
119281 ttgcctattt caaaattttc acagggtctt tttttttttt ttttttttgag acaaggtctt
119341 gctctgtcac ccaggctgaa gggcagtggt gtgatcatag cttcctgaag cttgcagcct
119401 cgaactcctg agctcaagcg atcatcctgc ttcagcctcc caaagcgctg ggattacagg
119461 catgagccac cacccccagc ctcagggtct ttttaatccc taagatctta aatttcccag
119521 ctactattgt tagtaatgag ttcaatttac ttactggtta aattggcttt tattattatt
119581 attatttatt ttattttatt ttattttatt tgagacagag tcttgtgctg tcgcccaggc
119641 tgtggtacag tggtgcaatc tcagctcact gcaagctctg cctccctggt tcatgccatt
119701 ctcctgcctc agcctcccga gtagctggga ctacaggcac ccgccaccac gcccagctaa
119761 tttttgtat ttttagtaga gacggggttt caccatgtta gccaggatgg tctcgatctc
119821 ctgacctcat cgtctggact aatactctaa acgctattgg caatagtttg tttacaggaa
119881 aaacatcttc ttataaagct gactgcaaat gtttaataa atttgcaaat atgtaattct
119941 gtttaaaata tcaggaagtg agaaacatgt tatgtgataa ctctttccca ttcccgaacc
120001 aagaaaatgt aaaggcagta agtgtgccaa ggacactgaa aggaagctgc ccgtacatct
120061 ttgaatcttc tcaggctgtt tggtttcatc tgattttaag ctccatggat aaatttcatt
120121 gtaacaattt ttatgtctgt aaatcttaac ccataaataa aatcacaaat cagaaaagta
120181 ctttattagg ccagtctttg tccaagcaaa tttggtccca aagctagttt acaaataatt
120241 gccacacagc tacaaggcca gtgggttgac tatcgtagca tcaatggtgt gggcagggct
120301 gcagcctctg gggcagcatt agccccaact gacagagggt ataggtgctc ttaacaatct
120361 atgagacccc ccacaacctg gactcagctg tcataagcct ttacttctta tgttcttcct
120421 aagtacttta tcgggccaac aaattcatcc catgaggaat ccaagatgga aacgtcaaaa
120481 ctatctttgg tatcatgctt cctcccccta ccttctctgc cttgatcccc tccttcattc
120541 tacacatttt ctctgatagt cgtgttctag ccactaggga gaacgtttca gtcaatgggt
120601 atgatttctg ctcacctctt tttgccatag ctattagtaa gctctcacca ctgatgtctc
120661 agctgactgt gacaactgcc agcggctggt cagcctgcct gccttcatct aacctgctct
120721 ttactgtcta ccagagtcag cttcgtaata cacaggtgcc ctcctcctcc tgtcaacatt
120781 cctcaaccaa agcctcagta aactgtctct catctacctt cccagcattc ttgagccatt
120841 cccaggatag cctaggccct ttagacctct gagatgttat acccttcctc attctggaat
120901 gcccttcagt gtcttaccca cctttcagag ttctatttat tctttttttt tttttagatg
120961 gagtctcact ctgtcaccca ggctggagag cagtggcacc atcttggctc actgcaccct
121021 ctgcctccca ggttcaagtg attctcctgc ctcagcctcc tgagtagctg ggattacagg
121081 tgcccgccac catgcccagc taattttgt atttttagta gagacgaggt ttcaccatgt
121141 tggtcagtct ggtcttgaac tcctgaactc aggtgatcca cccacctcag cctcccaaag
121201 tgctgggatt acaggtgtga gccaccacga ccagccttat ttattcttta gagttgaatt
121261 caaatgccac ctcctttgaa agccatcctt gatgtccttg tgaggaatta ctttctctac
121321 cacattatat attcctttta ctgtagaaac gtcattctgc tttatattat aaattattag
121381 aaatatgttg tttcccccat gaaagtttca agtttcctga gtttaggcct gatgatacaa
121441 tcattttcat atgactcaca gcaattgcct tagtatatat taaattgagc tgaataagcc
121501 atgtacccag ttgaacacac caaatatttt agtgatgtca tttctttatt ggtgaaagag
121561 caaggccaac gttgatctta atgttattct ctcttttatc ttattcagct ccctcaacaa
121621 aaatacattc tcatagatta atgtaatcac cacctcactc ctctcaactt tagaccctat
121681 actagcctga gaaatccaga tccaacttaa tgagttctcc tttatcccaa gtcctcatga
121741 cctataaatg taacctctag gacaatgtta caaagagtga gatctttata ccacctgcat
121801 tcttgtcacc aaacgtgtaa gttaaaaatt cagattctga gtgtggtgac tcacgcctgt
121861 aatcccagca ctttgggagg ctgagccagg tgggtcacct gaggtcagga gttcgagacc
121921 agcctgggca acatggtgaa accctgtctc tactagaaat acaaaaatta gccaggcatg
121981 gtgacaggca cctgtaatcc cagctactct agaggctaag gcaggagaat cacttgaacc
122041 tgggaggcgg aagttgcagt gagctgagat cactccatgg cactccagct tgggcaacaa
122101 agtgagactc tgtctccaaa aaaaaaaaaa aatttagatt ccaggacccg accctacacc
122161 tccctaatta gaatctcaga gagtggggcc ctgggaatct gcataattca taaccttctc
122221 agagctctgg tacacaagaa accatgagaa tctttgttct ggaaacttca gagaacttgg
122281 ctgagggcca ccccaggatt tggtggcgtc cctgaatctc ccattagctc tgacactgac
122341 tagacttcaa atatcacaga aggcaagcat tgaagtgtgt ttatattcaa atagttttct
122401 tgttgagaat cagaaatatt aataaaactt ttgggagtca aagtaatgag aagacaagga
122461 actgaacttg ccccctgatc tgttatcatt tggcagtaag gtacatattc aacaatagga
122521 taattctgta taagacctga atctgacctt ctttcatctt tcacactgac aattctgtct
```

# FIGURE 3-HH

122581 ctaaacacag gattaaatga ggaactaaaa ttcaccaaga tcctgctagg tgtcaggtac
122641 agtgctagct gcttccacac atttctcatt taatcagcca cctgtgaaat tgctatgatt
122701 tatcaccatt tggcagaaaa ggacatttag ggcacaagag attaaataag ctgctggttg
122761 ggtacattaa gtattaataa ataatcagat agataagtta ggtagttaag taagctgcca
122821 gattagtaaa cagctagttt caaatcccag ctccaacatt tactagttgt atgacccttg
122881 gggaaactca acctctgagt ctcatggcac tgctgataaa acggaaaaca tcatcctcac
122941 aaggttctga ggactaggga tggactcagg tacacacagc acccagtaca atgtctggcc
123001 ttcagccagt gatagcaacc cttcactcac ctctccattc ccttccagcc cctgcattca
123061 aaaactttta tttatttatt ttttaattt aggaggctct ttgctcagaa tcctgaaaag
123121 gcttttgtta ctttattttt tcttttttt catcttctgg gagcacagaa taggcttctg
123181 ctcctttaaa taactttaac aggcaccaaa ggaacactgc tagctctttc ttaattctgt
123241 aggtccacat ttaggaaaaa gaaattgtca gcctctgact tatttccagc ttacaaaaag
123301 gctgtgatgt tggcagcccg gggaaagcag ttcccgtgag tcatgctttc acctttcagc
123361 cagtgaagaa caggaaatag tcacatcact attgccaaca agcacagcga atcgcttcca
123421 ccaccgggct ttcccagatg acgtcagtga gccaagtgca gggcatcacc cttgccagat
123481 ggccccggaa gagtctcagc tgccctgtca agtttagctt ctcaagctcc ccagaaccag
123541 catggcaagg atcacccctc cagaaaagga aatgatttct ctgatcatga tcaaaccatg
123601 tcataatttt agctgtaggt ggtgagtatc agtgactgta ttatagatgg ttttggttca
123661 cagctatttt ttttctcggt atatttactc tcaagggcaa aggggtggca tttatcacaa
123721 tgctatgatt cactctacta gactggctgg gtttcatttc atctttgagt tctagaccta
123781 aggccaaaag gatgtcagaa tcgccaccca gagctagatg catgctcaat gcaacctggc
123841 catctctctc ggacagtggc cactaaacac aagctgcaat gattattgtt gatgttagtc
123901 aattgatttg tccccctttt aataatccat gatccacaca gacatgaatc taaacctttt
123961 ttttgaacct atcttttttct tactagtttt cttacatatt ttggaataac aatttccctg
124021 gttataatac acaatgtgtg aaacactaat tgcatttatt ggtcctaaat tcattcccaa
124081 gtctaggatt tggggattta gtgcacaaat ccactttctt cctgctcatg ttgttcatga
124141 ctttatggat tctgcttaca tttcctccca aagccttggt ctttctaagc agaaatctga
124201 ccagcctctg ttcctactct gcttcctcca ccaccttgac tgttaacata cactgttgtg
124261 ggagaagctt ttcattaaat acgcaaacaa atcaacaata aggaagaaaa caactgaagc
124321 acagaaggat gattgtaaca tggacttgtg cttgtttaac agtacccata ctgtttggtg
124381 agttgcctaa atacagaggg agatggcaac ttaacaggat gggtttggag tctgaattaa
124441 ataaacaaca tggcaaaaga aggaagaaac caaaagttgg gtgactaggg cttcttggat
124501 tctactcaac actttccaca cctacaggcc tctgattcta tccccaccgc tgctcctaca
124561 ctgtctatgt ttctctttta caacaacaac aaaaaaatag cacttgttta ctgttcacaa
124621 ttattgaaat aaatcactct aatttggatt cctttatatg agaacttcca tatgcttaaa
124681 actcccgtag cattttttata gtgtacaggt cataaacatg ttgtctgaag atgggcaaag
124741 taggtcgtag agataggaag tgagttcaca tcacggcgtg agtcaaggca gcagcatatg
124801 agctcagggg ccaaatctga gctgtgatca tgtgacacag tcctcacctt cagagatgac
124861 attctcccag aagtccaggg tgcagaaatg gataggctga tgttcacttt tgaagaggac
124921 cacaatgaaa ccctttttaat attactcaat gattccacag acctcctggg tgttaaatca
124981 ggtgaatcta tttttaaaaa ttgctattta cttttttagg tggctgatca ggacagtatg
125041 aaatttcata cagtttccta acttgagaaa acatggtgat gcaattcctc caaccaggag
125101 ggatttatgg acagcggtgg ctacgtccta atctgcccag aatacaggat gactaaacaa
125161 attggcaaac ggacgcagct ttctctctct ctaagaaaag tctgctgaga accctcgttc
125221 ccactctgtt tctgcctcca agaagaaaag cctaaaactc actttcttcc ggactctttc
125281 aaggtcagtg gagttcctct ggggttcata ttcagatact ttggggattg atgatggatc
125341 ataaatgttg ctgaagttca tttgagccca tggcctctgg tttcagaacc attcagccag
125401 tcaaatattt aaatttttagt gaggcagggg aaagggaacc ccttcggggg ctgctataca
125461 gcaacttata ttcacaattc acaattaaat acacttcagt ttctaaatat atgttacttt
125521 aaaattatac agtgcttagc aattttcaaa ttttcatttt gtgctcaaaa tattttcagg
125581 aggtagctgt tgttataccc atttgtacaa gcaagaaact gaggtttcaa gaggttatgt
125641 tgcttaaacc cagatctgcc tgattccaaa ccttatgctt tttttaaact tctattttga
125701 aataattata ttaagtcaca tgaggttgca aagaaatgga aagtctcatg tccccctttct
125761 ccaaacctca tccaatgtta acatctaaga aattgacact ggcacaatcc acagagccta
125821 ttcagggttc accagttatt cacgcacttg tgtgtgtgca tgtgtgtgtg cagctctgtg
125881 caattttgtc atgctctttt ttttttttt ttttgagaca tagtttcgct ctgttgccca
125941 ggctggagta cagtggcaca gtctctgctc actgtaacct ccaacttcca gtttcaagca
126001 attttcctgc ctcagcctcc tgagtagctg ggattacagg catctgccac cacgcccatc
126061 taatttttgt atttttagta gagacaaggt ttcaccatgc tagccagact ggtctcaaac
126121 tcctgacctc gtgatcttcc cacctcggcc tcccaaagtg ctgggattac aggcgtgaac
126181 caccgcgccc ggcctgtcat gctcttttta tacatgaacc atacactgtt ctgccaattt
126241 taaatatgaa ggcagattac agaaataaat aaaatgattt ttcttttact acaacagtat

# FIGURE 3-II

| | | | | | | |
|---|---|---|---|---|---|---|
| 126301 | ctaccaataa | tcacatacat | gaccaaatag | ctttcacttc | tagctgccgt | taagaagaaa |
| 126361 | gaaaaggatg | aaaagaaaaa | aaatcctata | aaccccagtt | cttgaaagca | ttagtctgtg |
| 126421 | ctatgtgcct | taggtacaga | ttaaggaaac | acaatttgtt | gatttattga | taattgtgac |
| 126481 | agcaatcttc | cctcttgtca | ggaagttcta | taagtaaaat | aaaggtaatt | ttaccttgac |
| 126541 | ttcaaattta | gtctcatcat | cttcctttcc | ccgggagttc | aactctgtct | ccgtataagg |
| 126601 | ttcctcagac | gttctcagag | gacgagctag | agggactaag | agaacctgcc | attagtgtgg |
| 126661 | ttatttaact | tataaactat | aactcagtgc | actttgtctg | atttggacaa | atagctggac |
| 126721 | cacgtcaatg | tggctaacta | taaaagctca | cttgagctgc | tgcgttgatt | tcctagggct |
| 126781 | ggatgaaatt | ggaggtgaag | gagagacaca | gaggagaaat | agaatccttg | actccaggga |
| 126841 | cttgacattt | cagcagaaga | gaaaaaagtc | acacccagca | ccatatgaaa | cattaaacta |
| 126901 | caacaaatct | gtacatatag | caatgtggta | aagaatagta | gatttctgt | cattcccatt |
| 126961 | tttaaggccc | agctaaaagg | tcacttttgg | ctgggtttgg | tggctcacac | ctgtaatccc |
| 127021 | agcactttgg | gaggctgagg | cgggcagatc | acctgaggtc | aggagttcga | gaccagcccg |
| 127081 | gccaacatgg | tgaaatcccg | tctctattaa | aaatacaaaa | attatccggg | aacagtggca |
| 127141 | agtgcctgta | atcctagtta | ctcgggaggc | tgaggcagga | gaatcacttg | aacctgggag |
| 127201 | gcggaggttg | cggtgagctg | agattgcgcc | actgcactcc | agcctgggca | acagagctag |
| 127261 | actccatctc | aaacaaacaa | acagacaaac | aaacaaaaag | gccactttcc | tcatgaagtc |
| 127321 | ttccatatta | caaactttat | ggattctttt | atgcactgct | gctttggcat | gcatcatgtg |
| 127381 | tacagcataa | tgttgcactt | gatgttgttc | ttggattcaa | agactaaatt | ctaggtacta |
| 127441 | cattgtattg | aatatatttt | ccattataaa | ggtaattaat | ttttgcttat | tacagaaaat |
| 127501 | ctgaaaaaca | ctaaactctt | aacgctgaag | cacaatcact | tagtatattt | ccttacagtc |
| 127561 | ttctttcaaa | tgctctccct | cttttacac | acatacatgt | acacacacaa | tcatactatg |
| 127621 | attgtattgt | aagaggatgg | ccatgcctgc | tgctcttgct | cttttctctt | ggccactcta |
| 127681 | tctcccttct | ccccacctgg | gaaatatcct | ttcttcaagc | tccatggcca | tttggtagta |
| 127741 | aaattgggac | ttgagaggag | aaggcctcaa | ggcttcctaa | cccaccttac | cagctttgcc |
| 127801 | aagcattgtg | ggtgatggcc | acaaggctaa | tagataagag | gtactttgaa | ttcttatttg |
| 127861 | tcacagtcac | caccccttgca | tatgctggtc | ccttgggaaa | ctcacaggag | acatgattaa |
| 127921 | tcccaggcag | gatttgagca | tttctttctt | tctttctttt | tttttttgag | acagagtctc |
| 127981 | gctctgtcgc | caggctggag | tgcagtggtg | tgatctcagc | tcactgcaac | ctccgcctcc |
| 128041 | caggttcaag | cgattcttct | gcctcagcct | cccaagtagc | tgggactaca | ggcgcgcacc |
| 128101 | accatacgca | gctaattttt | gtatttttag | tagcgacgga | gtttcaccac | gttggacagg |
| 128161 | atggtcttac | tctcttgacc | tcatgatctg | cctgcctcgg | cctcccaaag | tgctgggatt |
| 128221 | acaggcgtga | gccactgtgc | ccagccaatt | ttagcatttg | taggtcccaa | gaccaaaatg |
| 128281 | ccattcattg | aatgccacca | atatatcacc | ctgattctta | gctatggtag | acttgatgga |
| 128341 | ggttgaccat | ctgatctcca | gaggcccctt | ccaacccttc | cattctgtga | gccaactgga |
| 128401 | agcagcttgg | gctttctatg | ggtttttcat | agatgttatg | ttgaaaatcg | cagaaaatta |
| 128461 | cactgctacc | ccaggttata | catattacca | ttaatgctgc | tttgtaataa | cagacaatcc |
| 128521 | tttgggctcc | tccctctctg | tgggatctct | ataaagtgag | tgattccagg | cacaataact |
| 128581 | agatgctaga | aatgcatgca | ctataatttg | tgacaccatg | actgagaagc | gcctgtcctt |
| 128641 | gacactgtaa | gtagagtgaa | gcagtgaaag | gcggggccac | agaagccaca | ctgccagtgt |
| 128701 | tcaaaccttg | ctcttccatg | cacgagacaa | acatgattac | agtcacctct | gtaccttacc |
| 128761 | tttttcatct | gtaaagtagg | gaaaaatgag | aaatcctaca | gtaaagggct | gtgataaggc |
| 128821 | tttaatgagt | tcatttgtga | agaatgtaga | tcatatacaa | tgcataacac | attttagcta |
| 128881 | ttattgccac | atggcactaa | aaagtttttt | cacgttcctt | atgaacttca | gaggtgttag |
| 128941 | aagttgtagc | caaggcatcc | agctaacaag | agtcagagct | gaaattccag | ctcttaagcc |
| 129001 | cctccacaca | tcttctttat | actgtgaaat | acaattgctt | atcttggcac | agactcagtt |
| 129061 | actaaggaat | cagacaaaaa | tgtttgaaaa | tccttttagt | gacttgctgg | agtccacact |
| 129121 | cagtagatac | ttaccaaaaa | caaaagtgca | ggattgttct | gcaaaacgag | ctagttgttt |
| 129181 | gacttaggtc | aatgacaacc | tgttcagact | tgacaaagac | ctatttattc | tgggaaggag |
| 129241 | tgttatctat | tatgaaatct | tgtatttgaa | aagctgagaa | acaaccctgt | aattcctgct |
| 129301 | ttatttactt | gaaacaaaca | tatgtacctg | gaaacaacga | atgaggccaa | gtcatagatg |
| 129361 | tgaacatagt | ttagcttgag | gacagacgaa | aggtcaagag | aaccaagttc | tgtttccaat |
| 129421 | catctaagaa | ggatgtctct | gtgtatgttt | gggtatgtct | gtggtatgtg | tgtgcaagtg |
| 129481 | tgtgagagtg | tatgtgtgtg | gatgtgtgtg | tgtgtgtttg | cagataaaaa | ttcaggaaag |
| 129541 | aaatacacta | aatgttatca | aggcttactt | ctaaaaagta | ggatacatgg | tggaagacaa |
| 129601 | gggatcgtaa | attgtactaa | atggagaatt | accaaaacac | cccacaattt | tcatataaac |
| 129661 | aaaaactgaa | agaatttatt | gctaacaggc | caggcatggt | ggctcacaac | tgtaattcca |
| 129721 | gctcttaggg | aggcagaggc | aggaggatag | cttgagccca | ggagttcgaa | acctgcctgg |
| 129781 | acaatatagc | aagaccccgt | tctccacaaa | aaggaaaaaa | taaagacaag | aaaagaattt |
| 129841 | gttgctagta | gactccccaa | caataaatac | taacagaagc | tctttattcc | gaagagaaat |
| 129901 | gacacaagat | agtaattcaa | atctataata | aggaatgaag | aaccccagaa | atggtgaata |
| 129961 | agggggtatg | tacacacaca | cacacacaca | cacacacaca | cacatgcaca | cacacacgta |

# FIGURE 3-JJ

```
130021  tgtatttctc ttaatttgat acatgactat ttaaagcaaa aattataata ccatagtggg
130081  ggattaacat atatagatgt gatatttatg ataagaatag cacagaggat ggatggggag
130141  catatggaag tatgacattg tagtatgtag tatgatttcc tatattttgc atagaatggt
130201  atgaaactaa ttcaatagat catgacaagt taaggatgca tatggtaatc cccagtctgg
130261  tgaaaaacag tacaaagaga tatgataaca agccaataga ggaattaaaa caaaatatta
130321  aaaaatattt actaacccaa aggaaggcag gaaaggagga aaagaggaat aaaaagcaca
130381  tgagacatgt aggataacaa aacaggaact ctaaatccaa ctatatcaat aatgatgatg
130441  gatgtaaaca taaaaatcta atcactccaa ttacaaggca gaggtcacag tgtataaaaa
130501  ggcaagaccc aactatatgc tgcctacaac aaatatactt taaatacaaa gacagaagaa
130561  tagaaaacaa aaaaaatatg ttctgcaaac actaagcatg agaaaactgg aatggctgtg
130621  acaatatcgc acaagataga ctttaagagt atttctagag gtaaggaggg aaggagagac
130681  atttcataat tataaaagag ctaatatatc tgaaagacat aaaaaccatg aatatgtatg
130741  tgcttaatga cagaggtcca caatacacga cacaaacaaa tgacatgtac atcctgccca
130801  agggcagttt attccaagga tgtacagttg ttttaacatt tgaaaacaaa tcattgtaat
130861  ttaccatatt aacaaataaa gaagaaaaac catatcattc tctcaataca tggaccaaat
130921  aagcatttga aaactcaata atcattcacg ataaaaactc tcctaaaaaa tagaaataga
130981  agagaattcc ctcaatctga taaaagacat ctgtggaaaa cctatagctt acatcatact
131041  taaagatgaa acttgaactc ttttcctaat attgggaaaa cacacagatg tctgctctca
131101  ccatttctat ttgacattgt agtggaggtc ccagtcatta taatatgaca agaaaaaaag
131161  tataaagatt gaaacagaaa aaagtaaaag catccctatt cacagatgat aggattatat
131221  ttctatatag aaatctattc ctattcttaa ttatatatgg taaattccat tcattatagg
131281  gttaaaaaaa tgttaaatgc ctaaggcctt tacactcaaa acgactgcat tgttgagaga
131341  aattaaagat gacctaaaaa acataagtta taacatattc aggaattgga aaagtcaata
131401  ttggtaagat aatagttctc tccaaattag ctcatatatc cagtgtaatc cctatcataa
131461  tcccagcaga aattgaaagg tgattctaaa atttatggaa caatgaaaag gacctaaaat
131521  agccaaaaca accttgaaaa agaacaacgt tggaacatcg catgacttga tcttaaaggc
131581  tgactaataa gctatggtaa tcaggaccat gtgggattgg cataagaact tacctatttt
131641  ttaaatcttt gctcaaagaa ccaattttac tactccattg ctaataaaac atgggccttt
131701  taaaggtcct gaatggggtc tattttatgc tgttattatt atgttattat tatgatgcat
131761  ttgttgttgt tgttgttgtt gttgagacag agttttgctc ttgtcgccca ggctggaggg
131821  caatggcgca atctctgctc actgcaacct ctgcctcccg ggttcaagca attctcctgc
131881  ctcagcctcc tgagtagctg ggattacagg cgcccgccac catgcccagg ctaatttatt
131941  tatatatata tatgtatata tatgtatgtg tgtgtgtgtg tgtgtatata tatgtgtata
132001  tacatatatg tgtatgtgtg tgtgtgtgtg tatatatata tatatatatt tttttttttt
132061  tgagacggag tctcgctctg tcacccagga tggagtgcag tggcgccatc tccgctaact
132121  gcaagctctg cctcccaggt tcacgccatt ctcctgcctc agcctcccga gtagctggga
132181  ctacaggtgc ccaccaccac gcctggctaa tttttgtat ttttagtata gatggggttt
132241  caccgtgtta gccaggatgg tctcgatctc ctgacctcgt gatctgcccg cctcaggctc
132301  ccaaagtgct gggattacag gcgtgagcca ccgcgcccag cctaattttt ctatttttag
132361  tagagatggg tttcaacatg ttggccaggc tggtctcgaa ttcctgacct cagataatcc
132421  acccgccacg gcctcccaaa gtgctgggat tacaggcgtg agctaccgca cccggccttt
132481  atgatgcatt tttattattc cttcagtaga ttgtgtgttt ctccttgcat ccaggcactt
132541  ggtgttatct aagtgtttat gtcttccatg tctgcactat gctatatctg tagtttgttt
132601  taaatagtat ctgttaaaac agcgaccatt atccattgta gacaaccagt cacagtcctc
132661  atggtgctga tgaaacacaa aagccacaaa gcttcttttc ctcaaataac ctacccgagg
132721  tggagggctg gctggttttt gatgacaccc attttggtga agactcagga tgtggggcca
132781  caggttgcac tggacgagtc tgtttggctg ccagtttcat cagactcact tgcctcttgt
132841  gaaatatttc ctggacatca tccagcctct gcaaaacttt ctgggctttg gcctacagta
132901  acaaaagcaa atcatgatga acaggtctct ctgtatacag cctgaggaca cgaagcattc
132961  cctttctccc accttcccct actcccccca tccctacctc cctcaggtga ccccctctgg
133021  gcatcactat gcaagtcgct gcaggtcctg ccatgctcca gaattgggta tctaaggatt
133081  agcctctcct acttgagacc cttgaggaca atgactacat cctttcacta tggcgtctcc
133141  agtgcctgga acaccctggc acaggagttt gagaccagcc tggtcaacat ggtgaaatca
133201  catctctact aaaaatacaa aaattagccg ggtgtggtgg ctggcacctg tcatcccagc
133261  tacttgagaa gctgaggcag gagaatcgct tgaacccagg aggcagaggt tgcactgagc
133321  cgagatcatg ccactgcact ccagcctggg cgacagagcg actccatctc agaaataaac
133381  aaacaaacaa acaattagaa tacaatgtgg cttgtgtcat gttagacaga gcaaagaatt
133441  ttggcctgag tccatggatg gacttcaggg cattccccag aattacacaa acagttagac
133501  aggctggata taatctgttt gttcctccac tgcatcttct cttcctttct ttgtgcccag
133561  gaagctgatt aaaagaagct cctaaaccag aggtgcagac aaggtgatga tgtggctctc
133621  agaggaaggt gtgctaccta caaccctgct ggcttcatgg aagaatatgc ttctcaaatg
133681  caaatccaat caagttatct ccctgcttaa atccaaactc gctcatgtgg cccacaaagc
```

# FIGURE 3-KK

```
133741  ctaccttgcc tgcctctctc taacctcacc caaaacacac ttccattgct ctctaggttc
133801  cagcaccctg acctttttggt ctgcactgtg ccaggctccc tccagccctg aagcctttgc
133861  acatgctgtt cctcctgtct ggaaaggctc tcccatcttg tctattcgtt ctccaggctc
133921  aagtgactct tcctgagagc cttctctgac cccagtccag gtcaagttcc tctgtcacat
133981  gcctgaccct gcatccctcc ttgacagcac ttatatcagc ttgtaacaca ttttgtgtg
134041  attattttgt taatgtcagc ctctcccaca aactgtaagc tcagtgaggg atggaagcat
134101  gcctattttt aatcatcatg gtatcctcag cactcagcac agcacatggt acatcaggaa
134161  tgctcataag cattaataga gaaatgactg atttgagtca gacgaagact cagtatgcca
134221  ggacgttcag gccgagagtc taagaggccg ccggagggct tcggaaccag tgagcccact
134281  caattcagcc tgctcagtac cctgtgtgta ctttatcagg aagaaggtac agtccctccc
134341  tcccctatcc gtttcccaac cacaggatca aaataacccg gaagcattac ctttgcatcg
134401  agggtgagca gcaactcaaa ctcgttgtaa aactccttgg ggctgagcaa cgggtactcc
134461  ttgactgtgc ccaggaatgt cgcaatgtcg ttcaaggcga tatcaacccc ttctcgagac
134521  tggcacttgt ctacagcttg ggaagccaag aggtagattc ctgcctcaca ccattggctg
134581  accttttgga aagaaacagt gccctgtgca cttcgcctga ccacaactca gagccaccgt
134641  aatcctcagc aggtctgagc ttgtaaggtg tctacaagga ttcccagaca atgggaaatt
134701  gtcatgggac ggcatcgagt ttaaggggtc taaccactgt aaattacact atgaggggga
134761  caaacccaaa ccagctgata cctgtccact tctcagggaa gtcactcggt cagcaaaatg
134821  agtctcttcc attaacagta attgctacat ctccaggagg agacagctaa atatatattt
134881  aattaaagac agggtttcac tctgtcaccc aggctggagt acagtagtgc aattatggct
134941  taatgcagcc tggagaggtc taccccaggc tcaggtgatt cttccacctt agcctcctga
135001  gtagctggga ctacagatgc atgccaccac accaggctaa tttttccttc cttcctccct
135061  tccttccttc cttccttcct tcctctttct ttctctctgt ctctctttct ttctttcctt
135121  tttttgagac aggttttggc catgttgccg aggctgctct cgaactcctg ggctcaagca
135181  atcctcccac ctcggcctcc caaagtgctg ggattacagg tgtgaggcac tgcacccacc
135241  cagccttata gctaaatatc tcttaggctg gtctaaccat agagaaatgt aatcaatggc
135301  ttttggggtt tacctgctag tccactcttc ttgcatccga attggttacc agaacacagg
135361  gtgattaata agacattaga cctggccctt acatttcctg ggagaagggc atgtcccttt
135421  taacaaaaac acaactttca tctttggatt cccaggtgat ccctatttgc atttactggc
135481  gtttgtcttt cgtaaggaag caagaatgca aagctttgta actcagcaca gcgatactca
135541  agagtttctg gatgctggga ggacattaga taggtgatga gttctaatta ttcaattttt
135601  aaaaaagact cattttcttt gctcttttga actccactga gacacttgaa cagaaagaca
135661  gggagccctt atgctacctg aagagttata tgaaaagaag tttccagtgc caatacctct
135721  gcatttccat ttaccatgtg acctgaaaag caatataaag taactataca gagaaaaaag
135781  atcaaaatgc aacacgccaa agtggttgtc tttctaagtg ggaaaatgat aggcaactta
135841  agtttttgtc ttattttttt cctagattat ttacagtgaa tatgtaccac atttagaata
135901  ataaaaaatc ataaagatat ttcagaatta ataattacca ttatgtaggg atggggggata
135961  gtgggatatg tacgtgccca tgagtaacct ttttcttcct acatttttgg atttccacaa
136021  taatatgaac tcatgctttg atatatgctg tgaaaacttt ctctttacac atcatgccca
136081  gatctgcaaa aaacattagg ctgggatttt ctgtagagtt actatggcta cttgctttct
136141  ctctctctct gtctccctct ctctctctca aacacacatg cacacacaca cacacacaca
136201  cacacacacc aggcttataa tctctggagc aaaaatggag ttgggaaaac agcagctgat
136261  cccacagcga gctcagcaca cagagcaggc tgcacaccag ccccttcgga gctacttgct
136321  tctgaaacaa ctatatttca ttgacattaa aatcccttta gcaatgggat gaggtaagcg
136381  cctgggtggg tgccccctgc catgcaagag gaacagctgt ggggatggag catgctgact
136441  cattgccgtc agccacatgc tgcctgggac cagctcacag ggaagactcc agaaaccgct
136501  gctcctgctg actgggaaca taccctagag gtcacctcca aagggcaccc ctctccttgt
136561  gggcccctgg catgcgccct tgctgtctat cctgtgaagc cttggtgtgg aatctggcag
136621  gcctgacttt aattctggct tcccctgtgt gcaccatgtg tggtcttggg cagtcattta
136681  ccccagggtt tcttggtgac atccctgcac cacctgcacc agaatcattt agacacttta
136741  ctaaaatact agttcctggg cagtattcca gacctatcga cgctgagtct tcagggcagt
136801  gcataaggga atctgccata taacagctca ctaggtgatt ctcgtgtgct acattttgag
136861  acccagcaga gtatcagttt ctgcctctgc acaatgaggc cattaatacc ttcctcagag
136921  gttgtcttga ggattataaa tagatgacat aattatgttc ctggtaggaa tgcagtaggt
136981  gctcgataca tggtaggggg tataaaatgg ttgttattac ttaagcttct ccaaaaagag
137041  tcaaaaccct ttgcctttat atgagggaag cactctgcaa atatagtgct tggttctctg
137101  tacacctgaa aggaatcaa gaagtgttcc cacagggcca agccaaaaca cactcttaaa
137161  gtccggctcc acacagatag aaatgggctt cagttaactt taatgggatg tggtgggatg
137221  gccagagcac tttttacctt cgtgccaggt atacttctac accccaggat ctgcacagtt
137281  tttaatgcct ctcgaacatc ttttcacata aagcactcaa gcctctcagc aatcacacaa
137341  ggaagggagg acgagtatga tggaagcttc ttcacagaaa atcttgcaga catcatgttc
137401  ttggctgtgt tttgctcacc ttgtccagct gtctatgaaa ctctaaggac tttcctaaaa
```

# FIGURE 3-LL

137461 tgtcccattt tttcttgttt ccattgatga aatcgtcaca gaggtgcctg agctccacac
137521 accggggcct gatggcatct gctgcataat ggtggctttg gatgagctgg tccccaacca
137581 gtgccagcag cWgggccttt tccaggggct cctgcaaagt gaacacccac agccaggcgt
137641 cagaagtcag agcatcatga ggctgagagc tgcctggtac tgtgtgctcc aagcctggag
137701 ggaggctgtg gagatgctaa cccaggatga aggctggaga acctgagaaa gctcggaatg
137761 gttccaggcc caggaagtta agcctggccc agagttgtcc acatgctgaa caacagcagt
137821 cattcattca aatacctgtt gagttctggg ctagacatcg aggatatagt gttaacaagc
137881 ccaaaatttt cagattaatg gagaattcag atatcaacta aattacacaa atcgttaaat
137941 tattacaatt gcaataaact ctagggcaga aaagcatgga gtgcacataa taagagattt
138001 tgaatacccc tattaactaa aggaacaaga agagggactg acacctgtcc ttgaggagag
138061 tggggggtga gctgaagttg aaacactggt gaaaattgga tttggacaac tcaaaggtgg
138121 tcatgacact taagaaatat gcttgtctag gctgggcgtg gtggctccca cctgtaatgc
138181 cagcactttg ggaggccgag gtgggcggat cacctgaggt caggagttca agacgcgcct
138241 gaccaacgtg gaaaaacccc gtctctacta aatacaaaaa ttagatgggc gtggtggtag
138301 gtgtctgtaa tcccaactac tcaggaggct gaggcaggag aatcgcttga acccgggagg
138361 cggagattgc agtgaattga gattgcgcca ctgcactcca gcctgggtga cagagcaaga
138421 ctccatctca aaaaaaaaaa aaaaaaaaaa aagaaatatg cttgtctaaa ccctttccca
138481 aagcctgtaa gggccctgtc tacactgacc ctgtctgctc aatcacattt cctacctctt
138541 tctgcatgac acttcgctct aaccacacgt gccaagtttt cctgtttgtt taagcattcc
138601 ttcattcatc cttctcatat tattgagaac ctgctatttg ccaaacactg tcataggtgc
138661 tgggagttca ttggtaaaga aagcaggcaa aaacccccac ctcatgaaat gaagcttaca
138721 ttttaatggg agacagaggc aataaacaag tgcatgtgta ggtcatatgg tatcttagac
138781 agtgatgagt atgtggagaa aaattagatg gtggggaggg cagggagtgc tggagctagt
138841 attttaaatc ggatggtgag acagtctccc tgagaaggag tactcaagct atgcagaaaa
138901 gacttcacat agaaagcctg agactaggcc gggctgtggt ggctcacgcc tgtaatccta
138961 gcactttggg aggccgaggc aggcggattg cctgagttca ggagttggag accagcctcg
139021 gcaacacagg gaaaccccat ctctactaaa acaaaaaaaa ttagccaggc atggcagcat
139081 gcgccgatag tcccagctac tctggaagct gaggcaggag aattgcttga acctgggaag
139141 aggaggttgc agtgagccaa gatcgtgcca ctgcactcca gcctgggtga gagctaggct
139201 ctgtctccaa aaaaaaaaaa aaaagagaaa agaaagcctg acactacagc cttagaaaga
139261 aagacccgat tttaagattg gcccttttgtt ggcatctagg aacttagatt tttgggaagg
139321 tttcctccat tccctgatgt gaatggttca tgatccctga actgtttgtg caaacagtat
139381 ggtttatggt gattacttgc tttttcttct gggagtctga aatcttggta cttgccagac
139441 agaagctgct gacatgacca gccaccaata aaacctctgg gcattgagtc tctaatgagc
139501 tcccctggta gacaacattt cacacatgtt gtcacaaccc acagacgggg gcaggaagta
139561 tgtcctgtga gactcccaca ggagaggatt ctaagaagct tgtgcctggt ttcctccaga
139621 cttcacctcg catgcctttc tcctttgcta attttgctta gtactttctc atcttaatag
139681 atcataactc taatacagct acatgcagaa tcctcctagt gaatcttcga agaaatgtgt
139741 gcatggtctt gggagcgctc tgcatacaag ctgagacctg aagtcaggga caagccgtgc
139801 agtacttggc tgggaggtga aggcgtgttt gacaagtttg ggtagcagga aggaggccag
139861 tgtggctgca gcagagggag caagaaggaa atgagtggaa gactaactag aggaaagagg
139921 gggttccata gccggggtga gaggaaagcc cctgggtttg gagggaggat ctcgatcatg
139981 tttttgaaag gaccactccc ggtacagtgt ccagaataag aggaaatgca gggagactca
140041 ttaaggggct gctgcaggaa tccagccgag agatacggct tacggctggt caccaaacta
140101 gctcctgact cgacccttct ctggattgtt cttccctggg tcatgcagag ctgagttcct
140161 gggtcacctc ctcacagcgc ttccctggcg actatatctt aagtcattct ctacaaccta
140221 ttcgcccagt cacctaggcc ctctcccaca ctttaattct ttaacagcat tatctctatc
140281 taaaaactat tttctttatt tactttaatg tagaattcct ccctcctggc tgggcgcggt
140341 ggctcacacc agtaatccca gcactttggg aggccgagac gggcagatca tgaggtcagg
140401 agatcgagac catcctggct aacacggtga aaccccatct ctactaaaaa gacaaaaaat
140461 tagccaggca tggtggcaca cacctgtagt cccagctact cgggaggctg aggcaggaga
140521 atagcttgaa cctgggaggc ggaggttgca gtaagctgag attgtgccac tgcactccag
140581 cctgggcaac agaatgagac tctgtctcca aaaaaaaaaa agaattcctc cctcctccac
140641 tcactcccac caaaatgtaa gttccatgag agcatttagt aggcatgcaa taaatatttg
140701 atcaacaaaa taaatgaatg agccaatgag ctacatgatg cggtgacctg cttggctgga
140761 actctgtgaa gcaatcagca cagatgactg ccacattcca aaaggtctcc aggagcagag
140821 agcccatgag ccccccttgcc agccacacta agcatccagt gcatcactgc actgctttag
140881 ctgtttacag taacacaagg agtcacggta actccaggtg cgtacatctt gcccctctct
140941 taacttgcac tggtttatgg tttcaacaat ttccaaaccc ctttcctctt accctcccag
141001 tcattcacga agccccgcag ggaccgtcgc cttcttaatg cctttcaaat tctgacccca
141061 tctctgccac ctccttgtca ctgtctgctc aggcctcatg gcttctcatg ggacaactg
141121 gtctccctgc ttttcttcag gctctccttc aaatcatgct ccagactgct gacaaaggag

# FIGURE 3-MM

```
141181  caattccaac atatcttatt gtgtctacat cactcccaag cttcaagccc tttcctgggc
141241  gcccagggtc ttcaggagca agcttaaact cttgagcaat gcacacgggg aatttcatta
141301  tctagctcat acctgggtgt gcctgactta ccaagtcctc ttccacttcc tcaactccat
141361  tctctgctgc aaccatacta attatccact tattgttccc caaatattca ggcagcttca
141421  ggcctccatc ccttctccac ctgctgtctg cctagaaagc ccatccttcc ctactccccc
141481  tcacccatac tcctctgggg ccaactcgat ctaaatcata tttcagactt caccttctcc
141541  agaaaacttt ctctgtgctt ctcttgatca gagcacctgc ttcattatgt tgtgaccact
141601  tatctgctgc ctgcgctatg ccacagacac ctatggtcca gaaccagctc ttattcaact
141661  gaaaacagtg catgctgcat cttaggtact tacaaataaa tgctgcacaa aacaaagagg
141721  agtactggta tagaaacaag tgacacaaat caagtagtgg ctagttaaaa ggactgaggc
141781  aaaaataccc agaacacttg ttctaaacag caaaggccca ggtgcactgg ttaccacccc
141841  tacaagatga ctggaatact cattccctgg gcagtgattt tttttttttt tttttttttg
141901  agatggaatc ttgctctgta gctaggctgg agtgcagtgg cttgatctag gctcactgaa
141961  aattctgcct ccggggttca agcgattctc ctccctcagc ctcccgagta gctgggacta
142021  taggcgccca ccactatgcc tggctaattt tttttttttt taatttttag cagagatggg
142081  gtttcaccat gttgcccagg ctggtctcga actcctgagc tcaggcaatt cacccgcctc
142141  ggcctcccaa agtgctagga ttacaggcgt gagccaccgc gcctggtctg ggcagtgatt
142201  tttgactctt tggttcactg ctgtgtcctc agaacttaaa cagtacctgg catggtagga
142261  gatgaataaa tatttgcaga atagatgtcg aatgaatgac agacctttcc tagcactgct
142321  cagctaccgc agactgaacc tagcacatgt tccatagccc agaatttatc cctctacctc
142381  tgtaatgtta ataacaacag ctaacatcta ctgagagctt aacagatgcc aggcactgtt
142441  ctgaatgctt tacatttgct tatttcctta actcttcaca acaaaccctt tgatacaggt
142501  actgttttac caaagggaag tggagcacaa tggcccaagg tgacccaggt aggaaagaga
142561  agagccaggc ttcaaaccca ggcagtcttg ctccaggatc tggctcagac caacatacac
142621  agagaaaaat ggctgcactt gttctgccta aacttagaga cctcatttgg ttgctcgaac
142681  ttgtctgact ttagtcaaat atagaaacat gagacttggc atgaaagtct ctgttgacac
142741  tttttttttt tttaagtcca gctgcctaat ttattaagaa cagggcagaa ttttctgggt
142801  ccagggaaat tcaccacagg atacttccaa atcaccctgt ggtgatcaga gcctgccctt
142861  ccctcagcat acagtatttc acccttcaga attttccaga agacatttga gatgttagag
142921  ggaagatgtg tgtaacaggt ccaaatgggt ctagggagaa acgcccataa aggcaactga
142981  ggaggccggg cgcagtgggt cacgcctgta atcccagcac tttgggaagc caaggccggc
143041  ggatcacaag gtcaggagtt agagaccagc ctgaccaaca tggtgaaacc ccatctctac
143101  taaaaataca aaaattagcc gggtgtggtg gagggtacct gtagtcccag ctacttggga
143161  ggctgagaca gaagaatcgc ttgaacccag gaggcggagg ttgcagtgag ctgagattgt
143221  gccactgcac tccagcctgg gcggcaagag tgaaactccg tctcaaaaaa aagggcaact
143281  aaggagcaac ccggattcag ccaccacatg gctggaaggt cactattaca gaattgaaag
143341  gacaacaata gtttctgacc actgactact gaccaccaac catattgcaa gctgaccaca
143401  tgaagtccct accacacatc taactcattt aatcttcaca aggtctctgt gaggcaggaa
143461  ttattacctc tgctttacag atgaggggga ccatattcag aaaggctacg aaacgtgact
143521  agggtcatac agctggtact tgttagagcc aagatttgaa tccatcccta tccgacttcc
143581  aaagcctgtg ttcttgtcac cttccagccc acctggcctc tacacatgtg ctatgaaatc
143641  ctcctggtgc ctgcaaagaa atttatggtg aaacctccaa attgaagctt atttttttag
143701  tttatatacc tcccactcag gataaatagt catcaaaatc tccaaagtaa aaaagaaaga
143761  gtgagggaaa aaatggctaa aatcttctga tcacaagccc atctgggatc tttcagatgc
143821  tgaaaattcc agaaggctgg atcgtcaagt ttcagcttag aagttgcaat cagaaatctt
143881  tattgaaata agtttatttc cccccgagga ctctgctggt ataacagatg agagatgaga
143941  gtgcctacta gggaagaaag tggcgagaaa gcgagtgatg ctactctatc cgggaatcta
144001  gaacaaaccc acagaggatc cacagggatg gagtgtgtgt atggggaggt gggccagatg
144061  aaacaagaaa ggcagaaagc taatggttgt tgaaattgaa tgatggacac ttggagttca
144121  ttgtgccagt ctatctgctt ctctgtgtgt ttgaaaattt cacacatgta cacaaagcca
144181  tgaccctcca acaagatgac atccttagat gttaatggcc tgggttccat gccttcttag
144241  acacagcacc cttatgggga gaggtcaagg ctgaaatcag gtggttcctc caccctgtcc
144301  tggtctgttc cctgctggcc cctcagcaag ggtctcagta cggcccacag tcaaaaccct
144361  gcagcaagtt ctgcttttat agaagacctt ctgtaagtgg ttgaaactac tgtttttcag
144421  aatcaggaag gagcattcaa aagactgcta aaccctcaaa cgagaacaag ataaagaaca
144481  ggctgaatca gcatgcgaca ggagcctggt tagtgagttg actgaggtta cttgtgagaa
144541  tgatgtcact gtgtggaata aggaagctgg caacacccca gagccagcgt gtggctgtgc
144601  tgggagggag ctggcccctt agtgccgagt aaaggacaaa ctccaggcag atatttgtca
144661  gattgtggcc agacacagtc ttgttccttt aacactttaa aggacaaatg gaaagattag
144721  ccaagcccta atcatctgaa gaagccatgt gtctgagaag tctgagggaa gatatattga
144781  aaacgatctc agttacgatg agacagcttt gtttgattta cgcaaaggca gagacacagg
144841  tgtactacat ccttcaggaa gcaagtttag aaataagatc tgcaagtcat aggatgtgac
```

# FIGURE 3-NN

```
144901  aggtctgcaa atcctgcaag tcacaggtca tccacagtgg gctagtctag ctgcttcaag
144961  aatgttgccc acgaaaggtg ggtcatgggt tcacagagca ttcctgtgtt ccataaccac
145021  aggtttcatc cctaactctg gtcagcaact tttgaagtat ataccaagga ccacaagttc
145081  aaggaagtgg gcagaagaaa gaatgtgatg cagtcatgca gatccatcgc cactattcaa
145141  gacacagccc agagcgcctt ccaagggctt tctgttccac tgagaataaa attcaaatcc
145201  tcacaaaaga tctcagggtg caacaggact acctggcctc tgctcatctc tgttcactca
145261  tttcctcctg gcccacctgc tctagcaatg ttccttgaac tcaccaagct catttccact
145321  ttaggacctt tgcacctgca gctcgctctt gctttgatac tattccctaa gtctttgcat
145381  gattacacag atcaaacaga tctcagacca aaggtcgtct tctcaggcct ttccagacca
145441  ttctagctaa agtagcatcc ccagtcactt tctattatat cttgtttctt tttttatatc
145501  acttgttact atctgatgtt attatttatt tgattgcttt ttagtgaact gtctctctcc
145561  agtagaatgt aagctccatg agggcagatt ctggactgtc tggctcaccg ctgcacccac
145621  ggaacctagg ataaggaata cctcttgatg aataaataac cctctggata ggtcagtagc
145681  tactttcata tatatatgaa ggcatttttt ttgatgaggc acttttttctt aatatatgaa
145741  taagcaaaaa aatatagtat ctatattcag taaaaaatta ttttgaaaaa actaaaatga
145801  tataaggttg gggctgtcct ggagaatcta aggtatctag tcatcatata tatgaaattc
145861  aacttttcca ttgtttgata aatgttcctg atgactcatg tgtctttagc actagtttat
145921  ctgtcaaatg gtcactggta agagaaagag cgctcgctgt gaagggatgg gattcctgaa
145981  tggggatctg gatacacagc tgggtttgtg tgttcatgca tgtgcacaag cacctgcagc
146041  tcagaatcta caaccaacag ctcttagagt cagtgtggcc ttggtatact ttgttttgta
146101  tcctttaggg gtgtgtgtct ccaactttaa cgtacaagtc aatcacctgg ggatcttctt
146161  aaaatgcaga ttccaattcc agggtggggt ctgagatttt atatttctaa ttagcgctta
146221  ctcaggtgac atctgtgctg ctggccagag gctcacactt tggcaaatct ctagtactgc
146281  aaaattgccg tctgagaaac atgcctccag attagctaag aaaggaagcg ctaaccacag
146341  gggacttggt ccccaagacc caaaaatata gagaacctaa tttcaaacaa gcagaaagga
146401  acttctctgg aaagagacga ctcagctttc ttcatttgta ataaaccaaa tctgtaaaca
146461  cttaaaatgg aatcatttct aaatgctgac ttgtgtgcgg atctgggaca ctgtttaatt
146521  ccagtatggg actgtaaaat acatattctt ttacaagttt cacatttaca actcttggga
146581  catgagaaag gagaaagctt tgccaattct gtgtagcaat ggaactaaca tggaccaatt
146641  tcccccatct aattgtcttg ctccaggtat aaaactagtt gttttttttt ttctggtttc
146701  tcctttgaaa gtttcccatc cttactacca accccacaac ccccagcccc ctagtccagg
146761  ctccagggag gagctcaagc tgatgggtga gcctctgata taattgttct ggtgagtgat
146821  gagcctgttg tgaccttctc ccacttggct gctgaccacg tgagagggca gggctggtcc
146881  agagtcctca cccgacctga gcgtccaact gcaactgtat gttccggcag ctttaatagg
146941  actctgtgct gcccgtatta ttcatagcag ttgttaatgt gcctctctcc ttgggatcct
147001  ggggtgactg aagggcagga ctgggcattc ctgtgtcctt tagcaacctt ccaatagcaa
147061  tgactggaca tgatccatca ttgtccacag caacctccct cccagtacac aaaaatgtca
147121  gaaagcaaac ctggcttttt tcctccagtt ttttgtgttc cttaagaaKc tgctccacgt
147181  gcatcacgct gtctccaatg cctgtaaact ctgcttgctc ttccagcaaa ttatccaggg
147241  caagcttagc ctacaagaaa cattagaaca gtccagaagt aaaccaacac tcactcacct
```

# FIGURE 4-A

```
>11:71972801-72105900

1       attcccacat ttacagtaac ccctgtttcc agaaaataaa tatcaagata aggaaagtga
61      cactaaagag acacgcagcc actctgcctg taccctctct ggaccttaaa gagttaaagc
121     ctagccatga tttcctacca gggctgaggc taatgtaaac cacctgctca gacRcaagaa
181     gcaggaccag agggtcaagt tgctgagctc tgaagtctgt tctgaaaaag cttcagtttt
241     aagtacagaa caagtgatag gccaaaggcc agagggtctg agctgcagca gcactgctct
301     ccttaccccg cccttggcct gggtgccagc tcctttgaga ctgcctctta gccacttact
361     gcccatcaag ggaacaccag acctacctca agataacacc aaagtgacct ttggtcccag
421     agagttgtta aactataccg atctctcagc tcatgggccc ttgcttctgc tgaaccctca
481     ttctcttagt gctaactccc actcccagaa tttcagggcc caaagcccac acaagctccc
541     acctcaaagg gtccttccca gaaccccacc cccttctcca cctgatagtt ccaaaggcct
601     acagcctcac agctcacaaa ggaaatggct gccccatctg tgcgccagtg ctcacttgca
661     aaatttacca ccacttaccc tgagacgctg cctgcctgta gcctaagaat tccatgctct
721     gtacccctttt cttagaaaga tactccccat ctgagagaca ctcacatcca aagagtgaaa
781     accctgatct tggggctcct cctcctccaa ctctRggacg gataggccag gaggcacatg
841     caagcaaaca ccgagatctc acctggcagg ttacccagag gggccaagcc accttgccct
901     aggctaataa gctataaaaa cagcatagga aatactttgc tggtacatcc cctgcctctg
961     agaagataaa aggcaagagg accagtggct ccaagagacg ccatacagag aaaaacaatc
1021    tttctcaatt cctggggagt cctgggtaga ccgtctggtc caagtccaaa gagtttcctg
1081    aggtcggggc aaaccagttt ggtccatcta aagggactag cttgggctga agcctcctct
1141    tcacacagac cactccatct ttgtagccta gagtttggca cagaacccca aagccctgga
1201    gaggaacaag ggagggatta cagaggaaac caagcttctc tggtttcttc aaaagtggct
1261    tacagatgtc tgccccttct ccccgttctc tggagggtcc agatcaggat gatggcaaaa
1321    gagctgcaat caggaaccag acctgcaacc aggcctgcag ccatgtattg tactggggcg
1381    cgtacacata cacacacaca cacacacaca cacacacaca cacacacaca cacacacaca
1441    cacacacaca cacactgacc tgggcccccc accactatat tcacacaccc tggtccctat
1501    cagctgacaa aatgaacctt ttttcactgc ccaccaccgt gcacacaccc acggcctcac
1561    gcagaaatgg tgaagccctc tcctgagctg tctctacagc tcaacgtgca ggcaaacacg
1621    ctttccttct ctgaacaaaa acatgctgac atctacgcaa gggggaaaga aagcaaccct
1681    ttgctctgaa aggcgccaac tcccggatgt tggcaagctc tgctccacag cgcctgtttc
1741    tgagtatgtt tacttgctct ccctcttctc tccctcccct ttaaggatgg cgcaggaaag
1801    ggaggtgagg ggggagaatc caccccaccc cctcgtccat gtttgaacaa aaacccagag
1861    aaagagtgag ggagaaaaag tctcggtgcg gggggccttt accagactcc taaagccaaa
1921    cgtatctaca ggttttaatc cgccattagc atttaaatct tgaaaggaga ggaggaaggg
1981    ggggccagtg gaaagtaacc ccacacacaa aagaaaagtt gttggttttc ttctcagtct
2041    gcctggcagg agcgatcttc ctggctgaca gccagaagtg cgtcacagcc aggagtctgc
2101    tttctttaaa ggcacagcaa gcttccccga tctgggagcg acagcaagca ggggactctc
2161    agtgggggtg gggcccagag ggaggggtg cactggagag gaggggctgc aaaggggaag
2221    gctggaggct tggtccacga acaagaaaag caggacgaat gggggcggcg ggttgagggt
2281    gaaggcacaa gcaggcaggc aaaattactg aactccaaca gcagcaagag ccaaggcaaa
2341    ctgtggcata aacacacaaa acacatctta cagtggcctg cctgacaaac tgggatcaac
2401    cggacactcc cccttcgctc tgttttccac ccagagttta aggcagcagt aataatctgt
2461    ccatctctct ggacagcaat ggaagggtag gacaagggct gtcctgataa ttagatcagc
2521    ccctgcctgg cggaccgcag ggcaaggagg aagacccggg aacagaggct ttgaagaaca
2581    agcaacatgc aggccctctg atcgggggca ccttcctgcc ggccccaggg caggctgtgt
2641    ctttaagtac aaccatgcca ctcacacaca aagcgccctg ccaccttcca tgggcaccgc
2701    ctgccctgct ctgggacacg gccacccact aaatccattc cccgagggaa tctctccagc
2761    ccataccttc tgcgtggcaa ggccaggatg ccgtgctgtg cccgctcctc cccctccgcc
2821    acccaaacaa gaagcccagg cagattctgg ccctggagta caggcctgcg ctgtcctgtg
2881    ctgcgtgcta ttgtcggctg tggaatggcc gccaaaatcc cagctgtgct gccagaacct
2941    aatcccctgt tccgctgagc atttatgggg aggggctgag ggccaaggc acgagaccga
3001    gtacgccagg cactggctgc agcccttaac agacgcaggc acggggactt gtagactctg
3061    cttctcctac atggctgaaa acaaaagatg tctgactctt agaagacaaa atcacagcat
3121    ttccccccac tgaatagcga acttcaccaa ggctgaaagg gaagcccacc agatggtcaa
3181    caggcacccc ctaatcttat gccacaaaca tttactgagc acctactatg tgtaaggcac
3241    tcctatagga aactgtagga agtatacaaa gatgaattaa gcccagcatc cgcactgagg
3301    taggttgtaa ttcacaggag ataagaaata tgacttgaag attaatttca aactggtgtg
3361    ctaacttcca taaaagctgt aaagaaaaat tctaaccagt ttagaatagg gaaggattac
3421    gaaaatgaag tgccatttta atttggacct tgaaacacac aggtagcatt tccccagatR
3481    gaaacagaga ggtgtgggga ggaagagaaa gcagagagga ggcatggaag cagtgggaga
```

# FIGURE 4-B

```
3541   aagggcttga agagtgaggt ggggtcagag agctaggaga atcttgaacc tcaaaccaag
3601   gaatcaaaga cttgtaagtt tgtttgttat taacacagag ctgtggtatg acaaaaactg
3661   tgccccaagt ttattctgat atatacctga atgagactag cagcaatgag gtgagtcagg
3721   aagctgcaca gtcaggagac aggtaatgag ggcacacaag agggtaaggt aagaaagaat
3781   ggaaaggggc cttggcacga cggctcacac ctgtaatccc agcactttgg gtggaccact
3841   gaagtccgag agttggagac cagccggggc aacataggga gatcccatct ctacaaaaaa
3901   tttcatgaat aaaaaattag ccgggtgtgg tggcccgcac ctacagttcc agctacttgg
3961   gaggctgagg tgggaggatc acttgaaccc aggtggaagc tgcagtgaga gacattgtgg
4021   agacagggta aagaactgat aggaggcaga gagaggaact gtacagggga ggaaagtatg
4081   agggatgata tgcagaatta aactgaggag atgacaggat gtcagaacaa gagagaaaac
4141   agagtataat aaatagtagc tggtggggca cggtggctca cgcctgtaat cccagcactt
4201   tgagaggcca gcctgggcaa catggcgaaa ccctgtttct accaaaaaat acaaaaaacc
4261   acacaaaaaa acaaaaacaa aaaattagcc aggcggagtt cttttttttt ttttttttt
4321   ttttaaagca gggtctcact ctgtcaccca ggctggaggg cagtggcgag atcatagttc
4381   actgtaacct caaacttctg ggatcaagtg atccatctgc ctcagcctcc caagcagtgg
4441   gagtacaggt gtgtgccacc atgcctggct aatttttttt ttttttttt tctgtagaga
4501   caggctctca ctatgttgcc taggcacagc attattttaa attcttctct aaaaattcta
4561   tgcttctccc aaagccctgg gatacaggca tgaaccacca cacctggccc acagttgtct
4621   ttgtcttttt ctactaggag ctatactata gaggtcacaa tcattggcta cagtttataa
4681   tatataggct aaaatgtttt acatataaga taaccagtaa aacttcatga tttagaaaca
4741   aattagtgtc ttttttagtg tcagagtccc agctactcag gaggctaggg tgggagaatt
4801   ccttgaactc ggaatgagca gagatcatgc cactgcactc cagcctgggt gccagagcaa
4861   gactctgtct caataaatac ataaataaat ggacagatag ataaacagag agacagaaag
4921   acatatagta gctaatgctt actgagtgca tactatgtac gcgacactat tctactgacc
4981   taatatgtac gaactcatta atcttaaaaa ctcccttaat gaagtaggta ccattatcac
5041   ccaactttag agaggaggat attgagccac aagattaaat aatttgccaa gatcacatag
5101   caagtaaatg gcagaagtct ctaccacatg ctgctaacta tttgacattg gcaagaaact
5161   tctgaacttt agtatcccta tttgtaaaac aagaataaag acacctgtta taaagcaatg
5221   aaaattaatt caataacacc tataaggtgt ccaacacact ggctagcatg taaggcatag
5281   gatgggtgtt tcctgcagac taattcctac ttggcatttt ggtccccttgt caccaaggtg
5341   tccaagcaga agttggctca ccaattgttt gggaagccag agagggtatt ctgcctggag
5401   caggagactg gatcctctgt ctgtctgcct tcttcccatc agtctgcctc tctagatttt
5461   actttagggt ccagctcaaa tatttaatat atttgattta atattcctaa ctcctcaaat
5521   atttaacagt taacaggggt tactttgggg gggggagga gttagattgt agggaacatt
5581   cacttgggga ctttctatgc tgtatgtttg cttttttttt tttttttttg agatggagtc
5641   ttgccctgtc acccaggctg gagtacaatg gcatgatctc agctcaatgc aacctctgtc
5701   tcctgggttc aaatgattct cctgccacag cctaccaagt agctgagatt acaggcacct
5761   gccaccatgc ccagctgatt tttttatttt tagtagagac agggtttgac catgttggcc
5821   aggcttgtct cgaactcctg acctcgtgat ccgcctgcct caggctccca agtgctggat
5881   tacaggtgtg agccactgtg cctggccttg catttatttt taataatctt gtattacact
5941   ggtgataaga aaaatgtaac tacctcctac tcttggcact tatgtgtata tgcatgaaaa
6001   tcttgtcttc acatctagaa tggaaattcc tcaagggcat aaccagttcc acctctctct
6061   ctctgtgatc cccataaca cctagcacac tagggctcct taaatctgct tctccaaact
6121   acatctggat gtcaggttta caaattcaac tttctggcca ccagagaatg actgtggtgg
6181   gtggttggga tgaggtgggg gtgggggtca gaccaaggct cattgctgta ggctccccat
6241   cacctgctgc ttcttccagg ccctgtaagc tattcccact gtatcatttg tagtctgtag
6301   ttagcaatca ctattagtag aaatggaagt tcttatccaa aggagcacca tgaaagcaaa
6361   ggcaaagcag ataccaaaac ttttacagaa aagcaaagtg gcaaacataa ttgtttgggt
6421   ttttgttgtt tttgtttttct tgagacagtc tcactctgtc acccaggctt gagtgcaagt
6481   ggcacaatca tggctcactg cagcctcagc ctcctgggct caaacaatcc tcccacctca
6541   gcctccggag tagttgggac cacaggtgca tgccaccacg cctgactaat ttttgtattt
6601   ttttgtagag acagaatttc accatgttgc ccaggctggt ctcgaactcc tgggctcaag
6661   cgatctgcct gcttcagcct cccaaagtgc taggattaca ggcatgagcc atcacaggcc
6721   tggccaaact taatttaaag actggcatta tcaatgctcc agaaaatgta taaggtgaaa
6781   ataggaggga ggcctgctaa aactgccctg gccacatatg gcaatgcatc agccctgaag
6841   cttcctagcc cagatagaat tgatattgtc tcaaaagcgt acaagaggtc tccccagcct
6901   ggccccttgc tctaaactca aggcaacagc ttccagctgt gcagcttccg agaggctgaa
6961   ccttccccta gaacccctct gcaaagctgc caagtttctg ggccagctct tcaggacagc
7021   ccaaggagcc tggccagttt cgccaggctt tccctcctct tcctccctcc tgtggcagca
7081   gcagaatttc cacagcccct ctcacagcct agacaggcag caggcggcca catggctcct
7141   gcctgattct tactgtcctc agaagagcgg gaaaggagtg gtaaaacctt tctagaaaaa
7201   gagaagggcc gggcacggtg gctcacgcct gtaatcccag cactttggga ggccgaggca
```

# FIGURE 4-C

```
7261  ggcggatcac ctgaggtcag gagttcgaga ccagcctgac caacatggag aaaccccatc
7321  tctactaaaa atacaaaatt agctgggtgt ggtggcgcat gcctgtaatc ccagctattc
7381  gggaggctga ggcaggagaa ttgcttgaat cctggaggcg gaggttgtgg tgagccgaga
7441  tcgtgccatt gcactacacc ctgggtaaca agggtgaaac tccatctcaa aaaaagaaga
7501  aaaagagaag aaagaaaggg gtggagagcg gggagaggga gagagagaga gagagaaagg
7561  aacaaaggaa ggaaaagaga gaagaaagga aggaaagaaa ttactttaaa ttcagtaggg
7621  agggctgggc acagtggctc atgccttaat cccagaactt tgggaggtcg aggtgggcag
7681  attgtttgag ctcaagagtt caagaccaac ctatccaacg tgacaaaatc ccatgtctac
7741  taaaaataca aaaattagct gagtgtggtg gcaggagcct gtaatctcag ctacttggga
7801  ggctgaggca agagaatcac ttgaatccgg gaggcggagg ttgcagtgag ctgagattgc
7861  gccattgcac tccagtctgg gtgacagagt gggactccat tgcaaaaaaa gagaaaaaaa
7921  aagaatttta aattaaaata aataaataaa tttcagtaga gttcagatag aggttgaagc
7981  ggggtggtga ctggttagga gcacaaaggt tatttcaaag ggtcaggtga tcctcctgcc
8041  tcagcctcct gagtagctgg gactacatgc acatgccatc gcatcaggat ttacattatc
8101  atttttgaaa gtttaaacaa aaaaagactg gagctgggtg tggtggctca tgcccgtaat
8161  cccagaactt tgggaggcca aggtgggagg ataatttgag ctcaggagtt caaattcctc
8221  aggagttcaa gaccagcctg ggtaacatag cgagatcccc atctctacaa aaaataaaag
8281  aattagccag acatggtggt gtgtgcctgt agtcctagct actcaggagg ctgaggtggg
8341  aggattgctt gagtccagga gtttgaggtt atagtgagct atgatcatgc cactgcactc
8401  tagcctgggc aacagagcaa gacacgtctc aaaaaaaaac aaaaacaaaa acaaataagt
8461  actaggagag ttcaagtccc cagtcatccc catgggatga ttctcacaca aataattagc
8521  ctggcactca aggccctcca tgacctgcag cttctagagt tctagcttct agagtcgtta
8581  ttcccttacc caaaccctcc actctgccag gcaggtctat aaagttacag tttgtcctga
8641  gaattagaca agatgaccgc aggggacagg ctctggatta gctacgaatt acaacagtaa
8701  cagcaacgat aatgatagtc tttactatgc caggatgtaa gtgacagaga aaagactaga
8761  aggcattgta ttgggcagtt ttgtttattt tactttattt ttttaataaa attaaaaaat
8821  atatggaact cttcacaaat ttgcatgtca tcctcgcaca ggggccatgc taatctctgt
8881  atcgttccta ttttattttg ttccaatttt agtatacatg ctgccgaacc aagcactggg
8941  cagttttaaa aatgaagaaa tctgaggctc aggaattagg tgacctacgt gatctagtca
9001  tgtaagtagt aagttgtcta ccttgtccct atattattct tttttgtttt tttctttcaa
9061  gatggagtct cgctctgtcg cccaggttgg ggtgcagtgc tgcgatctcc gctcaccgca
9121  acctctgcct cccgggttca tgtgattctt gtacctcagc ctcccgagta gctgggataa
9181  taggcatgag ccactgcacc cagccagcct atattattgt ctacagctgt taaataggca
9241  atcattgagt gaatgaataa aagctagcac attctcagca aatcaaagaa gccgtgtgct
9301  tttcctcatg aaaatcaact ttttttttatt ttttgagaca gagtttgctt tgtcgcccag
9361  gctggagtac aatggcacga cctcagctca ctgcaacctc cacctgccag gttcaagcga
9421  ttctcctgcc tcagcctccc gagtagctgg gattacaggc gctcgccaac atgccggtct
9481  aattttttttt attttttagta gagatggcgt ttcaccatgt tggccaggct gggcttgaac
9541  tccttacctc aggtgatcca cctgcctggg cctcccaaag tgctgggatt acaggagtga
9601  gccaccgtgc caggccgaaa atcagcttct taatcaaaat aatcaaacag ctccaatttc
9661  aaaattaatc actaaagaat tttttctga ggatccgaga gctttacaaa cactRaaggg
9721  ccaaaactcc tctaaaaaca gcaagcacca agaaaagtct tgtactagga gggatgttgg
9781  cagtgtctca agggttagag ggggaggctg gaaggaaatg ctggtgctaa agggaaggac
9841  tgctgtttca ggagcctgac cttgagctta tttaccatgg cctcattgtc cctctccaga
9901  ggataatggc tgattaatga ggcagtgttg gtaagtctga ggcttcctgg agagaaaagc
9961  agagggcaaa tcaaaaccca gagaggattc attataacat ccaacaggcc cactcagttc
10021 tctctcaaga aggcctttct acaaaacagc taagaacctc tccaccctgt ttcactgtca
10081 cagaggagta aaatggctct gtccctacag gcaacatatc aacatatcta gcacaatgtt
10141 tgctcacaaa cacagaacta attataagaa atgtgaaaga acctgaaacg caggggggaat
10201 actcttactg gagacagcct ccatagtggt agaaaacata gtagatctgg tgttgaaagc
10261 ccctggaggg gtgatgggtc acttactagc tatgtggctt aatgcaagtc acagaggcaa
10321 attactgaga accaagctag ggtcggatca gctctaaagt accttttcag ggccaggcac
10381 agttgctcat gcttgtaatc ccggcacttt tggaggccaa gatgggagga tcacttgagc
10441 caaggaattt gagaccagcg tgggcaacat agtgagaacc tgtctaaaaa aaaaaataat
10501 aataatatat atatatatat atatatagtg tgtgtgtgtg tatatatata tatatatttg
10561 cctattcatc tatctataga tagataaata aatagatata tatcaaaaaa agaaaattgg
10621 ctaggcatgg cggttatgtg tctgtagtcc tggcaacttg ggaggctgag gtgggaggat
10681 cacttgagcc caggagtttg gggttacagt gagctatgat catgccactg cactctagcc
10741 tgggcaacag agtgagaccc tgtctctaaa aaaataaaat aaagtacctt tccacttcca
10801 aaaattccca tgattctttc cacaggatac cattctcaac aacaaatctt gtagtagaac
10861 ttgtctcctc agaatctggt caaaacatca ctagttaggg tgttctttca aaaaaaatag
10921 caaatctcct agaaacaggc acattgtgtg ttaccctatt aggacccatc cccaaagcga
```

# FIGURE 4-D

10981 ggcacatgtg ggtgcttcat taatactctg ttgaaaggag gaggtccttg gaaactggta
11041 gtaccaactt agcatggcat acaatgaaag gtgattggag ccacactcga ggaactacaa
11101 ggcagctttt cataaataaa agccacaaaa tgcaatttat aactacatct gtctaaacag
11161 actaaagaaa agtagctttc tatacctagg tcttcacccc ttctcctccc cacctctcta
11221 ctttatcatc cctgtcctac acagcactga gacccgtctt tctggttcca ggtccacttt
11281 tcctttttcc acaccagaat gcctttaatc actgatagaa atgaacaaac atgaatatga
11341 gttctaaaca ggaagacaga tagcctcaag aaccaacttg tcaatatgtt actaataagt
11401 gaaataagtg ggaatggtgt ctaacatggg catgataaaa taaaacccta aaatacatac
11461 tgtgctaggg atttgaacaa acttcaggat ttttaaggaa ggctgtgtct cacatctcaa
11521 ttaatcttcc caaaagaacc ctaagaggca aggtttattc caattttata aattaggaac
11581 gtaaggctca gtaaaaagaa aatacctgcc tcaaggtcga ctggtaaggc tgcagagcca
11641 gaactcaaag agatgtcttc taaatccagt gctctctcca gcacatctca gctacatcct
11701 ctatctttct catcagtata ggtcttcatg tatacaaagc atttctgcat tcattgttca
11761 cactgcttct gccacagctc atgaaaagga caatgcagat gtctaaatgg caccagatct
11821 atacccacct tttttttttt tttttttttg agacagggtc ttgctctgtc acccaggctg
11881 gagtgcagtg gcataatcat agctcgctgc aacctcaaac ttctgggctc aagtgatcct
11941 cccgcctcag ccttacaagt agctgctgct acaacaggca cgcgccacca cgcctggcta
12001 tttttaattt ttagtagcga tgaagtctcg ctatgttgcc taggctggtc tcaaacacct
12061 gggctcaagc gatctgcctg cattgacctc ccaaagtgtt ggaattaaac ggtgtgagcc
12121 accgtgctgt cctatccaca cttttaaaga tgctaataaa taacgtgatc accatcagag
12181 aaaataaatc ctacgtatct ggcatcactg agaagtacaa aatttcacac aagcaatttt
12241 cctactaact gaaatttatc cctttttaagg caaagctctt tctttatatc aaaacatatt
12301 ctccaaaata cataagatat gttactcttt acttagaatt aatgaaattt aatcagggat
12361 attcgactat aaacatgata taaacatcct acatactgtg ctaggaattc aaagattttt
12421 tttttttttt ttgagacaag gtctcactct gctgcccagg ctggagtgca gtggtgcaat
12481 ctcagctcac tgcaacctcc acctcccagg ctcaagtgat cctccctacc tcagcctccc
12541 aagtagctgg ggctaccggt gtgcaggacc atgcccagct agttttgtac tttttgtaga
12601 gatggagttt tgccatttgc ccaagctggt ctcaaactct tgagctcaag cgatcagccc
12661 accttggcct cccaaagtgc tgggattaca agtgtgagcc actgtgcctg gcccaagggt
12721 aaatctttaa acttgagttt ataatcttgt agaggaagag acacatctgt aaccataata
12781 tgaagccaaa gaacatgggc ttggggtgtg ctgaaaggaa ggtaagataa cacaagcaag
12841 gctagtgggg accaacaacc cagggtgact tactgaagag aagtgggagg cactgaaaag
12901 tcctgggcag gagaatggta tactacttgt gcttttttgaa aagatgactc tagcacaaca
12961 gtgaggccag aggcagaaaa tggacttagg tggcagcgac agaggtagag agaaaactgc
13021 actgccaggg caggggcagt aggaactaaa acaggtggct ctgaaaggca ttacagacgt
13081 agagacaatg ctcttagcat ataggggcgt gggaaaggtg gggagctaaa gagaacacta
13141 agattcttgc tttcatggaa agatttcaga acgagaaaat aagagtaggt actggtttaa
13201 aggagcgtga gggactaact caggatggga tgatggggaa tgctttatag aggaagtgac
13261 atttaagcta gatattaaaa tatgagcagg ataagtagag tttcaccaaa aagtaaaaaa
13321 gcattctagg cagagaaaac agcacatgca aagacagacc atattacagt tgaacaatgg
13381 catgcagttt tagggaggtg catcagatga taagaagggt aataagtaag gccaggggcg
13441 gtggctcacg cctgtaatcc cagcactttg ggaggccaag gcaggtggat catgaggtca
13501 ggagattgag accatcctgg ccaacatggt gaaaccccgt ctctactaaa aacacaaaaa
13561 ttagctgggt gtggtcacat gcctgtaatc ccagctactc aggaggctga ggcaggagaa
13621 tcatttgaac ccaggaggtg gaggttgcag tgagccgaga tcatgccact gcactccagt
13681 ctggtgtgct ccagtcagag caagactcca tctcaaaaaa aaaaacaagg gtaagtaata
13741 ttaaattgtg aggcacttgc ctcatactaa gaaattgtag gtgatgagaa gacaatcaag
13801 gtttctaaag gaagagggta aaggggagca gcatgatcag ggctatgttt cagaagggaa
13861 actgtataaa gtacagactg gaatggtaag acacaggagg aaggcaatag agcataatgg
13921 ctaagattac aaggtctgaa cttaagacaa cctaatacaa attctgggtt tatagcttaa
13981 tagctctgca accttgggca agttatttac tctctctgtg ccctcatttt ccccagctgt
14041 aaaatgggaa taataatagt actttatcag gcacaagcca ccacacctgg ctattttatt
14101 tttaattttt gtagagacag gatcttgcta tgttgtccaa gctggtctcg aactcctcaa
14161 ctaaagtgaa gctcccacct tgtcctccca gagcactggg ataacaggta tgagccactg
14221 tgcccagccc atagttccta attacgcctt tttgtatttt attaatttat ctttctctta
14281 ctgccttaag ttccatgaaa tcacaaactg tgtgctcacc gctgaattct tagaaatcag
14341 cacaatggct ggcacagcat gagtgcttga taaatacttc ttgaatgaat gaaaggttcc
14401 aaactatata ttttaaatga actctattta aatgtaacct ttatatagaa ccccaggaca
14461 cacagaagaa gacagtttga agtaagggat atttggagac ttgttccctc tgcttccccc
14521 tggttcttaa gtcagttcta tagttctgta aagctttaca gaatgcagtg caaaaaccat
14581 tgtctgttac aagcaagaag taatgtatgt ctgaagtaag gcaatgacaa taaagatgca
14641 acaatagctt tgagaactat ttatgacagg ccctgacaac caattacagt aggccctctg

# FIGURE 4-E

```
14701   tatccatggg ttccacatgt gtggattcaa ccaacctgag atgaaaaaca ttttRgggaa
14761   aaaaaagcat ctgtactgaa gatgtataaa cttttttttc ttgtcattat tctaaacaat
14821   acaatgtaac aactatttac atagtattta cattgcatta tataagtaat ccaaggatga
14881   tttaaactat acgaaggacg tgtgaaggtt atatgctaat attataccat tttatataga
14941   gacttgagca tccaaggatt ttggtatctg tgtgaagtcc tggaaccaat ccccacagat
15001   gctgagggac aactgtataa atgaagaatg agaaaaggaa aattcttgaa gcacctctaa
15061   atttcctagc ttgagtgaac aaataaatga acatgtgtgc caaaaagcaa gataagaatg
15121   taagagtggc tgggcacggt ggctcacgtt tgtaatccca gcactttggc aggtcaaggc
15181   aggtggatcg cttgaggcca ggagttcgag accagcctgg ccaacatggt gaaaccctgt
15241   ctccactaaa aatacaaaaa aattagccgg gcacggtggc aggcacctgt aatcccagct
15301   acgagggagg ctgaggcagg agaattgctc aaacccagga ggtggaggtt gcagttagcc
15361   aagatcacac aattgcaatc caccctggga aacaagagca aaactccatc tcaaaaaaaa
15421   aaaaaatgta agagtaacaa taacaatttc cattcatttg aacatagtat cacccctatc
15481   aagcaaatac tattaatacc ctcaagttat agaataaatc tttcacgatg ttaagcaact
15541   gtcatgatct tcattagcaa agatcatgag gctaataagt agcagaatta agtttcaaat
15601   ccaagactgt cccactccaa gcccaaaatc taaaccgtct tgctatattg gcaagcagtt
15661   tgagagttag gtcttagacg tgagatatct actggatata cagttagaaa taatcagtaa
15721   ctaatgaaaa atatgatttg gggttagaga taaggatctg ggctccattt ggaagactga
15781   ataaattcag aagagtgtct atgagaagat catgcttgct tgtaaggcct gtaaatgggt
15841   gatgtacgca tttgcgattg aagtttgttg atttaccaag gtctctgcct tgcatagatc
15901   tagtgggcat cRgcattaag agaggaagta aaaaacttta ggagaaattc cagatctctg
15961   gggattcctg ttaggacctt tatattattt ttgttttggg agaatttaac agaaagacag
16021   tgacaccaat gtcaacaaaa aaaaatgaga cagcaaaggt gataaagaga atagatttgg
16081   ggatcatcat tttctcttct gcttgagatt ccacagctct caacaaaaat gactattatt
16141   tgtgtatggc tctttacaac taacaaagtg tttcccattt acaaggtatt tcctgtttac
16201   caataatgtc agatgaacct tacaacagct gtttaaggtg agggcattat tattttcact
16261   ttaccaaaga ggaatgaagt ccagaaaagt taagtttacc ttgtctaagg tcatacagct
16321   aaaaagcaac cgggccacga tgtaaccacg gttttctggc tcctaatctg ggctctttc
16381   cagaagactc acctgagata acatgagcca ctcacttatg aaccaagagt catcatccct
16441   atgtatagtt tcactgtgtt taaaaataaa acaatagggg gatagtgact gggaaggggc
16501   taatggtatg ctggtaatgt tctgtttctt gatgtaggtg ctagtaacac aggtgtgttc
16561   accttgtgga aagtccttga gctgtaaact ttgtgtactt ttctgtatat atgtcatact
16621   gtaattatta aataccaggc aagccaggtc aggaggcttg cacttgtaat cccaatggct
16681   caggaggctg agatgggagg atcacttgag cccaggaatt caagaccagc ctgggtgaca
16741   aagcaagacc cccatctctc aaaaaaaaaa aaaattcagc tgggcgcgga agcttacacc
16801   tgtaatctta gcactttgag aggccgaggc agacagatta cctgaggcca ggagttcgag
16861   atcagcctga ccaacatggt aaaaccacat ctctactaaa aatacacata cacacacaca
16921   cacacacaca cacacacaca caattagcca ggtgtggtgg tgcacgccta taatcccagc
16981   tactcagaag gctgaggcac aagaattgct tgaacctgga aggtggaggc tgcagtgagc
17041   tgagatcata ccagttcact ccagcctggg tgacagagtg agactctgtc tcaagagaaa
17101   caacaacaaa taaaaaaaaa ttagccaggt atggtggcat gcctgaagtc tcagctactc
17161   aggaggctaa ggcaggagga tcacttgagc tcaggagttt gaggcttcag tgagctacga
17221   ttgtgccact gtaccccagc ctgagtgacg cagcaagatc ccatctctct tttttttttt
17281   ttgagttgga gttttactct tgttgcccag gctagagtgc agtggtgtga tcttggctca
17341   ttgcaacctc cgccttccag tttcaagcga ttctcctgcc tcaacctgcc aagtagctgg
17401   gattccaggt gtctaccacc acccccagct aatttttgta tttttagtag ggacagggtt
17461   ttactatgtt ggccaggctg gtcttgaact cctcaccttg tgatccgcct gcctcagcct
17521   cccaaagtgc tgggattact ggcatgagcc atcgcgcctg accaagatcc catctcttta
17581   aaaaaaaaaa aaaaaaaaaa aaaaggcaag ccaaactgat aatcaaaact caggaacact
17641   aagttggatg cgtcacattt tttactcaat ttctcaaaca gtagctacat ataggacatc
17701   taccagttat ataaggatgt acaaaattct tgcacactca aataacatat ccacccaatt
17761   gtcccaatcc agactgtctt agtgatgtga aagaatctga caggactgga agtaacagaa
17821   gcccaggaaa gggcggagat ttttgttaat gaaaacaaaa atggcattga aaatggaact
17881   atcctacatc atcatcaatg atgagatatc tcaatttcag agataacaaa aatgtgaaaa
17941   aaaaatggcc agtgacaatt ggaagatact atcaattgta aaatacaccc caatttcagt
18001   gttaaaatat taaaaaagca aaaaaaaaaa aaagaaagaa agcattttaa aacaaaggaa
18061   atatattcta atctcaaaag gtagctcaat tgtgggataa cagatttgtg aagcacatct
18121   ccttgtacaa gctctttgtt ttagagataa ggacactaaa gccttcattt aagttattaa
18181   ataaccttac taaattaaag ctcacacatc agaaagttga taactacaat tacaaagctg
18241   gttccttcaa tgctcgttct cctgccacta cccagatgcc tcctttaaat ccaaaagaag
18301   atttacatat taattccata ttaaaactct attttgactt tcatatttct ttacttcatt
18361   aactcactaa aaaataataa aagttaacct ttaaaaatca attaaagctg ggcgaggtgg
```

# FIGURE 4-F

```
18421  ctcatgccta taatcccagc actttggaag gctgaggcag gtggatcact tgaggtcagg
18481  agttccagac cagcctggcc aacatggtga aaccccatct ctactaaaaa tacaaaaatc
18541  agttgggcat ggtggcaggc gcctgtattc ccagctactt gggaggctga ggcatgagaa
18601  ttgcttgaac ccgggaggtg gaggttgcag tgagccaaga ttgcaccact gtactccagc
18661  ctaagaaaca gagcgagaca ccgtctcaaa aaaaaacaaa aaaaaacaaa aaacaaaaaa
18721  acaaaaaact aagctgggtg cggtggctca cgcctgtaat cccagcactt tgggaggctg
18781  aggcaggtgg atcgcctgag ggcaggagtt tgagaacagc ctggccaaca tagtgaaacc
18841  ctgtctctat taaaaaaaaa aaaaaatagc taggcgtggt agcaggcgcc tgtaatccca
18901  gctactaggg aggctgaggc agaagaatca cttgaaccca ggaggtggag gttgcagtga
18961  gccaagattg tgccactgca ttacagcctg ggcaacaaga gcaaaactac atctcaaaaa
19021  aaaacataat aataattaat taagatgaaa cgatgtttag gaaacttcat ttcttgctta
19081  tacaagtttg ttttccttgt tcttcaaaac cagtgaagca atacaaagtt agataaaaga
19141  caaaattagt aatctcccag ccccttttcca ttcctctcca aggcctccta caagatgaga
19201  aatggtaaca tatttttgtg tcccccaaaa acataaatac aaatatattg gaaagggttt
19261  tgttgttctc tgtttttaga aaactaggtt taaactttac acattgctct gcaacttatc
19321  tcatctaaca atttatcaac atctccccaa gcatcaataa ctaaaattct aactcatact
19381  tttaaaaagc tacataaaag tccatataag ggatatccat aacgtgttca catattttc
19441  tattgaggga atggctattt tcagtgttac catttcaaac aatgctgtaa tgagtatcct
19501  tgacgtattt caagttaaag aaaaaaaact atttctacac acatactttc cttgttgtcc
19561  catgttttga ttactcctgg tctcatcatc atccctacct cccttgctca gagaatcatc
19621  ctcagggtcc agtgtgatga cagagagcaa agtaacttgg agaatgtcaa gacaagacat
19681  gtgatagtta ctgttggtaa attggaaatg gcctgtcttg gctgcctctc tcagtcactt
19741  gcacatgcta tccccctcct ccccttacat gtgctccctg ttctggtctc agcagctgct
19801  tccaactcaa ctgatgtgag cacagctagc tacYttgtca cactcctaaY cctggcaagt
19861  gcagacaggc ccaggagctc tccaaacaat tggaaaggag aaggaataca ttatcactgt
19921  gaaataatat aatacggagg tatctagagt aaaatgtaaa actccctctt cattaagcct
19981  caacccacat ttgccagtga cactccccag ctcagaagtg accactgtta gaagtctgtt
20041  gtacaacctt ttggattttt cctaagcatg tacacataca ggtatatatt cacatacagg
20101  tatgtgtgtt tggtctacat atctgtaatc ataccatata atatgttctg caacttgccc
20161  ttttcatgta Maaatttatt tgacatgttt ccataccagc aaaaaacaga ttcctcacat
20221  ttttaattaa tttttttga gacaaagtct cactctgtca cccaggctgg agtgcagtgg
20281  caccatcatg gctcactgca acctcaacca cctgggctca ggtgatcctc ccacctcagc
20341  ctcctggtag ctgggactaa aggtgcgcac caccataccc agctaattta tttgtagaga
20401  tgggatttca ccatgttgcc caaactgttc tcaaactcct aggctcaaac aatccgccca
20461  ccttgtcctc ccaaagtgct gggattacag gtgtgagctR tcatggctgg caattcctta
20521  aactttttaaa agcttRaaaa aatcctatgt aagaatgtgc cacaacttta ttttcccatt
20581  ttgctacatc tggacattta ggttgttcac agtgttttgc atgtcaaaga atgctgtatt
20641  gataacactg gcagcacatc tctaatttta cacacaagct aatatttctc taggatatat
20701  accaataatt agaattaatg ggttgaaaag cttgtgtatt ttaaatgtca acagatattc
20761  ccaaattacc atctaaaact gtgtactagt ttatagtctc accaacagag gataaaaatt
20821  cccatctaca cccttctacc aatatatttt atcaatatat aagatagaat atattattgt
20881  tttaatttat atcttatttc cagaaaagtt aaacatctcc tcacatatgc aactgttcat
20941  tcttatttct tatgaaaggt attaccattt tgattttaaa catgagggtt cagagatgct
21001  agagtgacac aaccagtaag tccagacatc aaagctgttt ttttaacaat ttcccatcaa
21061  aactaggcct ctttctgtca agtgtaattc cccctaccag cagccagcaa actatggcct
21121  ataagttagc agcccatatt ttgtaaatca ggttttacta aaacgtagcc acgcccattc
21181  atttaagtat tgtctatggc tgcttttgtt ctccaatggc agaggtgagt agttacaacc
21241  acatggtctg caaagcctaa aatatttact actggggtct aaacagaaac aataacttgc
21301  ctaagcacag tgactcacac ctgtaatccc agtgctttgg gaggctgaag gtggtggact
21361  gcttgctgtt tgagcccagg agttcgagac caacctgggc aacatggcga gattccatct
21421  ctacaaaaat tagaaaaata gccagttgtg gtggcatgca cctgtaatcc cagatactca
21481  ggaggctgag gtgggaggac tgcttgagcc tgggaggcca aggccgtggt gaactgccat
21541  tgtgccacca cattccagcc tggaagacag agtgagactc catgtcgaaa aaacaaaacg
21601  cacacacaca aaaaaaacaa gagttaaaaa ctaaagaaaa gctcaggtct gggcacagtg
21661  gctcatgcct gcaatcccag tacttaggaa ggcttagcga gaccccatct ctacaaaaaa
21721  taaaaaaaat taggctaggc acggtagccc acacctgtaa tcccagcatt ttgggaggcc
21781  gaggcgggcg gatcacgagg tcaggagaat cgagactgtc ctggccaaca tggtgaaacc
21841  ccatctctac taaaaataca aaaattagct ggccgtggtg gcgtgtgcct gtaatcccag
21901  ctactcagga ggctgaggca ggagatcact tgaaccaagg agttggaggc tgcagtgagc
21961  cgagatcgtg ccactgcact ccagcctggc gacagagcaa gactgtctaa aaaaatttaa
22021  aaaattaaaa attagccagg cacagtgctg tgtgcctgta gttccagcta cttgggaggc
22081  tgaggcagga ggaccccttg aaccaaggag ttcgaggttg cagtgagcta tgatcacacc
```

# FIGURE 4-G

```
22141  acggcactcc aaaatctggg tgatagagca agaccctgtc tcaaaacaaa aaaagaaaag
22201  cttctcagtg gggattagtt atggtctaat tggccacagc cagagcatgg ctatgttaca
22261  gtgtgtggct cattttcaaa taactttata tgcagtatag gtctggtata taatcccaaa
22321  ggaaagagcc caaaacactc aaatgaagtc aaaagactta tctttactta tgggttgcaa
22381  cactaaaaga ttttctatgc actactcttc gtcttccttg tttcttttct ttttttttt
22441  tttttttttt ttgagacaga gcctcattct gttgcccagg ctggagtgca gcggtacaat
22501  cacagtttac cacagcctca acctcccagg ctcaagcgat cctccttcct cagactcctg
22561  agtagctggg accacaggca ggtaccacca cacctggcta atttttgtat tttttgtaga
22621  gacagggcct tgtcatgttg cctaagctca tctcaaactc ctgggctcaa gtaatcctgc
22681  ctctgcctcc caaagtgctg ggattatagg catgagccac catgcctggc cccttattta
22741  atttcatctg gttcctactg tccttcaatg attttgacaa tgtagttcct acccacacac
22801  cacaataata caaaaccaaa gcaaactcca ctatgtctgc tgctaaacac ccccaacaaa
22861  cacacataca catcacacca caccacacaa cacaacacag gtgtctcctt ccctttttgct
22921  agcagattcg ctaggaagca gctgcacatt tccagcaagt cgaacaaact gctgagagct
22981  agggcccctg accattgtgg gaaaggaagg ccaattctcc cctctagatt gtacccttct
23041  gaagatcctg gccatgaaag aagtcatttc tccagagaat tttaagccat tcagttccta
23101  tacggcaggc aggaaaggtg tcatcctccc catatcatca gtggaggaac tcagtgtaac
23161  ttgtttcatt tcctcttact tcaaaaSgtc attttaagta atagggtcat ttaggtaata
23221  tcagtgatct ttttaatttc tcctcgcatt ctctcccaaa ttttggtttt acaaaataat
23281  ggttaaaaaa aaaaagctat catactctcc acgaaattta acttaatcta gaattttgaa
23341  tgagaaatta ataatcaaag aacagtgcat tttaggccag gcgcggtggc tcactcctgt
23401  aatcccagca ctttgggagg ccaaggcgag cagatcacct gaggtcagga gtttgagacc
23461  agcctggcca acatggtgaa accccatctc tacttaaaat acaaaaaagt agctgggtgt
23521  ggtggtacgc acctgtaatc ccagctactt gggaggctga ggcaggagaa tcgcttgaac
23581  ctgggaggtg gaggttgcag tgagccgaga tcgtgccatt gcactccaga ctgggggaca
23641  agagtgagac ttcgtctcaa aaaacaaaca aagaacagtg cattttctta cacaaataag
23701  caggtattaa gtggttctct ggataggaat ttggcaatac aaatcaaaat taatgcctat
23761  aatcctagca ctttgggagg ccaaggtggg cggatcacct gaggtcaaga gtttgaaacc
23821  agcctgacca acatggagaa acccgtctta aaaaaataca aaattagctg ggcgtggtgg
23881  cacatgcctg tcatcctagc tactcgggag gctgaggcag gagaatcact tgaacctgga
23941  acaggtggag gttgcagtga gctgagattg tgccattgca ctccagcctg ggcaacaaga
24001  gcgaaactcc atctcaaaaa caaaacaaaa ccttgatatt tgggccaggt gtggtggtgg
24061  cacatgccca aaatcccagc actttgggag gctgaggtga aaggattgct tgagcccagg
24121  agttcaagac cagcctgggc aacatagtga gacctcatct ctacaaaaaa taaaaaatta
24181  gccaggcatg gtggtgcaca cctgtggtcc cagttactcg ggaggctgat gtgagaggat
24241  cacttgaacc ctgaaaggtt gaggctgtgg tgagctgtga tcataccact gcactctagc
24301  ctgggtgaca gagtgagacc ctgtctcaaa acaaagaaac aaagaaacaa aaatgttcat
24361  atttggctgg gtatggtggc tcacacctgc actttgagag gccgaggcag gatgatctct
24421  tgaagtcagg agttcaagac cagcaatctt ctcgcctcag cctcccaaag tgctggtgct
24481  aggatgatgg gtgtgagcca ctgcacccag cctaagatag taaattttgt tacatacatc
24541  ttaccaaaac tcaaaaaaaa aaaaaaaaaa aaaaagctgg ctgggtacaa tggttgaagc
24601  ctgtcatccc agcactttgg gaggccaagg taggaggatt gcttgagccc aggagttcaa
24661  gaccagcctg ggcaacacag tgagaccatg tctctacaaa aaatttaaaa attagccagg
24721  tgtggtggca tacacctata gtcccagcta ctcaggaggc taaggtggga ggatcacttg
24781  agtctgagcc tgggaggctg aagccataat aagctgtgat cataccactg cactctagcc
24841  tgggcaacag agtgagatct tgtgtcaaaa aaaaaaaaaa aagaaacaaa aagaattaaa
24901  aaaaaaaaaa tctcatgttc tttcatcaaa taattccaat cacagaaata aaagtttagg
24961  aaataatctg aaatacaaag atatatggac attaatagaa ttatttatca ttctgaaaaa
25021  ctgaaggaaa actaaatatc taacaataac ttaaattatg gcaagaatat acatatgatg
25081  aaatcttatg cagtcactta aaatgtttta aataagtttg gctgggtaca gtggctcacg
25141  tctataattc cagcactttg gaaggctgag gtgggatgat tgcttaagcc caggagttca
25201  agaccagcct gggcaacata gtagactcca cttctacaaa aaataaaaat aaaaaaaata
25261  gctgggcatg atggcatgcg cctgtagtYc agctacttgg gaagctaaag cgggaagatc
25321  acttgagccc aggaggtcaa ggctgcagtg agctaggatt gcaactgtac cactgcactc
25381  cagcctgagt gacagagcaa ggccccgtct ctggggaaaa aaaaaaaagt tttaaataat
25441  tttaatgaca tggggaaata ctcaagaaaK gttgaatttt aaaagcagga tataggctgg
25501  gtgcagtggc atatgcctgt aatcccagcc ctttgggagg ccaaggcagg tgaatcgctt
25561  gggctcagga gttcaagacc agcctgggca acatggtgaa accctgcctc tacaaaaaat
25621  acaaaaatta gccgggcatg gtggcatgca cctgtagtcc tagctatttg ggaggctgag
25681  gtaggaggat tgcttgagcc cagaaggttg aggctgcagt gagctgtgat cgtaccactg
25741  cactgaagcc tgggtgagag agtgagaccc tgtctcaaaa caaacaaaca aaaaaaaggc
25801  aggataaaac tattatggta tggccccatt tttgaaacta tatatacaaa tatttataga
```

# FIGURE 4-H

25861 cagatagata tatcatatac atatttatcc ctctccccac atacttgtga gtttgtatat
25921 gtgtatgtat acgtgtaagg aagtatacca aataatgttc atttctgagt tgtggaatta
25981 tgggtgcctt ttactttccc ccttaacaca ttatttttca tgttttctaa atagtaccat
26041 catatacaat taggaaaaaa ttactttcat attgtttaat ctagtaatct atttgtagat
26101 atgtatcata agaaaataac caaaaacgta gacaaaaact tatgcataat aatgttcata
26161 taaagataca cagaaaacta ttaataaata atggttactt ctgcagggca gaattagggt
26221 aaagacttat ttggggactt atttgttata tctattacaa aaatgatttg aaaaaaattc
26281 cattattatt tgaaaaaacc tgaaagcata taaaatcct atatgataga ctattatgca
26341 gccactacaa atatttatga agagtatata taaatttata taatgaaaaa atggtaaatg
26401 gggaaaaaag cagaatataa aatttttaggt aatatatgat ctggaaattg caaaaccaga
26461 aatatacaag cacaaaatac tgaaagaaaa taggctaaaa taagaacagt agttttctcc
26521 agatagtggg attacgggtg atttcagttt tcttccttat acttctctgc gtttttcaca
26581 tatcctagaa tgaatatata ctatttttca atcaggctta aaaaatgttt ttggtttctg
26641 ccctataatg aaaagcctgc tccttgtgtg aaattttatc cacaaaggat tatcagtagt
26701 aagcaaccca ctagcaagaa gactgttcat aatgtatatg cctaagaaaa tcatctttgg
26761 tgggtcagtg agctcagttt aaagggacac agctctgacc acttcctgcc ttcttgcttt
26821 tggagttgtt ttagtaaata taactgagga atcaagttct caaacatgag atgcctgaca
26881 aacttaatac tgtactgtaa gaaccaggac caccaccttc tctcaaccta acctctaaga
26941 ctttacactt aagaatagac aagaccgggc cgggcgtggt ggctcatgcc tgtaatccca
27001 gcactttggg aggccgaggt gggcggatta cctgaggtca ggagtttgag accagcctga
27061 ccaacatgga gaaaccccat ctctactaaa aatacaaaaa ttagccgggc gtggtggcac
27121 atgcctgtat tcccagctac tcgggaggct gaggcaggag aatcgcttga acccaggagg
27181 cggggggttgc ggtgagccga gatcacgcct atagcctggg caacaagagc gaaactctgc
27241 tcaagaaaaa aaaaaaaaaa gaatagacag gacctaaata acatagattg ggtcatgtta
27301 gtctgtaaat caaaaacact taacagctaa ttacatgaag aataaaatcc aaatttctta
27361 gcataacgct taaggttctc taagatccaa ccctcaaatt tccttcccag cctcatcttc
27421 agttcattcc ctaaaatatc ctctacctca ggcacatttt ccccatacat gccaggcatt
27481 ttccaagctg tgtgcctttg cccatggtgt gacctccatt aaatgccctg tctcctaaac
27541 ctactgaact cctattcttc ctttacaacc tggctcaaat gccttctccc aaactctcct
27601 tatcgccccc accatcagga acaattgttc tttcctctat gtgctcccat attgcttgtt
27661 cctctacaaa cttaattaca atcctccctg ggtggatgct agctgtaata cttgtttcat
27721 tttactaaac tcctggaggg cagaaaccat tttaaaatca tttccactcc agcacactgc
27781 ttgcacacag taggtactaa atatgctgcc tgagtaaatg acagccaatt ggcccttatc
27841 tgaattcttt cagtggtaca gccagggatg gcaaattgtc aaagctagtc cctctaattg
27901 caagttatac atatagcatg agtgaccagc agaaaatgcc aagggcctgc cactttaaga
27961 ggactggcaa gatgactatg tttacacaat cctaattaaa actaaagggc aatgagggaa
28021 agaagagaaa tagggagatt aagaggtgtg gattctacag gtcacctcct cttcttagtg
28081 agcttgctag gcactaggct ggaagatctg agctacaaag tagcctattt tatctttggg
28141 gtagcctgta ggatctaagt attgaaaagc aactaattag gccactttgt cctccagcaa
28201 ctggccctgc tcttgggcat taacttccaa ctggtacaca aggctttttc tctaaatgaa
28261 tcccactgta ccaatcacta ggagaatccc tttaaaaaag gcttgaaatt aataacagca
28321 ggaaacaaag tctctcaatc ctctgaatca caatatccac actgtccatt ttgatgaaat
28381 tcaccccccac ctcccttcaaa caataggtcc taccaaggaa tgacttctct caggaccaac
28441 atgtcctgtg attgtttttc tggagcaagc ttccagtgcc tgcttctcat taaatagcta
28501 cttaatgtct gatcacccag ctgtgactat aagtagagag taaagtaact ataatttagg
28561 aagtaaaaaa gaaattccac tcctacccca agtccagtag tcaaacagct tagaatagac
28621 atttccctac ctcaaacctt cttaggctta tccaattttc agatctagaa tttttagcat
28681 ctcttaactc tttcaatata aaaatgagga aaacaatgtY tagagtaaga aagagaattg
28741 caaatgatta tacagtaact tagtcacaga atcttttgac caggctgtct aacacctcat
28801 ttaactgatg agttttcaag accactgttt ccaggaacag gttacagggc actacaatat
28861 aatcagctta atttgcatat attcaagata acagccccta ggcttgaccc cttcaccatc
28921 ttcctgccta cctttttttca aacccacaac tgctaacaac aacaaaaaaa tcaatgtagt
28981 gatttaatac atcatttctg cctaaaaaaa gactggagct accaaaccat ctgcttcagc
29041 ctcaccacct gggattcatc ttgattgatt tgggtgtgtt ggtgggcagt ggctgccaca
29101 gcctgaagaa ctcttccaac tctttctggg ggtcttctgg agatgggact gcaatggctc
29161 ctcagctgca gtttcctata gagcaacatg ttgaaacaca cttgttccct caacacactt
29221 gtgtttcagc cctcaacaca cttgtgattt gggaggagta ccaggagaaa gaccagtttt
29281 tggcagggcg cggtggctca cgcctgtaat cctagcactt tgggcggcca aggcgggtgg
29341 atcgcttgag gtcaggagtt caagaccagc ctgaccaaca tggtgaaacc ctgtctctac
29401 taaaaataca aaaaattagc tgggcatggt ggcacatgcc tgtaatcata gctactaggg
29461 aggctgaggc aggagaatcg cttgaacccg agaggtggag gttgcagtga gctgagatcc
29521 cgccattgca ctccagtctg ggtgacagag agagaccctg tctggRaaaa aaaaagaaaa

# FIGURE 4-I

```
29581  gaaaggccag ttttctccat tatctagtca ccccaaccta taagcaagca tatgcacagt
29641  cactataaag gaaagtgact tgttttccag cagacatttc taatcttatt aaatcaaatg
29701  aaaactgcaa tcaaaatact ctccttagga agttatttac ttattctaac aaaacacttc
29761  tttgggagct gcctttaaag tctactccat gtgagacaaa cagaaaaatt cggtgttact
29821  gttttatagt cacacctcat ccttcaccaa aaaaggtatt gccttgcatg aatctttggc
29881  tcacggcatc ttctggctgt ttctaaaaat tgaatccacc aagctaagag taaagatttg
29941  ctacctctga ggatattcaa aagattggct cagaggcagt ctgaggcaat gacaccatca
30001  ccaaaggaaa tctaaaggct gagaacacaa ttactttgaa ggggtcaaac ttaaaaacat
30061  tctaattgcc ccataattac aatcaagggg gcaagacatg tacaatatct gtcacatttg
30121  ttcaactatt atatcacttt ataattacac attaYataat gataaatttt ggccctggaa
30181  gaaatcttca agatcatata atcctaactg tcccttttta catatgagaa aagttcagtc
30241  cattgaggtt aggcaatttg tccctggtca cacatctggt tagcaacagt caggatgaga
30301  aacccagaca cccaaactcc cagcataatg cacccacaca tacttatgta tacagctcct
30361  cctcccctgc ttctcacttc cttcactccc ctgatcaaaa gggacagaaa taacttggga
30421  cactaaagca caccaagaat ggttctgaaa cccagtagca gagaaatgat ggcttaaaaa
30481  ccaggacttc tctgcagtgg cctcaatggc caatgtttag tgtctggctc acaagagttc
30541  tgggcaatct acctcagagc aagggccaat gaaaacctct ggacaccacc ctcccacagt
30601  tagaaaaata tctttattaa acctctctgc tctcctccta cagtaacctg tttgcctcaa
30661  ataattgaaa gacaaagaca atcactttga agaatctggg tcagggaaaa agtaaatggt
30721  agagatcctg ctcaggacaa agctgaccta tSaaaaacta gagggcagaa accattttga
30781  cttacctcct ggtggaagcc tccacacact agaacagggt gatctccagc agtctggctt
30841  agaattagtt tccacctagg gaaggggcaa agggacagga atgattattc attcccctaa
30901  cccacccact cttagtgagc tcagcctcac attatcctgg gcaaccccag cgttctgtgg
30961  atcagacagc aagcagaagg gaagccatgc aggccaggtc aacggggtga gtcagtggca
31021  gagcaggaag agtcactggg gcagaaattc tggccttctg tggcaagact gccaagccac
31081  acccacactt ggtcagcact gtgaaggccc tgccctgtta ggatttcagt ctttaagcca
31141  gaaaacttct tcctcaggag gccccacacc actctcattt tcaagtgtct ctcctcctgc
31201  tcaaaaatct tccagttttc ttctccctaa agcatcatat ctaaattctt tggcttaact
31261  ttccaaatcc tccattatat ggcctcaagt catttattca accttatttc ccactattct
31321  acagaatata ctctgaattg atcaagccag tcttcccact tctctctgcg tgagccaagc
31381  cttttctcat ttccctgctt tcaattatcc aggagtggta aattcaaaca tcttcagggg
31441  tcaggcaggt aacagaaaca aattatccag gcaaggccca gtggctcaca gcggtaatcc
31501  cagcactttg ggaagccaag gtgggcagat cgcttgagct caggagttcc agaccaacct
31561  gggcaacata gtaaaactcc gtctctacaa aaatacaaaa aattagctag gtgtggtgga
31621  gtgtgcctgg gtcccaccca cttgggaggc tgcagtgagc tgagaccgtg ccactggact
31681  ccagcctggg caacagagcg aaaccacgta tccaaacaaa acaaaatgaa tgatccagtg
31741  gggcacatgg cagatctagg aaacgctgca cccccctaaa ggcattcaaa tattttaatt
31801  caaatattta tgtagtgtct cactgtgagc atcacgataa ccttttaaat agttattaat
31861  actctatctt gcaaatgaga aaacttcccc aaggttaggt aactggcaaa atgctgggca
31921  cagtctctag acctgtctga tgccaaagca agagctctac accattaggc ttctagcagt
31981  gcaaacacca tggaagatac agaaccctca aagtcacttt tcatgataca caaccttaat
32041  agtaggatgg gaagcccaaa tcacagctta tctaaccata tcccaactgc ctccaattct
32101  gttcattttc caaaccccat ctttctgcca aagtatttca ttcctagtta tccaagtcct
32161  actgaattcc ttcttctgta aattcctata gggtttatag attagacaac acaaataagt
32221  gctaaattat atactgtctt gcaaaatcag atactgcttc tatgtatttt agtgtcagcc
32281  tcatcaatca aactattgta ttaatctata tagttcttct accaaaaatg cctaacctga
32341  atctaatcaa gaagaatcag acaaatgcta tgaaagacaa aaaggcattc agattaaaaa
32401  agaactgttc aaagttaaag cagactaaag acatgatagt catgacaact aagtgcataa
32461  atacatgcat gatccttcaa tgaatcctga attttaaaaa actcaataaa agacatcact
32521  gggaaaagat gagaaatctg aacaagtatt atatactgga tgtattgaat caatgttaag
32581  tttcctgagt gttaatagta ctgtagttat ataggaagat accttttttct taggaaatac
32641  ttgctgaaat agggattaaa taacatgata tttacatcta acagtcaaaa agattcagaa
32701  aatgaaggaY aaatagacag caaatttggc aaaatgttaa tagtgactct aaatgagaag
32761  tatataagta tttgttacat tattttgcaa aatatctata ggtttgaaaa ttttcaaaat
32821  aaaataattt agtaaggagg gaaatcctgt attatatact tttttatata ctcagctctg
32881  tgacagatat aataggcact cattacttta ggtatctgct cactacctaa ggaaactggg
32941  catttggagg tcaRcaatca gacagagtcc actttgctgc agctcatgag gggtatagta
33001  ggaaattctt agggaataag aggtttctcc aaaactcaca ttgtcacacc acaggaaatc
33061  tagaaaagca ggcaattacc tcactcctca ttgaggccat cacagaagta caggcactga
33121  acaaatgccc actcgctagg cagaatgcag ctgctgccca cacgttagta cccagtggta
33181  aggtggatac aatgtgacct cagaggacca agagttgagg tcacattgca tccaccttgc
33241  caccgggtac caaaaatttg acaatctgca aaacaagtgt ctctggccag tcacaggaag
```

# FIGURE 4-J

```
33301 gcactcaagt atcaaataaa catgcctgaa atatatgcag tctttcctcc tcaccagata
33361 aaccagtcct cagctgcaat aaggcagaag aaatctgcaa ggctgaatca ctggtatttc
33421 ctgtgcagac ttcaaagaaa ctggcccttc aataggacag aaagggcagg cgacacagct
33481 gccaacagag gcctcaaccc aggagcatgc tatgcaataa tgccttctct cccttctaca
33541 gccatcagat acctcatttt tacttctgcc aaatggcccc ttattaccgc gactgattac
33601 ctaatagcca accctttacc ttggttctct cctcccctca atgagaccca agatactggc
33661 ctttctagac agctcctcct ccaatttgcc agaggaattg tcaaaactat tcccctggcc
33721 aggcRcggtg gctcacgcct gtaatcccag cactttggga ggccaaggcg ggcggatcac
33781 actgtcagga gatagagacc acggtgaaac cccgtctcta ctaaagatac aaaaaattag
33841 cYgggcgcgg tggcgggtgc ctgtagtccc agctactcgg gaggggaggc tgaggcagga
33901 gaatggcgtg aacctgggag gcggagcttg cagtgagccg agatcgcgcc actgcactca
33961 agcctgggtg acacagagcg agactccgtc tcaaaaaaaa aaaaaaaaaa aagctgccta
34021 ggccaggcac aatggcttac acccataaac ctagcacttt gcgaggccga ggcaggagga
34081 ttgcttgagc tcaggtgttc aagactagcc tgagcaacac agcaagacct tgtctctact
34141 aaaaatcaaa aaaattagcc gggtgtagtc ccagctacac tacttaggag gctgaggtgg
34201 gaagacagcc actgcactcc agcctgggtg acagagcgag accctgtctc aaaaaaaaaa
34261 aaaaaaaagt tgcccacagt aggaggtaga ggagaggcca ttattaagca cactctcagt
34321 aaaccatgaa tctgacagag ctacccaaaa catcaatgca gtcttaagct gcattagtag
34381 aagatgactc tatgccccac ctttgaagag caaagttaga ggacttgcag aggaagaaca
34441 ccaatgacca tggcaaaggg tctagaagta tatcatttca aatatggagg aagaactaca
34501 cacttttagc caggggagaa caagaaaact tttttcaaat atacgcatag ctttcacata
34561 gatgaggaga aagacttatg agtccactat aagaatacaa aaccagaact aatgagtgga
34621 agttacagga agactgattt tggtttgcca cagggaagaa atttcagcat gttgtagaag
34681 ttcagaatgt gggatgtaga acccagaatg cttggattag gatgtcaact ccaccactta
34741 taaactgtgt ggctttgggg agattaatct acctgtgcct cagttgcctt ccctgtaaaa
34801 gagaatagta ataatagaac ttagctcaca gagctttaag aagtagcttc cgaggctggc
34861 tgcggtggat caggcctgta atcccagcac tttgggaggc tgaggcaggt ggatcacctg
34921 aggtcaggag ttcaagacca gactggccaa catggggaaa cccgtctcta ctaaaaatac
34981 aaaaaaaatt agctaggctt ggtggcgcat gcctgtaatc ccagctactc aggaggataa
35041 ggcaggagaa tcgcttgaac ccaggaggca gaggttgcag tgagccaaga tcacgccatt
35101 gcactccagc ctgggcacca ggagcgaaac tccatctcaa aaaaaattaa ccagacggta
35161 gtggtgtgcg cctgtaatcc cagttactcg ggaggctgag gcaggagaat catttgaacc
35221 tgggaggtgg aagttgcagt gagcagaaat cacaccactg tactccagtc tgggtgatag
35281 agagaggctc catctcaaaa aaaataaaaa gaagtagttt ctgtaaagta cttagaacag
35341 tgcctagttc acaatatacg ctggaaaaaa aatgttacct attgctatta ctaaatgttg
35401 atacaacaga ccaggctggg tgtggtggtt tgtgcttgta atcccagcac tttgagagaa
35461 ctgcttgagc tcaagagttt gagaccagcc tgggcaacat agtgagacct cgtctctact
35521 aaaaataaaa agagttgcca ggcacagtgg cgtgcacttg tagttccagc tactcagcag
35581 gctgaggtgg gaggatagct tgagtccagg agatcgaggc tgcagtgagc tatgatgacg
35641 ccactgcact ccagccagag tgacagagca agactctgtc tcaaaataac aaacaggctg
35701 ggtgcaatgg ctcacgcctg taatcccagc actttgggag gctgaggcgg gtggatcacc
35761 tgaggtcagg agttcgagac cagcctgggc aacatggtca aaccctgtct ctactaaaaa
35821 tacaaaaatt agctggttgc agtggcacgt gcctgtaatc ccagctactt gggaggctga
35881 gacaggagaa tcacttgagc ccgagaggcg gaggttgcag tgagccaaga ctgtgcaatt
35941 gcactccagc ctgggcgaca gagtgagact ccatctcaaa ataaataaat aaataaataa
36001 ataaataaaa taaaaataat atacaataca aaaaacagac cagataacct gaagcccttc
36061 ctactaaata tgcctagaag tgctcagaaa aatataataa acatccttct agactgtgcc
36121 agctattaag gtattgtctc tcagctccaa atctaccctg ttatgctccg ctttgtgatg
36181 ctggggcctg gactccacta aaccccattt cacatttgcc agctggatcc ctgtcaacag
36241 ggggtgctaa ggggaaaaca gaagacaaga gaaggctaca agggacttcc tccttcttgt
36301 agcctggtgt ttcttgacaa cagaagttgg ttcttcccag ttgcYttcta aactcccaaa
36361 accagcctcc tgcccctcaa aggcaccagc atcagctaag taaggctcaa cagaagccag
36421 ctcctccagg ttcctcctat aagggcctga gaaaccagca gcaccaggta acaccctctc
36481 cagagaatct gggtacagct attccagatc cctctagact tctaggttct ggtagcccca
36541 accttcctcc ctttgttttt cagtcctgga tgagaaaaat tctttatgca gttattatat
36601 tgaagttact ttaatattcc ctttctgctt tttcagccct ccaccaaggg tttaaatcaa
36661 tctcctttat ggagttcact ctattgtgtg gtttctgttt tgctgattgg atctgaactg
36721 acatatagat aaactgagct ctcaagaaag taagaaaaat ccccaggaac cagaaacaaa
36781 ttagtgagga ggattagcga gctgatgaca tagctgtcct agccgtgtat ggaatttgta
36841 acaccctcat aggttcagaa gacagatctt gagtccacaa aaggcactga gatccctgtg
36901 cagaggctga gattcttaag tttatactca gtaagataat agactaggaa aaaaaatctg
36961 tccagagaga aataaaaaac ttgccttgct cacctagtga gaaaggaggg aaaaaccgtc
```

# FIGURE 4-K

```
37021  taggaattca aaacaggctg ggtgaagtgg ctcacgccag taatcccagc acattgggag
37081  gctgaggtgg gtggattgct tgagcccagg agttcgagac cagcccgggc aacatggcaa
37141  aaccctgtct ctacagaaaa tataaaaatt tgccgggtat ggtggtgtgc acctgtagtc
37201  ccagctactt gggaggctga ggtgggcgga ctgcttgagc ccaggaggca gaggttgcag
37261  tgagccaaga tcctgcaact gcactccaac ctgggcaaca aagccagaca gatcctatct
37321  caaaaaataa aataaaataa aataaaattc taggaagcag acattaatat gtagtgaaaa
37381  acaaacaaac gatcagtggt tcctggtatc agggtgaaag gaaaaacgga ctgtaaagaa
37441  gtatgagagc cggcacggt ggctcacgcc tgtaatccca gcactttggg aggccgagga
37501  gggcagatta cctgaggttg gcagttcaag atcagcctga ccaacatggt gaaaccccat
37561  ttctattaaa aatacaaaat tagctgggca tggtggtgca tgcctgtaat cccagctact
37621  caggaggctg aggcaggaga atcgcttgaa cccaggaggc agaggttgca gggagccgag
37681  attgcaccac tggactccag cctggagaat aagagtaaaa tccatcttaa aaaaaaaaaa
37741  gcatgagaaa tcttttgggg gtgagagaaa tcctctgtat cttgattatg gctgctgttt
37801  acaggtgtag acatctgcca aaactcaact ttcttgaaaa ttattacaga cacaaaattg
37861  aaaacatgag acaaaaacaa gatcacgaaa aacaagcaga tttgaaaatg aaccaaagag
37921  cacttctaat aaatgaaaaa gttagctaat aaaaataaga attcaaaaaa caaattagac
37981  ctagctgaag agtaaactag tatactagaa gatagatata aggaaattat ccagaagtta
38041  acacagaaag atagaaatgg aaattatgaa agaaagaata agatgtagac gacagaatga
38101  ggaagttcaa cataMgttac gcatgagatc tgagagaggt aaagaggtaa tgattgggaa
38161  tttttacaca attagtgaaa agatatgaaa tttcagatta aggaattact atatgccccc
38221  agtatatagt gatttaagaa aattaaattc acaccatgta atggaagtac agaacacaaa
38281  tgacagcaaa gaagatctta aaaacaaaca gaaagaaaga caggttaccc acaataaaat
38341  aagttaattt gcaagcatag ttctcaagag taacaacaaa aagccagaat ttcaatgaaa
38401  aaacactttc aaagtgctaa gagacaataa ttggaagcct agtacccagc taaactacca
38461  atcaagactg aaagtgagct gggcacagtg gctcatgcct ataatcccag cactctgaga
38521  ggccgaggcg ggaggatcac aaggtcaggg gatcgagacc atcatggcta acacaatgaa
38581  accctgtctc tactaaaaat acaaaaaatt agccaggcgt ggttgcgggc gcctgtagtc
38641  ccagctactc gggaggctga ggcaggagaa tggtgtgaac ctgggaggcg gagcttgcag
38701  tgagccgaga ctgtgccact gcactccagc ctgggcaaca gagtgagact ccatctcaaa
38761  aaaaaaaaaa aaacaaaaaa ctgaaagtga aataaagatt ttttaaatga agacagtgtt
38821  taccactaac agattcccac tggacaaaca tctgaaggat gtatgtatct atgcaataaa
38881  aaaggaagga ataaaatgca aggaaaaata gtgaaaaagc aaacagtaga catacatggg
38941  taaaatgaaa taatcactaa caatacaaaa caataaacat attttaattg tgttggggtc
39001  tcactctgtt gcccaggcta gctaagaact cctgggctca agccattctc ccgcctgctc
39061  ctcctgcctc agcctccaga gtagttggga tgacagggca agccaccatg cccagctaca
39121  aaataataat tatcaattag aattgttcaR cttagagatg gattgacttt gggaatgatc
39181  tcattctaga aagtactcat tttgagtctg gacaacacct tttggtatgt ttagtgtaca
39241  ctgggtaaga ggctaaatta aatgaccaca ccatatatta ggttaatgca aaagtaactg
39301  tggtttttgc cattactttc aatggcaata actgcaatta cttttgcaca aacctaatat
39361  cttctgatca tagttttatc aattagacac agctgtccaa acagcccctg atcagctctg
39421  ttcagccacc tgagcagggc tttgccctca tcctaaaagt tctagaattc aagtcttatt
39481  tcaaggcatt cttcccactt ccatgttcca catgtaaaaa agaattctac gtttcttaac
39541  gagctttcag gtaagatctt taagcttttg gaatgtaccg cttttcgctg tgaactaaga
39601  gcttagacat tacaattagt catcccttgc tagaacttag gctacgaaaa gaatactttg
39661  gaagctttca caaaaaagta tgtggttctt cctctaaaga agaaaatacc acacagaaag
39721  agggactgag gaaacataaa cccatctgtg ctgacagaaa gctaccttac atgatcctca
39781  tgttcgcccc aggttcccat atattcacag cagtaaagat ctccacaaag cagagaggcc
39841  caactccctt accattaaag ctttaacgac cagacaaccc agaagtttgg aaacatagaa
39901  ccaggcaaca gatactagag agagggaatt atgtattaaa gcaagaaaat atcatcacgt
39961  tttcaagagg gagtgccatt ccagtaaaga ggacagctta atactgaggt tcaaatgcca
40021  ctaaatgaaa acgggaaagc gaaattcctg tgccctttca gcccaagaaa ttattccaaa
40081  gcatacattt attcccatta catgtcgcaa atcctgccta gccattagga tacagtgacg
40141  aaaaagtctc tcgttatcta gaagggcaga caaacacgca gacttttaaa ataccagatg
40201  gcaggccggg cgtgatggct cacgcctgta atcccaagca ctttgggaag ccgaggcggg
40261  cggatcacga ggtcaagagt tcaagaccgg cctggccaag atggtgaaac cctgtgtcta
40321  ctaaaaatac aaaagttagc cgggtgtggt agcgggcgcc tgtaatccca gctactcggg
40381  aggctgaggc agggaactgt ttgaacccgg gtggcagagg ttgcagtgag ccgagatcgc
40441  gccactgcac tccggcctgg gcgatagggc gagactccgt ctcaaaaaaa aaaaaaaatg
40501  acaagtgcta actcagaggt aagcaaaaga gagtacccag aggcggcagc atcaacccag
40561  tgggctcccg cccacccaca atcttcacac agtcacacca cttatagtca taaatttgta
40621  ctctccgagg tgcgtgtatg aaacatgtta tctgtgagag tccatggatg aaaaacaaaa
40681  acaaacaaaa attaaaaaaa aaaaaaaaaa agacttaacc tgttttgacc aggacaacca
```

# FIGURE 4-L

```
40741 ccccaggaag taggtaaggc aggtaaggtc attgtttaca tatatgaaaa gtgaggctca
40801 aaatacactg accagtccaa gacaacgcgc gcccccacaM agcaccttcc ccgtccccat
40861 ttatgcccaa cccataaacc caaagtagag ccaacctcgc actggctacg taacccgggc
40921 caagctaaac attataaacc tcaggctccc catctttaaa gtataacagg tggttagaag
40981 attaaaaaga taaacatttg gcaggtcatt gtggacggtg tagctcagaa aatgctgact
41041 actgcactac cataactact gctattgtta acgataaaag gcaaagactt tccaggaccc
41101 gaccccctcgc ttacaagcct ttcgggaact tgccctatac ccccgacccc agtcaccttc
41161 agatttaatt ccacagacac gcccccaaac agaccctccc ctttaaggca cccccccccc
41221 ccccggctcc tccctctcag gcgcctctcc tcacaaacct taccccccata gattctgccc
41281 tttcggactc ggttatggag gaggccttcc gctcgcagcg gcctctccag cacccctttt
41341 aggcctgagc acccgcaggg gtgcccaggc cgcacgccaa acgcgggccc gcgccggtta
41401 cctggctgcg cgctcccgct ctgcctcgcg attctccgga atcgtacctc ttcgtgggct
41461 cgcgccagcg ctgtgagcgc acaattagtt taaactatgg ccccgccccc tcgcgctgcc
41521 ctctgattgg cctctgtcgg cggggcccgc ttaaggaccc gggaaaagga agtttgaggg
41581 ccactgggaa agagaagggg tatcaccgct tccggacccc ggcctgtact tgaaggcaaa
41641 gagaactaca aatcccagcg tcacccgcgg ccttgaagcc ccgcccctga caaactgaag
41701 gtcccggtaa gcatcgcgtc agtacttatg gcgcctgccg ggttgtggtg acgaaagcag
41761 ttgccatgga gttgctctga gtaaccctga ggcagtggga cgccaagact ggagaggaag
41821 cgactgcggg tgagtcgggc gggaaaacag gaaacccgtt ctaggggaca agagatctat
41881 ggagaaggga ggggcactca cagaacgact aagaaccttc ctgctcgggg atctgagatt
41941 tcccccaccg accctcatca cctttcacRg cgaacaagat acaacactca catgggacag
42001 actgggcgag ggggagcaga gggagggata ggcgttgaga tataYatcca tacagatttg
42061 gggatgctta agaaccaccc ccagggccgg gagcggtggc tcacgcctgt aatcccaaca
42121 ctgggagacc aaggcgggcg gaccacttaa ggccaggagt tcgagaccag cctgagaaac
42181 atggcgaacc ctgtctctac caaaaacata aaaaaattag ccgggcttgg tggcgcgctc
42241 cagtggcccc agctactagg gaggctgagg tagaaggatc gcttgagccc ggagattgaa
42301 gctgcagtga gccgtgatca cgcaactaca ctccagcctg ggctatagag cgatacctcc
42361 atctcaaaac aaaacaaaac gaaacaaaac aaaaacaaaa caaaacaacg aaccccgccc
42421 ccaacgcaaa cataaagctc ccaaaatcgg atgcttcaag ttccaggcat aacctgggag
42481 atcagaaacg ccctcagaaa tggggttggt tttcctctga ggaactgaga ccctcttcca
42541 agacatgcgg ttagcattag attgagtaaa tcgcagtcgc taccctaatt ctagagatgt
42601 aggaaacttc ctggggcgtg aagtgcctca ttccttcttc ttccaaggag taaaacccct
42661 tctcaatctg tccccagatt ccctttctc cccacacctt ctccgtggtc cgctactcaa
42721 agctctacag ttagagcttc atttttttta gtactcacga cagatctatt acatttagta
42781 acctacccag tgctttcaca gtctctcttt tgatactctt aacagctctg tgagtcaagg
42841 atatttatct tcatttcaca gattggaaaa ctgaaaccca gagaaaaggg acttgcccaa
42901 ggccacacac caaattagtg acagagctag aactagactc cagttctccg acttccagct
42961 aagtctcttt agcttcagcc tactgcctct atagcggaag gacttgttcc aaggaacaag
43021 ttaaactgcc ccagggaaaa ggaacctgtg tggcctactt tgtattattt acacttcagc
43081 tggaatgaat ttcagatcct cccctgaaac ttttaatggg gaaaaggaac tagagagact
43141 aagacaatga gacttgaaca tttttaacta aacaggaaac agaagggcta acacagcacc
43201 catgtctgaa gtgaccatga ttagctggtt tctagctggt gactagctgg ccctgactgg
43261 tccacttact accttaaagt tctcccaggt ttggtcctac gccctcttgc cactgcactc
43321 tatattctct tcttgggtac ccctccacac ccacaacttc attttccacc tgtatgcaaa
43381 tgtacacaag tgtgtatctc aagcccagcc ctcatctcta agctccagat ctatatagcc
43441 aattgcctca acatctcctt taggatacct caaaggcaac ccaaactcta catttctgaa
43501 attgaaacaa tctctgtgta tcccaacctt ttcctccaag cctggatctc ttccagtgct
43561 cctaccttta tccacaatat acccaagccc aaaacccaag ttagtacctc ttccatttac
43621 ttaatacctc ttccatttcc catctatcac taagtcttat cagctttact tcctgaatat
43681 cttttatata ggtcctagct accatcacat ccttcctgcc ttccctccca gccatatttg
43741 cgcactgcaa ccagagtgat ccttcaaaat gcaaagctga tactRttcac tctctacccc
43801 acccccaatt tttttttttt ctttttgaga cagagtttcg ctcttgctgc ccaggctgga
43861 gtgcaatggc gcaatctcgg ctcacagcaa cctccacctc ccggtttcaa gcaattctcc
43921 tgtctcagcc tcccaggtaa ctgggattac aggcatgcgc caccacaccc ggctaatttt
43981 atatttttag tagagacggg gtttctccat gttggtcagg ctggtctcga actcccgacc
44041 tcaggtgatc tgcccacctt ggcctcccaa agtgctgaga ttacaggcgt gagccgccac
44101 gcccagccag cccccattta atattaatac tatgtggttt cctattgctc ccatctctcc
44161 agcctccagt ctaaccatac atattcatcc ttctctcccc ttcagttata taacctttaa
44221 ggtcttcaaa atttccaagt acccttccac catagaacct ttgcatatag cagtcacatc
44281 tgcctggaat gctcattcca caccaccccc taccccataa gttaatttgt acttatcctt
44341 tagatttctc tcagtcactt cttcaggaat gtcttcctgt agactgttca gaccaggtca
44401 gatccctcaa ttacagcaac ctcatggccc tctcctttgt agtRttattt agttgcaatt
```

# FIGURE 4-M

```
44461   tttaatgtgt ttgttaagca cctactactt gccggcacta tgagatacaa ctgtaagaaa
44521   aaatatggtt tctgccctgc tggtgtttat ggacatacag atagtaagca gataattaYa
44581   gtgttacgag tgctaccatg gcaatcatag cactacaatt acagatttga ggataaaggt
44641   agacttctca gaaaagttga aacctgatct gagccctgaa ataagaatta gcaaagcaaa
44701   aggaggagag aaaaactata ttccaagcag acagagctgg aaggagacac tgttccactt
44761   agaggcccag tggcaggtgc agggaactta tggtgagtgg ttgcatttgg ctagactgca
44821   gaattggagt tgaggatgag atggtagtag agggggtgg acaggagcca ggttgaatct
44881   tggtcctcct tttggcacct gggaatataa cctcccagaa tatattagat tatgtggcag
44941   atttttcttc tttttctttt gacagggtct cgctctgttg cccagactgg agtgcagtgg
45001   cacagtcata gctcactgca gcctcgaact cctgggcttg agccatactc ctacctcagc
45061   ttcccaagta gctgggacta taggcgtgag ctgccatgcc tggctaaata tgtggcagat
45121   ttttcatagc agcctttgat ttttttcca ccatcagtcc atcatcagaa actctcgggc
45181   caggctgttg aatctcctat cagatttatg tgattattcc tcaaagcttc ctgtgttatc
45241   atgttgcaac ttgcctgaga gagggcagga ataggctagc aacaaatcaa attccaaatg
45301   ttagtaattt cccaaaactg aaaacagttt tctgtcaact tgggattggt taaataaatt
45361   aagtgaataa cattaaagta cgataYatag tataaaatac tttgcaacta ttaaaaaggg
45421   taatagtcta caacaatttg aaaattaaaa aataggttta caaaggaata ttgtttattt
45481   aaaaatatac ataatctagg ctgggcacag tggcttacac ctgtaatccc agcactttgg
45541   gaggccaagg tgagtgaata gcttgagccc aggagtttga gaccagccta agcaacatgg
45601   caaaactgtg tctctacaaa aaatacaaaa attagcaggg tgtggtgacg tgtgcctgta
45661   gtcccagcta ctcaggaagc taaggtggtg ggattgcctc agcccaggag gcagaggttg
45721   cagtgagctg aggtagcacc attgcactcc agcctgcaca acagagcgag accttgtctc
45781   aaaaaaaaaa aaaaaaaaga aaaccgtgtg tgtgtgtgtg tgtgtgtgtg tgtgtgtgtg
45841   tgtgtgtgta aaaccttatt agctatacat tctaggttat attcccaaac tgttaatgga
45901   gattcaaact ttgagtttct atttgatgta cttcttgtca tttcacttta ttcatctatc
45961   atattagctt ttacagttaa aaaacaaaac agttaaagat actgtcaatt tggaaatgag
46021   aaattccagt tccgaaacaa ggcaggtatg cccctaaccc tgtcattcat caagttagtc
46081   agtctggaca gatgaaggtg ctggcatctg attacctcct ggtcataagc cagaatagca
46141   gatgggaaag actgactcca aaaacacagc cacaggagta agcatggcag catcaacact
46201   ttgtcagttc tccccagcag tcaagctctc tatgtgacac cttgaagact ccattaattc
46261   cttcatttct tctttcattt ttccattgtc catccatttt catttgtcca tcaaaccttt
46321   agtgaatatg gatttttttt tttttttgg ccaggctcaa tgcagggtaa tgatgagaag
46381   ggagaaagaa gtctgagtct tgtccctgtc ctcaagctta tagggagtaa acaaagttgg
46441   ttataatgaa attaacagag aatacaactg cttccattta ttgagcacct gatgccagat
46501   gtacaatagt tctcaaggta gggtttgtct ccatttcaca gatgagtaaa caagctgtaa
46561   taaaatttg tttgaagtac agtgggggca tgcagaagtt agctattgcc gtgtaataaa
46621   ccatcccaaa acacagtggc ttaagataat aaacatttat tattgcttct aagtcttaga
46681   gtcagctgga gggttcttct gctttggctg ggcttactca tgtgtcggca gtcatctgtg
46741   ggttgggtga ctctcctgat cttgactggg ggtaggctga ctgtgggcta gtctaggatg
46801   gcttcagctg gtatctctgg ctctttYgca tgtctctcac ctacacatct cataaccctc
46861   cagctgatca gccatgttct catggcactg gcaaagagca agagggcagg cctcctttaa
46921   agcttctgtt tgcttcatgt ttatcgacat ctcattggct aaaacaagtc acatgtctgg
46981   tcccagcatc agagtgagat gggactacaa tgttataggc aaaaagagat ggatacaggg
47041   gagaccatga atagggatca tcaaaacaat cagcctaaca caggacacat taaccaattt
47101   tatctggaaa agaaaggctt tatagaagag gctacactag aactgagtgt tgaaacatga
47161   gagagtattc actaagtggg cagctgttac ttggaactgg atgcaggctg gtttaccaca
47221   atcaatagaa actcagcagg tctttctgtg ctcatgacag ggttctatta tttgtatctt
47281   ggcttcattt cttctggtca ggcacagagc agcaagcacW caacaaatgt ttgctgaatt
47341   gaattgaata tcttactgtg aacaaggtgt ggactgaaac agctgaacct gggaaccaat
47401   tgttagaaag aacagcttag catcaactct tctgaaacag aaatcctcag aattggcagt
47461   ttctcattca ttcatcccac aaacattttg agtgcctgtt gtaaacctgg ttctatgttg
47521   gataatgaat aagatgcgat actagctcac agcctgggct acagcttaaa tgcagaaggc
47581   taaaaattta accagcttct tggggctcca tttttaatac taatYcattc agaaaatgca
47641   acaggagctg gtgctacaca aaatcagaag tctttaggaa ctgcactggg ttgctctgtc
47701   aagaaaacta acagtaaaaa tctgcccttta agtattttaa gttaaaatcc atgttcagtg
47761   gtgcataact ctggggtacc aacccagtat ttactttgag aaaagtatcc aatcttgctc
47821   aaaggtatca aatctgYtgt aggtgctgaa gtgggatgag cttctRtcta agctaggttc
47881   ctctagttct gtagtagaca gccaggttcc tctattttta caaaagaata atgtgttctt
47941   tgttgtctgc ctgtagattt aatatctaaa agattcaagt ctccctaatt gacaatccat
48001   gcccatggga ccagttcata acagaattaa gctgtgaagt gactcaatct gtcacctccc
48061   caaagctcag ctggtactgc tattttttca gaacaagaca ttcatttcc tctaaaccta
48121   ctaaacttga tccatgtata tgttaaattg ccaaaaaagg aatttacatg aactgtatta
```

# FIGURE 4-N

48181 ggcttaaact gaattataag atatcaggtc cttggttttt aacagaaatt cttttttttt
48241 tttttttttt tttttttttc ctgagacagg gtctacaggg tcttgctctg tcgcccaggc
48301 tggagtgcag tggcacaatt atagctcact gcaacctcca acttctaggc tcaagcaatc
48361 caactgcctc agcctcccga gtaataaatt ccattttaaa gttgtttgcc aatttcattg
48421 tctctgcaaa ctaatttatc ctttgaatcc aatgatgaga actgcaatcc caaggtttct
48481 ctttttgctt tggctgccag gaatttcaga gctaaatgta ggcttagatt cttaaagtgc
48541 ctggcacatc tctctcttcc tgtaatctag tttctgatct ttctcttcca tttttaactt
48601 tctaatctgc actgtccagt atggtagcca ctagtcacgt acatttgaca ggtaggctag
48661 tccaaattgg gatgtgctgt aagtgtaaaa tatctcagta agttttaata ttgattacat
48721 attgaatgat tttttgatat attgggtaaa attaagttat taaaattaat ttcaccactt
48781 cattttactt attttggcta ctagaaaatt ttaaattata tatatggctt gcatttgtga
48841 cattatattt ctagtggatg gcactattct gcaaatcatc tgttttatga ctgtgttatt
48901 attgtaaacc tgaaatcctt ttggaaagca ggtgaagtaa tacaaataat aatacaaagg
48961 tggcagcagt ggatggaatg agaaagggga ggtcgactgg gRcaagttct tgtccttatc
49021 cttgtcaatt tgtcttttgc ttgtgtgatt cacatacaaa ccccttggta aagtgtgttg
49081 tcagctctct tcatcccagg tgagtagtat tctcatggct ctcccttgtc tccagaaaaa
49141 cctcagcttt actttagcta acaaattgga aacgatctga taagacaatt cacaccacat
49201 tgcaagtgac tgataacaac attgtgttca tccctgtcag ggagtatttc cattttaacc
49261 ggaaacaatc cctgaaccca caggaatgaa tgcctaatgg tggagtttca gccatcagtg
49321 acaggtgagt gagaggtaga ctctggtttt gtgtgtgtgt atgtttatat gtgtatggct
49381 acattcagga tagggctgaa agtagggtat ttggatggaa aggtaatggg gctggcagag
49441 aagccaagcg gagaaggtgg caaatcccca gctgggccag actgagaggg gtggtgagac
49501 accaaccaga tgcggtgcag gccctcaagg atttgttaag caatagaata gcccagttga
49561 tgtcaMcaga gggtgggtat caagacagtc tccacagtaa ctactgtaga gtaacagaaa
49621 aggtctcaga gtaggattca ggagattatg actgcagtct acaactgact cactggctta
49681 actctgggca agtcctttta ccccttgggt ttcagtttct tcatctggca ttttccagac
49741 aaagaaatgg gtgttcgatt ctcaaatgag ttctaagaga tgtgaaggtg ctctgagaag
49801 tagaaagtgc tctatacatg caaggatgca aggaagagtg atttcatatc ttaaaatgga
49861 aatacagcca ggcaggccta gagtagtaga ggacagaaag cagggatgga ggactatggg
49921 gaaacctgaa agccggtgta cttgaaacac tggtctttct ctgtcctccc aggctgaacc
49981 cagactccca gggcacctgc ttgcaccttt gaatgatggc ctgaactatg aacaaacggg
50041 actatatgaa cacttcggta caggagcccc ctcttgacta ctccttcaga agcatccacg
50101 tcattcaagg tcagccccca gagcacagta ctccactcac taaggcagca ggcccagtgt
50161 ggtccctagg aagaaaccat aatctactta aaagcctaaa gagattcttc ccatatgctt
50221 gcagatgtgt tgttactgtc ttttggaggg taggggagag tgagaggttt ttttctgtct
50281 ttttgtttgt tttgattttg ccccgataca gccccaggag atcctaagaa catgtgcccc
50341 tgtttgtttt ttttaagcag caaatctgaa aggaaacagg gggctatcac agtagttgat
50401 catctttaaa agaagccagg ctacagcagg aaagtcaagt ggaagaaaac tgggaagcca
50461 gtctatcagc caatagtttt attctcctgt ttttacctga agagaggagg aaaacacagg
50521 ctcctgtgga gtcccagcaa gtaggcaaaa cctttctcct ctgtctgcag acacaagcct
50581 aatctgactg gccaaacatg aggggtctta tctacaaact ggcatatttc cagtttaaat
50641 acttactgac aaatttctta cactcattcc agccaaatcc aagtttattc catcatcacc
50701 tacacttatt gcaggttcca acactgtcca cactcgctcc cattttgcca ttcaatgtct
50761 ttgtaaatgg gtagctagtc ccttcaggag ggaacatagg gaggctgtca gccatgtcat
50821 attcccaatg agtcatccgt tcttcctagg gaagttctgc catccctggc ttttgttatc
50881 tggtctttca ttaaagcatc agtccatctg tcctcacaga atacacgtta acatccacta
50941 tctcagaact attatttggc tttagtcttc tctcaggcaa ctggtagcca ctgcctttaa
51001 gctcgggaag ctggcaaata gcaccagtct tagattgggt ttagggtcta cttgggttat
51061 catcagccac agtggtaact cacgtttgtt ctctcacgag cacacagcca cctgctgtga
51121 tctttattgt gcactgcatc acttctgatg taatccatgt ttcacaaggg gttttagctg
51181 caatagtaac tatctctaag acaggtgcaa tgacagaggt gggaaggaag tatacctcct
51241 acctatcatg ggaatttcta actctgatgg aggaggtgta gctctgccct ctatggagtc
51301 ctcagaattg ggagagagac aagcagtacc aagggtaact ctacatcaca aaacgtaaat
51361 ggtacagatg gcccatcctt cattcaaccg ttcaatattt actgagcact tactaaaagc
51421 atttatatat gtgtgtgtgt gtgtgcgtca tatgtatatg ccaggtattg tgctccttgc
51481 taggtatgtg aggcacatca aaggcccaaa aggtcagagt cccttcagat tccccagagc
51541 ttctgaaaca cttatttaca gctggggtta agggatacaa tagagtttgg gggtacctgg
51601 cacactattt ggtgaatgaa taaatattac agaatctgaa acggcagtgc ctttggaagg
51661 catctaatct gtgctaccat caacccagtt cctctaactt ttcacaaacc tagagtgtta
51721 aggaagtccc ccaccatata cacctaccaa caccttccag ttggtggttg agggatgaca
51781 cagggaagtt caatcaaact aacttgtatt ctgacacatg gcaagagcta catggcagcc
51841 tttcagagag aggaaaacag gaacatagtt tcccagatct tcctggagct aaatgctccc

# FIGURE 4-O

```
51901   cagttcccaa agcattgata tttgtggtcc agcttccaga cctgctttgg cctactctga
51961   acaaggaact ttacagattc ttaggcagtg tagtgcattg gtggaatctg agaggagcag
52021   caacctgtgg cagcctggat ataccactcc atccttgRta agagcagctc cagccagcat
52081   caagttttag gcaactctgt cccaccagtg attccctctg agcttggcct ccaccccatg
52141   cttggtggca ttccccaagc tgctacctta tcacatgggg cactagttca tgtggggtta
52201   ttttgacctt aattttgatt catctgtgtc attctgtttc agggttctMa ggaggtRtgg
52261   ggagggaagc aggattagga attcatgagc tggggtacgc cgttccagta ccctcctgca
52321   accccaggcc ccaattgtgg gtcctgctca ctctaggatt aaggcagaga gccatctggt
52381   ggatgttgcc tgacctgcat ctgaggagga ggagaggtgg gggtggaagg gcaaagggtg
52441   tccttccagt agagagcaga agagagtgaa gaaagctggc tagcttccgc aaatactaaa
52501   tacttccttc cccagggtg aactagggaa ccaaaggctt agcagcaatg ggaactggac
52561   ctcattttga aaaagtggga gtgaggcttt ggggtgttcc atacaccggg gttctaacat
52621   aatcctattc cctattctct ctcctcctca gccaaacttt ctgaggatgt tgtttatgct
52681   aactgcttat atccattcac tctttttttg gtagagacgg ggtttcatga tgttgcccag
52741   gctggtctca aacgcctagg ctcaagcaat cctcctgtct tggcctccca aagtgctggg
52801   attacaggca tgaaaccagc ctccattcac tcttgaactc actcacttcM atctggcttc
52861   tgcctccagg cccttgccat tccactgaag cagcccttac caaggttacc agggacttct
52921   aagttgccaa aattactttt cagtccttac ctccctggaa cttactacta taaactattt
52981   actccctgaa catctttaaa ctgtcctctc ctgattttcc ctgatcattt aacttctcta
53041   atggattctt cttcctccct gaccttcctc cctaccctca ctctccctga ggaatctcat
53101   ctctttccct aacttgtatg ccttctatgc taatgattct caagtctgtg tctcctgagc
53161   ttccgacctt catctccaac tgcctacttg acttctccac ctttttgtac aggaacctca
53221   aatacaccaa agctaagcct gaactcatca cattcccaat gctatttctt cttctgtaat
53281   tccttaccag ttaatacaac tggcaaccac cctttaatat tcttctacct ttacatccag
53341   tcaaccaaat tatgttgatt gtagggccct ctcatgtctt gcttggatga ctcaaacagc
53401   caaattggtc atttggtcca actcactccc taaaatccat attccacact gacacttaag
53461   agtgatcttt ctgaaataaa agatttgaat catttgcttt aaacactcca gtgccttccc
53521   attacacaca gggtaaagcc caagatcctt agcctgacat ttgagacttt tgactggatt
53581   cctgcctcct tttcctctct gtccccttg tgcagtgcgt gcaagcactt gtgcaggcac
53641   acaaacccgc cccagactcc ctggctgttc tgtacctcta taccttttctt catgctgttc
53701   cctgcctgtt taccccttctt tgcctgataa acatccccta atccatggag attcagctca
53761   ggcttcacct tccctgggaa acttccctga gctcccatac tgggtcaata gcctctcttc
53821   tgtgctgcta caactattat atagcactta tcacaccatR ttgccagctt gctcccatct
53881   tgcccatata agctcctcca aggcacaaat gtctgctctc tgccctatta ttcatacaaa
53941   gtctggaaca gactgtctct cattttgttt atcactgtat atccagtacc tggcatactg
54001   taggtactca acattcatgg aaagagagac aggatatcaa agtgtattcc aYtgaagtga
54061   attctatagg ctttaccagt agttaattca ggattttgtg ctatcatcta ctcagctcaa
54121   gcccaacaat tctggttctt agttttcctc aaccccacac tggtttggta taaaaaagac
54181   ccagtggtac agtggacatg agaagtccat gtgacctgga aagccaagag cttgagatag
54241   aagtctctgg ccccttagtct aaacttactg attgctggaa gctgtgggac agggttgcac
54301   tagactcagg cacagggcta tggccccttcc agaccctcca gctacagcct ggatggctaa
54361   tctttccagc tctgtccctt cccaccccct aaagccaaaa aagcaaagat Ratacccaag
54421   tctcactttg tccccacaga tctggtaaat gaggagccaa ggacaggact acgaccactg
54481   aagcgttcaa agtcggggaa atcactgacc cagtccctgt ggctgaataa caatgttctc
54541   aatgatctga gagacttcaa ccaggtggct tcacagctgt tggagcaccc agagaacctg
54601   gcctggatcg acctgtcctt taatgacctg acttccattg accctgtgag ttcctaacag
54661   taggaatcca gtcaggggag cctgaagcca ctttgttcag cccccaacct ctccactcct
54721   acatgttatc aaggaagtaa tgaggcagca tggtacagtc caaagcacaa ggctaggggg
54781   ccagacagat agatgtaggt tcaaatcctg ggtttgcctc ttactgactg tgactttgag
54841   caagctacct aacctctcaa aactgcagcc tccctgtcgg aaaaatgggg ataatatgcc
54901   agtatcataa aggttttgtg aggattgaat gaaaatacat gaaaaccacc gggcacagcg
54961   gctcacgcct gtaatcccag cactttggga ggccgaggtg ggtggatcac aaggtcaaga
55021   gagatcaaga ccatcctggc caacatggtg aaaccccgcc tctactaaaa atacaaaaaa
55081   ttagctgggc gtggtggYgg gcgcctgtag tcccagctac tcgggaggct gaggcaggag
55141   aatcacttga acccaggagg cagaggttgc agtgagccga gattgcgcca ctgtactcca
55201   gcctggcaac agagtgagac tccgtctcaa aaaaaaaaa aaagaaaaga aaatacatga
55261   aaaccaacaa acaactggta catcagaggc tgaagtaaaa ttStaactgt cactgcgcac
55321   ccactgtgac ctgtgctggg ttctcctgtg gaagaggtag tcaataaagt acagcactta
55381   ctttctgtga actcagattc aagttttaag aaataaagtt tggccttttc tagtccaaga
55441   tccctaagag gggaaaggag ctggagcatg acttggacac tattcccctg gtgtggtgat
55501   ctcaatttaa ccagtatttt aaaagcttct tatgtgccta gcatgatgct ctgtcttcct
55561   gttgcttgct gtgatagaat cgtagatcac caaagctgga aaggtcttca gtgaaggggc
```

# FIGURE 4-P

55621 aactaaggct gagagaggca agtcagtggc agtgctggta cccaggccca cactcctgac
55681 cctcagatca cagctccttt tcagtgtgat gccctgtatg tcttaaaaca taaataaaag
55741 taggttacag tcatacagta tggctgtgga gggttggagt ggagggcagg ttgttcccca
55801 cattcttcct tctctgccca cgagtcagcc ctgagcagag attcagggtc ctgtcctgtc
55861 tagcctggct tttggtttcc ctccccaaca ggtcctaaca actttcttca acctgagtgt
55921 cctctatctt cacggcaaca gcatccagcg cctgggggag gtgaataagc tggctgtcct
55981 tcctcggctc cgtagcctga cactccatgg gaaccccatg gaggaagaga aagggtatag
56041 gtaagtgccc tgcccctgga ggtagcgtct agctgggctc ccctaaagga aaggaggagg
56101 agaaacaaga aatctacgac cattcttctg ccatgcttgg acctgggaag ggagtaacag
56161 acctctaagc attccttgct ctcagtcagg ataaacctaa tctttcccaa actatgctca
56221 agtccctcca ttccttgcag tcagggaaga caacagcaac tagtggaggg ggaaggggat
56281 tctggtgggg gtgctggccc ccagcctaag tcttccccac aggcaatatg tgctgtgcac
56341 cctgtcccgt atcaccacgt tcgacttcag tggggtcacc aaagcagacc gcaccacagc
56401 tgaagtctgg aaacgcatga acatcaagcc caagaaggcc tggaccaagc agaatacact
56461 ttgaggctcc cacgacccta gtagtcctaa aggcctaagc atagacagca tggtttgaca
56521 ataaataatt tgagctgttg agcagatgag aagtcactta caccttgtag agatgttctc
56581 taactcaggc aactgcaagt agctctagcc ttttcttttc ttttttttt ttttgagacg
56641 gagtctcact ctgtcacaca ggctggagtg cagtggcacg atcttggctc actgcaacct
56701 cagcctccca ggttcaagag attctcctgc ctcagcctcc ccagtagctg tgattaaagg
56761 cgcctgccac catgactggc taattttgtt gttgttgttg ttgttttgag acggtttcac
56821 tcgtcacccc agctggagtg cagtggtgcg atctcagctc actgcaacct ccgcctgctg
56881 ggttcaagcg attctcctgc ctcagcctcc tgagtagctg ggattacagg cgcctgctac
56941 cacatccagc taattttttt ttttgagacg gagtctcgct ctgtcaccca ggctggagtg
57001 cagtggcacg atctcggctc actgcaaact ccgccttcca ggttcacgcc attctcctgc
57061 ctcagcctcc caagtagctg ggactatagg cacctactac cacgcctggc taattttttg
57121 tatttttagt agagacgggg tttcaccatg ttagccagga tggtctccat ctcctgacct
57181 tgtgatccac ccacctcggc ttcccccagc taatttttta tattttagta gagacaaggt
57241 ttcgccatgt tggccaggct ggtcttgaac tcctgacctc aggtgatccg tctgctttgg
57301 cctcccaaag tgctgggatt agaagcgtga gccaccacgc ccagcctttt tttgtatttt
57361 tagtagagat ggggtttcgc catgttggcc aggctggtct caaactcctg acctcaggtg
57421 atctgcccac ctcagcctcc caaagtgcta ggattacagg ataagccact gcacctggcc
57481 agctctagtc ttattttgct gaaaaaggga ggcaattgag ggaaaggcct gctggcctgg
57541 gacactggag acgtgggttt accacacagc cttgggaaat ttatctaact ctctgggccc
57601 ctttgtactt ttcagcttag agatttgggg gttaaagtag atcagtaatt tccaaactct
57661 tcacagagta ccagggtctg tagagatgcc tcacaggcct ccatgacagg ccaagaagat
57721 ggcctatgat ctctgaaacc agcccccact tcaatcagat caaagtaatt ccattctgtt
57781 ttatatgctg ggattctgct taagatttca tttgataaaa agaatcttct gcttaaaaac
57841 atttgcttgc tgactccata ttaggtattc ccagaagagc ctggtctgct ttctctctcc
57901 accccccaac ccgccatgtc tcccttcacc cccaggggct gttggtaatt ccattgagtc
57961 tgaggcaggg cccccaagaa tcaaagctgc ctcagattca ggtctgttca gctttgacag
58021 tgttcccatc atcactggac aagggacaag aacagtctca tccagtctga ggttagccca
58081 agccaggtct cagctaagag ctgacaccct aaacccttgt ctcttgggtt catcttcaat
58141 atccctggaa gatgtcccct tccccccatc ttactctcat agacctaagt aatcaaaagt
58201 agaatgggta gaacagaggg gccagaacca gcacatggat gaatatactt cctttatcga
58261 gggtgacaaa ccaaaacaaa aaaagaccaa acatgtaaaa acccagggtt ctagaaatac
58321 aaactcaatt cattcaaatt caagctcatc cagaccctgg tcacaaaccc tagtgaggtg
58381 catgtgagca ccaagtcagg gagaggggc aggagtgact ctgaggccaa cagagagggt
58441 gggaagggga tctccctagg tcccctggtg atcacccccc acccaaactg gtatctccac
58501 attctcaatg actatgaaga cataccaggc tctgtccttt tggcccccac cccatccctg
58561 gccagagctt caaaggccag tcccaaaagg ttctaccctc acttgctcag gcttctagcc
58621 ttcctcttct cctccttctt cacattttc tctgtgatta gtagcaggtt agggtactgt
58681 ataagccgca gtgaggctgg gggccaaggg ggtggggtag agatgggatg aagagaggag
58741 aagagctgtc caaggacccc tctcttcatg ggatcccaaa ctgtacaacc agctcctctt
58801 ttgcgtccac acggatctga gaaagtgcac tgtaggcata agcagctatc ctggagacct
58861 gagacagaga ggggccgggg aggagaggaa cagagacagg tgaggagggc aatcaggaaa
58921 aatggggagg ggagattaga tggtgatgga gaaggaggga atgaagggga acaggaaggc
58981 aaagagaaag acagggatgg gagagacaga aggaaaagaa caaaggggag tgaaacaata
59041 aaggttgcag gaaggagata ggaaagacat aaaaagttaa gagaacgagg tgggagggag
59101 ctggggccta caaggctacc aggctcccta actccacctt ccctacctcc tctgaggcag
59161 agtgggtgaa ccctctgcca taccctgggc agtgtgagaa gggtgggcag ggcaggtga
59221 ggggtgtctc acctgctgca aatcagagaa cgggatgggc tcactggcca gcacttggtg
59281 gggctggctg gtaagagacg gcagcggtgg cagcttcttc caatgggtca ggctgctgct

# FIGURE 4-Q

```
59341 cagcacagcc aagcgggtgc tgaccaagag agagggggtg ggggtaggca gttaagcYac
59401 agtctgccct gcccagccca ggtaagcctc atcccctcag gccagagaag cagggtgtcg
59461 aggaggggta tctgggaggg aaggtctcag ctcatcacag actcaatcta ggcccatctc
59521 ccttggcctc agYgccctca agagtgtcac agggtaaaca gggctgacat gaataactac
59581 tgaagcaccc aagtgatctg tggtcagtct ccagggctgc accaaatgag aagcttggac
59641 taggggccag gctccaaagc tggaacagtg atggagggtg ggagcccaag tagcctgagg
59701 gacccacgct ggccaggtgc tcacctgtac tgcctggcac ggtccatgta ctcatgctgc
59761 tccatgccct gtgagtctgc agcagacaca tcaatgatgt tgctatgggg agaggagaca
59821 gagatgacat gacaggtgct tccgagaagg acaaacagga ctcagtgcca ggtaccaact
59881 ccagggctat ctgacaccag tttccctcct gcctactgga gcagggctgg acatggaggc
59941 acctctcccc agtgaccttg tcccatcccc catgtcctga gcctggctcc ttctatccta
60001 gcctttaata tggatagagg agctctgacc caggcctggt cctctgacac ttacctggct
60061 gtcttggcaa ggatggaaga gagcagggcc tgctcatcag tgcgagcgga aggcaggctg
60121 tggtagttgg gctcggctcc attgagagct ttggtagggg ggctgctagg gtccagcagc
60181 agcttccgct cctctcggtc ctagaagtgg tcatgggaga gaagtcagcc ccaggacccg
60241 tagatcctgc ctttcacggg tgctaccctt agcccagctc tgaccttaaa caccaaaaat
60301 gtttctataa ggaatatcta aagtgcccag ctttatggaa tgccagataa gactcagttc
60361 ctgcctatcc ttgggagctc aactgagttg gtgttctagg ataaatgaga gtggaaccag
60421 gcagtcctag aggccctgaa aaaatggaca gaggcacaaa gcaggtagtc agcaacagcc
60481 ctggagcttg aaaaacattc taggacctgg caaagaaagg atactagagg ctggaagaca
60541 gWggagctga gacatagcaa gcactgggat ctaaagggga aatcagtgca agtcaagagc
60601 ccaagaacag ccttcctcat cccctaggac caggccttcc ccagcaaagc tgttcgcttc
60661 caagatggaa gccctggcga gtccagacaa agccctaggc cccaagagaa agcaagccca
60721 acctctccat ggccatttct caaattatgt cagggcagga atcccagccc agctctgggt
60781 cacacagtac aagtgtgttc ccttcttgaa ggcaggtccc ttcgaaggtg ttcaccctcc
60841 tgtgctctgg tgctcttttt gtacgtgact cccagaacca aactgagtgt acatggtgta
60901 tcctgatctg agcagtacag ctgtagccat gtatggtgat tcacaccagt aatcccaaca
60961 ctttgggagc ccaaggcaga aggattgctt gagtccagga gtttgagacc agcctgggca
61021 acaaagcaag acctcgtctt tgcaaaaaaa aaaaattagc tggacttggt agtgtgagcc
61081 ctgcagtccc acctacttgg gaggctgaag tgcaacgact gtatgaaccc agggtttgag
61141 gctgcagtga gctaggactg tgccactgta ctccagtctg ggcaagagag caagacccca
61201 tctcaaaaca aacaacaatg gcgctgttcc ctcccttgct aaaccacctg agtagcccat
61261 gtaaattttc agcagccctg ttcctgctgg cctgtgctga taatcacaac ccctgaacta
61321 atgtaagacc aaaccaagct tcctcattgg tttggtcttc ctccttgtcc tctttcagtc
61381 ccctgttcta tctactgtaa tatttctgga tttgaccctg ccttgagacc atcagctgca
61441 aatccttgct tgctgtatca gcagtaaatg agaaaaacag acctgctggg tatctatcca
61501 acccataaag agcaagagtg ccctgaacaa agccaaggat Rggcccaggg ggacatcctg
61561 gctaatgctg actcaccaac cttcagtgcc agactcctca atgctcccct ccaggcctat
61621 ctcagttcct cagtctttcc tgctgcaggc cgcagctccc ctgcccacaa ctccacaact
61681 ctccttgtag agggctactt cctcctgccc tgcttaggga ggcccagcct tttcacctta
61741 ctcttctttc tcaaggctag gttgcccaag tgtcctcagg caatttgatc cagttcccaa
61801 cagcaagaga cagaagctgt gggctttgcc ccatcacctc tataaactcc cagacaccct
61861 tcctccctaa atacataggc tcagtattaa agagctaaaa agacaaaatg tttgccaaag
61921 ataaaactat gtatttctaa ggaaagctgg ctccataatg aggcccaagt tagcttccta
61981 tgtgtctgtc ctcccagctc cacttcagac agatcttcct gcaggacagg gtagtctctt
62041 ccctatcagg ggttcttcaa gaagaaggtt ggctgatcac ttctcagcct tcagctaaga
62101 tcaagtgaac aacaaggtta ttttcttgaa acccagactt aaagaagagg ccatgctacc
62161 tacttcagag cagacaaaca tcaccccact cccaaagtgc gagtggagca tgggttaggt
62221 ttgtcacatc aaggtttgtc tctgcattag tgtaacccaa gaggcagggt ttcactgtct
62281 accttagcac agacatagca gcctgcccRt gactggccac ctggtctagg ggcatggctg
62341 agacagcttg gccccatgga taaagtacat ggcctagcgt cagaacacct gggcactagc
62401 acttctcagc ttggccaaat cacttagctt ctctgaactt cttcctcacc tttcaataga
62461 cataacccac actaatatga cggatgtctg gcaagagtca aattagataa ctgtgaaggc
62521 cctttgtaaa ctgtaacgtg ttgtatattt tttattatga taaagagaca gagatgagag
62581 tcggagaggg gcctgcccaa gaactcaaag agggactgtg ggatggtgga tggttatggc
62641 ccttgctaca tgaggcctca aatcacccag cagtgattaa gcaccttctc tgggtgttag
62701 gaacagaaat gagagccccc caaggagaag gtagagattc aggagagcag caggcccagc
62761 tctcccttcc aagtagaagc ttgtatcctt tctcatacgg cattaatcct tcttgactca
62821 ttccctgcaa gagtccatat tatgctttaa gggacaattt ccccactaggc tacagctctc
62881 ccagtaggca gggttattgt tgtctctttc tcaaaaaggt ggaagtgaag tgagaggtat
62941 taggcacaac acatgcctaa cacagggcaa aggaaatatg tgttcattgg gtgtatggag
63001 aattgagaac cagaagccac ccttgcttcc catacaaacg acattttcct aggcttaagc
```

# FIGURE 4-R

```
63061    ccttaatcca aaatacgttc actgaagaac gaactacttc ccaagtacca gtgatataat
63121    ctatttttgt gaccatcttc cccattagac cgtgagctac ctgaaacctt tctactctct
63181    caggtctact atatgttcaa atgtagaatc cgggcttaga gacgatcaaa aaaggagagt
63241    agacagcaac ttgagacttc atccttgaat gtcaggccag gcagggcagg gattagagtg
63301    tggggagaag tgagaatgca ctaagaggaa gagggagaat ttaaaactgt acaggtctcc
63361    ccagcctaac cctttctcca aaggtatcag atctacagcc aaattctcag aataggcatg
63421    tctccagcta aaggaaccct caggccccag gcagactgca ctgaggcaag ccaaactcag
63481    aaccaaatga acttcccgct ccatttctct tttcccacaa tctagccaaa gctaaatcct
63541    ctttgttgct tcccagacaa tttcccacat gtcccgatgt cctcactgta tcgattaaat
63601    ccttcattgg ccaattcaaa ggccaccaac ttccagagtc gtcccctggc cctagactag
63661    cagcgacctt ccttctgcaa ccccagggac taagacgata cacgaattcc tgattcatct
63721    caggacagac taactcaggg cctcagctta cccacccta aaatggattc gcaaagccca
63781    ctaatacaca gaagacacag gaagctgggg aagggagtgt ttcccgggga cctagactga
63841    ggatccttga atctgaaaag ttttgaaggt aggagcaaac ggaagtggtg ggagcgagcc
63901    ggacaggcgg ctgcctgcac accccgaggc ggggactgtg tagaaatctg gggctttaac
63961    aggggtagga gcccggcttg gcgtctcctc tgtaatctgt cccctccagg cctccgtatt
64021    cagtgagccc tgcagtccgg ctgcccgtcc tccgcaggtt tctttctggg ccccatgccc
64081    cagtgtctta gccctcattc ccgagccccg cctgccgcgg ccgcacctgg tccgagtcct
64141    cgttctcgct gctgtagcag caccccatgg ccggggtcgg gccgggcgct caggccgcgc
64201    cgaggaggga cggcgtccgt gaggagccct tccggtcaca tgacccgcgg cggcctcccg
64261    caggcaacca cccgcccccca cccccgtac cccctgtccc ggagccgctt ggccgcctca
64321    gtcaataccc cggcttccgg tcccgcccgc tagcgcgtga tcgttgtcaa ccaatgggaa
64381    ggcgtaaccg tacacgcaag cacgtgatgc ggggaaaggg cggggcggat cacatgactt
64441    catctttcca gtctctaaac tggcagtagt ggccatcgtg aattgtgtta tgcgttctcg
64501    tggccactag gccacttttc tgggccattg agcgaactcg taccaaatgc cctcgtatgc
64561    ccttgtgata tactcttcta aatgccatgc gaggggcgac tgggatccag gcctgatgct
64621    ccagatcgta cggtctgggt gcacacacac agatgatcca gatgaccagt acctagtcgg
64681    ggaacacaga gggtgtatcg cccagctggg ctgggggtga atacacagag ggtgtattct
64741    ccacctgggc tggggatctg cgacttacag gaagaaggga ccttatacta ccttcacatg
64801    ctgacgcgca gcggagataa atcctggtag aggagataga agccacctca ctgacctctc
64861    tgctttcagg cttttatctt taattctgcc ttttttaaca gcacccattg taatcgaacg
64921    cacaaagctg accatcagtt ctcttttcct aacccccgct ccatgtcttc ccttgggccc
64981    tgaacacagg tccaagtccg tccaagattg tatagtgatc catttgttgt tcatgagcgt
65041    gtgattgggt gttcaccagc atgtatcaaa tgtgccaccc tcaaatcttg ttttggcaca
65101    ttacccgtct gacataacaa gagcctgaca cctctcccac tgcatgttct ggggtgaggg
65161    gtggaggggt cagcaatgtg tttcactgga catagagaaa taatgtagtt tcctgattga
65221    tctactggac tttgtacatg cagcctggtc tgcctactgt ctttactgtc ttcgagactc
65281    agtacaatgt cctcctgatt accttctctg actccattct ctggacatgc ccagctccct
65341    atgcttaatc ctcatccagt gttaattcat gcatttattg aatcaacaaa tatttgctga
65401    gcgtctacta tgtgccacac attgttatat ctggatatgc agcagttcac aaaacagcca
65461    aagtccctgt agtcatggaa catccaatag tgatcacact atattgtatt ctctgttcct
65521    gggtcagtct cacctaccaa acttgggagt tcctggagca cagtgacagg ttctgactct
65581    tccctgtagc ccaaggcatg gccttgctta cagaaaactt gcatatcaat gagcatgtta
65641    cagtcacccc atcaccttgg ctcaccccat gccccatctt ctgatccaaa gtattggaga
65701    agtcaccaga tctcatacct cttataaccc ctcaaatcaa gaccctgagg ttctgtaaat
65761    cttttgaggt tcctgaaggg taggaggcag ctcaagctcg tccctggggc tggtaacttg
65821    aactgcaggt ctcaggtgac agcagcagcc tcagcatgaa taattgaaag ccattccagc
65881    ctcctacctc agggttatcc cccgcagcag gccctggact ccccgacctg tcctgtgctc
65941    tggtcctcca gcccaggtaa gcagggccct ggattgtggg ttcccaagct caccagttca
66001    agcccaccgc atcactcacc tattcacacc catccataca ctctttcatt tttactcccc
66061    gccatcacca ggggccttgc aagaaggccg gcaaggaggt cagatctgga tgaggaggag
66121    aatggtccag gacatgggag gagaggccca ttccccatag gtccctggag agaagagctg
66181    agtagaggga aaatgtgtgc tgtgtgttat gtgtatgtac acatttgtga agggaattct
66241    gagacagcac aggagaagag gggaatacag cggagaggag atggggctgg agaattatta
66301    aagaatcaaa gactcctccc atcctcaggg aagagcaggc agagggagag tcctggggtg
66361    ggaaagagga catctcactc taacacccta tggggtattt tgattgcatt ttactgaggc
66421    agggcacagt gatagtgaag gtgtttttga ctgcaaatct atgctctttt gctacacagc
66481    agtggtcaga ggagaccagg caggcagagg gtaggggtgg gggaagatat cccagaggtg
66541    aattagtgaa aggggtcctg aagaaggggt gacttcaagg ataaagagaa acaagtcagg
66601    ggaacaatgt gaagatggga ccaggggttg ggaccgggaa gaggtggttt ggggctgggt
66661    gctgaaagta gacagtatag agtccttgaa agtacacagg cttggaatca cactaacctg
66721    gattcaaatc ccagttctgc tctgtgactc tggacaaaag acttagcctt tctgagccgt
```

# FIGURE 4-S

66781 ggtttgtgaa atataaggat aataattgct actggcaaaa gctacacaaa taggcaaatt
66841 gtgggtatgg gattccctcc ctacctccct ccaccccagg gcccaggtag ggaccatgtc
66901 ccctgccatt gcattggcct tcctgccact ggtggtaaca ttgctggtgc ggtaccggca
66961 ctacttccga ttgctggtgc gcacggtctt gctgcgaagc ctccgagact gcctgtcagg
67021 gctgcggatc gaggagcggg ccttcagcta cgtgctcacc catgccctgc ccggtgaccc
67081 tggtcacatc ctcaccaccc tggaccactg gagcagccgc tgcgagtact tgagccacat
67141 ggggcctgtc aaaggtcagt gttccctagc cttctgctcc aagaagtacc cccaagacag
67201 tgaaggaata tttgggatct gttgctgctt aactggatga attgggcagg ttcttgatcc
67261 tcttttaggg cctctttttt ttctcatctg gaaatgagga gcttggacta agtcatttat
67321 tcagcaaaca tttattgcca cctcttttgt attaggaatg tgctaggtgc cagggagagg
67381 ggtagaggcc acagagatga atgagccaca aatgctggcc tcaaaggaac cataatacac
67441 agtggaataa ccatctcagc agataaccca tagctttgta attatctgtc tccacatctt
67501 tcctctccac catggaggct tccacatacc atttagggta gctgtctgcc tggatttatc
67561 tcaatcccag cataagtgct tttagggtct tatttaagaa attcggctgt gtggtggctg
67621 gtgcctgtaa tcccagcact ttgagaggcc gaggtgggca gattgcttga gctcaggagt
67681 ttgagaccgg cttgggcaac atggcaaaaa cccatctcta caaaaaatac aaaaattacc
67741 caggcatggt ggcacatgcc tgtggtccca gctacttggg aggctgaggt gggaggattg
67801 cttgagccca ggaggctgag gctgcagtga gctgtgattg tgccactgca ctccagcatg
67861 ggtgacagag caagaacctg tctcaaaaaa aaaaaaaaaa agaaaaagaa aagaaaagaa
67921 attcactggc tggcatggt ggctcatgcc tgtaatccca gcactttggg aggccaaagc
67981 aggaggatca cttgagccca ggagttcgag actagcctgg gtaacaaagc aagacccccg
68041 tttctacaaa aaatttaaaa attagccggg tgtggtggtg agtaactgtg gttccagcta
68101 ctggaaatgc tgaggtggga ggattgcctg agcctgggtg gtcaaggctg cagtgagccg
68161 tgatcatgct actgcactcc agcctgggca acacagcaag acttggtctc aaaaataaat
68221 aaataaaagt tcatccaatt ccaagatcag aaatacattt gatgagcaat aaaaggggaa
68281 tgaaactaca aattctaaca gagacctttc ataaatctga aagatagggt ggttgtttct
68341 gcctgggact tcatggagaa agagcaacat ttgaactgaa tctggaagtt ctgagttggc
68401 caggcagatg gcgatggggg tgggaagggc aaagccagcc acatatacac atgtggcatg
68461 aaggaatgag acagcttgat ggattctggg cacagttgtt tgggatggct gtgatacagg
68521 ccacaggtgg gaagtactga gagatgaacc tggaaaggta ggataggatc aggtccaagg
68581 tcctgaatgc caggctgaga atcccaagtt caatcccaaa ggccttcagc tcacaggagc
68641 caaggagtaa taaggtcaga tttgttggaa agattccagg gctggtgtga agactacact
68701 gtaggagacg gggaccagga gggcagctgg ggctatggta caagagacag atgagacccc
68761 ggctggttgg gagctgcagt gaggcaggta ggcatttgag atatctttta tcaggggccc
68821 tgcatccatc tcccatgtct tctgcaacag ccatctcccc tcataggtca gatcctgatg
68881 cggctggtgg aggagaaggc ccctgcttgt gtgctggaat tgggaaccta ctgtggatac
68941 tctaccctgc ttattgcccg agccctgccc cctgggggtc gccttcttac tgtggagcgg
69001 gacccacgca cggcagcagt ggctgaaaaa ctcatccgcc tggccggctt tgatgagcac
69061 atggtcagcc tcccatctcc ccaacccaga tttttgtcac cccaggcctt gcccccagac
69121 atccccttgtg aaggactccc atctaaggag aaggaagcac ctccactctg ggactgtga
69181 tgctggatgg tgtgtgagct cctgccctcc tgtcccaagt catctgcaca ttatttctg
69241 ccggatgacg aaagaaacag ctaagaggaa ggtctctgga acccagcaaa tttgggtcca
69301 gctcttactc tgcctcttgt tagctacgtg accttgagca aagcatgcat cctctgaacc
69361 ttagcttctt cagaatggaa atcacaatac tgatcctgac ttcttaggtt ctgaggtcag
69421 aggaaatgtg agaacactca tgggaagcta agccaggacc tggcatgaag taagccagat
69481 cctggtgggg tcttgactgg gagaacaatt ccccccaccc tcacctccag ctccccctat
69541 ccccacaggt ggagctcatc gtgggcagct cagaggacgt gatcccgtgc ctacgcaccc
69601 agtatcagct gagtcgggca gacctggtgc tcctggcaca ccggccacga tgttacctga
69661 gggacctgca gctgctggag gcccatgccc tactgccagc aggtgccacc gtgctggctg
69721 accatgtgct cttccctggt gcaccccgct tcttgcagta tgctaagagc tgtggccgct
69781 accgctgccg cctccaccac actggccttc cagacttccc tgccatcaag gatggaatag
69841 ctcagctcac ctatgctgga ccaggctgag gtccaggccc aggggtactt actgatgccc
69901 acccccaccc ccacccaagc aggacctca aaatcccctc cctttcctgt ttggggcctt
69961 gacacacgct gggctcaggg ctagggagtc tctcttccca cctctgacct ctttcagcct
70021 ctacactgac ctcaagtgtc aagttctatc aggctgcttg gtctcactag gccccctctt
70081 tccagagaga accatggact gacagcaaga agcctgagct cccgacccag ctctgtcact
70141 gatttgctga gtgactccaa gggaatcccc accttgctct gagatttaat cttctctctt
70201 aacacgaagg aagctggatg ggagagctcc aggggcctcc cagttctcgg cctcagaaag
70261 cctcccatcc tcagcccatg ccattctggg tgggatcaga ggaagtggca atgagttaga
70321 cgccctgcag gaatagctgg atgcaagctg ggccagagaa aatggcacag aaccctggac
70381 ccagggccag ggatgccctg gccttcccta actctggccc acctagccaa ttaggtgtgg
70441 ctgatgtccc ttgagtgccc tcttcctaaa gcccaaaaga agatgctgga ctcctctggg

# FIGURE 4-T

```
70501   ccccaccaac aaatagggaa tagacatggg tggaaaatca ctcctttgtc tttattaaag
70561   aaacttagac cagacctggc aatcaagggg tgaggtactg gccaggaagg tggagtaggt
70621   ttcaggccct ggggatttca agtgcagact gatggcctgg gaggggccaa agagaccaga
70681   tcctggcagc agctgaggag gtgcccaagg gcactttcag gcactggggc catcagctgg
70741   ttctgtgggc aggggttggg ggttgggatg cagggtagtt tgggctggcc tggaatctcc
70801   ctgaggccac cctgccttgt ctacctagat catccactgg tcctgatcct gttcgttgcc
70861   ttccatgtcc acctggagag gaggctgggt gtgggtgggg aggggcctca gccagcctca
70921   gccccagatc ctgcccctgg ctggatccag ggtttctgta ccccttggcc atcaactggg
70981   tcaggagcaa gggtccagga acagaggccc tcccccatac cccttgccta cctcattgac
71041   ctctccatca tccggtgact cattgtagtc attcatctcg tccatgtcct gcatatcctc
71101   atcatcctct gagtcctctt cactatcctc atcatcttca tcatcctctt cttcctcgtc
71161   atcatagtgc tggtgggcag gacagagcct gtaagcccta caggcctgca tggaccagtt
71221   caagaactga cccacttgag cctctctcta gggccaatga atgacccct accccgacac
71281   tccctccttg agtctagcag gctggtgcat gttctgcagg accttaatgc taggcccaat
71341   gcccacccct tctatctccc cttttaggct tttacccaga tctgagaacc acaactgctc
71401   tgggtcagag acaggacatt cagaattaga gcagagcctc ggtccactgc ggcccccaca
71461   caggccccac ctgctagagc cactcacctc tgaggctggc ttgccaatag gaaccaggtt
71521   gttgtctttc tccgcgatgc tttggagctg tgggcaaagg cacagaggaa caaggccaga
71581   gcccaagtag ggcaggtcag gggcatggga ctggcccatt ctgcccagaa gacaacccac
71641   acgtgttggg gagaagcttc ctcccagttc tcagggagat acaatccctt tcttgtcatc
71701   tgccatttat gaacttgatc caaatactta aactctctga gccttctttt ttttgtataa
71761   ttatggtgag gatgaagtga gagacttatc agcctagaaa gcatcatctg ctagatcctc
71821   aattaatagt caaaattatt tcctgtgctt tagggaaaag agcttgggct gagtgtcagc
71881   agcctgggct ctaatctggc ccagaagtca catggtaccc ctgtaaaata gggtaccaca
71941   agatgagaac cttggctccc tggctcccag cccgattatt cccagctcag ggcaaaaagc
72001   tcttcctggg tagatttctg gagtaaggct gggtcatggc ccactgaagg aggtctctgt
72061   ctgtgctgag gtctctgtgc tgtgctagct gctcactcac ccaggcctga tgctgctgtt
72121   cctgctgctg cagctctgtc tcttccacac agggtcgatc cagattaaac cacagagtct
72181   cagtcaçacg agggaagagt gaggggaaca aagtggacat ggctcctaga ctgagggaaa
72241   gggtcaagtg aatgtgtttt gctttgtttt tgtttttaat ttggttgtaa tttgatttRg
72301   ggatagggtg gaagacagca ggaaagcagc cctccccct aggaatcaga cagacctggc
72361   tttgagtcct ggctccacca cttactagct gtttgacctt ggctgagtca cttaacctct
72421   ctgagcctca gtgtcttcat ctgtaaaatg gggataataa caattatacc actgtcaaag
72481   ttattctgat aattaagtaa ataatggatc aacaaagctg tttgcaggct gttcaaggag
72541   ggatgagaat gggagtcaag tgtagggata ggaatccaga atgcagactg gcattatgaa
72601   attatacaaa attagaggta gcagggttct actgaaacta tccctacctc ctccacacac
72661   taaaacctgc tttttgaccc tttatgatgg cccataccac acccacccag tgtcagaaat
72721   ttaggacact aacgacctct catgaatcac agccaaatgt cggggtggag ggattgtagc
72781   atatgcaagc agggttcagt aagacattct aggaggcatg aataagtgga taaataagta
72841   gatggaagat tgaatggatg aactaacatt ataagggtac aatcagcttt gagcggctga
72901   gggagccgac caggagtgcc agataatctg aattaaactt aggacaggcg aactgaggcc
72961   cagcgacagg cagagactgg ctttaggtca tacagtgtgt cggggcttag aattcctccg
73021   tgaaagggat Ragcaggaga aaataatcga tgcctggtcc tgcttttgtc ttaatttatc
73081   tgaccgaccc agggccagcc gctgcccttc cctgtatctc agattcccaa cccgcgtaat
73141   taggcacgac ggtaaacacc ctcatgtcca agaagtactg acagaccagc gatctcggct
73201   ccgaaagccc aaccccaccgt cgcaaagcca ccgccctttc tttgctcatt acgataatga
73261   ccatgcagtt gaccaacaga aagaaagcag ggcgggtctc agacagaaat ttggccttat
73321   agggttgcta aacgcgaaaa aagcggggcc tgggtaggac caattaactg aaagaaatgc
73381   tgggtgacag gaagtgacac caatgaatga cggaccttgt gtgacggggt ggggcttagt
73441   ctcgaggaag cggccaatga gctggagctc tttaagggtt aaggactctc aatatggagc
73501   tcctggggag tcgggtcaaa ggaaatgggt ttctctgggg gaaaaaggaa tgaaggggca
73561   aggagacggt tgcagccttg cgtctgtact taccgcgccg ggaggctgct gccggcggcg
73621   acccggaagc gcttcaccca gcctgagcgg aagaacccac cagaatccgg gactcaccaa
73681   acgcatgcgt ccttcgtctc cttttgtttt ccccgctccc gcccactaaa ccggatgtga
73741   cgttgaccta ccttagtcac attgttaggg aaggaagtgt gccgcgccta cctatctgcc
73801   ccSccttgag tctcagccag tcggcgtctc catcctggcg ccagcactac ggtctcgttt
73861   aaccactgcc ccgtttaacc cctgcccttt tgttgccagt caccatttgc ttgttaaccc
73921   cttctgaaga agagctgaca gcaacctttt agtgcgaggg ccattcgtcc tgctgcatcc
73981   cagaaaacta atctgttact acttcagaat tgctggttga tgttaggccc ctcctatctg
74041   tgctctctca gctacagttt cccgtttgag catattcatt ctttttattt tttgctctga
74101   acaaaaatat tagagttaca atattactat attccaggcc ttgctagaaa ctggggataa
74161   atctaggaat atggtcgctt ccctggaaga cctcacagtc cagggaagcc aaaccctgca
```

# FIGURE 4-U

74221 gacatgcagt agacttagtg gtctctctta aggttgcttg ttgagttttg acattggaga
74281 ttatgtacag acttgaatga ctagttagcc tcaggcacag cattctgttg ctgggggaat
74341 cagctgccct cctcaggcct gggccaagta cacttagaga tcgccctcgt cacctctccc
74401 atcctttgct gatgcctctg ttctagtaac ctctgactca gcttcgcctt tagagatact
74461 catgctttct ggcaacagag gtccttcaaa cccaattcct attaaactcc atcacttacc
74521 agcctctttc agggacagca gttatccgca tttccagtac tgtcctggcc agatgtgtga
74581 gttttggcaa ttcctttcc tctctctgga ctcagcttct ttgtcaacca gcatcataat
74641 ctctgcccag ccttccttct atcctgggct gatgtgaaga ttggataaga attgttcaag
74701 aggtcgtgta ctgtgcatat tgaatgtgat tatattgcca aatagcactg tttgttatag
74761 tttttcatta aagagtaaac tttgcctctt ggaaatgact accttttca ttcattcaac
74821 aaatacctac tgagtgcctt agtatatgtc catcactatt cttagtagtg ggtatacaat
74881 agtaaacaaa acagaccaaa aaagtccctc ttttcatgca gccttcattc tgagagaggt
74941 aaacaataaa gaagatagat aaataaaata tgtagttgta agattgcagt aaataagaga
75001 aaaaataaat ctgagaagtg ggaataaaat atttgcaggg gaggtgtttt atttcagact
75061 ggggggccaa agtcactggg aaggtgactt ttgagtaaag gtctgaagga gcagagagcc
75121 ttctgatatt tgtggaagag catttcagag agagagaata caagtgcaga gggcctagaa
75181 tggcttgggc atagtgtcct ctagggagag taataggtag gagatgagga gttggggagc
75241 cagtggaggg ttgtgagcac agggtccaca caatctgatt tacagtttaa ctgagttact
75301 ctggctgttc tccaaataga ctggggtagg ggagacaatg gcagaagcag ggagaccatt
75361 tgggagccta ttatgataat ctagaggtag tggatgagaa gtggttgaat tctgaattta
75421 tttagatggt agagactaca aggtttgcta acagatggac aatgccagaa taagaaagga
75481 gccacgattt ttgggctgaa cgactagaag attggtgttg ccactttttt gagttgggaa
75541 ggaatgggga ggtgatgagg ggtttggttt tttttgaca gggagcttag gtttgagtgt
75601 gttgagtctg agatgcccat tagagtagag atgttggggc ggcaggaagg catatgagtc
75661 tgcaggtcag ggcagagatc tgggctggat agatcatttg ggagtcattg gcatgtagat
75721 ggtgtttaaa gtcatgagac tggatgagat caccaaggga gtgaatgtag ttggaaaata
75781 gtttcctcca atattcagaa tttgaggaaa ctaggaaaag cagcaaacca gatggagaag
75841 gaaggccaaa aagataagta gaaagctggg tgagttgatg gtatcttgga agccatgtaa
75901 agaaagggtg gtaggagaaa atggtttact gcagcctcat tctgtacaaa attgtgcttt
75961 tgtttatttc cccaacagac catgagctcc ttgagggcag ggactgaatg gttactatgt
76021 ccccagggcc cagcatgacc ttctcctgga ttcctcatct tccttctgtg acctgtgtct
76081 ccatcagttt ctcctccggc atctttttct tacaggattc ttacctcagg taccatttgc
76141 cccgtccttc ctgataattc cgcctgctgg aaattcccta gtaacagagt ttcttttcag
76201 ggtcatcagc caggctcagt aagtaatgat gactggttag ctggtgacat ttattgagta
76261 ccaactgagt gccagacacc tttctaggcc ctggggacag aggggaagca gaaataattc
76321 ctgccctaga gaaaaataga gtatagtgag tactaagaca attatagaca attatagagg
76381 agggtaataa atgttaaaat caatgtatgt agagaataca tgagaacaca gaggagggat
76441 tgatgaatgg gttctgaaca aactaagttt agacttttgt tccagaaaag gaaacatttg
76501 agtgggcctt gagggatgag taggagttaa gcccactggt aaataagtag gaaaagaggg
76561 tgcacaaaga ggcatatttc attcaagggc atgtttggag aacattgaga atctgggcag
76621 aactagatat gtggagtgga gaaagatgag aaagaatgat aggggaccca gatagggaag
76681 gtctttggtg gccctatatg gagtggatgg gatgggatct gggcatcttg ggtctcacct
76741 ttgtcttatt gccgacttgc tggggacccc aggtagatgt ctccccattc acaagcttcc
76801 gtgttctcta acataatatc taaccatgtc gccagctctg atgtgagtcc ctggaaggga
76861 ttcccctagc ttgatccttt ccattcttag atttttacat ttgccttatt tgtgatttat
76921 acaaaccagg gcctaggaat ggaaagcagg gtcaggatgg agatttctca acagaaatca
76981 ataaatccag agcccagagg ctgaagatgg aaaccatgat tctatgcaga gaattctcta
77041 ggatccagga agggccagag ttgagtggtg tgaattcagt gctagtgaaa gctgcttact
77101 cattccttcc ataaaccttg aggagcatgg ctgaccctgg taggggacag gggagaatgt
77161 aacagtcaca gtcactacct ttcatacata tggagggata gttggtctcc agagagtttt
77221 cccactcata gtctccactt atccttagcc cagccctggg gattctgcct tcatttagca
77281 gatgaggaac ctgaggctca ggaaagtaaa gcggcttgtc tgagttgaag ctacaactca
77341 agaatgtcaa aatcttgcat tgttccacaa atcttcctta tgggctgata tgtgcttgcc
77401 gatggggact cacagaggaa ttcgatttga gtcctagtcc tctgcaacta cccagtatgg
77461 tgtggatacc agactaacgc aagtcattag gcccttgagg ccagatctct gtgtgattca
77521 tctgtatttc cccggcacct tgcacagtgt ctagcacaga gtagccactc aacaaatgtt
77581 tatataagat gggttttttt ccttgggtct aatggaatta tagttcacgt gatgaatggt
77641 ataacaggga taggttctaa aaagagaaaa gtgggaatgt taaaaagtga agagcagtgt
77701 gggaggggag gtggagaagg ctcctcagag aagatgagac cttgcaagag gagttaggaa
77761 agaatattct agtcaagagc agacagcatg tgcagaagtg cagaggcctg aaagggtgtg
77821 gtgtgcccag gacatggcat tgaccgaggg cctgttgtgt ggccatcgtg gtgatgtgca
77881 ctgtgaaaga aacaggacca ggagacagta ccctgtgctc aaaggacaag gcacagcctt

# FIGURE 4-V

77941 ggaggaggac agggacatgc tgagaaaggc ttccttgagc aggggagact tctgctgggt
78001 catgaaggat gcttcatagt tcgagtcaga gaggaggaaa gaggaaacct cccaggtgtg
78061 aggggcctcc ttaggcaggg gctgtggttg gaaggaataa ggagaatgcc caggcaggag
78121 agcatggtgt gtggagggca ggctggtcgg gtgggagggg ctgcagagct ccactgggtg
78181 ggctagctga gggagtgcac cttagcgtgg agtataaggc ttgttctgcc tcatggtcac
78241 tcaacacctc atttatttcc catcattgtg gcctccctgg acctgactct gagtttggga
78301 tggatagatg cgttggaagg gatctctgtg aattatcttc cagttcccta gaaagccagc
78361 cccaggagag gtgctccagc caggtcccta tctgcatggt ggttttgcgt atgtttgctt
78421 accacaaata tatggtaagg tttcctcact aaggtttaat gggctaaggt ttcctcactc
78481 tgactacagc aaaatagatg caaagtttga agagaggccc tctgcattgg aggcaaaggc
78541 tgaggagaca cagacctaca cacacacaca cccccccca ctttcatgca gaggtggtag
78601 gcagaatgaa ggctccctgg agatacccat gtcctaatcc tatggtgtgt atcctacctg
78661 gcaaaaggga ctttgcagat gtgattaagg ttaaggactt tcacatgggg tgattatcca
78721 ggtgggccca atctatttac atgaatcctt aaaatcagat aaaactttcc cagctgtggt
78781 cagaaaacaa gacgtgacaa ggaagaagca tcagagagat tctacattgc tgactttgaa
78841 gatggaggaa agggccatga gccaaggaac acaggtggcc tctagaagct ggaaaggca
78901 aggaaacgga ttctccccta gggcctccag aaagacctac tgtcacccta attttttttt
78961 aatttttaa tttttttgag acagtcttgc tctgtccccc aggctggagt gcaatagtgg
79021 gatctcggct cactgcaacc tctgcctccc aggttcaagc gattctcctg cctcagcctc
79081 ccgagtagtt gagattacag gcgcccacca ccacacccgg ctaatttttg tatttttagt
79141 agagacgggg tttgccatg ttggtcaggc tggtctcgaa cccctgacct caggtgatcc
79201 gcctgcctca gcctcccaaa gtgctggtgt gagccaccgc acccggcccc tgacacccta
79261 aattgagacg catgttacac ttgtaacctg cagaactgta agttaataag cttgtgttgt
79321 ttgctgggca cagtggcaca ttcctgtagt cccagctact cgggaggctg tgataggtgt
79381 gttactggat agtgctctgt aatttcaggc tcttggtgtc ctgaacaaag aactggacca
79441 gacacacaca gatattaagc acagtattac actcttagag ggggagagtg gactgacctc
79501 tggcagatga gatcaacatt agttttgtgt actctgggtc tttgtgtgtg tgtgtgtgtg
79561 tgtgtgtgtg tgtgtgtgtg tgtgtgtgtg tgtgttctct tcccaaggct gcctaatctc
79621 tagccagtgt ctgccttttt gattgataga tgtgttgctt agttactttg gcccttgtgt
79681 gtcacctcca tcccataatt ttaagtacat gagtgatttg cagtccatat gcatgagctt
79741 caatgagcta attaccaaac ggggtcattt taaggatact ttttctcttt aatgtgcatg
79801 cccatctctg aggagctgcc cgcaacaggt ttggtccgga tttagcccag atgggggctt
79861 ctttttcact tttgttttgg ctgttctggt ttttatctcg cttcttgctc acctgcccct
79921 tcacctggct tctgctccct gcttttactc attctgccct tgatccaaat tttaattccc
79981 tttgctattc tcctgctgta ttttccctct tcccctgctt caggaggatc gcttgaaccc
80041 tgaagtttgg gaccagcctg ggcaacagag cgagaccttg tctcaaaaat caattaatta
80101 attaatttgt gttgttttaa gccacttagc ttgtggtagt ttgttatggc accaacagaa
80161 aacaaatgca caggtacaca cgtatatcct cccaccctga tagacaacca cagtcataca
80221 cgctcaggca gatacaggca gggagaccca ggaacatcag ctggccctaa gtgggggctg
80281 ggggagaaatg aagcttcagg tgtgtgctgc ggggtgggtg gtaaggcctg ctccttggct
80341 ggtgtggaga aactttgacc acatttctca gtcttaactt tgctctgctt cctctcagtc
80401 cttacgtcat acttttagca acctttacaa caacattttg tcctaaaccc tgttgcaaat
80461 acatgtatga acctcaaaga ctatatatta aatgaaataa gccagtcgcg aaaggacaaa
80521 tactgtagga ttccactttt ctgaggttcc tggagaagtc aaattcagag acagaaaata
80581 ggatggtggt ttccaggggc tggggagtag ggagttatta ctgttttggt attttttttgt
80641 ttttacagat atcctgtttc tacttaaaga gttattaccg ttgtcaagct gggcatggtg
80701 gctcacgcct gtaatcccag cactttggga ggccgaagtg ggcagatcac gaggtcagga
80761 gatcgagacc acgctgaaac cccgtctgta ctaaaaatac aaaaaattag ccgggcgtgg
80821 tggcgggtgc ctgtagttcc atctactcgg gaggctgagg caggagaatg tcttgaaccc
80881 gggaggcgga gcttgcagtg atccgagatc gcccactgca ctccagcctg ggcgacagag
80941 ccagactccR tctcaaaaaa aaaaaaagag ttattaccat tgaccatgaa caacacaggt
81001 ttgaattgca tgggtccact aatatgtgga tttttttcaa tcacagataa aaaatacagt
81061 attcagaggt tgcgaaaccc acatatatga agggtgggac atcagtatgt gtggatttgg
81121 gtatgggggg ctcctggaac caatccccta catctgttga gggaggattg tatttaatgg
81181 atacagtttc agctggggaa gacaaaaagt gttctggatg agcctggtca tggtggctta
81241 tgcctgtaat cccaccattt tgggaggccg aggtgggagg attactcgag cacaggagct
81301 caagaccagc ttgggcaaca tggcaaaact ccatctctac caaaaataca aaaattagcc
81361 aggtatagtg gtgtgtgtct gtggtcccag ctacttggga ggctgaggtg ggaggattgt
81421 ttgagcccag gaggtagagg ttgcagtgag acaagatggt gccattgcac tccagcctgg
81481 gtgatagagc aagaccctgt ctaaaaaaaa aaaaaaaaaa agagttctgg atggattcac
81541 aacaatgtga atgtacttaa tgtgagtgta ctgaactgga catgtaaaaa ttgttaaaat
81601 gataattttt attatatgta tgttgtcaca attaaaaaaa aaaacaactt gtagattagg

# FIGURE 4-W

```
81661  caccattttg gagaatgggt tcgggggagc ctccagtcga acttccccac tgcagtgcag
81721  ctggaaactc agtaactggt tctctgacac ctccccgcca ctcccacccc agtgccacat
81781  atccccatct ccatgcccca ctccagtcca gagccatcta tcgcctgggc tactgcaata
81841  actggctagc ttcccgccac accacttcaa accatacgtg gctgcctaaa acctttccct
81901  ggcttcccat tgcttttagc ctgtgagttg aagtccttgc caggcaagat ctgcccctgc
81961  cttcctgtcc accctcatct agagccaacc tcgtccttgc tttctgcacc ccaaccacac
82021  cccaacagtt ttgcagatct tcagacccac taaaacatgt tctgcctcag ggcctttgca
82081  caatgctccc attgcctggg actctgtcct tcttctttac tgggtcagct ccctccRttt
82141  tggtctcaag agaggtcccc cctgcagatt agctctggtg caaggagcac tcttccatag
82201  catcttctct tttcctcatg gcatctcacc taatacatac actcacacac acatttatgt
82261  gtgtgtgtgt atatgtgtgt gtatatatat aaaacatata ttttatatat aaaatatatg
82321  ttttatatat ataaaatata ttttatatat aaaaaatatt ttatatatat aaaacatatt
82381  tttatatata tatataaaat gtatggtaaa atatatgtaa caaaaaattt acaattttca
82441  cctttttttt tggacactga gtttcactct tgttgcccag gctggagtgc aatggcgcga
82501  tcttggctca ccacaacctc cacctcctgg gttcaagtga ttctcccacc tcagcttccc
82561  aagtagctgg gattactaca ggcacgtgcc accatggcct ggctaatttt gtatttttag
82621  tagagacggg gtttctccat gttggttggg ctggtctcaa actcccgacc tcaggtgatc
82681  tgcccacctc agcctcccaa agtgctggga ttacaggcat gagccactgc gcccggccaa
82741  ttttagccat ttttaaatgt acagttcagt ggcattaagt acattcacat tgtgtgtaac
82801  catcactaca tccattcccc agaactctca tctttcccag ctgaaactct gtaattaaac
82861  agtagctccc cacccagcct ccccaagccc ctggcaccca ccattctact tagtctctat
82921  gaatttttac tactccaggt accttaaatg gaatcataca gtatttttct ttttgtaatt
82981  ggcttatttc acttagcata atgtcttcaa agttcatcta tgaagctgtt gttgcatgtg
83041  tcagaatttt cttccttttt aaggctgggt aatagtcatt tgtacatatc acatgttgca
83101  tatccattca tcttttgata aacatttgga ttgtttccac ttttgggcta ttgcaaatag
83161  tactgctata aaaatgattg aacaaatacc acctatttta actatacatc taattgtgtg
83221  cttattcgtt taaattgtgc ctctcttcct catctctggg catcacaagt gttcaccagc
83281  atatcctcag cacccagcac agtacctggg catcagtatt caacaagtat ctgttgaatg
83341  aatgaatgag acaccaaaat atacctactg gacatataca gtgtctgtag tgtgtaagga
83401  agagcagagg acttgaatct ggggcccagc tcattgattc ctcattgcgt gctctgacct
83461  tgaccaaggc ccttcacctt tgtaaaccaa agagtatctg agacaaatct caaccaattt
83521  aggaagtttg ttttgccaag gctaagcatg cacccatgac acagcctcag ggcgtcctga
83581  caacatgtgc ccaaggtggt tggggcacag catggtttta tatattttaa ggagacatga
83641  gacatcaatc aatatatgta agatgtacat tggttcagtc tggaaaagtg gaacagcttg
83701  aagtggggggt gggggtcttc ctggtcatag gtagttaaga gataaccatt tgcattcttt
83761  tgagtttctg gttagccttt ccaaaggaag caatcagata tgatatgctt ttatctcagt
83821  gagcagagag atgactttga gttctgtcta ttctttgtcc acaaattatg aggaaggtgt
83881  gtagtttttt ttctttttaa tcttagtagc tatttttctt ggaatagaat gtgaggcagg
83941  tttgccctaa gcagttctca gcttgactat tccgttagta attgacttgg tcttagtaat
84001  tgacttttcc catagtaatt gacttgccct tagtaatttt ggggccctaa gatttatttt
84061  cctttcacac cttcaaacct cagtttcccc atgtgtaatg taggggcact atcagcccca
84121  cagagttctt gtcagggtca aacgaaacaa tacatgtgat aaggttcggc ttctgaccca
84181  aggaactgct cagtgaccta atctagaggc cttgatctaa gtactgaggg agagaggagg
84241  ctccaactgg gggagctgac tgactggatt cgaatccagc ccaaggtctg ttacccatcc
84301  tctggggacc ttggctctgt attcatttcc tgtagctgct ataacaaatt actacaaact
84361  tcttggctta aaccacacag atttgttatc tttcaattct gaaggtcaga agccataaat
84421  caatgtgtca gcaggactac attccttgtg gaagcttcca agaacaatca atttccttgc
84481  cttttccagc ttccagaagc cacctgcatg cctgggctgg tggcctccct tccttgtgtc
84541  cctctaacct cttgtttcca cctttatgtg tcctactact ctctctgagc tcctgcctcc
84601  ctcgcatcag gattacatca agtccattca gaaaatccag gatcatctcc ccatctcaag
84661  acccatttct taatcacatt tgcaaagtcc cttttgccat ataaagtagc attcagatgt
84721  tccagagatt aggacgtgga cacaggtacc atctctctcc catgtgggcc tcagtttgca
84781  ttttaggaaa ataagagcat tggtcatgcc ctgtcctccc tgtcaccctt gctctgtgga
84841  gttctacctg aggcaggaat attgggtgga ggccagaggg ttgggtcctg cctgcctgac
84901  taccctagca cccaaattct cacaatcttg tcaccagcag ttatagcccc agggcctggc
84961  tgtcccccaa cccgtgttcc agtgccccac tgttcccttc catttcctgg gtgcttctga
85021  ttcaggactt tttcctggac ccttcactgg actcacaaga ggggtgccct gtttattcag
85081  tccaccacgc tcaatctctt gcaggaggga gcacatgagt gagtgagtgc aggatctggt
85141  tggctgatcc aggtgctggc agcagcaagc tccatgcagg gccctcggcc agtctagatg
85201  tgagcgagca agggcaggat ccagccggct gctctgggca ctggcaggag aaatctccat
85261  gtggggcctg cggcagtgcc caggtggggg tgcccatgac ccctaagccc cagagggagt
85321  attacagtgc tctcctagtt ctgctgtcca tggacggtgg tgtgttagca gctcagttgg
```

# FIGURE 4-X

85381 cccttgcat cgtctcatgg ggtggttgcc ctctgccagc aagggcaaag agccagtgtg
85441 acagcctttc tgggtaccca cacttggtgg gtcccgagcc cttggccagt gtccaagaag
85501 aatgaggttg tgcagacaat tgaagaatga tgaaggtgga gaattttatt aagcaatgaa
85561 aacagctctc agcagaaagg ggagctggag aggaaacagg aagggccagt catcttcccc
85621 cgaagtcagg ccatctcttc tgtagtccag ccatctcttc ctcaaagtcc ggttgtctcc
85681 tcgaagtcca gccatcttcc catctactga ctgagtctgg ggcctttata tgcacaggac
85741 ggggagtgca tgctgattgg tttgtgagta tgcaaaacag gttaaagtga agacatcact
85801 caaagatggg catgacagtg tagaaaacca attaggaaag ggtaagtata tgtaaaatag
85861 gtgaagggtg gggatcaatc agaggaaagc acaacaaatg ggaagacaag tcttcaatct
85921 ggtctgagga tttagcttgt agcttggctt tcaggcttta aactggcttt ggcttggaag
85981 tggggtttca ccagggacca gtccctattt gcctaggcat ttggctgcct cctgtccctc
86041 tcatttcccc ctctgaagag gtacaactac ctgttgttag aatagggaca aagacaatct
86101 taactacttc ctgctgacag gggatgctgt tttgagcaaa cagcaatcag agctccctca
86161 gaggcctatc taagcgtccc aggttaaagg gagccatcat tcaaggctct gttcggaatt
86221 tgatggcctc taagtgagaa gaaacagttt gggttattag atgacatgga tcaaaatgga
86281 acaacaagga ggtaaagaca gctcaaaaat cccaagtctg ctgacatgcc cagataacta
86341 gtggctatag ttatgcctgc taagatttgg atgcatggtg cttggctttg gtttgctccc
86401 ttgttttcat tttcccaaaa aaacctccag attatgagca ccacatccta tttactccta
86461 tcagctgcag gataattgcc cagaactaga atattgattc atatttttac attacccatc
86521 ccctctattt cttctgagct acaaccagag atcactagtt ggttcacagg aataagcagg
86581 attaatctaa aatgtaggca aagcttaaa aacaactgaa actagaattt aatgacaagt
86641 gtatgataaa ttttgaaaca taatttttct ctctccagtc ctcatttctg ttaaaaataa
86701 accatgatag gactgagttg tttgcaaaat aaactttagt cttatacttg gcctggtcat
86761 ttgcataaag tgcagcaaga ataatcattt tcacataggc ttttaaaatt ggctttgatg
86821 gaactctgtt tcacaaggaa tctcaggtag gaccttttaa agctgagctc agccatgggt
86881 ttgtaccctc aaatacctat gagttgggta aattcttctt ttcttgaggt cccagataac
86941 acagggctct tgggcctgtt agaaaatgac attctctact caccacaggt taggaaccct
87001 gtccagggac tgtgtagaca aggtatgagg ccagtttcc caagggggatt ttattggctc
87061 tgcaagtcaa gcttgattcc ctaaaggaaa gcataccatt ccagtcaaaa ccttggtaaa
87121 ataaccggtt tctccagtta tatcctgctg caaaagaaaa tagattctta ttgcactgat
87181 gcaaataact atattgccat aagttaagaa tatttgcaaa tagtttccaa attctagaga
87241 aaccaggcag agagaaatat gctccaaatt ttcttcacag gagtatacct tactcaattg
87301 ttaaaagctg tagatagctc aaaagaagtt tccttgactc tggaaaaaac aaaacaagga
87361 tcagcaatgt tttaagcaaa aagttaaaaa ggattacttc agttttctat tagttcagtt
87421 tattcagtta actcttgttc tgcttgatat tcaggagaat ttcagctctt tatgactcct
87481 gtatgttttt cctctgttcc aatgtcacaa tctccaaagt catcagaaac ctgcatttga
87541 gggcaccttt caaagtccaa tagctgtcca caaatcatct tttgaaaagg gtcaaaacaa
87601 gacaacaatt gtctgtgaat gacaaaatat ccttgggtag tcacagtcaa aaacacaatt
87661 gacaaagaaa tttgtttttt ctctgtggtt tacaataact taacacaata accttaatta
87721 ttactgatag catatactca gacatttgag ttttagaagg cccatacaat taggccgggc
87781 accatggctc atgcctgtaa tcccagcact ttgggaggcc gaggtgggtg gatcatgagg
87841 tcaagagatc tagaccatcc tggccaacat agtgaaaccc tgtctctact aaagtacaaa
87901 aattagctgg gcatggtggc acatgcctgt agtcccagct actcgggagg ctgagggagg
87961 agaatcgctt gaaccctgga ggcagaggtt gcagtgagcc aagattgtgc cactgcactc
88021 tagcctggta acagcgcaag actctttctc aaataaatcc catacaattt tggaacatat
88081 attaatatta ttcactaaaa tataccctga agaaagtcat acattatttt tattttggca
88141 atcccatgta actaaatgtg ttaaataatc ctgtttacct ctcttttgga tgctccaggg
88201 gccttcagta gcatccaaaa gttaggggtt agaaaagaca accttgaacc tgaagtttga
88261 atttgggaag tctatcaaac acattaaaga tttaaaacac ttattatttt gaaatagaac
88321 tccagatcac cataagttat ttattttagc caaaatgatg actcaaaaat tttaaaacaa
88381 ggcaaaaacc tttcattatc ctttattatt acatgaaaat cttcttcgag agaaagtcaa
88441 atttcaccct tgcatgtaaa tccattttca gtagtctcaa ttacatgttg taatggtaac
88501 tcttagcaat ttttaatttt aatgtaaaac ctggtaagtt tttttaatta tgtactaggc
88561 acagataaag tctgactctt tacaacatag ttaatggtgt ggttaatcca tatgacccca
88621 gaccttacca aattgtaaag ctggcaagtc aaacagttct caaaagccaa agaagcagtt
88681 tataacctta caacatttag caaacctagt atctgacctg catacattag accacatatt
88741 ttcattttga taacatttgt attttaccaa ttatcttcaa aattgttttt ctttctcaaa
88801 gattaaagtc acatgaacta aaaggcaata cagcttttgt ttttccttca aaaaatattt
88861 gatctaagca cttattttct ttaagcaaat tagagctctt tttttatata aacatcacac
88921 acaaataaca catatgtgat tacacagaca gaagattcag tagttgtaag atttttcatt
88981 tgccaatctc ctaattggat tattggcctc agggtggagc atttcaagaa gcagagctag
89041 gaaagcatgc agtttccagg gcctaataaa caggcatagc tggaagacaa aaacagattt

# FIGURE 4-Y

89101 tgaaaggggt ctatctgctt ttaattcctg ggttctata agaaaaacag agtttttttt
89161 tttttttcca aaatgggatc cgtggtgact tctctgtttt tcccaaggtg tcttatgctc
89221 tcagaagtta tcttagggcc tctcatgcat gcgttaagag tgacaagaca aattggggaa
89281 aaataattca gttgactgag aaaaaattgt tttgcagaga aacaagatcc atgaagaaaa
89341 aaacacaaag gccttttaaa tatacctata gcttggatat ccacttttaa ttaagctgag
89401 cactctttaa gataaccctt ttaaatccct tagtacccaa ctttagccat gccaagccac
89461 gaatatttct ggcttttgaa ctttaccatt tctttcaaca ttttgcacag ggagagagag
89521 gccagaggcc cgactggcag aaaactttta ccctttctgtc cacatgttca caattctttc
89581 cccttctcac tccatcctta ttccactctc agccttcctc atctcagtaa atagccaagc
89641 actcctctct aattgctcag accaacacac tctggagtca tcctaattcc tccctttctc
89701 tcatgtcctc catctcagca aatcctatca aaacatcctt caaaagacat gacattactc
89761 ctccctttc accacgtcca ctgccaccac atagccctgg ttcaagtcat ggttgcctta
89821 aggctgggcc attcacatag acatctgtgt caattgtttg ctttcccttg tccctagagt
89881 cagatcagat gctgaactgg aaaaagcaaa taaagtttcc atttcattca gagtaaaatc
89941 ccccatcttt gctgagtctg aaccacgggg ccctgaagat ctggcctgga ccccacacct
90001 gccgctccca gctcactgct ggccacactg gcctcctcct cgctgtttgt tcctgaaatg
90061 cactgagtac atttttgctt cagggccttt ggacctacgg gtccctctgt ctggagcact
90121 ccttcctcca tgttcacatg actcccttct tacctcatta tggtatcagc ccaagcatca
90181 cctcctagat ctttcctgta gaagatagta gcccctgctt catcactctc tgtcccctta
90241 gcctgcttca gtttcttca gagcacaatc atcatcttta tttgttttct gtcgtctctc
90301 tctaacaacc tcccaccccc aaactagaat gtcagctcta tgacagtagg gactttttct
90361 tggtcacttc tgtagctcta gaacccagaa cagcctagca catggtagat gcccaataac
90421 taattctgag cttagttttt gaatgaatcc ccacttctac cctcctggtc aaggcaccat
90481 catcccaggc ctggacttgt gaaacagctt tctaactggt ctaccttgcc caattccagc
90541 ctacacgtaa tcccatctga acacaccaag actgccaatt gaactgtgac attcccctgc
90601 ttaaaactct ttcactgtga ccagttgtag aaacaacatt ttgtttatcc atttatccat
90661 ccgtggacac ttgggttgct ttcaactttt agctatcgtg aattaatgct gctgtgaata
90721 tgactgcaca gatagctgtt tgcatccctg ctttcaattc ttttgggtgt gtgcgcacaa
90781 gaggaattgc tggatcatat ggtaattcta tgtttaattt tttttcacag catctggacc
90841 attttacatt ccccctccac tagatatgtt attatataca aaggtggacg gctatacaag
90901 gacattccct acagcattga ctataacagt gaaatatgag aagccactta aattcaatag
90961 aataatgaaa tagaatcata tagatctatg tacacagata agggaccatc tatagtacat
91021 tctgttaagt aaagcaagca aagtgagctg ggcacagtgg ctcatgcctg taattctagc
91081 actttgggag gctgaggtgg gaggatcact tgagcccaag acgtcaaggc tgctatgacc
91141 tatgattatg ccactgcact ccagcctggg tgacagagag agaccctgtc aaaaagaaaa
91201 agaaaaaaga aaagaaatca aaatgcaagg gaattagggg ttattcttct tcctgaggac
91261 tggtgccatg gcatccagag aaagaacatt ggtggggata ctgatgaaaa agaataaagt
91321 ttgtagatta gttaaaagta ttcccttaat gttaatttcc tgattttta agtaattggg
91381 ctttggtttt gtcagatgcc aacatttggg gaaactggat gaaagcatat gggaaacctc
91441 tttactactt ttgcaatttt tctgttaagt ctaaaattat ctcaaaacac taagttaaga
91501 aaatcaaata aacttgatgt cttaaaacaa taccaattag gctgggcgca gtggctcatg
91561 cctgtaatcc tagcaatttg ggaggctgaa gtgggccgat aacctgaggt cgggagttcg
91621 agaccagcct ggccaacatg gtgaatcccc acctctactg aaactacaga aattagcctg
91681 gcatagtggc atgtgcctat aatcccagct acctgggagg atgaggcagg agaatcactg
91741 gaaccggaag gcagaggctg cagtgagctg agatcacacc actgcactcc agcctggggg
91801 acagagcaag actctgtctc aaaacaaaac aaaacaaaaa cacaatactt atttacttat
91861 acttcaagat tttgtgagtt taattatact tcaagatttt gtgagatttt gcccaagaat
91921 ttgggcaaga ctcatgattg gcccatcttg gccgggcgtg gtggctcacc cctgtagtcc
91981 cagtattttg ggaggcctag gttggaggat tgtttgagcc caagagttga agaccagcct
92041 gggcaacatg gcgaaacctc ctctctacaa aaatacaaaa attagctggg cgtggtggtg
92101 cgtgcctgta gtcccagata ctcagggagg ttgaggctgc agtgagccat gatggtgcca
92161 ctgcactcca gctggggtga cagagtgaga ccctgtctcc taaaaataaa aaataaggta
92221 aaataaagat tggcccatct accctcaaca gttgcctgga ctccctgggg ctcagtgcac
92281 ctagatgctg gactcggata accatctgat ctttgatgta gtggacagac actttgttct
92341 tgccatcatc tttggtacct gagatagaaa gccttaaaga gaggccacgc ccatgattaa
92401 gtttcaggtc aaaacaacga gcaaaaaggc aacggctata gttgtaggtg tagcaaatca
92461 gtgtgccttt tatctagagg ctactgaaaa ccagacattc attaaagata ttctggtcac
92521 ttggatgaag gctggccttg gcattcacct gaatctttag tagagtcttt agcaccattg
92581 gcaactcagc cagacctggc cttggccacg atagtggagc ctgggattgg agggcatgaa
92641 ttttatgaag cctgtaatat cagaaattcg ttggttgagg acatacctct ttcttttttgg
92701 accctgtggg cgtaatgcta ttgatacatg tgcagaggag gaattaatat gactgtgatt
92761 gacagaataa ttatgaagtg caaagacctc agatctgtga tcagcttcca tagaagtatt

# FIGURE 4-Z

```
92821  tgctcagaag cttctcagaa acattctcat gattgaagaa gccttgaaga gcccgtattt
92881  ctgtctatga aatctcctgt ttattgtact atgaggaccc atcagaaagt aactgaggta
92941  gtgctgattt cctgagcaat tattcagtcc actgtctcaa atccatcatg cagaatgttc
93001  acagagggta aagtaagttt gactattata actactcact tttataagtg atttaattta
93061  aagattaata tgataagtac tctgtttttc ctgggaattt tgttgtagaa agagtaactg
93121  ctgtgtggct aaactaaggt tataagccaa attgtttcct cagagtttaa gaaacacaat
93181  actaactctc atgggtttac tagttgtctg ttgctgcata acaaatcatt ccataatttt
93241  gtggtgtatt gctgcagaca atgttaaact aagtggatga aaaggatatt cacatagtct
93301  cagagtgtct ccctacaagg taggattact aacaaaggga aactaataat tatatagtaa
93361  ggaaatctcc ttaacccaat aatcaccagc aataagatgc agcaaccctc atcatgtacc
93421  tcttgatatg atgcactgac aaaagcacct ctcttctcta attttcttgc caaaatgcat
93481  aagctcaagc taattacagg aaaatataga caaacccaaa ttgagggaca ttctgcaaaa
93541  taactgaaca gtaattctcc aaaagtgtca aggtcataaa agacaaagac attgaggaac
93601  tgtcacagat tggagggaga ctaaggggac atgacaacta catgcaacct ggaatcatgg
93661  actgaatcct gggccagaga aaggacattg ggggggaaac tggtgtaaag ggcataaagc
93721  ttgtagatta gttaacagta ttgcctcaat attaatttcc tgattttttt aagaactggg
93781  ctttggttac ataagatgcc aatatttggg gaagttgcat aaaaacatac gggaaatctt
93841  ttgacgatgt tttgcagttt ttctgcaaat ctaaaattat ttcaaaacaa aaagtttaaa
93901  aatcaaatac acatagttgc ttgaaatagt aactatttta ttatattcca agatgttgtg
93961  agtcaggaat ttggccaaaa ctcaggtggg cgattcttct gcaaagaccc ccacaacaca
94021  ttcaaagtca caggcagagg ttgttggggg agggcattga aaagaagaga agagtcatag
94081  gtgggtgcaa tggagggagg gcagagggct gctgactatg gtgcaggact catccataat
94141  ggagccctgg ggaggcaagg gcttcataac tagacactgg tcttgtcacc tcagactcac
94201  ctgtagcagg accagatact gaggtcagac tgaaaacaca ggctctgcct caggagaggc
94261  tctctactag ctgagtaaat gatgacagta ttggaaatgt tcccaacatc ataatgggaa
94321  aacatcactt cacactacat aagcaataca caggggcagt gccggtcgtc ttcccaggtt
94381  agtagcagtt ctactgcctc caagagtgtt ggagaaatac aaaccaagca ttaggcactt
94441  ttaacttgaa aacatgaagt tctctttcct aactttcttt gtttccttat ttcttcttct
94501  tcttcttctc cttcttcttc ttcttcttct tcttcttctt cttcttcttc ttcttcttcc
94561  tcttcttctt cttcttcttc tttcttcttc ttctccttct ccttttcctt cttctttttt
94621  tgctgagaca gggtctcact ctgacagtac agtggtgcca tcacagctca ctgcagcctc
94681  gacctccagg gctcaagcaa tcctcccagc tcaccctccc aaatggctga aactacaagc
94741  tcgcaccacc atacgtggct aatttttcta tttttgtgtg cagatgaagt ttcctatgt
94801  tgcccaagtg gtctcaaact cctgggatca agtgatccat ccacctcaac ctcccaaaac
94861  gctgggatta caggtgtaag ccaccacacc cagcccacta actttttat atcggctaat
94921  gaaatagttt taagtttaga ccctacgagg cataaagaaa taattttagt tatgttatca
94981  gatgtacagt aatactcaag tgtgcaactg tggataactt gagttcatga ggtttttgtt
95041  tttttgtcaa aagaataaat ttatagtgaa actacccaaa aaagcaaagt acagaacagt
95101  atgctaccat ttgtgcacag aaatgggata tatatggtgt aactgcatcg aatttactgg
95161  atgtatgtcc agggaccaga actcttggtg gcttcatgtt catactttg caagcacatg
95221  tgtagtatcc ttaacttaaa ggtactgttg tatacattct agtgttatca aaatttacat
95281  acatattatc aagtcagaga ggtcattctg tgtcttagta ttttcacttc atatttggta
95341  tatttatgta tgtatacaca catacctata tgtatttaaa taagatttat agtcacatgg
95401  tccaaaaatc aaaacaatgt ggaaaggttt acagagaaaa gtctcaagcc taatcctgtt
95461  ctctactgcc aggtgaccat gttattaatt tcttttcata ccttgccaca gaattttcac
95521  ctgcaaacac agatattctt ttcttttta atgacagagt cacgttctgt tatccaggct
95581  ggagtgcagt ggcgtgatct tggctcactg caaactcctc ccgggttcaa gtgattctcc
95641  tgtctcagcc tcctgagtag ctgggattac aggcatgtgc caccacaccc agctaatttt
95701  tgtattttta gtacagatgg ggttttatca tattgaccag gctgatgtcg aactcctgac
95761  ctcaagtgat ccgcctgcct cggcctccca cagtgctggg attacaggcg tgagccacca
95821  cgcccagtca acacagacat tcttactcct tttttacaga gaatttatta ttattatttt
95881  ttacatagca tttttctgca cctttctttt tccacttaac aatgcacttg aagatttttc
95941  catatttgta catcaggagc tttctctttc tttgttacca cattaaattc cactgggtag
96001  atgtaccata atttaactgg gtccttattg aaagacaatt gagctgtctc ctagacaaag
96061  ccttgtgcac cttcccKaac agagggtcta accaagcagg caggatgggg ttataaagta
96121  ggtggggagg tgggagagac tccaccttcc caggtgggct gagaatggag gtaaggccct
96181  gcaacaggac agagggaaaa gtggggatga gaggtgggag gcgagatagc gcccactgtt
96241  ctcgctcagc cccctcctcc gtttgccgct gacctgttgg cctcccccaa cctctgagcc
96301  tgcctctgcc taggtaattt cccaagaccc agaaggggtg aagggtgagg tgtgattgcc
96361  cccacctcct tgcctcccgc agcatctgct ccgggaccat gaacaatagc tgacagctcc
96421  atggcccttg ctgtccccat ctcagcttcc ctgggcatct aaacctcagY tgccatgggg
96481  taggaggaca ggctgaggaa gcagaagcct gaggctgtct agagtctcac tcctgcatca
```

# FIGURE 4-AA

```
96541  gcaggccacc acctgtggtt cctccttgtg caaatttgaa aagaattgca taaaacactg
96601  gagaaatcca agagggggaag tccacaaggg cggtggctcc ctacaaggtc acagagcaag
96661  ctggtgtcag agcctggacc tacagcgctg ttggtggagg tcctgcctcc aggtagggga
96721  agggctccct ctcacctcta cacgcagcgc atttcttggc tcagctgccc tgtaggggat
96781  gcagggtggg gacagcagag atctgggcct gggagggaga gagtacacaa tcacatggct
96841  gttgcccctg tctcaggcct tgtctacctc tgactgtggc tctctggcag gaatagatgg
96901  acatggcctg gcagatgatg cagctgctgc ttctggcttt ggtgactgct gcggggagtg
96961  cccagcccag gagtgcgcgg gccaggacgg acctgctcaa tgtctgcatg aacgccaagc
97021  accacaagac acagcccagc cccgaggacg agctgtatgg ccaggtgagg gcagcctggt
97081  gtaggacagc atgcacacag gtcagagggt gatggcacga gcaatggcag gtccagtgtg
97141  gtcagaacca agggtgccgc tgctgacaag gaaggggagg ggcggccagg gccaccatgc
97201  cacaggtaag gccactgagg cagcttgggg aatatgagct ccaatttgaa ctccaggctc
97261  aggagtgtgc ttgtatttca ttcctctggt ctcctggcct gctccctaca aggtttcaca
97321  ttcccagagg gctggggatg tgcctaggga gagactgtgg cgtggacaca atctgtgggt
97381  taaagcgaag acaggacagc ctggaagccc catgacatct gagtcactcc caacattcca
97441  tttgcttatt tttaaatcgg ggttaaaaaa aaaaaacaaa tacataacat acattttcca
97501  ctttagccat ttttaactgt acggttcagt ggcattaggt atgctcatgt ggttgtgcaa
97561  ccatcaccac catccatctc ctgacctctt tcattctcca aaactgaaat ggaaactctg
97621  tgcccaccac ttcatttgct tttcagaacc ttctagagca catcctcctt gccaggaaat
97681  ggtgtggatg tagacctttg agagagacag atgactatca ttctcagggc catgagctat
97741  atgagagtga tgatatttgt tgagccctta ctatagcaag ggagttcttc tcattgtact
97801  cagtaactct tttggaggca acaacccttg accctgacag gcaggaccca tgtctgccaa
97861  accctaagac ccatgatgtg caaggggtct tgcaggaaga ccaagagttg gaacatccaa
97921  ggaaaagcaa gtgtgaagtc gggctggcag ggaagcatgt tctgtgtcag ccggcactgg
97981  gcgtgggcca gggtgtggga ggtgggtagg tctggctccc ctcccatgga tttccctatt
98041  gtttctcctg ggtgctcagg cctgtcacgc ctctgccatc acttgaccct aggtgcaagg
98101  gttcagccca gaaatKttat gcaattgatt catgatttct caggttttct gagtcctggc
98161  ctagagtgac ttcccaagaa aaaMctccac catttctgct tgtcttacct gccttgtatt
98221  tacctttcta ggattgcctt ttccacattt agtcaagtct aggttcagac ccacgtgcag
98281  gctatagctc cttcgttctc caccactctc aggatctatc tagagtctcc ccacctggac
98341  ctccagaccc tgggagagcc agaccagccc cttgacctcc accctccccc acaacctggg
98401  ccaggttcct ctcctccctg tcctcagtta taattttttt ttttttaat ttgaggcaga
98461  gtttcgctct tgttgcccag gctggaatgc aatggcatga tcttggctca ctgcaacctc
98521  tgcctcctgg gttcaagtga ttctcctgcc tctgcctcct gagtagctgg gattacaggc
98581  gcctaccact gtgcctggct aatttttgg tatttttagt agagacaggg tttctctgtg
98641  ttggtcaggc tggtctcgaa cttctaacct caggtgatcc gcccgcctcc ttaaatctta
98701  acctcactgt ttaccatggg tgtagcttac ttaaactctg taaaaatggg ggtaaggatt
98761  cgtactgggt tgttgagagg ataaagcgca aaagcctcag ggactttgca cctatggttt
98821  tctatgccta gagtgttctt tgtctccctt ctacacacag cccacccacc cactaaaacc
98881  ataccctccc tgagggtaga ggtgttattt gttttcttca ctgtggtgtt cctaggacct
98941  agcacagtgc ctgatgtata atcagcactc agttaatatt ggctggatgc aaaatgaata
99001  atataaataa gctgaataac atgaaatagg ccgaacgcgg tgactcacgc ctgtaatccc
99061  aacactttgg gaggccaagg agggtggatc acctgaggtc aggagttcga gaccagcctg
99121  gcaaacatgg tgaacccccg tctctactaa aaatacaaaa ttagctgggc atggtggcac
99181  gtgcctgtaa tcccagctac ttgggaggct gaggcaggag aattgcttga acccgggagg
99241  tggaggttgc agtgagccaa gatcacgcca ctgcactcca gtctgggcaa caggagcgaa
99301  actctgtctc aaaaaaaagt tagtttaata acatgagatc actttttaaa ccgttaagag
99361  ctgtactact aataattact ctctcagaca gtggctctcc ctcatctcct atatcccgtg
99421  gattaccctc tgttaaaagc caaaattaag caggcatggt ggctcacgcc tctaatctta
99481  gtattttggg aagctgacat gagccaagga gtttgagacc agcctaggca acatagtgag
99541  accccatctc tacaaaaata ctttatatta gccaggcatg gtggcatgtg cctgaattcc
99601  agctcctcgg gaggctgagg tgggaggatt atttgagccc aggatgttga ggctgcagtg
99661  agctatgatc acaccactgc gctccagcct ggtcaacaga gcaagaccct gtctcaaata
99721  aataaatgaa taaatagggc ggaaagcacc aatattgtaa ttgcctccgt ccccaggtgg
99781  gagctcctca agggccctcc ccaggaagtg ttcctctgga tgacctacct ggggcagagg
99841  agccagaata tggaggagat ggctgtggtg gggagagact tagtcctgtg tcttccccac
99901  ccagtgcagt ccctggaaga agaatgcctg ctgcacggcc agcaccagcc aggagctgca
99961  caaggacacc tcccgcctgt acaactttaa ctgggatcac tgtggtaaga tggaacccac
100021 ctgcaagcgc cactttatcc aggacagctg tctctgagtg ctcacccaac ctggggccct
100081 ggatccggca ggtatgagtg ctgttcccac aaacattaac ctcagcagag ggcggagcct
100141 gccagttgct ggcagggagg gcttggtcca ggaattcggg tctgagggtg gtggacgccc
100201 tgccccctcc cacagctctg gtccccttca agggtaaagc tgctgagata cgtggctgac
```

# FIGURE 4-BB

```
100261 aggagtattc tgtctcctcc ccactcaggt caaccagagc tggcgcaaag agcgcattct
100321 gaacgtgccc ctgtgcaaag aggactgtga gcgctggtgg gaggactgtc gcacctccta
100381 cacctgcaaa agcaactggc acaaaggctg gaattggacc tcaggtgagg acctgaggag
100441 ataagatgag gagtgggagt ggggctttgg ggttgggagg ggtgcggtct ggcccagaag
100501 ctaagggtct tacgttctcc tccctcaggg attaatgagt gtccggccgg ggccctctgc
100561 agcacctttg agtcctactt ccccactcca gccgcccttt gtgaaggcct ctggagccac
100621 tccttcaagg tcagcaacta tagtcgaggg agcggccgct gcatccagat gtggtttgac
100681 tcagcccagg gcaaccccaa tgaggaggtg gccaagttct atgctgcggc catgaatgct
100741 ggggccccgt ctcgtgggat tattgattcc tgatccaaga agggtcctct ggggttcttc
100801 caacaaccta ttctaataga caaatccaca tgtgtcttgt gtcttgtaat ttcgggacga
100861 gtgggttgga gggacacatt gcttcatctt ttccattgac aggccccaaa ttgggctgga
100921 actagcctaa tgttcactgg gaaggagggt gtgggggttg agctagaatc caggtatctg
100981 atccgttagt ctgggtctct taccccctgca ctggcttccc cctcatgcca agctcatccc
101041 accggcacta cacatggaga aagacacaga cggagtgaag aagggcagag atagccgatg
101101 agttattggg cttcaagttg ggaagagagt ttctttagtg atgtgggctg ggtggagata
101161 ttggtgggga ggagggtctt gatgaacact tgccttgttt ccatttattt atttatttat
101221 ttatttattt atttatttat ttgcgacagg aatcttgata tattgcccag gctggtcttg
101281 aactcctgtg cccagacaat actccctcct tggcctccca gagtgctggg attataggca
101341 tgagtcactg tacctgacct gctttttaaa aaaaaatgac aactaatgtt gcaattaaca
101401 atcttttact tagtggaatg ttatacttaa attccagaaa ggattattgg gtcaagagta
101461 catataattt tgagagttac aactttatgg gggttgtatc aatgtgcctc ccccaccagc
101521 aaagtatgag agtgcctctt tctccactgg gtgtgatggc tcatgtctgt gatcccagca
101581 gtttgggagg ctgaggcagg agaatctctt gagcccagga gttcaatatc agcctgggca
101641 acatggtgaa accccaactc tacaaaaaac taaaaaatca gctgggcatg gtggtgtgtg
101701 cctgtagtcc caactactag ggaggctgag gtgagagaat cgcttgactc ccagcaggct
101761 gcggttacag taagctatga tcatgccact gcactccagc ctgggcaaca gggcaagaca
101821 ttgtctcaaa aaaaaaaaaa aaaaaagaga gagagagaga gtgcctgttt ctccacaacc
101881 ttgtcaaccc actatgccat taaactttgg ttttctgctc atcttacagg tggggaaagg
101941 gcagctcagt gcagttttca tctgccattg ttctgagatc tttccatatt gacctattga
102001 tatgttaaat taccaggtag taatccattc taggtgtaca ttacattgaa ttcagtctcc
102061 tactgatggg cattggaagg aatggggttc ttttatgatc ctgacattgc tgaaagccac
102121 aggccagtta tttagtagaa tactcctcca tttgggtttg tctgtttcct catgattcat
102181 tgaggttatg cgttttgggc acaaataccc tacatggtta tcccgtgcat cataatggga
102241 ggcatgtggt gtggggtttg ctcattcctg gtgacgttaa ctttgatctt tttcttgaga
102301 ttgtgtctgc caggtttctc cactacactt tccccctttgt aactagcaaa tatgttgtag
102361 agagagactg gagaactaga gatactctat tcatcagcac acttccacct atatgttcta
102421 gtagctgatg gaccttaaac atgtcttact atgttacagc tctttcacca acccttcaat
102481 acttttcagt ggtatcaact gtaaaaacca aacttcttca caatagcaca gaagaccatg
102541 cccctccttt gagccctcaa gccaaatctc tcccacacca ccccataaat gagctccagt
102601 cacatttctc atgttggggg tctttgctcc agcaactctt tctctgcatg cctgaaatgt
102661 tcttcattaa tcctctgcag cctagcactt tgtggccact tagatcacaa cctacaatcc
102721 cccccctaaaa tgacttctga ccactcaaac tgacaaaccc acccagctga tttctcatca
102781 cactactcta tgtccttgtg cagattttta tctactgtgt ttgttgtcag ccttttccct
102841 aacaaaacat actctcccga aagcagggtt gtgtctgtgt cattttatgc tacatcccca
102901 gggtctgggt cagtgcctgg caccgagaat gtgctcagta agttaaggaa tcgctgcatc
102961 cattctgtca gtcaacaatt ccttctgagc atccagtcca ctggcccatg ctggtctcag
103021 ccaacagaaa gggaattgag ttccttgcaa ggtctggtgg ggacagacag ttctgagaac
103081 cagtgactct tagaacaagg agatgaggct ttcacagagg gaagcacaga gtgtggtatt
103141 attctgctga agcccaaaga ggtcagggaa ggattccaaa ggagtaatgt ttgccaagca
103201 caaaacagca cagggaatgg cattccatgg acaggtctca gcaagtgcaa aggcttagag
103261 gtaatagaag tgcaagcaga attccagggt ggctaagaga ggttgtgtgg ggaatgggca
103321 gcagttaagg ccaaagaaga aacactggat ctgcattcct taggacggtg ccctgtgggc
103381 ccctcacaga gcttctctcc tgcaactgct aggtataaga aggcaggctt agaatcggac
103441 tgagatcagt agtttctgca gctgggaaga tgagtgaggt gagattgcat gtgtgatagg
103501 ctggcactca gggaagacct cagggttctc tcctccccac tctgaggccc ctctcctgct
103561 tcctgaagcc cagagtctcc acctctgtga gccaaacacc ttcctgttat ctgcctggtc
103621 ctagggtagc cttcgggttg gggttagaag gaatgccaat ggttaacctt gtggtattaa
103681 gcttccagct caggcagata ctctctggac tggttcaagc tgactgcctg actgtccctc
103741 cctgctggcc ctagcaccca gactccagat aggcacacca cctcaccctt cagagcaggg
103801 ctctgagact ccacaagtgg tgaggactta ccagaggaac tgaacttgac ctccaaccca
103861 accttccatt tgctggtatt attctgaagc cctcatggcc caggggcctg cctcaggtag
103921 caggtctcac tcctgcagtg gaggggccat aaagttcagt tcatgatcag cccccaagcc
```

# FIGURE 4-CC

```
103981  aggggcagcg gatgacaggg caggagttcc atctagagga gctcctgtgt tcaggcgttc
104041  ctagacacct gcctgccctt ctcccatcag cactggggta taaagaagga aagaggcctg
104101  aggtttccag aggcctctac ctgctgaggt cttaagagga ctcaaccctc tgggaaatac
104161  aaataaaatc ctcaaccctc caaacaactg aatggacacc tcttggccaa agagaccccg
104221  gaaaaacttt aaaatccaag tttcctggcc gtgatgatag gtcactcaca cctcagcaca
104281  cctgcttcct cacgaaccgt taccaggctt ctttcccaag agctaaacag aaaccagccc
104341  tgaaaaccaa gaacaggaga ctccttcact gatttcaatt tcaaccaatt ccgagactcc
104401  cttccctttc ctgattttga catgacagct gatcagctta caaaacattc ctggctgatc
104461  aatgactctc aaccatggac cagttctggc tggtttacag aggcagcaca caaagtgctt
104521  tgggtcctgt gtttcacatt ttgacataca gagcctaatt caactgcatt ttaatgtctc
104581  caccccgaag tgaatatggg acatatttaa catgttttat tggtacacat gtgtgcaact
104641  ctcatgaata ttcataaatc ctcttaaaac ttattaaata tatatgttta gccaactgat
104701  ttagtgtaaa acccctgtcc cttcaattcc tagcttgtag gttgcaaccc atcaaaagaa
104761  ataaagctct cttttccaaa tgtaaagatc ttatgatttt aagccaatat aattgaagac
104821  aagagtagga tccatggagc agcccaggtt cccccagcct aagtgaatgc acaggtgcca
104881  ggtggagcca ttgacagctc atttgtctcc ctgacagctt tgggaggaag gtgggtaagc
104941  tctccccgat ctaagatctc tcacttttga attgagatct cgaggacttc atttttttac \
105001  cctgatcccc tccatccgga ccctgcaagt gccatctttg ttggtcccag gtttctagat
105061  ggggaggttg gggggcagtta agcctgactc atccggcgca tcagcccatc aggtttggtg
105121  aggatgcttg ccctctagtg gcacacgaag gaatgtctta tgcttttctg ggaatctag
105181  attctaagga gacagtccca gaccagctgc catcaggaac atgcatgttt cagaattatg
105241  agaaaaagtc ttgtgaatat tttggatcct ggaaaaagc taacctggaa caatataaag
105301  atttataggc caaaatggag aacttttggc agaatgtctg acatgcctaa attagtttat
105361  ttgcatgcac aatagggttc aggacctctc agaagcaatg ggaggtcttt ttttttttt
105421  tttttttgag acagagtctc actctgtcat ggggagtgga gtggagtggc acgaacatgg
105481  ctcactgcag cctcgacctc ccaggctcaa tcatcctccc acctctgcct ctcaagtagc
105541  tgggattata ggcatgcacc accacacccc actaatttct ttattttcaa tttttaattt
105601  tttatagacc atgctggtct caaattcctg gactcaagga atcctcccac gggagaccat
105661  ttttcagtgt tattatgaga gctctaaaca cagttgggag tctcagataa cctccttaaa
105721  tgagactaat tcaaaactga aggagtctaa ttcaaaactt gaccagcata ttcaaaccta
105781  tctgccacta ttggacaggt agctgaagac actgcaaaaa gccttatagc ccaacaaatt
105841  gaattccctg gctcaagtag taatagataa tttaattgct tcagattttc ttttacccaa
105901  acaaggaaga gtctgtgcag tggcccataa cacctgttgc acttacatca acacttcagg
105961  tgaagtagaa actcatatag ggttgggcac agtggctcac acctataatc ccagcacttt
106021  gggaggctgg ggagggagga ttgcttgagc tcaggagttt gagaccagcc tgagcaacat
106081  ggtgaaaccc tatctctaca aaaactgcaa aaattaacca ggcatggtgg cacatgcctg
106141  taatcccagc tatttgggag gctgaggcaa gaggatcact tgggcctggg aggcataggt
106201  tgcagttagc ctagatcacg caattgcatt ccaaactgat aaagcaagac tctgtctcaa
106261  aaaaaaaaaa agaagaaaaa ctcatacaga aagaatttcc aaacaagcta aatgattaca
106321  agaaataaaa ctactgatcc tatcaatgat ctgttcagtt ggctttctac caaacagaaa
106381  ttcctttcaa gatgctattg aaggctgggc gtggcggctc atgcctgtaa tcccaacact
106441  ctgggaggct gaggcgggtg gatcatctga ggtcaggagt ttgtgaccag cttggccaac
106501  atggtgaaac cccgtctcta ctgaaaaata caaaaaatta gctgggtgtg gtggcgacac
106561  ctgtaatccc agctacttgg gaggctgagg caggagaatc gcttgaacct gctaggcgga
106621  gattgcagtg aaccgagatc acgccactgc actccagcct gggtgagagt gagactccat
106681  ctcaaaaaaa aaaaaaaaaa aaaaaggtgc tattgaagtt tttgttataa ttatagtctc
106741  catcaaactt ttcttcataa catttaaatt acttataata tgggacaggc atgggggctc
106801  aagcctgtaa tcccagcact ttgggaggtg ggcagatcat ttgaggtcag gagttcaaga
106861  ccagcttggc caacatggcg aaacactgtc tttactaaaa atacaaaaat tagccgggca
106921  tggtggcatg tgcctgtaat cctagctact cgggaggctg aggcaggaga attgcttgaa
106981  cctgggaggc ggaggttgca gtgagccgag atcacaccac tgcactccag cctcggcaac
107041  agagggatac tctatctaaa aagtaaatat aaataaataa ataacttata atatgtataa
107101  ccaactgctg taagtctgct gctaaaacca gaattatgct ggctcaatgc atagaactta
107161  tagacaagtt aaattggtag ccctaccttt tggcctttat attgcttgat agaccttagg
107221  ggttgatgag tacctgccca cctctatttc tgtctggcca agatgttcaa ttggctgtaa
107281  gtctcttggc cacaagggtc ccaccaaggg actggatgga tctggagcag gtagcctcag
107341  catcctggca acgacatggt acaaacaatt tggccattga tgctgactgt ggcagatctt
107401  ggctaaaagg aagaaatgtg gaataaaaag aaaatccaaa gacccctcaa ctgactgaac
107461  aaacccctct tggccaagga gaccccagaa aaactttaaa atctaggttt cctggctatg
107521  ataagacagg aggctggtca cacctcagta taccctcttc cttactgtta ccaggctttt
107581  ttcctaagag ttaagcagaa tcctgtcctg gaaaacagag aatggaagac tcctccgctg
107641  acttcagctt caactgcctg atgccatggc cagacttcac tctcttttg tgatttgaca
```

# FIGURE 4-DD

107701 cgacagctga ccagctcaca aagctatcct tcctgatcag ttcctgttaa ccatgggctg
107761 gttctggctg gtttacagag gctgcacaca aagtgccttt gtgtcctatg tttcaccttt
107821 tgatgtgtat ggcctaagtc tgcattttaa tgttaagtct cagctgggta tggtggctca
107881 atgcctgcaa tcctagcact ttgggaggcc aaggcaagag gattgcttga gcctaggagt
107941 tcaagaccag cctaggcaac atggtgagac cttgtctcta tttttttaat taaaaacaaa
108001 atacaaatat aaaaataatt actgttaact ctccacccca aagtgaacac aggatgtatg
108061 taacatgtat gtttgcttag tatacatgca tgtgactccc tttcatgaat attcatagct
108121 cctcctataa cttattaata agtatactag ctaacctatt tagcataaaa ctcctgtccc
108181 acctctcctc cctcaaagtg cctgctttcc atctcagcca gaggctccac ttcccagcct
108241 gcaggttaca acacaatatt agtaaaagtg ttctttccaa gtgtatacat ctggtgattt
108301 taagttgata cttcccaggt tgtccaagat tcaggtacag ctcactattg caggatacaa
108361 gctgggatct cctgggagtt ggtctctttg caaatcgtat tatctctgta tcacctttta
108421 tgaaatccta aaagaattta aatctgaaac atgtgactcc aaagatttttg aataaggaat
108481 tgtgaaccca tggtaaactc ctggcttaag ggtgtccaat tttttggctt ccctgggtca
108541 cattggaaga ataagaaagt ttaagaaagt ctgtgaattt gttttggggc acattcaaag
108601 ctgtcctggc cacatgaggc ctgagggcta taggttggac aagcttctag ctggctattt
108661 ctgcattttg tccaaatcca tcttttaatt attatcatta ttattattat tatttgagac
108721 ggagtctcgc tctgtcaccc aggctggagt gcagtggcgc gatctcagct cactgcaagc
108781 tctccctccc gggttcacgc cattctcctg cctcagcctc ccgagtagct gggactacag
108841 gcgcctgcca ccacgcccgg ctaattattt tgtattttta gtagagaagg ggtttcaccg
108901 tgttagccag gatggtcttg gtctcctgac ctcgtgatct gcccacctcg gcctcccaaa
108961 gtgctgggat tacaggtgtg agccaccgcg tccagctgaa acataatttt tctcttttttg
109021 gtttcacatt tttactaaag acaaatcatg gtaagactga tttgctttat tatacttggc
109081 ctgattattt gtgtaacgtg cagcaagaat acttactttt cacataggct ttttaagttg
109141 gctttgatgg aactttgttt tgtagaagaa tctcagatta gactttttct tttaaggccg
109201 ggctcagggg ctcactcctg taatcccagc actttgggag gctgaggagg gaagatcaca
109261 tcaggagttc aagaccagcc tggccaacat ggtaaaaccc cgtctctact aaaaaacacc
109321 aaaaactagc caggcatggt ggcaggtgcc tgtaatccca gctacttggg aggctgaggc
109381 aggagaatca cttgaacctt agaggcaaag gttgcagtga gccaagatca caccattgca
109441 ctccagcctg ggcgacagag cgagactctg tctcaaaaaa acaaaacaaa acaaaacaaa
109501 actttttttt taaagccgag tttggccatg cggttgtacc atcaaatacc tatgagttgg
109561 gtgaattcct cttgagaacc caagatgatt tggggctcct gggtctgtca gagagtgaca
109621 ttctttactt gccacaggtc agaaaccctg ctcaggatct gtgtagaaaa ggtatgaagt
109681 tagttttccc aaggggctct tatcagctct ataagtcaag tttgattcct taaaggaaag
109741 cacaccattc cagtcaaagc cttggtaaaa taactggttt ctccaattgt gtcctgttac
109801 aaaagaaaac agattctggc tgggcacagt ggtcatgcct gtaatctcag tactttggga
109861 ggctgaggtg ggcagatcac ttgaggtcag gagattgaga ccaacctggc caacacggtg
109921 aaaccccgtc tctactaaaa atacaaaaat tagctgcttg tgttgtgtaa ttgggtaata
109981 agagatttta aagaattttt tttgtagagc accatggttt aaagtcagct taattaaaat
110041 tagatattca agctctaaca gcctgggact ccttggggaa aacaggaggc gccagagaca
110101 ccattttgga aaaaaaccct gttttcctct tggaacccca ggaattgaaa gctgataaat
110161 tcctctcaaa atttaaggct ttgttctgtt ttggattgca gtatctgaag tttttgactt
110221 ttggtctatc agaaattata tcgcattatg agagagtttt ggtgtgtaat aactaggtag
110281 gaaatatact ttaaggaatg gctaatggaa attatagatg atcacgtagc tctttgcatg
110341 tttggattag agaagcatgc tcttggccac ctggaaggta tggaaatacc ttttttttt
110401 ttttttttt ttttgagaca gagtctcact ctgtcacccc ggctggagtg cagtggcaaa
110461 atctcagctc actgcaatct ctgcctcctg ggttcaaagg attctcctgc ctcagcctcc
110521 caagtagctg ggattacagg cacctggcac catgcccagc taatttttgt atttttagta
110581 gagacggggt ttcaccatgt tggccaggct ggttttgaac tcctgacctc aggtgatctg
110641 cctgcctcgg cctcccaaag tgctgggatt ataggtgtga gccaccgtga ctggctggaa
110701 atatcttctt atctcccact gagagataag acttccacag aagatgggct gattccccct
110761 tttttgggga gggatccagg gtctggtata aaatgggatc ttcattttgg gggatctgtt
110821 ttgccttcca gctgtgcctg cttattaggg ccaaaggtac ttgggagtcc cagctatttg
110881 ggagactcag gcaggagaat cacttgaatc caggagatag aggttacagt gagctgagat
110941 cacaccactg cattccagcc tgggtgacag agttagactc tgcctcaaaa aaaaaaaaaa
111001 aaaaaaaaaa gaaagaaaga aaaaagagac agattattta gattatttat ttacttactt
111061 tttttttgag atgcagtttt gctcttgtca cccaggctgg agtgcaatgg cacgatctcg
111121 gctcactgca acctctgcct cccgggttca agtgattctc ctgcctcagc ctcctgagta
111181 gctgagatta caggcacaca ccaccacacc cagctaattt ttatttattt atttttagta
111241 gagacagggt tttgccatgt tggccaggct ggtcttgaac tcctgtcctc aggtgatcca
111301 cccacctcag cctcccaaag tgttgggatt acaggcgtga gccactgcgc ccggccaaga
111361 aacagattct tattgcactt atgcaaataa ctatattgct ataagttaag aatactcaca

# FIGURE 4-EE

```
111421 cctagtttcc taattctgga aaaatcaggt agaaaaaaac aaatatgctc caaatatgtt
111481 gccagaagta tacttttactg aattgttttt tttttttttt ttgagacgga gtctcgctct
111541 gtcgcccagg ccggactgcg gactgcagtg gcgcaatctc ggctcactgc aagctccgct
111601 tcccgggttc acgccattct cctgcctcag cctcccgagt agctgggact acaggcgccc
111661 gccaccgctc ccggctaatt ttttgtattt ttagtagaga cggggtttca ccttgttagc
111721 caggatggtc tcgatctcct gacctcatga tccacccgcc tcggcctccc aaagtgctgg
111781 gattacaggc gtgagccacc gcgcccggcc tactgaattg ttaaaaggtg taaatagctc
111841 aaaatttttc ctgactctga aaacaaaaca aagtttcagc agcattttaa gcaaagtcaa
111901 aaacatttct tcagtcttct atttgttcat tccatgcagt taactcctgt cctgtttaat
111961 cgtcatgaac atttcagctg tccatgagtc ctgaaagttt ttcctctatt ctgacgtcac
112021 aatctccaaa cttatcagaa acctgcattc aagagaacct gtcaaaatcc tatagttgat
112081 tataaaccac cttttgaaga ggatcaaaat aagaaaacaa ttgtctgtgg ttgacagaag
112141 tcttaggaca gccactattt aagccacaat tgaaaaggaa atgtgggggt gggcacggtg
112201 gctcacgcct gtaatcccag cactttggga ggccgaggca ggcagatcac ctgaggtcag
112261 ggatttgata ccaacctggc caacatggtg aaaatccgtc tctactaaaa atacaaaaat
112321 tagctgggga tggtggtggg tgcctgcaat cccagctact gagagtctga ggcaggagaa
112381 tcgcttgaac ctgggaggtg gagttttcag tgagctgaaa ttgtgccact gtactacagc
112441 ctgggtgaca gagtgagact ctgtctcaaa aaataaaaaa gaaaagaaaa ggaaattttg
112501 gttagttctg tgacaacaat tttacataac aattatgact attaataaca tacactaagt
112561 tatcaaggaa ttatgggtgt tttccataat tttggaacat gtccaataac atatttatgc
112621 aaatataggc caaagaaagc caaatactat ttcacatttg acgatgcttc ctttatggat
112681 tttatactga ataagccaaa tttcaccttt acgttagtgt actattaatg ttaaacccaa
112741 ttctttaata aaaccttta gacaaattta tttaatctta atcagtttga ccataaagta
112801 agattcttat aaacttttgt ttcctcacct ccccagaaaa gtggatccta aacctttttat
112861 aaccctttac aatttttgtg aacgagcaga ttaatgctct aagaaaaacc tgttgtgctt
112921 ttattccaat gtttgattta tagaaaaaat gaataccect ttaactttag ccaacatgtt
112981 cacacagaat ttcttttatg acattaattt ttcacaaacc tcccacaatt tgttcaagcc
113041 ttcagcttta tctcatctaa aacaatcctt taacccttta atctaggcag aaaaatccac
113101 attcccatga cttcttataa tcttttacca aaaacacatt tcactttcct tacacacctt
113161 gcatgtaaaa ctgatgttat ttcccaaaga ttactaaagg catataaact aaaaggcatc
113221 acagttttta tttttctaat aaaatatttg atttaagctc ttattatttt ttaacctatt
113281 aatcaaagct cttttatatc tcacacacac aacatatata aatacacaga ccagaagatc
113341 cagtagttgt aagaattttc atttgccagt ttctttttct ttcttttttt ttgaatcgga
113401 gtcttgctct gtcgcccagg ctggagtgag gtggcttgat ctcggctcac tgcaagctcc
113461 gcctcccagg ttcacgccat tctcctggct cagcctcctg agcagctggg actacaggct
113521 tctgccacca cgcctggcta agtttttgta ctttagtgg agacggggtt tcactgtgtt
113581 agccaggatg gtctcgatct cctgacttcg ttatccacct gcctcggcct cccaaaatgc
113641 tgggattaca ggtgtgagcc accgcgcccg gccatcattt gccagtttct taattggatt
113701 actgacttta gggtggagcc cttgggggaa caaggtcagg aaagcatgca gtttctacag
113761 cctaataagc aggcaagctg gaaggcaaga cagatccccc aaaataaggg tcccatttta
113821 taccagatcc tggatccccc ccaccaaaaa aggggaaatc agcccatctt ccgtggaagt
113881 cttatctctc agtgggagat aagcgggtat ttccatacct tcgaagtggc caagagcatg
113941 cttctctaat ctaaatatgc aaagagttaa gtattcacca ataacttcca ctggccattc
114001 cttaaagtat atttcctacc tagttactac acaccaaagc tctgtcataa tacgatgtaa
114061 tttctgatac caccaaaagt caaaaacatc agatactgca atgcaaaaca gaacagagcc
114121 ttacattttg agaggaattt gtcaactttc aactcctggg gtttcaagag gaaaacagag
114181 tggtattttt tttttttttc caaaatggtg tctctggcac ctcctgtttt tcccagggag
114241 tcataggctg ttagagtttg aatatcctgc tttaatgaag ctgactttta accatagcac
114301 tcttaaaaat aaaaatcctt taatgtctct tattacccaa cttcagccat gccaaactac
114361 caatatttct ggcttctgaa ctttaccaaa ggcaaactcc caggtactcg gagaaaaaaa
114421 attcaagaca gtttgtggag ggaaagagaa tctacaaatg ttcagacaga tctcaaacta
114481 gaaaggactc attccctaag ctggggattg aaccctgaac ctgggctgcc actgtgaaaa
114541 gacagtctta gctgctgagc tacagcattg ggcagtctcc attgcccttc ccagaaggag
114601 gctagagtca ccaatttttga gcttgcaaag gcttttatct gatcaagata atttttagga
114661 gtaactatgg catgaacccc aaaactcctg tctgctggat ggtagaaacg aagagaaagt
114721 atcgccacgt ggttacaagg tgaagctccc aaggacataa aacaagacga aagagaaatt
114781 tcatccagct tttcagggac cttcagcaaa gtttataact gatcagtttg cttggccatt
114841 ttgaacagcg ggcttacagg cgtcatagtg agagacagga ctagctggat atcctaggcc
114901 tactaagaat ccctaagcct agctgggaag gtgactgcat ccacctttaa acacggggct
114961 tgcaacttag ctcacacccg accaatcagg tagtaaagag agctcactaa aacgctaatt
115021 aggcaaaaac aggaggtaaa gaaatagcca atcatctatc gcctgagagc acactgggag
115081 ggacaatgat aggatataaa cccaggcatt caaaccagca agggctaccc tctttgagtc
```

# FIGURE 4-FF

115141 ccctcccttt gtatgggagc tctgttttca ctctattaaa tcttgcaact gcacactctt
115201 ctggtcagtg tttgttacga ctcgagctga gctttcactt gccgtccacc actgctgttt
115261 gccgccatcg cagacccgcc gctgacttcc accectccag atctggcagg gtgtctgctg
115321 tgctcctgat ccagtgaggc acccattgcc gctctggatt gggctaaagt cttgtcattg
115381 ttcctgcacg gctaagtgcc cgggttcatt ctaatcgagc tgaacattag tcgctgggtt
115441 ccacagttct cttctgtgac ccacggcttc taatagagct ataacagtca ctgcatggcc
115501 caagattcca ttccttggaa tctgtgaggc caagaacccc aggtcagaga acaagaggct
115561 tgccgccatc ttggaagcgg cctgccacta tcttgggaac tctgggagca aggacccccct
115621 ggtaacagta ggcttgcatt ctatcctaag gtacccttct ttatggcaga acaatataga
115681 cacacaaagc acaccagatt cgttacagct taaggctagc ctcacaagtc tttttttctca
115741 ttaaccaaaa cttgcctgtg gtaaggtggg gaaagtgaag agctcaggaa gaccagagaa
115801 agactcacct atcccagtga cactgaatca aaagttcagg cagccgctca ttggttaaga
115861 agggatcttt tccaggagtt gcatcagctc ttaagtgtcc ccgtttgggg agcaaaaagc
115921 tctcatgtcc caggatcctg tacatacaca attctgtcac ccacagccat cagcaaagat
115981 ttcaaggtag attaatccaa agagaatagc aattaatatc ccatggtgct aaatccgttc
116041 ttagccaaga ggaactttac tctgagaggg gcttctaact ccctaaatct tagaagggac
116101 tctaacactc ctgagttggg cctcaaacca aagttcagtc aagtgtcctt gccttttatt
116161 aagaggggcc tttaacccac tctgtcttag gagagacttt aactccccta agttgagcct
116221 ctaacccaat tccatccttt acctgggtac cccgccactt accctagata gccaattggt
116281 gctgcagtct atttcctttg ggtagggtgt ctcctcaggg ttcgacagga agatgttacc
116341 agaaagggat ccagatccag accccaagag aaggttcttg gatcttgtat aagaaagaat
116401 ttggggtgag tccatagagt aaagtgaaag caagcttatt aaggaggtaa aggaatacag
116461 aatggctact ccataggcag ggcagtggct tgagctgctc cactaaggat acttattgtt
116521 acttcttgat tatatgctaa agaaggggtg gattattaat gagttttcca aggaaggggt
116581 ggccaattcc cgtaactgag ggttcctctc ctttttagac catttagggt aacttcctga
116641 tgttgccatg gcatctgtaa tcacggcact ggtgggagtg tcttttagca tgctaataca
116701 ttataattag catataatga gtgatgagga tgaccagagg tcactttggt cgccatcttg
116761 gttttggtgg gtttgggctg gcttctttac cacaggctgt tttatcagca aggtctttgt
116821 gacctgttat cttgtgctca cctcctagct catcctgtca cttggaatgc cacacctcct
116881 gggaatgcag cccagtaggc ttcagcttca ttttacccag cccctattca agatggggtt
116941 gctttggttg aaacgcctct gacagaccca ccagaggagc ccttcagttt gtattaatat
117001 gatatgtgac tacttatttc acagacactg tgtgtcaaat gtcggtacaa tgccaacaac
117061 tcactttctt ggttgttgag tttccgcatt acacaaataa ggaagcaggc ccagaggaga
117121 gcctgggaaa tgaagttgga gagacccatc ctggggttgc ttgatttagg gatttagact
117181 gggaatgact cctccataga tctgaaagaa gaaactgcac actgttcata gtggcttctt
117241 ttctgccagc cctaaacagc tcaagaaagg agagtctctc agttatgagg ctgggtgtga
117301 agcatttttt ttttctttga gagaggtctt gttatgttgc ccaggctggt ctccaaatcc
117361 tgggctcaag cgatccttcc accttggcct cccaaagtgc tgggattaca aatgtgagcc
117421 accacatccg gcctgaagca tcttggttca tgcatctagc aaaccttcag tctgtgtctc
117481 tcaatcccac tggacatgag gtctgcctat aacaattatt tggctacaac ccctttaata
117541 tcctgaactt agctaaacag ttattttaaa aatccatatt caaatataac taaagaagaa
117601 acagaggaaa agtaatttgt aatatggatt tctatttata ttcgttttga ctccattagc
117661 agatagcagt cactgccctg tttgcacttg ttttgaatca gtgagcctgt agctgccaag
117721 tgcagactaa caagaggtaa ataacctgta taccttgagc agctctgact ttaacttatt
117781 ctttatattt ttatattttt attttttttt atttttatct ctttttagac atgaggtctc
117841 actatgttgt cccagttgac ctcaaactcc tgcactcaag tgatcctccc accttggcct
117901 ccaaagtact gggattatag gtgtaaacca ctgtgcctgg gcttttatat attttttaag
117961 tgcacatttt aatgtttagc ttgtcagcct taagtaacga gatccagaaa gcttgaggat
118021 agctacacag aagcatagat tcaagttgtc ctgaatatac acttcgattc acagcagtta
118081 caagtgagtt tttaaggaaa cagaatagtt cctaagttgt ttatcaagaa tttaaaataa
118141 aataacataa gcaattcatt ggctatacat tgttttttgt atcacaaatt ccaggaacag
118201 ataacgggtg aggcagctag tcagggacaa aacgccttgg aacaattgtc tctgggcatg
118261 ggtggggagg gtgtgactga agttccatac tcatgccttt ctgggcctga taaattgggc
118321 atgtctcaca tcatgagact gctctgagct atttttcttt tctcaagctt tataccaaat
118381 tctaggccgt gggctttcat tcattcaata agtatgtatt gagtgtttat gtaatcggaa
118441 ctcttctggg gttacaattt aacttacttt ctcacctccg aatggttatc acctcaacga
118501 ggactaaatc caatgatttt tttttatctt gccaaaattc ctatctaagg ggtctgggga
118561 gtcatgccct acaaatcata aattctcatc agatgggttt tatttaaccc tatatatcat
118621 gacttacttt ccaacctgac tctggcataa catgacgaga caaggaagaa aagcaaaata
118681 ttttaatatt tttacctcaa aacatgtttc tttgccatat cttgaaatgg ccctgctact
118741 gtcctttgtg ggggaaaatt ttcatctgta aagaatctct attaacatag ctggatcttt
118801 ttctaccagg cccttccaat cataaagaga ttaactaagt gtagcacctt ttatagatct

# FIGURE 4-GG

```
118861  gaataggaaa cacttgtcat ctgttgtttc taagggcagc cactataaga cttcaaaaga
118921  accttggtct ccacagtctt ttatcttaac atttcttttc tatcaacccc aggtctttag
118981  acaaactcaa ccaattgtca accataaaat gtttaaattt acgtatagcc tgaaagccct
119041  gccaccccac tttgaattgt cccaccttcc tggaccaaac caatgatttt tttttttttt
119101  tttagatgga gtctccctct gccgcccgtg ctggagtgca gtggtgtgat ctcggctcgc
119161  tgcaacctcc gcctcctggg ttgaagcaat tctcctacct cagcctcctg agtagctagg
119221  attacaggtg cctgtcacca cgtccagcta atttttgta tttttagtag agacagggtt
119281  tcactatgtt ggccaggctg gtctcaaact cctgacctca agatcagcct gccttggcct
119341  cccaaagtgc tgggattaca ggcgtgagcc accgtgcctg gccaatgtat tccttaaatg
119401  catttgattg atgtctcatg cttccctaaa atgtatgaga catccttctc ccctgattgt
119461  tttcaaaagc agtgatccat tgaggtgctc atttttgtaa ccccagttac tagaaaagtg
119521  cctgggtaag agtatgtagg atctctataa aagctgaatt aacgaatttt gtaatgactg
119581  cacctccaga caggagctgt cttccgggct tccacagtct ctgacagccc tctcccacaa
119641  agagtttacc aacagcaagg actttcctgg atgacttcca ctgggttggg gattaaggat
119701  tgaaagggga gaggctgggt gtggaatatt ctggctgtgc tggctgtgga cttagtcctg
119761  tgtcttcccg catccagtgt agtctctgga gaaagaatgc ctgagcttta ccagcaccac
119821  ccaggaagcc cataagaata ttcccatcta tatggattca actggaacca ctgtggagag
119881  atggtacctg cctgcaaacg gcactttatc caggacacct gcctttacga gtgacccccc
119941  aacttggggc cctggatcca gcaggtacgc atggcttcct ggcatccaag agctagcaga
120001  ggagctgaat tttccaggcg tctctgcagg cagcaacccc agctccagtt ctattcaggg
120061  ctgggttcct gggattcttg agcctgagcc cttcttttct accaaaatct cccaggtgga
120121  tcagagctgg cgcaaagagt gggtgctgaa tgtgcccctg tgcaaagagg actgtgagca
120181  atggtgggaa gattgtcgca cctcctacac ctgcaagagc aatgggcaca agggctggaa
120241  ctggacctca ggtgagggct ggggtgggca ggaaaggagg gatttggaag tgaaggtgtg
120301  tgggtgtgga acaggtgtgt gacattttgg ggttgtaggg ctggcagaat cagagaccct
120361  ttggggccca gtggctaaag gtcttccctc ttccctacag ggtctaacaa gtgccaggtg
120421  gcagctgcct gactaccttt ccatctctac tttctcacac ccactgctct gtgcagtgaa
120481  atctggactc actcctacag ggtcagcaac tacaaccgag ggagcagccg ctgcatccag
120541  atgtggttcg acctggccca gggcaacccc aatgaggagg tggcaaggtt ctatgctgca
120601  gctctgagtg ggctgggcc ctgggcagcc tggcctctcc tgctcaacct ggccctaatg
120661  ctgctgtggc tgctcagctg acctcctttt accttctgat acttggacat ccctgccctg
120721  tttagcccca cagctcccaa ctatttggtt cctcttctat ggtcttgtct ctgacagcca
120781  ctttgaataa accagacacc acacatgtat cttgagaatt atttgggtat gaatgggaat
120841  gtggctgttt tgtttcccat ttcttattga ttgaagccag ttagactggg ctagttccca
120901  gctctgatgc ttgctatgaa ctagcctgat acttaagtat tcttctaagg taggagacat
120961  ttgtagctct cgattttatt attcactata gctccaattt agagccaagc ccaggcattt
121021  ttcttttttt gagacagggt ctcactctct cacccaggtt ggaatggagt ggcatgatct
121081  cagctcactg caacgtctgc ctcgtgggct caagtgatcc tcccacctca gcatcccaaa
121141  tacttgggac tacaagcatg tgccaccaca cctggctaat ttttcaattt tttgtagaga
121201  ggaaatcttg caatgttgtc caggctggtc tcaaactact gggctcaagc aatcctccta
121261  cctcagcctc ctgagtagct gggtctacag gtgtgagcct ccacattcag ctaatacata
121321  taatataaaa catgtattat atatgtaata gtagagacag ggtctcacta ttttacccag
121381  gttgttctca aactcctgcg ctggagtgat cctcccaaaa tgctgggatt acaggcatga
121441  gtcactgcat ctggccacaa gtcatgttta tttcttgctc aagctacaat gctgaatgtg
121501  ggttatcagg gggattctgt ttatttctca ttcctggact acattagcag ccaggggggtt
121561  ctgcttctca ctcaagaacc cacgctgatg gaggctccat cttccatgag acaggaagag
121621  ggaacttggc aaattccata aggctcttca aatttccacc catcacttat ttcattggcc
121681  aaatcaagtc atgagcatgc ctaaattcaa gaaagccagg aaaacataat cctatcatgt
121741  acttgcaagg agaggggaat cacaaggttt gcaacagacc taatcatgag taaaaagact
121801  tcattatttt ctcgtagatt tctgtttctg gcatggtcat agttacaaat ttaatttttt
121861  ggttagatcc tagtaagaag gaggaccaac tctctataca tatgcaaata actacatcaa
121921  cctgaaaagt ggagcaatcc actgatgacc gccctggcta gtcttgtaaa caaggccaat
121981  tgatgccttt tcgcccttca tttatttcta tttattattt ttatttttg agacagggtc
122041  tctttctgtt gctcaggctg gagtgcagtg gcacagtcat ggctcactgc agtcttgacc
122101  tcctgagctc aattgatccc tcaactttag cctcccaagt agctgggact acaggcacat
122161  gccaccatgc tgggcaaatg ttttgtattt tttttgtaga gatggggttt tgccatgttg
122221  cccaagctgg tcttgaactg ctgcgcccaa gtgattcatt cacccacctt gacctcctaa
122281  agtgctggga ttagaggcgt gagacacctc tgccagccca ctgatttctt ttttgaaccg
122341  aaagcatagt tgatcaatgc aaaagctaat aattggatat taacaaaatt caaaactctt
122401  gcactttaaa agacaccctt aagaaaatta aaaaacaagc aacagactgg aaggaaatat
122461  ttgcaaacac atctgataaa ggatctgtat ccataaaata caaagaacac ttacaactca
122521  gtaacaagaa ggcagacaac ccaatcaaat aatgggcaaa agaaatgaac aaatatgtca
```

# FIGURE 4-HH

122581 ttaaagaagg tacacaaatt atgaatagtt acacgaaaag atgctcaaca ttattaatca
122641 ttagaaaaat ggaaactaaa accatagtga tagacggttt tatacctact agaatggcaa
122701 taataataat gataggcaat aacaagcatt ggcaaggatg tggagaaatg ggaaccctca
122761 cacagtgctg gtggggatgt aaaatggtac agcccttcgg aaaacagtgt ggcagtttct
122821 caaaaagtta aatgtaaaac tgccatacaa cccaaaattc cactcccagg tgtctactca
122881 ggagaaacga aaacaaacat aacaaaactt aaatatgaat attaataata atcatattta
122941 tgtatgtaaa tatgatgtat catttatgta tgtaaatatg atatatcata ttatgtatca
123001 ttatatataa tagccccaag ctggcaataa cctaaatttt tagcagctgg tgagtagata
123061 gacaaaatgt ggtgaatcca tacaatagaa gactgtgcag caatgaaacg tgatgacaca
123121 tgctatgtcg tggatgagcg tcaaaaacat tatgttaagt gaaagaagcc agacctaaga
123181 ggcttcatgt ggcatgattt cacttacatg aaatgtgcag ggaaggcaaa tttatagagg
123241 cagaaagtag attagtagtt gcctggggct ggaagtgaca acagggatta attgtaaatg
123301 ggcatgaggg gggataagaa ttgaggggat gattctgagt ttctgtgggg tgtatccatg
123361 agggaaagta ttctaaggaa attggcatgt ggctccataa aaatctatca tctgagacaa
123421 actccattcc catcatgaac tttaaaatat tggttggaag ccaagcatgg tggctcatac
123481 ctgtaattcc agcactttgg gaggccaagg caggtagatc acttgagcca gggattctag
123541 aacagcttgg gcaacatggt gaaaccccgt ctctacataa aaatacaaaa aatttagccc
123601 agtgtggtgg tccacgcctg tagtcctagc tatgcaggaa gctgagatgg gaggatctct
123661 ttagctcagg aggtagaggc tgcagtgggc tatgactgtg ccactgcagt ccagcctggg
123721 ttgatagagt gagaccctgt ctcaaaaaaa aaaaaaaaaa aaaataggaa ttgctggatg
123781 gtaatgttgt aggagtttct cctcagttca gccaaagaca gggtccttgt cacctggcca
123841 tgaaatatta ggcttgcaga cactttgaag ggtgagaaaa atggaattta ttgggcaaaa
123901 aaaaaaagcg gggaaacggg gaccctcggc agagtgagag tcctgctagt acacgcttcc
123961 cacctctcag gttgaatccc aggttccacc ctggaagagg aggggccagg ctcctcagca
124021 cgaacttcca gaggctccac cccagtgcac attcctccca gtgtgcgggt ttgtcagagg
124081 ttctctgggg acccctttac acttggctgt ctcattcccc cctctcaaga agtacatcaa
124141 actactgtta gaacaaggat aggggtgagg acgaagaccg atctaaactg cttcctgcta
124201 acagggagcg gtgttttggg aaacggcagt cagagctccc tcagaagcct atctaagggt
124261 tcccggcaga aggcgccatc ttccgaggct ccggttgcac gaccatttgg agtttgatgg
124321 cctgaaggca agaggagaaa aaccaggtta ttagaaaaca tgtgttaaaa cgaaacaagg
124381 gagggtaagg acagcttaaa atccctaggc cttttaccag tttgcacagg gagagggaaa
124441 ccaaaagccc gactggtaaa aaaaacttta cccttttgcc agcatgtcat gcttgtgggt
124501 tcccttcctc tgagcccaat cctaagccaa ccagctgaag gtttgggaaa ttaactcttt
124561 ccagtttgga ggatgcatct gaggggagtg tcctatagta caaagacaca attacctatt
124621 agtgaagaga agacagtgga gaagaaagga aaaaaaggtg ctttttaaag gagtcccagg
124681 ggttcagcat gcattcaaaa ggggtacaga gtgaagatga atggctaccc atctagaaag
124741 aggggagcag gcatccctgt ctcctatctc tttctagcag ataccagggg tacatgaggg
124801 agagaaggaa gagcgtcctc tttccctctt ctgtccttgc atacccaagt cctggtgacc
124861 ttggcaggtg ctgccatggg tgccacagca gcttgcaccc atgaagcagg gagtacctag
124921 agaataggaa ttatcagcca gactcagtgg ctcacgcctg tacaacccag caccttggga
124981 ggccgaggtg ggcagatcat gatttcagga gtttgagacc agcctggcca acatggtgaa
125041 accccgtctc tactaaaaat acaaaactta gctgggtgtg gtggtgcatg cctgtagtcc
125101 cagctactcg ggaggctgag gcaggagaat cacttggtcc caggaggcaa aggttgcagt
125161 gagccaagat cacaccactg cactccagcc tgggcaacag agtaagactc catctcgggg
125221 aaaaaaaaag agagaatagg aattatccgc tcttacctat gtttctatac cccctacagt
125281 cagtagtctt ggagtttcct agacctcatt tatgctgtgg ggaaaagcaa gagagatcag
125341 attgttactc tgtctgtgta gaaagaagta gacataggag actccatttt gttatgtact
125401 aagaaaaatt cttctgcctt gagattctgt taatctataa ccttaccccc aaccctgtgc
125461 tctctgaaac atgtgctgtg tcaactcaga gttgaatgga ttaagggcgg tgcaagatgt
125521 gctttgttaa acagatgctt gagggcagca tgctccttaa gagtcatcac cactccctaa
125581 tctcaagtac ccagggacac aaaaactgcg gaaggccgca gggacctctg cctaggaaaa
125641 ccagatattg tccaaggttt ctccccatgt gatagtctga aatatggcct cgtgggaagg
125701 gaaagacctg accgtccccc agcccgacac ccgtaaaggg tctgtgctga ggaggattag
125761 taaaagagga aggaatgcct cttgcagttg agacaagagg aaggcatctg tctcctgcct
125821 gtccctgggc aatggaatgt ctcggtataa aacccgattg tatgctccat ctactgagat
125881 agggaaaaac cgccttaggg ctggaggtgg gacctgcggg cagcaatact gctttgtaaa
125941 gcattgagat gtttatgtgt atgcatatct aaaagcacag cacttaatcc tttacattgt
126001 ctatgatgca aagacctttg ttcacgtgtt tgtctgctga ccctctcccc acaattgtct
126061 tgtgaccctg acacatcccc ctctttgaga aacacccaca gatgatcaat aaatactaag
126121 ggaactcaga ggctggcggg atcctccata tgctgaacgc tggttcccag ggtcccctta
126181 tttctttctc tatactttgt ctctgtgtct ttttctttc caaatctctc gtcccacctt
126241 acgagaaaca cccacaggtg tgtagggca acccacccct acatatgcca tggatactag

# FIGURE 4-II

```
126301  catgacctt atccatgaaa tgggaggctt ggcttgattg gcaggaatta gccatgctca
126361  cttgcacgca ctgtgccttt tttttttttt tttttttttt tttttttgag acagagtctc
126421  gctctgttgc caggctgaag tgcagtggag tgaactcggc tcactgcaac ctccgactcc
126481  ctggttcaag tgattctcct gcctcagcct cctgagtagc tgggattaca ggcacgcacc
126541  accatgccca gctaattttt gtattttttt tttcgagatg gagtcttgct ctgtcgccca
126601  gtctggagtg cagtggcatg atctcagctc actgcaacct ccacctccca gttcaagcga
126661  ttctcctgcc tctacctccc aagtagctag gattacaggt gcatgccacc atgcctggct
126721  aatttttgta tatttagtag agacggggtt tcaccgtgtt tgccaggatg gtcccgatct
126781  cctgacctca tgatctgccc gccttggcct cccaaagtgc tgggattaca ggtgtgagcc
126841  actgtgcccg gccttgcact gtgcctttta acccctgtta tcatctgcct ctgaatccct
126901  tagatccagt tttctttcct ggggctttga ctcaaagctt ggaatagaat ttgaaacaaa
126961  aatatgtgtc taggaagggc tgcatggact ccttatcata agccaaatgc taaggaaggt
127021  gaagctgcag aattgagtcc tcctccaaca agggagagaa aaagatgtct tgtgacatgc
127081  ccagataatt ggtggctata gttatgtaag ctaggatttg ggtgcatggt gcttggcttt
127141  ggttagctct tactttccaa aaaaggaaac ctccgattaa tgggcatcct atttattccc
127201  atcacctggc aggatttgca ggataattgc tcagaactaa aatattgatc cagattttta
127261  cattacccat cctctccttc tttctgagct gcagctggag attgctggtt ggttcacagg
127321  aacaaggagt gttagcctaa aaatgtaggc aaaaacttaa acactaatga ctttagaatt
127381  tattgacaaa tgtatggtgt tttgaaacat aatttctctc tctccagtcc tcatttttgt
127441  taaaaaagca aattatggta ggattgagtt gtttgcaaaa tagactttag tcatatactt
127501  ggcctgatta tttgcataaa gtgcagcaag aataactatt tctacataag tcttttagat
127561  tggctttgat ggaactctgt tcttcaagga atttcagaca aaacgtttta aaactgagcc
127621  cagccatgga tttgtatcct caaatacctg taagttggat gatcttctcg tgttaaggtc
127681  ccatgacaaa tttggagctc ctagacctgt tagaaagtga cattcttggc caggcgtggt
127741  ggctcacacc tgtaatccta gcaccttggg aggccgaggt gggcggatca ccggaggtca
127801  ggagttcgag accagcctgg ccaacatggt gaaaccctgt ctctacttaa aatacaaaaa
127861  attagctggg tgtggtggca agtgcctgta gtcccagcta tttgggaaac tgaggcagga
127921  gaatcacttg aacctgggag gcggaggttg cagtgagccg agatcatgcc actgcactcc
127981  agcttggcga cagagcaaga gtctgcctca aaaaaaaaaa aaaaagaata ctcacggata
128041  atttccaaat tctggagaag ccaggcagag agagagaaaa atatgcttca aattttgttc
128101  acaggagtgt agcttactct agtattaaag gccttaaaga gttcaaaata attttccttg
128161  actctgagaa acaaaacaag gatcagcaat attccaagca aaagtcaaaa aggttgcttc
128221  agctttctga gttcagtcca ttcagttctt gttatgcttg atattcgtga acatttagc
128281  tcttcatgag tcctgtacat tttcctttat tccaatatca cagtctccaa agttatcaga
128341  aacctgtatt tgagagcacc tgtcagagtc ctatagctta ttacaaacca actttatttt
128401  tttttgagac aggatcttgc tctgttgccc aagctggaat gcagtggtga gatcttggct
128461  cactgcaacc tccacctcct ggattcaagc gattctcctg ccccaacatc ctgagtagct
128521  gggattacag gtatgtgcca ccatgcccgg ctaatttttg tatttttttt tttttagtag
128581  agatggggtt ttaccatgtt gaccaggttg gttttgaact cctgacctca ggtgataccc
128641  ctgacttggc cttccaaagt gctgggatta aaggtgtgag ccactgcacc cagccggatc
128701  aggcactttt aacttgaaaa catgaagttc tctttcctaa ctttttttctt tctttctttt
128761  tttttttttt ttccgagaca ggatctcact ctgtcaccca ggctggagtg cagggatgcc
128821  atcacagctc actgcagccc tgacctcctg ggctcaagca atcctcccag ctcatcctcc
128881  caaatggctg ggactatagg cttagaccac catacccaac taatttttgt atttttgtgt
128941  gaagatgagg tctccctatg tttcccaggc tggtcttgat ctcctgggct caagcaatcc
129001  atcagccttg gcctcccaaa gcactgggat tataggcatg agccaccaag gacggccccc
129061  taatgttttt tatattggct aatgagcttt aagtttatac atacaaggca taaagaaata
129121  actttaaagt tatgttatta gatgtacagt gatatgattt agctctgtgt ccccacccaa
129181  gtctcatctt gtagctccca taactcccat gtgttgtgaa agggacctgg tgggagatga
129241  ttgaatcgtg gaggtgggtc tttcccgtgc tgttctcatg ataatgaatg ggtctcacaa
129301  gatctgatgg ttttaaaaac gggagtttct ctgcacaagc tctctctttg cctggtgcca
129361  tccacataag atgtgacttg cttctccttg ccttccacca tgattgtaag gtctccccag
129421  ccatgtggaa ctgtaaatcc aataaacctc tttcgtaaat tgcccagtct tgcatatgtc
129481  tttatcagca gcgtgaaaac agatgaatac agtaaatttg tactgggagt ggggcattgc
129541  tgaaaagata cccaaaaatg tggaagcgac tttggaactg gggaacaggc agaggttgga
129601  acagtttgga gcgctcagat gaagacagga aaatgtggga aagtctggag cttcctagag
129661  acttgttgaa gggccttgac caaaagcctg atagtgatat ggacaataag gtccaggctg
129721  aggtggtctc agatgaagat gaggaacttg ttgggaactg gagcaaaggt aactcttgta
129781  tgttttagca aagagactgg cagcattttg tccctgccct agagatttgc agaactttga
129841  actcgagaga gatggtttag gctatctggt ggaagaaatt tgtttattat tattattatt
129901  attattatta ttattattat tttgagatag agtctcactc tgtcacccag gccagaggac
129961  agtagcgtga ccttggctca ctgcaacctt cgcctcctga gttcaagcgt ttctcatgtc
```

# FIGURE 4-JJ

```
130021  tcggcctccc aagcagctga gataacaggc atgtgcctcc atgcctggct aatttttgta
130081  ttttagaaga gacagggatt caccacattg gccaggctgg tctcaaactc ctgacctcaa
130141  atgatccaac tcagcctcct gaagtgttgg aattacaggc atgagccacc acacctggct
130201  gcggaagaaa tttctaagct gcaaagcatt caagaggtga cttgggtgtg gttaaaggaa
130261  ttcagtttta taagggaagc agagaataaa ggttcagaaa atttgcagcc tgacaatgcg
130321  atagaaaaga aaagccaatt ttctgaggag aaattcaagc cagctgcaga aatgtgcata
130381  agtaacaaga ggaacattaa cccccaatac aatggggaaa atgtctccag ggcatgtcag
130441  aggtcttctt ggcagcccct cccatcacag gcccagaggc ctaggagaaa tggttccgtg
130501  ggccaggtcc agggtccccg tgctgtatgt agcctaggga cttggtgcct tgtgtcccag
130561  ctgctccagc catggctgaa aggggccaac atagagctta ggccatggct gcagagggtg
130621  aaagccccag tccttggcag cttccacgtg gtattgagct tgcaagtgca cagaagtcaa
130681  gaattggggt ttgggaacct ccacctagat ttcagaagat gtatggaaat gcctggatgc
130741  ccaggcagaa gtttgctgca ggggtggggt gctcatggag aacctctgct agggcagggc
130801  ggaagggaaa tgtggggttg gagctcccac acagagtcct tactggggca ctgcctaggg
130861  cagctgtgag aagagggcca ttgtcctcta gccccaagaa tggtagatcc actgacagct
130921  tgcactgttt gcctggaaaa gctgcagaca ctcaatgcca gcctgtgaaa gcagccggga
130981  gggaggctgt acactgaaaa gccacagggg cggagctgcc agagaccatg ggaacccacc
131041  tcttacatta gtgtgacctg atatgagaga tggagtcaaa ggagatcatt ttggagcttt
131101  aagatttgac tgccctgctg gattttggac ttgcaggggc ctgtagcccc tctgttttgg
131161  ctaatttctc ccattttgga gtggctgtat ttacccaata cctgtactcc cattgtatcc
131221  aggaagtaac taacttgctt ttgattttac aggctgatag gcagaagtgt cttgcttttt
131281  ctttgctgag actttggact gtggactttt gagttaattc tgaaatgagt tgagactttg
131341  ggggactgtt gggaaggcat gattggtttt gaaatgtgaa gatatgagat ttgggagggg
131401  acagggatgg aatgatatgg cttggctgtg tccccaccca aatctcatct tgtagctccc
131461  ataattcact catgttgtgg gagggacctg gtgggagatg attgaatcat gggggtgggt
131521  ctttcccatg ttcttgtgag agtgaatggg tctcacgaga tctgatggtt tttaaaacag
131581  gagtttctct gcacaagctc tctctttgcc tgctgtcatc cacataagat gtgacttgct
131641  cctccttgcc ttctgccatt attgtgaggt ctccccagcc atgtggaact gtaagtccaa
131701  tgaacctctt tcttttgtaa attgctctat ctcgggtatg tctttatcat tcctgaaaat
131761  ggactaatac atacggcaat actcaagaga gcaagtgtgg atagcttggg ttcatgaggt
131821  ttttggtttt tgtcaaaagc ataaatgtat actgaaacta cccagaaaaa gcaaagtata
131881  aaacagtatg ctaccatttg tgcccataaa tgggatatgt gtagtgtaaa tgcatagaat
131941  ttgaccagat gtatacccag agaccagaac actcagtggc ctcatgttca tactttggca
132001  aacacatgta tagtatcctt aacttaaatg taccattgtt gtgtgcattc tagtgttatt
132061  gacatttaca tatctattat caagtcagat aaatcattct gtgtctttgt attttcactt
132121  cctattttgt atatttatgt atacatacac acataccaat acatatttaa ataaggtata
132181  acttcacctg gtccaaaaat caaaacaacg tagaaaggtt tacagtgaaa ggtcgcatcc
132241  ctgatgctgt cctcttcccc caggtgatca ccttattggt ttcttttcat acctttcagc
132301  atttctcct gcaagcacag atattctaac tcctcctttt ttacacagaa ttttttttta
132361  catagcattc ttctgcacct tccttctccc acttcacaat gcacatggag atttttccgt
132421  atttgtacat caggagcttc ctctttcttt gttaccacat taaattccac tgggtagatg
132481  taccataatt taactgggtc cttattgaaa gacaattgag ctgtctccta gacaaagcct
132541  tgtgcacctt cccgaacaga gggtctaacc aagcaggcag gatggggtta taaagtaggt
132601  ggggaggtgg gagagactcc accctcccag gtgggctgag gatggaggta aggccctgca
132661  acaggacaga gggaaaagtg gggatgagac atgggaggcg agatagcgct cactgttctc
132721  gctcagcccc ctcctccatt tgccgctgac ctgttggcct cccccaacct ctgagcctgc
132781  ctctgcctag gtaatttccc aagacccagg aggggtgaag ggtgaggtgc gattgccccc
132841  acctccttgc ctcccgcagc atctgctcca ggaccatgaa caatagctga cagctccatg
132901  gcccttgctg tccccatctc agcttccctg ggcatctaaa cctcagttgc cRtggggtag
132961  gaggacaggc tgaggaagca gaagcctgag gctgtctaga gtctcactcc tgcatcagca
133021  ggccaccacc tgtggttcct ccttgtgcaa atttgagagg aattgcataa aacactggag
133081  aaatccaaga ggggaagtcc
```

# FIGURE 5

>1:23174301-23174600

```
1       agttggctgc cacagcctct gccaagcttt gtctttgggg cttgctgcag aaacctggcc
61      tacggaagat acgacaccac tgggaggtt gtgtaggtgc caggggacca tcgtggttct
121     ctagggcgct gtggaaattg ggtcttgggc tgggtggcat ctggcagtca tggRtaacac
181     ttgcttttcc agttaatgtg gccatgtgat tccaagtgtc atgttgcttt gtggcaagat
241     tgttgtgtga cttgtttttt tgtttttgtt tttgtttttt taaaggaaac tatttgtggg
```

# FIGURE 6-A

NM_000201 [gi:4557877] Homo sapiens intercellular adhesion molecule 1 (CD54), human rhinovirus receptor (ICAM1), mRNA

gcgccccagtcgacgctgagctcctctgctactcagagttgcaacctcagcctcgctatggctcccagcagccccg
gcccgcgctgcccgcactcctggtcctgctcggggctctgttcccaggacctggcaatgcccagacatctgtgtccc
cctcaaaagtcatcctgccccggggaggctccgtgctggtgacatgcagcacctcctgtgaccagcccaagttgttg
ggcatagagaccccgttgcctaaaaaggagttgctcctgcctgggaacaaccggaaggtgtatgaactgagcaatgt
gcaagaagatagccaaccaatgtgctattcaaactgccctgatgggcagtcaacagctaaaaccttcctcaccgtgt
actggactccagaacgggtggaactggcacccctcccctcttggcagccagtgggcaagaaccttaccctacgctgc
caggtggagggtggggcaccccgggccaacctcaccgtggtgctgctccgtggggagaaggagctgaaacgggagcc
agctgtgggggagcccgctgaggtcacgaccacggtgctggtgaggagagatcaccatggagccaatttctcgtgcc
gcactgaactggacctgcggccccaagggctggagctgtttgagaacacctcggccccctaccagctccagaccttt
gtcctgccagcgactcccccacaacttgtcagcccccgggtcctagaggtggacacgcaggggaccgtggtctgttc
cctggacgggctgttcccagtctcggaggcccaggtccacctggcactggggggaccagaggttgaaccccacagtca
cctatggcaacgactccttctcggccaaggcctcagtcagtgtgaccgcagaggacgagggcacccagcggctgacg
tgtgcagtaatactggggaaccagagccaggagacactgcagacagtgaccatctacagctttccggcgcccaacgt
gattctgacgaagccagaggtctcagaagggaccgaggtgacagtgaagtgtgaggcccaccctagagccaaggtga
cgctgaatggggttccagcccagccactgggcccgagggcccagctcctgctgaaggccaccccagaggacaacggg
cgcagcttctcctgctctgcaaccctggaggtggccggccagcttatacacaagaaccagacccgggagcttcgtgt
cctgtatggcccccgactggacgagagggattgtccgggaaactggacgtggccagaaaattcccagcagactccaa
tgtgccaggcttggggaacccattgcccgagctcaagtgtctaaaggatggcactttcccactgcccatcggggaa
tcagtgactgtcactcgagatcttgagggcacctacctctgtcgggccaggagcactcaaggggaggtcacccgcga
ggtgaccgtgaatgtgctctccccccggtatgagattgtcatcatcactgtggtagcagccgcagtcataatgggca
ctgcaggcctcagcacgtacctctataaccgccagcggaagatcaagaaatacagactacaacaggcccaaaaaggg
acccccatgaaaccgaacacacaagccacgcctccctgaacctatcccgggacagggcctcttcctcggccttccca
tattggtggcagtggtgccacactgaacagagtggaagacatatgccatgcagctacacctaccggccctgggacgc
cggaggacagggcattgtcctcagtcagatacaacagcatttggggccatggtacctgcacacctaaaacactaggc
cacgcatctgatctgtagtcacatgactaagccaagaggaaggagcaagactcaagacatgattgatggatgttaaa
gtctagcctgatgagaggggaagtggtgggggagacatagccccaccatgaggacatacaactgggaaatactgaaa
cttgctgcctattgggtatgctgaggcccacagacttacagaagaagtggccctccatagacatgtgtagcatcaaa
acacaaaggcccacacttcctgacggatgccagcttgggcactgctgtctactgaccccaacccttgatgatatgta
tttattcatttgttattttaccagctatttattgagtgtcttttatgtaggctaaatgaacataggtctctggcctc
acggagctcccagtccatgtcacattcaaggtcaccaggtacagttgtacaggttgtacactgcaggagagtgcctg
gcaaaaagatcaaatggggctgggacttctcattggccaacctgcctttccccagaaggagtgatttttctatcggc
acaaaagcactatatggactggtaatggttcacaggttcagagattacccagtgaggccttattcctcccttccccc
caaaactgacacctttgttagccacctccccacccacatacatttctgccagtgttcacaatgacactcagcggtca
tgtctggacatgagtgcccagggaatatgcccaagctatgccttgtcctcttgtcctgtttgcatttcactgggagc
ttgcactattgcagctccagtttcctgcagtgatcagggtcctgcaagcagtggggaaggggccaaggtattggag
gactccctcccagctttggaagggtcatccgcgtgtgtgtgtgtgtatgtgtagacaagctctcgctctgtcacc
caggctggagtgcagtggtgcaatcatggttcactgcagtcttgaccttttgggctcaagtgatcctcccacctcag
cctcctgagtagctgggaccataggctcacaacaccacacctggcaaatttgatttttttttttttttcagagacg
gggtctcgcaacattgcccagacttcctttgtgttagttaataaagctttctcaactgcc

# FIGURE 6-B

NM_001544 [gi:12545400] Homo sapiens intercellular adhesion molecule 4, Landsteiner-Wiener blood group (ICAM4), transcript variant 1, mRNA.

cttttgccatggggtctctgttccctctgtcgctgctgtttttttggcggccgcctacccgggagttgggagcgc
gctgggacgccggactaagcgggcgcaaagccccaagggtagccctctcgcgccctccgggacctcagtgcccttct
gggtgcgcatgagcccggagttcgtggctgtgcagccggggaagtcagtgcagctcaattgcagcaacagctgtccc
cagccgcagaattccagcctccgcaccccgctgcggcaaggcaagacgctcagagggccgggttgggtgtcttacca
gctgctcgacgtgagggcctggagctccctcgcgcactgcctcgtgacctgcgcaggaaaaacacgctgggccacct
ccaggatcaccgcctacaaaccgccccacagcgtgatttggagcctccggtcttaaagggcaggaaatacactttg
cgctgccacgtgacgcaggtgttcccggtgggctacttggtggtgaccctgaggcatggaagccgggtcatctattc
cgaaagcctggagcgcttcaccggcctggatctggccaacgtgaccttgacctacgagtttgctgctggaccccgcg
acttctggcagcccgtgatctgccacgcgcgcctcaatctcgacggcctggtggtccgcaacagctcggcacccatt
acactgatgctcgcttggagccccgcgcccacagctttggcctccggttccatcgctgcccttgtagggatcctcct
cactgtgggcgctgcgtacctatgcaagtgcctagctatgaagtcccaggcgtaaaggggatgttctatgccggct
gagcgagaaaaagaggaatatgaaacaatctggggaaatggccatacatggtggctgacgcctgtaatcccagcact
ttgggaggccgaggcaggagaatcgcttgagcccaggagttcgagaccagcctggacaacatagtgagaccccgtct
atgcaaaaaatacacaaattagcctggtgtggtggcccgcacctgtggtcccagctacccgggaggctgagttggga
ggatcctttgagccctgaaagtcgaggttgcagtgagccttgatcgtgccactgcactccagcctgggggacagagc
acgaccctgtctccaaaaataaaataaaaataaaaataaatattggcgggggaaccctctggaatcaataaaggctt
ccttaaccagc

# FIGURE 6-C

NM_003259 [gi:12545403] Homo sapiens intercellular adhesion molecule 5, telencephalin (ICAM5), mRNA

ccgtcctctagcccagctcctcggctcgcgctctcctcgcctcctgtgctttccccgccgcggcgatgccagggcct
tcgccagggctgcgccgggcgctactcggcctctgggctgctctgggcctggggctcttcggcctctcagcggtctc
gcaggagcccttctgggcggacctgcagcctcgcgtggcgttcgtggagcgcgggggctcgctgtggctgaattgca
gcaccaactgccctcggccggagcgcggtggcctggagacctcgctgcgccgaaacgggacccagaggggtttgcgt
tggttggcgcggcagctggtggacattcgcgagccggagactcagcccgtctgcttcttccgctgcgcgcggcgcac
actacaggcgcgtgggctcattcgcactttccagcgaccagatcgcgtagagctgatgccgctgcctccctggcagc
cggtgggcgagaacttcaccctgagctgtagggtccccggcgccgggccccgtgcgagcctcacgctgaccctgctg
cggggcgcccaggagctgatccgccgcagcttcgccggtgaaccaccccgagcgcggggcgcggtgctcacagccac
ggtactggctcggagggaggaccatggagccaatttctcgtgtcgcgccgagctggacctgcggccgcacggactgg
gactgtttgaaaacagctcggccccagagagctccgaaccttctccctgtctccggatgccccgcgcctcgctgct
ccccggctcttggaagttggctcggaaaggcccgtgagctgcactctggacggactgtttccagcctcagaggccag
ggtctacctcgcactggggaccagaatctgagtcctgatgtcaccctcgaaggggacgcattcgtggccactgcca
cagccacagctagcgcagagcaggagggtgccaggcagctgatctgcaacgtcaccctgggggcgaaaaccgggag
acccgggagaacgtgaccatctacagcttcccggcaccactcctgaccctgagcgaacccagcgtctccgagggca
gatggtgacagtaacctgcgcagctgggacccaagctctggtcacactggagggagttccagccgcggtcccggggc
agcccgcccagcttcagctaaatgccaccgagaacgacgacagacgcagcttcttctgcgacgccaccctcgatgtg
gacggggagaccctgatcaagaacaggagcgcagagcttcgtgtcctatacgctccccggctagacgattcggactg
ccccaggagttggacgtggcccgagggcccagagcagacgctgcgctgcgaggcccgcgggaacccagaaccctcag
tgcactgtgcgcgctccgacggcggggccgtgctggctctgggcctgctgggtccagtcactcgggcgctctcaggc
acttaccgctgcaaggcggccaatgatcaaggcgaggcggtcaaggacgtaacgctaacggtggagtacgcaccagc
gctggacagcgtgggctgcccagaacgcattacttggctggagggaacagaagcctcgctgagctgtgtggcgcacg
ggtaccgccgcctgatgtgatctgcgtgcgctctggagaactcggggccgtcatcgagggggctgttgcgtgtggcc
cgggagcatgcgggcacttaccgctgcgaagccaccaaccctcggggctctgcggccaaaaatgtggccgtcacggt
ggaatatggccccaggtttgaggagccgagctgccccagcaattggacatgggtggaaggatctgggcgcctgtttt
cctgtgaggtcgatgggaagccacagccaagcgtgaagtgcgtgggctccgggggcgccactgagggggtgctgctg
ccgctggcacccccagaccctagtcccagagctcccagaatccctagagtcctggcacccggtatctacgtctgcaa
cgccaccaaccgccacggctccgtggccaaaacagtcgtcgtgagcgcggagtcgccaccggagatggatgaatcta
cctgcccaagtcaccagacgtggctggaaggggctgaggcttccgcgctggcctgcgccgcccggggtcgcccttcc
ccaggagtgcgctgctctcgggaaggcatcccatggcctgagcagcagcgcgtgtcccgagaggacgcgggcactta
ccactgtgtggccaccaatgcgcatggcacggactcccggaccgtcactgtgggcgtggaataccggccagtggtgg
ccgaacttgctgcctcgcccccctggaggcgtgcgcccaggaggaaacttcacgttgacctgccgcgcggaggcctgg
cctccagcccagatcagctggcgcgcgcccccgggggccctcaacatcggcctgtcgagcaacaacagcacactgag
cgtggcaggcgccatgggaagccacggcggcgagtacgagtgcgcacgcaccaacgcgcacggcgccacgcgcggc
gcatcacggtgcgcgtggccggtccgtggctatgggtcgccgtgggcggcgcggcgggggcgcggcgctgctggcc
gcgggggccggcctggccttctacgtgcagtccaccgcctgcaagaagggcgagtacaacgtgcaggaggccgagag
ctcaggcgaggccgtgtgtctcaacggagcgggcggcggcgctggcggggcggcaggcgcggagggcggacccgagg
cggcgggggcgcggccgagtcgccggcggagggcgaggtcttcgccatacagctgacatcggcgtgagccgctccc
ctctccccgcggccgggggacgcccccagactcacacggggcttatttattgctttatttatttacttattcat
ttatttatgtattcaactccaagggcgtcacccccatttctacccatcccctcaataaagttttataaagga

# FIGURE 7

NM_003259 [gi:12545403] Homo sapiens mitogen-activated protein kinase 10 (MAPK10), transcript variant 1, mRNA.

gagaaatggcgtggcaggggacccagcgagcccagagggatttgccgctgcttcctctaccccctgtatttcacgca
gctctctaaattgactcagctccaggctagtgtgagaaacaccaacagcaggcccatctcagatcttcactatggca
acttatgcaagaaactgttgaattagacccgtttcctatagatgagaaaccatacaagctgtggtatttatgagcct
ccatttcttatactactgcagtgaaccaacattggatgtgaaattgccttttgtcagggattcgataaacaagtgg
atgtgtcatatattgccaaacattacaacatgagcaaaagcaaagttgacaaccagttctacagtgtggaagtggga
gactcaaccttcacagttctcaagcgctaccagaatctaaagcctattggctctggggctcagggcatagtttgtgc
cgcgtatgatgctgtccttgacagaaatgtggccattaagaagctcagcagaccctttcagaaccaaacacatgcca
agagagcgtaccgggagctggtcctcatgaagtgtgtgaaccataaaaacattattagtttattaaatgtcttcaca
ccccagaaaacgctggaggagttccaagatgtttacttagtaatggaactgatggatgccaacttatgtcaagtgat
tcagatggaattagaccatgagcgaatgtcttacctgctgtaccaaatgttgtgtggcattaagcacctccattctg
ctggaattattcacagggatttaaaaccaagtaacattgtagtcaagtctgattgcacattgaaaatcctggacttt
ggactggccaggacagcaggcacaagcttcatgatgactccatatgtggtgacacgttattacagagcccctgaggt
catcctggggatgggctacaaggagaacgtggatatatggtctgtgggatgcattatgggagaaatggttcgccaca
aaatcctctttccaggaagggactatattgaccagtggaataaggtaattgaacaactaggaacaccatgtccagaa
ttcatgaagaaattgcaacccacagtaagaaactatgtggagaatcggcccaagtatgcgggactcaccttccccaa
actcttcccagattccctcttcccagcggactccgagcacaataaactcaaagccagccaagccagggacttgttgt
caaagatgctagtgattgacccagcaaaaagaatatcagtggacgacgccttacagcatccctacatcaacgtctgg
tatgacccagccgaagtggaggcgcctccacctcagatatatgacaagcagttggatgaaagagaacacacaattga
agaatggaaagaacttatctacaaggaagtaatgaattcagaagaaaagactaaaaatggtgtagtaaaaggacagc
cttctccttcagcacaggtgcagcagtgaacagcagtgagagtctccctccatcctcgtctgtcaatgacatctcct
ccatgtccaccgaccagaccctggcatctgacactgacagcagcctggaagcctcggcaggacccctgggttgttgc
aggtgactagccgcctgcctgcgaaacccagcgttcttcaggagatgatgtgatggaacacacacacacgcagacac
acacacacacacaaatgcagacacacaacatcaagaaaacagcaagggagagaatccaagcctaaaattaaataaat
ctttcagcctgcttcttccccagggttctgtattgcagctaagctcaaatgtatatttaacttctagttgctcttgc
tttggtcttcttccaatgatgcttactacagaaagcaaatcagacacaattagagaagccttttccataaagtgtaa
ttttaatggctgcaaaaccggcaacctgtaactgcccttttaaatggcatgacaaggtgtgcagtggccccatccag
catgtgtgtgtctctatcttgcatctacctgctccttggcctagtcagatggatgtagatacagatccgcatgtgtc
tgtattcatacagcactacttacttagagatgctactgtcagtgtcctcagggctctaccaagacataatgcactgg
ggtaccacatggtccatttcatgtgatctattactctgacataaacccatctgtaatatattgccagtatataagct
gtttagtttgttaattgattaaactgtatgtcttataagaaaacatgtaaaggggaatatatgggggagtgagct
ctctcagacccttgaagatgtagcttccaaatttgaatggattaaatggcacctgtatacca

Other isoforms are isoform 2 (NM_138982); isoform 3 (NM_138980); isoform 4 (NM_138981)

# FIGURE 8-A

NM_015078: KIAA0861

ttgggcggagatgcctttaaaaatcatccaccgcagcggtagaaacagtttgtttggctttatttatacggaatggtt
tttcagtgaaatgctgtcttgcttaaaagaagagatgcctccccaggagctcacccggcgactggccacagtgatcactc
atgtcgatgaaattatgcagcaggaagtcagacccctgatggcggtggagataatagaacaacttcacagacaatttgcc
attctttcaggaggccgaggggaggatggcgcccccatcatcacgttcccagagttttcggggttcaaacacatcccaga
tgaagacttcctgaatgtcatgacctacctgactagcatccccagtgtggaggctgccagcattggattcattgttgtta
tcgacagacgaagagacaagtggagctccgtaaaggcatccttgacacgaatagctgtggcatttccaggaaacttacag
ctcatattcatccttcgtccatctcgctttatccagaggacattcactgacattggcattaaatactatcgaaatgagtt
taaaacgaaagtgccgatcatcatggtaaactctgtctctgaccttcacggctacatcgacaaaagccaactgacccggg
aattagggggactttggaatatcgccacggtcagtgggtaaatcaccgcactgccatcgaaaactttgccttgaccttg
aagaccactgcccagatgctgcagacgtttgggtcctgcctggccacagcagagctgcccagaagcatgctatccacgga
agaccttctcatgtcccacacaaggcagcgggacaagctgcaggatgagctgaaattacttggaaagcaggggaccacat
tgctgtcatgcatccaagaaccagcaaccaaatgtcccaacagcaaactcaatctcaaccaacttgagaatgtaactacc
atggaaaggttattagttcaactggatgaaacagaaaaagcctttagtcacttttggtctgagcatcatctgaagcttaa
ccagtgcctacaactacagcattttgagcacgattttgtaaggctaagcttgccctggataatttgctggaagagcaag
cagagtttacaggcattggagacagcgtgatgcacgtggagcagattcttaaggaacacaaaaaactggaggaaaaagc
caggagcccctggaaaaggcccagctgctggcactggttggggaccagctcatccaaagccaccattatgcagcagatgc
catcaggccccggtgtgtggagctcaggcacctctgtgacgatttcatcaatggaaacaagaaaaaatgggacattttag
gaaagtccttagagtttcatagacagctggacaaggtcagccaatggtgtgaggcaggaatctacctcttggcttcccaa
gctgtagacaagtgccagtctcgagaaggggttgatatcgccttgaacgacattgcgacattcctgggcacagtcaagga
gtacccgttgctcagccccaaggagttttacaacgagtttgagttgctgctcaccctcgatgcaaaggccaaagcccaga
aagttttgcagaggctggatgatgtccaggaaatatttcacaagaggcaagtgagtctgatgaaactggcagccaaacag
actcgtccagtgcaacctgtggccccacatcctgagtcttcaccaaaatgggtgtcatcaaaaaccagccagccctccac
ctcggtccctctagctcgtcctctgagaacgtctgaggaaccttatacggagacagagttgaactcccggggaaaggaag
atgatgagactaaatttgaagtcaagagtgaagaaatctttgaaagccatcatgaaaggggaaccctgagctggagcag
caggccaggctcggagacctttcccccgcaggcgcattatacgtgacttgcttgagactgaagagatttacataaaaga
gattaaaagcataattgatggatatatcactccaatggattttatttggctaaagcatctaattccagatgttcttcaga
ataacaaggactttctctttgggaatattagagaactttacgaatttcacaacaggacttttctaaaagagttggaaaag
tgtgctgagaaccctgaacttctggcacattgctttctcaagagaaaagaagatcttcagatatattttaaataccataa
gaatctgccccgagctagggcaatctggcaagagtgtcaagactgcgcctactttggggtatgccagcgccaactggatc
acaatctccctcttttaagtatctcaaaggaccaagccagagacttataaaataccagatgctgttgaagggtctgctg
gatttcgagtctcctgaagatatggagatagacccaggtgaactaggaggctcggctaaggatgggccaaagagaaccaa
agattcagcattctcaactgaactacaacaagctttggcagtgatagaggatttgatcaagtcctgtgagttggctgtgg
acctagcagcagtgactgaatgtccggacgatattggaaaactaggcaagctgttgctgcacggccctttcagcgtctgg
acaattcacaaggatcgttataaaatgaaggatttgattcgatttaaacccagccagaggcaaatctacctatttgaaag
gggaatagtgttctgtaagatacgaatggagcctggggaccagggattatctcctcattacagcttcaagaagaccatga
agctgatgacactttcaattcgccagcttggaaggggagccatagaaagtttgagattgccagtcgaaatggacttgag
aaatacatcctgcaggcagcttcaaaagaaatcagagactgttggttttcagaaataagtaaattattgatggaacaaca
aaataatatcaaagaccaaggaaatccacagtttgaaatgagcacgagcaaaggcagtggagcaggatccggaccatgga
ttaaaaatatggaaagagctaccactagcaaggaagacccggcctccagcacaggagggattaaaggctgctccagcagg
gagtttagctccatggacacctttgaagactgtgaaggcgcagaagacatggaaaaggagagcagtgctctgagtctcgc
gggcctttttccagtcggacgacagtcacgaaacctgttcctccaaatctgctttcctggagaggggagaaagcagccagg
gagaaaaagaagaacgcgatgaggaggaaacggcgacccgcagcaccgaggaggagcgcgctggggcgtccacgggccgg
ctggctcctgcgggggcgacggctggtttccaggcgagggcgctgcgcccgaggacctccgcccaggagagctgacctcc
ctgcggacgcccccgctcctcggctccagagcgcccgcattcccgggagaggcggtgtgggggcccgggccctgcccagc
tacgcagaaagcagccggagcctcggcggcggcagaaaggggacaaccagggcctcctccgaggagcccgaggggtgtcc
tgggtgcgcgcctagctccgcacgggggacctcggagctgctctaaggcgcctgcagaggcgagcagagcccgcagccca
cgccttctcgaccgcgcacttcgacattcggagccgggcaattctttgctgcgtgggcttctctgtgctcccacggtagg
atgtttagtagcacccctggcctctacccactaggtgccaggaatgcgccaccccatcctccaccccgcccccaaatcgt
gacaatcaaaaatgcctgcagacacgcccgcatttctccagggcggggtgggaatcggttgagagccgcttagcccgagc

# FIGURE 8-B

cttggaggagccggagccgctcaaacccggcgggggccgcagactgggagctcccggtccgcctcccagcatccctgcga
gcgttcatggggtgttcgtgttagtgccaagattgcttcgttgtagagagagttcgttccaagttactttctgaggtatt
ttatgtatcgatttattagtttttaaatgagttttgttagtttcagttgtattttatttttagttttattagttgatttt
catttctttgtagctttctggttatttaattttatagtttcagttactggtttatagttacttttattttcaaagttattt
agttgttcattttcagttatttatatgtagttgttttgttttaggagttacagatgttcaaattaatttgcttggaattt
atttatttatttatttatttatttttcgagacggagtctcgctctgtcgcccaggctggagtgcagtggtgtgatctcgg
ctcactgcaaaccgcctcccgggttcacgccattctcctgcctcagcctcctgagtagctgggactacaggcgcccgcca
ccacgcccggctaatttttttatttggattttagtagagacggggtttccccgtgttagccaggatggtctcgatctcct
gacctcgtgatccccctgcctcggcctcccaaagtgctgggattacaggcgtgagccaccgcgcccagccggaatttatt
tttaattatctttacaattattatctgagttatttaccttcatagttatttcactttatcttattggagtttttgagtta
tttatttttacagtaacttatttgactattaaacactcactggaagttcatg

# FIGURE 9-A

NM_006185 [gi:5453819] Homo sapiens nuclear mitotic apparatus protein 1 (NUMA1), mRNA.

gcccacgaagaggtacgattccggagaatcgcgaggcagagcgggagcgcgcagccaggtggaaactaattctaagccag
actgctggagatcaccctgttctagtgtgtggaggcttccaccaggagtctggagtgcaatggcacgatctcggctcact
gcaacctccacctcccaggttcaagcgattctcctgcctcagcctcccaagtagctgggattacaggcgcattggagtga
ctgtctggcatcaccaagatgacactccacgccacccggggggctgcactcctctcttgggtgaacagtctacacgtggc
tgaccctgtggaggctgtgctgcagctccaggactgcagcatcttcatcaagatcattgacagaatccatggcactgaag
agggacagcaaatcttgaagcagccggtgtcagagagactggactttgtgtgcagttttctgcagaaaaatcgaaaacat
ccctcttccccagaatgcctggtatctgcacagaaggtgctagagggatcagagctggaactggcgaagatgaccatgct
gctcttataccactctaccatgagctccaaaagtcccagggactgggaacagtttgaatataaaattcaggctgagttgg
ctgtcattcttaaatttgtgctggaccatgaggacgggctaaaccttaatgaggacctagagaacttcctacagaaagct
cctgtgccttctacctgttctagcacattccctgaagagctctccccacctagccaccaggccaagagggagattcgctt
cctagagctacagaaggttgcctcctcttccagtgggaacaactttctctcaggttctccagcttctcccatgggtgata
tcctgcagaccccacagttccagatgagacggctgaagaagcagcttgctgatgagagaagtaatagggatgagctggag
ctggagctagctgagaaccgcaagctcctcaccgagaaggatgcacagatagccatgatgcagcagcgcattgaccgcct
agccctgctgaatgagaagcaggcggccagcccactggagcccaaggagcttgaggagctgcgtgacaagaatgagagcc
ttaccatgcggctgcatgaaaccctgaagcagtgccaggacctgaagacagagaagagccagatggatcgcaaaatcaac
cagctttcggaggagaatggagacctttcctttaagctgcgggagtttgccagtcatctgcagcagctacaggatgccct
caatgagctgacggaggagcacagcaaggccactcaggagtggctagagaagcaggcccagctggagaaggagctcagcg
cagccctgcaggacaagaaatgccttgaagagaagaacgaaatccttcagggaaaactttcacagctggaagaacacttg
tcccagctgcaggataacccaccccaggagaagggcgaggtgctgggtgatgtcttgcagctggaaaccttgaagcaaga
ggcagccactcttgctgcaaacaacacacagctccaagccagggtagagatgctggagactgagcgaggccagcaggaag
ccaagctgcttgctgagcgggcccacttcgaagaagaaaagcagcagctgtctagcctgatcactgacctgcagagctcc
atctccaacctcagccaggccaaggaagagctggagcaggcctcccaggctcatggggcccggttgactgcccaggtggc
ctctctgacctctgagctcaccacactcaatgccaccatccagcaacaggatcaagaactggctggcctgaagcagcagg
ccaaagagaagcaggcccagctagcacagaccctccaacagcaagaacaggcctcccagggcctccgccaccaggtggag
cagctaagcagtagcctgaagcagaaggagcagcagttgaaggaggtagcggagaagcaggaggcaactaggcaggacca
tgcccagcaactggccactgctgcagaggagcgagaggcctccttaagggagcgggatgcggctctcaagcagctggagg
cactggagaaggagaaggctgccaagctggagattctgcagcagcaacttcaggtggctaatgaagcccgggacagtgcc
cagacctcagtgacacaggcccagcgggagaaggcagagctgagccggaaggtggaggaactccaggcctgtgttgagac
agcccgccaggaacagcatgaggcccaggcccaggttgcagagctagagttgcagctgcggtctgagcagcaaaaagcaa
ctgagaaagaaagggtggcccaggagaaggaccagctccaggagcagctccaggccctcaaagagtccttgaaggtcacc
aagggcagccttgaagaggagaagcgcagggctgcagatgccctggaagagcagcagcgttgtatctctgagctgaaggc
agagacccgaagcctggtggagcagcataagcgggaacgaaaggagctggaagaagagagggctgggcgcaaggggctgg
aggctcgattactgcagcttggggaggcccatcaggctgagactgaagtcctgcggcgggagctggcagaggccatggct
gcccagcacacagctgagagtgagtgtgagcagctcgtcaaagaagtagctgcctggcgtgacgggtatgaggatagcca
gcaagaggaggcacagtatggcgccatgttccaggaacagctgatgactttgaaggaggaatgtgagaaggcccgccagg
agctgcaggaggcaaaggagaaggtggcaggcatagaatcccacagcgagctccagataagccggcagcagaacaaacta
gctgagctccatgccaacctggccagagcactccagcaggtccaagagaaggaagtcagggcccagaagcttgcagatga
cctctccactctgcaggaaaagatggctgccaccagcaaagaggtggcccgcttggagaccttggtgcgcaaggcaggtg
agcagcaggaaacagcctcccgggagttagtcaaggagcctgcgagggcaggagacagacagcccgagtggctggaagag
caacagggacgccagttctgcagcacacaggcagcgctgcaggctatggagcgggaggcagagcagatgggcaatgagct
ggaacggctgcgggccgcgctgatggagagccaggggcagcagcaggaggagcgtgggcagcaggaaagggaggtggcgc
ggctgacccaggagcggggccgtgcccaggctgaccttgccctggagaaggcggccagagcagagcttgagatgcggctg
cagaacgccctcaacgagcagcgtgtggagttcgctaccctgcaagaggcactggctcatgccctgacggaaaaggaagg
caaggaccaggagttggccaagcttcgtggtctggaggcagcccagataaaagagctggaggaacttcggcaaaccgtga
agcaactgaaggaacagctggctaagaaagaaaaggagcacgcatctggctcaggagcccaatctgaggctgctggcagg
acagagccaacaggccccaagctggaagcactgcgggcagaggtgagcaagctggaacagcaatgccagaagcagcagga
gcaggctgacagcctggaacgcagcctcgaggctgagcgggcctcccgggctgagcgggacagtgctctggagactctgc

# FIGURE 9-B

agggccagttagaggagaaggcccaggagctagggcacagtcagagtgccttagcctcggcccaacgggagttggctgcc
ttccgcaccaaggtacaagaccacagcaaggctgaagatgagtggaaggcccaggtggcccggggccggcaagaggctga
gaggaaaaatagcctcatcagcagcttggaggaggaggtgtccatcctgaatcgccaggtcctggagaaggagggggaga
gcaaggagttgaagcggctggtgatggccgagtcagagaagagccagaagctggaggagagctgcgcctgctgcaggcag
agacagccagcaacagtgccagagctgcagaacgcagctctgctctgcgggaggaggtgcagagcctccgggagggaggc
tgagaaacagcgggtggcttcagagaacctgcggcaggagctgacctcacaggctgagcgtgcggaggagctgggccaag
aattgaaggcgtggcaggagaagttcttccagaaagagcaggccctctccaccctgcagctcgagcacaccagcacacag
gccctggtgagtgagctgctgccagctaagcacctctgccagcagctgcaggccgagcaggccgctgccgagaaacgcca
ccgtgaggagctggagcagagcaagcaggccgctggggactgcgggcagagctgctgcgggcccagcgggagcttgggg
agctgattcctctgcggcagaaggtggcagagcaggagcgaacagctcagcagctgcgggcagagaaggccagctatgca
gagcagctgagcatgctgaagaaggcgcatggcctgctggcagaggagaaccggggctgggtgagcgggccaaccttgg
ccggcagtttctggaagtggagttggaccaggcccgggaaaagtatgtccaagagttggcagccgtacgtgctgatgctg
agacccgtctggctgaggtgcagcgagaagcacagagcactgcccgggagctggaggtgatgactgccaagtatgagggt
gccaaggtcaaggtcctggaggagaggcagcggttccaggaagagaggcagaaactcactgcccaggtggaagaactgag
taagaaactggctgactctgaccaagccagcaaggtgcagcagcagaagctgaaggctgtccaggctcagggaggcgaga
gccagcaggaggcccagcgcttccaggcccagctgaatgaactgcaagcccagttgagccagaaggagcaggcagctgag
cactataagctgcagatggagaaagccaaaacacattatgatgccaagaagcagcagaaccaagagctgcaggagcagct
gcggagcctggagcagctgcagaaggaaaacaaagagctgcgagctgaagctgaacggctgggccatgagctacagcagg
ctgggctgaagaccaaggaggctgaacagacctgccgccaccttactgcccaggtgcgcagcctggaggcacaggttgcc
catgcagaccagcagcttcgagacctgggcaaattccaggtggcaactgatgctttaaagagccgtgagccccaggctaa
gccccagctggacttgagtattgacagcctggatctgagctgcgaggaggggaccccactcagtatcaccagcaagctgc
ctcgtacccagccagacggcaccagcgtccctggagaaccagcctcacctatctcccagcgcctgcccccaaggtagaa
tccctggagagtctctacttcactcccatccctgctcggagtcaggccccctggagagcagcctggactccctgggaga
cgtcttcctggactcgggtcgtaagacccgctccgctcgtcggcgcaccacgcagatcatcaacatcaccatgaccaaga
agctagatgtggaagagccagacagcgccaactcatcgttctacagcacgcggtctgctcctgcttcccaggctagcctg
cgagccacctcctctactcagtctctagctcgcctgggttctcccgattatggcaactcagccctgctcagcttgcctgg
ctaccgccccaccactcgcagttctgctcgtcgttcccaggccggggtgtccagtggggcccctccaggaaggaacagct
tctacatgggcacttgccaggatgagcctgagcagctggatgactggaaccgcattgcagagctgcagcagcgcaatcga
gtgtgcccccacatctgaagacctgctatcccctggagtccaggccttccctgagcctgggcaccatcacagatgagga
gatgaaaactggagacccccaagagaccctgcgccgagccagcatgcagccaatccagatagccgagggcactggcatca
ccacccggcagcagcgcaaacgggtctccctagagccccaccagggccctggaactcctgagtctaagaaggccaccagc
tgtttcccacgccccatgactccccgagaccgacatgaagggcgcaaacagagcactactgaggcccagaagaaagcagc
tccagcttctactaaacaggctgaccggcgccagtcgatggccttcagcatcctcaacacacccaagaagctagggaaca
gccttctgcggcggggagcctcaaagaaggccctgtccaaggcttcccccaacactcgcagtggaacccgccgttctccg
cgcattgccaccaccacagccagtgccgccactgctgccgccattggtgccacccctcgagccaagggcaaggcaaagca
ctaaagggccagtaccagtgagtggccccacctgtgtccccgatgctgccgtcacctggtcctccgcctactgtccctct
cagtgccttctctcagctcccaggccaacagtagccaaacccctagagacagtgatgcctgcccgcaccctggcctggcc
cctggtccttcactggcgccttctcggagctggcccaggggcctggagcatggacagtgtgggcgctctccctaccttg
cctcctttttcttaaagcaaagtcacttctccatcacaaccagatttgaggctggttttgatggctgggtccttgggcc
tggccagtcttcctcttagcctctggatctagaagggaccataagaggagtaggccctggttcctgctgtcctggtggct
gggccagcaggggccctcactcttgaagtccaggactgggtctgacctggtgggagcacctgccagaggatgctctttcc
caggacggatgggccctgtgtctcaggagtggggttggggacagccttcagcagcagctcacaccctaccttccccaga
cttgcactggggtgggatttggagtgatgggaaggtttttaagggccggggatggatcttttctaaatgttattacttgt
aaataaagtctatttttt

# FIGURE 10A

NP_000192 [gi:4557878]. intercellular adhesion molecule 1 precursor; CD54 [Homo sapiens]

MAPSSPRPALPALLVLLGALFPGPGNAQTSVSPSKVILPRGGSVLVTCSTSCDQPK
LLGIETPLPKKELLLPGNNRKVYELSNVQEDSQPMCYSNCPDGQSTAKTFLTVY
WTPERVELAPLPSWQPVGKNLTLRCQVEGGAPRANLTVVLLRGEKELKREPAV
GEPAEVTTTVLVRRDHHGANFSCRTELDLRPQGLELFENTSAPYQLQTFVLPATP
PQLVSPRVLEVDTQGTVVCSLDGLFPVSEAQVHLALGDQRLNPTVTYGNDSFSA
KASVSVTAEDEGTQRLTCAVILGNQSQETLQTVTIYSFPAPNVILTKPEVSEGTEV
TVKCEAHPRAKVTLNGVPAQPLGPRAQLLLKATPEDNGRSFSCSATLEVAGQLI
HKNQTRELRVLYGPRLDERDCPGNWTWPENSQQTPMCQAWGNPLPELKCLKD
GTFPLPIGESVTVTRDLEGTYLCRARSTQGEVTREVTVNVLSPRYEIVIITVVAAA
VIMGTAGLSTYLYNRQRKIKKYRLQQAQKGTPMKPNTQATPP

# FIGURE 10B

NP_001535 [gi:4504561] intercellular adhesion molecule 4 isoform 1 precursor; Landsteiner-Wiener blood group protein [Homo sapiens].

MGSLFPLSLLFFLAAAYPGVGSALGRRTKRAQSPKGSPLAPSGTSVPFWVRMSPE
FVAVQPGKSVQLNCSNSCPQPQNSSLRTPLRQGKTLRGPGWVSYQLLDVRAWSS
LAHCLVTCAGKTRWATSRITAYKPPHSVILEPPVLKGRKYTLRCHVTQVFPVGY
LVVTLRHGSRVIYSESLERFTGLDLANVTLTYEFAAGPRDFWQPVICHARLNLDG
LVVRNSSAPITLMLAWSPAPTALASGSIAALVGILLTVGAAYLCKCLAMKSQA

# FIGURE 10C

NP_003250 [gi:12545404] intercellular adhesion molecule 5 precursor; telencephalin [Homo sapiens];

MPGPSPGLRRALLGLWAALGLGLFGLSAVSQEPFWADLQPRVAFVERGGSLWL
NCSTNCPRPERGGLETSLRRNGTQRGLRWLARQLVDIREPETQPVCFFRCARRTL
QARGLIRTFQRPDRVELMPLPPWQPVGENFTLSCRVPGAGPRASLTLTLLRGAQE
LIRRSFAGEPPRARGAVLTATVLARREDHGANFSCRAELDLRPHGLGLFENSSAP
RELRTFSLSPDAPRLAAPRLLEVGSERPVSCTLDGLFPASEARVYLALGDQNLSPD
VTLEGDAFVATATATASAEQEGARQLICNVTLGGENRETRENVTIYSFPAPLLTL
SEPSVSEGQMVTVTCAAGTQALVTLEGVPAAVPGQPAQLQLNATENDDRRSFFC
DATLDVDGETLIKNRSAELRVLYAPRLDDSDCPRSWTWPEGPEQTLRCEARGNP
EPSVHCARSDGGAVLALGLLGPVTRALSGTYRCKAANDQGEAVKDVTLTVEYA
PALDSVGCPERITWLEGTEASLSCVAHGVPPPDVICVRSGELGAVIEGLLRVARE
HAGTYRCEATNPRGSAAKNVAVTVEYGPRFEEPSCPSNWTWVEGSGRLFSCEV
DGKPQPSVKCVGSGGATEGVLLPLAPPDPSPRAPRIPRVLAPGIYVCNATNRHGS
VAKTVVVSAESPPEMDESTCPSHQTWLEGAEASALACAARGRPSPGVRCSREGIP
WPEQQRVSREDAGTYHCVATNAHGTDSRTVTVGVEYRPVVAELAASPPGGVRP
GGNFTLTCRAEAWPPAQISWRAPPGALNIGLSSNNSTLSVAGAMGSHGGEYECA
RTNAHGRHARRITVRVAGPWLWVAVGGAAGGAALLAAGAGLAFYVQSTACKK
GEYNVQEAESSGEAVCLNGAGGGAGGAAGAEGGPEAAGGAAESPAEGEVFAIQ
LTSA

# FIGURE 11

NP_002744 [gi:4506081] mitogen-activated protein kinase 10 isoform 1; JNK3 alpha protein kinase; stress activated protein kinase beta; MAP kinase; c-Jun kinase 3; c-Jun N-terminal kinase 3; stress activated protein kinase JNK3 [Homo sapiens].

MSLHFLYYCSEPTLDVKIAFCQGFDKQVDVSYIAKHYNMSKSKVDNQFYSVEVG
DSTFTVLKRYQNLKPIGSGAQGIVCAAYDAVLDRNVAIKKLSRPFQNQTHAKRA
YRELVLMKCVNHKNIISLLNVFTPQKTLEEFQDVYLVMELMDANLCQVIQMELD
HERMSYLLYQMLCGIKHLHSAGIIHRDLKPSNIVVKSDCTLKILDFGLARTAGTSF
MMTPYVVTRYYRAPEVILGMGYKENVDIWSVGCIMGEMVRHKILFPGRDYIDQ
WNKVIEQLGTPCPEFMKKLQPTVRNYVENRPKYAGLTFPKLFPDSLFPADSEHN
KLKASQARDLLSKMLVIDPAKRISVDDALQHPYINVWYDPAEVEAPPPQIYDKQ
LDEREHTIEEWKELIYKEVMNSEEKTKNGVVKGQPSPSAQVQQ

Additional isoforms are isoform 2 (NP_620448); isoform 3 (NP_620446); isoform 4 (NP_620447)

# FIGURE 12

NP_055893: KIAA0861

MLSCLKEEMPPQELTRRLATVITHVDEIMQQEVRPLMAVEIIEQLHRQFAILSGGR
GEDGAPIITFPEFSGFKHIPDEDFLNVMTYLTSIPSVEAASIGFIVVIDRRRDKWSSV
KASLTRIAVAFPGNLQLIFILRPSRFIQRTFTDIGIKYYRNEFKTKVPIIMVNSVSDL
HGYIDKSQLTRELGGTLEYRHGQWVNHRTAIENFALTLKTTAQMLQTFGSCLAT
AELPRSMLSTEDLLMSHTRQRDKLQDELKLLGKQGTTLLSCIQEPATKCPNSKLN
LNQLENVTTMERLLVQLDETEKAFSHFWSEHHLKLNQCLQLQHFEHDFCKAKL
ALDNLLEEQAEFTGIGDSVMHVEQILKEHKKLEEKSQEPLEKAQLLALVGDQLIQ
SHHYAADAIRPRCVELRHLCDDFINGNKKKWDILGKSLEFHRQLDKVSQWCEAG
IYLLASQAVDKCQSREGVDIALNDIATFLGTVKEYPLLSPKEFYNEFELLLTLDAK
AKAQKVLQRLDDVQEIFHKRQVSLMKLAAKQTRPVQPVAPHPESSPKWVSSKTS
QPSTSVPLARPLRTSEEPYTETELNSRGKEDDETKFEVKSEEIFESHHERGNPELEQ
QARLGDLSPRRRIIRDLLETEEIYIKEIKSIIDGYITPMDFIWLKHLIPDVLQNNKDF
LFGNIRELYEFHNRTFLKELEKCAENPELLAHCFLKRKEDLQIYFKYHKNLPRAR
AIWQECQDCAYFGVCQRQLDHNLPLFKYLKGPSQRLIKYQMLLKGLLDFESPED
MEIDPGELGGSAKDGPKRTKDSAFSTELQQALAVIEDLIKSCELAVDLAAVTECP
DDIGKLGKLLLHGPFSVWTIHKDRYKMKDLIRFKPSQRQIYLFERGIVFCKIRMEP
GDQGLSPHYSFKKTMKLMTLSIRQLGRGSHRKFEIASRNGLEKYILQAASKEIRD
CWFSEISKLLMEQQNNIKDQGNPQFEMSTSKGSGAGSGPWIKNMERATTSKEDP
ASSTGGIKGCSSREFSSMDTFEDCEGAEDMEKESSALSLAGLFQSDDSHETCSSKS
AFLERGESSQGEKEERDEEETATRSTEEERAGASTGRLAPAGATAGFQARALRPR
TSAQES

# FIGURE 13

NP_006176 [gi: 5453820] nuclear mitotic apparatus protein 1 [Homo sapiens].

MTLHATRGAALLSWVNSLHVADPVEAVLQLQDCSIFIKIIDRIHGTEEGQQILKQP
VSERLDFVCSFLQKNRKHPSSPECLVSAQKVLEGSELELAKMTMLLLYHSTMSS
KSPRDWEQFEYKIQAELAVILKFVLDHEDGLNLNEDLENFLQKAPVPSTCSSTFP
EELSPPSHQAKREIRFLELQKVASSSSGNNFLSGSPASPMGDILQTPQFQMRRLKK
QLADERSNRDELELELAENRKLLTEKDAQIAMMQQRIDRLALLNEKQAASPLEP
KELEELRDKNESLTMRLHETLKQCQDLKTEKSQMDRKINQLSEENGDLSFKLRE
FASHLQQLQDALNELTEEHSKATQEWLEKQAQLEKELSAALQDKKCLEEKNEIL
QGKLSQLEEHLSQLQDNPPQEKGEVLGDVLQLETLKQEAATLAANNTQLQARV
EMLETERGQQEAKLLAERGHFEEEKQQLSSLITDLQSSISNLSQAKEELEQASQA
HGARLTAQVASLTSELTTLNATIQQQDQELAGLKQQAKEKQAQLAQTLQQQEQ
ASQGLRHQVEQLSSSLKQKEQQLKEVAEKQEATRQDHAQQLATAAEEREASLR
ERDAALKQLEALEKEKAAKLEILQQQLQVANEARDSAQTSVTQAQREKAELSRK
VEELQACVETARQEQHEAQAQVAELELQLRSEQQKATEKERVAQEKDQLQEQL
QALKESLKVTKGSLEEEKRRAADALEEQQRCISELKAETRSLVEQHKRERKELEE
ERAGRKGLEARLLQLGEAHQAETEVLRRELAEAMAAQHTAESECEQLVKEVAA
WRDGYEDSQQEEAQYGAMFQEQLMTLKEECEKARQELQEAKEKVAGIESHSEL
QISRQQNKLAELHANLARALQQVQEKEVRAQKLADDLSTLQEKMAATSKEVAR
LETLVRKAGEQQETASRELVKEPARAGDRQPEWLEEQQGRQFCSTQAALQAME
REAEQMGNELERLRAALMESQGQQQEERGQQEREVARLTQERGRAQADLALEK
AARAELEMRLQNALNEQRVEFATLQEALAHALTEKEGKDQELAKLRGLEAAQI
KELEELRQTVKQLKEQLAKKEKEHASGSGAQSEAAGRTEPTGPKLEALRAEVSK
LEQQCQKQQEQADSLERSLEAERASRAERDSALETLQGQLEEKAQELGHSQSAL
ASAQRELAAFRTKVQDHSKAEDEWKAQVARGRQEAERKNSLISSLEEEVSILNR
QVLEKEGESKELKRLVMAESEKSQKLEESCACCRQRQPATVPELQNAALLCGRR
CRASGREAEKQRVASENLRQELTSQAERAEELGQELKAWQEKFFQKEQALSTLQ
LEHTSTQALVSELLPAKHLCQQLQAEQAAAEKRHREELEQSKQAAGGLRAELLR
AQRELGELIPLRQKVAEQERTAQQLRAEKASYAEQLSMLKKAHGLLAEENRGLG
ERANLGRQFLEVELDQAREKYVQELAAVRADAETRLAEVQREAQSTARELEVM
TAKYEGAKVKVLEERQRFQEERQKLTAQVEELSKKLADSDQASKVQQQKLKAV
QAQGGESQQEAQRFQAQLNELQAQLSQKEQAAEHYKLQMEKAKTHYDAKKQQ
NQELQEQLRSLEQLQKENKELRAEAERLGHELQQAGLKTKEAEQTCRHLTAQV
RSLEAQVAHADQQLRDLGKFQVATDALKSREPQAKPQLDLSIDSLDLSCEEGTPL
SITSKLPRTQPDGTSVPGEPASPISQRLPPKVESLESLYFTPIPARSQAPLESSLDSLG
DVFLDSGRKTRSARRRTTQIINITMTKKLDVEEPDSANSSFYSTRSAPASQASLRA
TSSTQSLARLGSPDYGNSALLSLPGYRPTTRSSARRSQAGVSSGAPPGRNSFYMG
TCQDEPEQLDDWNRIAELQQRNRVCPPHLKTCYPLESRPSLSLGTITDEEMKTGD
PQETLRRASMQPIQIAEGTGITTRQQRKRVSLEPHQGPGTPESKKATSCFPRPMTP
RDRHEGRKQSTTEAQKKAAPASTKQADRRQSMAFSILNTPKKLGNSLLRRGASK
KALSKASPNTRSGTRRSPRIATTTASAATAAIGATPRAKGKAKH

# FIGURE 14

## ICAM1, ICAM4 and ICAM5 Region

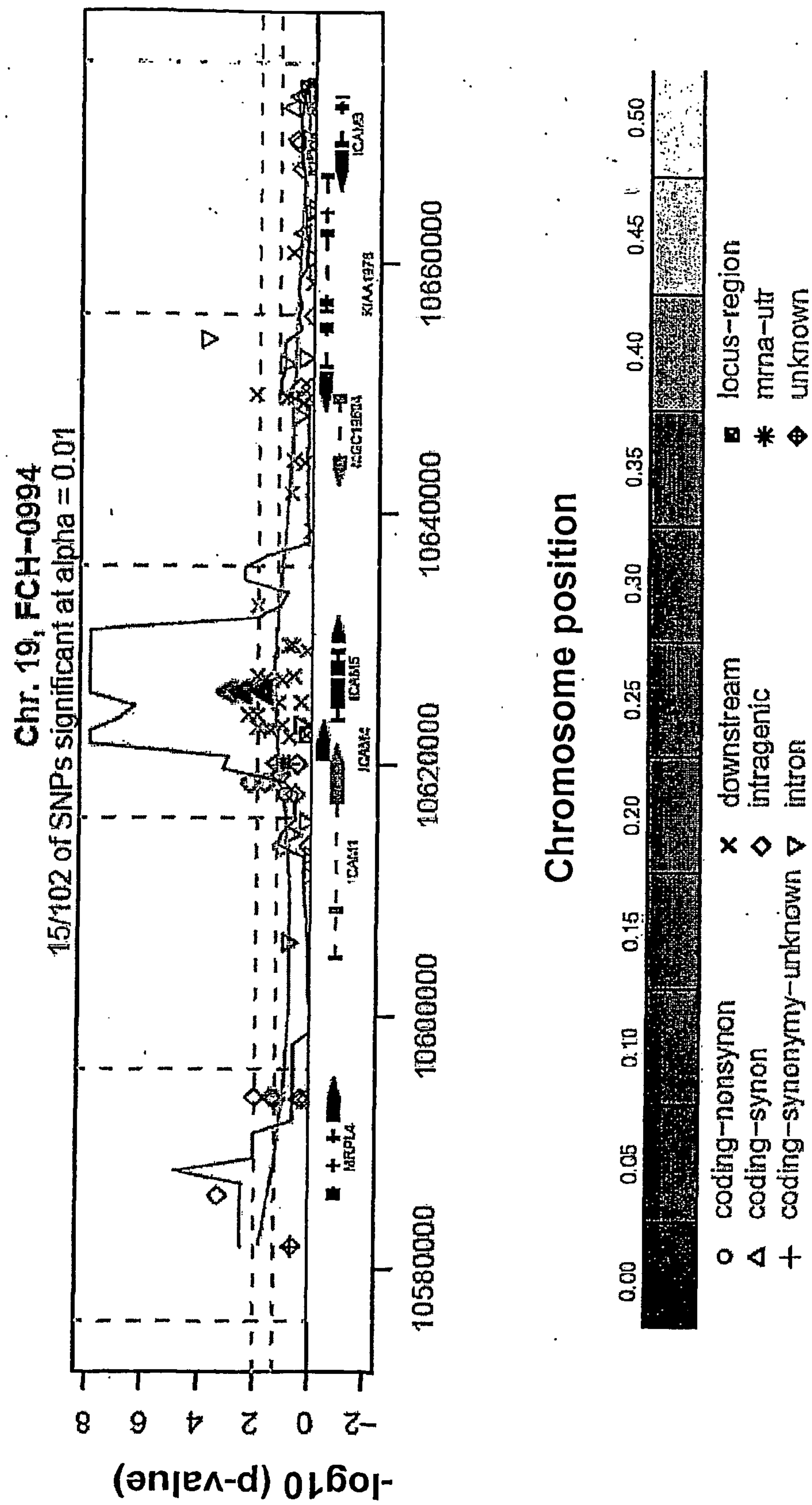

# FIGURE 15

## MAPK10

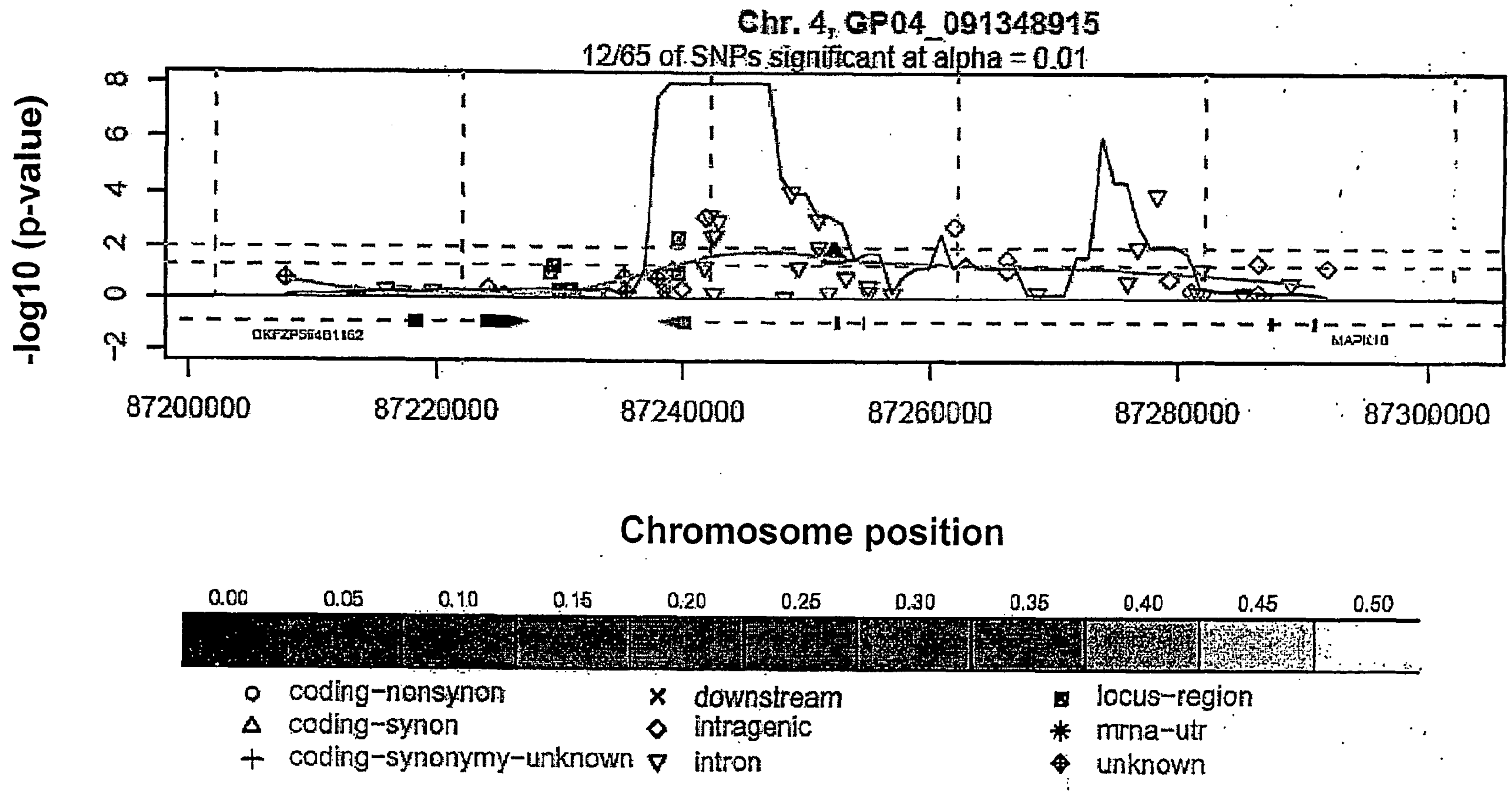

# FIGURE 16

## KIAA0861

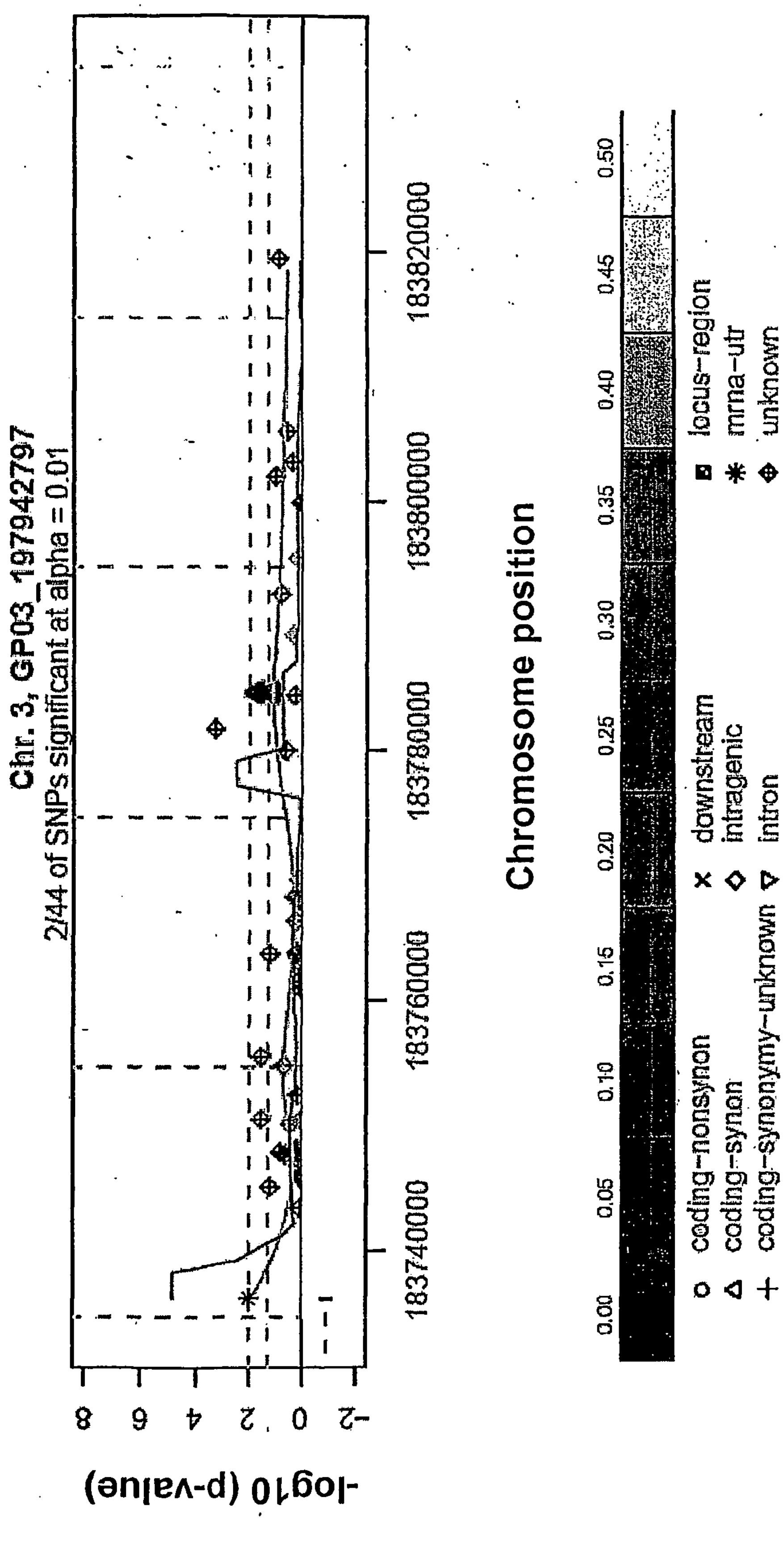

# FIGURE 17

## NUMA1/FLJ20625/LOC220074 region

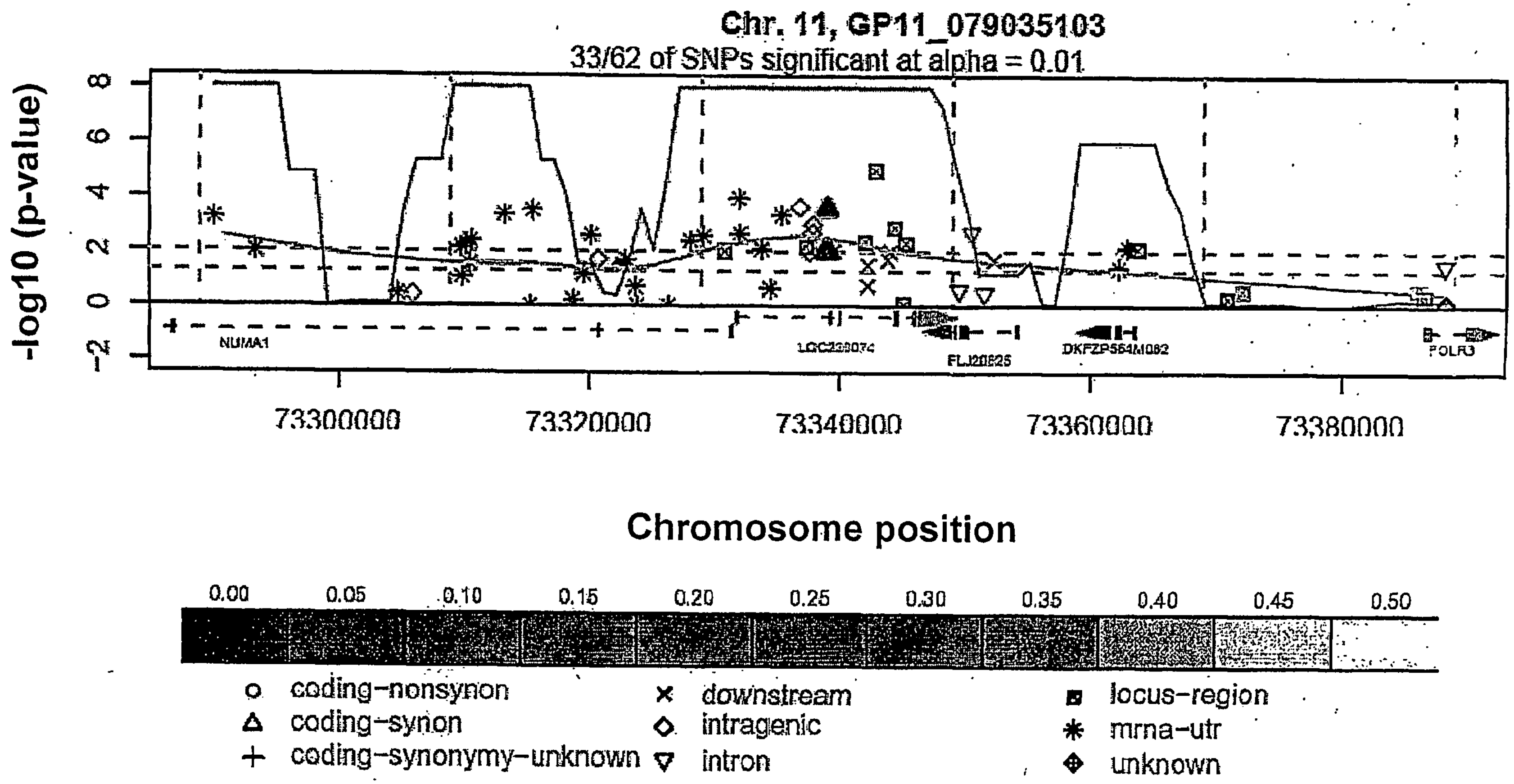

# FIGURE 18

ICAM region Meta Analysis

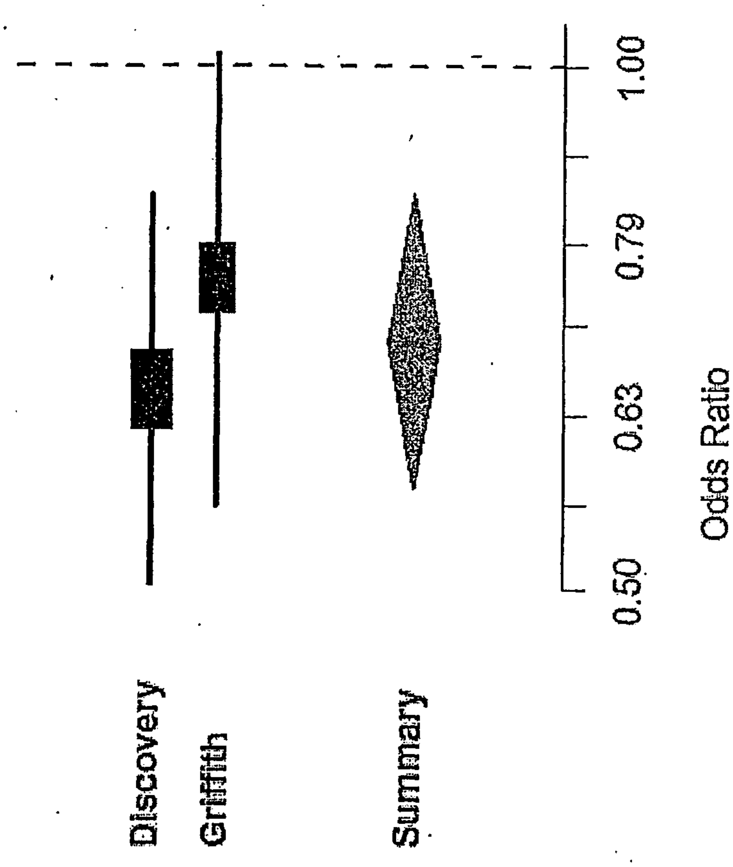

FIGURE 19

MAPK10 Meta Analysis

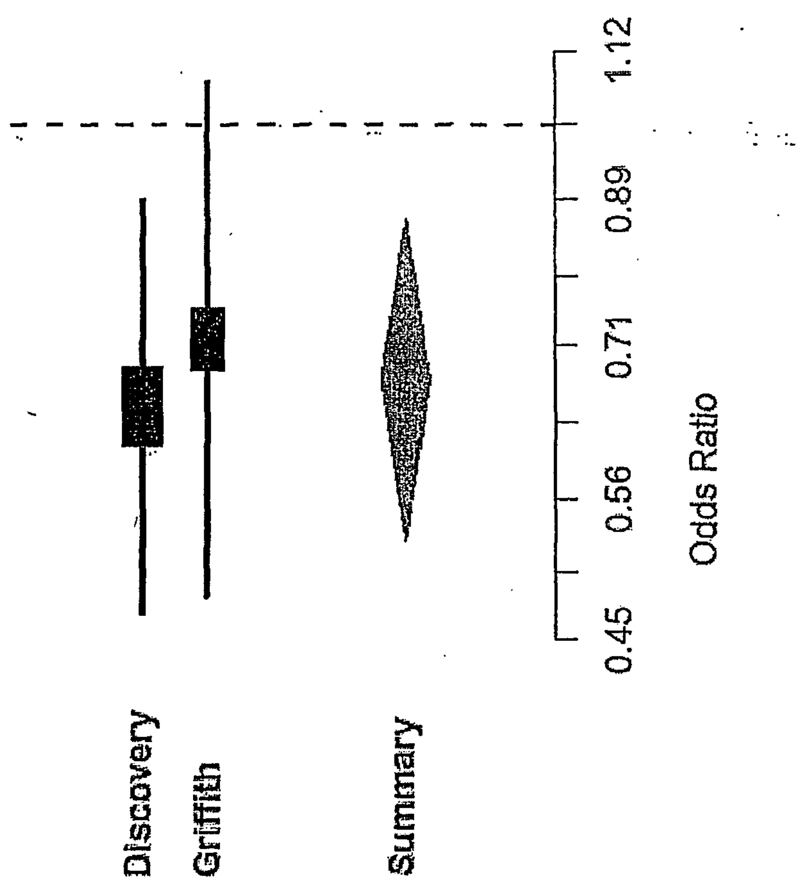

# FIGURE 20

KIAA0861 Meta Analysis

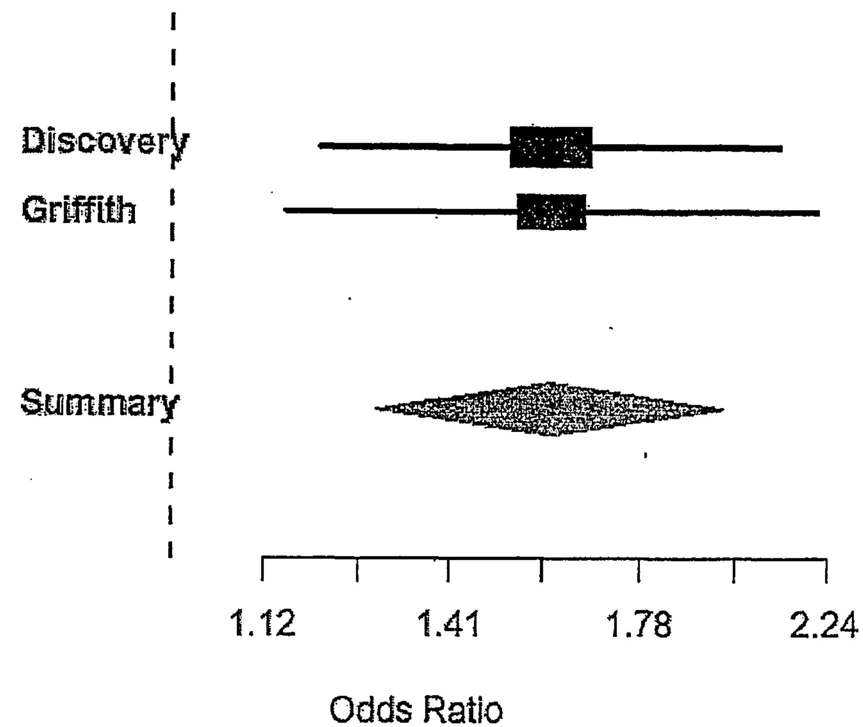

# FIGURE 21

NUMA1/LOC220074/FLJ20625 Meta Analysis

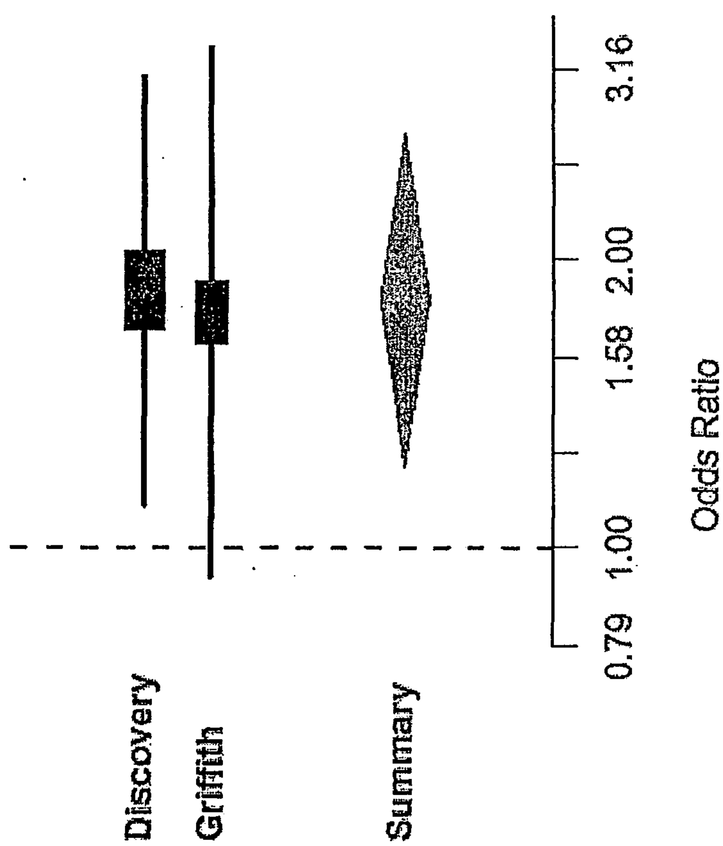

# FIGURE 22

ICAM1 in MCF7 cells

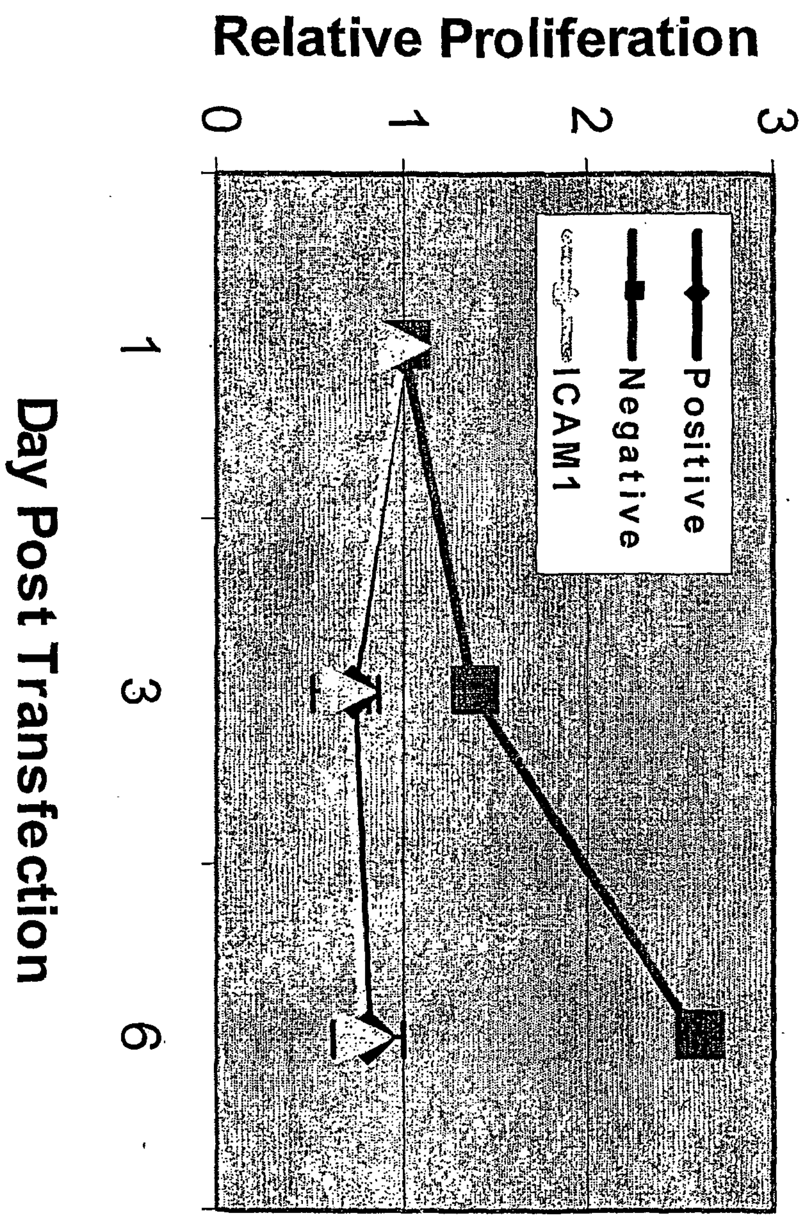

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4469863 A
- US 5536821 A
- US 5541306 A
- US 5637683 A
- US 5637684 A
- US 5700922 A
- US 5717083 A
- US 5719262 A
- US 5739308 A
- US 5773601 A
- US 5886165 A
- US 5929226 A
- US 5977296 A
- US 6140482 A
- WO 0056746 A
- WO 0114398 A
- US 5614622 A
- US 5739314 A
- US 5955599 A
- US 5962674 A
- US 6117992 A
- WO 0075372 A
- US 4873316 A
- EP 264166 A
- US 5272071 A
- WO 9106667 A
- US 4179337 A
- EP 0401384 A
- US 60429136 B
- US 60490234 B
- US 5679524 A
- US 5952174 A
- WO 0127326 A
- US 5851770 A
- US 5958692 A
- US 6110684 A
- US 6183958 A
- US 5891625 A
- US 6013499 A
- US 4683195 A
- US 4683202 A
- US 4965188 A
- US 5656493 A
- US 5998143 A
- US 6140054 A
- WO 0127327 A
- WO 0127329 A
- US 4656127 A
- US 4851331 A
- US 5834189 A
- US 5876934 A
- US 5908755 A
- US 5912118 A
- US 5976802 A
- US 5981186 A
- US 6004744 A
- US 6013431 A
- US 6017702 A
- US 6046005 A
- US 6087095 A
- US 6210891 A
- WO 0120039 A
- US 5547835 A
- US 5605798 A
- US 5691141 A
- US 5849542 A
- US 5869242 A
- US 5928906 A
- US 6043031 A
- US 6194144 A
- US 5492806 A
- US 5525464 A
- US 5589330 A
- US 5695940 A
- US 5849483 A
- US 6018041 A
- US 6045996 A
- US 6136541 A
- US 6142681 A
- US 6156501 A
- US 6197506 A
- US 6223127 A
- US 6225625 A
- US 6229911 A
- US 6239273 A
- WO 0052625 A
- WO 0125485 A
- WO 0129259 A
- US 4889818 A
- US 6077664 A
- WO 0220616 A
- US 9801373 W
- US 5223409 A
- US 5093246 A
- US 4987071 A, Cech
- US 5116742 A, Cech
- US 6506559 B, Fire
- WO 0175164 A, Tuschl

- WO 03010180 A1, Kay
- US 2001000993183 A
- WO 8809810 A
- WO 8910134 A
- US 5854033 A, Lizardi
- US 5866336 A, Nazarenko
- US 5876930 A, Livak
- US 8602269 W
- EP 184187 A
- EP 171496 A
- EP 173494 A
- WO 8601533 A, Neuberger
- US 4816567 A, Cabilly
- EP 125023 A
- US 5225539 A
- US 5625126 A
- US 5633425 A
- US 5569825 A
- US 5661016 A
- US 5545806 A
- US 4676980 A, Segal
- US 5631169 A, Lakowicz
- US 4868103 A, Stavrianopoulos
- US 4109496 A
- US 5283317 A
- WO 9410300 A, Brent
- US 4522811 A
- US 5328470 A
- DE 60429136
- DE 60490234

**Non-patent literature cited in the description**

- **Forbes.** *Seminars in Oncology,* 1997, vol. 24 (1), S1-20S1-35
- **Miki et al.** *Science,* 1994, vol. 266, 66-71
- **Wooster et al.** *Science,* 1994, vol. 265, 2088-2090
- **Ford et al.** *British J. Cancer,* 1995, vol. 72, 805-812
- **Goeddel.** Gene Expression Technology: Methods in Enzymology. Academic Press, 1990, vol. 185
- **Smith ; Johnson.** *Gene,* 1988, vol. 67, 31-40
- **Gottesman, S.** Gene Expression Technology: Methods in Enzymology. Academic Press, 1990, vol. 185, 119-128
- **Wada et al.** *Nucleic Acids Res.,* 1992, vol. 20, 2111-2118
- **Pinkert et al.** *Genes Dev.,* 1987, vol. 1, 268-277
- **Calame ; Eaton.** *Adv. Immunol.,* 1988, vol. 43, 235-275
- **Winoto ; Baltimore.** *EMBO J.,* 1989, vol. 8, 729-733
- **Banerji et al.** *Cell,* 1983, vol. 33, 729-740
- **Queen ; Baltimore.** *Cell,* 1983, vol. 33, 741-748
- **Byme ; Ruddle.** *Proc. Natl. Acad. Sci. USA,* 1989, vol. 86, 5473-5477
- **Edlund et al.** *Science,* 1985, vol. 230, 912-916
- **Kessel ; Gruss.** *Science,* 1990, vol. 249, 374-379
- **Campes ; Tilghman.** *Genes Dev.,* 1989, vol. 3, 537-546
- **Weintraub et al.** Antisense RNA as a molecular tool for genetic analysis. *Reviews - Trends in Genetics,* 1986, vol. 1 (1
- **Creighton.** Proteins. W. H. Freeman and Company, 1983
- **Hunkapiller et al.** *Nature,* 12 July 1984, vol. 310 (5973), 105-11
- **Malik et al.** *Exp Hematol.,* September 1992, vol. 20 (8), 1028-35
- **Meyers ; Miller.** *CABIOS,* 1989, vol. 4, 11-17
- **Needleman ; Wunsch.** *J. Mol. Biol.,* 1970, vol. 48, 444-453
- Current Protocols in Molecular Biology. John Wiley & Sons, 1989, 6.3.1-6.3.6
- **Altschul et al.** *J. Mol. Biol.,* 1990, vol. 215, 403-10
- **Altschul et al.** *Nucleic Acids Res.,* 1997, vol. 25 (17), 3389-3402
- **Agresti.** Categorical Data Analysis. Wiley, 2002
- **Zuckermann et al.** *J. Med. Chem.,* 1994, vol. 37, 2678-85
- **Lam.** *Anticancer Drug Des.,* 1997, vol. 12, 145
- **DeWitt et al.** *Proc. Natl. Acad. Sci. U.S.A.,* 1993, vol. 90, 6909
- **Erb et al.** *Proc. Natl. Acad. Sci. USA,* 1994, vol. 91, 11422
- **Zuckermann et al.** *J. Med. Chem.,* 1994, vol. 37, 2678
- **Cho et al.** *Science,* 1993, vol. 261, 1303
- **Carrell et al.** *Angew. Chem. Int. Ed. Engl.,* 1994, vol. 33, 2059
- **Carell et al.** *Angew. Chem. Int. Ed. Engl.,* 1994, vol. 33, 2061
- **Gallop et al.** *J. Med. Chem.,* 1994, vol. 37, 1233
- **Houghten.** *Biotechniques,* 1992, vol. 13, 412-421
- **Lam.** *Nature,* 1991, vol. 354, 82-84
- **Fodor.** *Nature,* 1993, vol. 364, 555-556
- **Cull et al.** *Proc. Natl. Acad. Sci. USA,* 1992, vol. 89, 1865-1869
- **Scott ; Smith.** *Science,* 1990, vol. 249, 386-390
- **Devlin.** *Science,* 1990, vol. 249, 404-406

- **Cwirla et al.** *Proc. Natl. Acad. Sci.,* 1990, vol. 87, 6378-6382
- **Felici.** *J. Mol. Biol.,* 1991, vol. 222, 301-310
- **Gaultier et al.** *Nucleic Acids. Res.,* 1987, vol. 15, 6625-6641
- **Inoue et al.** *Nucleic Acids Res.,* 1987, vol. 15, 6131-6148
- **Inoue et al.** *FEBS Lett.,* 1987, vol. 215, 327-330
- **Haselhoff ; Gerlach.** *Nature,* 1988, vol. 334, 585-591
- **Bartel ; Szostak.** *Science,* 1993, vol. 261, 1411-1418
- **Helene.** *Anticancer Drug Des.,* 1991, vol. 6 (6), 569-84
- **Helene et al.** *Ann. N.Y. Acad. Sci.,* 1992, vol. 660, 27-36
- **Maher.** *Bioassays,* 1992, vol. 14 (12), 807-15
- **Bosher JM, Labouesse.** *Nat Cell Biol,* February 2000, vol. 2 (2), E31-6
- **Caplen et al.** *Proc Natl Acad Sci U S A.,* 14 August 2001, vol. 98 (17), 9742-7
- **Elbashir et al.** *Methods,* February 2002, vol. 26 (2), 199-213
- **Caplen et al.** *Proc Natl Acad Sci U S A*
- **Abderrahmani et al.** *Mol Cell Biol,* 21 November 2001, vol. 21, 7256-67
- **Elbashir et al.** *Methods,* 2002, vol. 26, 199-213
- **Hyrup et al.** *Bioorganic & Medicinal Chemistry,* 1996, vol. 4 (1), 5-23
- **Perry-O'Keefe et al.** *Proc. Natl. Acad. Sci.,* 1996, vol. 93, 14670-675
- **Letsinger et al.** *Proc. Natl. Acad. Sci. USA,* 1989, vol. 86, 6553-6556
- **Lemaitre et al.** *Proc. Natl. Acad. Sci. USA,* 1987, vol. 84, 648-652
- **Krol et al.** *Bio-Techniques,* 1988, vol. 6, 958-976
- **Zon.** *Pharm. Res.,* 1988, vol. 5, 539-549
- **Better et al.** *Science,* 1988, vol. 240, 1041-1043
- **Liu et al.** *Proc. Natl. Acad. Sci. USA,* 1987, vol. 84, 3439-3443
- **Liu et al.** *J. Immunol.,* 1987, vol. 139, 3521-3526
- **Sun et al.** *Proc. Natl. Acad. Sci. USA,* 1987, vol. 84, 214-218
- **Nishimura et al.** *Canc. Res.,* 1987, vol. 47, 999-1005
- **Wood et al.** *Nature,* 1985, vol. 314, 446-449
- **Shaw et al.** *J. Natl. Cancer Inst.,* 1988, vol. 80, 1553-1559
- **Morrison, S. L.** *Science,* 1985, vol. 229, 1202-1207
- **Oi et al.** *BioTechniques,* 1986, vol. 4, 214
- **Jones et al.** *Nature,* 1986, vol. 321, 552-525
- **Verhoeyan et al.** *Science,* vol. 239, 1534
- **Beidler et al.** *J. Immunol.,* 1988, vol. 141, 4053-4060
- **Lonberg ; Huszar.** *Int. Rev. Immunol.,* 1995, vol. 13, 65-93
- **Jespers et al.** *Bio/Technology,* 1994, vol. 12, 899-903
- **Colcher et al.** *Ann. N Y Acad. Sci.,* 1999, vol. 880, 263-80
- **Reiter.** *Clin. Cancer Res.,* 1996, vol. 2, 245-52
- **McConnell, H. M. et al.** *Science,* 1992, vol. 257, 1906-1912
- **Sjolander ; Urbaniczk.** *Anal. Chem.,* 1991, vol. 63, 2338-2345
- **Szabo et al.** *Curr. Opin. Struct. Biol.,* 1995, vol. 5, 699-705
- **Rivas, G. ; Minton, A. P.** *Trends Biochem Sci,* August 1993, vol. 18 (8), 284-7
- Current Protocols in Molecular Biology. J. Wiley, 1999
- **Heegaard.** *J Mol. Recognit. Winter,* 1998, vol. 11 (1-6), 141-8
- **Hage ; Tweed.** *J. Chromatogr. B Biomed. Sci. Appl.,* 10 October 1997, vol. 699 (1-2), 499-525
- **Zervos et al.** *Cell,* 1993, vol. 72, 223-232
- **Madura et al.** *J. Biol. Chem.,* 1993, vol. 268, 12046-12054
- **Bartel et al.** *Biotechniques,* 1993, vol. 14, 920-924
- **Iwabuchi et al.** *Oncogene,* 1993, vol. 8, 1693-1696
- **Chen et al.** *Proc. Natl. Acad. Sci. USA,* 1994, vol. 91, 3054-3057
- **Osborne et al.** *Curr. Opin. Chem. Biol.,* 1997, vol. 1 (1), 5-9
- **Patel, D. J.** *Curr. Opin. Chem. Biol.,* June 1997, vol. 1 (1), 32-46
- **Herlyn, D.** *Ann. Med.,* 1999, vol. 31 (1), 66-78
- **Bhattacharya-Chatterjee ; Foon.** *Cancer Treat. Res.,* 1998, vol. 94, 51-68
- **Marasco et al.** *Proc. Natl. Acad. Sci. USA,* 1993, vol. 90, 7889-7893
- **Ansell et al.** *Current Opinion in Biotechnology,* 1996, vol. 7, 89-94
- **Shea.** *Trends in Polymer Science,* 1994, vol. 2, 166-173
- **Vlatakis et al.** *Nature,* 1993, vol. 361, 645-647
- **Kriz et al.** *Analytical Chemistry,* 1995, vol. 67, 2142-2144
- **W.S. Cleveland ; E. Grosse ; W.M. Shyu.** Local regression models. Chapter 8 of Statistical Models. Wadsworth & Brooks/Cole, 1992
- **W.S. Cleveland ; E. Grosse ; W.M. Shyu.** Local regression models. Chapter 8 of Statistical Models. Wadsworth & Brooks/Cole, 1992
- **Chunming ; Cantor.** *PNAS,* 2003, vol. 100 (6), 3059-3064